(19) Europäisches Patentamt
European Patent Office
Office européen des brevets

(11) EP 4 450 501 A1

(12) EUROPEAN PATENT APPLICATION
published in accordance with Art. 153(4) EPC

(43) Date of publication:
**23.10.2024 Bulletin 2024/43**

(21) Application number: **22906704.6**

(22) Date of filing: **16.12.2022**

(51) International Patent Classification (IPC):
***C07D 471/04*** (2006.01) ***C07D 519/00*** (2006.01)
***A61P 35/00*** (2006.01) ***A61P 35/02*** (2006.01)
***A61K 31/519*** (2006.01) ***A61K 31/4375*** (2006.01)
***A61K 31/5377*** (2006.01) ***A61K 31/439*** (2006.01)
***A61K 31/55*** (2006.01) ***A61K 31/5386*** (2006.01)

(52) Cooperative Patent Classification (CPC):
**A61K 31/4375; A61K 31/439; A61K 31/444; A61K 31/4545; A61K 31/519; A61K 31/5377; A61K 31/5386; A61K 31/55; A61P 35/00; A61P 35/02; C07D 471/04; C07D 519/00**

(86) International application number:
**PCT/CN2022/139704**

(87) International publication number:
**WO 2023/109959 (22.06.2023 Gazette 2023/25)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **16.12.2021 CN 202111543257**
**02.06.2022 CN 202210632338**
**03.08.2022 CN 202210928526**

(71) Applicants:
- **TransThera Sciences (Nanjing), Inc.**
**Nanjing, Jiangsu 210032 (CN)**
- **Teijin Pharma Limited**
**Tokyo 100-8585 (JP)**

(72) Inventors:
- **WU, Frank**
**Nanjing, Jiangsu 210032 (CN)**
- **LI, Lin**
**Nanjing, Jiangsu 210032 (CN)**
- **WANG, Wuwei**
**Nanjing, Jiangsu 210032 (CN)**
- **YOKOSAKA, Takuya**
**Tokyo 100--8585 (JP)**
- **MIYANO, Natsumi**
**Tokyo 100--8585 (JP)**
- **KAWASAKI, Masanori**
**Tokyo 100--8585 (JP)**

(74) Representative: **Carpmaels & Ransford LLP**
**One Southampton Row**
**London WC1B 5HA (GB)**

# (54) CDK9 INHIBITOR AND USE THEREOF

(57) The present invention falls within the technical field of medicine, and in particular relates to CDK9 inhibitor compounds as shown in formula (I), its pharmaceutically acceptable salt or isomer thereof, and also relates to pharmaceutical compositions and pharmaceutical formulation of the compounds and the uses thereof. $X_1$, $X_2$, $R_1$, $R_2$, $R_3$, $R_6$, A, L, n and m are as defined in the description. The compounds can be used to prepare drugs for treating or preventing related diseases mediated by CDK9.

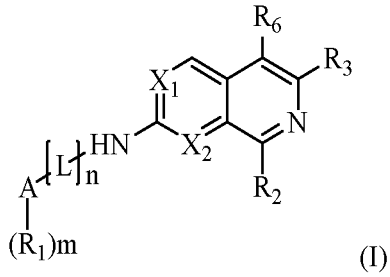

(I)

EP 4 450 501 A1

## Description

### TECHNICAL FIELD

The present invention relates to the technical field of pharmaceuticals, and particularly to a cyclin-dependent kinase 9 inhibitor compound of formula (I) or a pharmaceutically acceptable salt or an isomer thereof, and use thereof.

### BACKGROUND

Cyclin-dependent kinases (CDKs) are a group of serine/threonine protein kinases that act synergistically with cyclin. In the kinases of CMGC kinase family including cyclin-dependent kinase, mitogen-activated protein kinase, glycogen synthase kinase and CDC-like kinase, 20 of which belong to CDK family, and CDK inhibitors may fall into two categories based on the mechanism of action: control of the cell cycle (e.g., CDKs 1, 2, 4, 6) and control of cell transcription (e.g., CDKs 7, 8, 9, 12, 13). [1]Structural insights into the functional diversity of the CDK-cyclin family[J]. Open Biology, 2018, 8(9):180112.

Heterodimers formed by cyclin-dependent kinase 9 (CDK9) and cyclin T or K are involved in forming positive transcription elongation factor b (P-TEFb), which can phosphorylate the C-terminal domain (CTD) of RNA polymerase II (RNApoly II) to drive transcriptional elongation of mRNA. CDK9 plays an important role in the transcription and can regulate the expression of downstream short-cycle anti-apoptotic proteins such as MCL-1 and BCL-2. MCL-1 highly expressed in many cancers is closely related to the growth and survival of tumor cells, and tumor cells require continuously activated P-TEFb in order to maintain MCL-1 having a long half-life in a highly expressed state. The inhibition against CDK9 can inhibit transcription, reducing the expression level of MCL-1 and thereby promoting the apoptosis of tumor cells. [2] CDK9: A Comprehensive Review of Its Biology, and Its Role as a Potential Target for Anti-Cancer Agents[J]. Frontiers in Oncology, 2021, 11:678559.

In order to maintain MYC in a highly expressed state similar to MCL, tumor cells also require continuously activated P-TEFb. MYC protein is an important transcription regulator in cells and plays an important role in cell proliferation, differentiation, maintenance and other physiological processes. Normal MYC protein and mRNA have a short half-life, MYC transcription level is reduced after upstream signaling pathway stimulation is removed, protein level is rapidly reduced, and cell proliferation and differentiation are stopped. While MYC genes in cancer cells are mutated, amplified or translocated, resulting in MYC overexpression or overactivation, and the cancer cells begin irregulated proliferation and differentiation. Thus the MYC gene is one of the important proto-oncogenes. Due to the protein-protein interaction of MYC protein and the lack of protein binding pocket that small molecules can recognize and bind, the development of an inhibitor directly targeting MYC protein is full of challenges, and therefore, MYC protein is considered as an undruggable target by the pharmaceutical industry, and no drug aiming at the MYC target is approved currently. [3] Dang C V, Reddy E P, Shokat K M, et al. Drugging the 'undruggable' cancer targets[J]. Nature Reviews Cancer, 2017. Thus the inhibition against CDK9 also provides a new therapeutic approach for MYC-related tumors.

Although several CDK9 inhibitors have been put into clinical researches, the inhibitors are broad spectrum CDK inhibitors due to the conservation of CDK family structure and may cause unexpected side effects. For example, clinical researches on roniclib have been suspended due to severe toxic side effects. [4] Wu T, Qin Z, Tian Y, et al. Recent Developments in the Biology and Medicinal Chemistry of CDK9 Inhibitors: An Update[J]. Journal of Medicinal Chemistry, 2020, 63(22): 13228-13257. Thus there is a clinical need for the development of CDK9 inhibitors with high activity and high selectivity to improve the safety and efficacy of the compounds.

### SUMMARY

One purpose of the present invention is to provide a class of CDK9 inhibitors or pharmaceutically acceptable salts or isomers thereof. The compounds disclosed herein have good inhibitory activity against CDK9 and better selectivity for CDK9 compared with CDK1, 2, 3 and 5. The compounds disclosed herein can treat or prevent CDK9-mediated related diseases such as cancers.

The technical solution of the present invention is as follows.

**The present invention provides the compound represented by formula (I), its pharmaceutically acceptable salt or isomer thereof:**

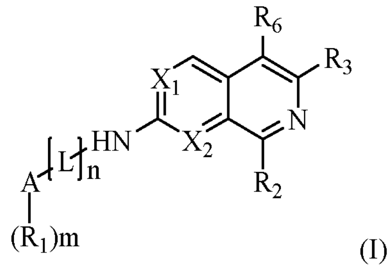

(I)

$X_1$ is selected from N or $CR_4$;
$X_2$ is selected from N or $CR_5$;
L is selected from bond, -C(O)-, $-(CH_2)p-$;
A is selected from bond, 3-12 membered cycloalkyl, 3-12 membered cycloalkenyl, 3-12 membered heterocyclyl, aryl, 5-10 membered heteroaryl, wherein the heteroatoms of the 3-12 membered heterocyclyl are selected from one of O, S, N or any combination thereof, C atom can be optionally oxidized to C(O), S atom can be optionally oxidized to S(O) or $S(O)_2$, the heteroatoms of the 5-10 membered heteroaryl are selected from one of O, S, and N or any combination thereof;
$R_1$ is selected from hydrogen, halogen, hydroxyl, carboxyl, amino, $C_{1-6}$ alkyl, $C_{1-6}$ alkoxy, $C_{1-6}$ alkylamino, $(C_{1-6}$ alkyl$)_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonylamino, $C_{1-6}$ alkylaminocarbonyl, $(C_{1-6}$ alkyl$)_2$ aminocarbonyl, $C_{1-6}$ alkoxycarbonyl, $C_{1-6}$ alkylcarbonyl, $C_{1-6}$ alkylsulfonyl, aminosulfonyl, $C_{1-6}$ alkylthio, 3-12 membered cycloalkyl, 3-12 membered heterocyclyl, 3-12 membered cycloalkenyl, wherein the heteroatoms of the 3-12 membered heterocyclyl are selected from one of O, S, N or any combination thereof, C atom can be optionally oxidized to C(O), S atom can be optionally oxidized to S(O) or $S(O)_2$, the heteroatoms of the 5-10 membered heteroaryl are selected from one of O, S, and N or any combination thereof,
wherein the amino, $C_{1-6}$ alkyl, $C_{1-6}$ alkoxy, $C_{1-6}$ alkylamino, $(C_{1-6}$ alkyl$)_2$ amino, halogenated $C_{1-6}$ alkyl , halogenated $C_{1-6}$ alkoxy, $C_{2-8}$ alkenyl, $C_{2-8}$ alkynyl, $C_{1-6}$ alkylcarbonylamino, $C_{1-6}$ alkylaminocarbonyl, $(C_{1-6}$ alkyl$)_2$ aminocarbonyl, $C_{1-6}$ alkoxycarbonyl,$C_{1-6}$ alkylcarbonyl, $C_{1-6}$ alkylsulfonyl, aminosulfonyl, $C_{1-6}$ alkylthio, 3-12 membered cycloalkyl, 3-12 membered heterocyclyl, 3-12 membered cycloalkenyl are unsubstituted or optionally substituted by one or more groups selected from hydroxyl, amino, carboxy, cyano, nitro, halogen, $C_{1-6}$ alkyl, $C_{1-6}$ alkoxy, halogenated $C_{1-6}$ alkyl, halogenated $C_{1-6}$ alkoxy, $C_{1-6}$ alkylamino, $(C_{1-6}$ alkyl$)_2$ amino, $C_{1-6}$ alkylsulfonyl, HS(O)(=NH)-, $C_{1-6}$ alkyl-S(O)(=NH)-, $(C_{1-6}$ alkyl$)_2$ aminocarbonyl, $C_{1-6}$ alkyl carbonyl, 3-12 membered cycloalkyl, 3-12 membered heterocyclyl;
$R_2$ is selected from halogen, cyano, hydroxyl, carboxyl, amino, $C_{1-6}$ alkyl, $C_{1-6}$ alkoxy, $C_{1-6}$ alkylthio, $C_{1-6}$ alkylamino, $(C_{1-6}$ alkyl$)_2$ amino, 3-12 membered cycloalkyl, 3-12 membered heterocyclyl, 3-12 membered cycloalkenyl, 3-12 membered heterocyclyloxy, 3-12 membered cycloalkyloxy, 3-12 membered cycloalkyl amino, 3-12 membered heterocyclyl-$CH_2$-amino, wherein the amino, $C_{1-6}$ alkyl, $C_{1-6}$ alkoxy, $C_{1-6}$ alkylthio, $C_{1-6}$ alkylamino, $(C_{1-6}$ alkyl$)_2$ amino, 3-12 membered cycloalkyl, 3-12 membered heterocyclyl, 3-12 membered cycloalkenyl, 3-12 membered heterocyclyloxy, 3-12 membered cycloalkyloxy, 3-12 membered cycloalkyl amino, 3-12 membered heterocyclyl-$CH_2$-amino are unsubstituted or optionally substituted by one or more groups selected from halogen, cyano, hydroxyl, carboxy, amino, $C_{1-6}$ alkyl, $C_{1-6}$ alkoxy, halogenated $C_{1-6}$ alkyl, halogenated $C_{1-6}$ alkoxy, $C_{1-6}$ alkylamino, $(C_{1-6}$ alkyl$)_2$ amino, 3-12 membered cycloalkyl, 3-12 membered heterocyclyl, aryl, 3-12 membered cycloalkenyl and 5-10 membered heteroaryl;
$R_3$ is selected from hydrogen, hydroxy, amino, carboxy, aminocarbonyl, cyano, nitro, halogen, $C_{1-6}$ alkyl, $C_{1-6}$ alkoxy, $C_{1-6}$ alkylamino, $(C_{1-6}$ alkyl$)_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonyl, $C_{1-6}$ alkylsulfonyl, $C_{1-6}$ alkylthio , 3-12 membered cycloalkyl, 3-12 membered cycloalkenyl, 3-12 membered heterocyclyl, wherein the amino, aminocarbonyl, $C_{1-6}$ alkyl, $C_{1-6}$ alkoxy, $C_{1-6}$ alkylamino, $(C_{1-6}$ alkyl$)_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonyl, $C_{1-6}$ alkylsulfonyl, $C_{1-6}$ alkylthio, 3-12 membered cycloalkyl, 3-12 membered cycloalkenyl, 3-12 membered heterocyclyl are unsubstituted or optionally substituted by one or more groups selected from hydroxyl, amino, carboxyl, cyano, nitro, halogen, $C_{1-6}$ alkyl, $C_{1-6}$ alkoxy, $C_{1-6}$ alkoxy$C_{1-6}$ alkoxy, $C_{1-6}$ alkylamino, $(C_{1-6}$ alkyl$)_2$ amino, $C_{1-6}$ alkylcarbonylamino and $C_{1-6}$ alkylsulfonylamino;
n is 0 or 1;
m is 0, 1, 2 or 3;
p is 1, 2 or 3;
$R_4$, $R_5$ and $R_6$ are each independently selected from hydrogen, halogen, hydroxy, amino, carboxy, cyano, nitro, $C_{1-6}$ alkyl, $C_{2-8}$ alkenyl, $C_{2-8}$ alkynyl, and halogenated $C_{1-6}$ alkyl.

**Some embodiments of the present invention relate to the compound shown in formula (I), or the pharmaceutically acceptable salt or the isomer thereof,**

the following cases are excluded from the compound represented by formula (I) ; case (i) $X_1$ is N, $X_2$ is N, n is 0, A is piperidinyl, and $R_1$ is selected from $C_{1-6}$ alkylsulfonyl, aminosulfonyl or $C_{1-6}$ alkylaminosulfonyl,
case (ii) $X_1$ is N, $X_2$ is N, n is 0, A is bond and $R_1$ is piperidinyl, wherein the piperidinyl is substituted by $C_{1-6}$ alkylsulfonyl, aminosulfonyl or $C_{1-6}$ alkylaminosulfonyl.

**Some embodiments of the present invention relate to the compound shown in formula (I), or the pharmaceutically acceptable salt or the isomer thereof,**

$X_1$ is selected from N or $CR_4$;
$X_2$ is selected from N or $CR_5$;
L is selected from bond, -C(O)-, $-(CH_2)p-$;
A is selected from bond, 3-12 membered cycloalkyl, 3-12 membered cycloalkenyl, 3-12 membered heterocyclyl, aryl, 5-10 membered heteroaryl, wherein the heteroatoms of the 3-12 membered heterocyclyl are selected from one of O, S, N or any combination thereof, C atom can be optionally oxidized to C(O), S atom can be optionally oxidized to S(O) or $S(O)_2$, the heteroatoms of the 5-10 membered heteroaryl are selected from one of O, S, and N or any combination thereof;
$R_1$ is selected from hydrogen, halogen, hydroxyl, carboxyl, amino, $C_{1-6}$ alkyl, $C_{1-6}$ alkoxy, $C_{1-6}$ alkylamino, $(C_{1-6}$ alkyl$)_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonylamino, $C_{1-6}$ alkylaminocarbonyl, $(C_{1-6}$ alkyl$)_2$ aminocarbonyl, $C_{1-6}$ alkoxycarbonyl, $C_{1-6}$ alkylcarbonyl, $C_{1-6}$ alkylsulfonyl, aminosulfonyl, $C_{1-6}$ alkylthio, 3-12 membered cycloalkyl, 3-12 membered heterocyclyl, 3-12 membered cycloalkenyl, wherein the heteroatoms of the 3-12 membered heterocyclyl are selected from one of O, S, N or any combination thereof, C atom can be optionally oxidized to C(O), S atom can be optionally oxidized to S(O) or $S(O)_2$, the heteroatoms of the 5-10 membered heteroaryl are selected from one of O, S, and N or any combination thereof, wherein the amino, $C_{1-6}$ alkyl, $C_{1-6}$ alkoxy, $C_{1-6}$ alkylamino, $(C_{1-6}$ alkyl$)_2$ amino, halogenated $C_{1-6}$ alkyl , halogenated $C_{1-6}$ alkoxy, $C_{2-8}$ alkenyl, $C_{2-8}$ alkynyl, $C_{1-6}$ alkylcarbonylamino, $C_{1-6}$ alkylaminocarbonyl, $(C_{1-6}$ alkyl$)_2$ aminocarbonyl, $C_{1-6}$ alkoxycarbonyl,$C_{1-6}$ alkylcarbonyl, $C_{1-6}$ alkylsulfonyl, aminosulfonyl, $C_{1-6}$ alkylthio, 3-12 membered cycloalkyl, 3-12 membered heterocyclyl, 3-12 membered cycloalkenyl are unsubstituted or optionally substituted by one or more groups selected from hydroxyl, amino, carboxy, cyano, nitro, halogen, $C_{1-6}$ alkyl, $C_{1-6}$ alkoxy, halogenated $C_{1-6}$ alkyl, halogenated $C_{1-6}$ alkoxy, $C_{1-6}$ alkylamino, $(C_{1-6}$ alkyl$)_2$ amino, $C_{1-6}$ alkylsulfonyl, 3-12 membered cycloalkyl, 3-12 membered heterocyclyl;
$R_2$ is selected from halogen, cyano, hydroxyl, carboxyl, amino, $C_{1-6}$ alkyl, $C_{1-6}$ alkoxy, $C_{1-6}$ alkylamino, $(C_{1-6}$ alkyl$)_2$ amino, 3-12 membered cycloalkyl, 3-12 membered heterocyclyl, 3-12 membered cycloalkenyl, wherein the amino, $C_{1-6}$ alkyl, $C_{1-6}$ alkoxy, $C_{1-6}$ alkylamino, $(C_{1-6}$ alkyl$)_2$ amino, 3-12 membered cycloalkyl, 3-12 membered heterocyclyl, 3-12 membered cycloalkenyl are unsubstituted or optionally substituted by one or more groups selected from halogen, cyano, hydroxyl, carboxy, amino, $C_{1-6}$ alkyl, $C_{1-6}$ alkoxy, halogenated $C_{1-6}$ alkyl, halogenated $C_{1-6}$ alkoxy, $C_{1-6}$ alkylamino, $(C_{1-6}$ alkyl$)_2$ amino, 3-12 membered cycloalkyl, 3-12 membered heterocyclyl, aryl and 5-10 membered heteroaryl;
$R_3$ is selected from hydrogen, hydroxy, amino, carboxy, cyano, nitro, halogen, $C_{1-6}$ alkyl, $C_{1-6}$ alkoxy, $C_{1-6}$ alkylamino, $(C_{1-6}$ alkyl$)_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonyl, $C_{1-6}$ alkylsulfonyl, $C_{1-6}$ alkylthio , 3-12 membered cycloalkyl, 3-12 membered cycloalkenyl, 3-12 membered heterocyclyl, wherein the amino, $C_{1-6}$ alkyl, $C_{1-6}$ alkoxy, $C_{1-6}$ alkylamino, $(C_{1-6}$ alkyl$)_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonyl, $C_{1-6}$ alkylsulfonyl, $C_{1-6}$ alkylthio, 3-12 membered cycloalkyl, 3-12 membered cycloalkenyl, 3-12 membered heterocyclyl are unsubstituted or optionally substituted by one or more groups selected from hydroxyl, amino, carboxyl, cyano, nitro, halogen, $C_{1-6}$ alkyl, $C_{1-6}$ alkoxy, $C_{1-6}$ alkoxy$C_{1-6}$ alkoxy, $C_{1-6}$ alkylamino, $(C_{1-6}$ alkyl$)_2$ amino, $C_{1-6}$ alkylcarbonylamino and $C_{1-6}$ alkylsulfonylamino;
n is 0 or 1;
m is 0, 1, 2 or 3;
p is 1, 2 or 3;
$R_4$, $R_5$ and $R_6$ are each independently selected from hydrogen, halogen, hydroxy, amino, carboxy, cyano, nitro, $C_{1-6}$ alkyl, $C_{2-8}$ alkenyl, $C_{2-8}$ alkynyl, and halogenated $C_{1-6}$ alkyl.

**Some embodiments of the present invention relate to the compound shown in formula (I), or the pharmaceutically acceptable salt or the isomer thereof,**

$X_1$ is N;

$X_2$ is N;
L is selected from bond -C(O)-, or $-(CH_2)p-$;
A is selected from bond, 3-12 membered cycloalkyl, or 3-12 membered heterocyclyl , wherein the heteroatoms of the 3-12 membered heterocyclyl are selected from any one of O, S, N or any combination thereof;
$R_1$ is selected from hydrogen, hydroxyl, $C_{1-6}$ alkyl, $C_{1-6}$ alkoxy, amino, $(C_{1-6}$ alkyl$)_2$ amino, $C_{1-6}$ alkylcarbonyl, 3-12 membered heterocyclyl, halogen, carboxyl, or 3-12 membered cycloalkyl,
wherein the heteroatoms of the 3-12 membered heterocyclyl are selected from any one of O, S, N or any combination thereof,
wherein the $C_{1-6}$ alkyl, $C_{1-6}$ alkoxy, amino, $(C_{1-6}$ alkyl$)_2$ amino, $C_{1-6}$ alkylcarbonyl, 3-12 membered heterocyclyl and 3-12 membered cycloalkyl are optionally substituted by any one or more groups selected from hydroxyl, amino, carboxy, cyano, nitro, halogen, $C_{1-6}$ alkyl, $C_{1-6}$ alkoxy, halogenated $C_{1-6}$ alkyl, halogenated $C_{1-6}$ alkoxy, $C_{1-6}$ alkylamino, $(C_{1-6}$ alkyl$)_2$ aminocarbonyl, $C_{1-6}$ alkyl carbonyl, $(C_{1-6}$ alkyl$)_2$ amino, $C_{1-6}$ alkylsulfonyl, 3-12 membered cycloalkyl, or 3-12 membered heterocyclyl; $R_2$ is selected from amino, $C_{1-6}$ alkylamino, 3-12 membered heterocyclyl, or 3-12 membered cycloalkyl amino, halogen, cyano, hydroxyl, or $(C_{1-6}$ alkyl$)_2$ amino, wherein the heteroatoms of the 3-12 membered heterocyclyl are selected from any one of O, S, N or any combination thereof,
wherein the amino, $C_{1-6}$ alkylamino, 3-12 membered heterocyclyl, 3-12 membered cycloalkyl amino, and $(C_{1-6}$ alkyl$)_2$ amino are optionally substituted by any one or more groups selected from $C_{1-6}$ alkyl, $C_{1-6}$ alkoxy, 3-12membered cycloalkyl, 3-12 membered heterocyclyl, or 3-12 membered cycloalkenyl;
$R_3$ is selected from cyano, $C_{1-6}$ alkyl, aminocarbonyl, hydrogen, hydroxy, amino, halogen, or $C_{1-6}$ alkoxy,
wherein the $C_{1-6}$ alkyl, aminocarbonyl, amino and $C_{1-6}$ alkoxy are optionally substituted by any one or more groups selected from halogen;
$R_6$ is selected from hydrogen, halogen, hydroxy, amino, or $C_{1-6}$ alkyl;
n is 0 or 1;
m is 0, 1, 2 or 3;
p is 1, 2 or 3;
provided that
a case in which .n is 0, A is bond, $R_1$ is piperidinyl, and m is 1, and wherein the piperidinyl is substituted by $C_{1-6}$ alkylsulfonyl is excluded.

**Some embodiments of the present invention relate to the compound shown in formula (II), or the pharmaceutically acceptable salt or the isomer thereof, wherein L is bond, and n is 0:**

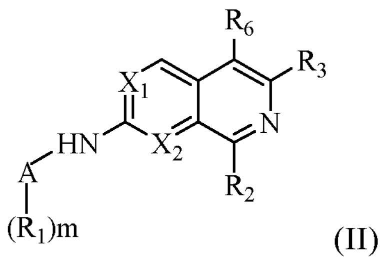

(II)

$X_1$ is selected from N or $CR_4$;
$X_2$ is selected from N or $CR_5$;
A is selected from 3-12 membered cycloalkyl, 3-12 membered cycloalkenyl, 3-12 membered heterocyclyl, aryl, 5-10 membered heteroaryl, wherein the heteroatoms of the 3-12 membered heterocyclyl are selected from one of O, S, N or any combination thereof, C atom can be optionally oxidized to C(O), S atom can be optionally oxidized to S(O) or $S(O)_2$, the heteroatoms of the 5-10 membered heteroaryl are selected from one of O, S, and N or any combination thereof;
$R_1$ is selected from hydrogen, halogen, hydroxyl, carboxyl, amino, $C_{1-6}$ alkyl, $C_{1-6}$ alkoxy, $C_{1-6}$ alkylamino, $(C_{1-6}$ alkyl$)_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonylamino, $C_{1-6}$ alkylaminocarbonyl, $(C_{1-6}$ alkyl$)_2$ aminocarbonyl, $C_{1-6}$ alkoxycarbonyl, $C_{1-6}$ alkylcarbonyl, $C_{1-6}$ alkylsulfonyl, aminosulfonyl, $C_{1-6}$ alkylthio, 3-12 membered cycloalkyl, 3-12 membered heterocyclyl, 3-12 membered cycloalkenyl, wherein the heteroatoms of the 3-12 membered heterocyclyl are selected from one of O, S, N or any combination thereof, C atom can be optionally oxidized to C(O), S atom can be optionally oxidized to S(O) or $S(O)_2$, the heteroatoms of the 5-10 membered heteroaryl are selected from one of O, S, and N or any combination thereof, wherein the amino, $C_{1-6}$ alkyl, $C_{1-6}$ alkoxy, $C_{1-6}$ alkylamino, $(C_{1-6}$ alkyl$)_2$ amino, halogenated $C_{1-6}$ alkyl , halogenated $C_{1-6}$ alkoxy, $C_{2-8}$ alkenyl, $C_{2-8}$ alkynyl, $C_{1-6}$ alkylcarbonylamino, $C_{1-6}$ alkylaminocarbonyl, $(C_{1-6}$ alkyl$)_2$ aminocarbonyl, $C_{1-6}$ alkox-

ycarbonyl,$C_{1-6}$ alkylcarbonyl, $C_{1-6}$ alkylsulfonyl, aminosulfonyl, $C_{1-6}$ alkylthio, 3-12 membered cycloalkyl, 3-12 membered heterocyclyl, 3-12 membered cycloalkenyl are unsubstituted or optionally substituted by one or more groups selected from hydroxyl, amino, carboxy, cyano, nitro, halogen, $C_{1-6}$ alkyl, $C_{1-6}$ alkoxy, halogenated $C_{1-6}$ alkyl, halogenated $C_{1-6}$ alkoxy, $C_{1-6}$ alkylamino, ($C_{1-6}$ alkyl)$_2$ amino, $C_{1-6}$ alkylsulfonyl, HS(O)(=NH)-, $C_{1-6}$ alkyl-S(O)(=NH)-, 3-12 membered cycloalkyl, 3-12 membered heterocyclyl;

$R_2$ is selected from halogen, cyano, hydroxyl, carboxyl, amino, $C_{1-6}$ alkyl, $C_{1-6}$ alkoxy, $C_{1-6}$ alkylamino, ($C_{1-6}$ alkyl)$_2$ amino, 3-12 membered cycloalkyl, 3-12 membered heterocyclyl, 3-12 membered cycloalkenyl, 3-12 membered heterocyclyloxy, 3-12 membered cycloalkyl amino, 3-12 membered heterocyclyl-$CH_2$-amino, wherein the amino, $C_{1-6}$ alkyl, $C_{1-6}$ alkoxy, $C_{1-6}$ alkylamino, ($C_{1-6}$ alkyl)$_2$ amino, 3-12 membered cycloalkyl, 3-12 membered heterocyclyl, 3-12 membered cycloalkenyl, 3-12 membered heterocyclyloxy, 3-12 membered cycloalkyl amino, 3-12 membered heterocyclyl-$CH_2$-amino are unsubstituted or optionally substituted by one or more groups selected from halogen, cyano, hydroxyl, carboxy, amino, $C_{1-6}$ alkyl, $C_{1-6}$ alkoxy, halogenated $C_{1-6}$ alkyl, halogenated $C_{1-6}$ alkoxy, $C_{1-6}$ alkylamino, ($C_{1-6}$ alkyl)$_2$ amino, 3-12 membered cycloalkyl, 3-12 membered heterocyclyl, aryl and 5-10 membered heteroaryl;

$R_3$ is selected from hydrogen, hydroxy, amino, carboxy, cyano, nitro, halogen, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonyl, $C_{1-6}$ alkylsulfonyl, $C_{1-6}$ alkylthio , 3-12 membered cycloalkyl, 3-12 membered cycloalkenyl, 3-12 membered heterocyclyl, wherein the amino, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonyl, $C_{1-6}$ alkylsulfonyl, $C_{1-6}$ alkylthio , 3-12 membered cycloalkyl, 3-12 membered cycloalkenyl, 3-12 membered heterocyclyl are unsubstituted or optionally substituted by one or more groups selected from hydroxyl, amino, carboxyl, cyano, nitro, halogen, $C_{1-6}$ alkyl, $C_{1-6}$ alkoxy, $C_{1-6}$ alkoxy$C_{1-6}$ alkoxy, $C_{1-6}$ alkylamino, ($C_{1-6}$ alkyl)$_2$ amino, $C_{1-6}$ alkylcarbonylamino and $C_{1-6}$ alkylsulfonylamino;

m is 0, 1, 2 or 3;

$R_4$, $R_5$ and $R_6$ are each independently selected from hydrogen, halogen, hydroxy, amino, carboxy, cyano, nitro, $C_{1-6}$ alkyl, $C_{2-8}$ alkenyl, $C_{2-8}$ alkynyl, and halogenated $C_{1-6}$ alkyl;

preferably, at least one of $X_1$ and $X_2$ is N.

**Some embodiments of the present invention relate to the compound shown in formula (II), or the pharmaceutically acceptable salt or the isomer thereof,**

$X_1$ is selected from N or $CR_4$;

$X_2$ is selected from N or $CR_5$;

A is selected from 3-12 membered cycloalkyl, 3-12 membered cycloalkenyl, 3-12 membered heterocyclyl, aryl, 5-10 membered heteroaryl, wherein the heteroatoms of the 3-12 membered heterocyclyl are selected from one of O, S, N or any combination thereof, C atom can be optionally oxidized to C(O), S atom can be optionally oxidized to S(O) or $S(O)_2$, the heteroatoms of the 5-10 membered heteroaryl are selected from one of O, S, and N or any combination thereof;

$R_1$ is selected from hydrogen, halogen, hydroxyl, carboxyl, amino, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonylamino, $C_{1-6}$ alkylaminocarbonyl, ($C_{1-6}$ alkyl)$_2$ aminocarbonyl, $C_{1-6}$ alkoxycarbonyl, $C_{1-6}$ alkylcarbonyl, $C_{1-6}$ alkylsulfonyl, aminosulfonyl, $C_{1-6}$ alkylthio, 3-12 membered cycloalkyl, 3-12 membered heterocyclyl, 3-12 membered cycloalkenyl, wherein the heteroatoms of the 3-12 membered heterocyclyl are selected from one of O, S, N or any combination thereof, C atom can be optionally oxidized to C(O), S atom can be optionally oxidized to S(O) or $S(O)_2$, the heteroatoms of the 5-10 membered heteroaryl are selected from one of O, S, and N or any combination thereof, wherein the amino, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl , halogenated $C_{1-6}$ alkoxy, $C_{2-8}$ alkenyl, $C_{2-8}$ alkynyl, $C_{1-6}$ alkylcarbonylamino, $C_{1-6}$ alkylaminocarbonyl, ($C_{1-6}$ alkyl)$_2$ aminocarbonyl, $C_{1-6}$ alkoxycarbonyl,$C_{1-6}$ alkylcarbonyl, $C_{1-6}$ alkylsulfonyl, aminosulfonyl, $C_{1-6}$ alkylthio, 3-12 membered cycloalkyl, 3-12 membered heterocyclyl, 3-12 membered cycloalkenyl are unsubstituted or optionally substituted by one or more groups selected from hydroxyl, amino, carboxy, cyano, nitro, halogen, $C_{1-6}$ alkyl, $C_{1-6}$ alkoxy, halogenated $C_{1-6}$ alkyl, halogenated $C_{1-6}$ alkoxy, $C_{1-6}$ alkylamino, ($C_{1-6}$ alkyl)$_2$ amino, $C_{1-6}$ alkylsulfonyl, 3-12 membered cycloalkyl, 3-12 membered heterocyclyl;

$R_2$ is selected from halogen, cyano, hydroxyl, carboxyl, amino, $C_{1-6}$ alkyl, $C_{1-6}$ alkoxy, $C_{1-6}$ alkylamino, ($C_{1-6}$ alkyl)$_2$ amino, 3-12 membered cycloalkyl, 3-12 membered heterocyclyl, 3-12 membered cycloalkenyl, wherein the amino, $C_{1-6}$ alkyl, $C_{1-6}$ alkoxy, $C_{1-6}$ alkylamino, ($C_{1-6}$ alkyl)$_2$ amino, 3-12 membered cycloalkyl, 3-12 membered heterocyclyl, 3-12 membered cycloalkenyl are unsubstituted or optionally substituted by one or more groups selected from halogen, cyano, hydroxyl, carboxy, amino, $C_{1-6}$ alkyl, $C_{1-6}$ alkoxy, halogenated $C_{1-6}$ alkyl, halogenated $C_{1-6}$ alkoxy, $C_{1-6}$ alkylamino, ($C_{1-6}$ alkyl)$_2$ amino, 3-12 membered cycloalkyl, 3-12 membered heterocyclyl, aryl and 5-10 membered heteroaryl;

$R_3$ is selected from hydrogen, hydroxy, amino, carboxy, cyano, nitro, halogen, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonyl, $C_{1-6}$ alkylsulfonyl, $C_{1-6}$ alkylthio , 3-12 membered cycloalkyl, 3-12 membered cycloalkenyl, 3-12 membered heterocyclyl, wherein the amino, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonyl, $C_{1-6}$ alkylsulfonyl, $C_{1-6}$ alkylthio , 3-12 membered cycloalkyl, 3-12 membered cycloalkenyl, 3-12 membered heterocyclyl are unsubstituted or optionally substituted by one or more groups selected from hydroxyl, amino, carboxyl, cyano, nitro, halogen, $C_{1-6}$ alkyl, $C_{1-6}$ alkoxy, $C_{1-6}$ alkoxy$C_{1-6}$ alkoxy, $C_{1-6}$ alkylamino, ($C_{1-6}$ alkyl)$_2$ amino, $C_{1-6}$ alkylcarbonylamino and $C_{1-6}$ alkylsulfonylamino;
m is 0, 1, 2 or 3;
$R_4$, $R_5$ and $R_6$ are each independently selected from hydrogen, halogen, hydroxy, amino, carboxy, cyano, nitro, $C_{1-6}$ alkyl, $C_{2-8}$ alkenyl, $C_{2-8}$ alkynyl, and halogenated $C_{1-6}$ alkyl;
preferably, at least one of $X_1$ and $X_2$ is N.

**Some embodiments of the present invention relate to the compound shown in formula (II), or the pharmaceutically acceptable salt or the isomer thereof,**

$R_1$ is selected from hydrogen, halogen, hydroxyl, carboxyl, amino, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl , halogenated $C_{1-6}$ alkoxy, $C_{2-8}$ alkenyl, $C_{2-8}$ alkynyl, $C_{1-6}$ alkylaminocarbonyl, ($C_{1-6}$ alkyl)$_2$ aminocarbonyl, $C_{1-6}$ alkoxycarbonyl, $C_{1-6}$ alkylcarbonyl, $C_{1-6}$ alkylsulfonyl, $C_{1-6}$ alkylthio, 3-12 membered cycloalkyl, 3-12 membered heterocyclyl, 3-12 membered cycloalkenyl, wherein the amino, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl , halogenated $C_{1-6}$ alkoxy, $C_{2-8}$ alkenyl, $C_{2-8}$ alkynyl, $C_{1-6}$ alkylaminocarbonyl, ($C_{1-6}$ alkyl)$_2$ aminocarbonyl, $C_{1-6}$ alkoxycarbonyl, $C_{1-6}$ alkylcarbonyl, $C_{1-6}$ alkylsulfonyl, $C_{1-6}$ alkylthio, 3-12 membered cycloalkyl, 3-12 membered heterocyclyl, 3-12 membered cycloalkenyl are unsubstituted or optionally substituted by one or more groups selected from hydroxyl, amino, carboxy, cyano, nitro, halogen, $C_{1-6}$ alkyl, $C_{1-6}$ alkoxy, halogenated $C_{1-6}$ alkyl, halogenated $C_{1-6}$ alkoxy, $C_{1-6}$ alkylamino, ($C_{1-6}$ alkyl)$_2$ amino, $C_{1-6}$ alkylsulfonyl, HS(O)(=NH)-, $C_{1-6}$ alkyl-S(O)(=NH)-, 3-12 membered cycloalkyl, 3-12 membered heterocyclyl;
$R_3$ is selected from cyano, and halogenated $C_{1-6}$ alkyl.

**Some embodiments of the present invention relate to the compound shown in formula (II), or the pharmaceutically acceptable salt or the isomer thereof,**

$X_1$ is N;
$X_2$ is N;
A is selected from bond, 3-12 membered cycloalkyl, or 3-12 membered heterocyclyl , wherein the heteroatoms of the 3-12 membered heterocyclyl are selected from any one of O, S, N or any combination thereof;
$R_1$ is selected from hydrogen, hydroxyl, $C_{1-6}$ alkyl, $C_{1-6}$ alkoxy, amino, ($C_{1-6}$ alkyl)$_2$ amino, $C_{1-6}$ alkylcarbonyl, 3-12 membered heterocyclyl, halogen, carboxyl, or 3-12 membered cycloalkyl,
wherein the heteroatoms of the 3-12 membered heterocyclyl are selected from any one of O, S, N or any combination thereof,
wherein the $C_{1-6}$ alkyl, $C_{1-6}$ alkoxy, amino, ($C_{1-6}$ alkyl)$_2$ amino, $C_{1-6}$ alkylcarbonyl, 3-12 membered heterocyclyl and 3-12 membered cycloalkyl are optionally substituted by any one or more groups selected from hydroxyl, amino, carboxy, cyano, nitro, halogen, $C_{1-6}$ alkyl, $C_{1-6}$ alkoxy, halogenated $C_{1-6}$ alkyl, halogenated $C_{1-6}$ alkoxy, $C_{1-6}$ alkylamino, ($C_{1-6}$ alkyl)$_2$ aminocarbonyl, $C_{1-6}$ alkyl carbonyl, ($C_{1-6}$ alkyl)$_2$ amino, $C_{1-6}$ alkyl sulfonyl, 3-12 membered cycloalkyl, or 3-12 membered heterocyclyl; $R_2$ is selected from amino, $C_{1-6}$ alkylamino, 3-12 membered heterocyclyl, or 3-12 membered cycloalkyl amino, halogen, cyano, hydroxyl, or ($C_{1-6}$ alkyl)$_2$ amino,
wherein the heteroatoms of the 3-12 membered heterocyclyl, or 3-12 membered cycloalkyl amino, are selected from any one of O, S, N or any combination thereof, wherein the amino, $C_{1-6}$ alkylamino, 3-12 membered heterocyclyl and ($C_{1-6}$ alkyl)$_2$ amino are optionally substituted by any one or more groups selected from $C_{1-6}$ alkyl, $C_{1-6}$ alkoxy, 3-12membered cycloalkyl, 3-12 membered heterocyclyl, or 3-12 membered cycloalkenyl;
$R_3$ is selected from cyano, $C_{1-6}$ alkyl, aminocarbonyl, hydrogen, hydroxy, amino, halogen, or $C_{1-6}$ alkoxy,
wherein the $C_{1-6}$ alkyl, aminocarbonyl, amino and $C_{1-6}$ alkoxy are optionally substituted by any one or more groups selected from halogen;
$R_6$ is selected from hydrogen, halogen, hydroxy, amino, or $C_{1-6}$ alkyl;
m is 0, 1, 2 or 3;
p is 1, 2 or 3.

**Some embodiments of the present invention relate to the compound shown in formula (III), or the pharmaceutically acceptable salt or the isomer thereof, wherein L is -C(O)-, n is 1:**

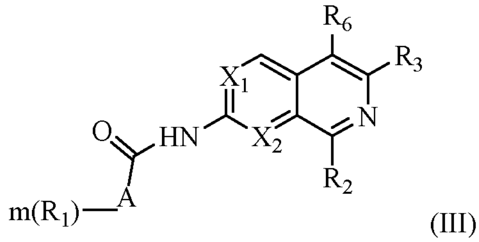

(III)

$X_1$ is selected from N or $CR_4$;
$X_2$ is selected from N or $CR_5$;
A is selected from 3-12 membered cycloalkyl, 3-12 membered cycloalkenyl, 3-12 membered heterocyclyl, aryl, 5-10 membered heteroaryl, wherein the heteroatoms of the 3-12 membered heterocyclyl are selected from one of O, S, N or any combination thereof, C atom can be optionally oxidized to C(O), S atom can be optionally oxidized to S(O) or $S(O)_2$, the heteroatoms of the 5-10 membered heteroaryl are selected from one of O, S, and N or any combination thereof;
$R_1$ is selected from hydrogen, halogen, hydroxyl, carboxyl, amino, $C_{1-6}$ alkyl, $C_{1-6}$ alkoxy, $C_{1-6}$ alkylamino, $(C_{1-6}$ alkyl$)_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonylamino, $C_{1-6}$ alkylaminocarbonyl, $(C_{1-6}$ alkyl$)_2$ aminocarbonyl, $C_{1-6}$ alkoxycarbonyl, $C_{1-6}$ alkylcarbonyl, $C_{1-6}$ alkylsulfonyl, aminosulfonyl, $C_{1-6}$ alkylthio, 3-12 membered cycloalkyl, 3-12 membered heterocyclyl, 3-12 membered cycloalkenyl, wherein the heteroatoms of the 3-12 membered heterocyclyl are selected from one of O, S, N or any combination thereof, C atom can be optionally oxidized to C(O), S atom can be optionally oxidized to S(O) or $S(O)_2$, the heteroatoms of the 5-10 membered heteroaryl are selected from one of O, S, and N or any combination thereof, wherein the amino, $C_{1-6}$ alkyl, $C_{1-6}$ alkoxy, $C_{1-6}$ alkylamino, $(C_{1-6}$ alkyl$)_2$ amino, halogenated $C_{1-6}$ alkyl , halogenated $C_{1-6}$ alkoxy, $C_{2-8}$ alkenyl, $C_{2-8}$ alkynyl, $C_{1-6}$ alkylcarbonylamino, $C_{1-6}$ alkylaminocarbonyl, $(C_{1-6}$ alkyl$)_2$ aminocarbonyl, $C_{1-6}$ alkoxycarbonyl,$C_{1-6}$ alkylcarbonyl, $C_{1-6}$ alkylsulfonyl, aminosulfonyl, $C_{1-6}$ alkylthio, 3-12 membered cycloalkyl, 3-12 membered heterocyclyl, 3-12 membered cycloalkenyl are unsubstituted or optionally substituted by one or more groups selected from hydroxyl, amino, carboxy, cyano, nitro, halogen, $C_{1-6}$ alkyl, $C_{1-6}$ alkoxy, halogenated $C_{1-6}$ alkyl, halogenated $C_{1-6}$ alkoxy, $C_{1-6}$ alkylamino, $(C_{1-6}$ alkyl$)_2$ amino, $C_{1-6}$ alkylsulfonyl, HS(O)(=NH)-, $C_{1-6}$ alkyl-S(O)(=NH)-, 3-12 membered cycloalkyl, 3-12 membered heterocyclyl;
$R_2$ is selected from halogen, cyano, hydroxyl, carboxyl, amino, $C_{1-6}$ alkyl, $C_{1-6}$ alkoxy, $C_{1-6}$ alkylamino, $(C_{1-6}$ alkyl$)_2$ amino, 3-12 membered cycloalkyl, 3-12 membered heterocyclyl, 3-12 membered cycloalkenyl, 3-12 membered heterocyclyloxy, 3-12 membered cycloalkyl amino, 3-12 membered heterocyclyl-$CH_2$-amino, wherein the amino, $C_{1-6}$ alkyl, $C_{1-6}$ alkoxy, $C_{1-6}$ alkylamino, $(C_{1-6}$ alkyl$)_2$ amino, 3-12 membered cycloalkyl, 3-12 membered heterocyclyl, 3-12 membered cycloalkenyl, 3-12 membered heterocyclyloxy, 3-12 membered cycloalkyl amino, 3-12 membered heterocyclyl-$CH_2$-amino are unsubstituted or optionally substituted by one or more groups selected from halogen, cyano, hydroxyl, carboxy, amino, $C_{1-6}$ alkyl, $C_{1-6}$ alkoxy, halogenated $C_{1-6}$ alkyl, halogenated $C_{1-6}$ alkoxy, $C_{1-6}$ alkylamino, $(C_{1-6}$ alkyl$)_2$ amino, 3-12 membered cycloalkyl, 3-12 membered heterocyclyl, aryl and 5-10 membered heteroaryl;
$R_3$ is selected from hydrogen, hydroxy, amino, carboxy, cyano, nitro, halogen, $C_{1-6}$ alkyl, $C_{1-6}$ alkoxy, $C_{1-6}$ alkylamino, $(C_{1-6}$ alkyl$)_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonyl, $C_{1-6}$ alkylsulfonyl, $C_{1-6}$ alkylthio , 3-12 membered cycloalkyl, 3-12 membered cycloalkenyl, 3-12 membered heterocyclyl, wherein the amino, $C_{1-6}$ alkyl, $C_{1-6}$ alkoxy, $C_{1-6}$ alkylamino, $(C_{1-6}$ alkyl$)_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonyl, $C_{1-6}$ alkylsulfonyl, $C_{1-6}$ alkylthio , 3-12 membered cycloalkyl, 3-12 membered cycloalkenyl, 3-12 membered heterocyclyl are unsubstituted or optionally substituted by one or more groups selected from hydroxyl, amino, carboxyl, cyano, nitro, halogen, $C_{1-6}$ alkyl, $C_{1-6}$ alkoxy, $C_{1-6}$ alkoxy$C_{1-6}$ alkoxy, $C_{1-6}$ alkylamino, $(C_{1-6}$ alkyl$)_2$ amino, $C_{1-6}$ alkylcarbonylamino and $C_{1-6}$ alkylsulfonylamino;
m is 0, 1, 2 or 3;
$R_4$, $R_5$ and $R_6$ are each independently selected from hydrogen, halogen, hydroxy, amino, carboxy, cyano, nitro,$C_{1-6}$ alkyl, $C_{2-8}$ alkenyl, $C_{2-8}$ alkynyl, and halogenated $C_{1-6}$ alkyl;
preferably, at least one of $X_1$ and $X_2$ is N.

**Some embodiments of the present invention relate to the compound shown in formula (III), or the pharmaceutically acceptable salt or the isomer thereof,**

$X_1$ is selected from N or $CR_4$;
$X_2$ is selected from N or $CR_5$;
A is selected from 3-12 membered cycloalkyl, 3-12 membered cycloalkenyl, 3-12 membered heterocyclyl, aryl, 5-10

membered heteroaryl, wherein the heteroatoms of the 3-12 membered heterocyclyl are selected from one of O, S, N or any combination thereof, C atom can be optionally oxidized to C(O), S atom can be optionally oxidized to S(O) or $S(O)_2$, the heteroatoms of the 5-10 membered heteroaryl are selected from one of O, S, and N or any combination thereof;

$R_1$ is selected from hydrogen, halogen, hydroxyl, carboxyl, amino, $C_{1-6}$ alkyl, $C_{1-6}$ alkoxy, $C_{1-6}$ alkylamino, $(C_{1-6}$ alkyl$)_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonylamino, $C_{1-6}$ alkylaminocarbonyl, $(C_{1-6}$ alkyl$)_2$ aminocarbonyl, $C_{1-6}$ alkoxycarbonyl, $C_{1-6}$ alkylcarbonyl, $C_{1-6}$ alkylsulfonyl, aminosulfonyl, $C_{1-6}$ alkylthio, 3-12 membered cycloalkyl, 3-12 membered heterocyclyl, 3-12 membered cycloalkenyl, wherein the heteroatoms of the 3-12 membered heterocyclyl are selected from one of O, S, N or any combination thereof, C atom can be optionally oxidized to C(O), S atom can be optionally oxidized to S(O) or $S(O)_2$, the heteroatoms of the 5-10 membered heteroaryl are selected from one of O, S, and N or any combination thereof, wherein the amino, $C_{1-6}$ alkyl, $C_{1-6}$ alkoxy, $C_{1-6}$ alkylamino, $(C_{1-6}$ alkyl$)_2$ amino, halogenated $C_{1-6}$ alkyl , halogenated $C_{1-6}$ alkoxy, $C_{2-8}$ alkenyl, $C_{2-8}$ alkynyl, $C_{1-6}$ alkylcarbonylamino, $C_{1-6}$ alkylaminocarbonyl, $(C_{1-6}$ alkyl$)_2$ aminocarbonyl, $C_{1-6}$ alkoxycarbonyl,$C_{1-6}$ alkylcarbonyl, $C_{1-6}$ alkylsulfonyl, aminosulfonyl, $C_{1-6}$ alkylthio, 3-12 membered cycloalkyl, 3-12 membered heterocyclyl, 3-12 membered cycloalkenyl are unsubstituted or optionally substituted by one or more groups selected from hydroxyl, amino, carboxy, cyano, nitro, halogen, $C_{1-6}$ alkyl, $C_{1-6}$ alkoxy, halogenated $C_{1-6}$ alkyl, halogenated $C_{1-6}$ alkoxy, $C_{1-6}$ alkylamino, $(C_{1-6}$ alkyl$)_2$ amino, $C_{1-6}$ alkylsulfonyl, 3-12 membered cycloalkyl, 3-12 membered heterocyclyl;

$R_2$ is selected from halogen, cyano, hydroxyl, carboxyl, amino, $C_{1-6}$ alkyl, $C_{1-6}$ alkoxy, $C_{1-6}$ alkylamino, $(C_{1-6}$ alkyl$)_2$ amino, 3-12 membered cycloalkyl, 3-12 membered heterocyclyl, 3-12 membered cycloalkenyl, wherein the amino, $C_{1-6}$ alkyl, $C_{1-6}$ alkoxy, $C_{1-6}$ alkylamino, $(C_{1-6}$ alkyl$)_2$ amino, 3-12 membered cycloalkyl, 3-12 membered heterocyclyl, 3-12 membered cycloalkenyl are unsubstituted or optionally substituted by one or more groups selected from halogen, cyano, hydroxyl, carboxy, amino, $C_{1-6}$ alkyl, $C_{1-6}$ alkoxy, halogenated $C_{1-6}$ alkyl, halogenated $C_{1-6}$ alkoxy, $C_{1-6}$ alkylamino, $(C_{1-6}$ alkyl$)_2$ amino, 3-12 membered cycloalkyl, 3-12 membered heterocyclyl, aryl and 5-10 membered heteroaryl;

$R_3$ is selected from hydrogen, hydroxy, amino, carboxy, cyano, nitro, halogen, $C_{1-6}$ alkyl, $C_{1-6}$ alkoxy, $C_{1-6}$ alkylamino, $(C_{1-6}$ alkyl$)_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonyl, $C_{1-6}$ alkylsulfonyl, $C_{1-6}$ alkylthio , 3-12 membered cycloalkyl, 3-12 membered cycloalkenyl, 3-12 membered heterocyclyl, wherein the amino, $C_{1-6}$ alkyl, $C_{1-6}$ alkoxy, $C_{1-6}$ alkylamino, $(C_{1-6}$ alkyl$)_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonyl, $C_{1-6}$ alkylsulfonyl, $C_{1-6}$ alkylthio , 3-12 membered cycloalkyl, 3-12 membered cycloalkenyl, 3-12 membered heterocyclyl are unsubstituted or optionally substituted by one or more groups selected from hydroxyl, amino, carboxyl, cyano, nitro, halogen, $C_{1-6}$ alkyl, $C_{1-6}$ alkoxy, $C_{1-6}$ alkoxy$C_{1-6}$ alkoxy, $C_{1-6}$ alkylamino, $(C_{1-6}$ alkyl$)_2$ amino, $C_{1-6}$ alkylcarbonylamino and $C_{1-6}$ alkylsulfonylamino;

m is 0, 1, 2 or 3;

$R_4$, $R_5$ and $R_6$ are each independently selected from hydrogen, halogen, hydroxy, amino, carboxy, cyano, nitro,$C_{1-6}$ alkyl, $C_{2-8}$ alkenyl, $C_{2-8}$ alkynyl, and halogenated $C_{1-6}$ alkyl;

preferably, at least one of $X_1$ and $X_2$ is N.

**Some embodiments of the present invention relate to the compound shown in formula (IV), or the pharmaceutically acceptable salt or the isomer thereof, wherein L is-$(CH_2)$p-, p is 1, and n is 1:**

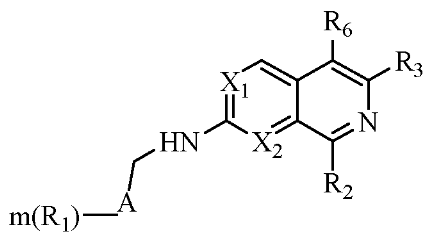

(IV)

$X_1$ is selected from N or $CR_4$;

$X_2$ is selected from N or $CR_5$;

A is selected from 3-12 membered cycloalkyl, 3-12 membered cycloalkenyl, 3-12 membered heterocyclyl, aryl, 5-10 membered heteroaryl, wherein the heteroatoms of the 3-12 membered heterocyclyl are selected from one of O, S, N or any combination thereof, C atom can be optionally oxidized to C(O), S atom can be optionally oxidized to S(O) or $S(O)_2$, the heteroatoms of the 5-10 membered heteroaryl are selected from one of O, S, and N or any combination thereof;

$R_1$ is selected from hydrogen, halogen, hydroxyl, carboxyl, amino, $C_{1-6}$ alkyl, $C_{1-6}$ alkoxy, $C_{1-6}$ alkylamino, $(C_{1-6}$

alkyl)$_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonylamino, $C_{1-6}$ alkylaminocarbonyl, ($C_{1-6}$ alkyl)$_2$ aminocarbonyl, $C_{1-6}$ alkoxycarbonyl, $C_{1-6}$ alkylcarbonyl, $C_{1-6}$ alkylsulfonyl, aminosulfonyl, $C_{1-6}$ alkylthio, 3-12 membered cycloalkyl, 3-12 membered heterocyclyl, 3-12 membered cycloalkenyl, wherein the heteroatoms of the 3-12 membered heterocyclyl are selected from one of O, S, N or any combination thereof, C atom can be optionally oxidized to C(O), S atom can be optionally oxidized to S(O) or $S(O)_2$, the heteroatoms of the 5-10 membered heteroaryl are selected from one of O, S, and N or any combination thereof, wherein the amino, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl , halogenated $C_{1-6}$ alkoxy, $C_{2-8}$ alkenyl, $C_{2-8}$ alkynyl, $C_{1-6}$ alkylcarbonylamino, $C_{1-6}$ alkylaminocarbonyl, ($C_{1-6}$ alkyl)$_2$ aminocarbonyl, $C_{1-6}$ alkoxycarbonyl,$C_{1-6}$ alkylcarbonyl, $C_{1-6}$ alkylsulfonyl, aminosulfonyl, $C_{1-6}$ alkylthio, 3-12 membered cycloalkyl, 3-12 membered heterocyclyl, 3-12 membered cycloalkenyl are unsubstituted or optionally substituted by one or more groups selected from hydroxyl, amino, carboxy, cyano, nitro, halogen, $C_{1-6}$ alkyl, $C_{1-6}$ alkoxy, halogenated $C_{1-6}$ alkyl, halogenated $C_{1-6}$ alkoxy, $C_{1-6}$ alkylamino, ($C_{1-6}$ alkyl)$_2$ amino, $C_{1-6}$ alkylsulfonyl, HS(O)(=NH)-, $C_{1-6}$ alkyl -S(O)(=NH)-, 3-12 membered cycloalkyl, 3-12 membered heterocyclyl;

$R_2$ is selected from halogen, cyano, hydroxyl, carboxyl, amino, $C_{1-6}$ alkyl, $C_{1-6}$ alkoxy, $C_{1-6}$ alkylamino, ($C_{1-6}$ alkyl)$_2$ amino, 3-12 membered cycloalkyl, 3-12 membered heterocyclyl, 3-12 membered cycloalkenyl, 3-12 membered heterocyclyloxy, 3-12 membered cycloalkyl amino, 3-12 membered heterocyclyl-$CH_2$-amino, wherein the amino, $C_{1-6}$ alkyl, $C_{1-6}$ alkoxy, $C_{1-6}$ alkylamino, ($C_{1-6}$ alkyl)$_2$ amino, 3-12 membered cycloalkyl, 3-12 membered heterocyclyl, 3-12 membered cycloalkenyl, 3-12 membered heterocyclyloxy, 3-12 membered cycloalkyl amino, 3-12 membered heterocyclyl-$CH_2$-amino are unsubstituted or optionally substituted by one or more groups selected from halogen, cyano, hydroxyl, carboxy, amino, $C_{1-6}$ alkyl, $C_{1-6}$ alkoxy, halogenated $C_{1-6}$ alkyl, halogenated $C_{1-6}$ alkoxy, $C_{1-6}$ alkylamino, ($C_{1-6}$ alkyl)$_2$ amino, 3-12 membered cycloalkyl, 3-12 membered heterocyclyl, aryl and 5-10 membered heteroaryl;

$R_3$ is selected from hydrogen, hydroxy, amino, carboxy, cyano, nitro, halogen, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonyl, $C_{1-6}$ alkylsulfonyl, $C_{1-6}$ alkylthio , 3-12 membered cycloalkyl, 3-12 membered cycloalkenyl, 3-12 membered heterocyclyl, wherein the amino, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonyl, $C_{1-6}$ alkylsulfonyl, $C_{1-6}$ alkylthio , 3-12 membered cycloalkyl, 3-12 membered cycloalkenyl, 3-12 membered heterocyclyl are unsubstituted or optionally substituted by one or more groups selected from hydroxyl, amino, carboxyl, cyano, nitro, halogen, $C_{1-6}$ alkyl, $C_{1-6}$ alkoxy, $C_{1-6}$ alkoxy$C_{1-6}$ alkoxy, $C_{1-6}$ alkylamino, ($C_{1-6}$ alkyl)$_2$ amino, $C_{1-6}$ alkylcarbonylamino and $C_{1-6}$ alkylsulfonylamino;

m is 0, 1, 2 or 3;

$R_4$, $R_5$ and $R_6$ are each independently selected from hydrogen, halogen, hydroxy, amino, carboxy, cyano, nitro, $C_{1-6}$ alkyl, $C_{2-8}$ alkenyl, $C_{2-8}$ alkynyl, and halogenated $C_{1-6}$ alkyl;

preferably, at least one of $X_1$ and $X_2$ is N.

**Some embodiments of the present invention relate to the compound shown in formula (IV), or the pharmaceutically acceptable salt or the isomer thereof,**

$X_1$ is selected from N or $CR_4$;

$X_2$ is selected from N or $CR_5$;

A is selected from 3-12 membered cycloalkyl, 3-12 membered cycloalkenyl, 3-12 membered heterocyclyl, aryl, 5-10 membered heteroaryl, wherein the heteroatoms of the 3-12 membered heterocyclyl are selected from one of O, S, N or any combination thereof, C atom can be optionally oxidized to C(O), S atom can be optionally oxidized to S(O) or $S(O)_2$, the heteroatoms of the 5-10 membered heteroaryl are selected from one of O, S, and N or any combination thereof;

$R_1$ is selected from hydrogen, halogen, hydroxyl, carboxyl, amino, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonylamino, $C_{1-6}$ alkylaminocarbonyl, ($C_{1-6}$ alkyl)$_2$ aminocarbonyl, $C_{1-6}$ alkoxycarbonyl, $C_{1-6}$ alkylcarbonyl, $C_{1-6}$ alkylsulfonyl, aminosulfonyl, $C_{1-6}$ alkylthio, 3-12 membered cycloalkyl, 3-12 membered heterocyclyl, 3-12 membered cycloalkenyl, wherein the heteroatoms of the 3-12 membered heterocyclyl are selected from one of O, S, N or any combination thereof, C atom can be optionally oxidized to C(O), S atom can be optionally oxidized to S(O) or $S(O)_2$, the heteroatoms of the 5-10 membered heteroaryl are selected from one of O, S, and N or any combination thereof, wherein the amino, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl , halogenated $C_{1-6}$ alkoxy, $C_{2-8}$ alkenyl, $C_{2-8}$ alkynyl, $C_{1-6}$ alkylcarbonylamino, $C_{1-6}$ alkylaminocarbonyl, ($C_{1-6}$ alkyl)$_2$ aminocarbonyl, $C_{1-6}$ alkoxycarbonyl,$C_{1-6}$ alkylcarbonyl, $C_{1-6}$ alkylsulfonyl, aminosulfonyl, $C_{1-6}$ alkylthio, 3-12 membered cycloalkyl, 3-12 membered heterocyclyl, 3-12 membered cycloalkenyl are unsubstituted or optionally substituted by one or more groups selected from hydroxyl, amino, carboxy, cyano, nitro, halogen, $C_{1-6}$ alkyl, $C_{1-6}$ alkoxy, halogenated $C_{1-6}$ alkyl, halogenated $C_{1-6}$ alkoxy, $C_{1-6}$ alkylamino, ($C_{1-6}$ alkyl)$_2$ amino, $C_{1-6}$ alkylsulfonyl, 3-12 membered cycloalkyl, 3-12

membered heterocyclyl;

$R_2$ is selected from halogen, cyano, hydroxyl, carboxyl, amino, $C_{1-6}$ alkyl, $C_{1-6}$ alkoxy, $C_{1-6}$ alkylamino, ($C_{1-6}$ alkyl)$_2$ amino, 3-12 membered cycloalkyl, 3-12 membered heterocyclyl, 3-12 membered cycloalkenyl, wherein the amino, $C_{1-6}$ alkyl, $C_{1-6}$ alkoxy, $C_{1-6}$ alkylamino, ($C_{1-6}$ alkyl)$_2$ amino, 3-12 membered cycloalkyl, 3-12 membered heterocyclyl, 3-12 membered cycloalkenyl are unsubstituted or optionally substituted by one or more groups selected from halogen, cyano, hydroxyl, carboxy, amino, $C_{1-6}$ alkyl, $C_{1-6}$ alkoxy, halogenated $C_{1-6}$ alkyl, halogenated $C_{1-6}$ alkoxy, $C_{1-6}$ alkylamino, ($C_{1-6}$ alkyl)$_2$ amino, 3-12 membered cycloalkyl, 3-12 membered heterocyclyl, aryl and 5-10 membered heteroaryl;

$R_3$ is selected from hydrogen, hydroxy, amino, carboxy, cyano, nitro, halogen, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonyl, $C_{1-6}$ alkylsulfonyl, $C_{1-6}$ alkylthio , 3-12 membered cycloalkyl, 3-12 membered cycloalkenyl, 3-12 membered heterocyclyl, wherein the amino, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonyl, $C_{1-6}$ alkylsulfonyl, $C_{1-6}$ alkylthio , 3-12 membered cycloalkyl, 3-12 membered cycloalkenyl, 3-12 membered heterocyclyl are unsubstituted or optionally substituted by one or more groups selected from hydroxyl, amino, carboxyl, cyano, nitro, halogen, $C_{1-6}$ alkyl, $C_{1-6}$ alkoxy, $C_{1-6}$ alkoxy$C_{1-6}$ alkoxy, $C_{1-6}$ alkylamino, ($C_{1-6}$ alkyl)$_2$ amino, $C_{1-6}$ alkylcarbonylamino and $C_{1-6}$ alkylsulfonylamino;

m is 0, 1, 2 or 3;

$R_4$, $R_5$ and $R_6$ are each independently selected from hydrogen, halogen, hydroxy, amino, carboxy, cyano, nitro, $C_{1-6}$ alkyl, $C_{2-8}$ alkenyl, $C_{2-8}$ alkynyl, and halogenated $C_{1-6}$ alkyl;

preferably, at least one of $X_1$ and $X_2$ is N.

**Some embodiments of the present invention relate to the compound shown in formula (I), or the pharmaceutically acceptable salt or the isomer thereof,**

A is selected from the group consisting of 3-8 membered cycloalkyl, 6-11 membered ortho-fused cycloalkyl, 6-11 membered bridged cycloalkyl, 7-12 membered spirocycloalkyl, 3-8 membered monocycloalkenyl, 7-11 membered spiro cycloalkenyl, 7-11 membered ortho-fused cycloalkenyl, 6-11 membered bridged cycloalkenyl, 3-8 membered heterocyclyl, 6-12 membered ortho-fused heterocyclyl, 6-12 membered spiro heterocyclyl, 6-12 membered bridged heterocyclyl, 5-10 membered heteroaryl, wherein the heteroatoms of 3-8 membered heterocyclyl, 6-12 membered ortho-fused heterocyclyl, 6-12 membered spiro heterocyclyl, 6-12 membered bridged heterocyclyl are selected from one of O, S, N or any combination thereof, the C atom can be optionally oxidized to C(O), and the S atom can be optionally oxidized to S(O) or $S(O)_2$, The heteroatoms of the 5-10 membered heteroaryl are selected from one of O, S, and N or any combination thereof.

**Some embodiments of the present invention relate to the compound shown in formula (I), or the pharmaceutically acceptable salt or the isomer thereof,**

A is selected from

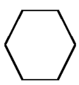、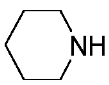、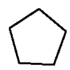、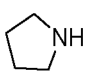、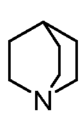, 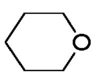, 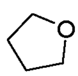, 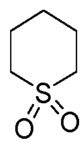,

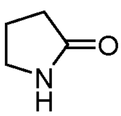, 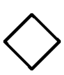, 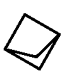, 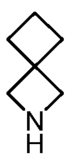, 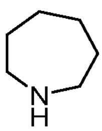, 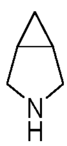, 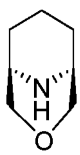, 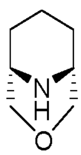, 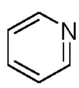, 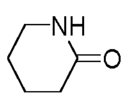,

or

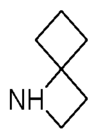

**Some embodiments of the present invention relate to the compound shown in formula (I), or the pharmaceutically acceptable salt or the isomer thereof,**

$X_1$ is N;
$X_2$ is $CR_5$;
L is selected from bond or -C(O)-;
A is selected from 3-12 membered heterocyclyl, 3-12 membered cycloalkyl, or 5-10 membered heteroaryl, wherein the heteroatoms of the 3-12 membered heterocyclyl and 5-10 membered heteroaryl are N;
$R_1$ is selected from hydrogen, hydroxyl, amino, $C_{1-6}$ alkyl, or $C_{1-6}$ alkylcarbonylamino,
wherein the amino, $C_{1-6}$ alkyl, or $C_{1-6}$ alkylcarbonylamino are unsubstituted or optionally substituted by one or more groups selected from HS(O)(=NH)-, $C_{1-6}$ alkyl-S(O)(=NH)-;
$R_2$ is selected from amino, $C_{1-6}$ alkylamino, ($C_{1-6}$ alkyl)$_2$ amino, $C_{1-6}$ alkoxy, 3-12 membered heterocyclyloxy, 3-12 membered heterocyclyl, or 3-12 membered cycloalkenyl, 3-12 membered cycloalkyl amino;
wherein the heteroatoms of the 3-12 membered heterocyclyl and 3-12 membered heterocyclyloxy are N, O, or any combination thereof,
wherein the amino, $C_{1-6}$ alkylamino, ($C_{1-6}$ alkyl)$_2$ amino, $C_{1-6}$ alkoxy, 3-12 membered heterocyclyloxy, 3-12 membered heterocyclyl, or 3-12 membered cycloalkenyl, 3-12 membered cycloalkyl amino are unsubstituted or optionally substituted by one or more groups selected from 3-12 membered cycloalkyl, 3-12 membered heterocyclyl, aryl, $C_{1-6}$ alkyl;
$R_3$ is selected from cyano, or halogenated $C_{1-6}$ alkyl;
$R_5$ and $R_6$ are each independently hydrogen;
n is 0 or 1;
m is 0 or 1.

**Some embodiments of the present invention relate to the compound shown in formula (I), or the pharmaceutically acceptable salt or the isomer thereof,**

$X_1$ is selected from N or $CR_4$;
$X_2$ is selected from N or $CR_5$;
L is selected from bond, -C(O)-, -($CH_2$)p-;
A is bond;
$R_1$ is selected from hydrogen, halogen, hydroxyl, carboxyl, amino, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonylamino, $C_{1-6}$ alkylaminocarbonyl, ($C_{1-6}$ alkyl)$_2$ aminocarbonyl, $C_{1-6}$ alkoxycarbonyl, $C_{1-6}$ alkylcarbonyl, $C_{1-6}$ alkylsulfonyl, aminosulfonyl, $C_{1-6}$ alkylthio, 3-12 membered cycloalkyl, 3-12 membered heterocyclyl, 3-12 membered cycloalkenyl, wherein the heteroatoms of the 3-12 membered heterocyclyl are selected from one of O, S, N or any combination thereof, C atom can be optionally oxidized to C(O), S atom can be optionally oxidized to S(O) or $S(O)_2$, the heteroatoms of the 5-10 membered heteroaryl are selected from one of O, S, and N or any combination thereof, wherein the amino, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl , halogenated $C_{1-6}$ alkoxy, $C_{2-8}$ alkenyl, $C_{2-8}$ alkynyl, $C_{1-6}$ alkylcarbonylamino, $C_{1-6}$ alkylaminocarbonyl, ($C_{1-6}$ alkyl)$_2$ aminocarbonyl, $C_{1-6}$ alkoxycarbonyl,$C_{1-6}$ alkylcarbonyl, $C_{1-6}$ alkylsulfonyl, aminosulfonyl, $C_{1-6}$ alkylthio, 3-12 membered cycloalkyl, 3-12 membered heterocyclyl, 3-12 membered cycloalkenyl are unsubstituted or optionally substituted by one or more groups selected from hydroxyl, amino, carboxy, cyano, nitro, halogen, $C_{1-6}$ alkyl, $C_{1-6}$ alkoxy, halogenated $C_{1-6}$ alkyl, halogenated $C_{1-6}$ alkoxy, $C_{1-6}$ alkylamino, ($C_{1-6}$ alkyl)$_2$ amino, $C_{1-6}$ alkylsulfonyl, 3-12 membered cycloalkyl, 3-12 membered heterocyclyl;
$R_2$ is selected from halogen, cyano, hydroxyl, carboxyl, amino, $C_{1-6}$ alkyl, $C_{1-6}$ alkoxy, $C_{1-6}$ alkylamino, ($C_{1-6}$ alkyl)$_2$ amino, 3-12 membered cycloalkyl, 3-12 membered heterocyclyl, 3-12 membered cycloalkenyl, wherein the amino, $C_{1-6}$ alkyl, $C_{1-6}$ alkoxy, $C_{1-6}$ alkylamino, ($C_{1-6}$ alkyl)$_2$ amino, 3-12 membered cycloalkyl, 3-12 membered heterocyclyl, 3-12 membered cycloalkenyl are unsubstituted or optionally substituted by one or more groups selected from halogen, cyano, hydroxyl, carboxy, amino, $C_{1-6}$ alkyl, $C_{1-6}$ alkoxy, halogenated $C_{1-6}$ alkyl, halogenated $C_{1-6}$ alkoxy, $C_{1-6}$ alkylamino, ($C_{1-6}$ alkyl)$_2$ amino, 3-12 membered cycloalkyl, 3-12 membered heterocyclyl, aryl and 5-10 membered heteroaryl;
$R_3$ is selected from hydrogen, hydroxy, amino, carboxy, cyano, nitro, halogen, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonyl, $C_{1-6}$ alkylsulfonyl, $C_{1-6}$ alkylthio , 3-12 membered cycloalkyl, 3-12 membered cycloalkenyl, 3-12 membered heterocyclyl, wherein the amino, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonyl, $C_{1-6}$ alkylsulfonyl, $C_{1-6}$ alkylthio , 3-12 membered cycloalkyl, 3-12 membered cycloalkenyl, 3-12 membered heterocyclyl are unsubstituted or optionally substituted by one or more groups selected from hydroxyl, amino, carboxyl, cyano, nitro, halogen, $C_{1-6}$ alkyl, $C_{1-6}$ alkoxy, $C_{1-6}$ alkoxy$C_{1-6}$ alkoxy, $C_{1-6}$ alkylamino, ($C_{1-6}$ alkyl)$_2$ amino, $C_{1-6}$ alkylcarbonylamino and $C_{1-6}$ alkylsulfonylamino;
n is 0 or 1;

m is 0, 1, 2 or 3;
p is 1, 2 or 3;
$R_4$, $R_5$ and $R_6$ are each independently selected from hydrogen, halogen, hydroxy, amino, carboxy, cyano, nitro, $C_{1-6}$ alkyl, $C_{2-8}$ alkenyl, $C_{2-8}$ alkynyl, and halogenated $C_{1-6}$ alkyl.

**Some embodiments of the present invention relate to the compound shown in formula (I), or the pharmaceutically acceptable salt or the isomer thereof,**

$X_1$ is N;
$X_2$ is N;
L is bond;
A is selected from 3-12 membered cycloalkyl, or 3-12 membered heterocyclyl, wherein the heteroatoms of the 3-12 membered heterocyclyl are N,
$R_1$ is selected from hydrogen, hydroxyl, $C_{1-6}$ alkyl, $C_{1-6}$ alkoxy, amino, $(C_{1-6}$ alkyl$)_2$ amino, $C_{1-6}$ alkylcarbonyl, or 3-12 membered heterocyclyl,
wherein the heteroatoms of the 3-12 membered heterocyclyl are selected from any one of O, N or any combination thereof,
wherein the $C_{1-6}$ alkyl, $C_{1-6}$ alkoxy, amino, $(C_{1-6}$ alkyl$)_2$ amino, $C_{1-6}$ alkylcarbonyl and 3-12 membered heterocyclyl are optionally substituted by any one or more groups selected from hydroxyl, amino, $C_{1-6}$ alkyl, $(C_{1-6}$ alkyl$)_2$ aminocarbonyl or $C_{1-6}$ alkyl carbonyl;
$R_2$ is selected from amino, $C_{1-6}$ alkylamino, 3-12 membered heterocyclyl, or 3-12 membered cycloalkyl amino,
wherein the heteroatoms of the 3-12 membered heterocyclyl are selected from any one of O, N, any combination thereof,
wherein the amino, $C_{1-6}$ alkylamino, 3-12 membered heterocyclyl, or 3-12 membered cycloalkyl amino are optionally substituted by any one or more groups selected from $C_{1-6}$ alkyl, $C_{1-6}$ alkoxy, 3-12membered cycloalkyl, or 3-12 membered heterocyclyl; $R_3$ is selected from cyano, $C_{1-6}$ alkyl, aminocarbonyl, hydrogen, hydroxy, amino, halogen, or $C_{1-6}$ alkoxy,
wherein the $C_{1-6}$ alkyl and aminocarbonyl are optionally substituted by any one or more halogen;
$R_6$ is hydrogen;
n is 0;
m is 0, 1, 2 or 3;
p is 1, 2 or 3.

**Some embodiments of the present invention relate to the compound shown in formula (I), or the pharmaceutically acceptable salt or the isomer thereof,**

$X_1$ is N;
$X_2$ is $CR_5$;
L is bond;
n is 0;
A is selected from 3-12 membered cycloalkyl, or 3-12 membered heterocyclyl; wherein the heteroatoms of the 3-12 membered heterocyclyl is N; the 3-12 membered cycloalkyl is substituted by amino;
$R_1$ is hydrogen;
$R_2$ is selected from amino, $C_{1-6}$ alkoxy or 3-12 membered heterocyclyloxy, wherein the amino is substituted by 3-12 membered heterocyclyl;
$R_3$ is selected from cyano or halogenated $C_{1-6}$ alkyl;
$R_5$ and $R_6$ are each independently hydrogen; and
m is 1.

**Some embodiments of the present invention relate to the compound shown in formula (I), or the pharmaceutically acceptable salt or the isomer thereof,**

$X_1$ is selected from N or $CR_4$;
$X_2$ is selected from N or $CR_5$;
L is selected from bond, -C(O)-, $-(CH_2)p-$;
A is selected from bond, 3-12 membered cycloalkyl, 3-12 membered cycloalkenyl, 3-12 membered heterocyclyl, aryl, 5-10 membered heteroaryl, wherein the heteroatoms of the 3-12 membered heterocyclyl are selected from one of O, S, N or any combination thereof, C atom can be optionally oxidized to C(O), S atom can be optionally

oxidized to S(O) or $S(O)_2$, the heteroatoms of the 5-10 membered heteroaryl are selected from one of O, S, and N or any combination thereof;

$R_1$ is selected from hydrogen, halogen, hydroxyl, carboxyl, amino, $C_{1-6}$ alkyl, $C_{1-6}$ alkoxy, $C_{1-6}$ alkylamino, $(C_{1-6}$ alkyl$)_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonylamino, $C_{1-6}$ alkylaminocarbonyl, $(C_{1-6}$ alkyl$)_2$ aminocarbonyl, $C_{1-6}$ alkoxycarbonyl, $C_{1-6}$ alkylcarbonyl, $C_{1-6}$ alkylsulfonyl, aminosulfonyl, $C_{1-6}$ alkylthio, 3-12 membered cycloalkyl, 3-12 membered heterocyclyl, 3-12 membered cycloalkenyl, wherein the heteroatoms of the 3-12 membered heterocyclyl are selected from one of O, S, N or any combination thereof, C atom can be optionally oxidized to C(O), S atom can be optionally oxidized to S(O) or $S(O)_2$, the heteroatoms of the 5-10 membered heteroaryl are selected from one of O, S, and N or any combination thereof,

wherein the amino, $C_{1-6}$ alkyl, $C_{1-6}$ alkoxy, $C_{1-6}$ alkylamino, $(C_{1-6}$ alkyl$)_2$ amino, halogenated $C_{1-6}$ alkyl , halogenated $C_{1-6}$ alkoxy, $C_{2-8}$ alkenyl, $C_{2-8}$ alkynyl, $C_{1-6}$ alkylcarbonylamino, $C_{1-6}$ alkylaminocarbonyl, $(C_{1-6}$ alkyl$)_2$ aminocarbonyl, $C_{1-6}$ alkoxycarbonyl,$C_{1-6}$ alkylcarbonyl, $C_{1-6}$ alkylsulfonyl, aminosulfonyl, $C_{1-6}$ alkylthio, 3-12 membered cycloalkyl, 3-12 membered heterocyclyl, 3-12 membered cycloalkenyl are unsubstituted or optionally substituted by one or more groups selected from hydroxyl, amino, carboxy, cyano, nitro, halogen, $C_{1-6}$ alkyl, $C_{1-6}$ alkoxy, halogenated $C_{1-6}$ alkyl, halogenated $C_{1-6}$ alkoxy, $C_{1-6}$ alkylamino, $(C_{1-6}$ alkyl$)_2$ amino, $C_{1-6}$ alkylsulfonyl, HS(O)(=NH)-, $C_{1-6}$ alkyl-S(O)(=NH)-, $(C_{1-6}$ alkyl$)_2$ aminocarbonyl, $C_{1-6}$ alkyl carbonyl, 3-12 membered cycloalkyl, 3-12 membered heterocyclyl;

$R_2$ is selected from halogen, cyano, hydroxyl, carboxyl, amino, $C_{1-6}$ alkyl, $C_{1-6}$ alkoxy, $C_{1-6}$ alkylamino, $(C_{1-6}$ alkyl$)_2$ amino, 3-12 membered cycloalkyl, 3-12 membered heterocyclyl, 3-12 membered cycloalkenyl, 3-12 membered heterocyclyloxy, 3-12 membered cycloalkyl amino, 3-12 membered heterocyclyl-$CH_2$-amino, wherein the amino, $C_{1-6}$ alkyl, $C_{1-6}$ alkoxy, $C_{1-6}$ alkylamino, $(C_{1-6}$ alkyl$)_2$ amino, 3-12 membered cycloalkyl, 3-12 membered heterocyclyl, 3-12 membered cycloalkenyl, 3-12 membered heterocyclyloxy, 3-12 membered cycloalkyl amino, 3-12 membered heterocyclyl-$CH_2$-amino are unsubstituted or optionally substituted by one or more groups selected from halogen, cyano, hydroxyl, carboxy, amino, $C_{1-6}$ alkyl, $C_{1-6}$ alkoxy, halogenated $C_{1-6}$ alkyl, halogenated $C_{1-6}$ alkoxy, $C_{1-6}$ alkylamino, $(C_{1-6}$ alkyl$)_2$ amino, 3-12 membered cycloalkyl, 3-12 membered heterocyclyl, aryl and 5-10 membered heteroaryl;

$R_3$ is selected from hydrogen, hydroxy, amino, carboxy, cyano, nitro, halogen, $C_{1-6}$ alkyl, $C_{1-6}$ alkoxy, $C_{1-6}$ alkylamino, $(C_{1-6}$ alkyl$)_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonyl, $C_{1-6}$ alkylsulfonyl, $C_{1-6}$ alkylthio , 3-12 membered cycloalkyl, 3-12 membered cycloalkenyl, 3-12 membered heterocyclyl, wherein the amino, $C_{1-6}$ alkyl, $C_{1-6}$ alkoxy, $C_{1-6}$ alkylamino, $(C_{1-6}$ alkyl$)_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonyl, $C_{1-6}$ alkylsulfonyl, $C_{1-6}$ alkylthio, 3-12 membered cycloalkyl, 3-12 membered cycloalkenyl, 3-12 membered heterocyclyl are unsubstituted or optionally substituted by one or more groups selected from hydroxyl, amino, carboxyl, cyano, nitro, halogen, $C_{1-6}$ alkyl, $C_{1-6}$ alkoxy, $C_{1-6}$ alkoxy$C_{1-6}$ alkoxy, $C_{1-6}$ alkylamino, $(C_{1-6}$ alkyl$)_2$ amino, $C_{1-6}$ alkylcarbonylamino and $C_{1-6}$ alkylsulfonylamino;

n is 0 or 1;

m is 0, 1, 2 or 3;

p is 1, 2 or 3;

$R_4$, $R_5$ and $R_6$ are each independently selected from hydrogen, halogen, hydroxy, amino, carboxy, cyano, nitro, $C_{1-6}$ alkyl, $C_{2-8}$ alkenyl, $C_{2-8}$ alkynyl, and halogenated $C_{1-6}$ alkyl.

The present invention also provides a pharmaceutical composition comprising a compound shown in formula (I), (II), (III)or (IV), or a pharmaceutically acceptable salt or isomer thereof, and one or more second therapeutically active agents.

The present invention also provides a pharmaceutical formulation comprising a compound shown in formula (I), (II), (III)or (IV), or a pharmaceutically acceptable salt or isomer thereof, and one or more pharmaceutically acceptable carriers.

The present invention also provides a pharmaceutical formulation having cyclin-dependent kinase 9 inhibitory activity, comprising a compound shown in formula (I), (II), (III)or (IV), or pharmaceutically acceptable salt or isomer thereof, and one or more pharmaceutically acceptable carriers.

The present invention also provides use of the compound shown in formula(I), (II), (III)or (IV), or a pharmaceutically acceptable salt or isomer, the pharmaceutical composition or the pharmaceutical formulation in the manufacture of a medicament for treating or preventing a CDK9-mediated related disease.

In particular, the CDK9-mediated related diseases are cancer, preferably, the cancer is solid tumor or hematological malignancies, more preferably, the cancer is selected from adrenal tumor, melanoma, head and neck cancer, kidney cancer, bladder cancer, prostate cancer, endometrial carcinoma, cervical cancer, gastric carcinoma, colon cancer, pancreatic cancer, rectal cancer, esophageal cancer, liver cancer, lung cancer, sarcoma, breast cancer ovarian cancer, non hodgkin's lymphoma, acute myeloid leukemia, acute lymphoblastic leukemia or myeloma.

**Some embodiments of the present invention relate to the compound shown in formula (I), (II), or phar-**

**maceutically acceptable salts or isomer thereof,**

A is selected from 3-12 membered cycloalkyl, or 3-12 membered heterocyclyl,
wherein the heteroatoms of the 3-12 membered heterocyclyl are N.

**Some embodiments of the present invention relate to the compound shown in formula (I), (II), or pharmaceutically acceptable salts or isomer thereof,**

A is selected from 3-8 membered cycloalkyl, 3-8 membered heterocyclyl, 6-12 membered ortho-fused heterocyclyl, or 6-12 membered spiro heterocyclyl,
wherein the heteroatoms of the 3-12 membered heterocyclyl, 6-12 membered ortho-fused heterocyclyl and 6-12 membered spiro heterocyclyl are N.

**Some embodiments of the present invention relate to the compound shown in formula (I), (II), or pharmaceutically acceptable salts or isomer thereof,**
A is selected from

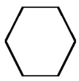, 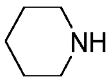, 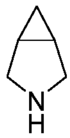, 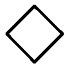, 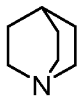, 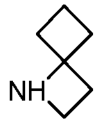, or 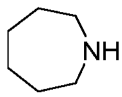.

**Some embodiments of the present invention relate to the compound shown in formula (I), (II), or pharmaceutically acceptable salts or isomer thereof,**

$R_1$ is selected from hydrogen, hydroxyl, $C_{1-6}$ alkyl, $C_{1-6}$ alkoxy, amino, $(C_{1-6}$ alkyl$)_2$ amino, $C_{1-6}$ alkylcarbonyl, or 3-12 membered heterocyclyl,
wherein the heteroatoms of the 3-12 membered heterocyclyl are selected from any one of O, N or any combination thereof,
wherein the $C_{1-6}$ alkyl, $C_{1-6}$ alkoxy, amino, $(C_{1-6}$ alkyl$)_2$ amino, $C_{1-6}$ alkylcarbonyl and 3-12 membered heterocyclyl are optionally substituted by any one or more groups selected from hydroxyl, amino, $C_{1-6}$ alkyl, $(C_{1-6}$ alkyl$)_2$ aminocarbonyl, or $C_{1-6}$ alkyl carbonyl.

**Some embodiments of the present invention relate to the compound shown in formula (I), (II), or pharmaceutically acceptable salts or isomer thereof,**

$R_2$ is selected from amino, $C_{1-6}$ alkylamino, 3-12 membered heterocyclyl, or 3-12 membered cycloalkyl amino,
wherein the heteroatoms of the 3-12 membered heterocyclyl are selected from any one of O, N or any combination thereof,
wherein the amino, $C_{1-6}$ alkylamino, 3-12 membered heterocyclyl and 3-12 membered cycloalkyl amino are optionally substituted by any one or more groups selected from $C_{1-6}$ alkyl, $C_{1-6}$ alkoxy, 3-12 membered cycloalkyl, or 3-12 membered heterocyclyl.

**Some embodiments of the present invention relate to the compound shown in formula (I), (II), or pharmaceutically acceptable salts or isomer thereof,**

$R_3$ is selected from cyano, $C_{1-6}$ alkyl, aminocarbonyl, hydrogen, hydroxy, amino, halogen, or $C_{1-6}$ alkoxy,
wherein the $C_{1-6}$ alkyl and aminocarbonyl are optionally substituted by any one or more halogen.

**Some embodiments of the present invention relate to the compound shown in formula (I), (II), or pharmaceutically acceptable salts or isomer thereof,**
$R_6$ is hydrogen.
**Some embodiments of the present invention relate to the compound shown in formula (I), or pharmaceutically acceptable salts or isomer thereof,**

when L is bond, n is 0, A is selected from 3-12 membered cycloalkyl, or 3-12 membered heterocyclyl, wherein the heteroatoms of the 3-12 membered heterocyclyl are N,

$R_1$ and $R_3$ are selected from the following (i) or (ii),

(i) when $R_1$ is selected from $C_{1-6}$ alkyl, amino or ($C_{1-6}$ alkyl)$_2$ amino, $R_3$ is selected from cyano, aminocarbonyl, hydroxy or amino,
wherein the $C_{1-6}$ alkyl, amino and ($C_{1-6}$ alkyl)$_2$ amino in $R_1$ are optionally substituted by any one or more groups selected from hydroxyl, amino, $C_{1-6}$ alkyl, or ($C_{1-6}$ alkyl)$_2$ aminocarbonyl or $C_{1-6}$ alkyl carbonyl, wherein the aminocarbonyl and amino in $R_3$ are optionally substituted by any one or more groups selected from halogen,
(ii) when $R_1$ is selected from hydrogen, hydroxyl, $C_{1-6}$ alkoxy, $C_{1-6}$ alkylcarbonyl, 3-12 membered heterocyclyl, halogen, carboxyl, or 3-12 membered cycloalkyl, $R_3$ is selected from cyano, $C_{1-6}$ alkyl, aminocarbonyl, hydrogen, hydroxy, amino, halogen, or $C_{1-6}$ alkoxy,

wherein the heteroatoms of the 3-12 membered heterocyclyl are selected from any one of O, N or any combination thereof;
wherein the $C_{1-6}$ alkoxy, $C_{1-6}$ alkylcarbonyl, 3-12 membered heterocyclyl and 3-12 membered cycloalkyl in $R_1$ are optionally substituted by any one or more groups selected from hydroxyl, amino, $C_{1-6}$ alkyl, or ($C_{1-6}$ alkyl)$_2$ aminocarbonyl or $C_{1-6}$ alkyl carbonyl, wherein the $C_{1-6}$ alkyl, aminocarbonyl, amino and $C_{1-6}$ alkoxy in $R_3$ are optionally substituted by any one or more groups selected from halogen,
and
when L is bond or $-(CH_2)p-$, A is bond, and $R_1$ is selected from 3-12 membered cycloalkyl, or 3-12 membered heterocyclyl, wherein the heteroatoms of the 3-12 membered heterocyclyl are N, and wherein $R_1$ is not substituted with amino, $C_{1-6}$ alkyl, halogenated $C_{1-6}$ alkyl, $C_{1-6}$ alkylamino, ($C_{1-6}$ alkyl)$_2$ amino or $C_{1-6}$ alkylsulfonyl.

**Some embodiments of the present invention relate to the compound shown in formula (I), or pharmaceutically acceptable salts or isomer thereof,**

$R_2$ is selected from $C_{1-6}$ alkylamino or ($C_{1-6}$ alkyl)$_2$ amino; and
$R_3$ is cyano.

**Some embodiments of the present invention relate to the compound shown in formula (I), or pharmaceutically acceptable salts or isomer thereof,**

L is bond;
n is 0;
A is 3-12 membered cycloalkyl;
$R_1$ is amino;
$R_2$ is selected from $C_{1-6}$ alkylamino or ($C_{1-6}$ alkyl)$_2$ amino;
$R_3$ is cyano;
$R_6$ is hydrogen; and
m is 1.

**The present invention provides the compound represented by formula (I), its pharmaceutically acceptable salt or isomer:**
provided that

a case in which $X_1$ is N, $X_2$ is N, n is 0, A is piperidinyl, $R_1$ is $C_{1-6}$ alkylsulfonyl, aminosulfonyl or $C_{1-6}$ alkyl aminosulfonyl and m is 1, and,
a case in which $X_1$ is N, $X_2$ is N, n is 0, A is bond, $R_1$ is piperidinyl, and m is 1, and wherein the piperidinyl is substituted by $C_{1-6}$ alkylsulfonyl, are excluded.

**Some embodiments of the present invention relate to the compound shown in formula (I), or pharmaceutically acceptable salts or stereoisomers thereof,**

$X_1$ is selected from N or $CR_4$;
$X_2$ is selected from N or $CR_5$;
L is selected from bond, -C(O)-, $-(CH_2)p-$;
A is bond;
$R_1$ is selected from hydrogen, halogen, hydroxyl, carboxyl, amino, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl , halogenated $C_{1-6}$ alkoxy, $C_{2-8}$ alkenyl, $C_{2-8}$ alkynyl, $C_{1-6}$ alkylcarbonylamino,

$C_{1-6}$ alkylaminocarbonyl, ($C_{1-6}$ alkyl)$_2$ aminocarbonyl, $C_{1-6}$ alkoxycarbonyl, $C_{1-6}$ alkylcarbonyl, $C_{1-6}$ alkylsulfonyl, aminosulfonyl, $C_{1-6}$ alkylthio, 3-12 membered cycloalkyl, 3-12 membered heterocyclyl, 3-12 membered cycloalkenyl, wherein the heteroatoms of the 3-12 membered heterocyclyl are selected from one of O, S, N or any combination thereof, C atom can be optionally oxidized to C(O), S atom can be optionally oxidized to S(O) or $S(O)_2$, the heteroatoms of the 5-10 membered heteroaryl are selected from one of O, S, and N or any combination thereof, wherein the amino, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl , halogenated $C_{1-6}$ alkoxy, $C_{2-8}$ alkenyl, $C_{2-8}$ alkynyl, $C_{1-6}$ alkylcarbonylamino, $C_{1-6}$ alkylaminocarbonyl, ($C_{1-6}$ alkyl)$_2$ aminocarbonyl, $C_{1-6}$ alkoxycarbonyl,$C_{1-6}$ alkylcarbonyl, $C_{1-6}$ alkylsulfonyl, aminosulfonyl, $C_{1-6}$ alkylthio, 3-12 membered cycloalkyl, 3-12 membered heterocyclyl, 3-12 membered cycloalkenyl are unsubstituted or optionally substituted by one or more groups selected from hydroxyl, amino, carboxy, cyano, nitro, halogen, $C_{1-6}$ alkyl, $C_{1-6}$ alkoxy, halogenated $C_{1-6}$ alkyl, halogenated $C_{1-6}$ alkoxy, $C_{1-6}$ alkylamino, ($C_{1-6}$ alkyl)$_2$ amino, $C_{1-6}$ alkylsulfonyl, HS(O)(=NH)-, $C_{1-6}$ alkyl-S(O)(=NH)-, 3-12 membered cycloalkyl, 3-12 membered heterocyclyl;

$R_2$ is selected from halogen, cyano, hydroxyl, carboxyl, amino, $C_{1-6}$ alkyl, $C_{1-6}$ alkoxy, $C_{1-6}$ alkylamino, ($C_{1-6}$ alkyl)$_2$ amino, 3-12 membered cycloalkyl, 3-12 membered heterocyclyl, 3-12 membered cycloalkenyl, 3-12 membered heterocyclyloxy, 3-12 membered cycloalkyl amino, 3-12 membered heterocyclyl-$CH_2$-amino wherein the amino, $C_{1-6}$ alkyl, $C_{1-6}$ alkoxy, $C_{1-6}$ alkylamino, ($C_{1-6}$ alkyl)$_2$ amino, 3-12 membered cycloalkyl, 3-12 membered heterocyclyl, 3-12 membered cycloalkenyl, 3-12 membered heterocyclyloxy, 3-12 membered cycloalkyl amino, 3-12 membered heterocyclyl-$CH_2$-amino are unsubstituted or optionally substituted by one or more groups selected from halogen, cyano, hydroxyl, carboxy, amino, $C_{1-6}$ alkyl, $C_{1-6}$ alkoxy, halogenated $C_{1-6}$ alkyl, halogenated $C_{1-6}$ alkoxy, $C_{1-6}$ alkylamino, ($C_{1-6}$ alkyl)$_2$ amino, 3-12 membered cycloalkyl, 3-12 membered heterocyclyl, aryl and 5-10 membered heteroaryl;

$R_3$ is selected from hydrogen, hydroxy, amino, carboxy, cyano, nitro, halogen, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonyl, $C_{1-6}$ alkylsulfonyl, $C_{1-6}$ alkylthio , 3-12 membered cycloalkyl, 3-12 membered cycloalkenyl, 3-12 membered heterocyclyl, wherein the amino, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonyl, $C_{1-6}$ alkylsulfonyl, $C_{1-6}$ alkylthio , 3-12 membered cycloalkyl, 3-12 membered cycloalkenyl, 3-12 membered heterocyclyl are unsubstituted or optionally substituted by one or more groups selected from hydroxyl, amino, carboxyl, cyano, nitro, halogen, $C_{1-6}$ alkyl, $C_{1-6}$ alkoxy, $C_{1-6}$ alkoxy$C_{1-6}$ alkoxy, $C_{1-6}$ alkylamino, ($C_{1-6}$ alkyl)$_2$ amino, $C_{1-6}$ alkylcarbonylamino and $C_{1-6}$ alkylsulfonylamino;

n is 0 or 1;

m is 0, 1, 2 or 3;

p is 1, 2 or 3;

$R_4$, $R_5$ and $R_6$ are each independently selected from hydrogen, halogen, hydroxy, amino, carboxy, cyano, nitro, $C_{1-6}$ alkyl, $C_{2-8}$ alkenyl, $C_{2-8}$ alkynyl, and halogenated $C_{1-6}$ alkyl.

**Some embodiments of the present invention relate to the compound shown in formula (I), or pharmaceutically acceptable salts or stereoisomers thereof,**

$R_1$ is selected from hydrogen, halogen, hydroxyl, carboxyl, amino, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl , halogenated $C_{1-6}$ alkoxy, $C_{2-8}$ alkenyl, $C_{2-8}$ alkynyl, $C_{1-6}$ alkylcarbonylamino, $C_{1-6}$ alkylaminocarbonyl, ($C_{1-6}$ alkyl)$_2$ aminocarbonyl, $C_{1-6}$ alkoxycarbonyl, $C_{1-6}$ alkylcarbonyl, $C_{1-6}$ alkylsulfonyl, aminosulfonyl, $C_{1-6}$ alkylthio, 3-12 membered cycloalkyl, 3-12 membered heterocyclyl, 3-12 membered cycloalkenyl, wherein the heteroatoms of the 3-12 membered heterocyclyl are selected from one of O, S, N or any combination thereof, C atom can be optionally oxidized to C(O), S atom can be optionally oxidized to S(O) or $S(O)_2$, the heteroatoms of the 5-10 membered heteroaryl are selected from one of O, S, and N or any combination thereof, wherein the amino, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl , halogenated $C_{1-6}$ alkoxy, $C_{2-8}$ alkenyl, $C_{2-8}$ alkynyl, $C_{1-6}$ alkylcarbonylamino, $C_{1-6}$ alkylaminocarbonyl, ($C_{1-6}$ alkyl)$_2$ aminocarbonyl, $C_{1-6}$ alkoxycarbonyl,$C_{1-6}$ alkylcarbonyl, $C_{1-6}$ alkylsulfonyl, aminosulfonyl, $C_{1-6}$ alkylthio, 3-12 membered cycloalkyl, 3-12 membered heterocyclyl, 3-12 membered cycloalkenyl are unsubstituted or optionally substituted by one or more groups selected from hydroxyl, amino, carboxy, cyano, nitro, halogen, $C_{1-6}$ alkyl, $C_{1-6}$ alkoxy, halogenated $C_{1-6}$ alkyl, halogenated $C_{1-6}$ alkoxy, $C_{1-6}$ alkylamino, $C_{1-6}$ alkylsulfonyl, HS(O)(=NH)-, $C_{1-6}$ alkyl-S(O)(=NH)-, 3-12 membered cycloalkyl, 3-12 membered heterocyclyl;

**Some embodiments of the present invention relate to the compound shown in formula (I), or pharmaceutically acceptable salts or stereoisomers thereof,**

$R_3$ is selected from hydrogen, hydroxy, amino, carboxy, cyano, nitro, halogen, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonyl, $C_{1-6}$ alkylsulfonyl, $C_{1-6}$ alkylthio , 3-12 membered cycloalkyl, 3-12 membered cycloalkenyl, 3-12 membered heterocyclyl, wherein the amino, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonyl, $C_{1-6}$ alkylsulfonyl, $C_{1-6}$ alkylthio , 3-12 membered cycloalkyl, 3-12 membered cycloalkenyl, 3-12 membered heterocyclyl are unsubstituted or optionally substituted by one or more

groups selected from amino, carboxyl, cyano, nitro, halogen, $C_{1-6}$ alkyl, $C_{1-6}$ alkoxy, $C_{1-6}$ alkoxy$C_{1-6}$ alkoxy, $C_{1-6}$ alkylamino, $(C_{1-6}$ alkyl$)_2$ amino, $C_{1-6}$ alkylcarbonylamino and $C_{1-6}$ alkylsulfonylamino.

**Some embodiments of the present invention relate to the compound shown in formula (I), or pharmaceutically acceptable salts or stereoisomers thereof,**

$X_1$ is selected from N or $CR_4$;
$X_2$ is selected from N or $CR_5$;
L is selected from bond, -C(O)-, $-(CH_2)p-$;
A is selected from bond, 3-12 membered cycloalkyl, 3-12 membered heterocyclyl, wherein the heteroatoms of the 3-12 membered heterocyclyl is N;
$R_1$ is selected from hydrogen, halogen, hydroxyl, carboxyl, amino, $C_{1-6}$ alkyl, $C_{1-6}$ alkylamino, $C_{1-6}$ alkylcarbonylamino, $C_{1-6}$ alkylaminocarbonyl, $C_{1-6}$ alkylcarbonyl, $C_{1-6}$ alkylsulfonyl, 3-12 membered heterocyclyl, wherein the amino, $C_{1-6}$ alkyl, $C_{1-6}$ alkylamino, $C_{1-6}$ alkylcarbonylamino, $C_{1-6}$ alkylaminocarbonyl, $C_{1-6}$ alkylcarbonyl, $C_{1-6}$ alkylsulfonyl, 3-12 membered heterocyclyl, are unsubstituted or optionally substituted by one or more groups selected from hydroxyl, amino, carboxy, cyano, nitro, halogen, $C_{1-6}$ alkyl, $C_{1-6}$ alkoxy, halogenated $C_{1-6}$ alkyl, halogenated $C_{1-6}$ alkoxy, $C_{1-6}$ alkylamino, $(C_{1-6}$ alkyl$)_2$ amino, $C_{1-6}$ alkylsulfonyl, HS(O)(=NH)-, $C_{1-6}$ alkyl-S(O)(=NH)-, 3-12 membered cycloalkyl, 3-12 membered heterocyclyl;
$R_2$ is selected from amino, $C_{1-6}$ alkyl, $C_{1-6}$ alkoxy, $C_{1-6}$ alkylamino, $(C_{1-6}$ alkyl$)_2$ amino, 3-12 membered heterocyclyl, 3-12 membered heterocyclyloxy, 3-12 membered cycloalkyl amino, 3-12 membered heterocyclyl-$CH_2$-amino, wherein the amino, $C_{1-6}$ alkyl, $C_{1-6}$ alkoxy, $C_{1-6}$ alkylamino, $(C_{1-6}$ alkyl$)_2$ amino, 3-12 membered heterocyclyl, 3-12 membered heterocyclyloxy, 3-12 membered cycloalkyl amino, 3-12 membered heterocyclyl-$CH_2$-amino are unsubstituted or optionally substituted by one or more groups selected from halogen, cyano, hydroxyl, carboxy, amino, $C_{1-6}$ alkyl, $C_{1-6}$ alkoxy, halogenated $C_{1-6}$ alkyl, halogenated $C_{1-6}$ alkoxy, $C_{1-6}$ alkylamino, $(C_{1-6}$ alkyl$)_2$ amino, 3-12 membered cycloalkyl, 3-12 membered heterocyclyl, aryl;
$R_3$ is selected from hydrogen, hydroxy, amino, carboxy, cyano, nitro, halogen, $C_{1-6}$ alkyl, $C_{1-6}$ alkoxy, $C_{1-6}$ alkylamino, , halogenated $C_{1-6}$ alkyl, , $C_{2-8}$ alkenyl, $C_{2-8}$ alkynyl, $C_{1-6}$ alkylcarbonyl, $C_{1-6}$ alkylsulfonyl, , 3-12 membered cycloalkyl, 3-12 membered heterocyclyl,
wherein the amino, $C_{1-6}$ alkyl, $C_{1-6}$ alkoxy, $C_{1-6}$ alkylamino, halogenated $C_{1-6}$ alkyl, $C_{2-8}$ alkenyl, $C_{1-6}$ alkylcarbonyl, $C_{1-6}$ alkylsulfonyl, 3-12 membered cycloalkyl, 3-12 membered heterocyclyl are unsubstituted or optionally substituted by one or more groups selected from hydroxyl, amino, carboxyl, cyano, nitro, halogen, $C_{1-6}$ alkyl, $C_{1-6}$ alkoxy, $C_{1-6}$ alkoxy$C_{1-6}$ alkoxy, $C_{1-6}$ alkylamino, $(C_{1-6}$ alkyl$)_2$ amino, $C_{1-6}$ alkylcarbonylamino and $C_{1-6}$ alkylsulfonylamino;
n is 0 or 1;
m is 0, 1, 2 or 3;
p is 1, 2 or 3;
$R_4$, $R_5$ and $R_6$ are each independently selected from hydrogen, and halogen.

**Some embodiments of the present invention relate to the compound shown in formula (I), or pharmaceutically acceptable salts or stereoisomers thereof,**

$X_1$ is N;
$X_2$ is selected from N or $CR_5$;
L is selected from bond or -C(O)-;
A is selected from 3-12 membered cycloalkyl, 3-12 membered heterocyclyl, wherein the heteroatoms of the 3-12 membered heterocyclyl are N,
$R_1$ is selected from hydrogen, halogen, hydroxyl, amino, $C_{1-6}$ alkyl, $C_{1-6}$ alkylamino, $C_{1-6}$ alkylcarbonylamino, wherein the $C_{1-6}$ alkyl, $C_{1-6}$ alkylamino, $C_{1-6}$ alkylcarbonylamino are unsubstituted or optionally substituted by one or more groups selected from hydroxyl, $C_{1-6}$ alkoxy, $(C_{1-6}$ alkyl$)_2$ amino, HS(O)(=NH)-, $C_{1-6}$ alkyl-S(O)(=NH)-;
$R_2$ is selected from amino, $C_{1-6}$ alkoxy, $C_{1-6}$ alkylamino, 3-12 membered heterocyclyloxy, 3-12 membered cycloalkyl amino, 3-12 membered heterocyclyl-$CH_2$-amino, wherein the amino, $C_{1-6}$ alkylamino, 3-12 membered heterocyclyloxy, 3-12 membered cycloalkyl amino, 3-12 membered heterocyclyl-$CH_2$-amino are unsubstituted or optionally substituted by 3-12 membered cycloalkyl, aryl;
$R_3$ is selected from cyano, $C_{1-6}$ alkyl, wherein the $C_{1-6}$ alkyl are unsubstituted or optionally substituted by one or more groups selected from hydroxyl, halogen;
n is 0 or 1;
m is 0 or 1
$R_4$, $R_5$ and $R_6$ are each independently selected from hydrogen or halogen.

**Some embodiments of the present invention relate to the compound shown in formula (II), or pharma-**

**ceutically acceptable salts or stereoisomers thereof,**

$R_1$ is selected from hydrogen, halogen, hydroxyl, carboxyl, amino, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl , halogenated $C_{1-6}$ alkoxy, $C_{2-8}$ alkenyl, $C_{2-8}$ alkynyl, $C_{1-6}$ alkylcarbonylamino, $C_{1-6}$ alkylaminocarbonyl, ($C_{1-6}$ alkyl)$_2$ aminocarbonyl, $C_{1-6}$ alkoxycarbonyl, $C_{1-6}$ alkylcarbonyl, $C_{1-6}$ alkylsulfonyl, aminosulfonyl, $C_{1-6}$ alkylthio, 3-12 membered cycloalkyl, 3-12 membered heterocyclyl, 3-12 membered cycloalkenyl, wherein the heteroatoms of the 3-12 membered heterocyclyl are selected from one of O, S, N or any combination thereof, C atom can be optionally oxidized to C(O), S atom can be optionally oxidized to S(O) or S(O)$_2$, the heteroatoms of the 5-10 membered heteroaryl are selected from one of O, S, and N or any combination thereof, wherein the amino, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl , halogenated $C_{1-6}$ alkoxy, $C_{2-8}$ alkenyl, $C_{2-8}$ alkynyl, $C_{1-6}$ alkylcarbonylamino, $C_{1-6}$ alkylaminocarbonyl, ($C_{1-6}$ alkyl)$_2$ aminocarbonyl, $C_{1-6}$ alkoxycarbonyl,$C_{1-6}$ alkylcarbonyl, $C_{1-6}$ alkylsulfonyl, aminosulfonyl, $C_{1-6}$ alkylthio, 3-12 membered cycloalkyl, 3-12 membered heterocyclyl, 3-12 membered cycloalkenyl are unsubstituted or optionally substituted by one or more groups selected from hydroxyl, amino, carboxy, cyano, nitro, halogen, $C_{1-6}$ alkyl, $C_{1-6}$ alkoxy, halogenated $C_{1-6}$ alkyl, halogenated $C_{1-6}$ alkoxy, $C_{1-6}$ alkylamino, $C_{1-6}$ alkylsulfonyl, HS(O)(=NH)-, $C_{1-6}$ alkyl-S(O)(=NH)-, 3-12 membered cycloalkyl, 3-12 membered heterocyclyl;

$R_3$ is selected from hydrogen, hydroxy, amino, carboxy, cyano, nitro, halogen, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonyl, $C_{1-6}$ alkylsulfonyl, $C_{1-6}$ alkylthio , 3-12 membered cycloalkyl, 3-12 membered cycloalkenyl, 3-12 membered heterocyclyl, wherein the amino, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonyl, $C_{1-6}$ alkylsulfonyl, $C_{1-6}$ alkylthio , 3-12 membered cycloalkyl, 3-12 membered cycloalkenyl, 3-12 membered heterocyclyl are unsubstituted or optionally substituted by one or more groups selected from amino, carboxyl, cyano, nitro, halogen, $C_{1-6}$ alkyl, $C_{1-6}$ alkoxy, $C_{1-6}$ alkoxy$C_{1-6}$ alkoxy, $C_{1-6}$ alkylamino, ($C_{1-6}$ alkyl)$_2$ amino, $C_{1-6}$ alkylcarbonylamino and $C_{1-6}$ alkylsulfonylamino.

**Some embodiments of the present invention relate to the compound shown in formula (I), or pharmaceutically acceptable salts or stereoisomers thereof,**

$X_1$ is N;
$X_2$ is $CR_5$;
L is bond;
n is 0;
A is 3-12 membered heterocyclyl;
wherein the heteroatoms of the 3-12 membered heterocyclyl is N;
$R_1$ is hydrogen;
$R_2$ is selected from $C_{1-6}$ alkylamino, $C_{1-6}$ alkoxy or 3-12 membered cycloalkyl amino,
wherein $C_{1-6}$ alkylamino, $C_{1-6}$ alkoxy or 3-12 membered cycloalkyl amino are unsubstituted or optionally substituted by $C_{1-6}$ alkyl;
$R_3$ is cyano;
$R_5$ and $R_6$ are each independently hydrogen; and
m is 1.

**Some embodiments of the present invention relate to the compound shown in formula (II), or pharmaceutically acceptable salts or stereoisomers thereof,**

$R_1$ is selected from hydrogen, halogen, hydroxyl, carboxyl, amino, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl , halogenated $C_{1-6}$ alkoxy, $C_{2-8}$ alkenyl, $C_{2-8}$ alkynyl, $C_{1-6}$ alkylcarbonylamino, $C_{1-6}$ alkylaminocarbonyl, ($C_{1-6}$ alkyl)$_2$ aminocarbonyl, $C_{1-6}$ alkoxycarbonyl, $C_{1-6}$ alkylcarbonyl, $C_{1-6}$ alkylsulfonyl, aminosulfonyl, $C_{1-6}$ alkylthio, 3-12 membered cycloalkyl, 3-12 membered heterocyclyl, 3-12 membered cycloalkenyl, wherein the heteroatoms of the 3-12 membered heterocyclyl are selected from one of O, S, N or any combination thereof, C atom can be optionally oxidized to C(O), S atom can be optionally oxidized to S(O) or S(O)$_2$, the heteroatoms of the 5-10 membered heteroaryl are selected from one of O, S, and N or any combination thereof, wherein the amino, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl , halogenated $C_{1-6}$ alkoxy, $C_{2-8}$ alkenyl, $C_{2-8}$ alkynyl, $C_{1-6}$ alkylcarbonylamino, $C_{1-6}$ alkylaminocarbonyl, ($C_{1-6}$ alkyl)$_2$ aminocarbonyl, $C_{1-6}$ alkoxycarbonyl,$C_{1-6}$ alkylcarbonyl, $C_{1-6}$ alkylsulfonyl, aminosulfonyl, $C_{1-6}$ alkylthio, 3-12 membered cycloalkyl, 3-12 membered heterocyclyl, 3-12 membered cycloalkenyl are unsubstituted or optionally substituted by one or more groups selected from hydroxyl, amino, carboxy, cyano, nitro, halogen, $C_{1-6}$ alkyl, $C_{1-6}$ alkoxy, halogenated $C_{1-6}$ alkyl, halogenated $C_{1-6}$ alkoxy, $C_{1-6}$ alkylamino, $C_{1-6}$ alkylsulfonyl, 3-12 membered cycloalkyl, 3-12 membered heterocyclyl;

$R_3$ is selected from hydrogen, hydroxy, amino, carboxy, cyano, nitro, halogen, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonyl, $C_{1-6}$ alkylsulfonyl, $C_{1-6}$ alkylthio , 3-12 membered cycloalkyl, 3-12 membered cycloalkenyl, 3-12 membered heterocyclyl, wherein the amino, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonyl, $C_{1-6}$ alkylsulfonyl, $C_{1-6}$ alkylthio , 3-12 membered cycloalkyl, 3-12 membered cycloalkenyl, 3-12 membered heterocyclyl are unsubstituted or optionally substituted by one or more groups selected from amino, carboxyl, cyano, nitro, halogen, $C_{1-6}$ alkyl, $C_{1-6}$ alkoxy, $C_{1-6}$ alkoxy$C_{1-6}$ alkoxy, $C_{1-6}$ alkylamino, ($C_{1-6}$ alkyl)$_2$ amino, $C_{1-6}$ alkylcarbonylamino and $C_{1-6}$ alkylsulfonylamino.

**Some embodiments of the present invention relate to the compound shown in formula (II), or pharmaceutically acceptable salts or stereoisomers thereof,**

$R_1$ is selected from hydrogen, halogen, hydroxyl, carboxyl, amino, $C_{1-6}$ alkyl, $C_{1-6}$ alkoxy, $C_{1-6}$ alkylamino, ($C_{1-6}$ alkyl)2 amino, halogenated $C_{1-6}$ alkyl , halogenated $C_{1-6}$ alkoxy, $C_{2-8}$ alkenyl, $C_{2-8}$ alkynyl, $C_{1-6}$ alkylaminocarbonyl, ($C_{1-6}$ alkyl)$_2$ aminocarbonyl, $C_{1-6}$ alkoxycarbonyl, $C_{1-6}$alkylcarbonyl, $C_{1-6}$ alkylsulfonyl, $C_{1-6}$ alkylthio, 3-12 membered cycloalkyl, 3-12 membered heterocyclyl, 3-12 membered cycloalkenyl, wherein the amino, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl , halogenated $C_{1-6}$ alkoxy, $C_{2-8}$ alkenyl, $C_{2-8}$ alkynyl, $C_{1-6}$ alkylaminocarbonyl, ($C_{1-6}$ alkyl)$_2$ aminocarbonyl, $C_{1-6}$ alkoxycarbonyl,$C_{1-6}$ alkylcarbonyl, $C_{1-6}$ alkylsulfonyl, $C_{1-6}$ alkylthio, 3-12 membered cycloalkyl, 3-12 membered heterocyclyl, 3-12 membered cycloalkenyl are unsubstituted or optionally substituted by one or more groups selected from hydroxyl, amino, carboxy, cyano, nitro, halogen, $C_{1-6}$ alkyl, $C_{1-6}$ alkoxy, halogenated $C_{1-6}$ alkyl, halogenated $C_{1-6}$ alkoxy, $C_{1-6}$ alkylamino, ($C_{1-6}$ alkyl)$_2$ amino, $C_{1-6}$ alkylsulfonyl, 3-12 membered cycloalkyl, 3-12 membered heterocyclyl;

$R_3$ is selected from cyano, and halogenated $C_{1-6}$ alkyl.

**Some embodiments of the present invention relate to the compound shown in formula (I), (II), (III) or (IV), or pharmaceutically acceptable salts or stereoisomers thereof,**
A is selected from the group consisting of 3-8 membered cycloalkyl, 6-11 membered ortho-fused cycloalkyl, 6-11 membered bridged cycloalkyl, 7-12 membered spirocycloalkyl, 3-8 membered monocycloalkenyl, 7-11 membered spiro cycloalkenyl, 7-11 membered ortho-fused cycloalkenyl, 6-11 membered bridged cycloalkenyl, 3-8 membered heterocyclyl, 6-12 membered ortho-fused heterocyclyl, 6-12 membered spiro heterocyclyl, 6-12 membered bridged heterocyclyl, wherein the heteroatoms of 3-8 membered heterocyclyl, 6-12 membered ortho-fused heterocyclyl, 6-12 membered spiro heterocyclyl, 6-12 membered bridged heterocyclyl are selected from one of O, S, N or any combination thereof, the C atom can be optionally oxidized to C(O), and the S atom can be optionally oxidized to S(O) or $S(O)_2$. The heteroatoms of the 5-10 membered heteroaryl are selected from one of O, S, and N or any combination thereof.
**Some embodiments of the present invention relate to the compound shown in formula (I), (II), (III) or (IV)" or pharmaceutically acceptable salts or stereoisomers thereof,**
A is selected from

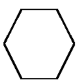, 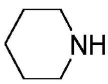, 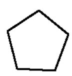, 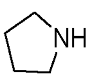, 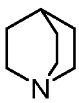, 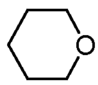, 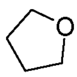, 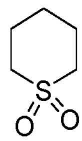,

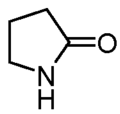, 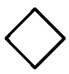, 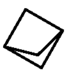, 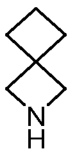, 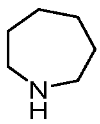, 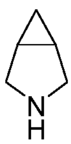, 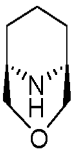, 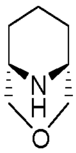.

**Some embodiments of the present invention relate to the compound shown in formula (I), or pharmaceutically acceptable salts or stereoisomers thereof,**
$R_1$ is selected from hydrogen, halogen, hydroxyl, carboxyl, amino, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl , halogenated $C_{1-6}$ alkoxy, $C_{2-8}$ alkenyl, $C_{2-8}$ alkynyl, $C_{1-6}$ alkylcarbonylamino, $C_{1-6}$ alkylaminocarbonyl, ($C_{1-6}$ alkyl)$_2$ aminocarbonyl, $C_{1-6}$ alkoxycarbonyl, $C_{1-6}$ alkylcarbonyl, $C_{1-6}$ alkylsulfonyl, aminosulfonyl, $C_{1-6}$ alkylthio, 3-12 membered cycloalkyl, 3-12 membered heterocyclyl, 3-12 membered cycloalkenyl, wherein the

heteroatoms of the 3-12 membered heterocyclyl are selected from one of O, S, N or any combination thereof, C atom can be optionally oxidized to C(O), S atom can be optionally oxidized to S(O) or $S(O)_2$, the heteroatoms of the 5-10 membered heteroaryl are selected from one of O, S, and N or any combination thereof, wherein the amino, $C_{1-6}$ alkyl, $C_{1-6}$ alkoxy, $C_{1-6}$ alkylamino, $(C_{1-6}$ alkyl$)_2$ amino, halogenated $C_{1-6}$ alkyl , halogenated $C_{1-6}$ alkoxy, $C_{2-8}$ alkenyl, $C_{2-8}$ alkynyl, $C_{1-6}$ alkylcarbonylamino, $C_{1-6}$ alkylaminocarbonyl, $(C_{1-6}$ alkyl$)_2$ aminocarbonyl, $C_{1-6}$ alkoxycarbonyl,$C_{1-6}$ alkylcarbonyl, $C_{1-6}$ alkylsulfonyl, aminosulfonyl, $C_{1-6}$ alkylthio, 3-12 membered cycloalkyl, 3-12 membered heterocyclyl, 3-12 membered cycloalkenyl are unsubstituted or optionally substituted by one or more groups selected from hydroxyl, amino, carboxy, cyano, nitro, halogen, $C_{1-6}$ alkyl, $C_{1-6}$ alkoxy, halogenated $C_{1-6}$ alkyl, halogenated $C_{1-6}$ alkoxy, $C_{1-6}$ alkylamino, $C_{1-6}$ alkylsulfonyl, 3-12 membered cycloalkyl, 3-12 membered heterocyclyl.

**Some embodiments of the present invention relate to the compound shown in formula (I), or pharmaceutically acceptable salts or stereoisomers thereof,**

$R_3$ is selected from hydrogen, hydroxy, amino, carboxy, cyano, nitro, halogen, $C_{1-6}$ alkyl, $C_{1-6}$ alkoxy, $C_{1-6}$ alkylamino, $(C_{1-6}$ alkyl$)_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonyl, $C_{1-6}$ alkylsulfonyl, $C_{1-6}$ alkylthio , 3-12 membered cycloalkyl, 3-12 membered cycloalkenyl, 3-12 membered heterocyclyl, wherein the amino, $C_{1-6}$ alkyl, $C_{1-6}$ alkoxy, $C_{1-6}$ alkylamino, $(C_{1-6}$ alkyl$)_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonyl, $C_{1-6}$ alkylsulfonyl, $C_{1-6}$ alkylthio , 3-12 membered cycloalkyl, 3-12 membered cycloalkenyl, 3-12 membered heterocyclyl are unsubstituted or optionally substituted by one or more groups selected from amino, carboxyl, cyano, nitro, halogen, $C_{1-6}$ alkyl, $C_{1-6}$ alkoxy, $C_{1-6}$ alkoxy$C_{1-6}$ alkoxy, $C_{1-6}$ alkylamino, $(C_{1-6}$ alkyl$)_2$ amino, $C_{1-6}$ alkylcarbonylamino and $C_{1-6}$ alkylsulfonylamino.

**Some embodiments of the present invention relate to the compound shown in formula (I), or pharmaceutically acceptable salts or stereoisomers thereof,**

$X_1$ is selected from N or $CR_4$;
$X_2$ is selected from N or $CR_5$;
L is selected from bond, -C(O)-, $-(CH_2)p-$;
A is selected from bond, 3-12 membered cycloalkyl, 3-12 membered heterocyclyl, wherein the heteroatoms of the 3-12 membered heterocyclyl is N;
$R_1$ is selected from hydrogen, halogen, hydroxyl, carboxyl, amino, $C_{1-6}$ alkyl, $C_{1-6}$ alkylamino, $C_{1-6}$ alkylcarbonylamino, $C_{1-6}$ alkylaminocarbonyl, $C_{1-6}$ alkylcarbonyl, $C_{1-6}$ alkylsulfonyl, 3-12 membered heterocyclyl, wherein the amino, $C_{1-6}$ alkyl, $C_{1-6}$ alkylamino, $C_{1-6}$ alkylcarbonylamino, $C_{1-6}$ alkylaminocarbonyl, $C_{1-6}$ alkylcarbonyl, $C_{1-6}$ alkylsulfonyl, 3-12 membered heterocyclyl, are unsubstituted or optionally substituted by one or more groups selected from hydroxyl, amino, carboxy, cyano, nitro, halogen, $C_{1-6}$ alkyl, $C_{1-6}$ alkoxy, halogenated $C_{1-6}$ alkyl, halogenated $C_{1-6}$ alkoxy, $C_{1-6}$ alkylamino, $(C_{1-6}$ alkyl$)_2$ amino, $C_{1-6}$ alkylsulfonyl, 3-12 membered cycloalkyl, 3-12 membered heterocyclyl;
$R_2$ is selected from amino, $C_{1-6}$ alkyl, $C_{1-6}$ alkoxy, $C_{1-6}$ alkylamino, $(C_{1-6}$ alkyl$)_2$ amino, 3-12 membered heterocyclyl, wherein the amino, $C_{1-6}$ alkyl, $C_{1-6}$ alkoxy, $C_{1-6}$ alkylamino, $(C_{1-6}$ alkyl$)_2$ amino, 3-12 membered heterocyclyl are unsubstituted or optionally substituted by one or more groups selected from halogen, cyano, hydroxyl, carboxy, amino, $C_{1-6}$ alkyl, $C_{1-6}$ alkoxy, halogenated $C_{1-6}$ alkyl, halogenated $C_{1-6}$ alkoxy, $C_{1-6}$ alkylamino, $(C_{1-6}$ alkyl$)_2$ amino, 3-12 membered cycloalkyl, 3-12 membered heterocyclyl;
$R_3$ is selected from hydrogen, hydroxy, amino, carboxy, cyano, nitro, halogen, $C_{1-6}$ alkyl, $C_{1-6}$ alkoxy, $C_{1-6}$ alkylamino, , halogenated $C_{1-6}$ alkyl, , $C_{2-8}$ alkenyl, $C_{2-8}$ alkynyl, $C_{1-6}$ alkylcarbonyl, $C_{1-6}$ alkylsulfonyl, , 3-12 membered cycloalkyl, 3- 12 membered heterocyclyl,
wherein the amino, $C_{1-6}$ alkyl, $C_{1-6}$ alkoxy, $C_{1-6}$ alkylamino, halogenated $C_{1-6}$ alkyl, $C_{2-8}$ alkenyl, $C_{1-6}$ alkylcarbonyl, $C_{1-6}$ alkylsulfonyl, 3-12 membered cycloalkyl, 3-12 membered heterocyclyl are unsubstituted or optionally substituted by one or more groups selected from hydroxyl, amino, carboxyl, cyano, nitro, halogen, $C_{1-6}$ alkyl, $C_{1-6}$ alkoxy, $C_{1-6}$ alkoxy$C_{1-6}$ alkoxy, $C_{1-6}$ alkylamino, $(C_{1-6}$ alkyl$)_2$ amino, $C_{1-6}$ alkylcarbonylamino and $C_{1-6}$ alkylsulfonylamino;
n is 0 or 1;
m is 0, 1, 2 or 3;
p is 1, 2 or 3;
$R_4$, $R_5$ and $R_6$ are each independently selected from hydrogen, and halogen.

**Some embodiments of the present invention relate to the compound shown in formula (I), or pharmaceutically acceptable salts or stereoisomers thereof,**

Xi is N;
$X_2$ is selected from N or $CR_5$;
L is selected from bond or -C(O)-;
A is selected from 3-12 membered cycloalkyl, 3-12 membered heterocyclyl, wherein the heteroatoms of the 3-12

membered heterocyclyl are N,

$R_1$ is selected from hydrogen, halogen, hydroxyl, amino, $C_{1-6}$ alkylamino, $C_{1-6}$ alkylcarbonylamino, wherein the $C_{1-6}$ alkyl, $C_{1-6}$ alkylamino, $C_{1-6}$ alkylcarbonylamino are unsubstituted or optionally substituted by one or more groups selected from hydroxyl, $C_{1-6}$ alkoxy, $(C_{1-6}$ alkyl$)_2$ amino;

$R_2$ is selected from amino, $C_{1-6}$ alkoxy, $C_{1-6}$ alkylamino, wherein the amino, $C_{1-6}$ alkylamino are unsubstituted or optionally substituted by 3-12 membered cycloalkyl;

$R_3$ is selected from cyano, $C_{1-6}$ alkyl, wherein the $C_{1-6}$ alkyl are unsubstituted or optionally substituted by one or more groups selected from hydroxyl, halogen;

n is 0 or 1;

m is 0 or 1

$R_4$, $R_5$ and $R_6$ are halogen.

In one embodiment of the present invention, the compounds or pharmaceutically acceptable salts or stereoisomers thereof are shown in Table 1:

Table 1

| | | | |
|---|---|---|---|
| 1 | 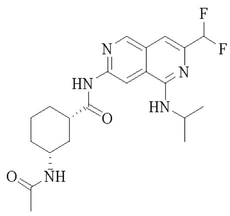 | 2 | 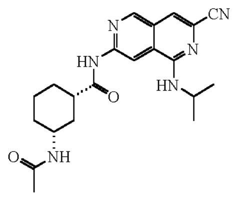 |
| 3 | 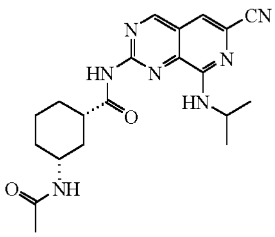 | 4 | 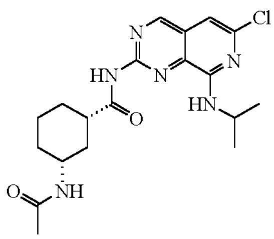 |
| 5 | 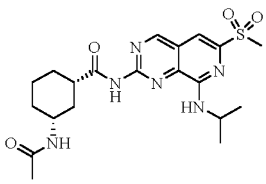 | 6 | 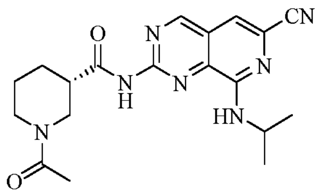 |
| 7 | 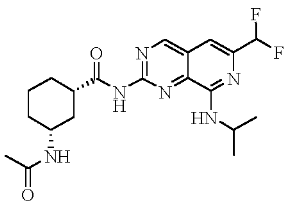 | 8 | 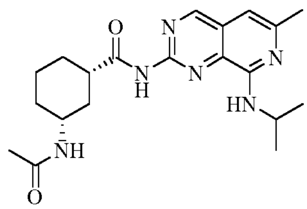 |
| 9 | 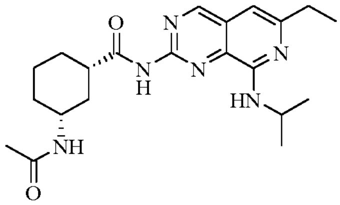 | 10 | 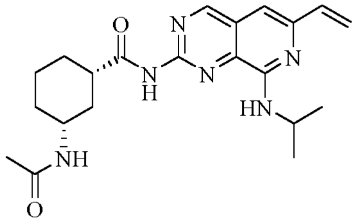 |

(continued)

| | | | |
|---|---|---|---|
| 11 | 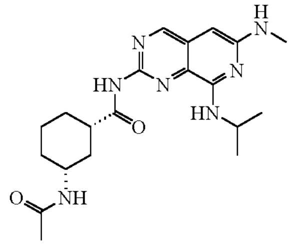 | 12 | 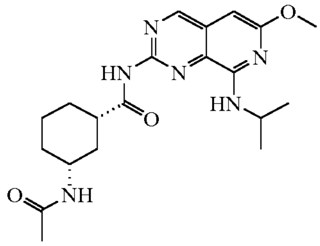 |
| 13 | 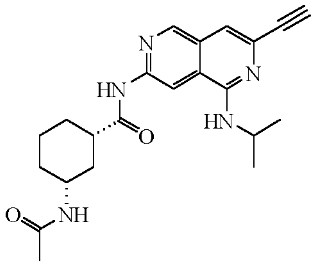 | 14 | 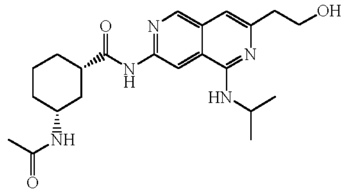 |
| 15 | 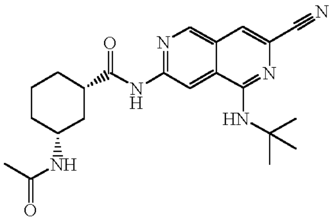 | 16 | 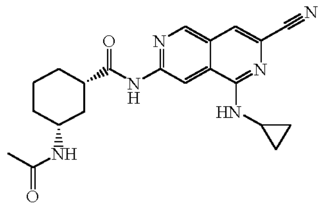 |
| 17 | 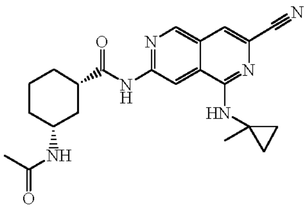 | 18 | 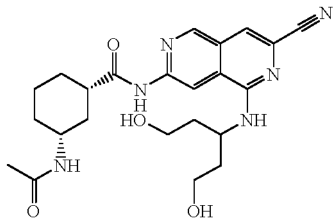 |
| 19 | 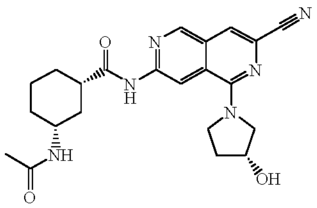 | 20 | 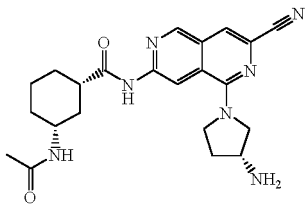 |
| 21 | 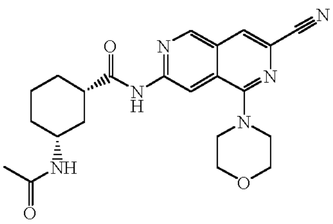 | 22 | 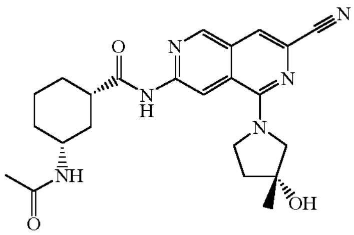 |

(continued)

| | | | |
|---|---|---|---|
| 23 | 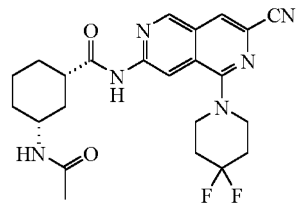 | 24 | 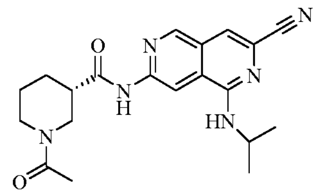 |
| 25 | 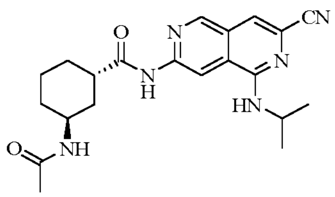 | 26 | 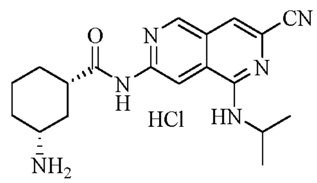 |
| 27 | 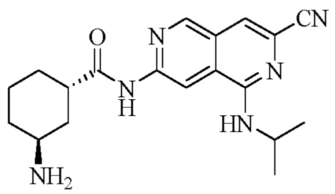 | 28 | 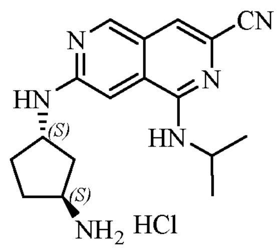 |
| 29 | 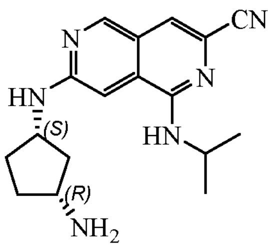 | 30 | 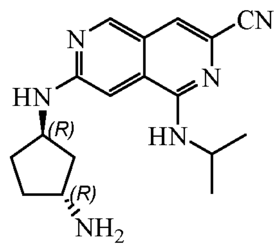 |
| 31 | 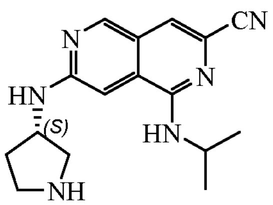 | 32 | 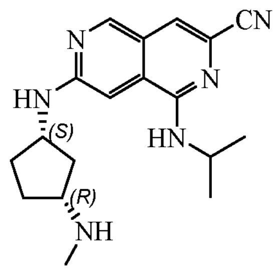 |
| 33 | 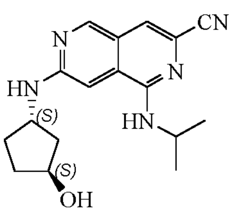 | 34 | 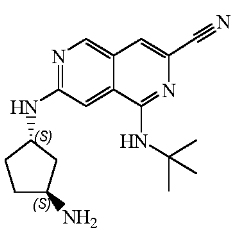 |

(continued)

| | | | |
|---|---|---|---|
| 35 | 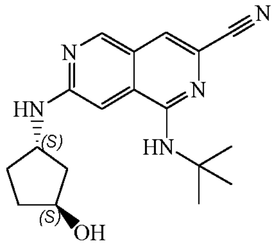 | 36 | 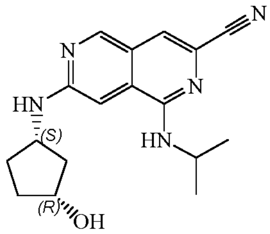 |
| 37 | 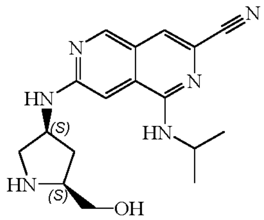 | 38 | 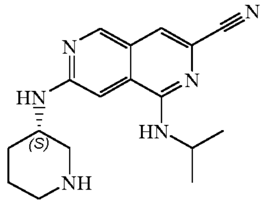 |
| 39 | 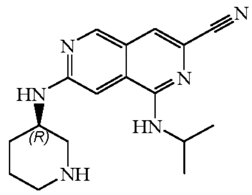 | 40 | 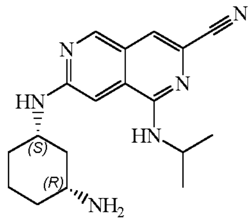 |
| 41 | 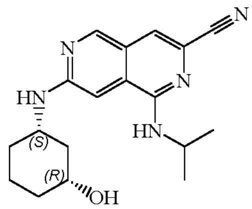 | 42 | 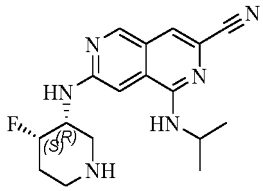 |
| 43 | 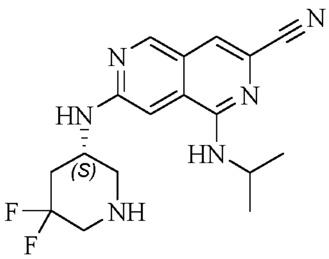 | 44 | 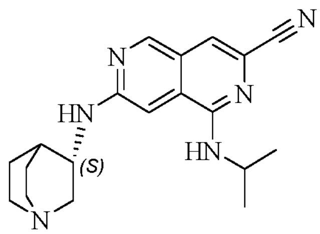 |
| 45 | 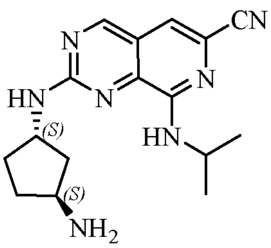 | 46 | 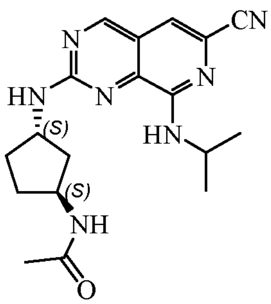 |

(continued)

| | | | |
|---|---|---|---|
| 47 | 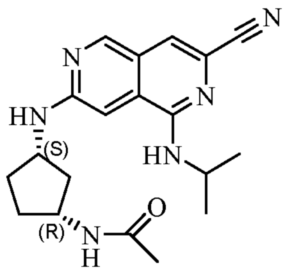 | 48 | 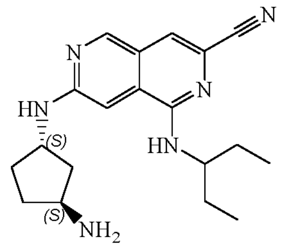 |
| 49 | 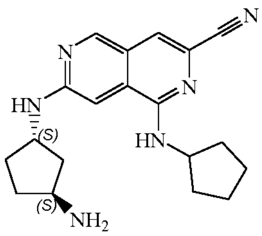 | 50 | 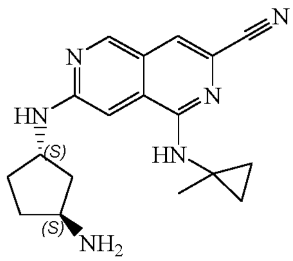 |
| 51 | 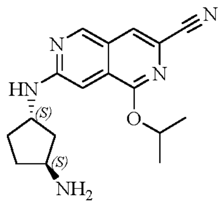 | 52 | 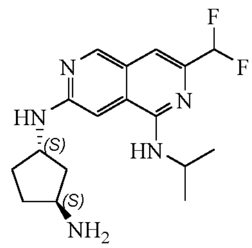 |
| 53 | 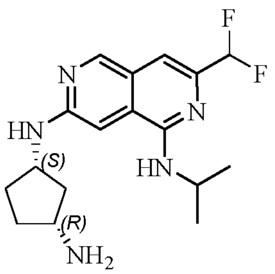 | 54 | 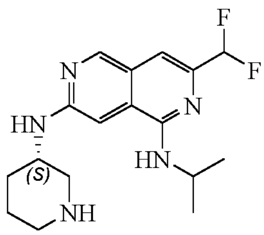 |
| 55 | 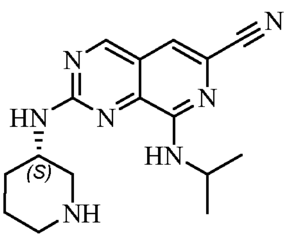 | 56 | 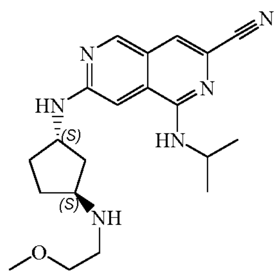 |
| 57 | 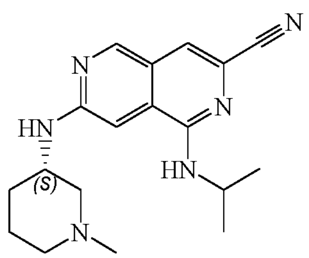 | 58 | 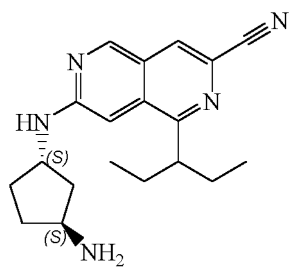 |

(continued)

| | | | |
|---|---|---|---|
| 59 | 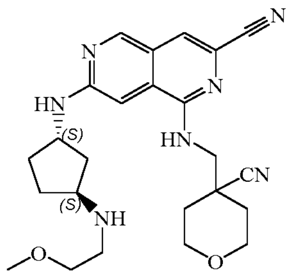 | 60 | 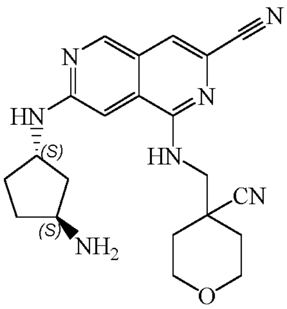 |
| 61 | 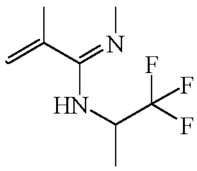 | 62 | |
| 63 | 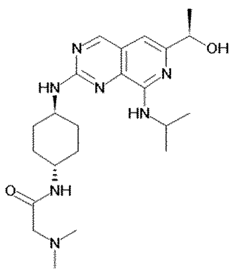 | 64 | 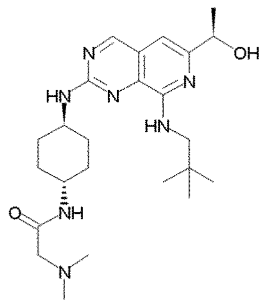 |
| 65 | 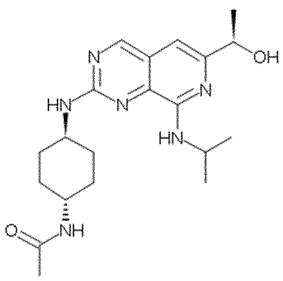 | 66 | 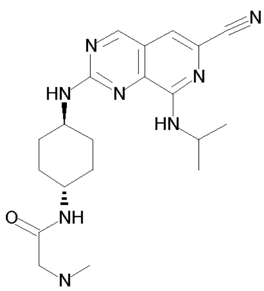 |
| 67 | 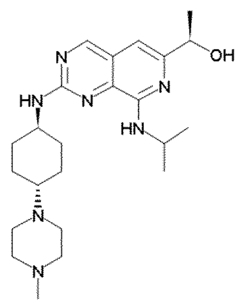 | 68 | 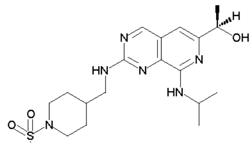 |
| 69 | 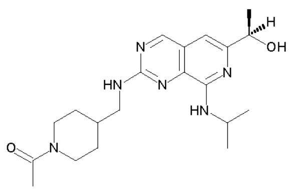 | 70 | 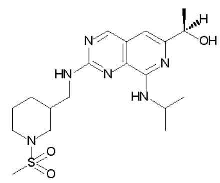 |

(continued)

| | | | |
|---|---|---|---|
| 71 | 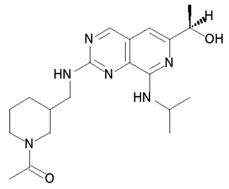 | 72 | 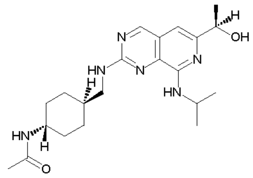 |
| 73 | 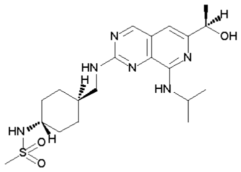 | 74 | 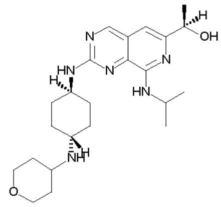 |
| 75 | 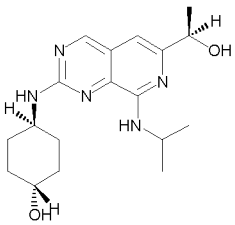 | 76 | 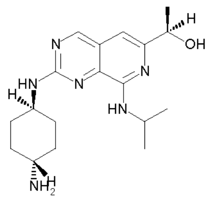 |
| 77 | 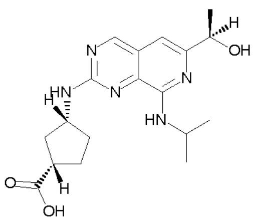 | 78 | 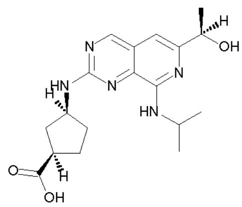 |
| 79 | 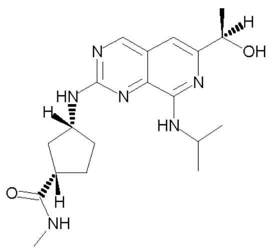 | 80 | 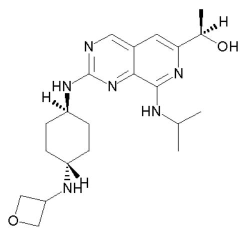 |
| 81 | 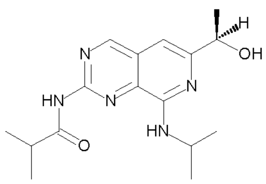 | 82 | 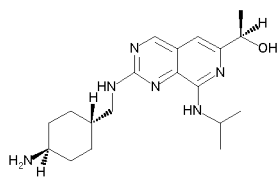 |
| 83 | 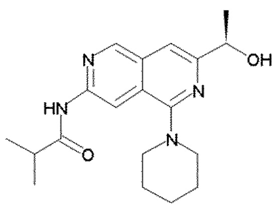 | 84 | 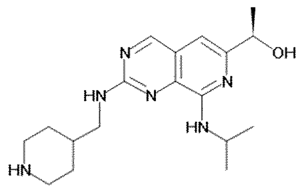 |

(continued)

| | | | |
|---|---|---|---|
| 85 | 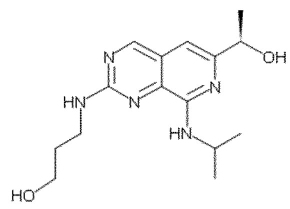 | 86 | 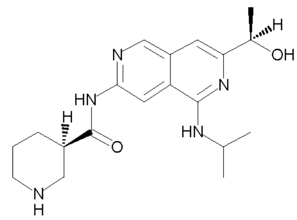 |
| 87 | 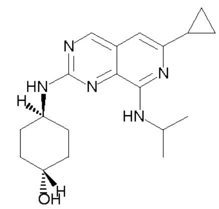 | 88 | 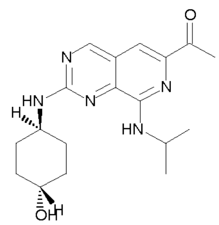 |
| 89 | 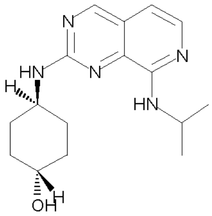 | 90 | 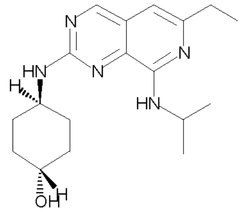 |
| 91 | 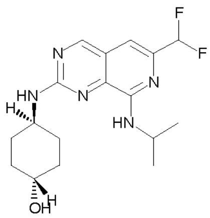 | 92 | 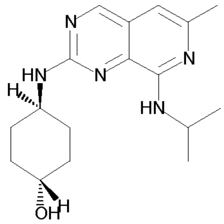 |
| 93 | 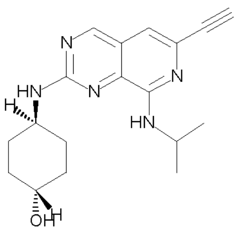 | 94 | 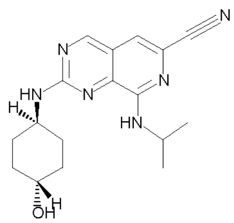 |
| 95 | 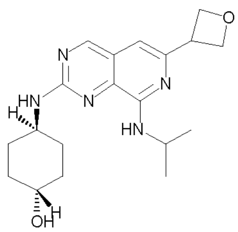 | 96 | 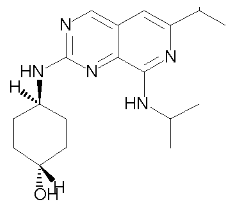 |
| 97 | 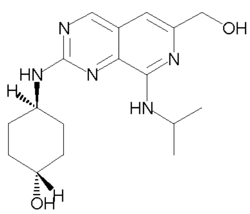 | 98 | 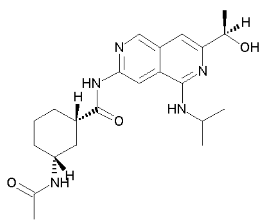 |

(continued)

| | | | |
|---|---|---|---|
| 99 | 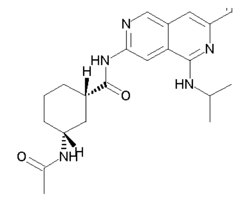 | 100 | 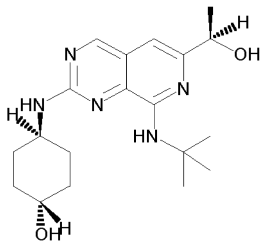 |
| 101 | 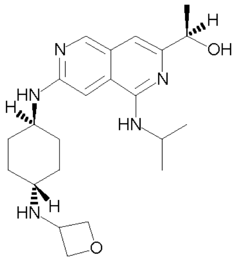 | 102 | 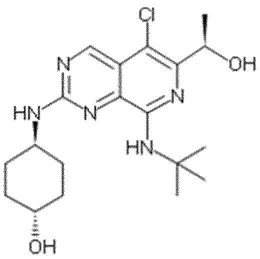 |
| 103 | | 104 | |
| 105 | 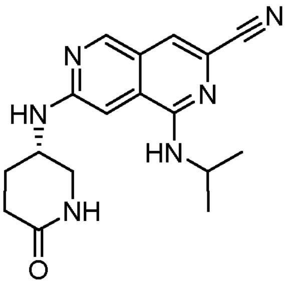 | 106 | 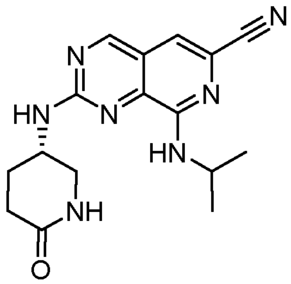 |
| 107 | 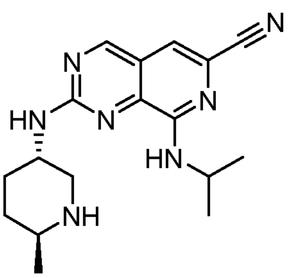 | 108 | 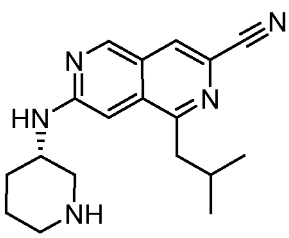 |
| 109 | 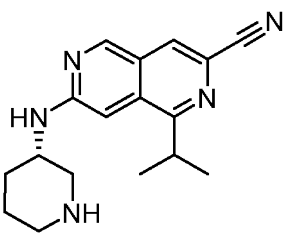 | 110 | 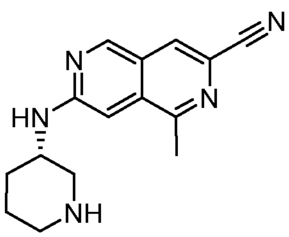 |
| 111 | 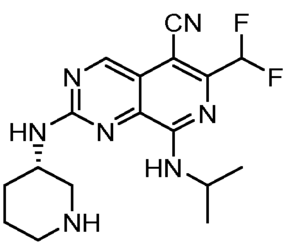 | 112 | 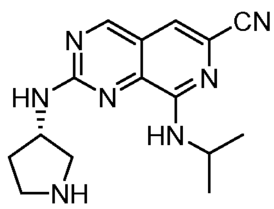 |

(continued)

| | | | |
|---|---|---|---|
| 113 | 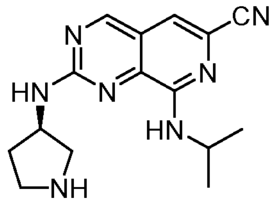 | 114 | 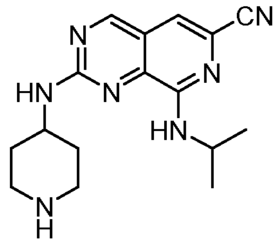 |
| 115 | 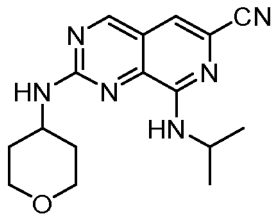 | 116 | 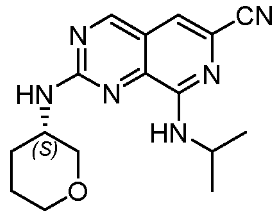 |
| 117 | 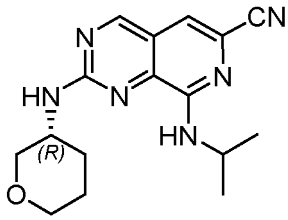 | 118 | 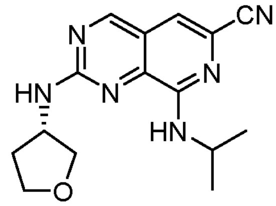 |
| 119 | 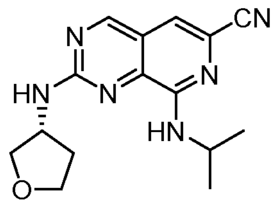 | 120 | 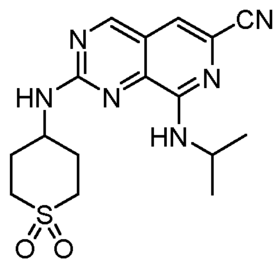 |
| 121 | 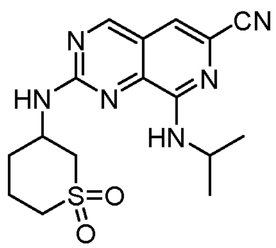 | 122 | 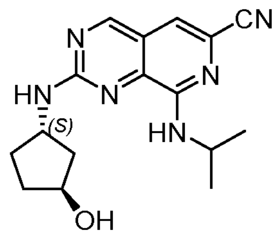 |
| 123 | 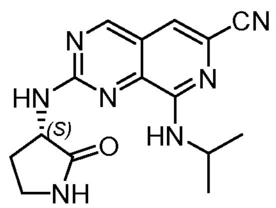 | 124 | 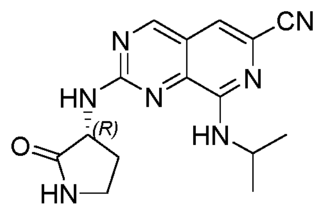 |

(continued)

| | | | |
|---|---|---|---|
| 125 | 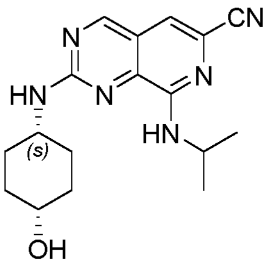 | 126 | 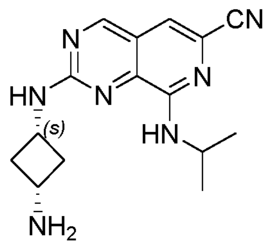 |
| 127 | 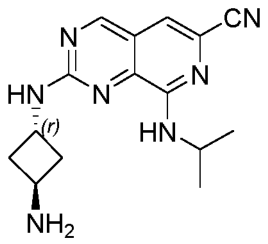 | 128 | 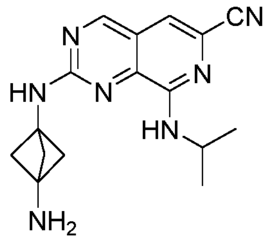 |
| 129 | 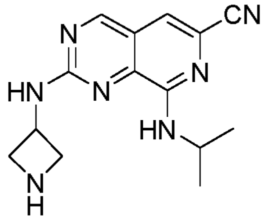 | 130 | 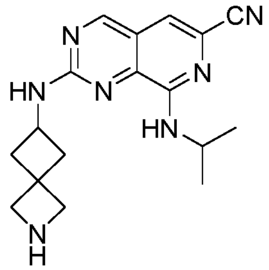 |
| 131 | 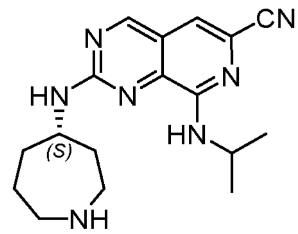 | 132 | 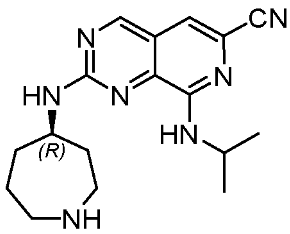 |
| 133 | 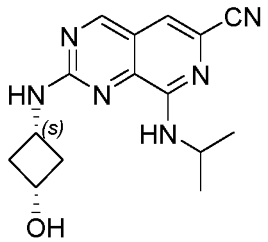 | 134 | 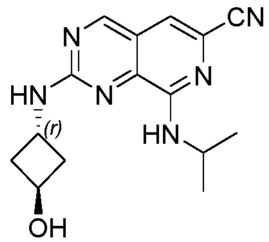 |
| 135 | 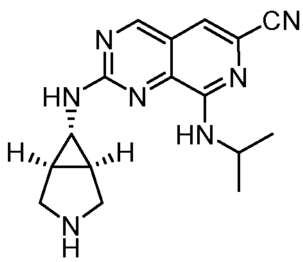 | 136 | 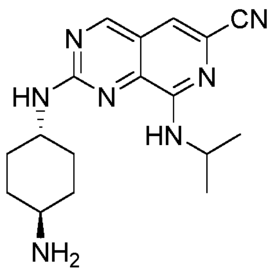 |

(continued)

| | | | |
|---|---|---|---|
| 137 | 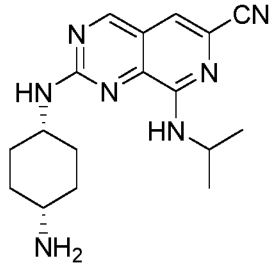 | 138 | 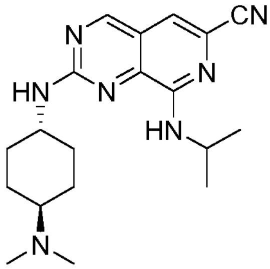 |
| 139 | 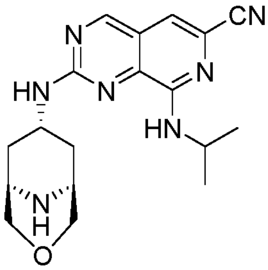 | 140 | 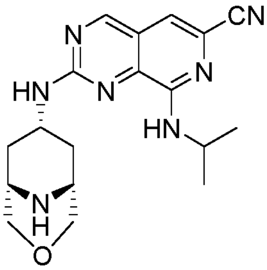 |
| 141 | 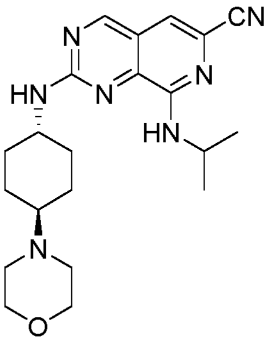 | 142 | 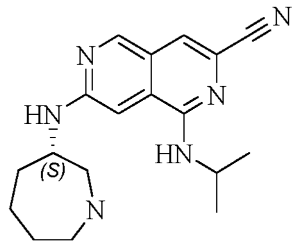 |
| 143 | 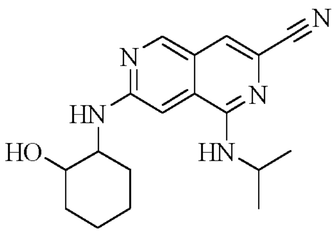 | 144 | 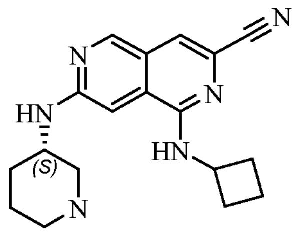 |
| 145 | 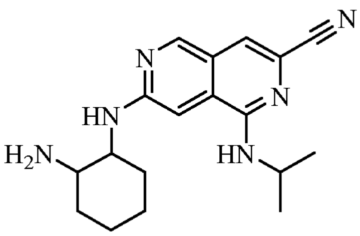 | 146 | 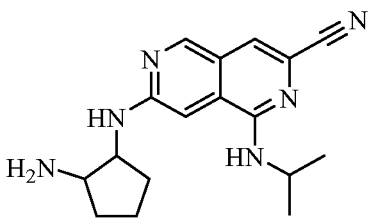 |
| 147 | 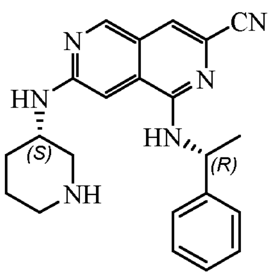 | 148 | 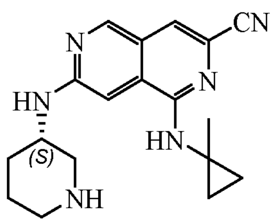 |

(continued)

| | | | |
|---|---|---|---|
| 149 | 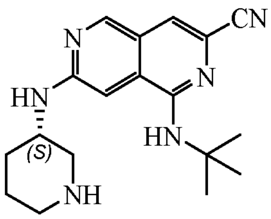 | 150 | 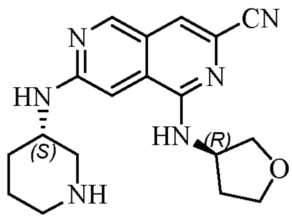 |
| 151 | 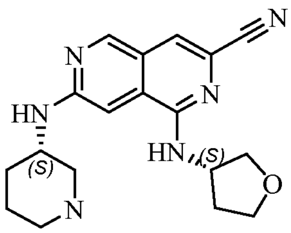 | 152 | 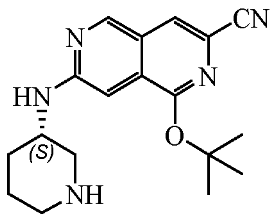 |
| 153 | 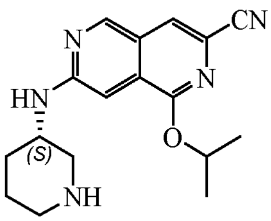 | 154 | 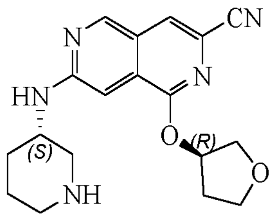 |
| 155 | 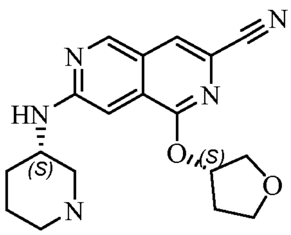 | 156 | 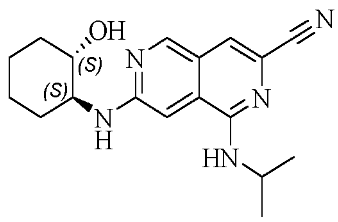 |
| 157 | 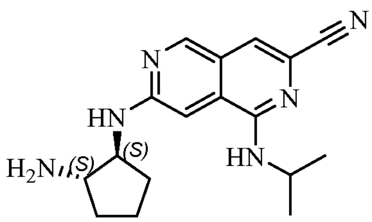 | 158 | 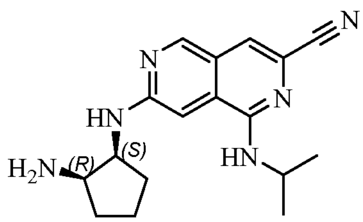 |
| 159 | 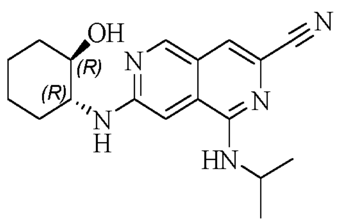 | 160 | 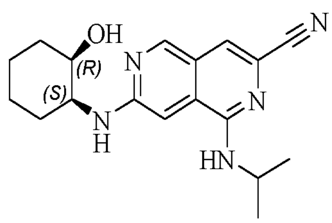 |
| 161 | 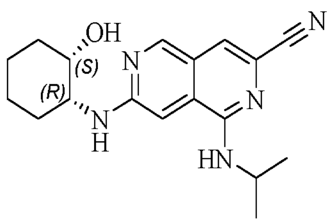 | 162 | 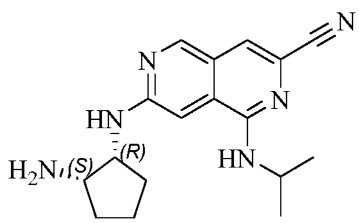 |

(continued)

| | | | |
|---|---|---|---|
| 163 | 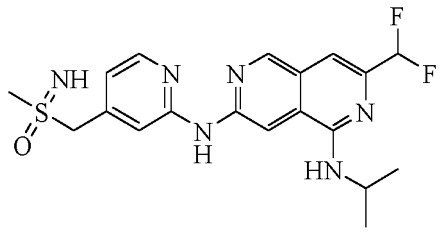 | 164 | 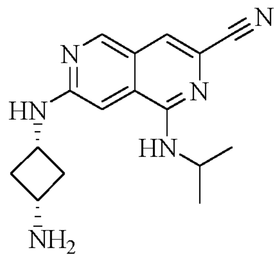 |
| 165 | 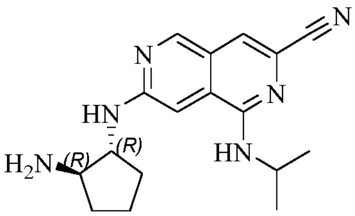 | 166 | 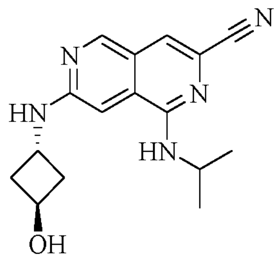 |
| 167 | 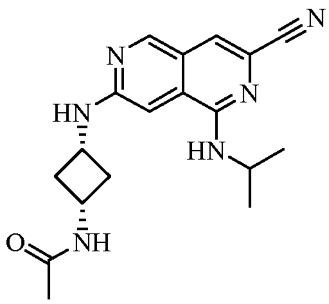 | 168 | 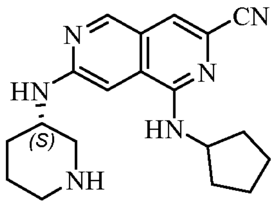 |
| 169 | 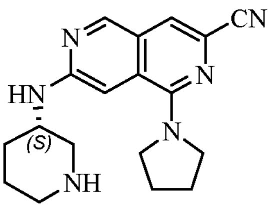 | 170 | 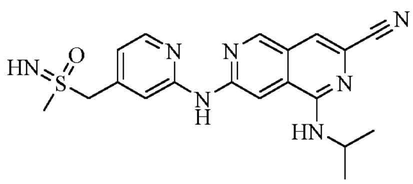 |
| 171 | 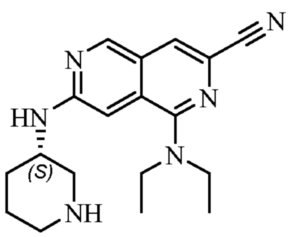 | 172 | 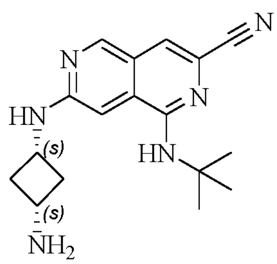 |
| 173 | 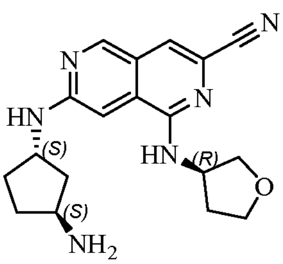 | 174 | 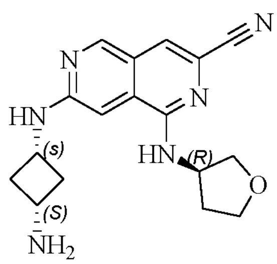 |

(continued)

| | | | |
|---|---|---|---|
| 175 | 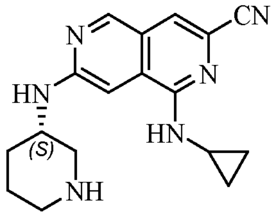 | 176 | 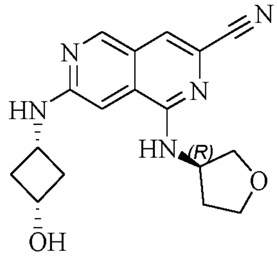 |
| 177 | 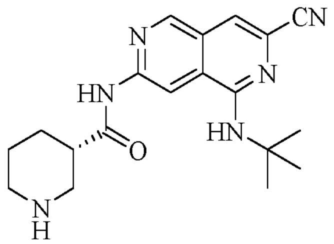 | 178 | 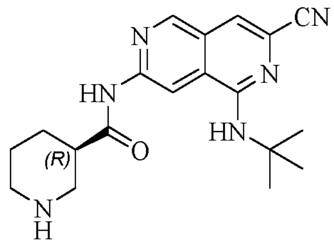 |
| 179 | 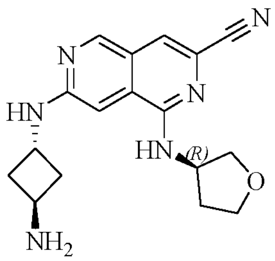 | 180 | 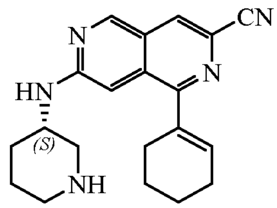 |
| 181 | 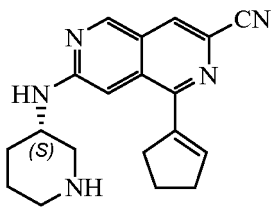 | 182 | 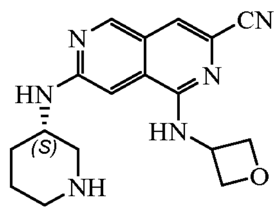 |
| 183 | 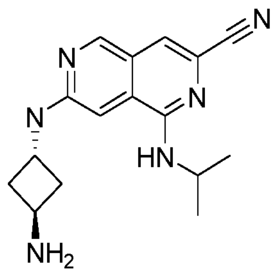 | 184 | 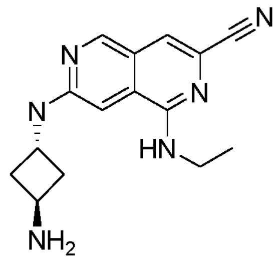 |
| 185 | 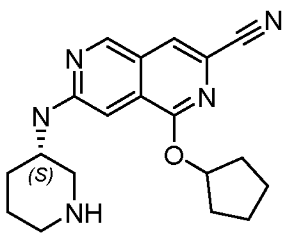 | 186 | 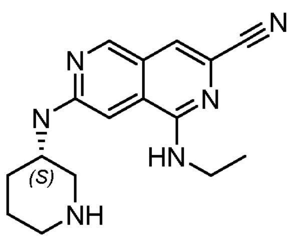 |

(continued)

| | | | |
|---|---|---|---|
| 187 | 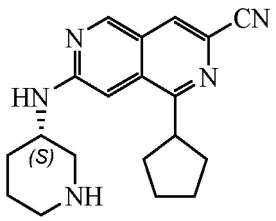 | 188 | 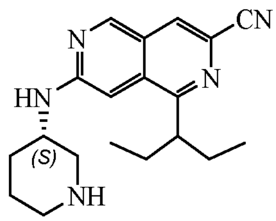 |
| 189 | 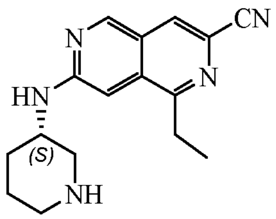 | 190 | 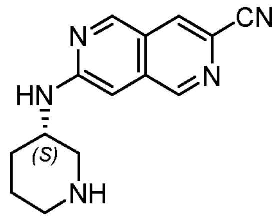 |
| 191 | 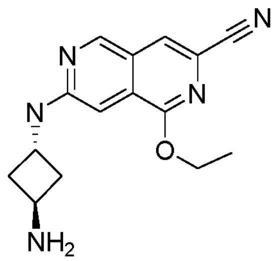 | 192 | 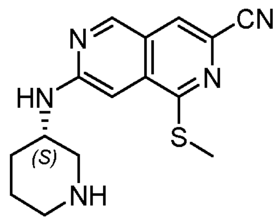 |
| 193 | 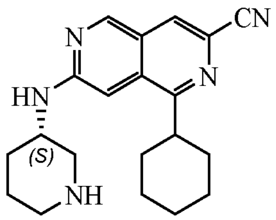 | 194 | 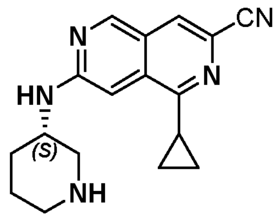 |
| 195 | 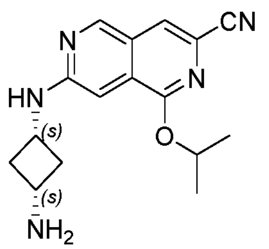 | 196 | 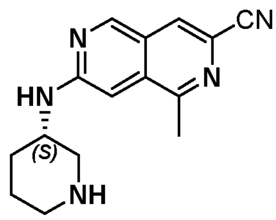 |
| 197 | 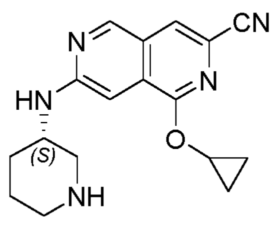 | 198 | 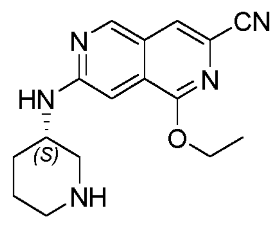 |

(continued)

| | | | |
|---|---|---|---|
| 199 | 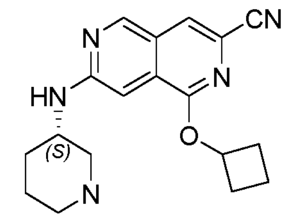 | 200 | 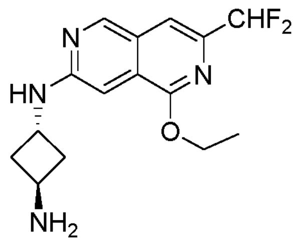 |
| 201 | 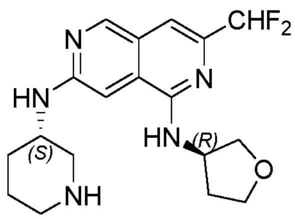 | 202 | 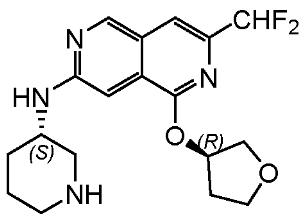 |
| 203 | 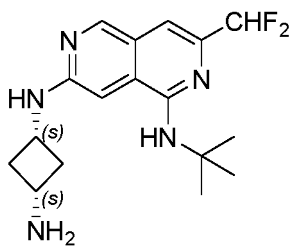 | 204 | 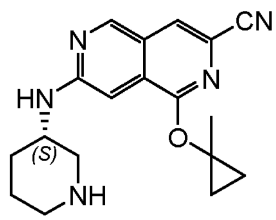 |
| 205 | 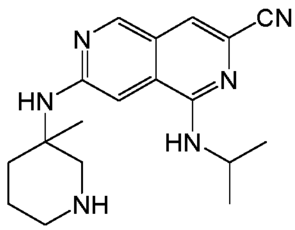 | 206 | 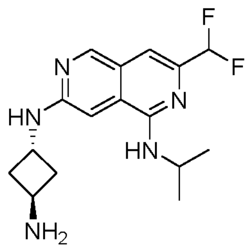 |
| 207 | 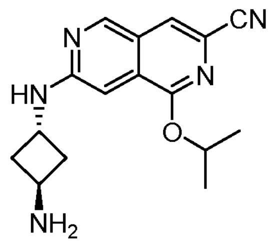 | 208 | 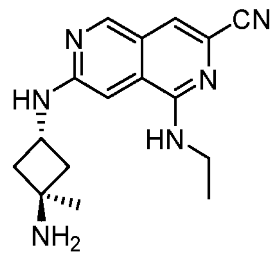 |
| 209 | 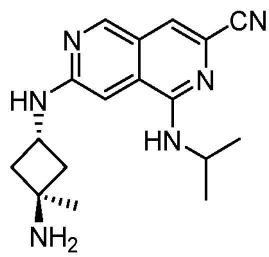 | 210 | 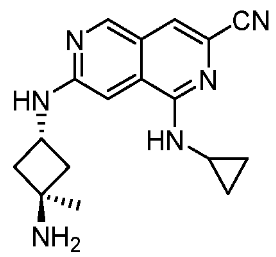 |

(continued)

| | | | |
|---|---|---|---|
| 211 | 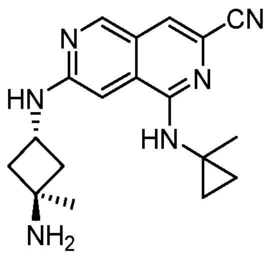 | | |
| C1 | 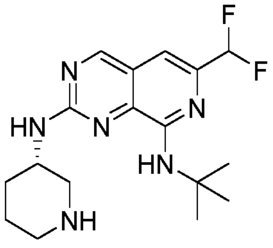 | C2 | 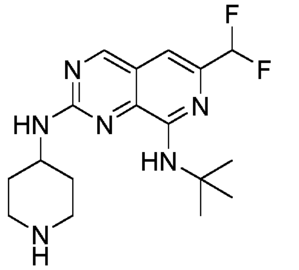 |
| C3 | 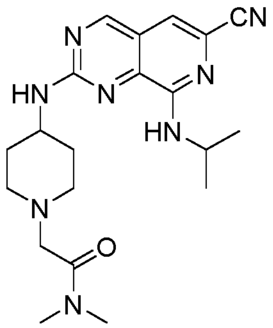 | C4 | 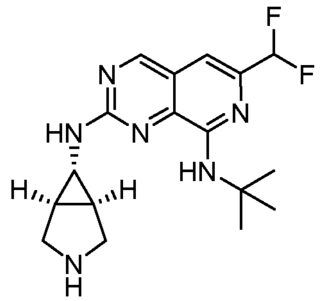 |
| C5 | 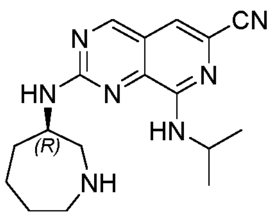 | C6 | 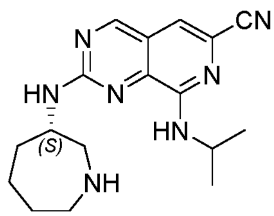 |
| C7 | 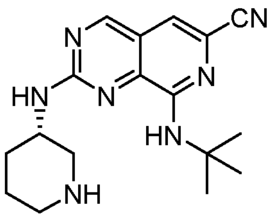 | C8 | 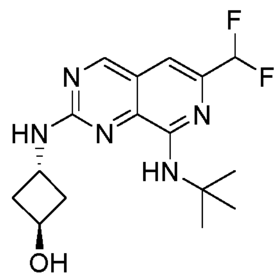 |
| C9 | 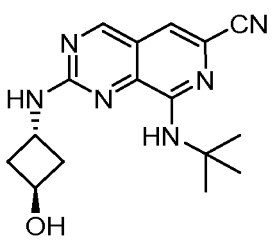 | C10 | 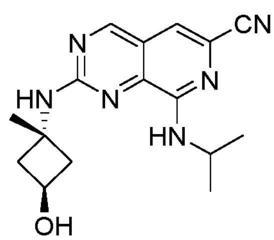 |

(continued)

| | | | |
|---|---|---|---|
| C11 | 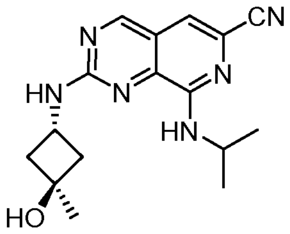 | C12 | 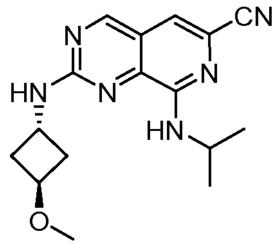 |
| C13 | 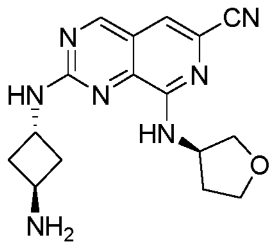 | C14 | 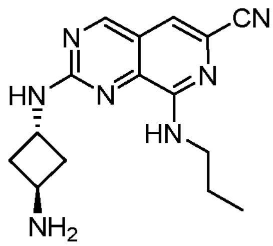 |
| C15 | 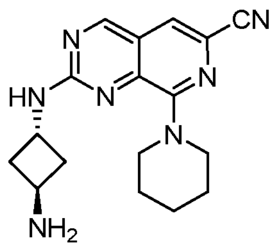 | C16 | 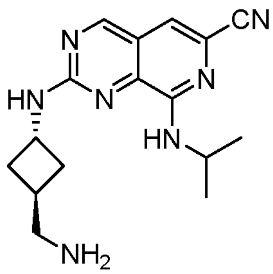 |
| C17 | 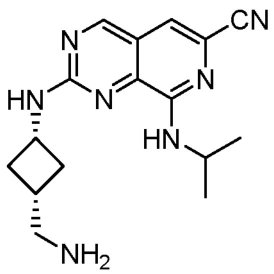 | C18 | 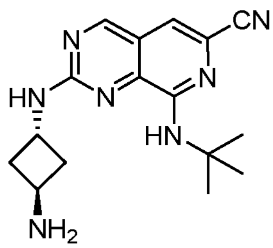 |
| C19 | 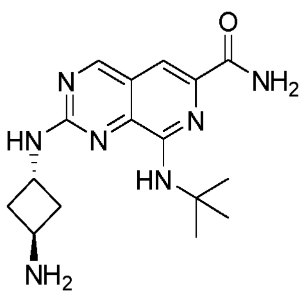 | C20 | 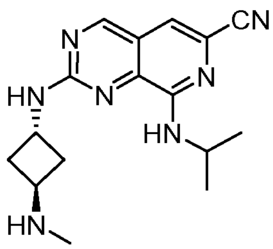 |

(continued)

| | | | |
|---|---|---|---|
| C21 | 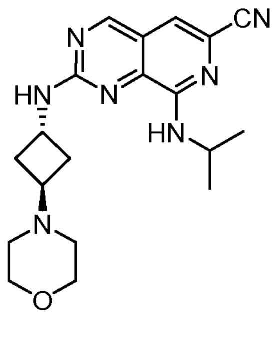 | C22 | 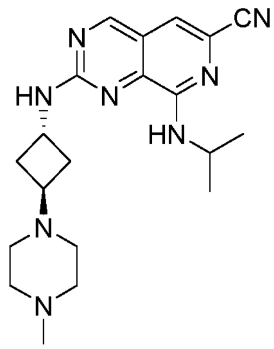 |
| C23 | 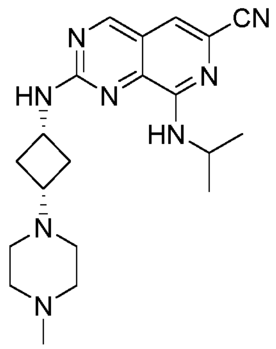 | C24 | 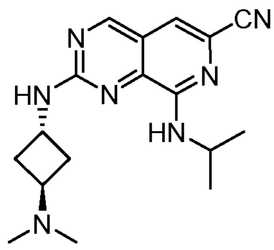 |
| C25 | 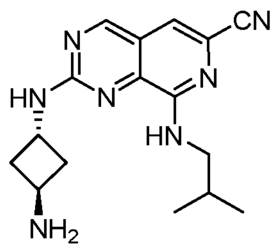 | C26 | 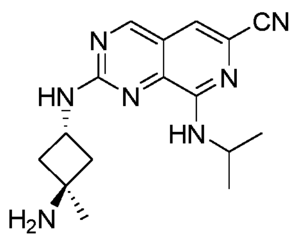 |
| C27 | 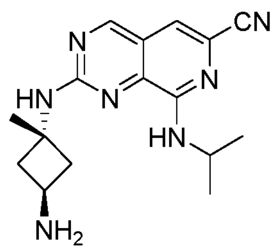 | C28 | 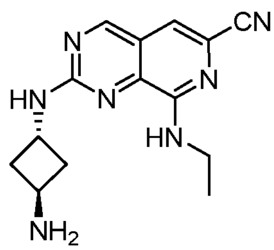 |
| C29 | 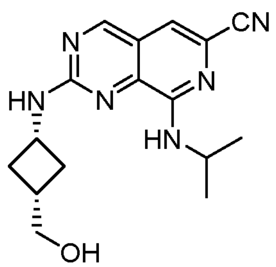 | C30 | 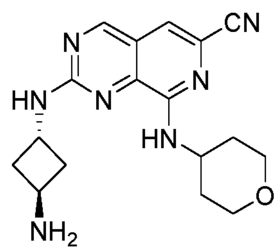 |

(continued)

| | | | |
|---|---|---|---|
| C31 | 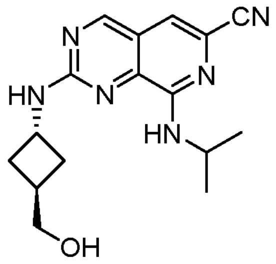 | C32 | 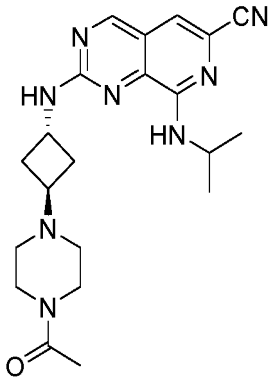 |
| C33 | 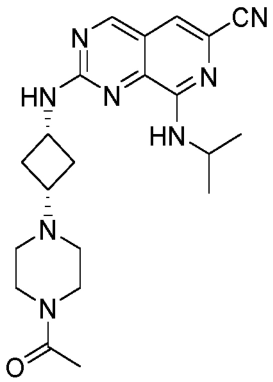 | C34 | 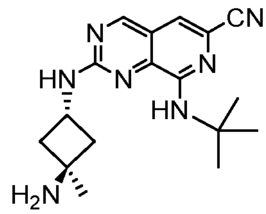 |
| C35 | 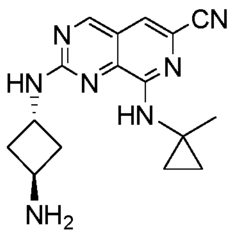 | C36 | 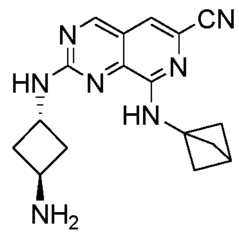 |
| C37 | 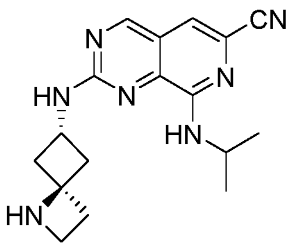 | C38 | 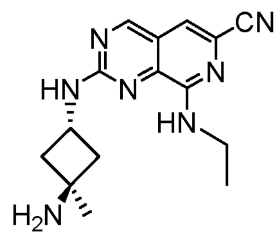 |
| C39 | 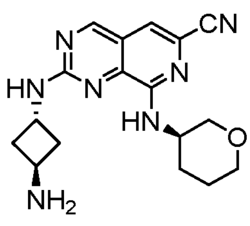 | C40 | 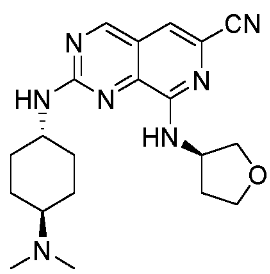 |

(continued)

| | | | |
|---|---|---|---|
| C41 | 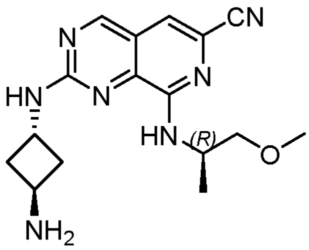 | C42 | 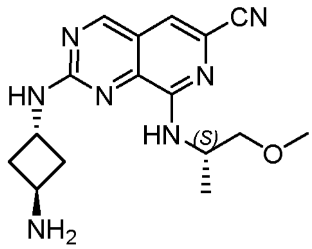 |
| C43 | 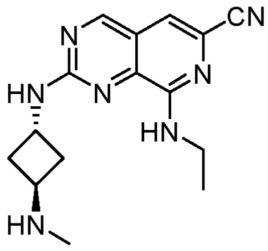 | C44 | 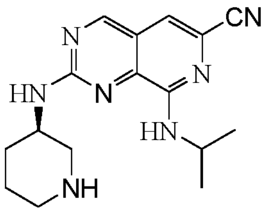 |
| C45 | 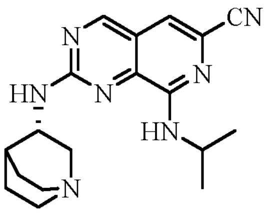 | C46 | 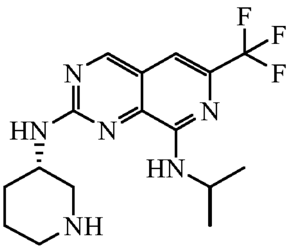 |
| C47 | 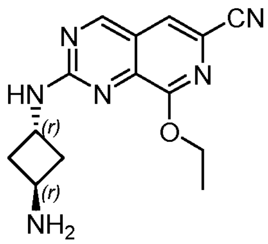 | C48 | 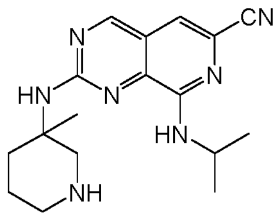 |
| C49 | 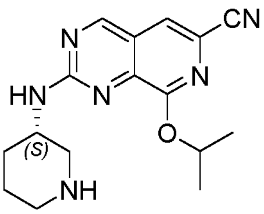 | | |

The present invention also provides a pharmaceutical composition comprising a compound shown in formula (I), (II), (III) or (IV), or a pharmaceutically acceptable salt or a isomer thereof, and one or more second therapeutically active agents.

The present invention also provides a pharmaceutical formulation comprising the compound shown in formula (I), (II), (III) or (IV), or pharmaceutically acceptable salts or a isomer thereof, and one or more pharmaceutically acceptable carriers.

The present invention also provides a pharmaceutical formulation having cyclin-dependent kinase 9 inhibitory activity, comprising the compound shown in formula (I), (II), (III) or (IV), or pharmaceutically acceptable salts or a isomer thereof, and one or more pharmaceutically acceptable carriers.

The invention also provides use of the compound shown in formula (I), (II), (III) or (IV), or a pharmaceutically acceptable salt or a isomer thereof, the pharmaceutical composition or the pharmaceutical formulation in the manufacture of a medicament for treating or preventing a CDK9-mediated related disease. In particular, the CDK9-mediated related disease is cancer. Preferably, the cancer is solid tumor or hematological malignancies. More specifically, the cancer is

selected from adrenal tumor, melanoma, head and neck cancer, kidney cancer, bladder cancer, prostate cancer, endometrial carcinoma, cervical cancer, gastric carcinoma, colon cancer, pancreatic cancer, rectal cancer, esophageal cancer, liver cancer, lung cancer, sarcoma, breast cancer, ovarian cancer, non hodgkin's lymphoma, acute myeloid leukemia, acute lymphoblastic leukemia, myeloma.

Also, the compounds of the present invention show obvious inhibition effect on CDK9, and compared with CDK1, 2, 3 and 5, the compounds of the present invention have better selectivity for CDK9, which indicates that the compounds of the present invention has better clinical application potential in treating CDK9-mediated diseases, which can reduce the side effects caused by drug off-target.

## DETAILED DESCRIPTION OF THE INVENTION

The "halogen" described herein refers to fluorine, chlorine, bromine, iodine and the like, and preferably fluorine and chlorine.

The "halogenated" described herein means that any hydrogen atom in a substituent can be substituted with one or more identical or different halogen atoms. "Halogen" is defined as above.

The "$C_{1-6}$ alkyl" described herein refers to linear or branched alkyl derived by removing one hydrogen atom from a hydrocarbon moiety containing 1 to 6 carbon atoms, such as methyl, ethyl, *n*-propyl, isopropyl, *n*-butyl, isobutyl, *sec*-butyl, *tert*-butyl, *n*-pentyl, isopentyl, 2-methylbutyl, neopentyl, 1-ethylpropyl, *n*-hexyl, isohexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl and 1-methyl-2-methylpropyl. The "$C_{1-4}$ alkyl" refers to the aforementioned examples containing 1 to 4 carbon atoms.

The "$C_{2-8}$ alkenyl" described herein refers to linear, branched or cyclic alkenyl derived by removing one hydrogen atom from an alkene moiety containing 2 to 8 carbon atoms and a carbon-carbon double bond, such as vinyl, 1-propenyl, 2-propenyl, 1-butenyl, 2-butenyl, 1,3-butadienyl, 1-pentenyl, 2-pentenyl, 3-pentenyl, 1,3-pentadienyl, 1,4-pentadienyl, 1-hexenyl and 1,4-hexadienyl.

The "$C_{2-8}$ alkynyl" described herein refers to linear or branched alkynyl derived by removing one hydrogen atom from an alkyne moiety containing 2 to 8 carbon atoms and a carbon-carbon triple bond, such as ethynyl, propynyl, 2-butynyl, 2-pentynyl, 3-pentynyl, 4-methyl-2-pentynyl, 2-hexynyl and 3-hexynyl.

The "$C_{1-6}$ alkoxy" described herein refers to a group in which the "$C_{1-6}$ alkyl" defined above is linked to a parent molecule via an oxygen atom, i.e., a "$C_{1-6}$ alkyl-O-" group, such as methoxy, ethoxy, *n*-propoxy, isopropoxy, *n*-butoxy, *tert*-butoxy, *n*-pentyloxy, neopentyloxy and *n*-hexyloxy. The "$C_{1-4}$ alkoxy" refers to the aforementioned examples containing 1 to 4 carbon atoms, i.e., a "$C_{1-4}$ alkyl-O-" group.

The "$C_{1-6}$ alkylamino", "($C_{1-6}$ alkyl)$_2$amino", "$C_{1-6}$ alkylcarbonylamino", "$C_{1-6}$ alkylsulfonylamino", "$C_{1-6}$ alkylaminocarbonyl", "($C_{1-6}$ alkyl)$_2$aminocarbonyl", "$C_{1-6}$ alkoxycarbonyl", "$C_{1-6}$ alkylsulfonyl", "$C_{1-6}$ alkylthio", "$C_{1-6}$ alkylcarbonyl", "$C_{1-6}$ alkoxy $C_{1-6}$ alkoxy" and "$C_{1-6}$ alkylaminosulfonyl" described herein refer to $C_{1-6}$ alkyl-NH-, ($C_{1-6}$ alkyl)($C_{1-6}$alkyl)N-, $C_{1-6}$ alkyl-C(O)-NH-, $C_{1-6}$ alkyl-S(O)$_2$-NH$_2$-, $C_{1-6}$ alkyl-NH-C(O)-, ($C_{1-6}$ alkyl)($C_{1-6}$ alkyl)N-C(O)-, $C_{1-6}$ alkyl-O-C(O)-, $C_{1-6}$ alkyl-S(O)$_2$-, $C_{1-6}$ alkyl-S-, $C_{1-6}$ alkyl-C(O)-, $C_{1-6}$ alkyl-O- $C_{1-6}$ alkyl-O- and $C_{1-6}$ alkyl-S(O)$_2$-NH-, respectively, wherein the "$C_{1-6}$ alkyl" is defined as above, and is preferably "$C_{1-4}$ alkyl".

The "$C_{1-6}$ alkyl" in "$C_{1-6}$ alkyl-S(O)(=NH)-" is defined as above, and is preferably "$C_{1-4}$ alkyl".

The "polycyclic ring" described herein refers to a multi-ring system structure formed by two or more ring structures connected by an ortho-fused, spiro- or bridged linkage. The ortho-fused ring refers to a polycyclic structure formed by two or more ring structures sharing two adjacent ring atoms (i.e., sharing a bond) with each other. The bridged ring refers to a polycyclic structure formed by two or more ring structures sharing two non-adjacent ring atoms with each other. The spiro-ring refers to a polycyclic structure formed by two or more ring structures sharing a ring atom with each other.

Unless otherwise specified, the "3-12 membered cycloalkenyl" described herein includes all possibly formed monocyclic and polycyclic (including fused in the form of ortho-, spiro- or bridged) cases, such as 3-8 membered monocyclic cycloalkenyl, 7-11 membered spiro-cycloalkenyl, 7-11 membered ortho-fused cycloalkenyl and 6-11 membered bridged cycloalkenyl.

The cycloalkyl described herein includes all possibly formed monocyclic and polycyclic (including fused in the form of ortho-, spiro- or bridged) cases. For example, "3-12 membered cycloalkyl" can be a monocyclic, bicyclic or polycyclic cycloalkyl system (also referred to as a polycyclic ring system). Unless otherwise specified, the monocyclic ring system is a cyclic hydrocarbon group containing 3 to 8 carbon atoms. Examples of 3-8 membered cycloalkyl include, but are not limited to: cyclopropyl, cyclobutyl, cyclopentyl, cyclohexyl, cycloheptyl, cyclooctyl, bicyclo[1.1.1]pentyl and the like.

Polycyclic cycloalkyl includes ortho-fused cycloalkyl, bridged cycloalkyl and spiro-cycloalkyl.

Ortho-fused cycloalkyl may be 6-11 membered ortho-fused cycloalkyl or 7-10 membered ortho-fused cycloalkyl, and the representative examples thereof include, but are not limited to, bicyclo[3.1.1]heptane, bicyclo[2.2.1]heptane,

bicyclo[2.2.2]octane, bicyclo[3.2.2]nonane, bicyclo[3.3.1]nonane and bicyclo[4.2.1]nonane. The spiro-cycloalkyl may be 7-12 membered spiro-cycloalkyl or 7-11 membered spiro-cycloalkyl, and the examples thereof include, but are not limited to:

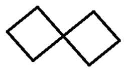,

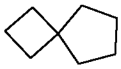, 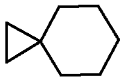, 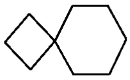, 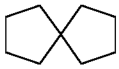, 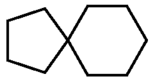 and 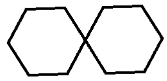.

The bridged cycloalkyl may be 6-11 membered bridged cycloalkyl or 7-10 membered bridged cycloalkyl, and the examples thereof include, but are not limited to:

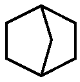,

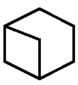, 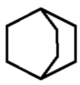, 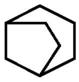, 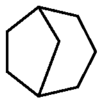 and 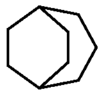.

The "heterocyclyl" described herein refers to a 3-12 membered non-aromatic cyclic group in which at least one ring carbon atom is replaced with a heteroatom selected from O, S and N, and preferably 1 to 3 heteroatoms are present, wherein a carbon atom, a nitrogen atom and a sulfur atom may be oxidized.

"3-12 membered heterocyclyl" refers to a monocyclic heterocyclyl, bicyclic heterocyclyl, or polycyclic heterocyclyl system (also referred to as a fused ring system), including saturated and partially saturated heterocyclyl groups, but excluding aromatic rings. Unless otherwise specified, all possibly formed monocyclic, polycyclic (including fused in the form of ortho-, spiro- or bridged), saturated, partially saturated cases are included.

The monocyclic heterocyclyl may be 3-8 membered heterocyclyl, 3-8 membered saturated heterocyclyl, 3-6 membered heterocyclyl, 4-7 membered heterocyclyl, 5-7 membered heterocyclyl, 5-6 membered heterocyclyl, 5-6 membered oxygen-containing heterocyclyl, 3-8 membered nitrogen-containing heterocyclyl, 5-6 membered nitrogen-containing heterocyclyl, 5-6 membered saturated heterocyclyl, or the like. Examples of the "3-8 membered saturated heterocyclyl" include, but are not limited to, aziridinyl, oxiranyl, thiiranyl, azetidinyl, oxetanyl, thietanyl, tetrahydrofuranyl, pyrrolidinyl, tetrahydrothienyl, imidazolidinyl, pyrazolidinyl, 1,2-oxazolidinyl, 1,3-oxazolidinyl, 1,2-thiazolidinyl, 1,3-thiazolidinyl, tetrahydro-2*H*-pyranyl, tetrahydro-2*H*-thiapyranyl, piperidinyl, piperazinyl, morpholinyl, 1,4-dioxanyl and 1,4-oxathianyl. Examples of the "3-8 membered partially saturated heterocyclyl" include, but are not limited to, 4,5-dihydroisoxazolyl, 4,5-dihydrooxazolyl, 2,5-dihydrooxazolyl, 2,3-dihydrooxazolyl, 3,4-dihydro-2*H*-pyrrolyl, 2,3-dihydro-1*H*-pyrrolyl, 2,5-dihydro-1*H*-imidazolyl, 4,5-dihydro-1*H*-imidazolyl, 4,5-dihydro-1*H*-pyrazolyl, 4,5-dihydro-3*H*-pyrazolyl, 4,5-dihydrothiazolyl, 2,5-dihydrothiazolyl, 2*H*-pyranyl, 4*H*-pyranyl, 2*H*-thiapyranyl, 4*H*-thiapyranyl, 2,3,4,5-tetrahydropyridinyl, 1,2-isoxazinyl, 1,4-isoxazinyl, 6*H*-1,3-oxazinyl and the like.Polycyclic heterocyclyl includes ortho-fused heterocyclyl, spiro-heterocyclyl and bridged heterocyclyl, which may be saturated, partially saturated or unsaturated, but non-aromatic. Polycyclic heterocyclyl may be 5-6 membered monocyclic heterocyclyl ring which is fused to a benzene ring, 5-6 membered monocyclic cycloalkyl, 5-6 membered monocyclic cycloalkenyl, 5-6 membered monocyclic heterocyclyl or 5-6 membered monocyclic heteroaryl. The ortho-fused heterocyclyl may be 6-12 membered ortho-fused heterocyclyl, 7-10 membered ortho-fused heterocyclyl, 6-10 membered ortho-fused heterocyclyl or 6-12 membered saturated ortho-fused heterocyclyl, and representative examples include, but are not limited to: 3-azabicyclo[3.1.0]hexyl, 3,6-diazabicyclo[3.2.0]heptyl, 3,8-diazabicyclo[4.2.0]octyl, 3,7-diazabicyclo[4.2.0]octyl, octahydropyrrolo[3,4-c]pyrrolyl, octahydropyrrolo[3,4-*b*]pyrrolyl, octahydropyrrolo[3,4-*b*][1,4]oxazinyl, octahydro-1*H*-pyrrolo[3,4-c]pyridinyl, 2,3-dihydrobenzofuran-2-yl, 2,3-dihydrobenzofuran-3-yl, indolin-1-yl, indolin-2-yl, indolin-3-yl, 2,3-dihydrobenzothiophen-2-yl, octahydro-1*H*-indolyl and octahydrobenzofuranyl. The spiro-heterocyclyl may be 6-12 membered spiro-heterocyclyl, 7-11 membered spiro-heterocyclyl or 6-12 membered saturated spiro-heterocyclyl, and examples thereof include, but are not limited to:

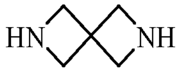, 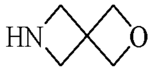, 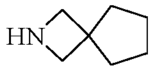, 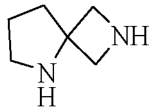,

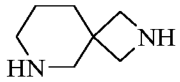, 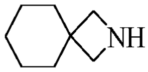, 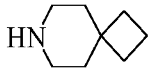, 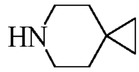, 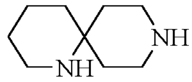, 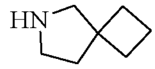,

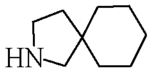, 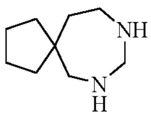, 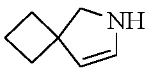, 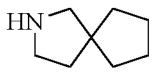, 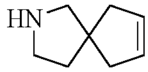, 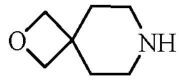,

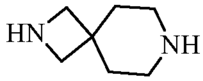, 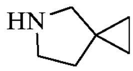 and 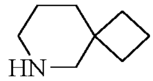.

The bridged heterocyclyl may be 6-12 membered bridged heterocyclyl, 7-11 membered bridged heterocyclyl or 6-12 membered saturated bridged heterocyclyl, and examples thereof include, but are not limited to

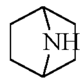, 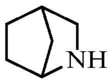, 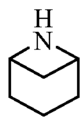, 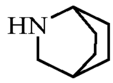,

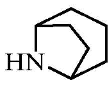, 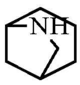, 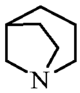, 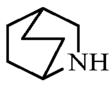, 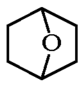, 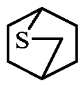, 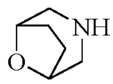, 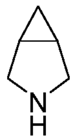

and

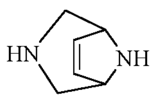.

The "3-12 membered heterocyclyloxy" described herein refers to a 3-12 membered heterocyclyl-O- group, wherein the "3-12 membered heterocyclyl" is defined as above.

The "3-12 membered cycloalkyloxy " described herein refers to a 3-12 membered cycloalkyl-O- group, wherein the "3-12 membered cycloalkyl" is defined as above.

The "3-12 membered heterocyclyl-$CH_2$-amino" described herein refers to a 3-12 membered heterocyclyl-$CH_2$-NH- group, wherein the "3-12 membered heterocyclyl" is defined as above.

The "3-12 membered cycloalkyl amino" described herein refers to a 3-12 membered cycloalkyl -NH- group, wherein the "3-12 membered cycloalkyl " is defined as above.

The "aryl" described herein refers to a cyclic aromatic group containing 6 to 14 carbon atoms, including phenyl, naphthalene, phenanthrene, and the like.

The heteroaryl described herein includes all possibly formed monocyclic, polycyclic, fully aromatic and partially aromatic cases. For example,"5-10 membered heteroaryl" refers to an aromatic cyclic group in which at least one ring carbon atom is substituted with a heteroatom selected from O, S and N, and preferably 1 to 3 heteroatoms are present. Moreover, the case where carbon atoms or sulfur atoms are oxidized is included. For example, carbon atoms are replaced with C(O) and sulfur atoms are substituted by S(O) or $S(O)_2$. Unless otherwise specified, heteroaryl includes monocyclic heteroaryl and polycyclic heteroaryl. Monocyclic heteroaryl may be 5-7 membered heteroaryl or 5-6 membered heteroaryl, and examples thereof include, but are not limited to, furanyl, imidazolyl, isoxazolyl, thiazolyl, isothiazolyl, oxadiazolyl,

oxazolyl, pyridinyl, pyridazinyl, pyrimidinyl, pyrazinyl, pyrazolyl, pyrrolyl, tetrazolyl, thiadiazolyl, thienyl, triazolyl and triazinyl. In certain examples, polycyclic heteroaryl refers to a group in which a monocyclic heteroaromatic ring is fused to phenyl, cycloalkenyl, heteroaryl, cycloalkyl or heterocyclyl. Polycyclic heteroaryl may be 8-12 membered ortho-fused heteroaryl or 9-10 membered ortho-fused heteroaryl, and examples include, but are not limited to, benzimidazolyl, benzofuranyl, benzothienyl, benzoxadiazolyl, benzothiadiazolyl, benzothiazolyl, cinnolinyl, 5,6-dihydroquinolin-2-yl, 5,6-dihydroisoquinolin-1-yl, furopyridinyl, indazolyl, indolyl, isoindolyl, isoquinolinyl, naphthyridinyl, purinyl, quinolinyl, 5,6,7,8-tetrahydroquinolin-2-yl, 5,6,7,8-tetrahydroquinolinyl, 5,6,7,8-tetrahydroquinolin-4-yl, 5,6,7,8-tetrahydroisoquinolin-1-yl, thienopyridinyl, 4,5,6,7-tetrahydro[*c*][1,2,5]oxadiazolyl and 6,7-dihydro[*c*][1,2,5]oxadiazol-4(5*H*)keto.

The "pharmaceutically acceptable salt" described herein refers to a pharmaceutically acceptable addition salt of acid and base or a solvate thereof. Such pharmaceutically acceptable salts include salts of the following acids: hydrochloric acid, phosphoric acid, hydrobromic acid, sulfuric acid, sulfurous acid, formic acid, toluenesulfonic acid, methanesulfonic acid, nitric acid, benzoic acid, citric acid, tartaric acid, maleic acid, hydroiodic acid, alkanoic acid (such as acetic acid, HOOC-$(CH_2)$n-COOH (wherein n is 0-4)), and the like. The following salts of bases are also included: sodium salt, potassium salt, calcium salt, ammonium salt, and the like. Those skilled in the art know a variety of pharmaceutically acceptable non-toxic addition salts.

The "isomer" described herein refers to a stereoisomer and a tautomer.

The stereoisomer refers to an enantiomer in the case that atoms are asymmetric in a compound, and a cis-trans isomer in the case that a double bond or a cyclic structure exists in a compound. All enantiomers, diastereomers, racemic isomers, cis-trans isomers, geometric isomers, epimers and mixtures thereof of the compound of formula (I) are included in the scope of the present invention.

The "tautomer" means a functional group isomer that is produced due to the rapid shifting of a certain atom between two positions in a molecule, and the tautomer is a special functional group isomer. Examples include tautomerization of a carbonyl compound containing $\alpha$-H, specifically as follows:

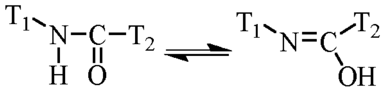

and

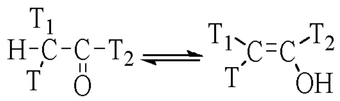

The "tautomer" may be, for example, other prototropic tautomers, specifically such as a phenol-keto tautomer, a nitroso-oximino tautomer and an imine-enamine tautomer.

T, $T_1$ and $T_2$ are each independently selected from any group meeting the bonding rule of compounds.

In one embodiment of the present invention, L is preferably bond.

In one embodiment of the present invention, A is preferably 3-12 membered cycloalkyl or 3-12 membered heterocyclyl. The 3-12 membered cycloalkyl of A is preferably cyclobutyl. The 3-12 membered heterocyclyl of A is preferably piperidinyl, azepan-3-yl,

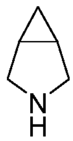, or 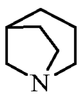.

In one embodiment of the present invention, $R_1$ is preferably hydrogen, hydroxyl, $C_{1-6}$ alkyl, $C_{1-6}$ alkoxy, amino, $(C_{1-6}$ alkyl$)_2$ amino, $C_{1-6}$ alkylcarbonyl or 3-12 membered heterocyclyl. The $C_{1-6}$ alkyl of $R_1$ is preferably methyl, methyl substituted with amino, methyl substituted with hydroxyl, or methyl substituted with dimethylaminocarbonyl. The $C_{1-6}$ alkoxy of $R_1$ is preferably methoxy. The amino of $R_1$ is preferably amino substituted with methyl. The $(C_{1-6}$ alkyl$)_2$ amino of $R_1$ is preferably dimethylamino. The $C_{1-6}$ alkylcarbonyl of $R_1$ is preferably acethyl. The 3-12 membered heterocyclyl of $R_1$ is preferably

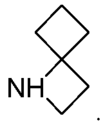

In one embodiment of the present invention, $R_2$ is preferably amino, $C_{1-6}$ alkylamino, or 3-12 membered heterocyclyl. The amino of $R_2$ is preferably amino, amino substituted with furan, amino substituted with pyran, amino substituted with bicyclo[1.1.1]pentyl. The $C_{1-6}$ alkylamino of $R_2$ is preferably ter-butylamino, isopropylamino, isopropylamino substituted with methoxy, propylamino, sec-butylamino, ethylamino, or ethylamino substituted with cyclopropyl. The 3-12 membered heterocyclyl of $R_2$ is preferably piperidinyl.

In one embodiment of the present invention, $R_3$ is preferably cyano, $C_{1-6}$ alkyl, or amino carbonyl. The $C_{1-6}$ alkyl of $R_3$ is preferably methyl, difluoromethyl or trifluoromethyl.

In one embodiment of the present invention, $R_6$ is preferably hydrogen.

In one embodiment of the present invention, n is preferably 0.

In one embodiment of the present invention, m is preferably 1 or 2.

The "optionally substituted by one or more groups" means optionally substituted by one groups, optionally substituted by two groups, optionally substituted by three groups, optionally substituted by four groups, or optionally substituted by five groups.

## Examples

List of abbreviations:

LDA: lithium diisopropylamide; THF: tetrahydrofuran; EA: ethyl acetate; PE: petroleum ether; DIPEA: *N*,*N*-diisopropylethylamine; DCM: dichloromethane; DMSO: dimethyl sulfoxide; IBX: 2-iodoxybenzoic acid; DMAC: *N*,*N*-dimethylacetamide; DMF: *N*,*N*-dimethylformamide; MTBE: methyl *tert*-butyl ether; BINAP: (±)-2,2'-bis-(diphenylphosphino)-1,1'-dinaphthalene; TFA: trifluoroacetic acid; DAST: diethylaminosulfur trifluoride; HATU: 1-[Bis(dimethylamino)methylene]-1H-1,2,3-triazolo[4,5-b]pyridinium 3-Oxide Hexafluorophosphate; NCS: N-chlorosuccinimide; DCE: dichloroethane; mCPBA: *m*-chloroperoxybenzoic acid; and DMA: dimethylacetamide.

### Example 1: Synthesis of (1*S*,3*R*)-3-acetamido-*N*-(7-(difluoromethyl)-5-(isopropylamino)-2,6-naphthyridin-3-yl)cyclohexane-1-carboxamide (compound 1)

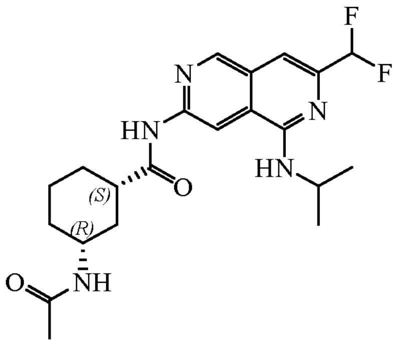

Steps:

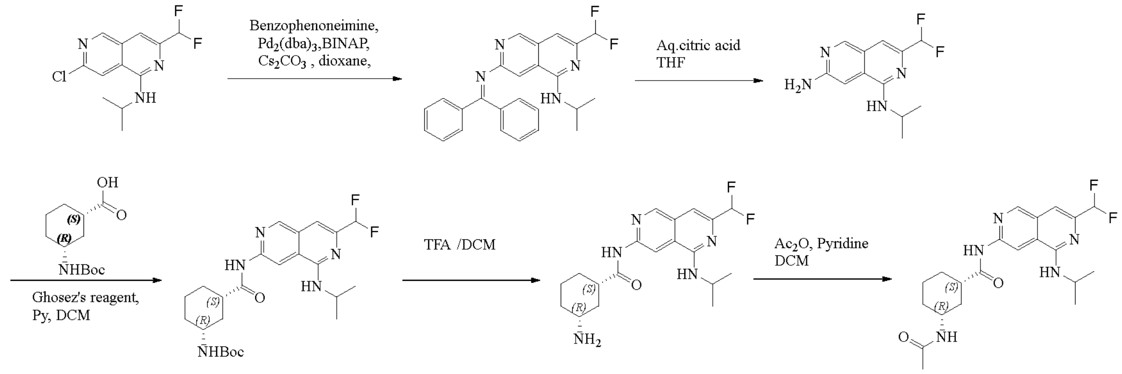


Step 1: synthesis of 3-(difluoromethyl)-7-((diphenylmethylene)amino)-*N*-isopropyl-2,6-naphthyridin-1 -amine

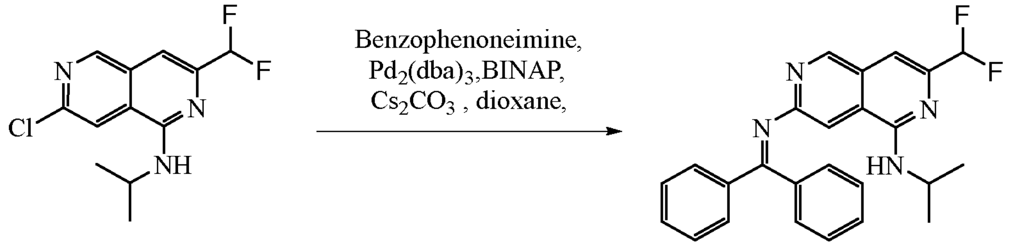


7-chloro-3-(difluoromethyl)-*N*-isopropyl-2,6-naphthyridin-1-amine (1150 mg, 4.2 mmol, 1.0 eq.), diphenylmethanimine (0.86 mL, 1.2 mmol, 1.2 eq.), $Pd_2(dba)_3$ (389 mg, 0.42 mmol, 0.1 eq.), BINAP (528 mg, 0.85 mmol, 0.2 eq.) and cesium carbonate (4148 mg, 12.7 mmol, 3.0 eq.) were added into 1,4-dioxane (30 mL). The mixture was reacted at 120 °C for overnight in a sealed tube in nitrogen atmosphere. When there were no materials left, as detected by TLC, the reaction solution was added with ethyl acetate (80 mL), stirred for 5 min and filtered, the filter cake was rinsed with ethyl acetate, and the filtrate was concentrated under reduced pressure to give a crude product, which was purified by silica gel column chromatography to give the product (1450 mg, yield: 82.1%).

Step 2: synthesis of 3-(difluoromethyl)-*N*1-isopropyl-2,6-naphthyridine-1,7-diamine

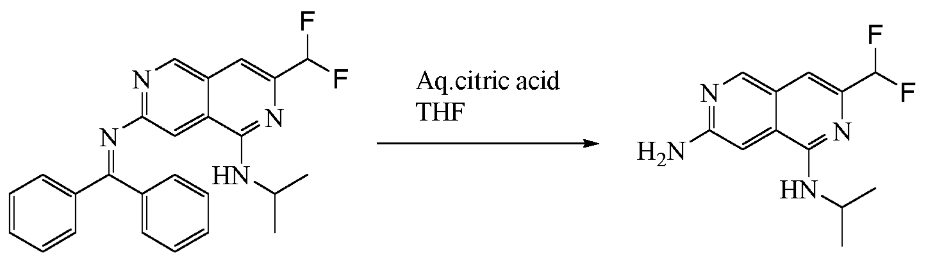


3-(difluoromethyl)-7-((diphenylmethylene)amino)-N-isopropyl-2,6-naphthyridin-1-amine (1600 mg, 3.85 mmol, 1.0 eq.) was dissolved in THF (20 mL). The reaction solution was added with 2N aqueous citric acid solution (10 mL) and reacted at room temperature for 3 h. When there were no materials left, as detected by TLC, the solvent was removed under reduced pressure and compound was extracted with ethyl acetate (30 mL × 3). The organic phases were combined, dried over anhydrous sodium sulfate and filtered, and the filtrate was concentrated under reduced pressure to give a crude product, which was purified by silica gel column chromatography (DCM:MeOH = 98:2-95:5) to give the product (990 mg,) which has some impurities .

Step 3: synthesis of *tert*-butyl ((1*R*,3*S*)-3-((7-(difluoromethyl)-5-(isopropylamino)-2,6-naphthyridin-3-yl)carbamoyl)cyclohexyl)carbamate

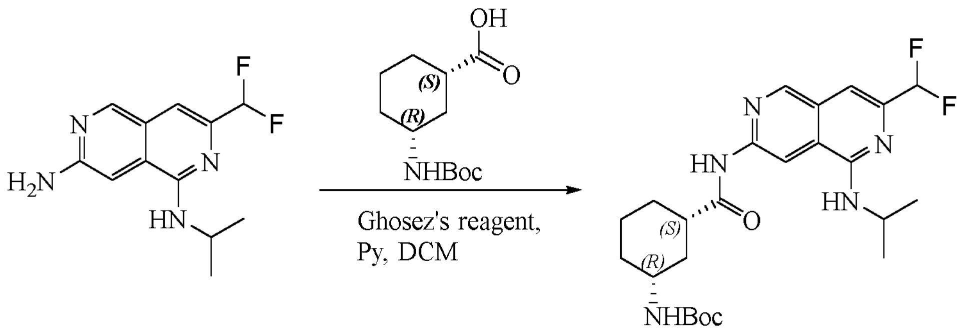


(1*S*,3*R*)-3-((*tert*-butoxycarbonyl)amino)cyclohexane-1-carboxylic acid (482.1 mg, 1.98 mmol, 1.0 eq.) was dissolved in DCM (10 mL). The reaction solution was cooled to 0 °C to 5 °C, added with 1-chloro-*N*,*N*,2-trimethylprop-1-en-1-amine (316 mg, 2.38 mmol, 1.2 eq.), reacted at 0 °C to 5 °C for 1.5 h, added with a solution of 3-(difluoromethyl)-N1-isopropyl-2,6-naphthyridine-1,7-diamine (500 mg, 1.98 mmol, 1.0 eq.) and pyridine (0.48 mL, 5.95 mmol, 3.0 eq.) in DCM (5 mL). The reaction mixture was stirred at rt for overnight. When LCMS showed SM was consumed, the reaction solution was added with water (20 mL) and extracted with DCM (20 mL × 3). The organic phases were combined, washed with brine (20 mL) successively, concentrated under reduced pressure to give a crude product, which was purified by silica gel column chromatography to give the product (478 mg, yield: 71.8%).

Step 4: synthesis of (1*S*,3*R*)-3-amino-*N*-(7-(difluoromethyl)-5-(isopropylamino)-2,6-naphthyridin-3-yl)cyclohexane-1-carboxamide

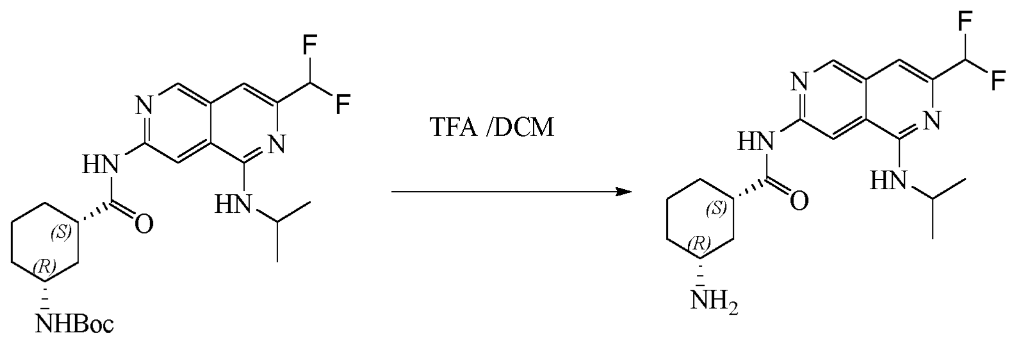


*tert*-Butyl ((1*R*,3*S*)-3-((7-(difluoromethyl)-5-(isopropylamino)-2,6-naphthyridin-3-yl)carbamoyl)cyclohexyl)carbamate (180 mg, 0.38 mmol, 1.0 eq.) was dissolved in DCM (3 mL). The reaction solution was cooled down in an ice water bath, added with TFA (2 mL) and reacted at room temperature for 4 h. When there were no materials left, as detected by TLC, the reaction solution was concentrated under reduced pressure. The crude was used in the next step.

Step 5: synthesis of (1S,3R)-3-acetamido-*N*-(7-(difluoromethyl)-5-(isopropylamino)-2,6-naphthyridin-3-yl)cyclohexane-1-carboxamide

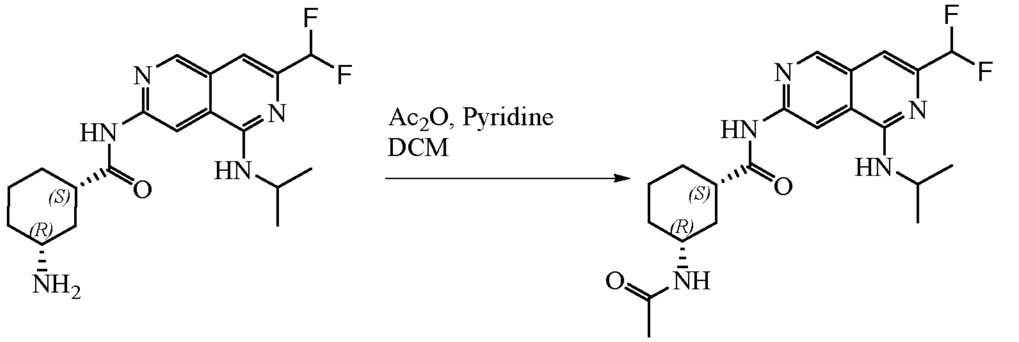


(1*S*,3*R*)-3-amino-*N*-(7-(difluoromethyl)-5-(isopropylamino)-2,6-naphthyridin-3-yl)cyclohexane-1-carboxamide (143 mg, 0.38 mmol, 1.0 eq.) was dissolved in DCM (3 mL). The reaction solution was cooled down in an ice water bath, added with pyridine (0.3mL, 3.78 mmol, 10.0 eq.) and acetic anhydride (0.047 mL, 0.49 mmol, 1.3 eq.) and reacted at room temperature for 3 h. When there were no materials left, as detected by LC-MS, the reaction solution was poured into water (20 mL) and extracted with ethyl acetate (20 mL × 3). The organic phases were combined, dried over anhydrous sodium sulfate and filtered. The filtrate was concentrated under reduced pressure to give a crude product, which was purified by silica gel column chromatography (DCM:MeOH = 98:2-95:5) to give the product (40 mg, yield: 26%,).
$^1$HNMR(400 MHz, DMSO) δ(ppm):10.6 (s, 1H), 9.02 (s, 1H), 8.64 (s, 1H), 7.80 (d, 1H), 7.55 (d, 1H), 7.24 (s, 1H), 6.78 (t, 1H), 4.47-4.38 (m, 1H), 3.62-3.59 (m, 1H), 2.71-2.63 (m, 1H), 1.96-1.93 (m, 1H), 1.80-1.78 (m, 6H), 1.36-1.11 (m, 9H), 1.12-108 (m, 1H).
Molecular formula: $C_{21}H_{27}F_2N_5O_2$ Exact mass: 419.21 LC-MS (Pos, m/z)=420.4 $[M+H]^+$.

**Example 2: Synthesis of (1*S*,3*R*)-3-acetamido-*N*-(7-cyano-5-(isopropylamino)-2,6-naphthyridin-3-yl)cyclohexane-1-carboxamide (compound 2)**

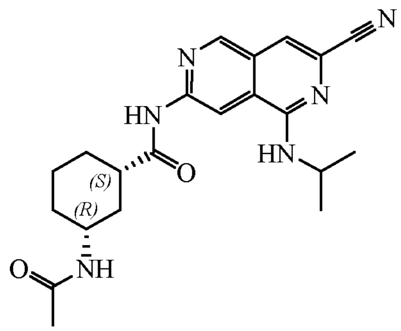

Steps:

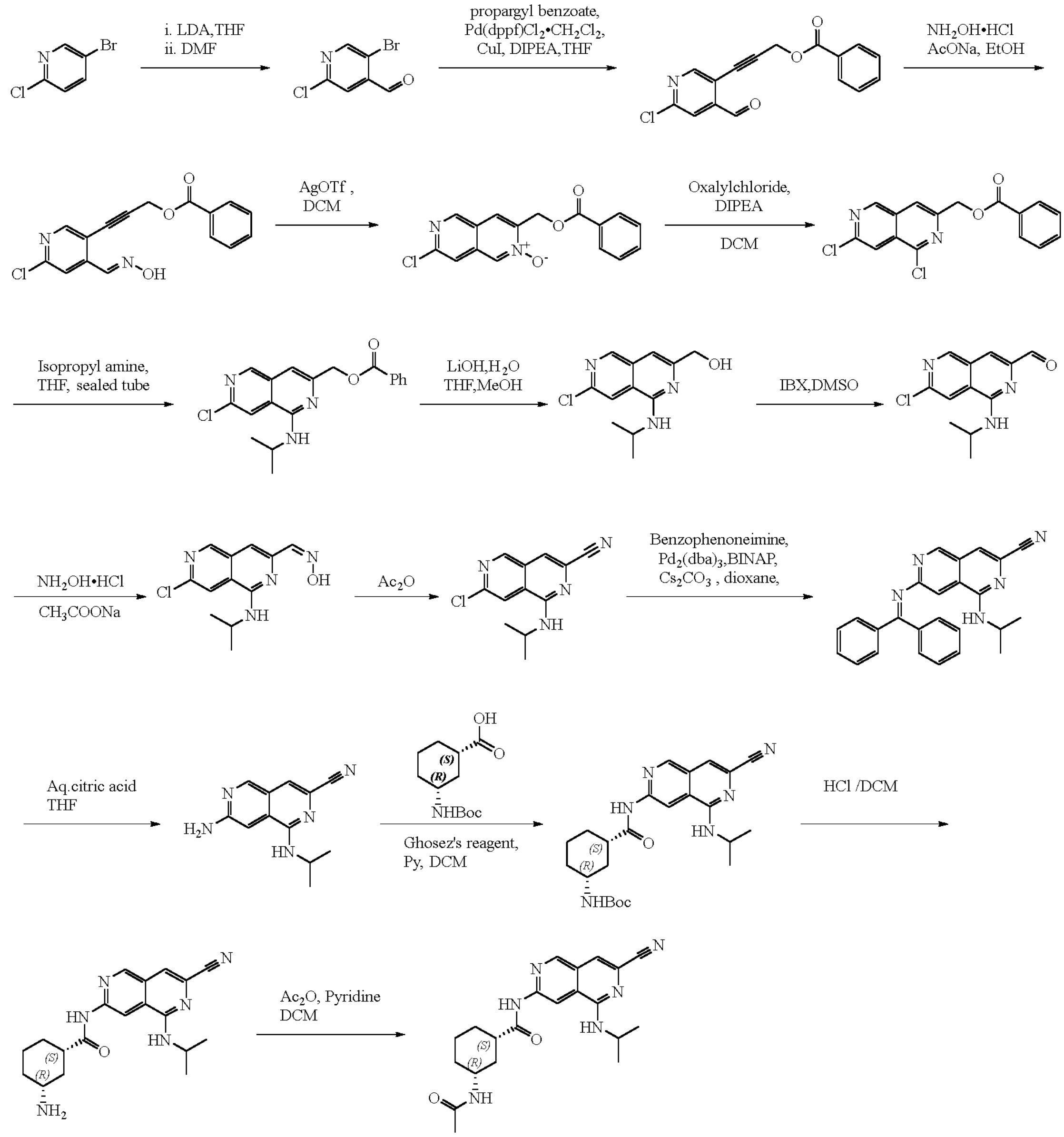


Step 1: synthesis of 5-bromo-2-chloroisonicotinaldehyde

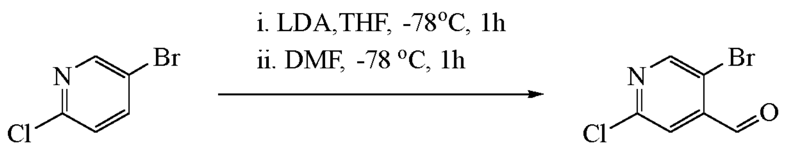


A solution of LDA (2 mol/L) in THF (58.5 mL, 116.93 mmol, 1.5 eq.) was added into anhydrous THF (50 mL). The reaction solution was cooled to -70 °C in nitrogen atmosphere, added dropwise with a solution of 5-bromo-2-chloropyridin (15 g, 77.95 mmol, 1.0 eq.) in anhydrous THF (100 mL), and reacted at -70 °C to -65 °C for 0.5 h after 1 h of addition. The reaction solution was added dropwise with DMF (18 mL, 233.85 mol, 3.0 eq.) and reacted at -70 °C to -65 °C for 1 h after addition. When there were no materials left, as detected by TLC, the reaction solution was added dropwise to a saturated ammonium solution (500 mL), stirred for 10 min, dissolved in water (100 mL) and extracted with ethyl acetate (500 mL × 2). The organic phases were combined, dried over anhydrous sodium sulfate and filtered, and the filtrate was concentrated under reduced pressure to give a crude product, which was purified by silica gel column

chromatography (EA:PE = 1:40) to give the product (10.6 g, yield: 62.3%).

Step 2: synthesis of 3-(6-chloro-4-formylpyridin-3-yl)prop-2-yn-1-yl benzoate

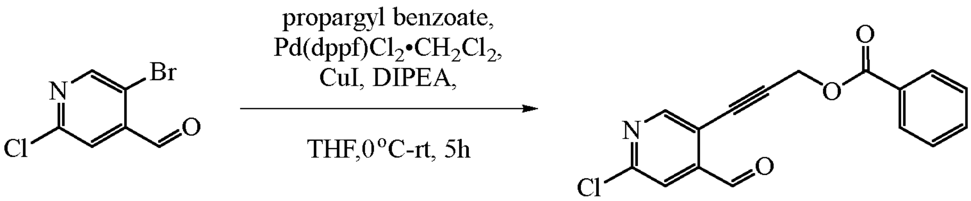


DIPEA (16.5 g, 127.92 mmol, 3.0 eq.), Pd(dppf)$Cl_2CH_2Cl_2$ (2.5 g, 3.14 mmol, 0.08 eq.) and CuI (811.3 mg, 4.26 mmol, 0.1 eq.) were added into anhydrous THF (100 mL). The mixture was stirred for 10 min in nitrogen atmosphere, added with a solution of 5-bromo-2-chloroisonicotinaldehyde (9.4 g, 42.64 mmol, 1.0 eq.) in anhydrous THF (50 mL), cooled down in an ice water bath after addition, and added with a solution of propargyl benzoate (8.2 g, 51.17 mmol, 1.2 eq.) in anhydrous THF (50 mL). After addition, the mixture was incubated at room temperature for 22 h. When there were no materials left, as detected by TLC, the reaction solution was added with water (200 mL), stirred for 10 min, and filtered. The filter cake was rinsed by ethyl acetate, the liquid separation was performed, the organic phase was retained, and the aqueous phase was extracted with ethyl acetate (100 mL × 2). The organic phases were combined, dried over anhydrous sodium sulfate and filtered, and the filtrate was concentrated under reduced pressure to give a crude product, which was purified by silica gel column chromatography (EA:PE = 1:30-1:10) to give the product (5.8 g, yield: 45.6%).

Step 3: synthesis of (*E*)-3-(6-chloro-4-((hydroxyimino)methyl)pyridin-3-yl)prop-2-yn-1-yl benzoate

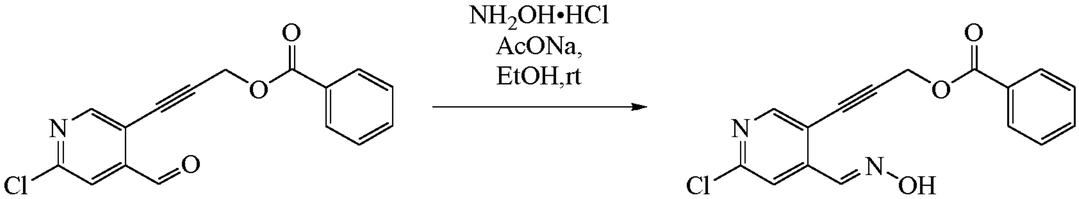


(*E*)-3-(6-chloro-4-((hydroxyimino)methyl)pyridin-3-yl)prop-2-yn-1-yl benzoate (5.5 g, 18.35 mmol, 1.0 eq.) was dissolved in absolute ethanol (800 mL). The reaction solution was added with hydroxylamine hydrochloride (1.9 g, 27.53 mmol, 1.5 eq.) and sodium acetate (2.2 g, 27.53 mmol, 1.5 eq.), and reacted at room temperature for 5 h. When there were no materials left, as detected by TLC, the reaction solution was concentrated under reduced pressure, added with water (50 mL) and extracted with ethyl acetate (50 mL × 3). The organic phases were combined, dried over anhydrous sodium sulfate and filtered, and the filter cake was concentrated under reduced pressure to give the product (5.57 g, yield: 96.5%).

Step 4: synthesis of 3-((benzoyloxy)methyl)-7-chloro-2,6-naphthyridine 2-oxide

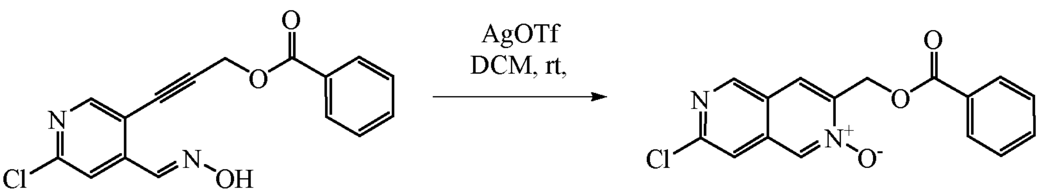


(*E*)-3-(6-chloro-4-((hydroxyimino)methyl)pyridin-3-yl)prop-2-yn-1-yl benzoate (5.57 g, 17.70 mmol, 1.0 eq.) was

dissolved in DCM (80 mL). The reaction solution was added with silver trifluoromethanesulfonate (454.7 mg, 1.77 mmol, 0.1 eq.) and reacted at room temperature for 15h. When there were a small amount of materials left, as detected by TLC, the reaction solution was concentrated under reduced pressure, slurried with diethyl ether (100 mL) for 0.5 h and filtered, and the filter cake was rinsed with diethyl ether (50 mL) and dried to give the product (4.8 g, yield: 86.1%).

Step 5: synthesis of (1,7-dichloro-2,6-naphthyridin-3-yl)methyl benzoate

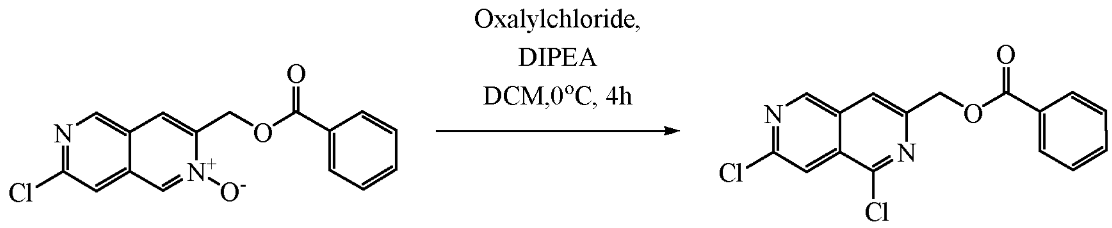


3-((benzoyloxy)methyl)-7-chloro-2,6-naphthyridine 2-oxide (4.8 g, 15.25 mmol, 1.0 eq.) and DIPEA(5.9 g, 45.75 mmol, 3.0 eq.) were dissolved in DCM (100 mL). The reaction solution was cooled to 0 °C to 5 °C, added dropwise with oxalyl chloride (3.87 g, 30.50 mmol, 2.0 eq.) and reacted for 2 h. When a large number of materials were left, as detected by TLC, the reaction solution was supplemented with DIPEA (5.9 g, 45.75 mmol, 3.0 eq.) and oxalyl chloride (3.87 g, 30.50 mmol, 2.0 eq.) and reacted for 1 h. When there were no materials left, as detected by TLC, the reaction solution was added with water (100 mL), concentrated under reduced pressure and extracted with ethyl acetate (200 mL × 2). The organic phases were combined, dried over anhydrous magnesium sulfate and filtered, and the filtrate was concentrated under reduced pressure to give a crude product, which was purified by silica gel column chromatography (EA:PE = 1:30-1:20) to give the product (3.5 g, yield: 70%).

Step 6: synthesis of (7-chloro-1-(isopropylamino)-2,6-naphthyridin-3-yl)methyl benzoate

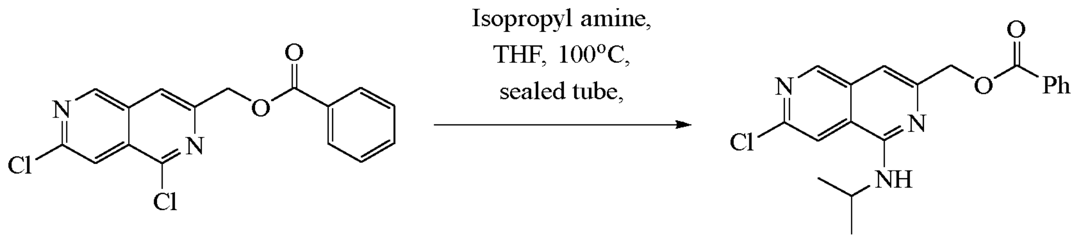


(1,7-dichloro-2,6-naphthyridin-3-yl)methyl benzoate (3.5 g, 10.51 mmol, 1.0 eq.) and isopropylamine (7.4 g, 126.12 mmol, 12.0 eq.) were added into anhydrous THF (20 mL). The mixture was reacted at 100 °C for 6 h in a sealed tube. When there were no materials left, as detected by TLC, the reaction solution was concentrated under reduced pressure to give a light yellow product, which was used in the next step according to a theoretical amount.

Step 7: synthesis of (7-chloro-1-(isopropylamino)-2,6-naphthyridin-3-yl)methanol

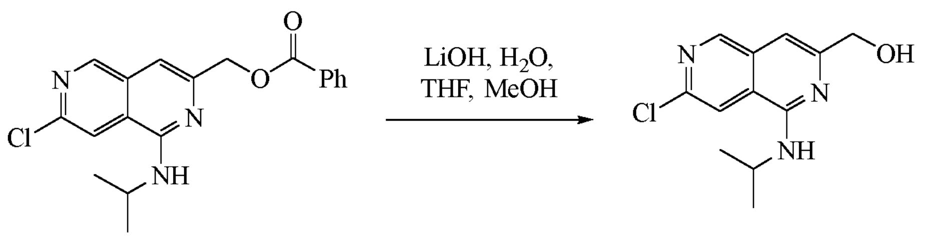

(7-chloro-1-(isopropylamino)-2,6-naphthyridin-3-yl)methyl benzoate (10.51 mmol, 1.0 eq.) and lithium hydroxide monohydrate (1.3 g, 31.53 mmol, 3.0 eq.) were dissolved in a mixed solution of THF (30 mL), methanol (15 mL) and water (15 mL), and the reaction solution was reacted at room temperature for 16 h. When there were no materials left, as detected by TLC, the reaction solution was concentrated under reduced pressure, added with water (30 mL) and extracted with ethyl acetate (30 mL × 2). The organic phases were combined, dried over anhydrous sodium sulfate and filtered, and the filtrate was concentrated under reduced pressure to give a crude product, which was purified by silica gel column chromatography (EA:PE = 1:20-1:6) to give the product (2.48 g, two-step yield: 95.3%).

Step 8: synthesis of 7-chloro-1-(isopropylamino)-2,6-naphthyridine-3-carbaldehyde

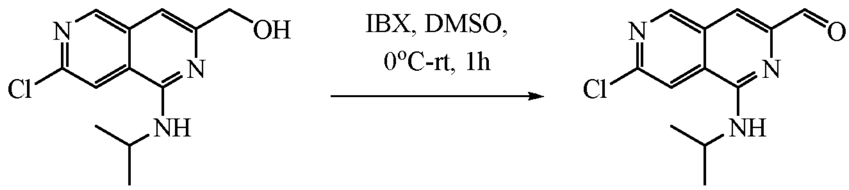


(7-chloro-1-(isopropylamino)-2,6-naphthyridin-3-yl)methanol (2.48 g, 9.85 mmol, 1.0 eq.) was dissolved in DMSO (25 mL), and the reaction solution was added with IBX (4.1 g, 14.77 mmol, 1.5 eq.) and reacted at room temperature for 1 h. When there were no materials left, as detected by TLC, the reaction solution was poured into water (200 mL) and extracted with ethyl acetate (100 mL × 2). The organic phases were combined, dried over anhydrous sodium sulfate and filtered, and the filtrate was concentrated under reduced pressure to give the product, which was used in the next step according to a theoretical amount.

Step 9: synthesis of (Z)-7-chloro-1-(isopropylamino)-2,6-naphthyridine-3-carbaldehyde oxime

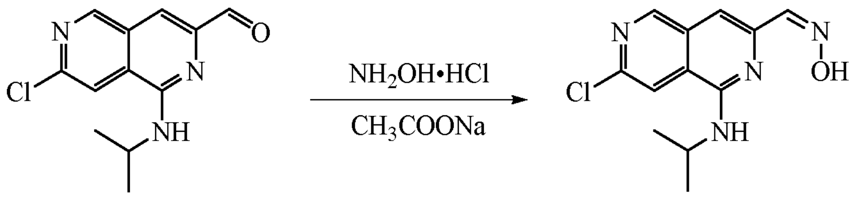


7-chloro-1-(isopropylamino)-2,6-naphthyridine-3-carbaldehyde (9.85 mmol, 1.0 eq.) was dissolved in absolute ethanol (25 mL). The mixture was added with hydroxylamine hydrochloride (1.03 g, 14.77 mmol, 1.5 eq.) and sodium acetate (1.2 g, 14.77 mmol, 1.5 eq.), and reacted at room temperature for 16 h. When there were no materials left, as detected by TLC, the reaction solution was concentrated under reduced pressure, added with water (50 mL) and extracted with ethyl acetate (50 mL × 2). The organic phases were combined, dried over anhydrous sodium sulfate and filtered, and the filtrate was concentrated under reduced pressure to give the product (2.1 g, two-step yield: 80.7%).

Step 10: synthesis of 7-chloro-1-(isopropylamino)-2,6-naphthyridine-3-carbonitrile

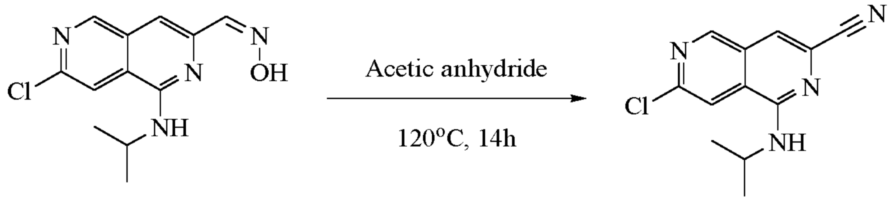

(Z)-7-chloro-1-(isopropylamino)-2,6-naphthyridine-3-carbaldehyde oxime (2.1 g, 7.93 mmol, 1.0 eq.) was dissolved in acetic anhydride (10 mL). The reaction solution was heated to 120 °C and reacted for 15 h. When there were no materials left, as detected by TLC, the reaction solution was concentrated under reduced pressure, added with water (20 mL) and extracted with ethyl acetate (20 mL × 3). The organic phases were combined, dried over anhydrous sodium sulfate and filtered, and the filtrate was concentrated under reduced pressure to give a crude product, which was purified by silica gel column chromatography (EA:PE = 1:20-1:15) to give the product (522 mg, yield: 27.4%).

Step 11: synthesis of 7-((diphenylmethylene)amino)-1-(isopropylamino)-2,6-naphthyridine-3-carbonitrile

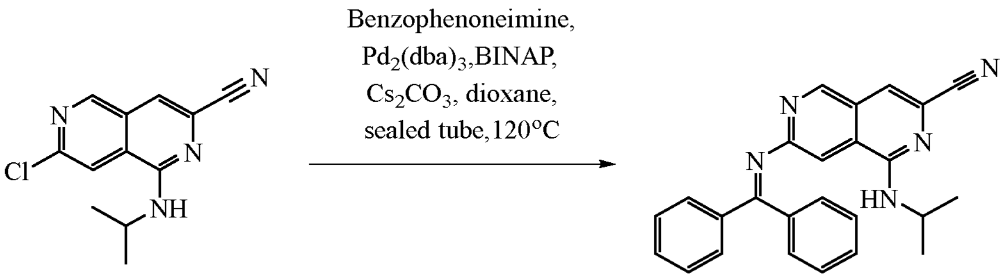


7-chloro-1-(isopropylamino)-2,6-naphthyridine-3-carbonitrile (520 mg, 2.11 mmol, 1.0 eq.), diphenylmethanimine (458.9 mg, 2.53 mmol, 1.2 eq.), $Pd_2(dba)_3$ (192.3 mg, 0.21 mmol, 0.1 eq.), BINAP (262.8 mg, 0.42 mmol, 0.2 eq.) and cesium carbonate (1.7 g, 5.27 mmol, 2.5 eq.) were added into 1,4-dioxane (15 mL). The mixture was reacted at 120 °C for 23 h in a sealed tube in nitrogen atmosphere. When there were no materials left, as detected by TLC, the reaction solution was filtered, and the filtrate was concentrated under reduced pressure to give the product, which was used in the next step according to a theoretical amount.

Step 12: synthesis of 7-amino-1-(isopropylamino)-2,6-naphthyridine-3-carbonitrile

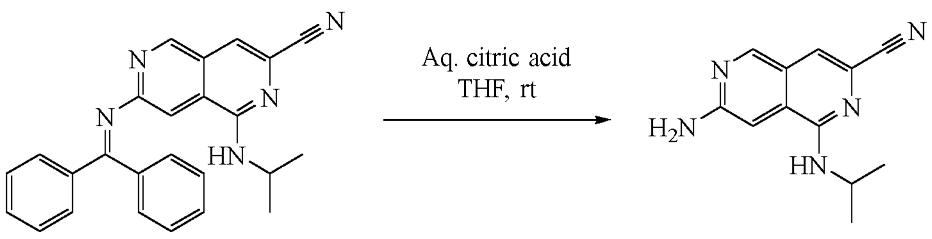


7-((diphenylmethylene)amino)-1-(isopropylamino)-2,6-naphthyridine-3-carbonitrile (2.11 mmol, 1.0 eq.) was dissolved in THF (10 mL). The reaction solution was added with aqueous citric acid solution (mass fraction: 10%, 5 mL) and reacted at room temperature for 23 h. When there were no materials left, as detected by TLC, the reaction solution was added with water (20 mL) and extracted with ethyl acetate (15 mL × 3). The organic phases were combined, dried over anhydrous sodium sulfate and filtered, and the filtrate was concentrated under reduced pressure to give a crude product, which was purified by silica gel column chromatography (EA:PE = 1:5-1:1) to give the product (310 mg, two-step yield: 64.6%).

Step 13: synthesis of *tert*-butyl ((1*R*,3*S*)-3-((7-cyano-5-(isopropylamino)-2,6-naphthyridin-3-yl)carbamoyl)cyclohexyl)carbamate

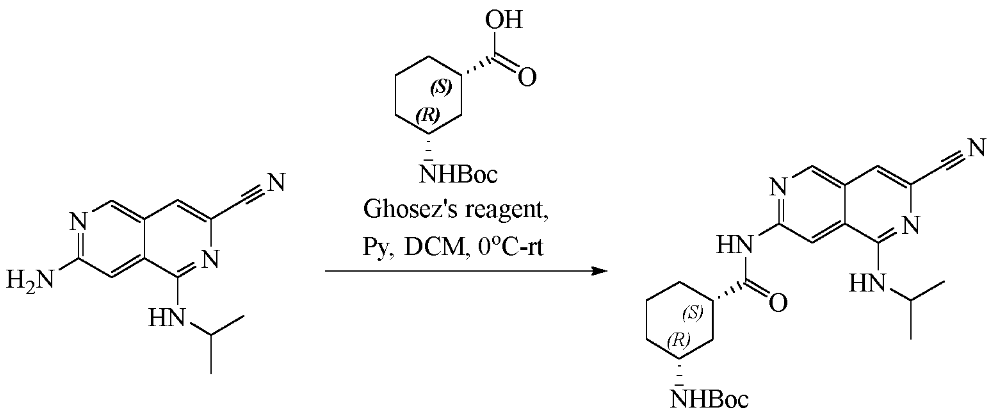


(1S,3R)-3-((*tert*-butoxycarbonyl)amino)cyclohexane-1-carboxylic acid (192.2 mg, 0.79 mmol, 1.05 eq.) was dissolved in DCM (3 mL). The reaction solution was cooled to 0 °C to 5 °C, added with 1-chloro-*N*,*N*,2-trimethylprop-1-en-1-amine (mass fraction: 95%, 157.5 mg, 1.12 mmol, 1.5 eq.), reacted at 0 °C to 5 °C for 1.5 h, added with a solution of 7-amino-1-(isopropylamino)-2,6-naphthyridine-3-carbonitrile (170 mg, 0.75 mmol, 1.0 eq.) and pyridine (177.9 mg, 2.25 mmol, 3.0 eq.) in THF (3 mL), and incubated at room temperature for 19 h. When there were a small amount of materials left, as detected by TLC, the reaction solution was added with water (20 mL) and extracted with ethyl acetate (20 mL × 3). The organic phases were combined, washed with water (20 mL) and brine (20 mL) successively, concentrated under reduced pressure to give a crude product, which was purified by silica gel column chromatography (EA:PE = 1:3) to give the product (300 mg, yield: 88.4%).

Step 14: synthesis of (1*S*,3*R*)-3-amino-*N*-(7-cyano-5-(isopropylamino)-2,6-naphthyridin-3-yl)cyclohexane-1-carboxamide

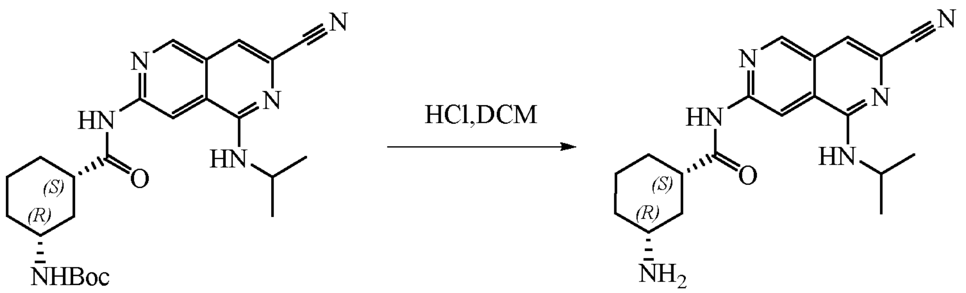


Tert-butyl ((1*R*,3*S*)-3-((7-cyano-5-(isopropylamino)-2,6-naphthyridin-3-yl)carbamoyl)cyclohexyl)carbamate (300 mg, 0.66 mmol, 1.0 eq.) was dissolved in DCM (10 mL). The reaction solution was cooled down in an ice water bath, added with hydrogen chloride ethanol solution (10 mol/L, 2 mL) and reacted at room temperature for 20 h. When there were no materials left, as detected by TLC, the reaction solution was concentrated under reduced pressure, slurried with ethyl acetate (20 mL) for 10 min and filtered. The filter cake was dissolved in water (50 mL) and back-extracted with ethyl acetate (30 mL × 2). The aqueous phase was retained, adjusted to pH of about 8 with sodium bicarbonate and extracted with ethyl acetate (30 mL × 3). The organic phases were combined, dried over anhydrous sodium sulfate and filtered, and the filtrate was concentrated under reduced pressure to give the product (128 mg, yield: 54.8%).

Step 15: synthesis of (1*S*,3*R*)-3-acetamido-*N*-(7-cyano-5-(isopropylamino)-2,6-naphthyridin-3-yl)cyclohexane-1-carboxamide

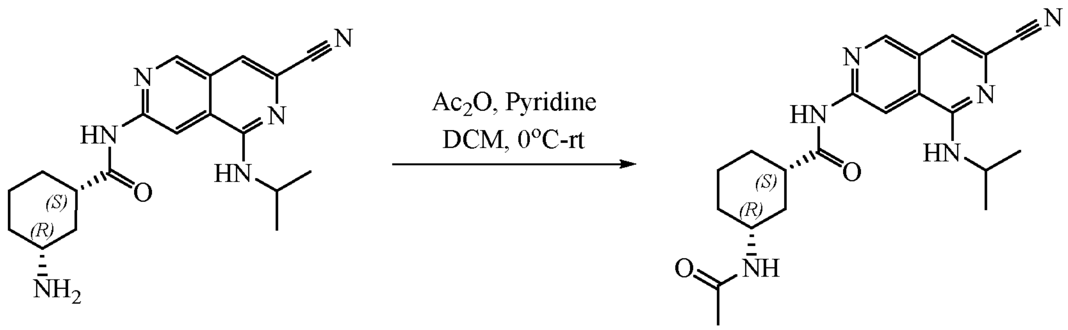


(1*S*,3*R*)-3-amino-*N*-(7-cyano-5-(isopropylamino)-2,6-naphthyridin-3-yl)cyclohexane-1-carboxamide (128 mg, 0.36 mmol, 1.0 eq.) was dissolved in DCM (3 mL). The reaction solution was cooled down in an ice water bath, added with DIPEA (139.5 mg, 1.08 mmol, 3.0 eq.) and acetic anhydride (55 mg, 0.54 mmol, 1.5 eq.) and reacted at room temperature for 3 h. When there were no materials left, as detected by LC-MS, the reaction solution was poured into water (20 mL) and extracted with ethyl acetate (20 mL × 3). The organic phases were combined, dried over anhydrous sodium sulfate and filtered. The filtrate was concentrated under reduced pressure to give a crude product, which was slurried with a mixed solution of diethyl ether (10 mL) and petroleum ether (10 mL) and filtered, and the filter cake was dried to give the product (91.5 mg, yield: 64.4%).

$^1$HNMR(300 MHz, $CD_3OD$) δ(ppm): 8.93 (s, 1H), 8.52 (s, 1H), 7.50 (s, 1H), 4.52-4.43 (m, 1H), 3.37-3.72 (m, 1H), 2.71-2.63 (m, 1H), 2.12-2.10 (m, 1H), 1.95 (m, 6H), 1.55-1.47 (m, 3H), 1.36-1.34 (m, 7H).

Molecular formula: $C_{21}H_{26}N_6O_2$ Exact mass: 394.21 LC-MS(Pos, *m*/*z*)=395.27 $[M+H]^+$.

**Example 3: Synthesis of (1*S*,3*R*)-3-acetamido-*N*-(6-cyano-8-(isopropylamino)pyrido[3,4-*d*]pyrimidin-2-yl)cyclohexane-1-carboxamide (compound 3)**

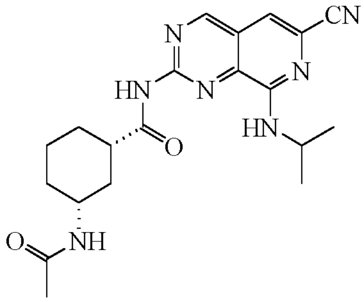

Step 1: synthesis of 2-amino-8-(isopropylamino)pyrido[3,4-*d*]pyrimidine-6-carbonitrile

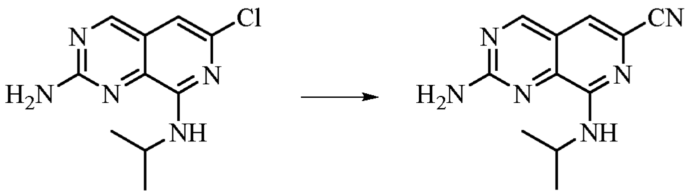

6-chloro-$N^8$-isopropylpyrido[3,4-*d*]pyrimidine-2,8-diamine (500 mg, 2.10 mmol, 1.0 eq.), zinc cyanide (494 mg, 4.21 mmol, 2.0 eq.) and tetrakis(triphenylphosphine)palladium(0) (485 mg, 0.42 mmol, 0.2 eq.) were dissolved in DMAC (10 mL), and the reaction solution was reacted at 120 °C for 24 h in nitrogen atmosphere. When there were a small amount of materials left, as detected by LC-MS, the reaction solution was cooled to room temperature, poured into water (20 mL) and extracted with EA (20 mL × 2). The organic phases were washed with saturated aqueous sodium chloride solution (15 mL × 2), concentrated under reduced pressure to give a crude product, which was purified by silica gel

column chromatography (PE:EA= 3:1) to give the product (320 mg, yield: 66.6%).

Step 2: synthesis of *tert*-butyl ((1*R*,3*S*)-3-((6-cyano-8-(isopropylamino)pyrido[3,4-*d*]pyrimidin-2-yl)carbamoyl)cyclohexyl)carbamate

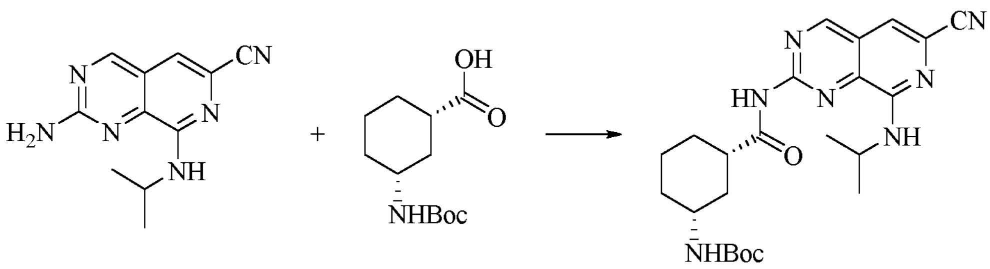

2-amino-8-(isopropylamino)pyrido[3,4-*d*]pyrimidine-6-carbonitrile (160 mg, 0.71 mmol, 1.0 eq.) and (1S,3*R*)-3-((*tert*-butoxycarbonyl)amino)cyclohexane-1-carboxylic acid (188 mg, 0.77 mmol, 1.1 eq.) were dissolved in pyridine (3 mL), and the reaction solution was added with phosphorus oxychloride (107 mg, 0.71 mmol, 1.0 eq.) and reacted at room temperature for 23 h. When there were materials left, as detected by TLC, the reaction solution was poured into water (10 mL) and extracted with EA (15 mL × 2). The organic phase was washed with hydrochloric acid (10 mL × 3), dried and concentrated to give a crude product, which was purified by silica gel column chromatography (PE:EA= 3:1) to give the product (160 mg, yield: 50.4%).

Step 3: synthesis of (1*S*,3*R*)-3-amino-*N*-(6-cyano-8-(isopropylamino)pyrido[3,4-d]pyrimidin-2-yl)cyclohexane-1 -carboxamide

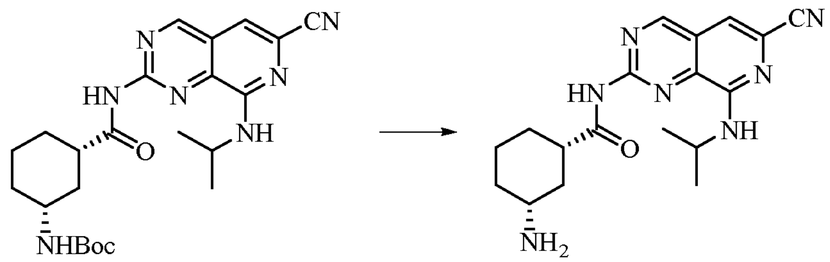

*Tert*-butyl ((1*R*,3*S*)-3-((6-cyano-8-(isopropylamino)pyrido[3,4-*d*]pyrimidin-2-yl)carbamoyl)cyclohexyl)carbamate (160 mg, 0.35 mmol, 1.0 eq.) was dissolved in EA (4 mL). The reaction solution was added dropwise with a solution of HCl in 1,4-dioxane (4 mol/L, 2 mL) and reacted at room temperature for 4 h. After the reaction was substantially completed, as detected by LC-MS, the crude product was dissolved with saturated aqueous sodium carbonate solution (20 mL). The aqueous phase was extracted with DCM (10 mL × 3). The organic phase was dried and concentrated to give the product (100 mg, yield: 80.2%).

Step 4: synthesis of (1*S*,3*R*)-3-acetamido-*N*-(6-cyano-8-(isopropylamino)pyrido[3,4-*d*]pyrimidin-2-yl)cyclohexane-1 -carboxamide

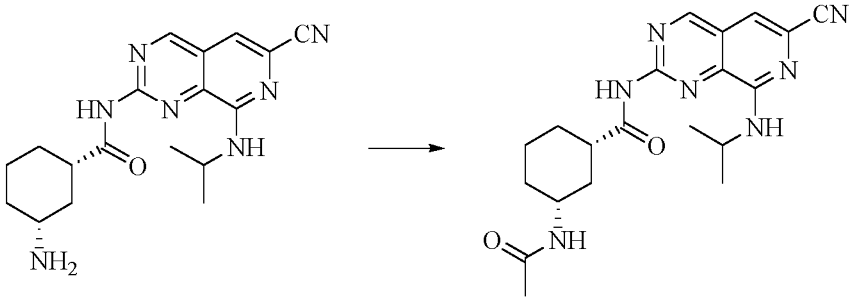

(1$S$,3$R$)-3-amino-$N$-(6-cyano-8-(isopropylamino)pyrido[3,4-$d$]pyrimidin-2-yl)cyclohexane-1-carboxamide (100 mg, 0.28 mmol, 1.0 eq.), DIPEA (109 mg, 0.84 mmol, 3.0 eq.) and acetic anhydride (43 mg, 0.43 mmol, 1.5 eq.) were dissolved in DCM (2 mL), and the reaction solution was reacted at room temperature for 5 h. After the reaction was completed, as detected by LC-MS, the reaction system was added with water (5 mL), the aqueous phase was extracted with DCM (5 mL × 3), and the organic phase was dried and concentrated to give a crude product. The crude product was slurried with MTBE (5 mL) and filtered under vacuum, and the filter cake was dried at 50 °C to give the product (30 mg, yield: 26.8%).

$^1$HNMR(300 MHz, DMSO-$d_6$) δ(ppm):11.10 (s, 1H), 9.38 (s, 1H), 7.81-7.78 (d, 1H), 7.65 (s, 1H), 6.93-6.90 (d, 1H), 4.29-4.22 (m ,1H), 3.60-3.57 (t, 1H), 2.76-2.73 (d, 1H), 1.96-1.92 (d, 1H), 1.77 (s, 6H), 1.29-1.27 (d, 10H).

Molecular formula: $C_{20}H_{25}N_7O_2$ Exact mass: 395.21 LC-MS(Pos, $m/z$)=396.28[M+H]$^+$.

**Example 4: Synthesis of (1*S*,3*R*)-3-acetamido-*N*-(6-chloro-8-(isopropylamino)pyrido[3,4-*d*]pyrimidin-2-yl)cyclohexane-1-carboxamide (compound 4)**

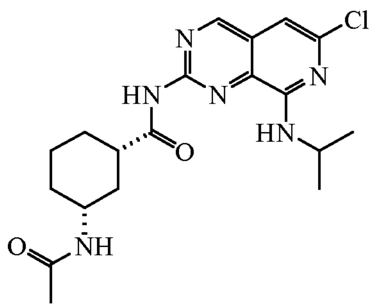

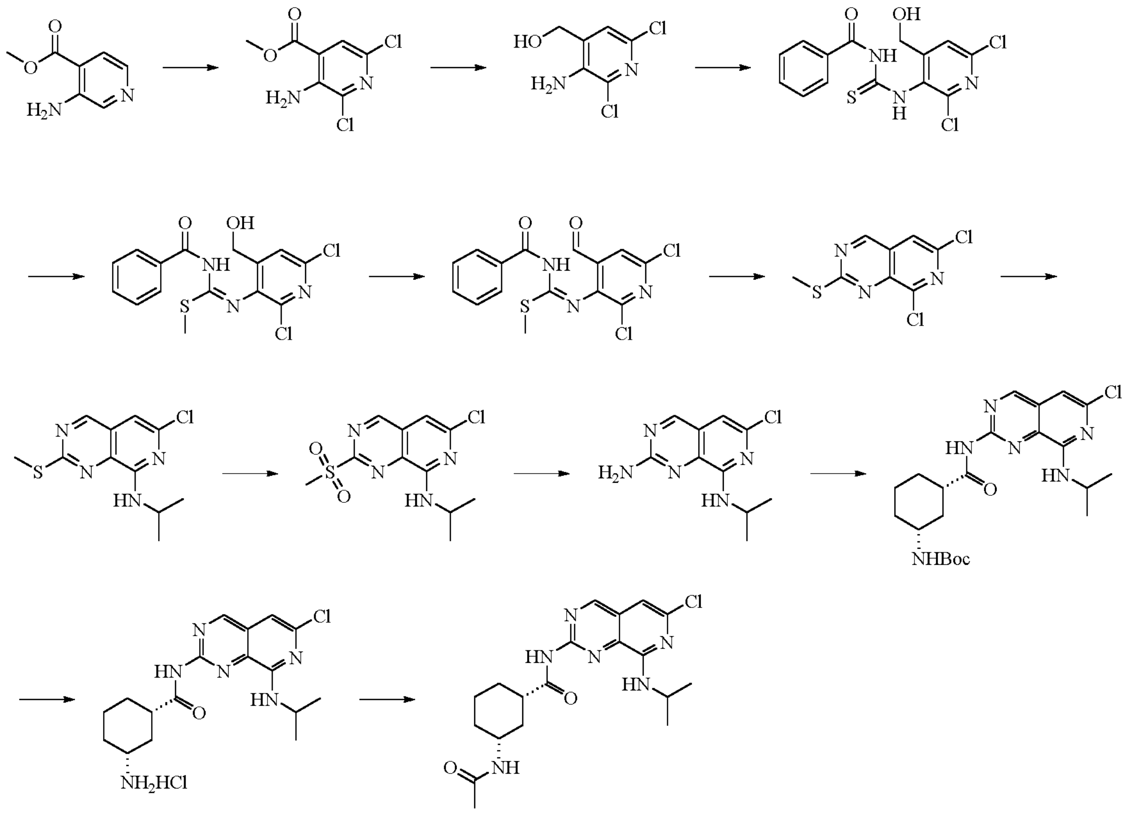

Step 1: synthesis of methyl 3-amino-2,6-dichloroisonicotinate

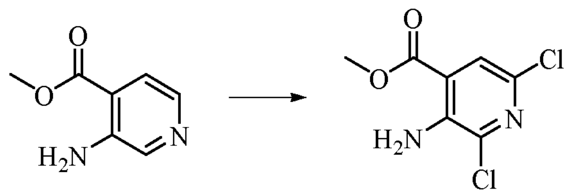

Methyl 3-aminoisonicotinate (105 g, 690.11 mmol, 1.0 eq.) and *N*-chlorosuccinimide (193.52 g, 1449.23 mmol, 2.1 eq.) were dissolved in DMF (500 mL), and the reaction solution was reacted at 30 °C for 22 h. After the reaction was completed, as detected by TLC, the reaction solution was poured into water (2 L), a large amount of solid was precipitated, and the reaction solution was stirred for 1 h and filtered under vacuum. The filter cake was dissolved by EA (2 L), dried and filtered, and the filtrate was concentrated under reduced pressure to give the product (152.54 g, yield: 100%).

Step 2: synthesis of (3-amino-2,6-dichloropyridin-4-yl)methanol

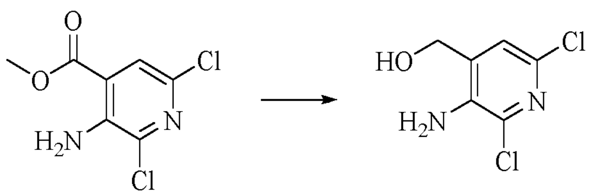

Methyl 3-amino-2,6-dichloroisonicotinate (152.54 g, 690.11 mmol, 1.0 eq.) was dissolved in THF (1.5 L), and

the reaction solution was cooled to 0 °C, added with lithium aluminum hydride (26.18 g, 690.11 mmol, 1.0 eq.) in batches and reacted at 0 °C to 5 °C for 40 min. After the reaction was completed, as detected by TLC, the reaction solution was added with a proper amount of water to quench lithium aluminum hydride, then added with a proper amount of anhydrous sodium sulfate, stirred for 30 min and filtered. The filtrate was concentrated under reduced pressure to give a crude product, which was slurried for 1 h with (PE: EA = 5:1, 1.2 L) and filtered, and the filter cake was dried to give the product (84 g, yield: 63%).

Step 3: synthesis of *N*-((2,6-dichloro-4-(hydroxymethyl)pyridin-3-yl)carbamothioyl)benzamide

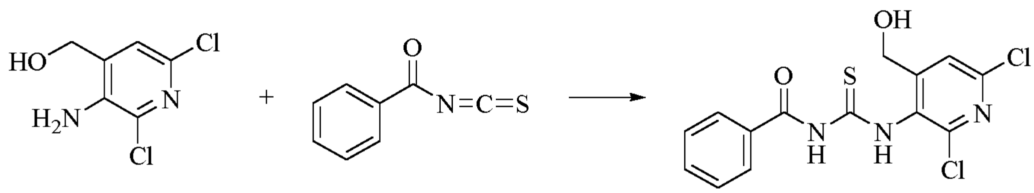

(3-amino-2,6-dichloropyridin-4-yl)methanol (84 g, 435.16 mmol, 1.0 eq.) and benzoyl isothiocyanate (78.12 g, 478.68 mmol, 1.1 eq.) were dissolved in THF (840 mL), and the reaction solution was reacted for 4 h. After the reaction was completed, as detected by TLC, the reaction solution was concentrated under reduced pressure to give a crude product, which was slurried for 0.5 h with (PE:EA = 20:1, 1.05 L) and filtered under vacuum, and the filter cake was dried to give the product (151 g, yield: 97.4%).

Step 4: synthesis of methyl (*E*)-*N*-benzoyl-*N*'-(2,6-dichloro-4-(hydroxymethyl)pyridin-3-yl)carbamimidothioate

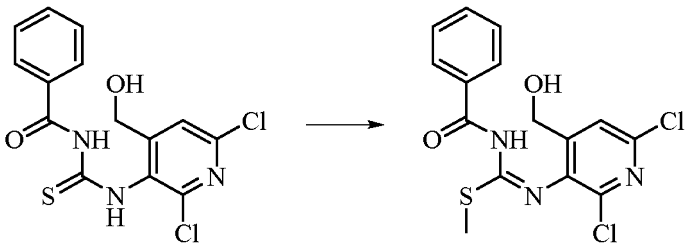

*N*-((2,6-dichloro-4-(hydroxymethyl)pyridin-3-yl)carbamothioyl)benzamide (151 g, 423.90 mmol, 1.0 eq.), iodomethane (66.19 g, 466.29 mmol, 1.1 eq.) and potassium carbonate (64.35 g, 466.29 mmol, 1.1 eq.) were dissolved in THF (1.5 L), and the reaction solution was reacted at room temperature for 12 h. When there were no materials left, as detected by LC-MS, the reaction solution was filtered. The filtrate was concentrated under reduced pressure to give a crude product, which was slurried with MTBE (500 mL) and filtered, and the filtrate was concentrated under reduced pressure to give the product (50 g, yield: 31.8%).

Step 5: synthesis of methyl (*E*)-*N*-benzoyl-*N*'-(2,6-dichloro-4-formylpyridin-3-yl)carbamimidothioate

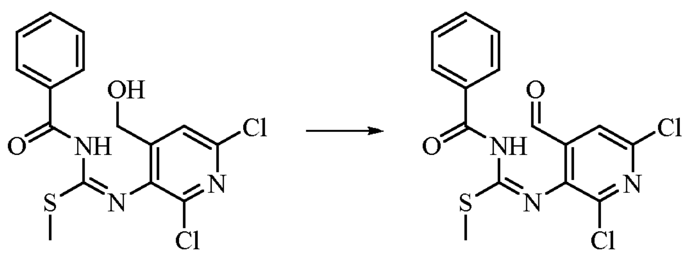

Methyl (*E*)-*N*-benzoyl-*N'*-(2,6-dichloro-4-(hydroxymethyl)pyridin-3-yl)carbamimidothioate (50 g, 135.04 mmol, 1.0 eq.) and IBX (37.81 g, 135.04 mmol, 1.0 eq.) were dissolved in DMSO (250 mL), and the reaction solution was reacted at room temperature for 1 h. After the reaction was completed, as detected by TLC, the reaction solution was poured into saturated aqueous potassium carbonate (500 mL) and extracted with MTBE (500 mL × 3). The organic phases were combined, washed with water (500 mL × 2), dried and filtered, and the filtrate was concentrated under reduced pressure to give the product (40 g, yield: 80.4%).

Step 6: synthesis of 6,8-dichloro-2-(methylthio)pyrido[3,4-*d*]pyrimidine

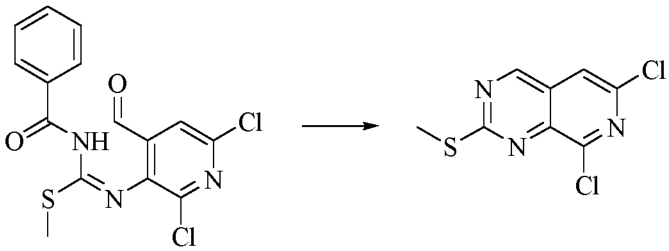

Methyl (*E*)-*N*-benzoyl-*N'*-(2,6-dichloro-4-formylpyridin-3-yl)carbamimidothioate (40 g, 108.63 mmol, 1.0 eq.) and potassium carbonate (15.01 g, 108.63 mmol, 1.0 eq.) were dissolved in acetonitrile (400 mL), and the reaction solution was reacted at 90 °C for 1 h. After the reaction was completed, as detected by TLC, the reaction solution was concentrated under reduced pressure, added with water (100 mL) and extracted with ethyl acetate (100 mL × 2). The organic phase was dried and concentrated under reduced pressure to give a crude product, which was slurried with a small amount of ethyl acetate (50 mL), and filtered under vacuum to give the product (13.3 g, yield: 49.8 %).

Step 7: synthesis of 6-chloro-*N*-isopropyl-2-(methylthio)pyrido[3,4-*d*]pyrimidin-8-amine

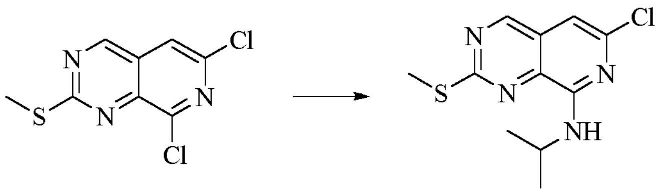

6,8-dichloro-2-(methylthio)pyrido[3,4-*d*]pyrimidine (13.3 g, 54.04 mmol, 1.0 eq.) and isopropylamine (15.97 g, 270.21 mmol, 5.0 eq.) were dissolved in THF (150 mL), and the reaction solution was reacted at 35 °C for 14 h. After the reaction was completed, as detected by TLC, the reaction solution was concentrated under reduced pressure to give a crude product, which was slurried with ethyl acetate (50 mL) to give the product (11 g, yield: 75.7%).

Step 8: synthesis of 6-chloro-*N*-isopropyl-2-(methylsulfonyl)pyrido[3,4-*d*]pyrimidin-8-amine

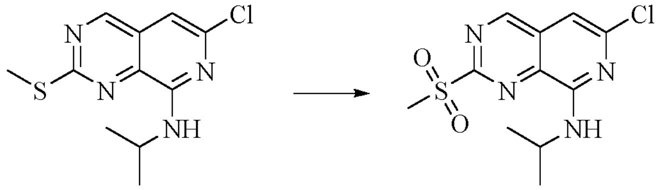

6-chloro-*N*-isopropyl-2-(methylthio)pyrido[3,4-*d*]pyrimidin-8-amine (11 g, 40.92 mmol, 1.0 eq.) was dissolved

in DCM (220 mL), and the reaction solution was added with *m*-chloroperoxybenzoic acid (mass fraction: 80%, 19.42 g, 90.02 mmol, 2.2 eq.) under an ice bath. After the reaction was completed, as detected by TLC, the reaction solution was poured into saturated aqueous sodium carbonate (100 mL), the liquid separation was performed, the aqueous phase was extracted with DCM (100 mL), and the organic phases were combined, dried and concentrated to give the product (10 g, yield: 81.3%).

Step 9: synthesis of 6-chloro-$N^8$-isopropylpyiido[3,4-*d*]pyiimidine-2,8-diamine

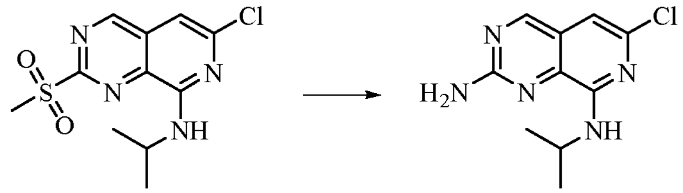

6-chloro-*N*-isopropyl-2-(methylsulfonyl)pyrido[3,4-*d*]pyrimidin-8-amine (10 g, 33.25 mmol, 1.0 eq.) was dissolved in a solution of ammonia in isopropanol (3.5 mol/L, 100 mL), and the reaction solution was reacted at 60 °C for 17.5 h. After the reaction was completed, as detected by LC-MS, the reaction solution was concentrated to give a crude product, which was purified by silica gel column chromatography (PE:EA = 5:1) to give the product (6.0 g, yield: 75.9%).

Step 10: synthesis of *tert*-butyl ((1*R*,3*S*)-3-((6-chloro-8-(isopropylamino)pyrido[3,4-*d*]pyrimidin-2-yl)carbamoyl)cyclohexyl)carbamate

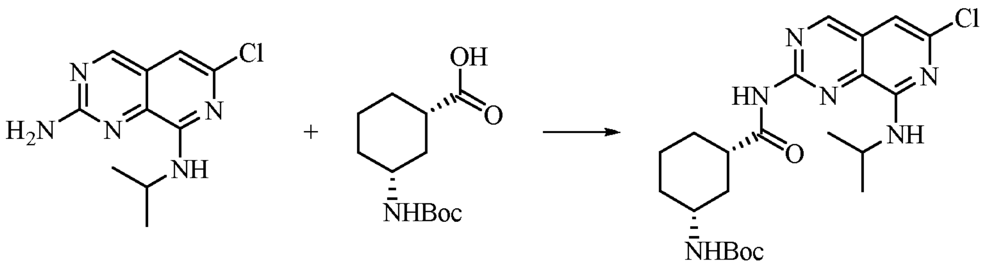

(1S,3R)-3-((*tert*-butoxycarbonyl)amino)cyclohexane-1-carboxylic acid (512 mg, 2.10 mmol, 1.0 eq.), 1-chloro-*N,N*,2-trimethylprop-1-en-1-amine (365 mg, 2.73 mmol, 1.3 eq.) and pyridine (499 mg, 6.31 mmol, 3.0 eq.) were dissolved in DCM (10 mL), and the reaction solution was reacted at 0 °C for 0.5 h in nitrogen atmosphere. The reaction solution was added with 6-chloro-$N^8$-isopropylpyrido[3,4-*d*]pyrimidine-2,8-diamine (500 mg, 2.10 mmol, 1.0 eq.), and incubated at room temperature for 12 h. When there were a small amount of materials left, as detected by LC-MS, the reaction solution was concentrated to give a crude product, which was purified by silica gel column chromatography (PE:EA= 3:1) to give the product (250 mg, yield: 25.6%).

Step 11: synthesis of (1*S*,3*R*)-3-amino-*N*-(6-chloro-8-(isopropylamino)pyrido[3,4-*d*]pyrimidin-2-yl)cyclohexane-1-carboxamide hydrochloride

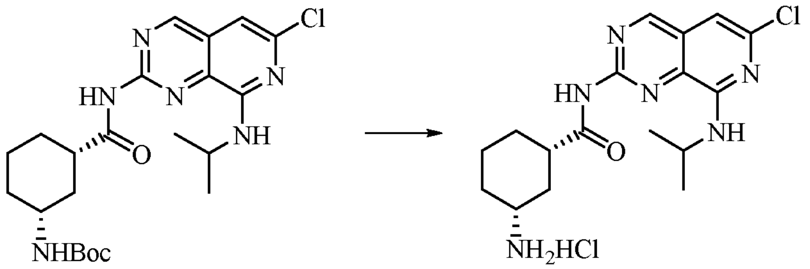

*Tert*-butyl ((1*R*,3*S*)-3-((6-chloro-8-(isopropylamino)pyrido[3,4-*d*]pyrimidin-2-yl)carbamoyl)cyclohexyl)carbamate (200 mg, 0.43 mmol, 1.0 eq.) was dissolved in EA (4 mL). The reaction solution was added dropwise with a solution of HCl in 1,4-dioxane (4 mol/L, 2 mL) and reacted at room temperature for 3 h. After the reaction was substantially completed, as detected by LC-MS, the reaction solution was added with MTBE (4 mL), stirred for 10 min and filtered under vacuum, and the filter cake was dried to give the product (160 mg, yeild: 92.7%).

Step 12: synthesis of (1*S*,3*R*)-3-acetamido-*N*-(6-chloro-8-(isopropylamino)pyrido[3,4-*d*]pyrimidin-2-yl)cyclohexane-1-carboxamide

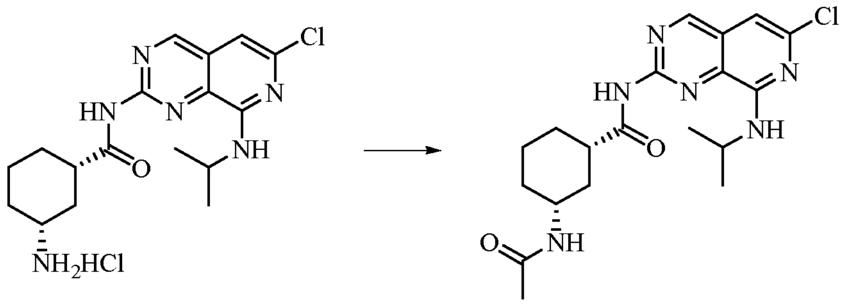

(1*S*,3*R*)-3-amino-*N*-(6-chloro-8-(isopropylamino)pyrido[3,4-*d*]pyrimidin-2-yl)cyclohexane-1-carboxamide hydrochloride (160 mg, 0.40 mmol, 1.0 eq.) and pyridine (95 mg, 1.21 mmol, 3.0 eq.) were dissolved in DCM (3 mL), and the reaction solution was reacted at room temperature for 5 min, added with acetic anhydride (61 mg, 0.60 mmol, 1.5 eq.) and reacted at room temperature for 7 h. After the reaction was completed, as detected by LC-MS, the reaction solution was concentrated to give a crude product, which was dissolved with EA (15 mL) and washed with dilute hydrochloric acid (10 mL × 2). The organic phase was dried and concentrated to give a crude product, which was slurried with MTBE (10 mL) and filtered under vacuum, and the filter cake was dried to give the product (82 mg, yield: 50.5%). $^1$HNMR (300 MHz, DMSO-$d_6$) δ(ppm): 10.89 (s, 1H), 9.27 (s, 1H), 7.81-7.78 (d, 1H), 7.01 (s, 1H), 6.89-6.86 (d, 1H), 4.27-4.20 (m ,1H), 3.60-3.56 (m, 1H), 2.73 (s, 1H), 1.95-1.91 (d, 1H), 1.77 (s, 6H), 1.35-1.23 (m, 10H).
Molecular formula: $C_{19}H_{25}ClN_6O_2$ Exact mass: 404.17 LC-MS(Pos, *m*/*z*)=405.12[M+H]$^+$.

**Example 5: Synthesis of (1*S*,3*R*)-3-acetamido-*N*-(8-(isopropylamino)-6-(methylsulfonyl)pyrido[3,4-*d*]pyrimidin-2-yl)cyclohexane-1-carboxamide (compound 5)**

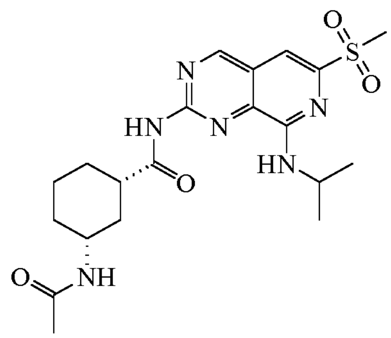

Step 1: synthesis of $N^8$-isopropyl-6-(methylsulfonyl)pyrido[3,4-*d*]pylimidine-2,8-diamine

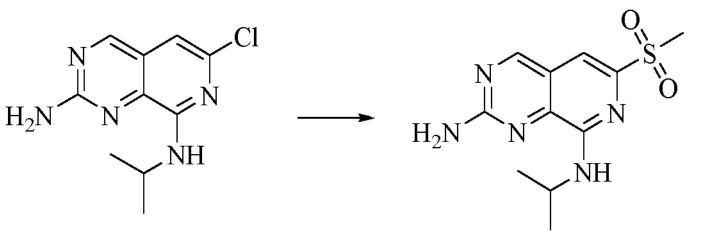

6-chloro-$N^8$-isopropylpyiido[3,4-*d*]pyiimidine-2,8-diamine(500 mg, 2.10 mmol, 1.0 eq.), *N,N'*-dimethyl-1,2-cyclohexanediamine (150 mg, 1.05 mmol, 0.5 eq.), sodium methylenesulfonate (430 mg, 4.21 mmol, 2.0 eq.) cuprous iodide (200 mg, 1.05 mmol, 0.5 eq.) and potassium phosphate (1.34 g, 6.31 mmol, 3.0 eq.) were dissolved in DMSO (10 mL), and the reaction solution was reacted at 120 °C for 21 h in nitrogen atmosphere. After the reaction was completed, as detected by LC-MS, the reaction solution was poured into water (50 mL) and the aqueous phase was extracted with EA (50 mL × 2). The organic phase was washed with saturated brine (50 mL × 2), dried and concentrated to give a crude product, which was purified by silica gel column chromatography (PE:EA= 2:1) to give the product (160 mg, yield: 27.04%).

Step 2: synthesis of *tert*-butyl ((1*R*,3*S*)-3-((8-(isopropylamino)-6-(methylsulfonyl)pyrido[3,4-*d*]pyrimidin-2-yl)carbamoyl)cyclohexyl)carbamate

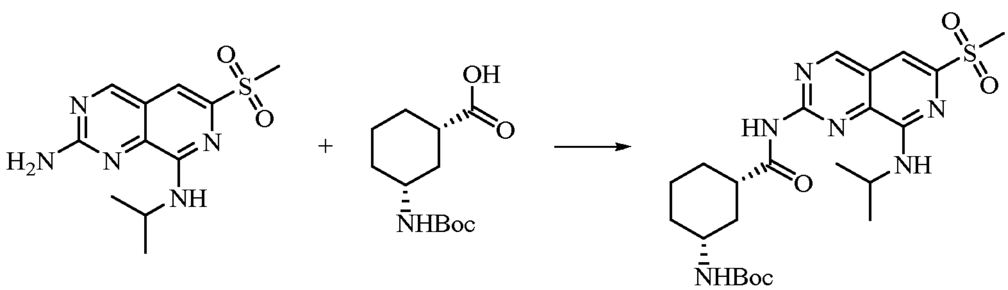

$N^8$-isopropyl-6-(methylsulfonyl)pyrido[3,4-*d*]pyrimidine-2,8-diamine (160 mg, 0.57 mmol, 1.0 eq.) and (1*S*,3*R*)-3-((*tert*-butoxycarbonyl)amino)cyclohexane-1-carboxylic acid (152 mg, 0.62 mmol, 1.1 eq.) were dissolved in pyridine (3 mL), and the reaction solution was added with phosphorus oxychloride (436 mg, 2.84 mmol, 5.0 eq.) and reacted at room temperature for 0.5 h. After the reaction was completed, as detected by TLC, the reaction solution was poured into hydrochloric acid (20 mL), the aqueous phase was extracted with EA (15 mL × 3), and the organic phase was dried and concentrated to give a crude product. The crude product was slurried with (PE:EA = 5:1, 60 mL) and filtered under vacuum, and the filter cake was dried at 50 °C to give the product (190 mg, yield: 65.9%).

Step 3: synthesis of (1*S*,3*R*)-3-amino-*N*-(8-(isopropylamino)-6-(methylsulfonyl)pyrido[3,4-*d*]pyrimidin-2-yl)cyclohexane-1-carboxamide hydrochloride

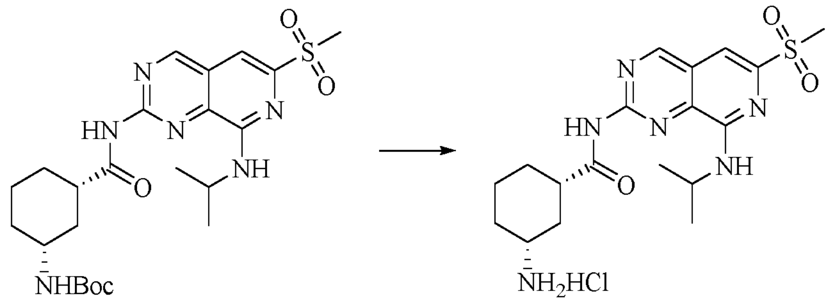

*Tert*-butyl ((1*R*,3*S*)-3-((8-(isopropylamino)-6-(methylsulfonyl)pyrido[3,4-*d*]pyrimidin-2-yl)carbamoyl)cyclohexyl)carbamate (180 mg, 0.36 mmol, 1.0 eq.) was dissolved in EA (2 mL). The reaction solution was added dropwise with a solution of HCl in 1,4-dioxane (4 mol/L, 2 mL) and reacted at room temperature for 3 h. After the reaction was substantially completed, as detected by LC-MS, the reaction solution was filtered under vacuum give the product (150 mg, yeild: 95.3%).

Step 4: synthesis of (1*S*,3*R*)-3-acetamido-*N*-(8-(isopropylamino)-6-(methylsulfonyl)pyrido[3,4-*d*]pyrimidin-2-yl)cyclohexane-1-carboxamide

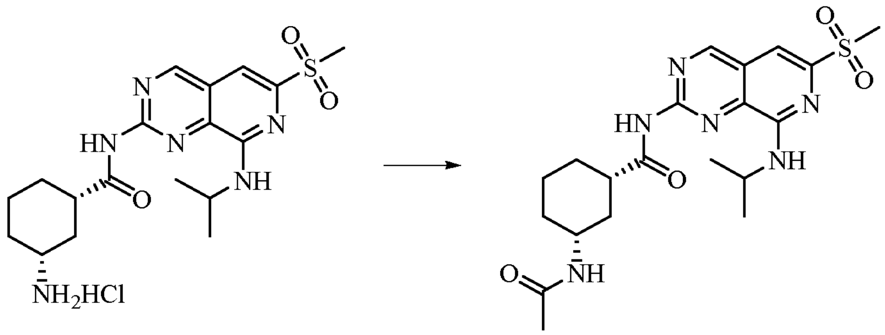

(1*S*,3*R*)-3-amino-*N*-(8-(isopropylamino)-6-(methylsulfonyl)pyrido[3,4-*d*]pyrimidin-2-yl)cyclohexane-1-carboxamide hydrochloride (150 mg, 0.34 mmol, 1.0 eq.), DIPEA (131.3 mg, 1.02 mmol, 3.0 eq.) and acetic anhydride (52 mg, 0.51 mmol, 1.5 eq.) were dissolved in DCM (3 mL), and the reaction solution was reacted at room temperature for 1 h. After the reaction was completed, as detected by LC-MS, the reaction solution was poured into water (5 mL) and extracted with DCM (10 mL × 2). The organic phase was dried and concentrated to give a crude product, which was slurried with EA (3 mL) and filtered under vacuum, and the filter cake was dried to give the product (50 mg, yield: 32.9%). $^1$HNMR (300 MHz, DMSO-$d_6$) δ(ppm): 11.08 (s, 1H), 9.55 (s, 1H), 7.82-7.80 (d, 1H), 6.69 (s, 1H), 7.01-6.98 (d, 1H), 4.34-4.25 (m, 1H), 3.61-3.59 (t, 1H), 3.23 (s, 3H), 2.78-2.75 (d, 1H), 1.96-1.94 (d 1H), 1.78 (s, 6H), 1.33-1.31 (d, 10H). Molecular formula: $C_{20}H_{28}N_6O_4S$ Exact mass: 448.19 LC-MS(Pos, *m*/*z*)=449.08[M+H]$^+$.

**Example 6: Synthesis of (*S*)-1-acetyl-*N*-(6-cyano-8-(isopropylamino)pyrido[3,4-*d*]pyrimidin-2-yl)piperidine-3-carboxamide (compound 6)**

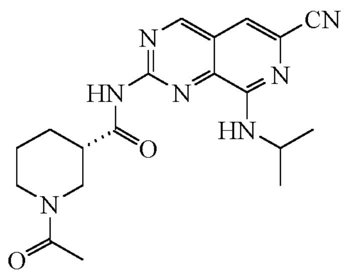

Step 1: synthesis of *tert*-butyl (*S*)-3-((6-cyano-8-(isopropylamino)pyrido[3,4-*d*]pyrimidin-2-yl)carbamoyl)piperidine-1-carboxylate

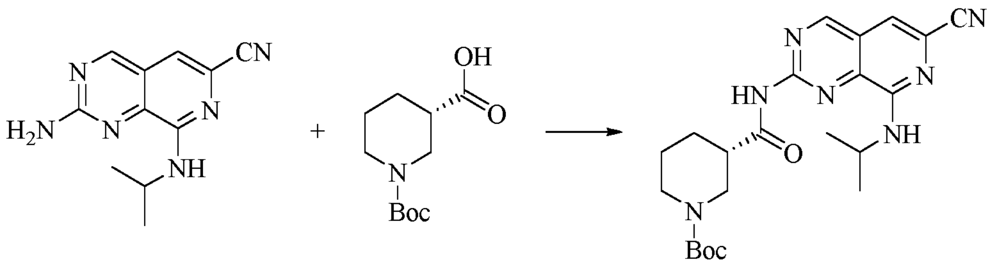

(*S*)-1-(*tert*-butoxycarbonyl)piperidine-3-carboxylic acid (177 mg, 0.77 mmol, 1.0 eq.), 1-chloro-*N,N*,2-trimethylprop-1-en-1-amine (140 mg, 1.05 mmol, 1.5 eq.) and pyridine (166 mg, 2.10 mmol, 3.0 eq.) were dissolved in DCM (4 mL), and the reaction solution was reacted at 0 °C for 1.5 h. The reaction solution was added with 2-amino-8-(isopropylamino)pyrido[3,4-*d*]pyrimidine-6-carbonitrile (1600 mg, 0.7 mmol, 1.0 eq.), and incubated at room temperature for 16 h. After the reaction was completed, as detected by TLC, the reaction solution was concentrated to give a crude product, which was purified by silica gel column chromatography (PE:EA = 3:1) to give the product (160 mg, yield: 51.9%).

Step 2: synthesis of (*S*)-*N*-(6-cyano-8-(isopropylamino)pyrido[3,4-*d*]pyrimidin-2-yl)piperidine-3-carboxamide hydrochloride

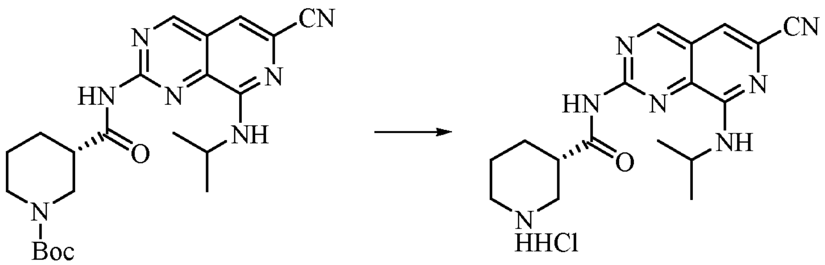

*Tert*-butyl (*S*)-3-((6-cyano-8-(isopropylamino)pyrido[3,4-*d*]pyrimidin-2-yl)carbamoyl)piperidine-1-carboxylate (160 mg, 0.36 mmol, 1.0 eq.) was dissolved in EA (2 mL). The reaction solution was added dropwise with a solution of HCl in 1,4-dioxane (4 mol/L, 2 mL) and reacted at room temperature for 15 h. After the reaction was substantially completed, as detected by LC-MS, the reaction solution was filtered under vacuum give the product (136 mg, yeild: 100%).

Step 3: synthesis of (*S*)-1-acetyl-*N*-(6-cyano-8-(isopropylamino)pyrido[3,4-*d*]pyrimidin-2-yl)piperidine-3-carboxamide

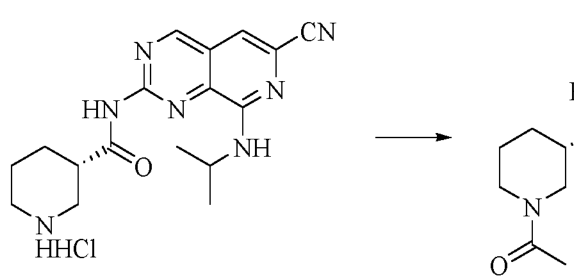

(*S*)-*N*-(6-cyano-8-(isopropylamino)pyrido[3,4-*d*]pyrimidin-2-yl)piperidine-3-carboxamide hydrochloride (136 mg, 0.36 mmol, 1.0 eq.), DIPEA (140 mg, 1.09 mmol, 3.0 eq.) and acetic anhydride (55 mg, 0.54 mmol, 1.5 eq.) were dissolved in DCM (3 mL), and the reaction solution was reacted at room temperature for 3 h. After the reaction was completed, as detected by LC-MS, the reaction solution was concentrated under reduced pressure to give a crude product, which was purified by silica gel column chromatography (DCM:MeOH = 50:1) to give the product (30 mg, yield: 21.7%).

$^1$HNMR (300 MHz, DMSO-$d_6$) δ(ppm): 11.27-11.24 (d, 1H), 9.42-9.41 (d, 1H), 7.68-7.67 (d, 1H), 7.93-7.90 (d, 1H), 6.93-6.90 (d, 1H), 4.45-4.24 (m ,2H), 3.96-3.77 (m, 1H), 3.23-3.16 (t, 1H), 3.08-2.86 (m, 1H), 2.74-2.73 (d, 1H), 2.71-2.60 (m, 1H), 2.05-2.02 (d, 4H), 1.74-1.63 (m, 2H), 1.29-1.27 (d, 6H).

Molecular formula: $C_{19}H_{23}N_7O_2$ Exact mass: 381.19 LC-MS(Pos, *m*/*z*)=382.15[M+H]$^+$.

**Example 7: Synthesis of (1*S*,3*R*)-3-acetamido-*N*-(6-(difluoromethyl)-8-(isopropylamino)pyrido[3,4-*d*]pyrimidin-2-yl)cyclohexane-1-carboxamide (compound 7)**

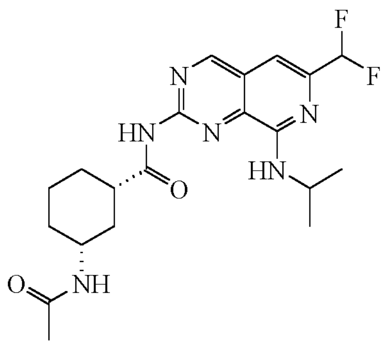

Step 1: synthesis of 2-amino-8-(isopropylamino)pyrido[3,4-*d*]pyrimidine-6-carbaldehyde

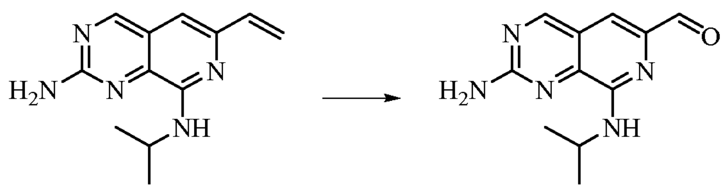

*N*$^8$-isopropyl-6-vinylpyrido[3,4-*d*]pyrimidine-2,8-diamine (900 mg, 3.93 mmol, 1.0 eq.), sodium periodate (2.52 g, 11.78 mmol, 3.0 eq.) and potassium osmate dihydrate (144 mg, 0.39 mmol, 0.1 eq.) were dissolved in $H_2O$ (4 mL) and THF (9 mL), and the reaction solution was reacted at room temperature for 18 h. After the reaction was completed, as detected by LC-MS, the reaction solution was poured into water (20 mL) and extracted with EA (20 mL × 3). The organic phase was dried and concentrated to give a crude product, which was purified by silica gel column chromatography (PE:EA= 5:1) to give the product (200 mg, yield: 22.0%).

Step 2: synthesis of 6-(difluoromethyl)-*N*[8]-isopropylpyrido[3,4-*d*]pyrimidine-2,8-diamine

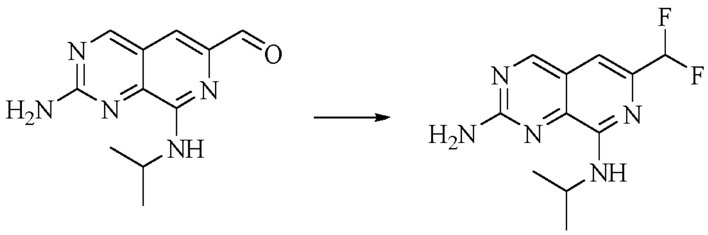

2-amino-8-(isopropylamino)pyrido[3,4-*d*]pyrimidine-6-carbaldehyde (200 mg, 0.86 mmol, 1.0 eq.) and bis(2-methoxyethyl)aminosulfur trifluoride (951 mg, 4.30 mmol, 5.0 eq.) were dissolved in DCM (9 mL), and the reaction solution was reacted at 40 °C for 17 h. The reaction solution was concentrated under reduced pressure to give a crude product, which was purified by silica gel column chromatography (PE:EA = 6:1) to give the product (40 mg, yield: 18.2%).

Step 3: synthesis of *tert*-butyl ((1*R*,3*S*)-3-((6-(difluoromethyl)-8-(isopropylamino)pyrido[3,4-*d*]pyrimidin-2-yl)carbamoyl)cyclohexyl)carbamate

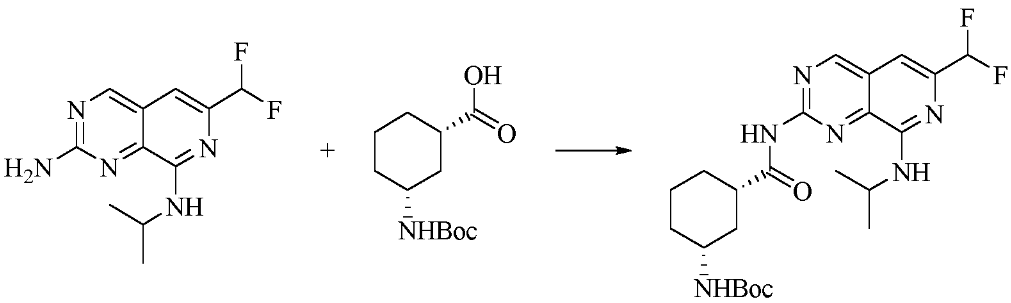

6-(difluoromethyl)-*N*[8]-isopropylpyrido[3,4-d]pyrimidine-2,8-diamine (40 mg, 0.16 mmol, 1.0 eq.) and (1*S*,3*R*)-3-((*tert*-butoxycarbonyl)amino)cyclohexane-1-carboxylic acid (42 mg, 0.17 mmol, 1.1 eq.) were dissolved in pyridine (1 mL), and the reaction solution was added with phosphorus oxychloride (121 mg, 0.79 mmol, 5.0 eq.) and reacted at room temperature for 10 min. After the reaction was completed, as detected by TLC, the reaction solution was poured into water (3 mL) and extracted with EA (5 mL × 2). The organic phase was dried and concentrated to give a crude product, which was purified by preparative thin-layer chromatography (PE:EA= 2:1) to give the product (20 mg, yield: 26.4%).

Step 4: synthesis of (1*S*,3*R*)-3-amino-*N*-(6-(difluoromethyl)-8-(isopropylamino)pyrido[3,4-*d*]pyrimidin-2-yl)cyclohexane-1-carboxamide hydrochloride

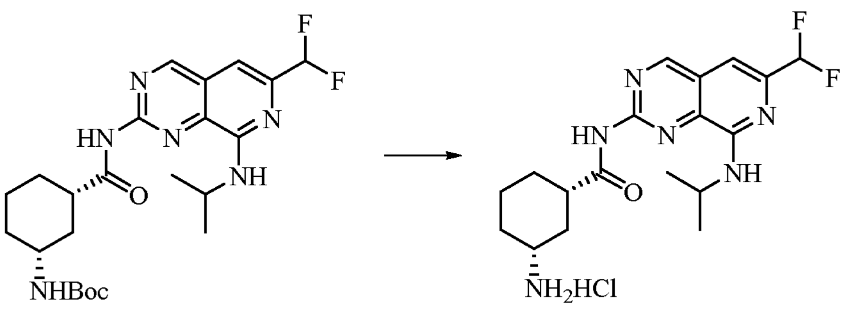

*Tert*-butyl ((1*R*,3*S*)-3-((6-(difluoromethyl)-8-(isopropylamino)pyrido[3,4-*d*]pyrimidin-2-yl)carbamoyl)cyclohexyl)carbamate (20 mg, 0.04 mmol, 1.0 eq.) was dissolved in EA (1 mL). The reaction solution was added dropwise with a solution of HCl in 1,4-dioxane (4 mol/L, 0.5 mL) and reacted at room temperature for 18 h. After the reaction was substantially completed, as detected by LC-MS, the reaction solution was filtered under vacuum give the product (16 mg, yeild: 100%).

Step 5: synthesis of (1*S*,3*R*)-3-acetamido-*N*-(6-(difluoromethyl)-8-(isopropylamino)pyrido[3,4-*d*]pyrimidin-2-yl)cyclohexane-1-carboxamide

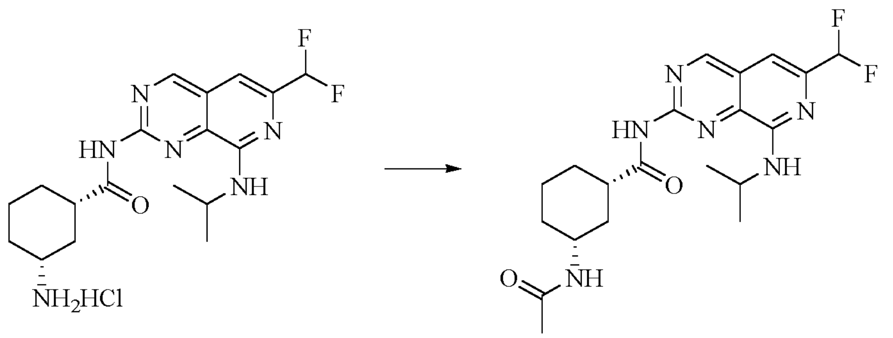

(1*S*,3*R*)-3-amino-*N*-(6-(difluoromethyl)-8-(isopropylamino)pyrido[3,4-*d*]pyrimidin-2-yl)cyclohexane-1-carboxamide hydrochloride (16 mg, 0.038 mmol, 1.0 eq.), DIPEA (15.5 mg, 0.12 mmol, 3.0 eq.) and acetic anhydride (6 mg, 0.058 mmol, 1.5 eq.) were dissolved in DCM (1 mL), and the reaction solution was reacted at room temperature for 20 min. After the reaction was completed, as detected by TLC, the reaction solution was poured into water (3 mL) and extracted with EA (5 mL × 2). The organic phase was dried and concentrated to give a crude product, which was slurried with MTBE (1 mL) and purified to give the product (4 mg, yield: 25.0%).
$^1$HNMR (300 MHz, DMSO-$d_6$) δ(ppm): 10.97 (s, 1H), 9.44 (s, 1H), 7.82-7.79 (d, 1H), 7.27 (s, 1H), 7.02 (s, 0.3H), 6.84 (s, 0.5H), 6.79-6.76 (d, 1H), 6.66 (s, 0.3H), 4.36-4.25 (m, 1H), 3.61-3.59 (t, 1H), 2.77-2.73 (d,1H), 1.93-1.91 (d, 3H), 1.78 (s, 4H), 1.33-1.24 (d, 10H).
Molecular formula: $C_{20}H_{26}F_2N_6O_2$ Exact mass: 420.21 LC-MS(Pos, *m*/*z*)=421.14[M+H]$^+$.

**Example 8: Synthesis of (1*S*,3*R*)-3-acetamido-*N*-(8-(isopropylamino)-6-methylpyrido[3,4-*d*]pyrimidin-2-yl)cyclohexane-1-carboxamide (compound 8)**

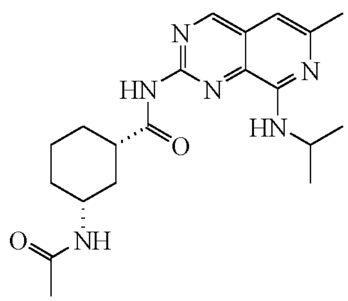

Step 1: synthesis of $N^8$-isopropyl-6-methylpyrido[3,4-d]pyrimidine-2,8-diamine

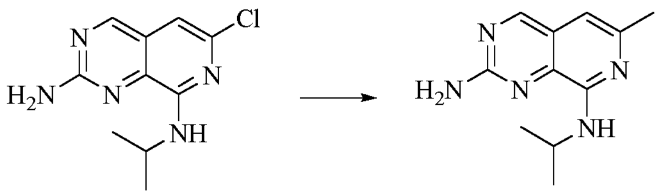

6-chloro-*N*[8]-isopropylpyiido[3,4-*d*]pyiimidine-2,8-diamine(500 mg, 2.10 mmol, 1.0 eq.), 50% trimethylboroxine (2.11 g, 8.41 mmol, 4.0 eq.), cesium carbonate (1.37 g, 4.21 mmol, 2.0 eq.) and [1,1'-bis(diphenylphosphino)ferrocene]palladium dichloride (307 mg, 0.42 mmol, 0.2 eq.) were dissolved in $H_2O$ (2 mL) and 1,4-dioxane (10 mL), and the reaction solution was reacted at 100 °C for 16 h in nitrogen atmosphere. After the reaction was completed, as detected by LC-MS, the reaction solution was poured into water (20 mL) and extracted with EA (30 mL × 2). The organic phase was dried and concentrated to give a crude product, which was purified by silica gel column chromatography (PE:EA= 3:1) to give the product (300 mg, yield: 65.6%).

Step 2: synthesis of *tert*-butyl ((1*R*,3*S*)-3-((8-(isopropylamino)-6-methylpyrido[3,4-*d*]pyrimidin-2-yl)carbamoyl)cyclohexyl)carbamate

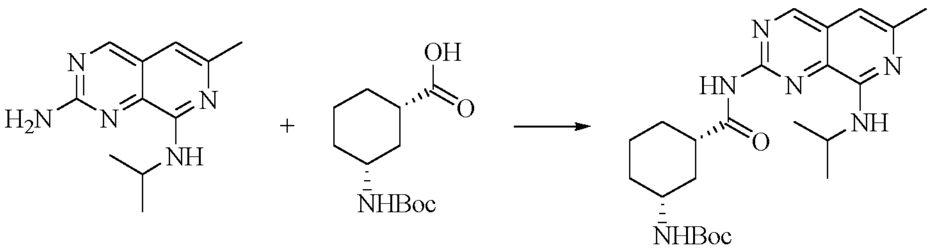

*N*[8]-isopropyl-6-methylpyrido[3,4-*d*]pyrimidine-2,8-diamine (300 mg, 1.38 mmol, 1.0 eq.) and (1*S*,3*R*)-3-((*tert*-butoxycarbonyl)amino)cyclohexane-1-carboxylic acid (370 mg, 1.52 mmol, 1.1 eq.) were dissolved in pyridine (5 mL), and the reaction solution was added with phosphorus oxychloride (1.06 g, 6.90 mmol, 5.0 eq.) and reacted at room temperature for 0.5 h. After the reaction was completed, as detected by TLC, the reaction solution was poured into hydrochloric acid (20 mL) and extracted with EA (15 mL × 3). The organic phase was dried and concentrated to give a crude product, which was purified by silica gel column chromatography (PE:EA = 3:1) to give the product (80 mg, yield: 13.1%).

Step 3: synthesis of (1*S*,3*R*)-3-amino-*N*-(8-(isopropylamino)-6-methylpyrido[3,4-*d*]pyrimidin-2-yl)cyclohexane-1-carboxamide hydrochloride

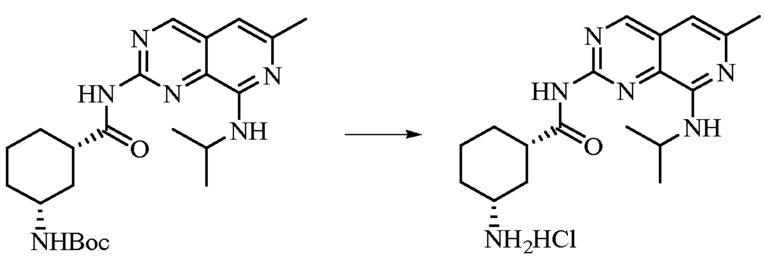

*Tert*-butyl ((1*R*,3*S*)-3-((8-(isopropylamino)-6-methylpyrido[3,4-*d*]pyrimidin-2-yl)carbamoyl)cyclohexyl)carbamate (80 mg, 0.18 mmol, 1.0 eq.) was dissolved in EA (1 mL). The reaction solution was added dropwise with a solution of HCl in 1,4-dioxane (4 mol/L, 0.5 mL) and reacted at room temperature for 19 h. After the reaction was substantially completed, as detected by LC-MS, the reaction solution was filtered under vacuum give the product (68 mg, yeild: 100%).

Step 4: synthesis of (1*S*,3*R*)-3-acetamido-*N*-(8-(isopropylamino)-6-methylpyrido[3,4-*d*]pyrimidin-2-yl)cyclohexane-1 -carboxamide

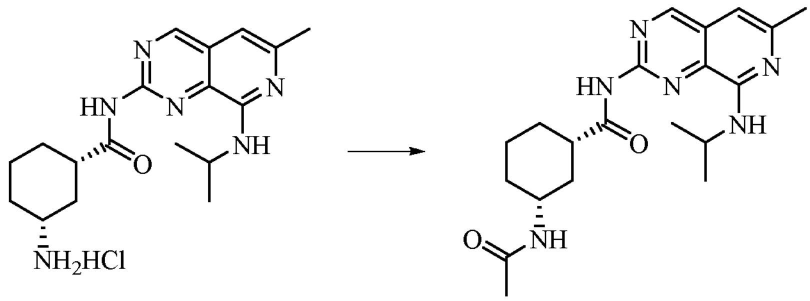

(1*S*,3*R*)-3-amino-*N*-(8-(isopropylamino)-6-methylpyrido[3,4-*d*]pyrimidin-2-yl)cyclohexane-1-carboxamide hydrochloride (68 mg, 0.18 mmol, 1.0 eq.), DIPEA (69 mg, 0.54 mmol, 3.0 eq.) and acetic anhydride (28 mg, 0.27 mmol, 1.5 eq.) were dissolved in DCM (2 mL), and the reaction solution was reacted at room temperature for 20 min. After the reaction was completed, as detected by LC-MS, the reaction solution was concentrated under reduced pressure to give a crude product, which was purified by silica gel column chromatography (MeOH:DCM = 1:20) and slurried with MTBE (2 mL) to give the product (14 mg, yield: 20.3%).
$^1$HNMR (300 MHz, DMSO-$d_6$) δ(ppm): 10.77 (s, 1H), 9.23 (s, 1H), 7.82-7.79 (d, 1H), 6.76 (s, 1H), 6.53-6.50 (d, 1H), 4.35-4.26 (d ,1H), 3.60-3.58 (d, 1H), 2.73 (s, 1H), 2.40 (s, 1H), 1.95-1.91 (d, 1H), 1.78 (s, 6H), 1.28-1.26 (d, 10H).
Molecular formula: $C_{20}H_{28}N_6O_2$ Exact mass: 384.23 LC-MS(Pos, *m*/*z*)=385.15[M+H]$^+$.

**Example 9: Synthesis of (1*S*,3*R*)-3-acetamido-*N*-(6-ethyl-8-(isopropylamino)pyrido[3,4-*d*]pyrimidin-2-yl)cyclohexane-1-carboxamide (compound 9)**

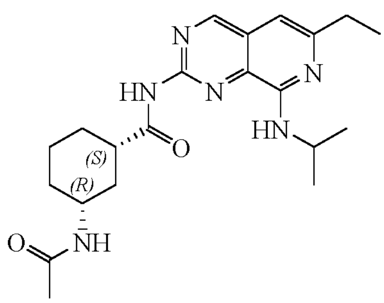

Step 1: synthesis of 6-ethyl-$N^8$-isopropylpyiido[3,4-*d*]pyiimidine-2,8-diamine

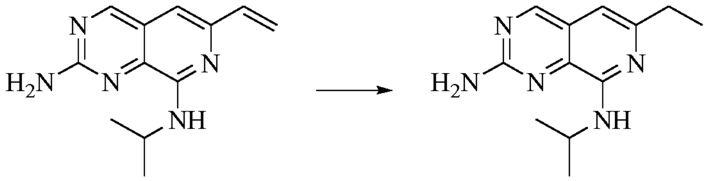

$N^8$-isopropyl-6-vinylpyrido[3,4-*d*]pyrimidine-2,8-diamine (200 mg, 0.87 mmol, 1.0 eq.) and 10% palladium on carbon (40 mg) were added into MeOH (5 mL), and the reaction solution was reacted for 3 h under hydrogen atmosphere. After the reaction was completed, as detected by TLC, the reaction solution was filtered, and the filtrate was concentrated under reduced pressure to give the product (201 mg, yield: 100%).

Step 2: synthesis of *tert*-butyl ((1*R*,3*S*)-3-((6-ethyl-8-(isopropylamino)pyrido[3,4-*d*]pyrimidin-2-yl)carbamoyl)cyclohexyl)carbamate

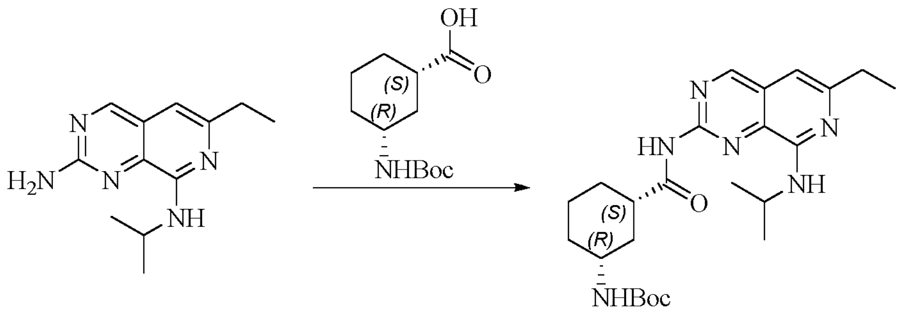

6-ethyl-$N^8$-isopropylpyrido[3,4-*d*]pyrimidine-2,8-diamine (201 mg, 0.87 mmol, 1.0 eq.) and (1S,3R)-3-((*tert*-butoxycarbonyl)amino)cyclohexane-1-carboxylic acid (234 mg, 0.96 mmol, 1.1 eq.) were dissolved in pyridine (4 mL), and the reaction solution was added with phosphorus oxychloride (667 mg, 4.35 mmol, 5.0 eq.) and reacted at room temperature for 20 min. After the reaction was completed, as detected by TLC, the reaction solution was poured into hydrochloric acid (20 mL), and the aqueous phase was extracted with EA (20 mL × 2). The organic phase was dried and concentrated to give a crude product, which was slurried with MTBE (10 mL) and filtered under vacuum, and the filter cake was dried to give the product (200 mg, yield: 50.3%).

Step 3: synthesis of (1*S*,3*R*)-3-amino-*N*-(6-ethyl-8-(isopropylamino)pyrido[3,4-*d*]pyrimidin-2-yl)cyclohexane-1-carboxamide hydrochloride

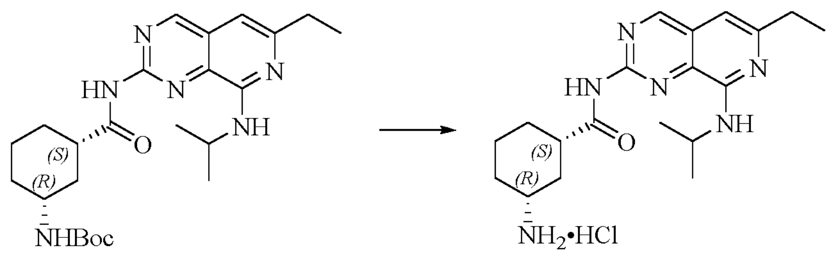

*Tert*-butyl ((1*R*,3*S*)-3-((6-ethyl-8-(isopropylamino)pyrido[3,4-*d*]pyrimidin-2-yl)carbamoyl)cyclohexyl)carbamate (200 mg, 0.44 mmol, 1.0 eq.) was dissolved in EA (4 mL), and the reaction solution was added dropwise a solution of hydrogen chloride in 1,4-dioxane (4 mol/L, 2 mL) and reacted at room temperature for 17 h. After the reaction was substantially completed, as detected by LC-MS, the reaction solution was filtered under vacuum give the product (172 mg, yeild: 100%).

Step 4: synthesis of (1*S*,3*R*)-3-acetamido-*N*-(6-ethyl-8-(isopropylamino)pyrido[3,4-*d*]pyrimidin-2-yl)cyclohexane-1 -carboxamide

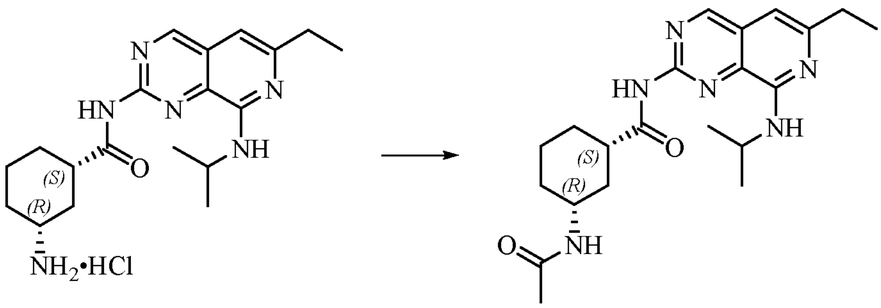

(1*S*,3*R*)-3-amino-*N*-(6-ethyl-8-(isopropylamino)pyrido[3,4-*d*]pyrimidin-2-yl)cyclohexane-1-carboxamide hydrochloride (172 mg, 0.44 mmol, 1.0 eq.), DIPEA (170 mg, 1.32 mmol, 3.0 eq.) and acetic anhydride (67 mg, 0.66 mmol, 1.5 eq.) were dissolved in DCM (4 mL), and the reaction solution was reacted at room temperature for 20 min. After the reaction was completed, as detected by LC-MS, the reaction solution was concentrated under reduced pressure to give a crude product, which was slurried with EA (10 mL) and filtered under vacuum. The filter cake was dried to give the product (40 mg, yield: 17.4%).

$^1$HNMR (300 MHz, DMSO-$d_6$) δ(ppm): 10.76 (s, 1H), 9.25 (s, 1H), 7.82-7.79 (d, 1H), 6.77 (s, 1H), 6.51-6.48 (d, 1H), 4.34-4.27 (m, 1H), 3.61-3.58 (t, 1H), 2.74-2.63 (m, 3H), 1.96-1.91 (d, 1H), 1.78 (s, 6H), 1.29-1.23 (m, 12H), 1.11-1.08 (t, 1H).

Molecular formula: $C_{21}H_{30}N_6O_2$ Exact mass: 398.24 LC-MS(Pos, *m*/*z*)=399.16[M+H]$^+$.

**Example 10: Synthesis of (1*S*,3*R*)-3-acetamido-*N*-(8-(isopropylamino)-6-vinylpyrido[3,4-*d*]pyrimidin-2-yl)cyclohexane-1-carboxamide (compound 10)**

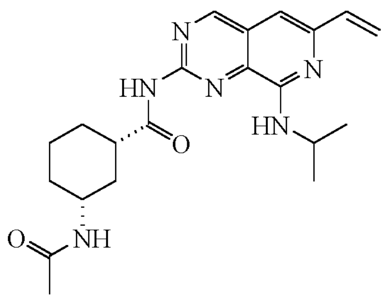

Step 1: synthesis of $N^8$-isopropyl-6-vinylpyrido[3,4-*d*]pyrimidine-2,8-diamine

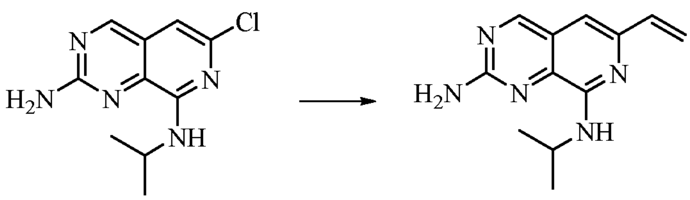

6-chloro-$N^8$-isopropylpyiido[3,4-*d*]pyiimidine-2,8-diamine(2.0 g, 8.41 mmol, 1.0 eq.), potassium vinylfluoroborate (2.25 g, 16.83 mmol, 2.0 eq.), cesium carbonate (5.48 g, 16.83 mmol, 2.0 eq.) and [1,1'-bis(diphenylphosphino)ferrocene]palladium dichloride (614 mg, 0.84 mmol, 0.1 eq.) were dissolved in $H_2O$ (5 mL) and dioxane (25 mL), and the reaction solution was reacted at 100 °C for 21 h in nitrogen atmosphere. After the reaction was completed, as detected by LC-MS, the reaction solution was poured into water (20 mL) and extracted with EA (30 mL × 2). The organic phase was dried and concentrated to give a crude product, which was purified by silica gel column chromatography (PE:EA= 5:1) to give the product (1.2 g, yield: 62.1%).

Step 2: synthesis of *tert*-butyl ((1*R*,3*S*)-3-((8-(isopropylamino)-6-vinylpyrido[3,4-*d*]pyrimidin-2-yl)carbamoyl)cyclohexyl)carbamate

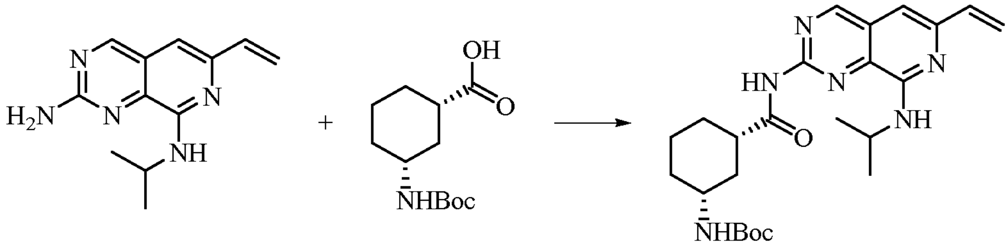

*N*[8]-isopropyl-6-vinylpyrido[3,4-*d*]pyrimidine-2,8-diamine (300 mg, 1.31 mmol, 1.0 eq.) and (1*S*,3*R*)-3-((*tert*-butoxycarbonyl)amino)cyclohexane-1-carboxylic acid (350 mg, 1.44 mmol, 1.1 eq.) were dissolved in pyridine (3 mL), and the reaction solution was added with phosphorus oxychloride (1.0 g, 6.54 mmol, 5.0 eq.) and reacted at room temperature for 0.5 h. After the reaction was completed, as detected by TLC, the reaction solution was poured into hydrochloric acid (20 mL) and extracted with EA (15 mL × 3). The organic phase was dried and concentrated to give a crude product, which was slurried with (PE:EA = 5:1, 12 mL) and filtered under vacuum, and the filter cake was dried to give the product (350 mg, yield: 58.8%).

Step 3: synthesis of (1*S*,3*R*)-3-amino-*N*-(8-(isopropylamino)-6-vinylpyrido[3,4-*d*]pyrimidin-2-yl)cyclohexane-1-carboxamide hydrochloride

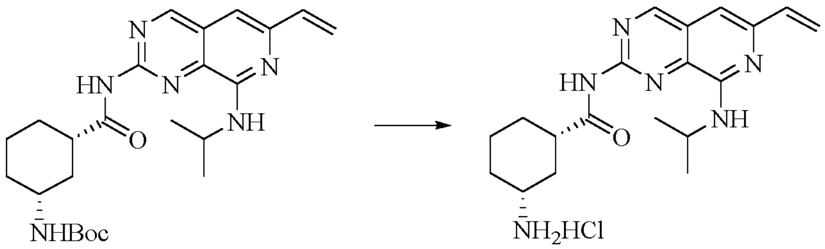

*Tert*-butyl ((1*R*,3*S*)-3-((8-(isopropylamino)-6-vinylpyrido[3,4-*d*]pyrimidin-2-yl)carbamoyl)cyclohexyl)carbamate (350 mg, 0.77 mmol, 1.0 eq.) was dissolved in EA (5 mL). The reaction solution was added dropwise with a solution of HCl in 1,4-dioxane (4 mol/L, 3 mL) and reacted at room temperature for 18 h. After the reaction was substantially completed, as detected by LC-MS, the reaction solution was filtered under vacuum give the product (300 mg, yeild: 100%).

Step 4: synthesis of (1*S*,3*R*)-3-acetamido-*N*-(8-(isopropylamino)-6-vinylpylido[3,4-*d*]pyrimidin-2-yl)cyclohexane-1-carboxamide

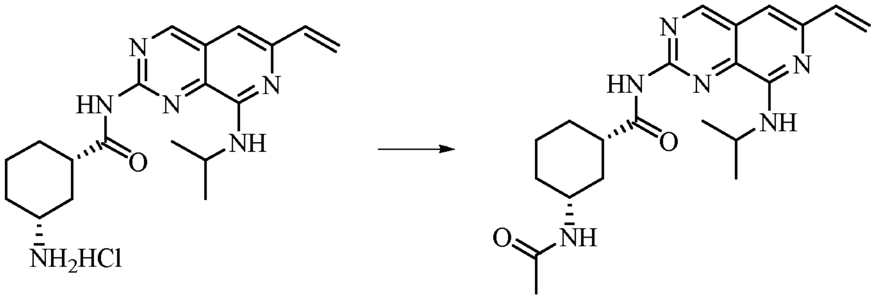

(1*S*,3*R*)-3-amino-*N*-(8-(isopropylamino)-6-vinylpyrido[3,4-*d*]pyrimidin-2-yl)cyclohexane-1-carboxamide hydrochloride (300 mg, 0.76 mmol, 1.0 eq.), DIPEA (298 mg, 2.13 mmol, 3.0 eq.) and acetic anhydride (117 mg, 1.15 mmol, 1.5 eq.) were dissolved in DCM (5 mL) and reacted at room temperature for 2.5 h. After the reaction was completed, as detected by LC-MS, the reaction solution was concentrated under reduced pressure to give a crude product, which was purified by silica gel column chromatography (MeOH:DCM = 1:50) to give the product (120 mg, yield: 39.4%).
$^1$HNMR (300 MHz, DMSO-$d_6$) δ(ppm): 10.84 (s, 1H), 9.28 (s, 1H), 7.82-7.80 (d, 1H), 6.91 (s, 1H), 6.80-6.71 (m, 1H), 6.59-6.56 (d, 1H), 6.27-6.21 (d, 1H), 5.39-5.36 (d, 1H), 4.37-4.25 (d, 1H), 3.58 (s, 1H), 2.75 (s, 1H), 1.96 (s, 1H), 1.78 (s, 6H), 1.32-1.30 (d, 9H), 1.11 (s, 1H).
Molecular formula: $C_{21}H_{28}N_6O_2$ Exact mass: 396.23 LC-MS(Pos, *m*/*z*)=397.18[M+H]$^+$.

**Example 11: Synthesis of (1*S*,3*R*)-3-acetamido-*N*-(8-(isopropylamino)-6-(methylamino)pyrido[3,4-*d*]pyrimidin-2-yl)cyclohexane-1-carboxamide (compound 11)**

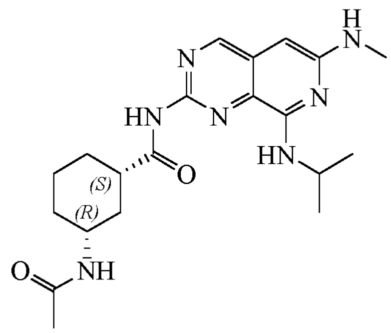

Step 1: synthesis of *tert*-butyl (8-(isopropylamino)-2-(methylthio)pyrido[3,4-*d*]pyrimidin-6-yl)(methyl)carbamate

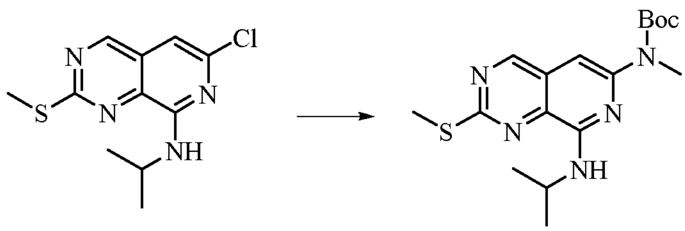

6-chloro-*N*-isopropyl-2-(methylthio)pyrido[3,4-*d*]pyrimidin-8-amine (1.5 g, 5.58 mmol, 1.0 eq.), *tert*-butyl methylcarbamate (1.10 g, 8.37 mmol, 1.5 eq.), 2-di-*tert*-butylphosphino-2',4',6' -triisopropylbiphenyl (476 mg, 1.12 mmol, 0.2 eq.), cesium carbonate (3.64 g, 11.16 mmol, 2.0 eq.) and allylpalladium chloride dimer (204 mg, 0.56 mmol, 0.1 eq.) were dispersed in dioxane (20 mL), and the reaction solution was reacted at 80 °C for 4.5 h in nitrogen atmosphere. After the reaction was completed, as detected by TLC, the reaction solution was concentrated under reduced pressure to give a crude product, which was purified by silica gel column chromatography (PE:EA = 30:1) to give the product (1.3 g, yield: 64.3%).

Step 2: synthesis of *tert*-butyl (8-(isopropylamino)-2-(methylsulfinyl)pyrido[3,4-*d*]pyrimidin-6-yl)(methyl)carbamate

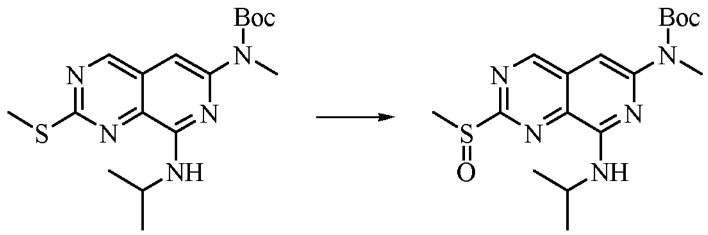

*Tert*-butyl (8-(isopropylamino)-2-(methylthio)pyrido[3,4-*d*]pyrimidin-6-yl)(methyl)carbamate (1.3 g, 3.57 mmol, 1.0 eq.) was dissolved in DCM (200 mL), and the reaction solution was added with *m*-chloroperoxybenzoic acid (mass fraction: 80%, 771 mg, 3.57 mmol, 1.0 eq.). After the reaction was completed, as detected by TLC, the reaction solution was poured into saturated aqueous potassium carbonate (20 mL), the liquid separation was performed, the aqueous phase was extracted with DCM (20 mL × 2), and the organic phases were combined, dried and concentrated to give the product (1.1 g, yield: 81.4%).

Step 3: synthesis of *tert*-butyl (2-amino-8-(isopropylamino)pyrido[3,4-*d*]pyrimidin-6-yl)(methyl)carbamate

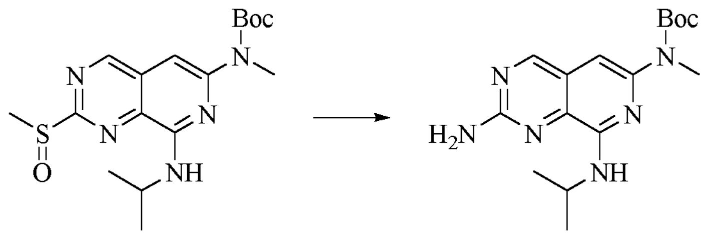

*Tert*-butyl (8-(isopropylamino)-2-(methylsulfinyl)pyrido[3,4-*d*]pyrimidin-6-yl)(methyl)carbamate (1.1 g, 2.90 mmol, 1.0 eq.) was dissolved in a solution of amino in isopropanol (3.5 mol/L, 20 mL), and the reaction solution was reacted at 150 °C for 15 h. After the reaction was completed, as detected by TLC, the reaction solution was concentrated under reduced pressure to give a crude product, which was purified by silica gel column chromatography (PE:EA = 4:1) to give the product (500 mg, yield: 51.9%).

Step 4: synthesis of *tert*-butyl (2-((1*S*,3*R*)-3-acetamidocyclohexane-1-carboxamido)-8-(isopropylamino)pyrido[3,4-*d*]pyrimidin-6-yl)(methyl)carbamate

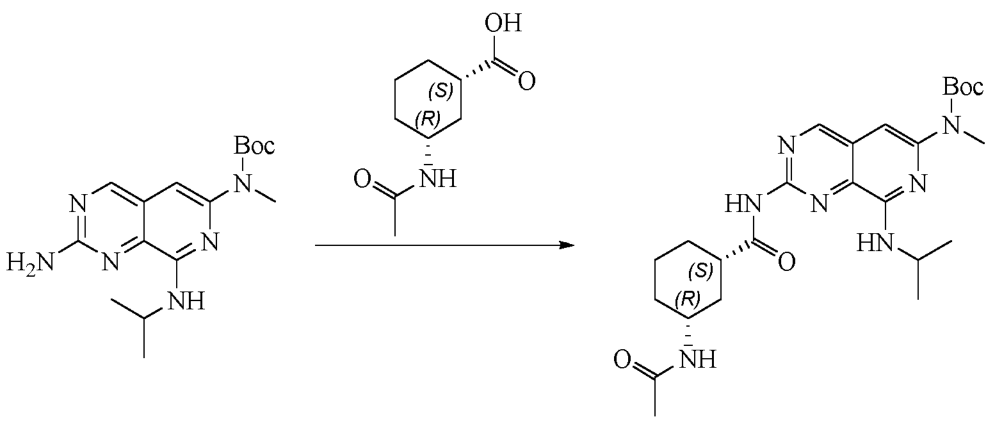

*Tert*-butyl (2-amino-8-(isopropylamino)pyrido[3,4-*d*]pyrimidin-6-yl)(methyl)carbamate (300 mg, 0.90 mmol, 1.0 eq.) and (1*S*,3*R*)-3-acetamidocyclohexane-1-carboxylic acid (183 mg, 0.99 mmol, 1.1 eq.) were dissolved in pyridine (3 mL), and the reaction solution was added with phosphorus oxychloride (414 mg, 2.70 mmol, 3.0 eq.) and reacted at

room temperature for 5 min. When the reaction was performed and there were materials left, as detected by TLC, the reaction solution was poured into EA (20 mL), washed with aqueous citric acid solution (30 mL × 3), and washed with saturated aqueous sodium bicarbonate solution (30 mL). The organic phase was dried and concentrated to give a crude product, which was purified by silica gel column chromatography (MeOH:DCM = 1:40) to give the product (130 mg, yield: 28.9%).

Step 5: synthesis of *tert*-butyl (2-((1*S*,3*R*)-3-acetamidocyclohexane-1-carboxamido)-8-(isopropylamino)pyrido[3,4-*d*]pyrimidin-6-yl)(methyl)carbamate

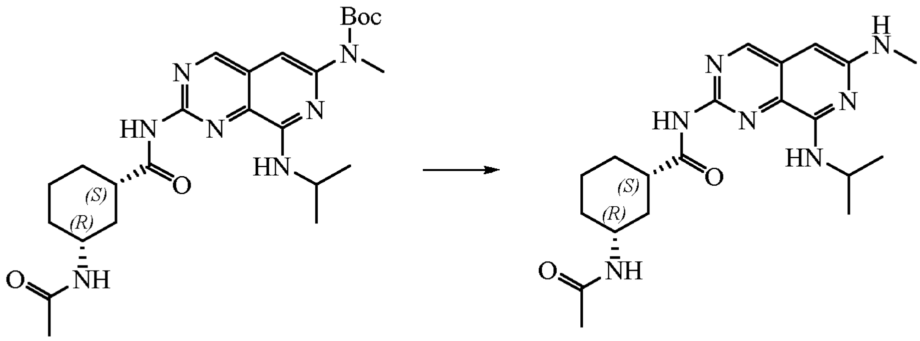

*Tert*-butyl (2-((1*S*,3*R*)-3-acetamidocyclohexane-1-carboxamido)-8-(isopropylamino)pyrido[3,4-*d*]pyrimidin-6-yl)(methyl)carbamate (120 mg, 0.24 mmol, 1.0 eq.) and 2,6-dimethylpyridine (386 mg, 3.60 mmol, 15 eq.) were dissolved in DCM (2 mL), and the reaction solution was cooled to 5 °C, added with trimethylsilyl trifluoromethanesulfonate (533 mg, 2.40 mmol, 10 eq.), and reacted for 20 min. After the reaction was completed, as detected by TLC, the reaction solution was poured into water (10 mL) and extracted with DCM (10 mL × 3). The organic phase was washed with saturated aqueous sodium bicarbonate solution (10 mL), dried and concentrated to give a crude product, which was slurried for 1h with MTBE (10 mL) and filtered under vacuum, and the filter cake was dried to give the product (56 mg, yield: 58.3%).
$^1$HNMR (300 MHz, DMSO-$d_6$) δ(ppm): 10.40 (s, 1H), 8.96 (s, 1H), 7.79-7.76 (d, 1H), 6.42-6.40 (d, 1H), 6.20-6.18 (d, 1H), 5.65 (s, 1H), 4.27-4.20 (m ,1H), 3.58-3.55 (t, 1H), 2.74-2.72 (d, 3H), 2.65-2.62 (d, 1H), 1.91-1.87 (d, 1H), 1.79 (s, 6H), 1.25-1.23 (d, 10H).
Molecular formula: $C_{20}H_{29}N_7O_2$ Exact mass: 399.24 LC-MS(Pos, *m*/*z*)=400.46[M+H]$^+$.

**Example 12: Synthesis of (1*S*,3*R*)-3-acetamido-*N*-(8-(isopropylamino)-6-methoxypyrido[3,4-*d*]pyrimidin-2-yl)cyclohexane-1-carboxamide (compound 12)**

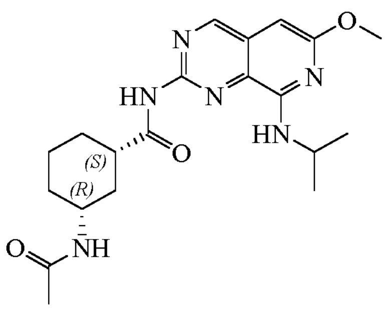

Step 1: synthesis of (1*S*,3*R*)-3-aminocyclohexane-1-carboxylic acid hydrochloride

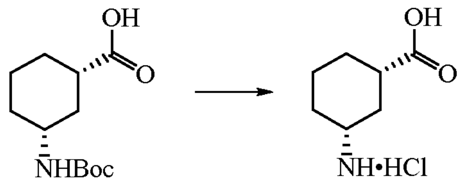

(1*S*,3*R*)-3-((*tert*-butoxycarbonyl)amino)cyclohexane-1-carboxylic acid (2.0 g, 8.22 mmol, 1.0 eq.) was dissolved in EA (20 mL), and the reaction solution was added with a solution of hydrogen chloride in 1,4-dioxane (4 mol/L, 10 mL) and reacted at room temperature for 17 h. The reaction solution was filtered under vacuum give the product (1.476 g, yeild: 100%).

Step 2: synthesis of (1*S*,3*R*)-3-acetamidocyclohexane-1-carboxylic acid

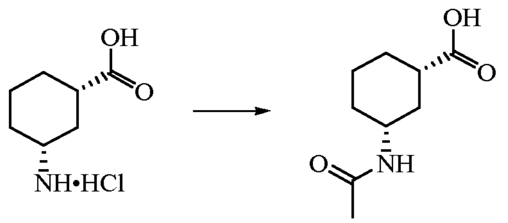

(1*S*,3*R*)-3-((*tert*-butoxycarbonyl)amino)cyclohexane-1-carboxylic acid (1.476 g, 8.22 mmol, 1.0 eq.) and triethylamine (2.49 g, 24.65 mmol, 3.0 eq.) were dissolved in DCM (20 mL) and isopropanol (2 mL), and the reaction solution was added with acetic anhydride (1.26 mg, 12.32 mmol, 1.5 eq.) and reacted at room temperature for 16 min. After the reaction was completed, as detected by LC-MS, the reaction solution was concentrated under reduced pressure to give a crude product, which was dissolved with water (20 mL) and adjusted to pH = 1 with hydrochloric acid. The aqueous phase was extracted with EA (20 mL × 3). The organic phase was dried and concentrated to give a crude product, which was slurried with (PE:MTBE = 1:1, 30 mL) and filtered under vacuum, and the filter cake was dried to give the product (1.3 g, yield: 85.56%).

Step 3: synthesis of *N*-isopropyl-6-methoxy-2-(methylthio)pyrido[3,4-*d*]pyrimidin-8-amine

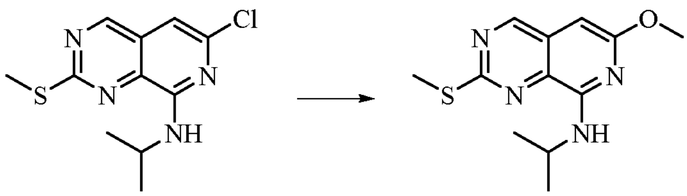

6-chloro-*N*-isopropyl-2-(methylthio)pyrido[3,4-*d*]pyrimidin-8-amine (1.5 g, 5.58 mmol, 1.0 eq.), 2-di-*tert*-butylphosphino-2',4',6' -triisopropylbiphenyl (476 mg, 1.12 mmol, 0.2 eq.), cesium carbonate (3.64 g, 11.16 mmol, 2.0 eq.) and allylpalladium chloride dimer (205 mg, 0.56 mmol, 0.1 eq.) were dispersed in methanol (5 mL) and 1,4-dioxane (15 mL), and the reaction solution was reacted at 80 °C for 0.5 h in nitrogen atmosphere. After the reaction was completed, as detected by TLC, the reaction solution was concentrated under reduced pressure to give a crude product, which was purified by silica gel column chromatography (PE:EA = 20: 1) to give the product (1.2 g, yield: 81.6%).

Step 4: synthesis of *N*-isopropyl-6-methoxy-2-(methylsulfinyl)pyrido[3,4-*d*]pyrimidin-8-amine

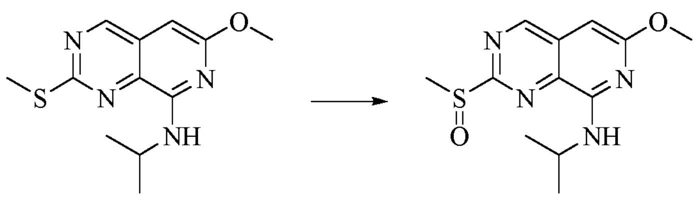

8-(isopropylamino)-2-(methylthio)pyrido[3,4-*d*]pyrimidine-6-carbonitrile (1.2 g, 4.54 mmol, 1.0 eq.) was dissolved in DCM (200 mL), and the reaction solution was added with *m*-chloroperoxybenzoic acid (mass fraction: 80%, 979 mg, 4.54 mmol, 1.0 eq.). After the reaction was completed, as detected by TLC, the reaction solution was poured into saturated aqueous sodium carbonate (20 mL) and extracted with DCM (15 mL × 2). The organic phase was dried and concentrated to give a crude product, which was purified by silica gel column chromatography (MeOH: DCM = 1:80) to give the product (1.0 g, yield: 78.7%).

Step 5: synthesis of *N*-isopropyl-6-methoxypyrido[3,4-*d*]pyrimidine-2,8-diamine

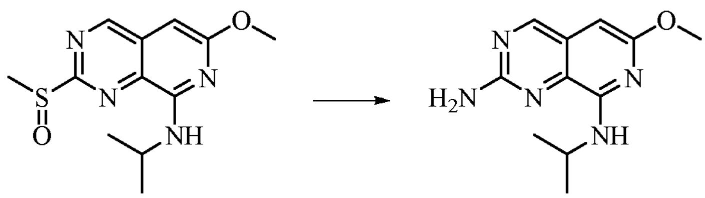

*N*-isopropyl-6-methoxy-2-(methylsulfinyl)pyrido[3,4-*d*]pyrimidin-8-amine (1.0 g, 3.56 mmol, 1.0 eq.) was dissolved in a solution of amino in isopropanol (3.5 mol/L, 20 mL), and the reaction solution was reacted at 150 °C for 17 h. After the reaction was completed, as detected by TLC, the reaction solution was cooled to room temperature and concentrated under reduced pressure to give a crude product, which was purified by silica gel column chromatography (PE:EA= 3:1) to give the product (200 mg, yield: 24.0%).

Step 6: synthesis of (1*S*,3*R*)-3-acetamido-*N*-(8-(isopropylamino)-6-methoxypyrido[3,4-*d*]pyrimidin-2-yl)cyclohexane-1-carboxamide

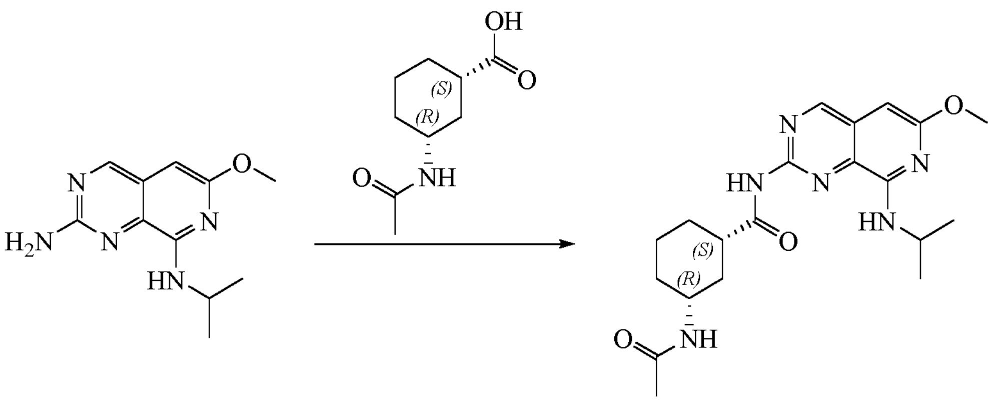

$N^8$-isopropyl-6-methoxypyiido[3,4-*d*]pyiimidine-2,8-diamine (100 mg, 0.42 mmol, 1.0 eq.) and (1*S*,3*R*)-3-acetamidocyclohexane-1-carboxylic acid (85 mg, 0.46 mmol, 1.1 eq.) were dissolved in pyridine (2 mL), and the reaction solution was added with phosphorus oxychloride (193 mg, 1.26 mmol, 3.0 eq.) and reacted at room temperature for 5 min. When a product was generated, as detected by TLC, the reaction solution was poured into EA (20 mL). EA phase was washed with aqueous citric acid solution (20 mL × 3), and washed with saturated aqueous sodium bicarbonate solution (20 mL). The organic phase was dried and concentrated to give a crude product, which was slurried with EA (3

mL) and filter under vacuum to give the product (20 mg, yield: 11.9%).
$^1$HNMR (300 MHz, DMSO-$d_6$) δ(ppm): 10.63 (s, 1H), 9.18 (s, 1H), 7.79-7.77 (d, 1H), 6.71-6.68 (d, 1H), 6.21 (s, 1H), 4.26-4.22 (t, 1H), 3.83 (s, 3H), 3.58 (s, 2H), 2.70 (s, 1H), 1.93-1.89 (d, 1H), 1.77 (s, 5H), 1.29-1.27 (d, 10H).
Molecular formula: $C_{20}H_{28}N_6O_3$ Exact mass: 400.22 LC-MS(Pos, *m/z*)=401.20[M+H]$^+$.

**Example 13: Synthesis of (1*S*,3*R*)-3-acetamido-*N*-(7-ethynyl-5-(isopropylamino)-2,6-naphthyridin-3-yl)cyclohexane-1-carboxamide (compound 13)**

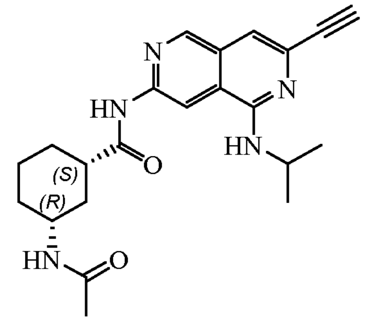

Steps:

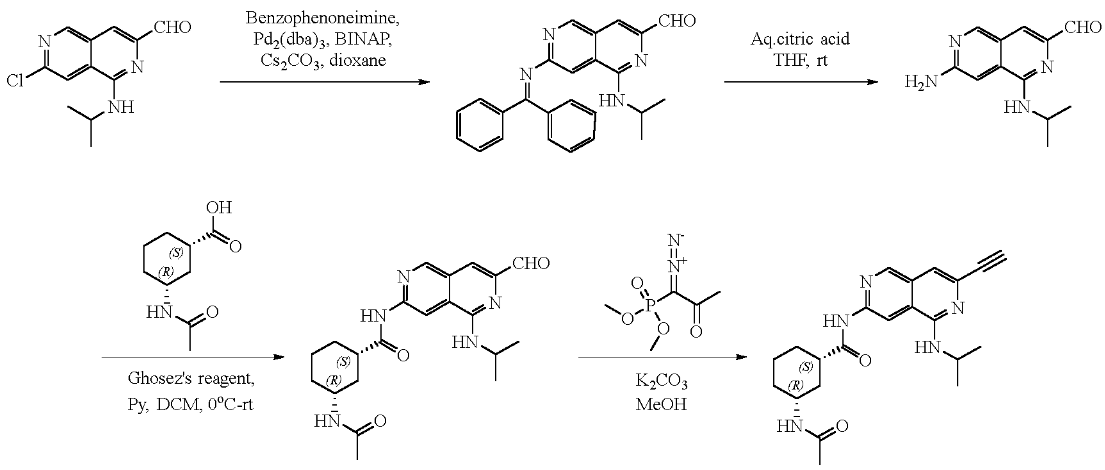


Step 1: synthesis of 7-((diphenylmethylene)amino)-1-(isopropylamino)-2,6-naphthyridine-3-carbaldehyde

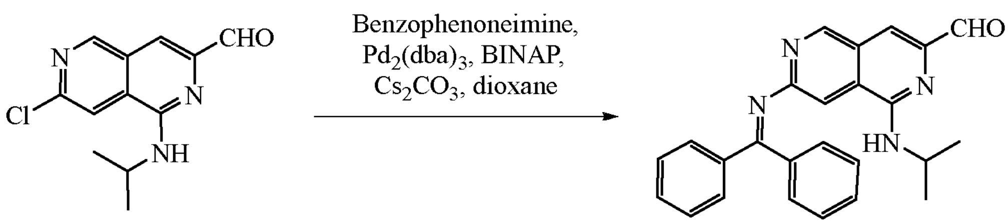


7-chloro-1-(isopropylamino)-2,6-naphthyridine-3-carbaldehyde (249.7 mg, 1 mmol, 1.0 eq.), diphenylmethanimine (217.5 mg, 1.2 mmol, 1.2 eq.), $Pd_2(dba)_3$ (91.5 mg, 0.1 mmol, 0.1 eq.), BINAP (124.5 mg, 0.2 mmol, 0.2 eq.) and cesium carbonate (814.5 mg, 5.27 mmol, 2.5 eq.) were added into 1,4-dioxane (10 mL). The mixture was reacted at 120 °C for 18 h in a sealed tube in nitrogen atmosphere. When there were no materials left, as detected by TLC, the

reaction solution was added with ethyl acetate (20 mL), stirred for 5 min and filtered, the filter cake was rinsed with ethyl acetate, and the filtrate was concentrated under reduced pressure to give a crude product, which was purified by silica gel column chromatography (EA:PE = 1:20-1:6) to give the product (340 mg, yield: 86.1%).

Step 2: synthesis of 7-amino-1-(isopropylamino)-2,6-naphthyridine-3-carbaldehyde

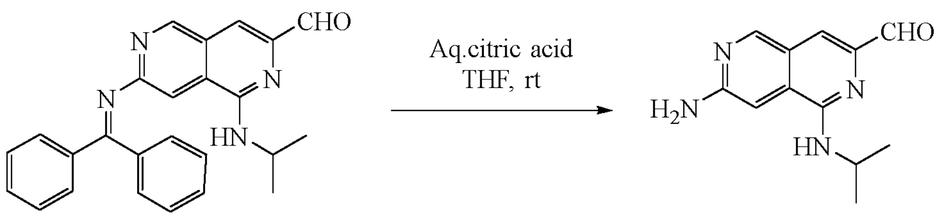


7-((diphenylmethylene)amino)-1-(isopropylamino)-2,6-naphthyridine-3-carbaldehyde (340 mg, 0.86 mmol, 1.0 eq.) was dissolved in THF (5 mL). The reaction solution was added with aqueous citric acid solution (mass fraction: 10%, 5 mL) and reacted at room temperature for 17 h. When there were no materials left, as detected by TLC, the reaction solution was added with saturated aqueous sodium carbonate (20 mL) and extracted with ethyl acetate (30 mL × 3). The organic phases were combined, dried over anhydrous sodium sulfate and filtered, and the filtrate was concentrated under reduced pressure to give a crude product, which was purified by silica gel column chromatography (EA:PE = 1:10-1:1) to give the product (123 mg, yield: 62.1%).

Step 3: synthesis of (1*S*,3*R*)-3-acetamido-*N*-(7-formyl-5-(isopropylamino)-2,6-naphthyridin-3-yl)cyclohexane-1-carboxamide

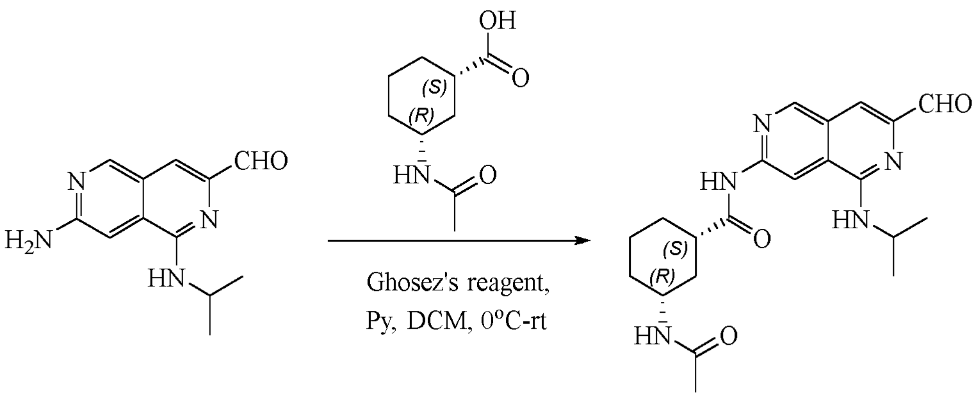


(1*S*,3*R*)-3-acetamidocyclohexane-1-carboxylic acid (101.8 mg, 0.55 mmol, 1.1 eq.) was dissolved in DCM (5 mL). The reaction solution was cooled to 0 °C to 5 °C in an ice water bath, added with 1-chloro-*N,N*,2-trimethylprop-1-en-1-amine (mass fraction: 95%, 105.4 mg, 0.75 mmol, 1.5 eq.), reacted at 0 °C to 5 °C for 1.5 h, added with a solution of 7-amino-1-(isopropylamino)-2,6-naphthyridine-3-carbaldehyde (115 mg, 0.50 mmol, 1.0 eq.) and pyridine (118.6 mg, 1.50 mmol, 3.0 eq.) in THF (10 mL), and incubated at room temperature for 17 h. When there were a small amount of materials left, as detected by TLC, the reaction solution was added with saturated aqueous sodium carbonate (20 mL) and extracted with ethyl acetate (20 mL × 3). The organic phases were combined, dried over anhydrous sodium sulfate and filtered, and the filtrate was concentrated under reduced pressure to give the product (150 mg, yield: 75.5%).

Step 4: synthesis of (1*S*,3*R*)-3-acetamido-*N*-(7-ethynyl-5-(isopropylamino)-2,6-naphthyridin-3-yl)cyclohexane-1-carboxamide

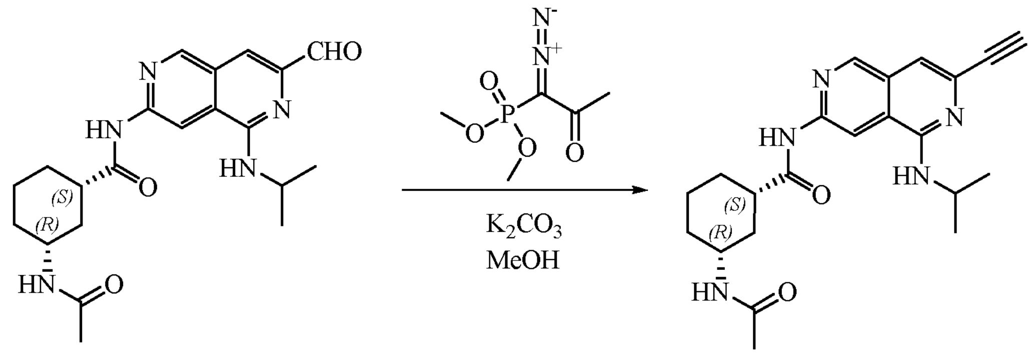


(1*S*,3*R*)-3-acetamido-*N*-(7-formyl-5-(isopropylamino)-2,6-naphthyridin-3-yl)cyclohexane-1-carboxamide (150 mg, 0.37 mmol, 1.0 eq.), dimethyl (1-diazo-2-oxopropyl)phosphonate (107.6 mg, 0.56 mmol, 1.5 eq.) and potassium carbonate (153.2 mg, 1.11 mmol, 3.0 eq.) were dissolved in methanol (5 mL), and the reaction solution was reacted at room temperature for 2 h. When there were no materials left, as detected by TLC, the reaction solution was added with saturated aqueous sodium carbonate (20 mL) and extracted with ethyl acetate (30 mL × 3). The organic phases were combined, dried over anhydrous sodium sulfate and filtered, and the filtrate was concentrated under reduced pressure to give a crude product, which was purified by preparative thin-layer chromatography (MeOH:DCM = 1:20) to give the product (41 mg, yield: 28.2%).
$^1$HNMR (400 MHz, $CD_3OD$) δ(ppm): 8.83 (s, 1H), 8.44 (s, 1H), 7.19 (s, 1H), 4.52-4.46 (m, 1H), 3.80-3.75 (m, 1H), 3.50 (m, 1H), 2.69-2.63 (m, 1H), 2.13-2.10 (m, 1H), 1.95 (s, 3H), 1.54-1.48 (m,4H), 1.35-1.31(m, 9H).
Molecular formula: $C_{22}H_{27}N_5O_2$ Exact mass: 393.22 LC-MS (Pos, *m*/*z*) =394.27 $[M+H]^+$.

**Example 14: Synthesis of (1*S*,3*R*)-3-acetamido-*N*-(7-(2-hydroxyethyl)-5-(isopropylamino)-2,6-naphthyridin-3-yl)cyclohexane-1-carboxamide (compound 14)**

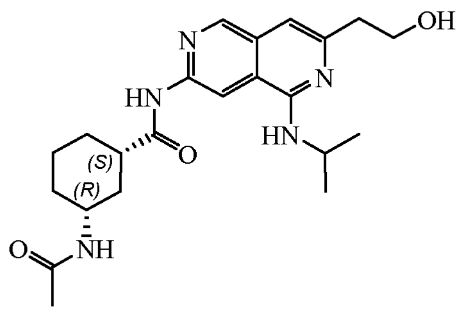

Steps:

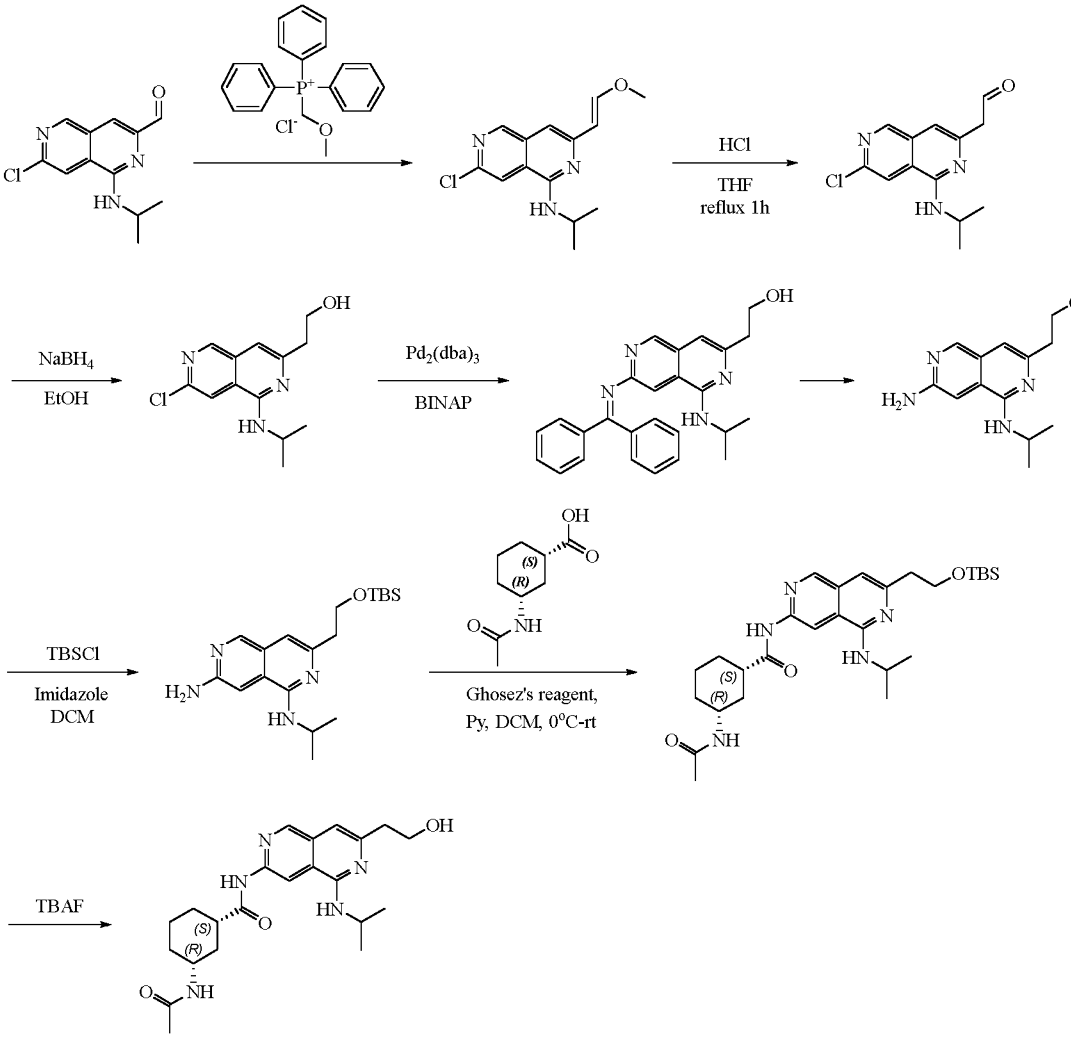


Step 1: synthesis of (*E*)-7-chloro-*N*-isopropyl-3-(2-methoxyvinyl)-2,6-naphthyridin-1-amine

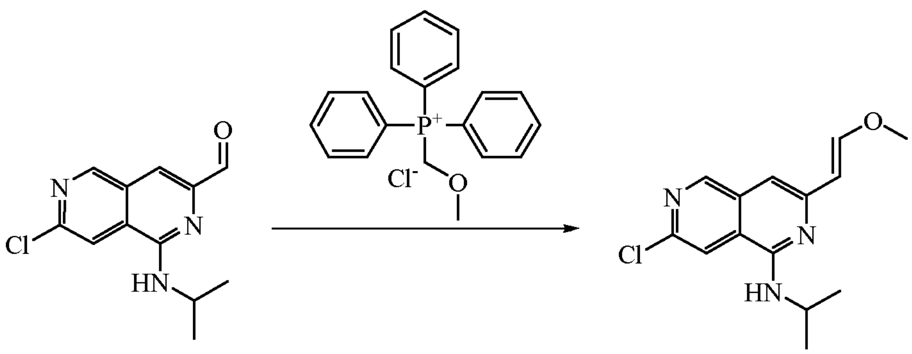

(Methoxymethyl)triphenylphosphonium (4.9 g, 14.4 mmol, 3.0 eq.) was added into anhydrous THF (30 mL). The reaction solution was cooled to 0 °C in an ice water bath in nitrogen atmosphere, added dropwise with a solution of potassium *tert*-butoxide (1.6 g, 14.4 mmol, 3.0 eq.) in anhydrous THF (5 mL), and reacted at 0 °C to 5 °C for 40 min after addition. The reaction solution was added dropwise with 7-chloro-1-(isopropylamino)-2,6-naphthyridine-3-carbaldehyde (1.2 g, 4.8 mmol, 1.0 eq.) in anhydrous THF (10 mL), and incubated at room temperature for 48 h. When there were materials left, as detected by TLC, the reaction solution was added with water (30 mL) and extracted with ethyl

acetate (50 mL × 2). The organic phases were combined, dried over anhydrous sodium sulfate and filtered, and the filtrate was concentrated under reduced pressure to give a crude product, which was purified by silica gel column chromatography (EA:PE = 1:50-1:30) to give the product (710 mg, yield: 54.6%).

Step 2: synthesis of 2-(7-chloro-1-(isopropylamino)-2,6-naphthyridin-3-yl)acetaldehyde

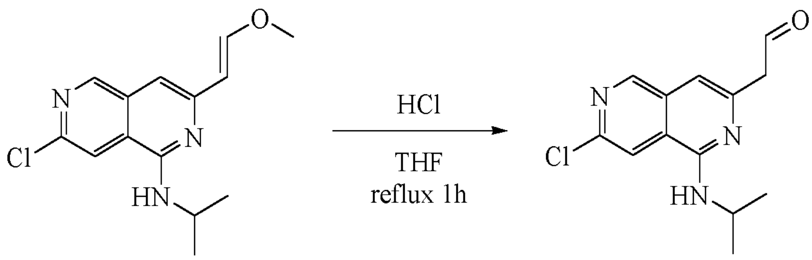


(*E*)-7-chloro-*N*-isopropyl-3-(2-methoxyvinyl)-2,6-naphthyridin-1-amine (710 mg, 2.56 mmol, 1.0 eq.) was dissolved in THF (12 mL), and the reaction solution was added with aqueous hydrochloric acid solution (4 mol/L, 3 mL), heated to 80 °C and reacted for 1 h. When there were no materials left, as detected by TLC, the reaction solution was concentrated under reduced pressure, added with water (20 mL), adjusted to pH of about 8 with sodium carbonate and extracted with ethyl acetate (20 mL × 3). The organic phases were combined, dried over anhydrous sodium sulfate and filtered, and the filtrate was concentrated under reduced pressure to give the product, which was used in the next step according to a theoretical amount.

Step 3: synthesis of 2-(7-chloro-1-(isopropylamino)-2,6-naphthyridin-3-yl)ethanol

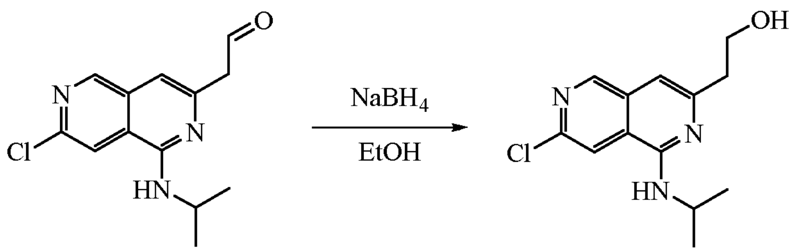


2-(7-chloro-1-(isopropylamino)-2,6-naphthyridin-3-yl)acetaldehyde (2.56 mmol, 1.0 eq.) was dissolved in absolute ethanol (10 mL). The reaction solution was added with sodium triacetoxyborohydride (968.4 mg, 25.6 mmol, 10 eq.) and reacted at room temperature for 0.5 h. When there were no materials left, as detected by TLC, the reaction solution was concentrated under reduced pressure, added with water (10 mL), and extracted with ethyl acetate (20 mL × 3). The organic phases were combined, dried over anhydrous sodium sulfate and filtered, and the filtrate was concentrated under reduced pressure to give a crude product, which was purified by silica gel column chromatography (EA:PE = 1:10-1:3) to give the product (353 mg, two-step yield: 54.6%).

Step 4: synthesis of 2-(7-((diphenylmethylene)amino)-1-(isopropylamino)-2,6-naphthyridin-3 -yl)ethan-1 -ol

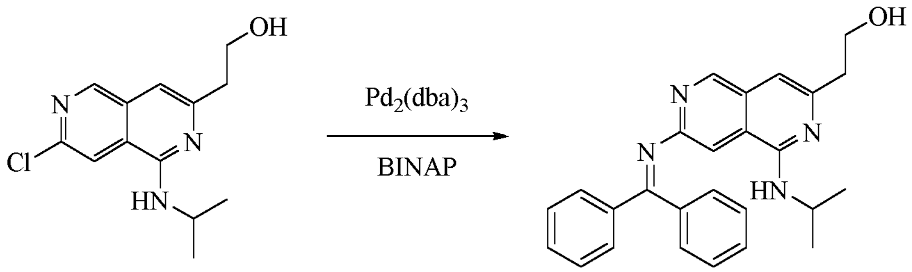


2-(7-chloro-1-(isopropylamino)-2,6-naphthyridin-3-yl)ethanol (350 mg, 1.32 mmol, 1.0 eq.), diphenylmethanimine (286.3 mg, 1.58 mmol, 1.2 eq.), $Pd_2(dba)_3$ (119 mg, 0.13 mmol, 0.1 eq.), BINAP (161.9 mg, 0.26 mmol, 0.2 eq.) and cesium carbonate (1.1 g, 3.3 mmol, 2.5 eq.) were added into 1,4-dioxane (15 mL). The mixture was reacted at 120 °C for 16 h in a sealed tube in nitrogen atmosphere. When there were no materials left, as detected by TLC, the reaction solution was added with ethyl acetate (20 mL), stirred for 20 min and filtered, and the filtrate was concentrated under reduced pressure to give a crude product, which was purified by silica gel column chromatography (EA:PE = 1:10-1:3) to give the product (457 mg, yield: 84.3%).

Step 5: synthesis of 2-(7-amino-1-(isopropylamino)-2,6-naphthyridin-3-yl)ethan-1-ol

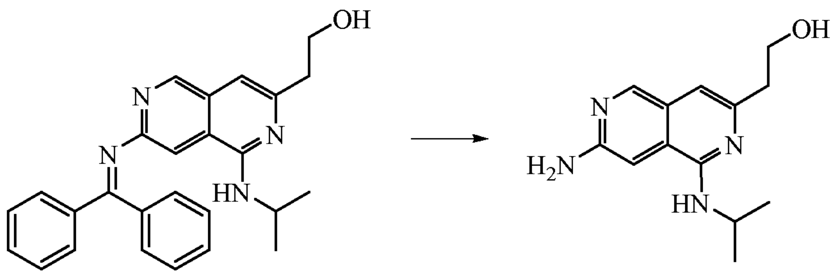

2-(7-((diphenylmethylene)amino)-1-(isopropylamino)-2,6-naphthyridin-3-yl)ethan-1-ol (457 mg, 1.11 mmol, 1.0 eq.) was dissolved in THF (10 mL). The reaction solution was added with aqueous citric acid solution (mass fraction: 10%, 10 mL) and reacted at room temperature for 1 h. When there were no materials left, as detected by TLC, the reaction solution was added with water (10 mL), adjusted to pH of about 8 with sodium carbonate and extracted with ethyl acetate (20 mL × 2). The organic phases were combined, dried over anhydrous sodium sulfate and filtered, and the filtrate was concentrated under reduced pressure to give a crude product, which was slurried for 5 min with methyl *tert*-butyl ether (5 mL) and filtered, and the filter cake was dried to give the product (191 mg, yield: 69.8%).

Step 6: synthesis of 3-(2-((*tert*-butyldimethylsilyl)oxy)ethyl)-$N^1$-isopropyl-2,6-naphthyridine-1,7-diamine

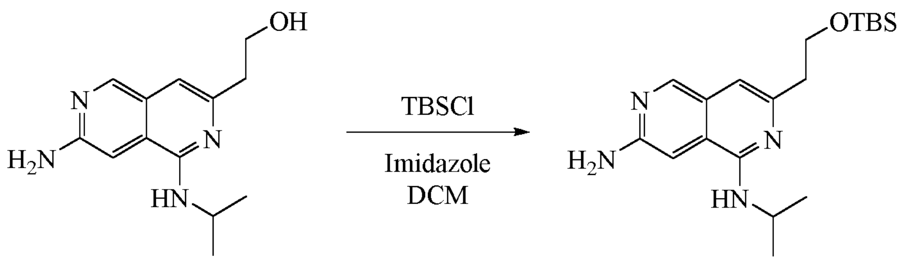


2-(7-amino-1-(isopropylamino)-2,6-naphthyridin-3-yl)ethanol (190 mg, 0.77 mmol, 1.0 eq.) was dissolved in DCM (5 mL). The reaction solution was added with imidazole (157.3 mg, 2.31 mmol, 3.0 eq.) and *tert*-butyldimethylsilyl chloride (173.3 mg, 1.15 mmol, 1.5 eq.), and reacted at room temperature for 15 h. When there was about 50% materials left, as detected by TLC, the reaction solution was concentrated under reduced pressure, added with water (20 mL),

and extracted with ethyl acetate (20 mL × 3). The organic phases were combined, dried over anhydrous sodium sulfate and filtered, and the filtrate was concentrated under reduced pressure to give a crude product, which was purified by preparative thin-layer chromatography (MeOH:DCM = 1:20) to give the product (110 mg, yield: 39.6%).

Step 7: synthesis of (1*S*,3R)-3-acetamido-*N*-(7-(2-((*tert*-butyldimethylsilyl)oxy)ethyl)-5-(isopropylamino)-2,6-naphthyridin-3-yl)cyclohexane-1-carboxamide

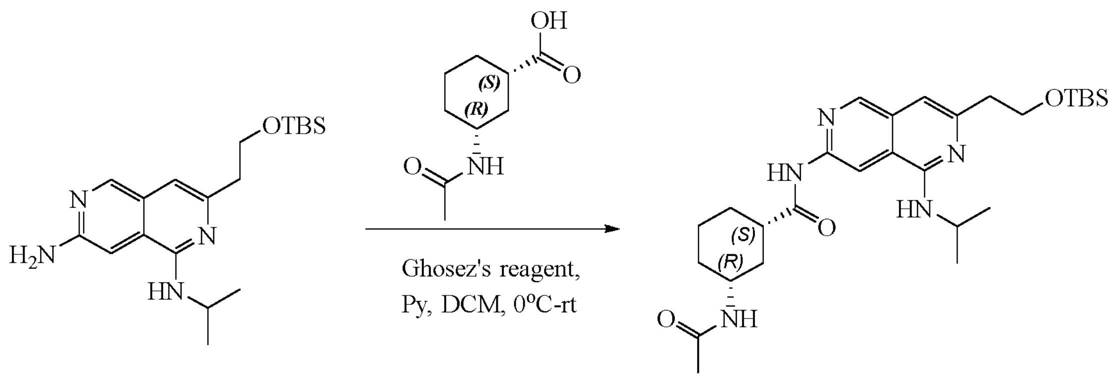


(1*S*,3*R*)-3-acetamidocyclohexane-1-carboxylic acid (66.7 mg, 0.36 mmol, 1.2 eq.) was dissolved in DCM (3 mL). The reaction solution was cooled to 0 °C to 5 °C in an ice water bath, added with 1-chloro-*N,N*,2-trimethylprop-1-en-1-amine (mass fraction: 95%, 63.3 mg, 0.45 mmol, 1.5 eq.), reacted at 0 °C to 5 °C for 1.5 h, added with a solution of 3-(2-((*tert*-butyldimethylsilyl)oxy)ethyl)-$N^1$-isopropyl-2,6-naphthyridine-1,7-diamine (110 mg, 0.30 mmol, 1.0 eq.) and pyridine (71.2 mg, 0.90 mmol, 3.0 eq.) in DCM (2 mL), and incubated at room temperature for 18 h. When there were 16% materials left, as detected by LC-MS, the reaction solution was added with water (20 mL), adjusted to pH of about 8 with sodium carbonate and extracted with ethyl acetate (20 mL × 3). The organic phases were combined, dried over anhydrous sodium sulfate and filtered, and the filtrate was concentrated under reduced pressure to give a crude product, which was purified by preparative thin-layer chromatography (MeOH:DCM = 1:20) to give the product (48 mg, yield: 30.3%).

Step 8: synthesis of (1*S*,3*R*)-3-acetamido-*N*-(7-(2-hydroxyethyl)-5-(isopropylamino)-2,6-naphthyridin-3-yl)cyclohexane-1-carboxamide

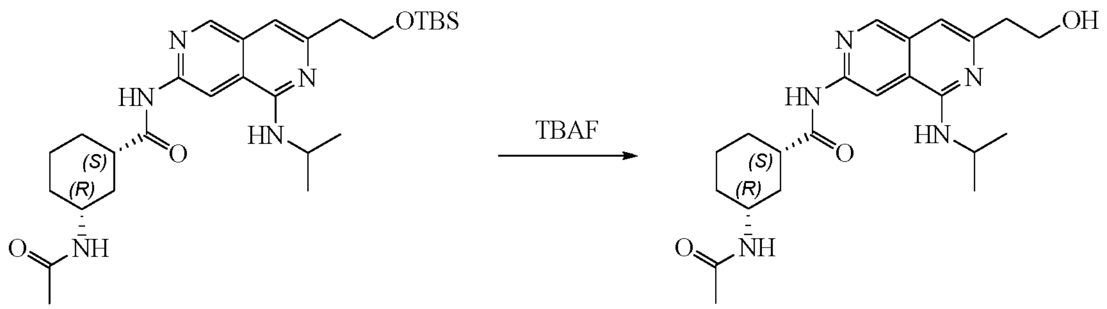


(1*S*,3*R*)-3-acetamido-*N*-(7-(2-((*tert*-butyldimethylsilyl)oxy)ethyl)-5-(isopropylamino)-2,6-naphthyridin-3-yl)cyclohexane-1-carboxamide (48 mg, 0.09 mmol, 1.0 eq.) was dissolved in THF (2 mL), and the reaction solution was added with a solution of tetra-n-butylammonium fluoridereacted (1 mol/L) in THF (0.18 mL, 0.18 mmol, 2.0 eq.), and reacted at room temperature for 2 h. When there were no materials left, as detected by TLC, the reaction solution was added with water (10 mL) and extracted with DCM (10 mL × 4). The organic phases were combined, dried over anhydrous sodium sulfate and filtered, and the filtrate was concentrated under reduced pressure to give a crude product, which was purified by preparative thin-layer chromatography (MeOH:DCM = 1: 10) to give the product (27 mg, yield: 72.5%). $^1$HNMR (400 MHz, $CD_3OD$) δ(ppm): 8.79 (s, 1H), 8.39 (s, 1H), 6.83 (s, 1H), 4.44-4.00 (m, 1H), 3.98-3.81 (m, 2H), 3.79-3.75 (m, 1H), 3.28-3.24 (m, 2H), 2.95-2.92 (m, 2H), 2.68-2.62 (m, 1H), 2.13-2.10 (m, 1H), 2.00 (s, 3H), 1.70-1.66

(m, 1H), 1.55-1.49 (m, 2H), 1.47-1.41 (m, 2H), 1.40-1.36 (m, 6H).
Molecular formula: $C_{22}H_{31}N_5O_2$ Exact mass: 413.24 LC-MS (Pos, *m*/*z*) =414.33 $[M+H]^+$.

**Example 15: Synthesis of (1*S*,3*R*)-3-acetamido-*N*-(5-(*tert*-butylamino)-7-cyano-2,6-naphthyridin-3-yl)cyclohexane-1-carboxamide (compound 15)**

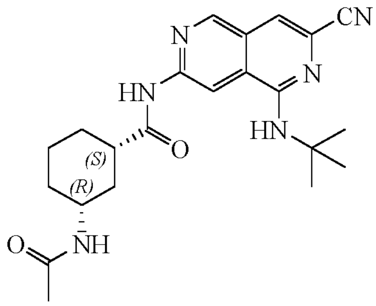

Step 1: synthesis of (1*S*,3*R*)-3-acetamido-*N*-(5-(*tert*-butylamino)-7-cyano-2,6-naphthyridin-3-yl)cyclohexane-1-carboxamide

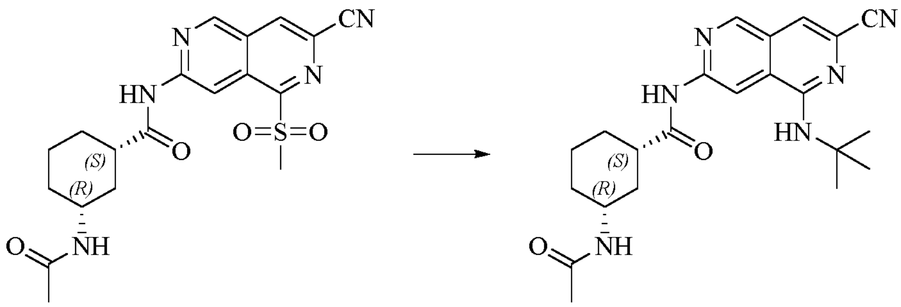

(1*S*,3*R*)-3-acetamido-*N*-(7-cyano-5-(methylsulfonyl)-2,6-naphthyridin-3-yl)cyclohexane-1-carboxamide (80 mg, 0.19 mmol, 1.0 eq.) and *tert*-butylamine (69 mg, 0.95 mmol, 5.0 eq.) were dissolved in THF (3 mL), and the reaction solution was reacted at 80 °C for 20 h. After the reaction was completed, as detected by TLC, the reaction solution was concentrated under reduced pressure to give a crude product, which was slurried with DCM (2 mL) and filtered under vacuum. The filter cake was dried at 50 °C to give the product (25 mg, yield: 31.6%).
$^1$HNMR (300 MHz, DMSO-$d_6$) δ(ppm): 10.83 (s, 1H), 9.01 (s, 1H), 8.67 (s, 1H), 7.83-7.81 (d, 1H), 7.70 (s, 1H), 7.06 (s ,1H), 3.62-3.59 (d, 1H), 2.68 (s, 1H), 1.96-1.92 (d, 1H), 1.79 (s, 6H), 1.52 (s, 9 H), 1.34-1.25 (m, 4H).
Molecular formula: $C_{22}H_{28}N_6O_2$ Exact mass: 408.23 LC-MS(Pos, *m*/*z*)=409.22$[M+H]^+$.

**Example 16: Synthesis of (1*S*,3*R*)-3-acetamido-*N*-(7-cyano-5-(cyclopropylamino)-2,6-naphthyridin-3-yl)cyclohexane-1-carboxamide (compound 16)**

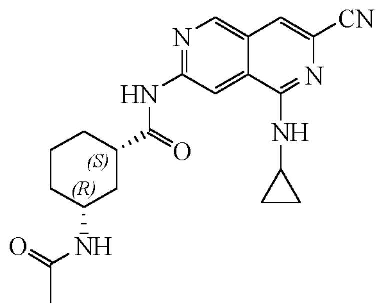

Step 1: synthesis of (1*S*,3*R*)-3-acetamido-*N*-(7-cyano-5-(cyclopropylamino)-2,6-naphthyridin-3-yl)cyclohexane-1-carboxamide

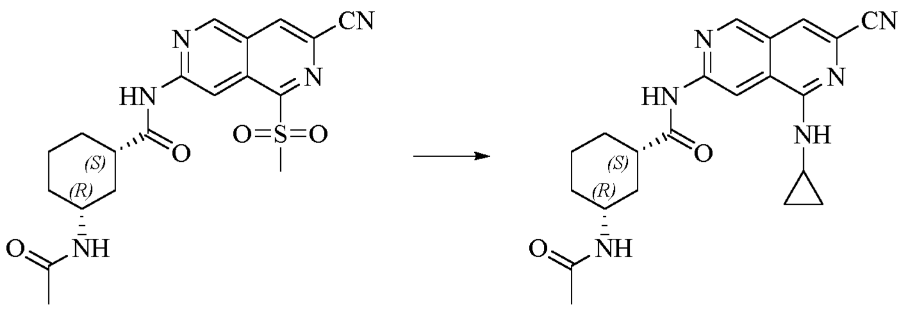

(1*S*,3*R*)-3-acetamido-*N*-(7-cyano-5-(methylsulfonyl)-2,6-naphthyridin-3-yl)cyclohexane-1-carboxamide (80 mg, 0.19 mmol, 1.0 eq.) and cyclopropylamine (54 mg, 0.95 mmol, 5.0 eq.) were dissolved in THF (2 mL), and the reaction solution was reacted at 40 °C for 2 h. After the reaction was completed, as detected by LC-MS, the reaction system was poured into water (5 mL) and extracted with EA (10 mL × 3). The organic phase was dried and concentrated to give a crude product. The crude product was slurried with (MTBE:EA= 1:1) and filtered under vacuum, and the filter cake was dried at 50 °C to give the product (30 mg, yield: 40.5%).
$^1$HNMR (300 MHz, DMSO-$d_6$) δ(ppm): 10.83 (s, 1H), 9.04 (s, 1H), 8.65 (s, 1H), 8.26 (s, 1H), 7.84-7.81 (d, 1H), 7.74 (s, 1H), 3.6 (s ,1H), 2.93-2.92 (d, 1H), 2.70-2.67 (d, 1H), 1.95-1.92 (d, 1H), 1.79 (s, 6H), 1.33-1.24 (t, 4H), 0.79-0.78 (d, 2H), 0.65 (s, 2H).
Molecular formula: $C_{21}H_{24}N_6O_2$ Exact mass: 392.20 LC-MS(Pos, *m*/*z*)=393.17[M+H]$^+$.

**Example 17: Synthesis of (1*S*,3*R*)-3-acetamido-*N*-(7-cyano-5-((1-methylcyclopropyl)amino)-2,6-naphthyridin-3-yl)cyclohexane-1-carboxamide (compound 17)**

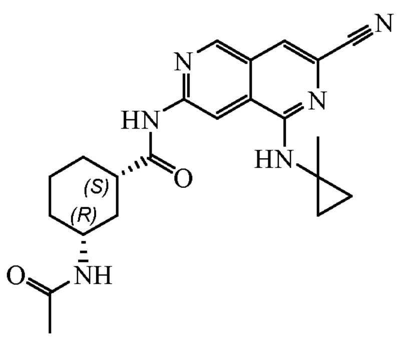

Steps:

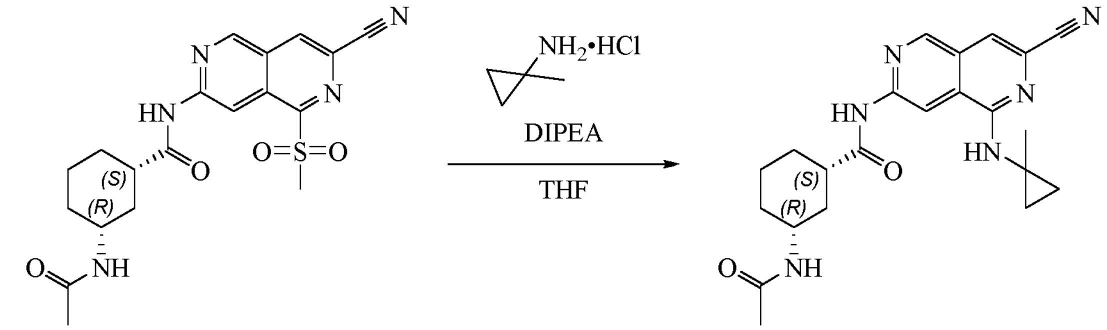


(1*S*,3*R*)-3-acetamido-*N*-(7-cyano-5-(methylsulfonyl)-2,6-naphthyridin-3 - yl)cyclohexane-1-carboxamide (100 mg, 0.24 mmol, 1.0 eq.) was dissolved in THF (3 mL), and the reaction solution was added with DIPEA (248 mg, 1.92 mmol, 8.0 eq.) and 1-methylcyclopropan-1-amine hydrochloride (129 mg, 1. 2 mmol, 5.0 eq.), and reacted at 100 °C for 16 h in a sealed tube. When there were no materials left, as detected by LC-MS, the reaction solution was added with water (20 mL) and extracted with ethyl acetate (20 mL × 3). The organic phases were combined, washed with hydrochloric acid solution (0.1 mol/L, 30 mL), dried over anhydrous sodium sulfate and filtered, and the filtrate was concentrated under reduced pressure to give a crude product, which was purified by preparative thin-layer chromatography (MeOH:DCM = 1:20) to give the product (11 mg, yield: 11.2%).
$^1$HNMR (400 MHz, MeOD) δ(ppm): 8.95 (s, 1H), 8.49 (s, 1H), 7.57 (s, 1H), 3.80-3.74 (m, 1H), 2.69-2.64 (m, 1H), 2.12-2.09 (m, 1H), 1.95 (m, 4H), 1.54 (m, 4H), 1.31 (m, 5H), 0.92-0.88 (m, 2H), 0.80 (m, 2H).
Molecular formula: $C_{22}H_{26}N_6O_2$ Exact mass: 406.21 LC-MS (Pos, *m*/*z*) =407.39 $[M+H]^+$.

**Example 18: Synthesis of (1*S*,3*R*)-3-acetamido-*N*-(7-cyano-5-((1,5-dihydroxypentan-3-yl)amino)-2,6-naphthyridin-3-yl)cyclohexane-1-carboxamide (compound 18)**

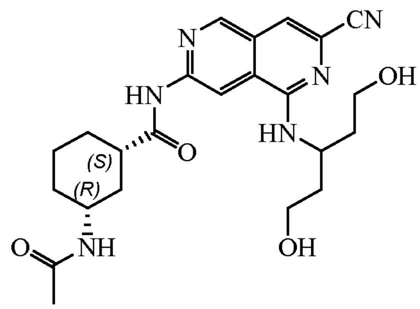

Step 1: synthesis of dimethyl 3-(benzylamino)pentanedioate

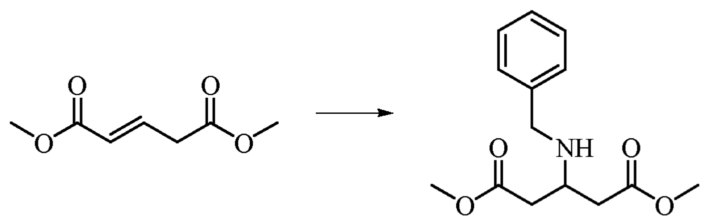

Dimethyl (*E*)-pent-2-enedioate (2.0 g, 12.64 mmol, 1.0 eq.) and benzylamine (1.42 g, 13.27 mmol, 1.05 eq.) were dissolved in methanol (200 mL), and the reaction solution was reacted at 65 °C for 17 h. When a product was generated, as detected by LC-MS, the reaction solution was concentrated under reduced pressure to give a crude product, which was purified by silica gel column chromatography (PE:EA = 2:1) to give the product (2.5 g, yield: 74.6%).

Step 2: synthesis of 3-(benzylamino)pentane-1,5-diol

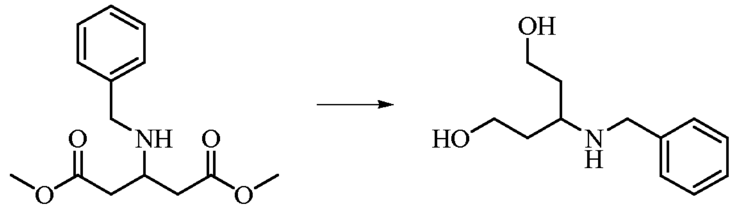

Dimethyl 3-(benzylamino)pentanedioate (2.5 g, 9.42 mmol, 1.0 eq.) was dissolved in THF (50 mL), and the reaction solution was added with lithium aluminum hydride (714 mg, 18.84 mmol, 2.0 eq.) in batches at room temperature, and reacted for 10 min. The reaction solution was added with water (714 mg), 15% aqueous NaOH solution (714 mg) and water (2.142 g) successively, added with a proper amount of anhydrous sodium sulfate, stirred for 30 min and filtered under vacuum, and the filtrate was concentrated under reduced pressure to give the product (1.97 g, yield: 100%).

Step 3: synthesis of 3-aminopentane-1,5-diol

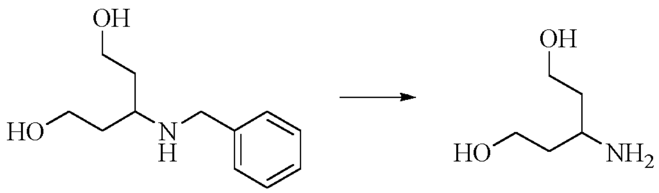

3-(benzylamino)pentane-1,5-diol (1.97 g, 9.42 mmol, 1.0 eq.) and 20% $Pd(OH)_2$ (200 mg) were dissolved in methanol (20 mL), and the reaction solution was reacted for 16 h under hydrogen atmosphere and filtered, and the filtrate was concentrated under reduced pressure to give the product (1.0 g, yield: 89.2%).

Step 4: synthesis of (1*S*,3*R*)-3-acetamido-*N*-(7-cyano-5-((1,5-dihydroxypentan-3-yl)amino)-2,6-naphthyridin-3-yl)cyclohexane-1-carboxamide

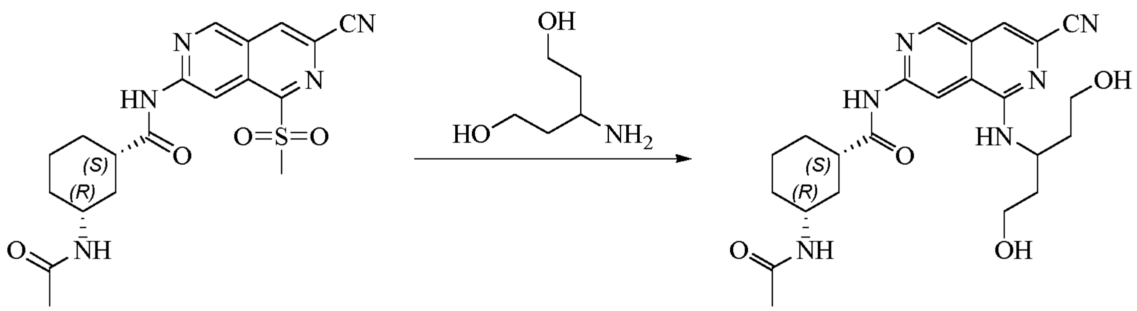

(1*S*,3*R*)-3-acetamido-*N*-(7-cyano-5-(methylsulfonyl)-2,6-naphthyridin-3-yl)cyclohexane-1-carboxamide (80 mg, 0.19 mmol, 1.0 eq.), 3-aminopentane-1,5-diol (226 mg, 1.90 mmol, 10 eq.) and DIPEA (74 mg, 0.57 mmol, 3.0 eq.) were dissolved in THF (2 mL), and the reaction solution was reacted at 30 °C for 17 h. When a product was generated, as detected by LC-MS, the reaction solution was poured into water (5 mL) and extracted with EA (5 mL × 4). The organic phase was dried and concentrated to give a crude product, which was purified by preparative thin-layer chromatography (MeOH:DCM = 1:10) to give the product (20 mg, yield: 23.2%).

$^1$HNMR (300 MHz, DMSO-$d_6$) δ(ppm): 10.84 (s, 1H), 9.02 (s, 1H), 8.68 (s, 1H), 7.86-7.82 (m, 2H), 7.65 (s, 1H), 4.50-4.46 (m, 1H), 3.48-3.46 (d, 5H), 2.74-2.68 (d, 1H), 2.02-1.93 (m, 3H), 1.79 (s, 9 h), 1.34-1.30 (d, 4H).

Molecular formula: $C_{23}H_{30}N_6O_4$ Exact mass: 454.23 LC-MS(Pos, *m*/*z*)=455.32[M+H]+.

**Example 19: Synthesis of (1*S*,3*R*)-3-acetamido-*N*-(7-cyano-5-((*R*)-3-hydroxypyrrolidin-1-yl)-2,6-naphthyridin-3-yl)cyclohexane-1-carboxamide (compound 19)**

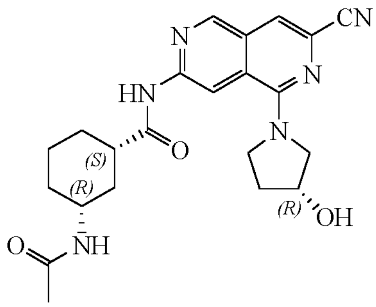

Step 1: synthesis of (1*S*,3*R*)-3-acetamido-*N*-(7-cyano-5-((*R*)-3-hydroxypyrrolidin-1-yl)-2,6-naphthyridin-3-yl)cyclohexane-1-carboxamide

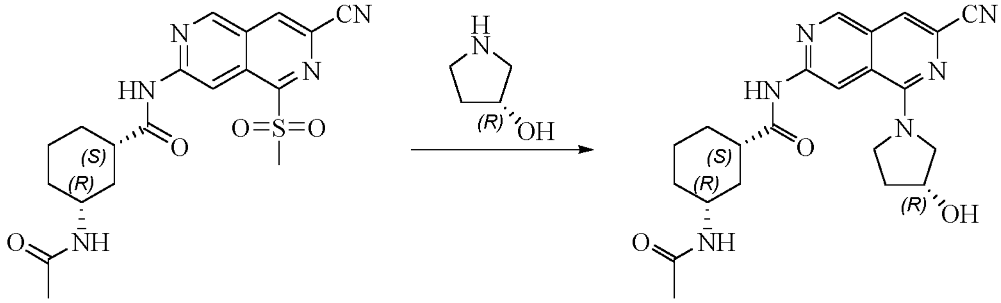

(1*S*,3*R*)-3-acetamido-*N*-(7-cyano-5-(methylsulfonyl)-2,6-naphthyridin-3-yl)cyclohexane-1-carboxamide (80 mg, 0.19 mmol, 1.0 eq.), (R)-pyrrolidin-3-ol (24 mg, 0.28 mmol, 1.5 eq.) and DIPEA(74 mg, 0.57 mmol, 3.0 eq.) were dissolved in THF (3 mL), and the reaction solution was reacted at room temperature for 2 h. When a product was generated and there were no materials left, as detected by TLC, the reaction solution was concentrated under reduced pressure to give a crude product, which was slurried with DCM (3 mL) and filtered under vacuum. The filter cake was dried at 50 °C to give the product (20 mg, yield: 25%).

$^1$HNMR (300 MHz, DMSO-$d_6$) δ(ppm): 10.92 (s, 1H), 9.11-9.05 (d, 2H), 7.81-7.76 (t, 2H), 5.09 (s, 1H), 4.44 (s, 1H), 4.01-3.97 (t ,2H), 3.83-3.78 (t, 1H), 3.36-3.58 (m, 2H), 2.68 (s, 1H), 2.05 (s, 3H), 1.79 (s, 6H), 1.34-1.24 (m, 4H).

Molecular formula: $C_{22}H_{26}N_6O_3$ Exact mass: 422.21 LC-MS(Pos, *m*/*z*)=423.22[M+H]+.

**Example 20: synthesis of (1*S*,3*R*)-3-acetamido-*N*-(5-((*R*)-3-aminopyrrolidin-1-yl)-7-cyano-2,6-naphthyridin-3-yl)cyclohexane-1-carboxamide (compound 20)**

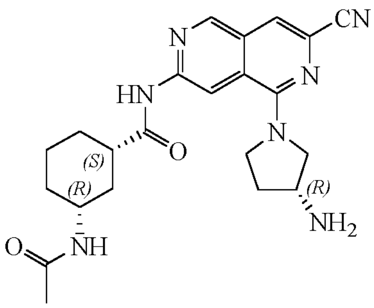

Step 1: synthesis of *tert*-butyl ((*R*)-1-(7-((1*S*,3*R*)-3-acetamidocyclohexane-1-carboxamido)-3-cyano-2,6-naphthyridin-1-yl)pyrrolidin-3-yl)carbamate

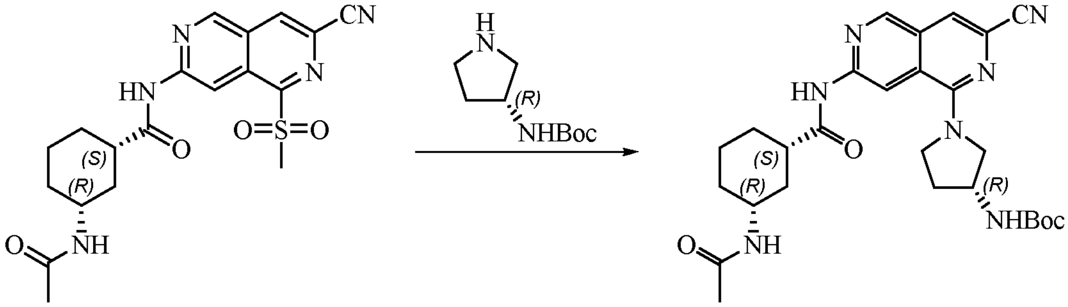

(1*S*,3*R*)-3-acetamido-*N*-(7-cyano-5-(methylsulfonyl)-2,6-naphthyridin-3-yl)cyclohexane-1-carboxamide (180 mg, 0.43 mmol, 1.0 eq.), *tert*-butyl (*R*)-pyrrolidin-3-ylcarbamate (119 mg, 0.64 mmol, 1.5 eq.) and DIPEA(167 mg, 1.29 mmol, 3.0 eq.) were dissolved in THF (5 mL), and the reaction solution was reacted at 40 °C for 2 h. After the reaction was completed, as detected by LC-MS, the reaction solution was concentrated under reduced pressure to give a crude product, which was slurried with EA (5 mL) and filtered under vacuum. The filter cake was dried to give the product (100 mg, yield: 44.6%).

Step 2: synthesis of (1*S*,3*R*)-3-acetamido-*N*-(5-((*R*)-3-aminopyrrolidin-1-yl)-7-cyano-2,6-naphthyridin-3-yl)cyclohexane-1-carboxamide

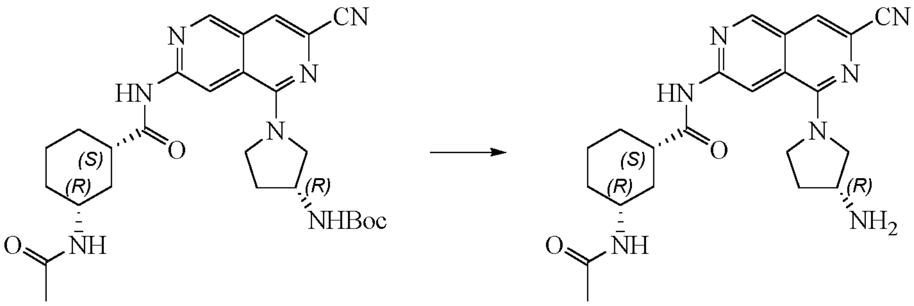

*Tert*-butyl ((*R*)-1-(7-((1*S*,3*R*)-3-acetamidocyclohexane-1-carboxamido)-3-cyano-2,6-naphthyridin-1-yl)pyrrolidin-3-yl)carbamate (100 mg, 0.19 mmol, 1.0 eq.) was dissolved in EA (2 mL). The reaction solution was added dropwise with a solution of hydrogen chloride in 1,4-dioxane (4 mol/L, 1 mL), reacted at room temperature for 17 h, and filtered under vacuum. The filter cake was dissolved in water (3 mL), and added with saturated aqueous sodium bicarbonate solution with stirring to adjust pH=9. A white solid was precipitated. Filtration under vacuum was performed, and the filter cake was dried at 60 °C to give the product (20 mg, yield: 25%).
$^1$HNMR (300 MHz, DMSO-$d_6$) δ(ppm): 10.90 (s, 1H), 9.11-9.04 (d, 2H), 7.81-7.74 (t, 2H), 3.95 (s, 2H), 3.60-3.54 (d, 2H), 2.74-2.67 (d, 2H), 2.04-1.92 (m, 4H), 1.79 (s, 6H), 1.33-1.25 (t, 6H).

Molecular formula: $C_{22}H_{27}N_7O_2$ Exact mass: 421.22 LC-MS(Pos, *m*/*z*)=422.20[M+H]$^+$.

**Example 21: Synthesis of (1*S*,3*R*)-3-acetamido-*N*-(7-cyano-5-morpholino-2,6-naphthyridin-3-yl)cyclohexane-1-carboxamide (compound 21)**

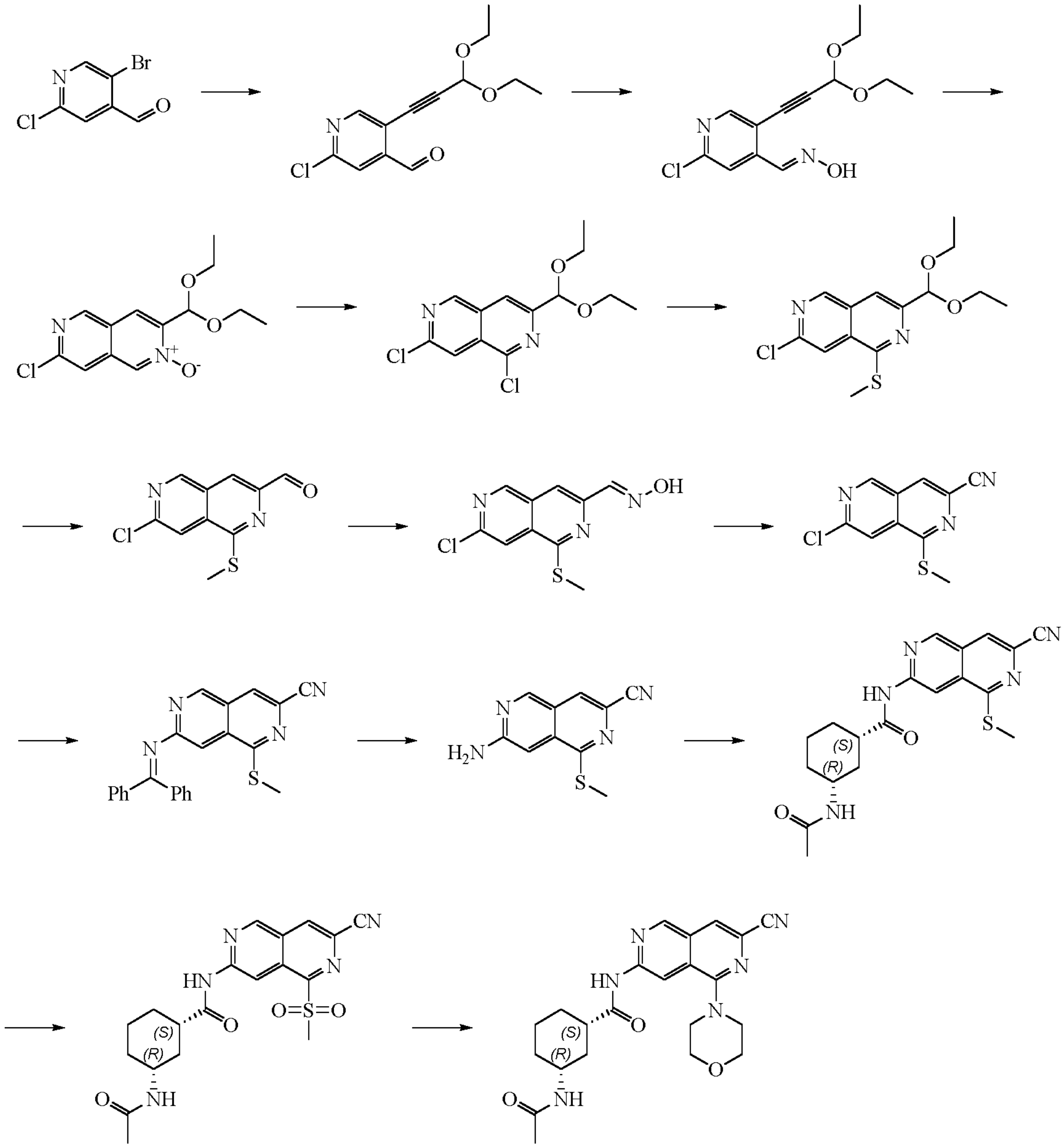

Step 1: synthesis of 2-chloro-5-(3,3-diethoxyprop-1-yn-1-yl)isonicotinaldehyde

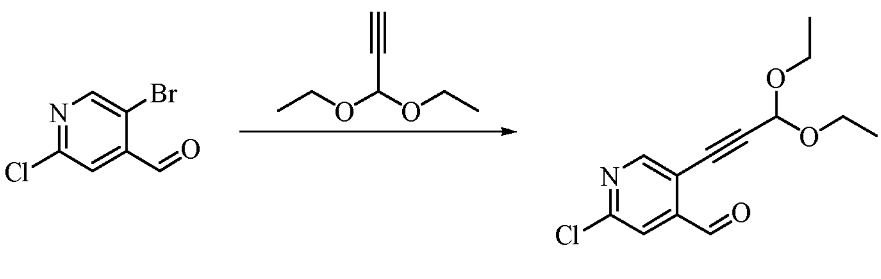

5-bromo-2-chloroisonicotinaldehyde (30 g, 136.08 mmol, 1.0 eq.), cuprous iodide (2.59 g, 13.6 mmol, 0.1 eq.), bis(triphenylphosphine)palladium(II) chloride (7.64 g, 10.89 mmol, 0.08 eq.) and DIPEA (52.76 g, 408.24 mmol, 3.0 eq.) were dispersed in THF (300 mL), and the reaction solution was cooled to 0 °C to 5 °C, reacted for 30 min, added dropwise with 3,3-diethoxyprop-1-yn (20.93 g, 163.30 mmol, 1.2 eq.), and reacted at 0 °C to 5 °C for 20 h. After the reaction was completed, as detected by TLC, the reaction solution was poured into water (300 mL) and extracted with EA (300 mL × 2). The organic phase was dried and concentrated to give a crude product, which was purified by silica gel column chromatography (PE:EA = 20:1) to give the product (23 g, yield: 63.1%).

Step 2: synthesis of (*E*)-2-chloro-5-(3,3-diethoxyprop-1-yn-1-yl)isonicotinaldehyde oxime

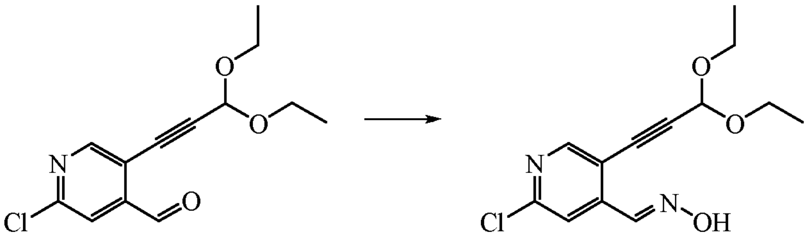

2-chloro-5-(3,3-diethoxyprop-1-yn-1-yl)isonicotinaldehyde (23 g, 85.91 mmol, 1.0 eq.), sodium acetate (10.6 g, 128.86 mmol, 1.5 eq.) and hydroxylamine hydrochloride (8.95 g, 128.86 mmol, 1.5 eq.) were dispersed in ethanol (230 mL), and the reaction solution was reacted at room temperature for 1 h. After the reaction was completed, as detected by TLC, the reaction solution was concentrated under reduced pressure to give a crude product. The crude product was added into water (100 mL) and extracted with (100 mL × 2). The organic phase was dried and concentrated to give the product (24.29 g, yield: 100%).

Step 3: synthesis of 7-chloro-3-(diethoxymethyl)-2,6-naphthyridine 2-oxide

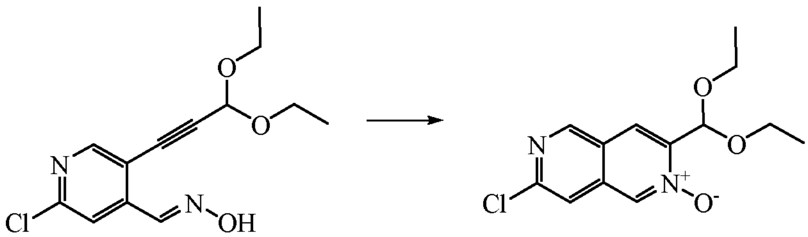

(*E*)-2-chloro-5-(3,3-diethoxyprop-1-yn-1-yl)isonicotinaldehyde oxime (24.29 g, 85.91 mmol, 1.0 eq.) was dissolved in DCM (250 mL), and the reaction solution was added with silver trifluoromethanesulfonate (2.21 g, 8.6 mmol, 0.1 eq.) and reacted at room temperature for 16 h. After the reaction was completed, as detected by TLC, the reaction solution was filtered, and the filtrate was directly used in the next step.

Step 4: synthesis of 1,7-dichloro-3-(diethoxymethyl)-2,6-naphthyridine

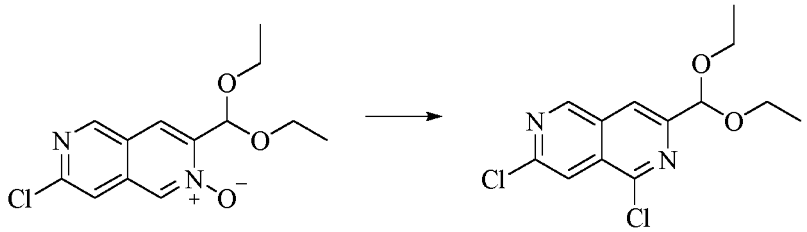

DIPEA (33.31 g, 257.73 mmol, 3.0 eq.) was added into 1,7-dichloro-3-(diethoxymethyl)-2,6-naphthyridine (85.91 mmol, 1.0 eq.) solution obtained in the previous step, and the reaction solution was added dropwise with oxalyl chloride (16.1 g, 126.86 mmol, 1.5 eq.) at room temperature and reacted for 20 min. After the reaction was completed, as detected by TLC, the reaction solution was concentrated under reduced pressure to give a crude product, which was purified by silica gel column chromatography (PE:EA= 10:1) to give the product (12.4 g, yield: 47.9%).

Step 5: synthesis of 7-chloro-3-(diethoxymethyl)-1-(methylthio)-2,6-naphthyridine

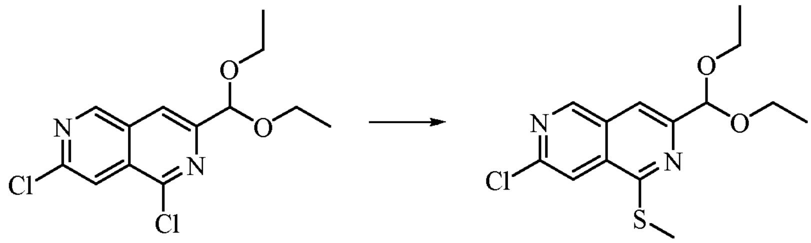

1,7-dichloro-3-(diethoxymethyl)-2,6-naphthyridine (12 g, 39.84 mmol, 1.0 eq.) was dissolved in THF (150 mL), and the reaction solution was added with aqueous sodium thiomethoxide solution (mass fraction: 20%, 15.36 g, 43.82 mmol, 1.0 eq.) and reacted for 16 h. After the reaction was completed, as detected by TLC, the reaction solution was directly used in the next step without any treatment.

Step 6: synthesis of 7-chloro-1-(methylthio)-2,6-naphthyridine-3-carbaldehyde

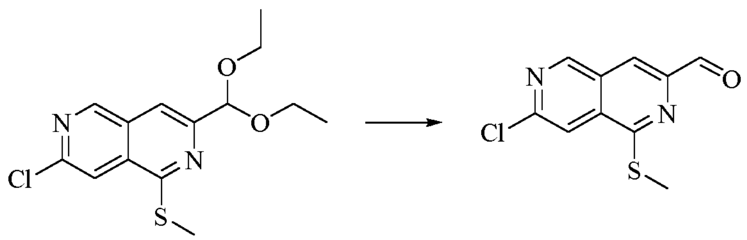

Water (20 mL) and TFA (20 mL) were added into 7-chloro-1-(methylthio)-2,6-naphthyridine-3-carbaldehyde (12.46 g, 39.84 mmol, 1.0 eq.) obtained from the previous step at room temperature, and the mixture was reacted at room temperature for 3 h. After the reaction was completed, as detected by TLC, the reaction solution was concentrated under reduced pressure to the residual 80 mL, which was filtered, and the filter cake was dried to give the product (10 g, yield: 100 %).

Step 7: synthesis of (*E*)-7-chloro-1-(methylthio)-2,6-naphthyridine-3-carbaldehyde oxime

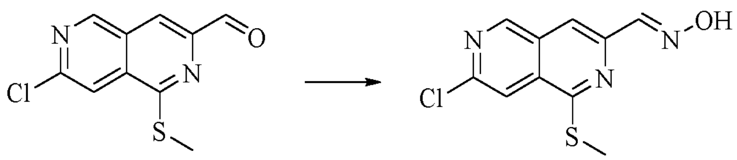

7-chloro-1-(methylthio)-2,6-naphthyridine-3-carbaldehyde (10 g, 41.89 mmol, 1.0 eq.), sodium acetate (5.2 g, 64.34 mmol, 1.5 eq.) and hydroxylamine hydrochloride (4.47 g, 64.34 mmol, 1.5 eq.) were dispersed in ethanol (200 mL), and the reaction solution was reacted at room temperature for 1 h. After the reaction was completed, as detected by TLC, the reaction solution was concentrated under reduced pressure to give a crude product, slurried with water (100 mL) and filtered under vacuum, and the filter cake was dried to give the product (10.62 g, yield: 100%).

Step 8: synthesis of 7-chloro-1-(methylthio)-2,6-naphthyridine-3-carbonitrile

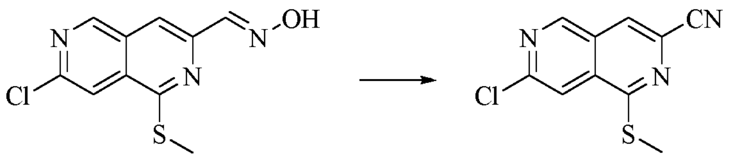

(*E*)-7-chloro-1-(methylthio)-2,6-naphthyridine-3-carbaldehyde oxime (10.62 g, 41.89 mmol, 1.0 eq.) was dissolved in acetic anhydride (200 mL), and the reaction solution was reacted at 120 °C for 143 h. After the reaction was completed, as detected by LC-MS, the reaction solution was concentrated under reduced pressure to give a crude product, which was slurried with (PE:MTBE = 1:1, 150 mL) and filtered under vacuum, and the filter cake was dried to give the product (6.1 g, yield: 61.8%).

Step 9: synthesis of 7-((diphenylmethylene)amino)-1-(methylthio)-2,6-naphthyridine-3-carbonitrile

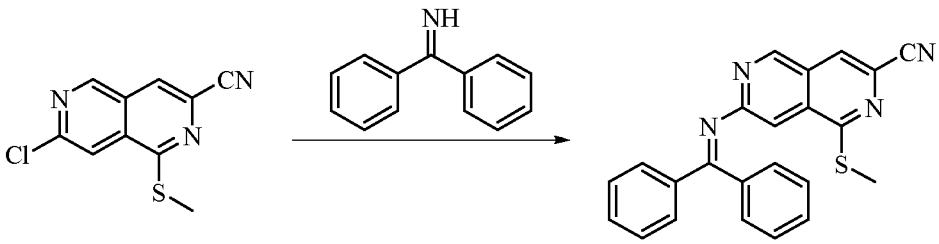

7-chloro-1-(methylthio)-2,6-naphthyridine-3-carbonitrile (3 g, 12.73 mmol, 1.0 eq.), diphenylmethanimine (2.77 g, 15.27 mmol, 1.2 eq.), $Pd_2(dba)_3$ (1.16 g, 1.27 mmol, 0.1 eq.), BINAP (1.58 g, 2.54 mmol, 0.2 eq.) and cesium carbonate (8.30 g, 25.46 mmol, 2.0 eq.) were dispersed in 1,4-dioxane (60 mL). The mixture was reacted at 100 °C for 19 h in nitrogen atmosphere. After the reaction was completed, as detected by TLC, the reaction solution was concentrated under reduced pressure to give a crude product, which was purified by silica gel column chromatography (PE:EA = 10:1) to give the product (3.2 g, yield: 66.1%).

Step 10: synthesis of 7-amino-1-(methylthio)-2,6-naphthyridine-3-carbonitrile

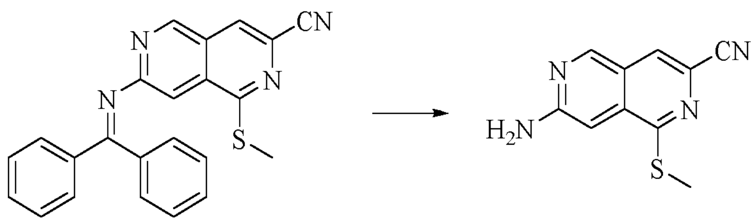

7-((diphenylmethylene)amino)-1-(methylthio)-2,6-naphthyridine-3-carbonitrile (3.2 g, 8.41 mmol, 1.0 eq.) and citric acid (4.85 g, 25.23 mmol, 3.0 eq.) were dissolved in a mixed solution of THF (32 mL) and $H_2O$ (16 mL), and the reaction solution was reacted at room temperature for 40 min. After the reaction was completed, as detected by TLC-MS, the reaction solution was concentrated under reduced pressure to give a crude product, which was slurried with water (30 mL) and filtered under vacuum, the filter cake was slurried with (PE:EA = 5:1, 60 mL) and filtered under vacuum, and the obtained filter cake was dried to give the product (1.7 g, yield: 93.4%).

Step 11: synthesis of (1*S*,3*R*)-3-acetamido-*N*-(7-cyano-5-(methylthio)-2,6-naphthyridin-3-yl)cyclohexane-1-carboxamide

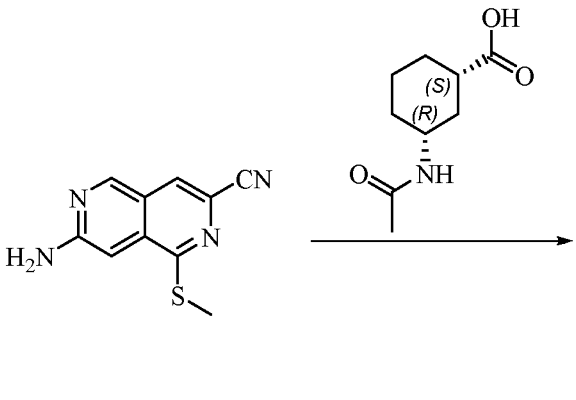

7-amino-1-(methylthio)-2,6-naphthyridine-3-carbonitrile (1.7 g, 7.86 mmol, 1.0 eq.) and (1*S*,3*R*)-3-acetamidocyclohexane-1-carboxylic acid (2.18 g, 11.79 mmol, 1.5 eq.) were dissolved in pyridine (20 mL), and the reaction solution was added with phosphorus oxychloride (3.62 g, 23.58 mmol, 3.0 eq.) and reacted at room temperature for 10 min. After the reaction was completed, as detected by TLC, the reaction solution was poured into ice water (50 mL) and extracted with EA (30 mL × 3). The organic phase was concentrated under reduced pressure to give a crude product, which was purified by silica gel column chromatography (MeOH:DCM = 1:50) to give the product (1.4 g, yield: 46.5%).

Step 12: synthesis of (1*S*,3*R*)-3-acetamido-*N*-(7-cyano-5-(methylsulfonyl)-2,6-naphthyridin-3-yl)cyclohexane-1-carboxamide

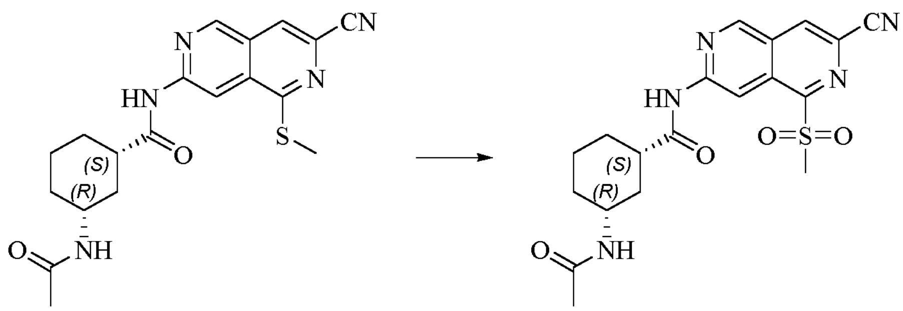

(1*S*,3*R*)-3-acetamido-*N*-(7-cyano-5-(methylthio)-2,6-naphthyridin-3-yl)cyclohexane-1-carboxamide (1.4 g, 0.40 mmol, 1.0 eq.) was dissolved in DCM (30 mL), and the reaction solution was added with *m*-chloroperoxybenzoic acid (mass fraction: 80%, 1.65 g, 7.66 mmol, 2.1 eq.) and reacted at room temperature for 0.5 h. After the reaction was completed, as detected by TLC, the reaction solution was poured into water (20 mL), added with a proper amount of potassium carbonate, and extracted with DCM (20 mL × 3). The organic phase was dried and concentrated to give a crude product, which was slurried with MTBE (20 mL) and filtered under vacuum, and the filter cake was dried to give the product (1.36 g, yield: 90%).

Step 13: synthesis of (1*S*,3*R*)-3-acetamido-*N*-(7-cyano-5-morpholino-2,6-naphthyridin-3-yl)cyclohexane-1-carboxamide

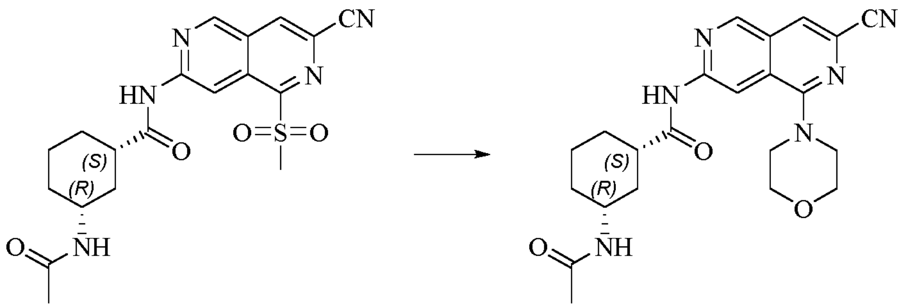

(1*S*,3*R*)-3-acetamido-*N*-(7-cyano-5-(methylsulfonyl)-2,6-naphthyridin-3-yl)cyclohexane-1-carboxamide (80 mg, 0.19 mmol, 1.0 eq.) and morpholine (50 mg, 0.57 mmol, 3.0 eq.) were dissolved in THF (2 mL), and the reaction solution was reacted at 40 °C for 19 h. After the reaction was completed, as detected by LC-MS, the reaction solution was poured into water (5 mL) and extracted with EA (5 mL × 3). The organic phase was dried and concentrated to give a crude product, which was purified by preparative thin-layer chromatography (MeOH:DCM = 1:15) to give the product (25 mg, yield: 31.2%).

$^1$HNMR (300 MHz, DMSO-$d_6$) δ(ppm): 11.03 (s, 1H), 9.22 (s, 1H), 8.72 (s, 1H), 8.18 (s, 1H), 7.82-7.79 (d, 1H), 3.85 (s, 4H), 3.59-3.58 (d, 1H), 3.46 (s, 4H), 2.73-2.69 (d, 1H), 1.95-1.92 (d, 1H), 1.79 (s, 6H), 1.40-1.25 (m, 4H).

Molecular formula: $C_{22}H_{26}N_6O_3$ Exact mass: 422.21 LC-MS(Pos, *m*/*z*)=423.21 [M+H]$^+$.

**Example 22: Synthesis of (1*S*,3*R*)-3-acetamido-*N*-(7-cyano-5-((*R*)-3-hydroxy-3-methylpyrrolidin-1-yl)-2,6-naphthyridin-3-yl)cyclohexane-1-carboxamide (compound 22)**

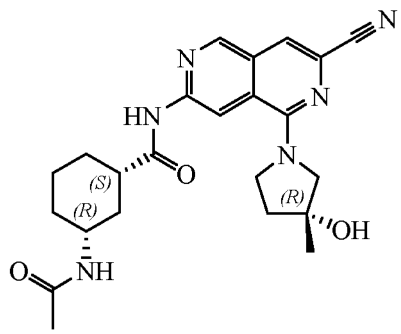

Steps:

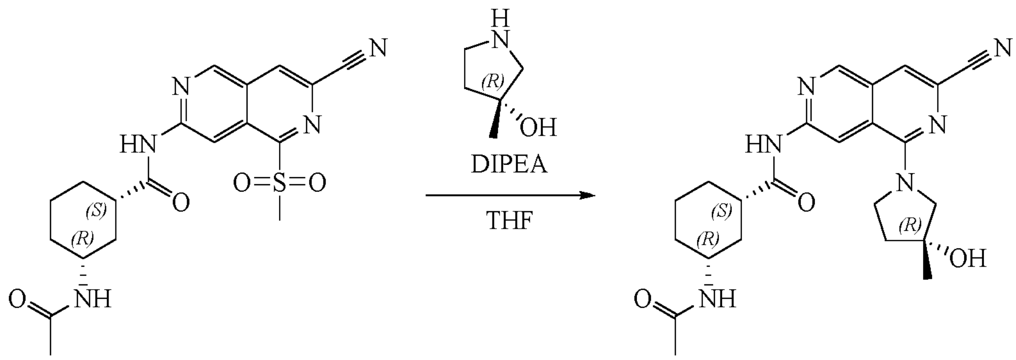


(1*S*,3*R*)-3-acetamido-*N*-(7-cyano-5-(methylsulfonyl)-2,6-naphthyridin-3-yl)cyclohexane-1-carboxamide (200 mg, 0.48 mmol, 1.0 eq.) was dissolved in THF (3 mL), and the reaction solution was added with DIPEA (248 mg, 1.92 mmol, 4.0 eq.) and (*R*)-3-methylpyrrolidin-3-ol (97.3 mg, 0.48 mmol, 1.0 eq.), and reacted at room temperature for 16

h. When there were no materials left, as detected by LC-MS, the reaction solution was cooled at room temperature, added with water (20 mL) and extracted with ethyl acetate (20 mL × 3). The organic phases were combined, washed with hydrochloric acid solution (0.1 mol/L, 30 mL), dried over anhydrous sodium sulfate and filtered, and the filtrate was concentrated under reduced pressure to give a crude material, which was purified by preparative thin-layer chromatography (MeOH:DCM = 1:20) to give a crude product (150 mg). The crude product was dissolved with water (10 mL) and extracted with ethyl acetate (10 mL), the organic phase was dried over anhydrous magnesium sulfate and filtered, and the filtrate was concentrated under reduced pressure, slurried with a mixed solution (5 mL) of ethyl acetate methyl *tert*-butyl ether = 1:10 and filtered, and the filter cake was dried to give the product (42 mg, yield: 20%).

$^1$HNMR (400 MHz, DMSO) δ(ppm): 10.92 (s, 1H), 9.08-9.05 (s, 2H), 7.83-7.80 (s, 1H), 7.75 (s, 1H), 4.92 (s, 1H), 4.11-4.02 (m, 1H), 3.84-3.74 (m, 2H), 3.67-3.58 (m, 2H), 2.68 (m, 1H), 1.96-1.93 (m, 3H), 1.79 (s, 6H), 1.39 (m, 3H), 1.36-1.31 (m, 3H), 1.11 (m, 1H).

Molecular formula: $C_{23}H_{28}N_6O_3$ Exact mass: 436.22 LC-MS (Pos, *m*/*z*) =436.52[M+H]$^+$.

**Example 23: Synthesis of (1*S*,3*R*)-3-acetamido-*N*-(7-cyano-5-(4,4-difluoropiperidin-1-yl)-2,6-naphthyridin-3-yl)cyclohexane-1-carboxamide (compound 23)**

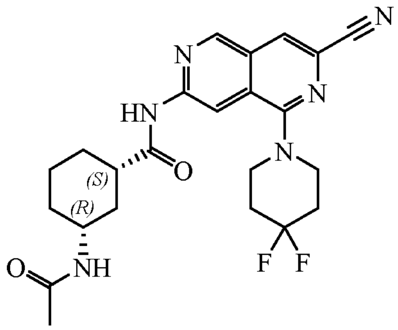

Steps:

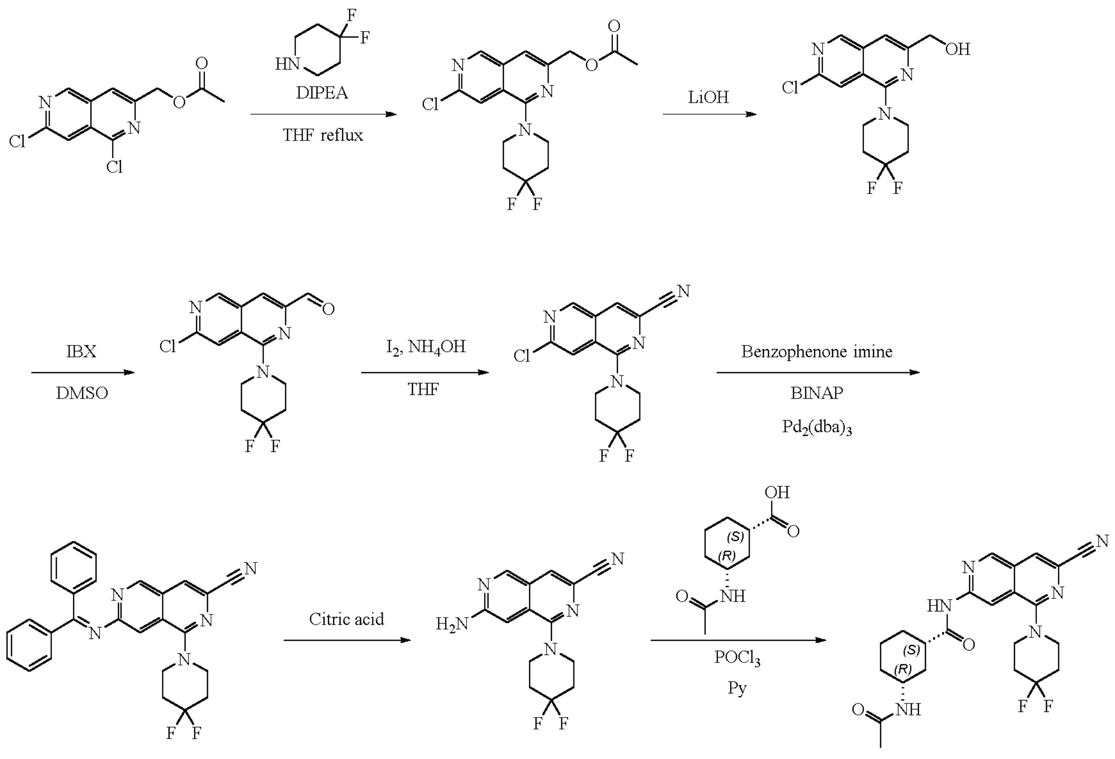

Step 1: synthesis of (7-chloro-1-(4,4-difluoropiperidin-1-yl)-2,6-naphthyridin-3-yl)methyl acetate

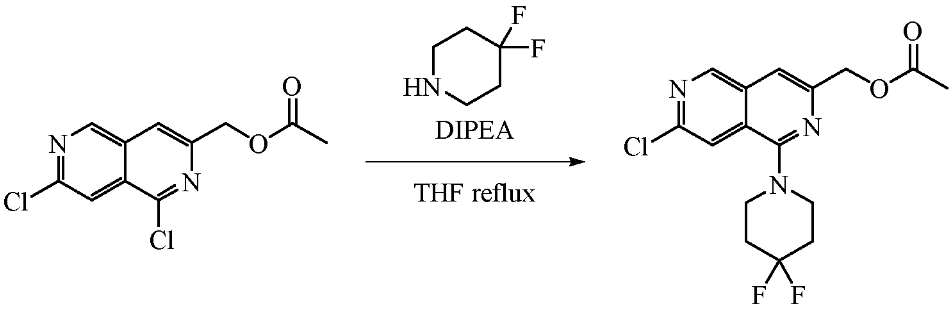


(1,7-dichloro-2,6-naphthyridin-3-yl)methyl acetate (2.0 g, 7.38 mmol, 1.0 eq.), 4,4-difluoropiperidine (2.68 g, 22.14 mmol, 3.0 eq.) and DIPEA (2.86 g, 22.14 mmol, 3.0 eq.) were dissolved in anhydrous THF (20 mL), and the reaction solution was reacted at 100 °C for 4 h in a sealed tube. When there were no materials left, as detected by TLC, the reaction solution was concentrated under reduced pressure, added with water (30 mL) and extracted with ethyl acetate (30 mL × 2). The organic phases were combined, washed with hydrochloric acid solution (0.5 mol/L, 50 mL), dried over anhydrous sodium sulfate and filtered, and the filtrate was concentrated under reduced pressure to give the product, which was used in the next step according to a theoretical amount.

Step 2: synthesis of (7-chloro-1-(4,4-difluoropiperidin-1-yl)-2,6-naphthyridin-3-yl)methanol

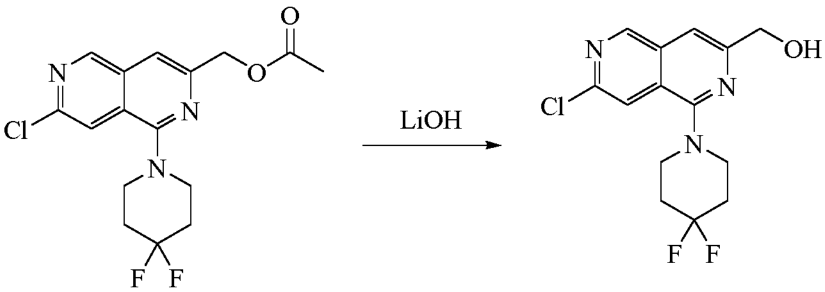


(7-chloro-1-(4,4-difluoropiperidin-1-yl)-2,6-naphthyridin-3-yl)methyl acetate (7.38 mmol, 1.0 eq.) and lithium hydroxide monohydrate (928.7 mg, 22.14 mmol, 3.0 eq.) were dissolved in a mixed solution of THF (20 mL), methanol (10 mL) and water (10 mL), and the reaction solution was reacted at room temperature for 16 h. When there were no materials left, as detected by TLC, the reaction solution was concentrated under reduced pressure, added with water (20 mL) and extracted with ethyl acetate (20 mL × 3). The organic phases were combined, dried over anhydrous sodium sulfate and filtered, and the filtrate was concentrated under reduced pressure to give the product (2.2 g, yield: 95.6%).

Step 3: synthesis of 7-chloro-1-(4,4-difluoropiperidin-1-yl)-2,6-naphthyridine-3-carbaldehyde

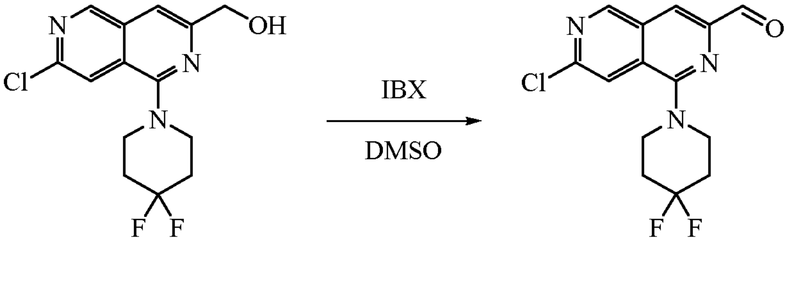

(7-chloro-1-(4,4-difluoropiperidin-1-yl)-2,6-naphthyridin-3-yl)methanol (2.2 g, 7.0 mmol, 1.0 eq.) was dissolved in DMSO (30 mL), and the reaction solution was added with IBX (2.9 g, 10.5 mmol, 1.5 eq.) and reacted at room temperature for 2 h. When there were no materials left, as detected by TLC, the reaction solution was poured into water (200 mL) and filtered, and the filter cake was retained, slurried with 2-methyltetrahydrofuran (50 mL × 2) and filtered. The organic phases were combined, and the filtrate was extracted with ethyl acetate (50 mL × 2), combined with the organic phase described above, washed with saturated aqueous sodium carbonate solution (100 mL), dried over anhydrous sodium sulfate and filtered, and the filtrate was concentrated under reduced pressure to give the product (2.1 g, yield: 96.3%).

Step 4: synthesis of 7-chloro-1-(4,4-difluoropiperidin-1-yl)-2,6-naphthyridine-3-carbonitrile

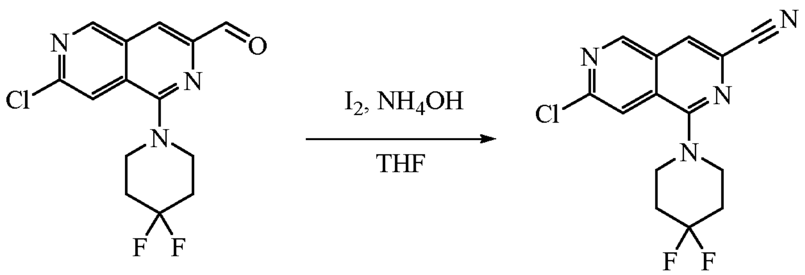


7-chloro-1-(4,4-difluoropiperidin-1-yl)-2,6-naphthyridine-3-carbaldehyde (1 g, 3.2 mmol, 1.0 eq.) was dissolved in THF (30 mL). The reaction solution was added with aqueous ammonia (mass fraction: 28%, 30 mL), added with iodine (974.6 mg, 3.84 mmol, 1.2 eq.) and reacted at room temperature for 18 h. When there were no materials left, as detected by TLC, the reaction solution was added with water (30 mL) and extracted with ethyl acetate (50 mL × 2). The organic phases were combined, washed with aqueous sodium thiosulfate solution (mass fraction: 10%, 50 mL), dried over anhydrous sodium sulfate and filtered, and the filtrate was concentrated under reduced pressure to give a crude product, which was purified by silica gel column chromatography (EA:PE = 1:10-1:5) to give the product (970 mg, yield: 98.2%).

Step 5: synthesis of 1-(4,4-difluoropiperidin-1-yl)-7-((diphenylmethylene)amino)-2,6-naphthyridine-3-carbonitrile

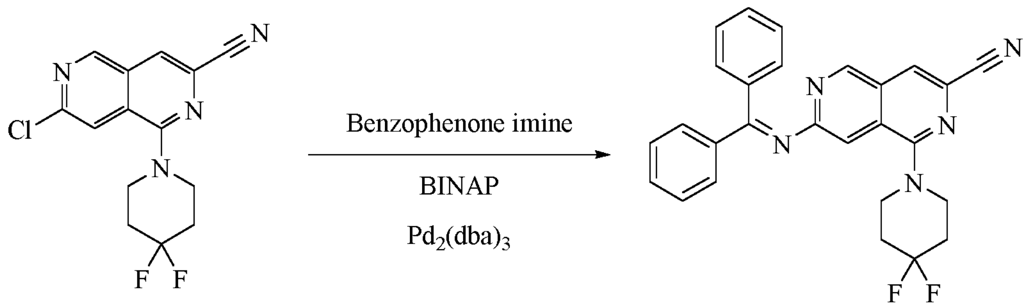


7-chloro-1-(4,4-difluoropiperidin-1-yl)-2,6-naphthyridine-3-carbonitrile (600 mg, 1.94 mmol, 1.0 eq.), diphenylmethanimine (422.3 mg, 2.33 mmol, 1.2 eq.), $Pd_2(dba)_3$ (177.6 mg, 0.19 mmol, 0.1 eq.), BINAP (236.6 mg, 0.38 mmol, 0.2 eq.) and cesium carbonate (1.58 g, 4.85 mmol, 2.5 eq.) were added into 1,4-dioxane (30 mL). The mixture was reacted at 120 °C for 18 h in a sealed tube in nitrogen atmosphere. When there were no materials left, as detected by TLC, the reaction solution was added with water (50 mL) and extracted with ethyl acetate (50 mL × 2). The organic phases were combined, dried over anhydrous sodium sulfate and filtered, and the filtrate was concentrated under reduced pressure to give a crude product, which was purified by silica gel column chromatography (EA:PE = 1:15-1:10) to give the product (546 mg, yield: 62%).

Step 6: synthesis of 7-amino-1-(4,4-difluoropiperidin-1-yl)-2,6-naphthyridine-3-carbonitrile

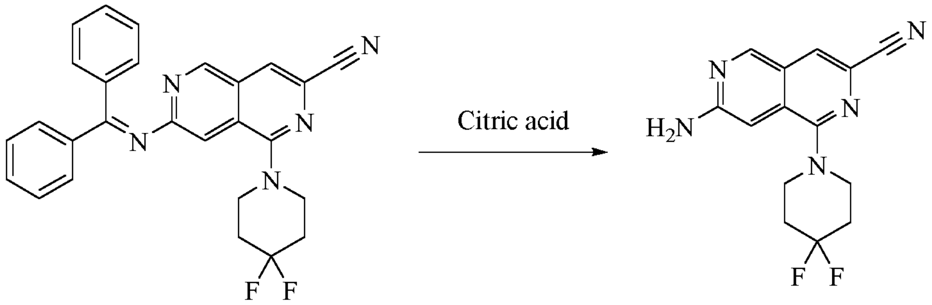


1-(4,4-difluoropiperidin-1-yl)-7-((diphenylmethylene)amino)-2,6-naphthyridine-3-carbonitrile (546 mg, 1.20 mmol, 1.0 eq.) was dissolved in THF (10 mL). The reaction solution was added with aqueous citric acid solution (mass fraction: 10%, 10 mL) and reacted at room temperature for 1 h. When there were no materials left, as detected by TLC, the reaction solution was added with water (30 mL), adjusted to pH of about 8 with sodium carbonate and extracted with ethyl acetate (30 mL × 3). The organic phases were combined, dried over anhydrous sodium sulfate and filtered, and the filtrate was concentrated under reduced pressure to give a crude product, which was purified by silica gel column chromatography (EA:PE = 1:5-EA = 100%) to give the product (268 mg, yield: 77.2%).

Step 7: synthesis of (1S,3R)-3-acetamido-N (7-cyano-5-(4,4-difluoropiperidin-1-yl)-2,6-naphthyridin-3-yl)cyclohexane-1-carboxamide

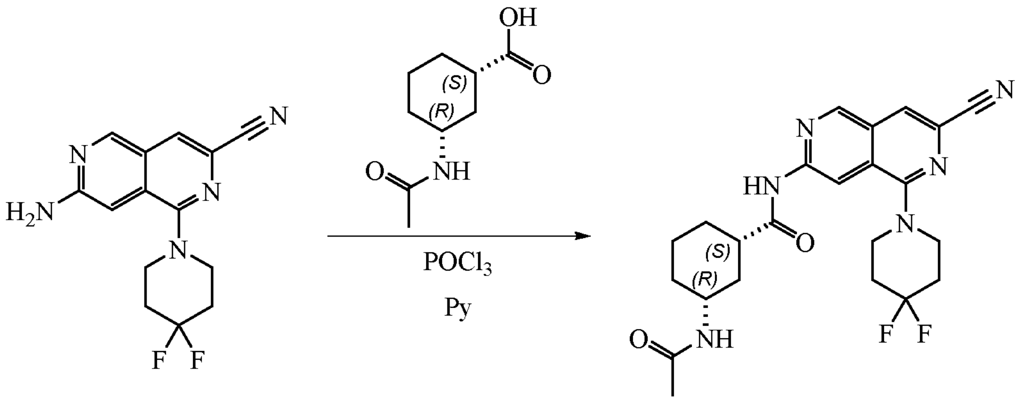


7-amino-1-(4,4-difluoropiperidin-1-yl)-2,6-naphthyridine-3-carbonitrile (268 mg, 0.93 mmol, 1.0 eq.) and (1*S*,3*R*)-3-acetamidocyclohexane-1-carboxylic acid (254.4 mg, 1.39 mmol, 1.5 eq.) were dissolved in pyridine (3 mL), and the reaction solution was added dropwise with phosphorus oxychloride (213.1 mg, 1.39 mmol, 1.5 eq.) and reacted at room temperature for 1 h. When there were no materials left, as detected by TLC, the reaction solution was added with water (30 mL) and extracted with ethyl acetate (30 mL × 3). The organic phases were combined, washed with hydrochloric acid solution (0.2 mol/L, 50 mL × 2) and aqueous sodium carbonate solution (50 mL) successively, dried over anhydrous sodium sulfate and filtered, and the filtrate was concentrated under reduced pressure to give a crude product, which was purified by preparative thin-layer chromatography (MeOH:DCM = 1:20) to give the product (175 mg, yield: 41.2%).
$^1$HNMR (300 MHz, $CD_3OD$) δ(ppm): 9.12 (s, 1H), 8.78 (s, 1H), 7.96 (s, 1H), 3.81-3.76 (m, 1H), 3.69-3.67 (m, 4H), 2.71-2.65 (m, 1H), 2.32-2.25 (m, 4H), 2.13-2.10 (m, 1H), 1.95 (m, 5H), 1.53-1.44 (m, 3H), 1.36-1.33 (m, 2H).
Molecular formula: $C_{23}H_{26}F_2N_6O_2$ Exact mass: 456.21 LC-MS (Pos, *m/z)* =457.50 $[M+H]^+$.

**Example 24: Synthesis of (*S*)-1-acetyl-*N*-(7-cyano-5-(isopropylamino)-2,6-naphthyridin-3-yl)piperidine-3-carboxamide (compound 24)**

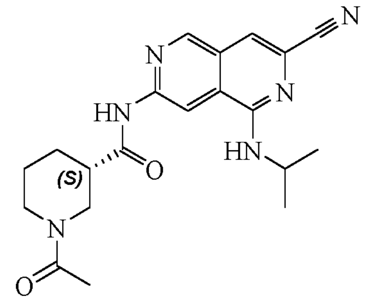

Step 1: synthesis of *tert*-butyl (*S*)-3-((7-cyano-5-(isopropylamino)-2,6-naphthyridin-3-yl)carbamoyl)piperidine-1-carboxylate

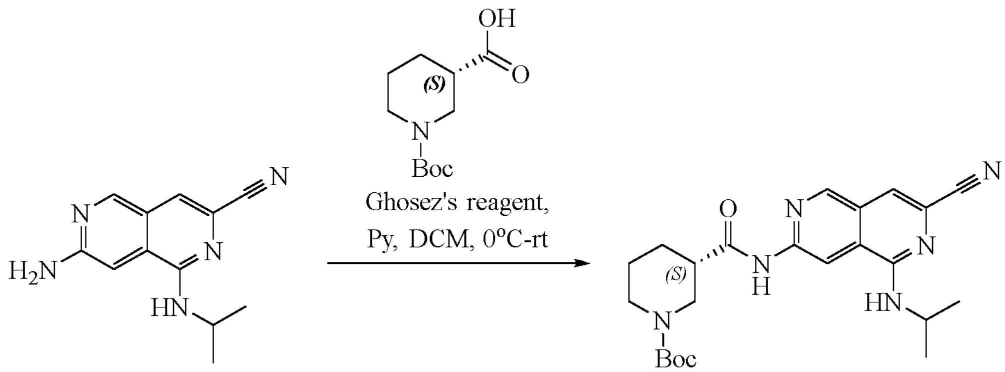


(*S*)-1-(*tert*-butoxycarbonyl)piperidine-3-carboxylic acid (110 mg, 0.48 mmol, 1.1 eq.) was dissolved in DCM (3 mL). The reaction solution was cooled to 0 °C to 5 °C in an ice water bath, added with 1-chloro-*N,N*,2-trimethylprop-1-en-1-amine (mass fraction: 95%, 92.5 mg, 0.66 mmol, 1.5 eq.), reacted at 0 °C to 5 °C for 1.5 h, added with a solution of 7-amino-1-(isopropylamino)-2,6-naphthyridine-3-carbonitrile (100 mg, 0.44 mmol, 1.0 eq.) and pyridine (104.3 mg, 1.32 mmol, 3.0 eq.) in THF (3 mL), and incubated at room temperature for 22 h. When there were no materials left, as detected by TLC, the reaction solution was added with water (20 mL) and extracted with ethyl acetate (20 mL × 2). The organic phases were combined, washed with water (20 mL) and concentrated under reduced pressure to give a crude product, which was purified by silica gel column chromatography (EA:PE = 1:3) to give the product (120 mg, yield: 62.2%).

Step 2: synthesis of (*S*)-*N*-(7-cyano-5-(isopropylamino)-2,6-naphthyridin-3-yl)piperidine-3-carboxamide

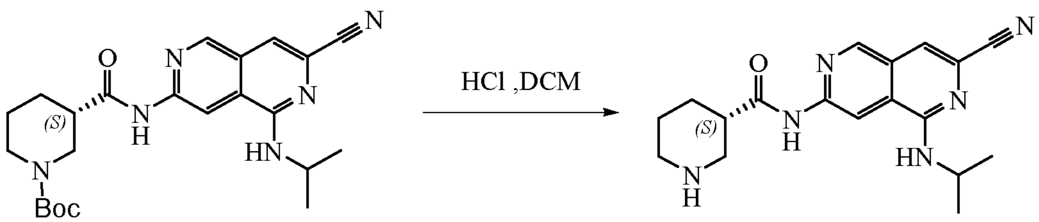


*Tert*-butyl (*S*)-3-((7-cyano-5-(isopropylamino)-2,6-naphthyridin-3-yl)carbamoyl)piperidine-1-carboxylate (120 mg, 0.27 mmol, 1.0 eq.) was dissolved in DCM (4 mL). The reaction solution was added dropwise with a solution of hydrogen chloride (4 mol/L) in dioxane (2 mL) and reacted at room temperature for 21 h. When there were no materials left, as detected by TLC, the reaction solution was concentrated under reduced pressure, added with water (20 mL) and back-extracted with ethyl acetate (20 mL × 2). The aqueous phase was retained, adjusted to pH of about 8 with sodium bicarbonate and extracted with ethyl acetate (20 mL × 2). The organic phases were combined, dried over anhydrous sodium sulfate and filtered, and the filtrate was concentrated under reduced pressure to give the product (68 mg, yield: 74.4%).

Step 3: synthesis of (*S*)-1-acetyl-*N*-(7-cyano-5-(isopropylamino)-2,6-naphthyridin-3-yl)piperidine-3-carboxamide

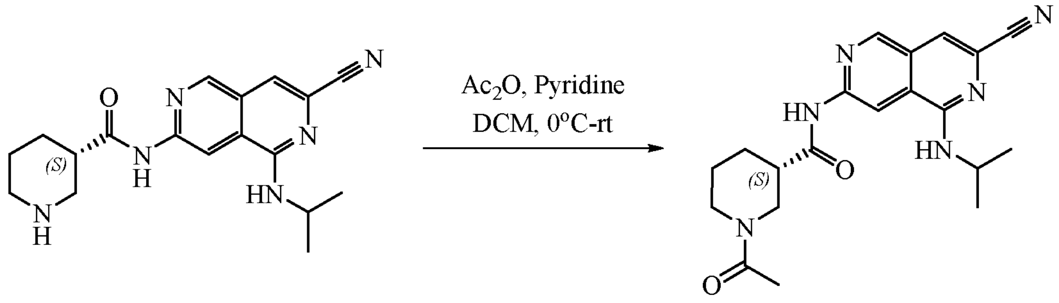


(*S*)-*N*-(7-cyano-5-(isopropylamino)-2,6-naphthyridin-3-yl)piperidine-3-carboxamide (68 mg, 0.20 mmol, 1.0 eq.) was dissolved in DCM (3 mL). The reaction solution was added with DIPEA (77.5 mg, 0.60 mmol, 3.0 eq.) and acetic anhydride (30.7 mg, 0.30 mmol, 1.5 eq.) and reacted at room temperature for 3 h. When there were no materials left, as detected by TLC, the reaction solution was poured into water (20 mL) and extracted with ethyl acetate (20 mL × 3). The organic phases were combined, dried over anhydrous sodium sulfate and filtered. The filtrate was concentrated under reduced pressure to give a crude product, which was purified by preparative thin-layer chromatography (MeOH:DCM = 1:20) to give the product (45 mg, yield: 59.2%).

$^1$HNMR(300 MHz, $CD_3OD$) δ(ppm): 8.94 (s, 1H), 8.54 (s, 1H), 7.51 (s, 1H), 4.42-4.25 (m, 2H), 4.05-3.89 (m, 1H), 3.56-3.48 (m, 1H), 3.28-3.19 (m, 1H), 3.08-2.92 (m, 1H), 2.83-2.63 (m, 1H), 2.19-2.16 (m, 4H), 1.94-1.82 (m, 2H), 1.38-1.35 (m, 6H).

Molecular formula: $C_{20}H_{24}N_6O_2$ Exact mass: 380.20 LC-MS (Pos, *m/z*) =381.25 $[M+H]^+$.

**Example 25: Synthesis of (1*S*,3*S*)-3-acetamido-*N*-(7-cyano-5-(isopropylamino)-2,6-naphthyridin-3-yl)cyclohexane-1-carboxamide (compound 25)**

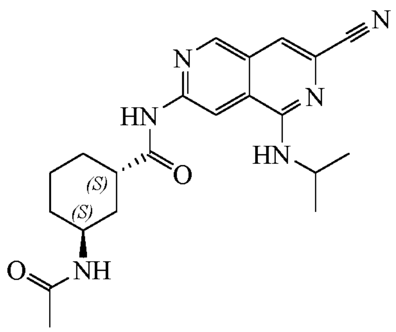

Step: synthesis of (1*S*,3*S*)-3-acetamido-*N*-(7-cyano-5-(isopropylamino)-2,6-naphthyridin-3-yl)cyclohexane-1-carboxamide

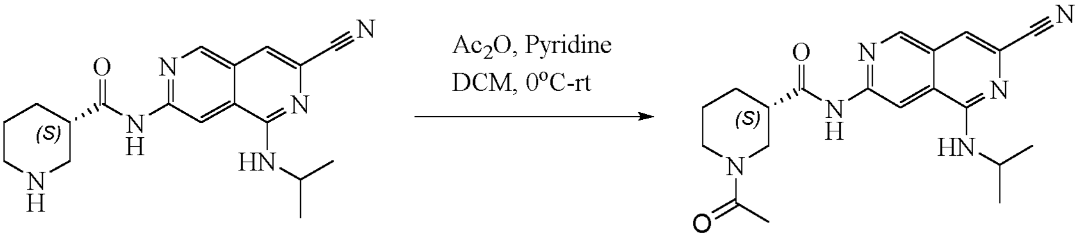

(1*S*,3*S*)-3-acetamido-*N*-(7-cyano-5-(isopropylamino)-2,6-naphthyridin-3-yl)cyclohexane-1-carboxamide (73 mg, 0.21 mmol, 1.0 eq.) was dissolved in DCM (3 mL). The reaction solution was added with DIPEA (81.3 mg, 0.63 mmol, 3.0 eq.) and acetic anhydride (32 mg, 0.31 mmol, 1.5 eq.) and reacted at room temperature for 1 h. When there were no materials left, as detected by TLC, the reaction solution was added with water (20 mL) and extracted with ethyl acetate (20 mL × 3). The organic phases were combined, dried over anhydrous sodium sulfate and filtered. The filtrate was concentrated under reduced pressure to give a crude product, which was purified by preparative thin-layer chromatography (MeOH:DCM = 1:20) to give the product (68 mg, yield: 82.1%).

$^1$HNMR (400 MHz, $CD_3OD$) δ(ppm): 8.97 (s, 1H), 8.64 (s, 1H), 7.58 (s, 1H), 4.50-4.43 (m, 1H), 4.25-4.24 (m, 1H), 2.89-2.87 (m, 1H), 2.00 (m, 4H), 1.90-1.71 (m, 6H), 1.63-1.60 (m, 1H), 1.39-1.37 (m, 6H).

Molecular formula: $C_{21}H_{26}N_6O_2$ Exact mass: 394.21 LC-MS (Pos, *m/z)* =395.18 [M+H]$^+$.

**Example 26: Synthesis of (1*S*,3*R*)-3-amino-*N*-(7-cyano-5-(isopropylamino)-2,6-naphthyridin-3-yl)cyclohexane-1-carboxamide (compound 26)**

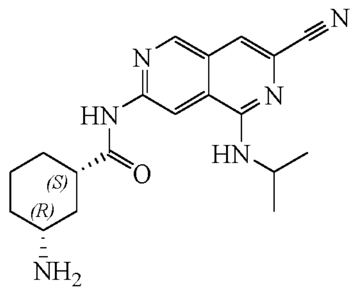

Step 1: synthesis of *tert*-butyl ((1*R*,3*S*)-3-((7-cyano-5-(isopropylamino)-2,6-naphthyridin-3-yl)carbamoyl)cyclohexyl)carbamate

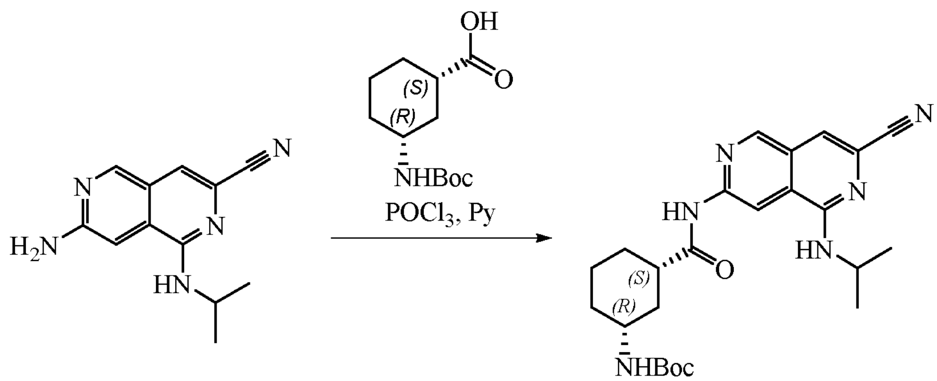


7-amino-1-(isopropylamino)-2,6-naphthyridine-3-carbonitrile (120 mg, 0.53 mmol, 1.0 eq.) and (1*S*,3*R*)-3-((*tert*-butoxycarbonyl)amino)cyclohexane-1-carboxylic acid (128.9 mg, 0.53 mmol, 1.0 eq.) were dissolved in pyridine (1 mL), and the reaction solution was added with phosphorus oxychloride (162.4 mg, 1.06 mmol, 2 eq.) and reacted at room temperature for 0.5-1 h. When there were no materials left, as detected by TLC, the reaction solution was added with water (20 mL) and extracted with ethyl acetate (20 mL × 3). The organic phases were combined, washed with hydrochloric acid (1 mol/L, 30 mL × 2), dried over anhydrous sodium sulfate and filtered, and the filtrate was concentrated under reduced pressure to give a crude product, which was purified by silica gel column chromatography (EA:PE = 1:10-1:2) to give the product (115 mg, yield: 47.9%).

Step 2: synthesis of (1*S*,3*R*)-3-amino-*N*-(7-cyano-5-(isopropylamino)-2,6-naphthyridin-3-yl)cyclohexane-1-carboxamide

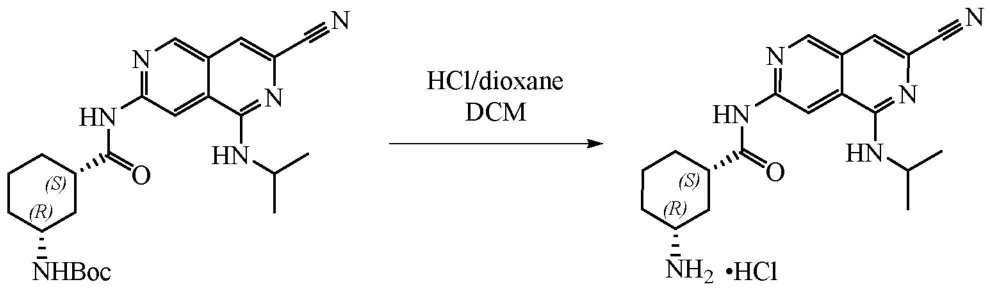


Tert-butyl ((1*R*,3*S*)-3-((7-cyano-5-(isopropylamino)-2,6-naphthyridin-3-yl)carbamoyl)cyclohexyl)carbamate (115 mg, 0.25 mmol, 1.0 eq.) was dissolved in DCM (6 mL). The reaction solution was added dropwise with a solution of hydrogen chloride (4 mol/L) in dioxane (4 mL) and reacted at room temperature for 18 h. When there were no materials left, as detected by LC-MS, the reaction solution was filtered, and the filter cake was dissolved in water (5 mL) and lyophilized to give the product (86 mg, yield: 88.4%).

$^1$HNMR(400 MHz, $CD_3OD$) δ(ppm): 9.07 (s, 1H), 8.80 (s, 1H), 7.78 (s, 1H), 4.46-4.40 (m, 1H), 3.33-3.24 (m, 1H), 2.79-2.73 (m, 1H), 2.28-2.25 (m, 1H), 2.12-2.02 (m, 4H), 1.78-1.53 (m, 1H), 1.51-1.43 (m,2H), 1.41-1.40 (m, 6H).

Molecular formula: $C_{19}H_{25}N_6$ Cl O Exact mass: 388.18 LC-MS (Pos, *m/z)* =353.17 $[M+H]^+$.

**Example 27: Synthesis of (1*S*,3*S*)-3-amino-*N*-(7-cyano-5-(isopropylamino)-2,6-naphthyridin-3-yl)cyclohexane-1-carboxamide (compound 27)**

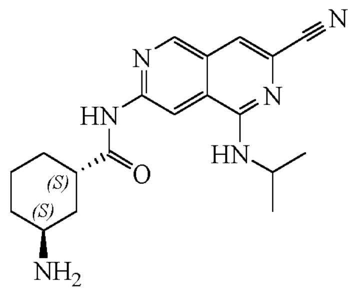

Step 1: synthesis of *tert*-butyl ((1*S*,3*S*)-3-((7-cyano-5-(isopropylamino)-2,6-naphthyridin-3-yl)carbamoyl)cyclohexyl)carbamate

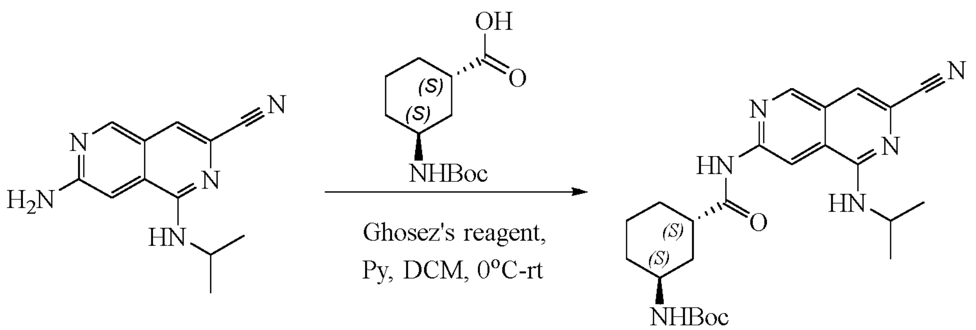


(1*S*,3*S*)-3-((*tert*-butoxycarbonyl)amino)cyclohexane-1-carboxylic acid (267.6 mg, 1.10 mmol, 1.05 eq.) was dissolved in DCM (5 mL). The reaction solution was cooled to 0 °C to 5 °C in an ice water bath, added with 1-chloro-*N,N*,2-trimethylprop-1-en-1-amine (mass fraction: 95%, 220.8 mg, 1.57 mmol, 1.5 eq.), reacted at 0 °C to 5 °C for 1.5 h, added with a solution of 7-amino-1-(isopropylamino)-2,6-naphthyridine-3-carbonitrile (240 mg, 1.05 mmol, 1.0 eq.) and pyridine (249.1 mg, 3.15 mmol, 3.0 eq.) in THF (10 mL), and incubated at room temperature for 18 h. The supernate

was poured into water (20 mL) and extracted with ethyl acetate (20 mL × 2). The organic phases were combined, washed with hydrochloric acid solution (1 mol/L, 20 mL), dried over anhydrous sodium sulfate and filtered, and the filtrate was concentrated under reduced pressure to give a crude product, which was purified by silica gel column chromatography (EA:PE = 1:5-1:2) to give the product (269 mg, yield: 56.6%).

Step 2: synthesis of (1*S*,3*S*)-3-amino-*N*-(7-cyano-5-(isopropylamino)-2,6-naphthyridin-3-yl)cyclohexane-1-carboxamide

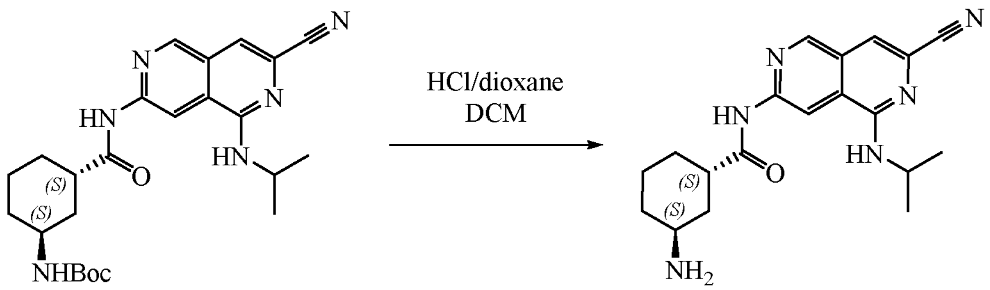


Tert-butyl ((1*S*,3*S*)-3-((7-cyano-5-(isopropylamino)-2,6-naphthyridin-3-yl)carbamoyl)cyclohexyl)carbamate (269 mg, 0.59 mmol, 1.0 eq.) was dissolved in DCM (8 mL). The reaction solution was added dropwise with a solution of hydrogen chloride (4 mol/L) in dioxane (4 mL) and reacted at room temperature for 3 h. When there were no materials left, as detected by LC-MS, the reaction solution was filtered, and a part of filter cake (68 mg) was dissolved in water (10 mL), adjusted to pH of about 8 with sodium bicarbonate and extracted with ethyl acetate (20 mL × 3). The organic phases were combined, dried over anhydrous sodium sulfate and filtered, and the filtrate was concentrated under reduced pressure to give a crude product, which was purified by preparative thin-layer chromatography (MeOH:DCM = 1:20) to give the product (31 mg, yield: 30.9%).
$^1$HNMR(400 MHz, $CD_3OD$) δ(ppm): 8.94 (s, 1H), 8.54 (s, 1H), 7.51 (s, 1H), 4.52-4.46 (m, 1H), 3.51-3.46 (m, 1H), 3.00-2.96 (m, 1H), 2.12-2.11 (m, 1H), 1.87-1.82 (m, 2H), 1.76-1.72 (m, 3H), 1.52-1.47 (m,2H), 1.37-1.35 (m, 6H).
Molecular formula: $C_{19}H_{24}N_6O$ Exact mass: 352.20 LC-MS (Pos, *m/z)* =353.17 $[M+H]^+$.

**Example 28: Synthesis of 7-(((1*S*,3*S*)-3-aminocyclopentyl)amino)-1-(isopropylamino)-2,6-naphthyridine-3-carbonitrile (compound 28)**

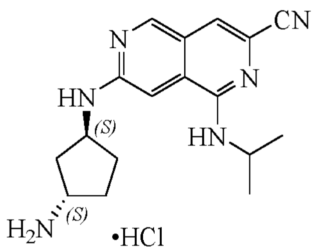

Steps:

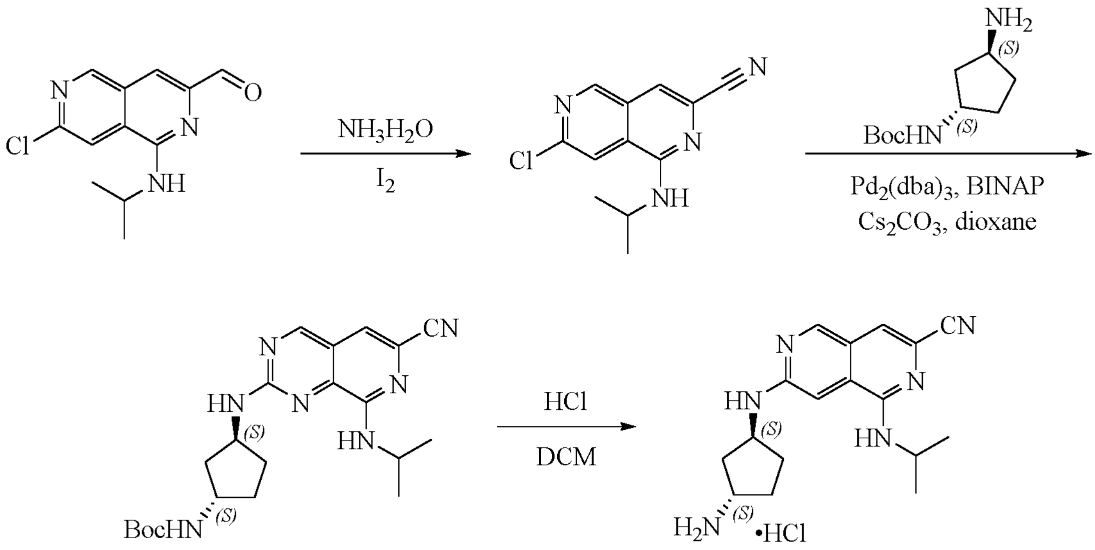


Step 1: synthesis of 7-chloro-1-(isopropylamino)-2,6-naphthyridine-3-carbonitrile

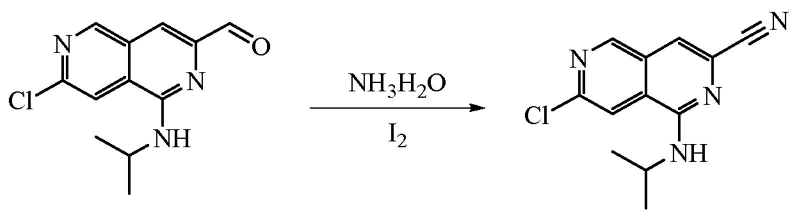


7-chloro-1-(isopropylamino)-2,6-naphthyridine-3-carbaldehyde (2.5 g, 10 mmol, 1.0 eq.) was dissolved in THF (50 mL). The reaction solution was added with aqueous ammonia (mass fraction: 28%, 50 mL), added with iodine (2.54 g, 10 mmol, 1.0 eq.) and reacted at room temperature for 4 h. When there were no materials left, as detected by TLC, the reaction solution was added with water (100 mL) and extracted with ethyl acetate (100 mL × 4). The organic phases were combined, washed with aqueous sodium thiosulfate solution (mass fraction: 10%, 200 mL), dried over anhydrous sodium sulfate and filtered, and the filtrate was concentrated under reduced pressure to give a crude product, which was purified by silica gel column chromatography (EA:PE = 1:10-1:5) to give the product (1.8 g, yield: 73.2%).

Step 2: synthesis of *tert*-butyl ((1*S*,3*S*)-3-((6-cyano-8-(isopropylamino)pyrido[3,4-*d*]pyrimidin-2-yl)amino)cyclopentyl)carbamate

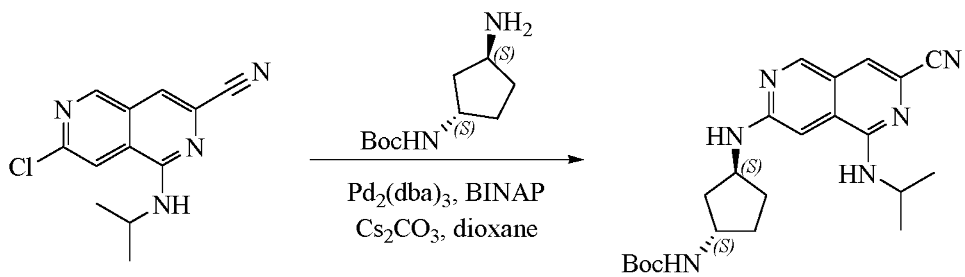


7-chloro-1-(isopropylamino)-2,6-naphthyridine-3-carbonitrile (100 mg, 0.41 mmol, 1.0 eq.), *tert*-butyl ((1*S*,3*S*)-3-aminocyclopentyl)carbamate (98.1 mg, 0.49 mmol, 1.2 eq.), $Pd_2(dba)_3$ (36.6 mg, 0.04 mmol, 0.1 eq.), BINAP (49.8 mg, 0.08 mmol, 0.2 eq.) and cesium carbonate (335.6 mg, 1.03 mmol, 2.5 eq.) were added into 1,4-dioxane (6 mL). The mixture was reacted at 120 °C for 22 h in a sealed tube in nitrogen atmosphere. When there were no materials left, as

detected by TLC, the reaction solution was filtered, and the filtrate was concentrated under reduced pressure to give a crude product, which was purified by silica gel column chromatography (MeOH:DCM = 1:150-1:100) to give the product (86 mg, yield: 50.9%).

Step 3: synthesis of 7-(((1*S*,3*S*)-3-aminocyclopentyl)amino)-1-(isopropylamino)-2,6-naphthyridine-3-carbonitrile

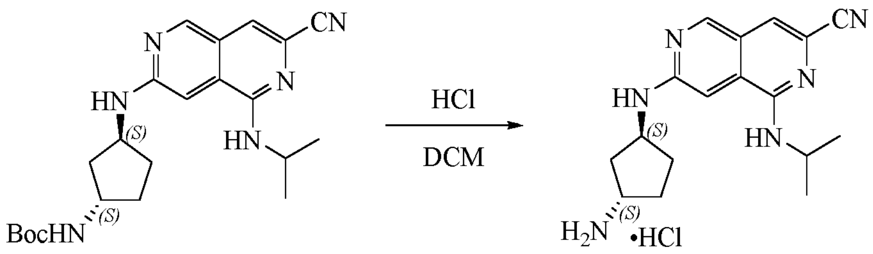


*Tert*-butyl ((1*S*,3*S*)-3-((6-cyano-8-(isopropylamino)pyrido[3,4-*d*]pyrimidin-2-yl) amino)cyclopentyl)carbamate (85 mg, 0.20 mmol, 1.0 eq.) was dissolved in DCM (4 mL). The reaction solution was added dropwise with a solution of hydrogen chloride (4 mol/L) in dioxane (3 mL) and reacted at room temperature for 4 h. When there were no materials left, as detected by LC-MS, the reaction solution was filtered, and the filter cake was dissolved in water (10 mL) and back-extracted with ethyl acetate (5 mL × 2). The aqueous phase was retained, adjusted to pH of about 8 with sodium bicarbonate and extracted with ethyl acetate (10 mL × 4). The organic phases were combined, dried over anhydrous magnesium sulfate and filtered, and the filtrate was concentrated under reduced pressure to give a crude product, which was dissolved in methanol (2 mL), added with hydrogen chloride ethanol solution (10 mol/L, 0.1 mL), stirred for 5 min, added with water (10 mL) and lyophilized to give the product (42.5 mg, yield: 61.3%).
$^1$HNMR(300 MHz, $CD_3OD$) δ(ppm): 8.83 (s, 1H), 7.73 (s, 1H), 7.68 (s, 1H), 4.68-4.65 (m, 1H), 4.44-4.37 (m, 1H), 3.90-3.87 (m, 1H), 2.44-2.35 (m, 2H), 2.31-2.24 (m, 2H), 2.21-2.16 (m, 2H), 1.85-1.83 (m,6H).
Molecular formula: $C_{17}H_{23}N_6Cl$ Exact mass: 346.17 LC-MS (Pos, *m/z)* =311.17 $[M+H]^+$.

**Example 29: Synthesis of 7-(((1*S*,3*R*)-3-aminocyclopentyl)amino)-1-(isopropylamino)-2,6-naphthyridine-3-carbonitrile (compound 29)**

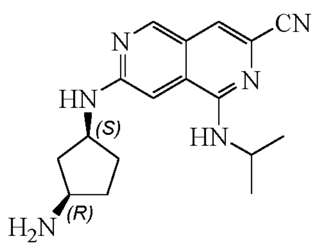

Step 1: synthesis of *tert*-butyl ((1*R*,3*S*)-3-((7-cyano-5-(isopropylamino)-2,6-naphthyridin-3-yl)amino)cyclopentyl)carbamate

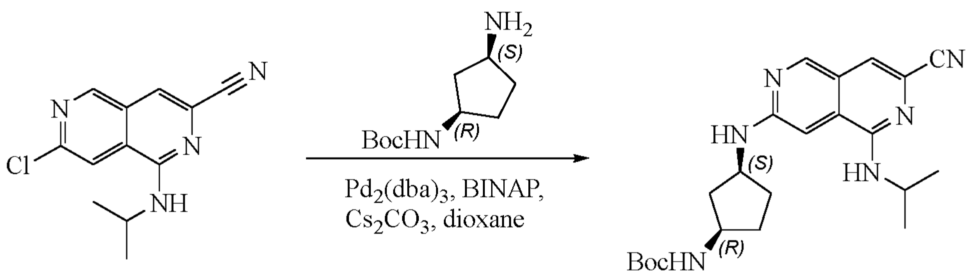

7-chloro-1-(isopropylamino)-2,6-naphthyridine-3-carbonitrile (130 mg, 0.53 mmol, 1.0 eq.), *tert*-butyl ((1*R*,3*S*)-3-aminocyclopentyl)carbamate (128.2 mg, 0.64 mmol, 1.2 eq.), $Pd_2(dba)_3$ (73.2 mg, 0.08 mmol, 0.15 eq.), BINAP (99.6 mg, 0.16 mmol, 0.3 eq.) and cesium carbonate (433.3 mg, 1.33 mmol, 2.5 eq.) were added into 1,4-dioxane (10 mL). The mixture was reacted at 120 °C for 17 h in a sealed tube in nitrogen atmosphere. When there were no materials left, as detected by TLC, the obtained crude material was purified by silica gel column chromatography (MeOH:DCM = 1:100) to give a crude product (145 mg), and the crude product was purified by preparative thin-layer chromatography (MeOH:DCM = 1:20) to give the product (86 mg, yield: 39.5%).

Step 2: synthesis of 7-(((1*S*,3*R*)-3-aminocyclopentyl)amino)-1-(isopropylamino)-2,6-naphthyridine-3-carbonitrile

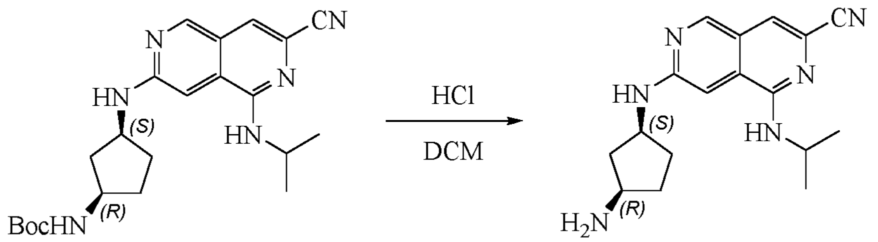


*Tert*-butyl ((1*R*,3*S*)-3-((7-cyano-5-(isopropylamino)-2,6-naphthyridin-3-yl)amino)cyclopentyl)carbamate (86 mg, 0.21 mmol, 1.0 eq.) was dissolved in DCM (4 mL). The reaction solution was added dropwise with a solution of hydrogen chloride (4 mol/L) in dioxane (4 mL) and reacted at room temperature for 2 h. When there were no materials left, as detected by LC-MS, the reaction solution was added with water (10 mL) and back-extracted with ethyl acetate (10 mL × 3). The aqueous phase was retained, adjusted to pH of about 8 with sodium bicarbonate and extracted with dichloromethane (10 mL × 3). The organic phases were combined, dried over anhydrous sodium sulfate and filtered, and the filtrate was concentrated under reduced pressure to give a crude product, which was purified by preparative thin-layer chromatography (MeOH:DCM = 1:20) to give the product (27 mg, yield: 41.4%).
$^1$HNMR (400 MHz, $CD_3OD$) δ(ppm): 8.67 (s, 1H), 7.37 (s, 1H), 6.93 (s, 1H), 4.48-4.45 (m, 1H), 4.33-4.23 (m, 1H), 3.58-3.54 (m, 1H), 2.67-2.58 (m, 1H), 2.23-2.06 (m, 1H), 1.81-1.73 (m, 2H), 1.53-1.45 (m, 2H), 1.35-1.33 (m, 6H).
Molecular formula: $C_{17}H_{22}N_6$ Exact mass: 310.19 LC-MS (Pos, *m/z)* =311.27 $[M+H]^+$.

**Example 30: Synthesis of 7-(((1*R*,3*R*)-3-aminocyclopentyl)amino)-1-(isopropylamino)-2,6-naphthyridine-3-carbonitrile (compound 30)**

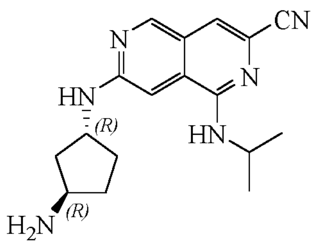

Step 1: synthesis of *tert*-butyl ((1*R*,3*R*)-3-((7-cyano-5-(isopropylamino)-2,6-naphthyridin-3-yl)amino)cyclopentyl)carbamate

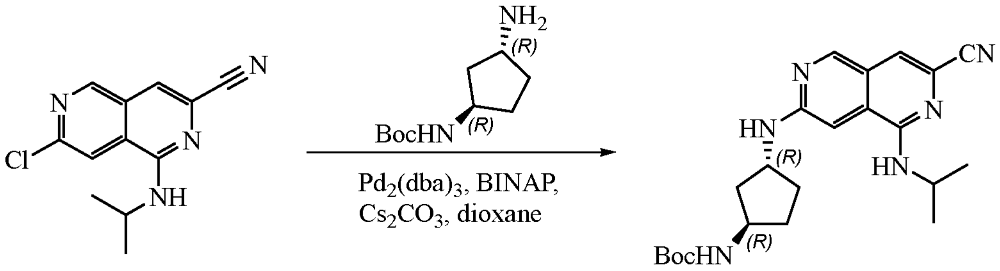


7-chloro-1-(isopropylamino)-2,6-naphthyridine-3-carbonitrile (150 mg, 0.61 mmol, 1.0 eq.), *tert*-butyl ((1*R*,3*R*)-3-aminocyclopentyl)carbamate (146.2 mg, 0.73 mmol, 1.2 eq.), $Pd_2(dba)_3$ (82.4 mg, 0.09 mmol, 0.15 eq.), BINAP (112 mg, 0.18 mmol, 0.3 eq.) and cesium carbonate (498.5 mg, 1.53 mmol, 2.5 eq.) were added into 1,4-dioxane (10 mL). The mixture was reacted at 120 °C for 17 h in a sealed tube in nitrogen atmosphere. When there were no materials left, as detected by TLC, the obtained crude material was purified by silica gel column chromatography (MeOH:DCM = 1:100) to give a crude product (215 mg), and the crude product was purified by preparative thin-layer chromatography (MeOH:DCM = 1:20) to give the product (88 mg, yield: 35.1%).

Step 2: synthesis of 7-(((1*R*,3*R*)-3-aminocyclopentyl)amino)-1-(isopropylamino)-2,6-naphthyridine-3-carbonitrile

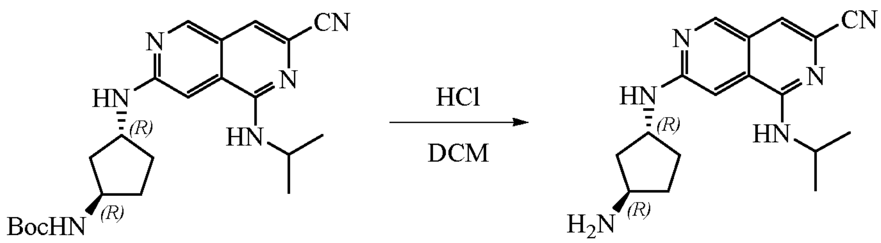


*Tert*-butyl ((1*R*,3*R*)-3-((7-cyano-5-(isopropylamino)-2,6-naphthyridin-3-yl)amino)cyclopentyl)carbamate (88 mg, 0.21 mmol, 1.0 eq.) was dissolved in DCM (3 mL). The reaction solution was added dropwise with hydrogen chloride ethanol solution (10 mol/L, 3 mL) and reacted at room temperature for 2 h. When there were no materials left, as detected by LC-MS, the reaction solution was added with water (10 mL) and back-extracted with ethyl acetate (10 mL × 3). The aqueous phase was retained, adjusted to pH of about 8 with sodium bicarbonate and extracted with dichloromethane (10 mL × 3). The organic phases were combined, dried over anhydrous sodium sulfate and filtered, and the filtrate was concentrated under reduced pressure to give a crude product, which was purified by preparative thin-layer chromatography (MeOH:DCM = 1: 10) to give the product (34 mg, yield: 52.1%).
$^1$HNMR (400 MHz, $CD_3OD$) δ(ppm): 8.68 (s, 1H), 7.37 (s, 1H), 6.90 (s, 1H), 4.47-4.42 (m, 2H), 3.77-3.72 (m, 1H), 2.39-2.28 (m, 2H), 2.10-2.06 (m, 2H), 1.74-1.63 (m, 2H), 1.35-1.33 (m, 6H).
Molecular formula: $C_{17}H_{22}N_6$ Exact mass: 310.19 LC-MS (Pos, *m/z)* =311.37 $[M+H]^+$.

**Example 31: Synthesis of (S)-1-(isopropylamino)-7-(pyrrolidin-3-ylamino)-2,6-naphthyridine-3-carbonitrile (compound 31)**

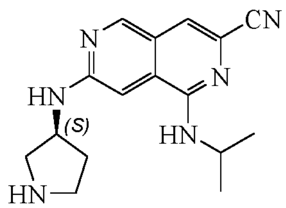

Step 1: synthesis of *tert*-butyl (*S*)-3-((7-cyano-5-(isopropylamino)-2,6-naphthyridin-3-yl)amino)pyrrolidine-1 -carboxylate

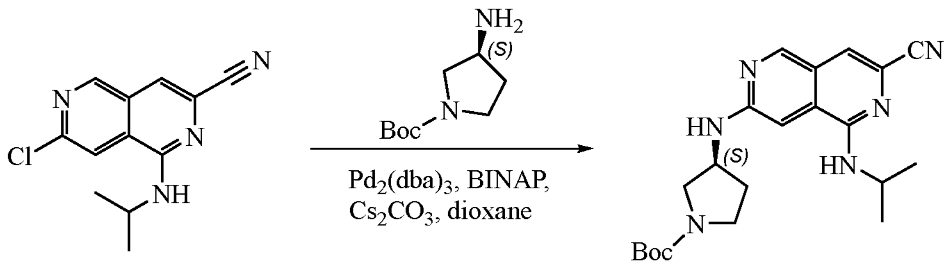


7-chloro-1-(isopropylamino)-2,6-naphthyridine-3-carbonitrile (150 mg, 0.61 mmol, 1.0 eq.), *tert*-butyl (*S*)-3-aminopyrrolidine-1-carboxylate (136 mg, 0.73 mmol, 1.2 eq.), $Pd_2(dba)_3$ (82.4 mg, 0.09 mmol, 0.15 eq.), BINAP (112 mg, 0.18 mmol, 0.3 eq.) and cesium carbonate (498.5 mg, 1.53 mmol, 2.5 eq.) were added into 1,4-dioxane (10 mL). The mixture was reacted at 120 °C for 17 h in a sealed tube in nitrogen atmosphere. When there were no materials left, as detected by TLC, the obtained crude material was purified by silica gel column chromatography (MeOH:DCM = 1:100) to give a crude product (146 mg), and the crude product was purified by preparative thin-layer chromatography (MeOH:DCM = 1:30) to give the product (83 mg, yield: 34.3%).

Step 2: synthesis of (*S*)-1-(isopropylamino)-7-(pyrrolidin-3-ylamino)-2,6-naphthyridine-3-carbonitrile

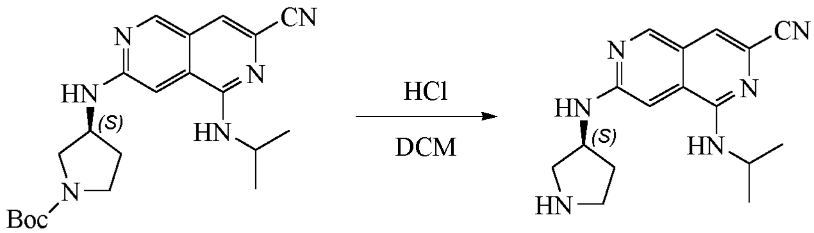


*Tert*-butyl (*S*)-3-((7-cyano-5-(isopropylamino)-2,6-naphthyridin-3-yl)amino)pyrrolidine-1-carboxylate (83 mg, 0.21 mmol, 1.0 eq.) was dissolved in DCM (3 mL). The reaction solution was added dropwise with hydrogen chloride ethanol solution (10 mol/L, 3 mL) and reacted at room temperature for 1 h. When there were no materials left, as detected by LC-MS, the reaction solution was added with water (10 mL) and back-extracted with ethyl acetate (10 mL × 2). The aqueous phase was retained, adjusted to pH of about 8-9 with sodium bicarbonate and extracted with dichloromethane (10 mL × 3). The organic phases were combined, dried over anhydrous sodium sulfate and filtered, and the filtrate was concentrated under reduced pressure to give a crude product, which was purified by preparative thin-layer chromatography (MeOH:DCM = 1:10) to give the product (18 mg, yield: 28.9%).
$^1$HNMR (400 MHz, DMSO+$D_2O$) δ(ppm): 8.67 (s, 1H), 7.44 (s, 1H), 6.86 (s, 1H), 4.31-4.21 (m, 2H), 2.20-2.14 (m, 1H), 1.93-1.86 (m, 2H), 1.69-1.60 (m, 1H), 1.51-1.35 (m, 2H), 1.22-1.20 (m, 6H).
Molecular formula: $C_{16}H_{20}N_6$ Exact mass: 296.17 LC-MS (Pos, *m/z)* =297.22 $[M+H]^+$.

**Example 32: Synthesis of 1-(isopropylamino)-7-(((1*S*,3*R*)-3-(methylamino)cyclopentyl)amino)-2,6-naphthyridine-3-carbonitrile (compound 32)**

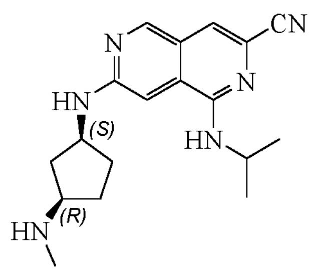

Steps:

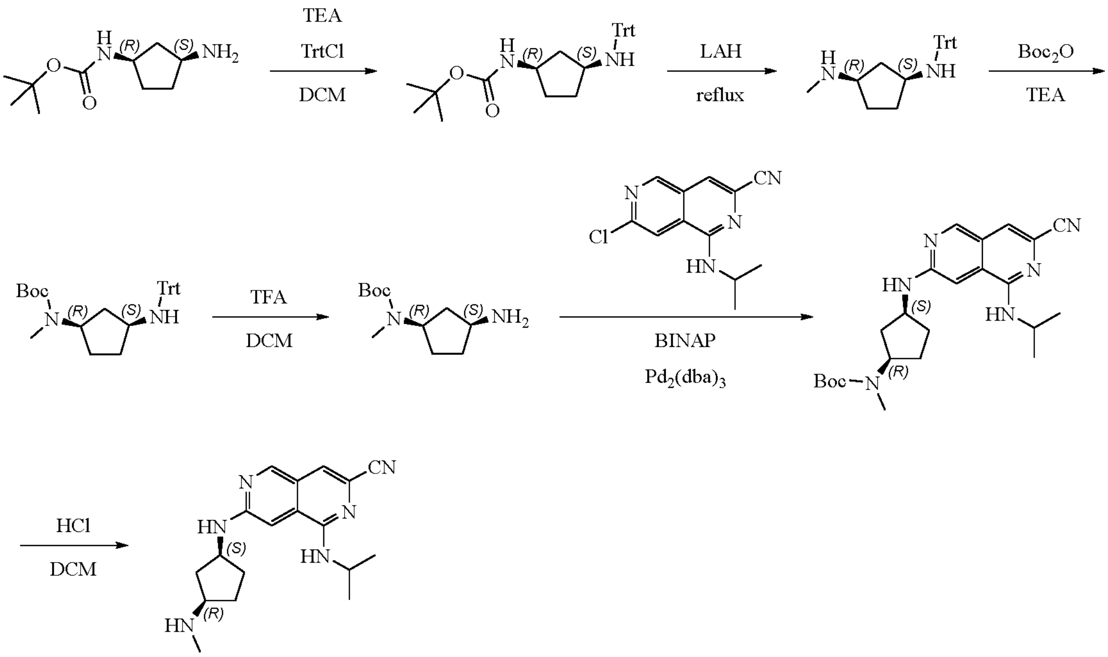


Step 1: synthesis of *tert*-butyl ((1*R*,3*S*)-3-(tritylamino)cyclopentyl)carbamate

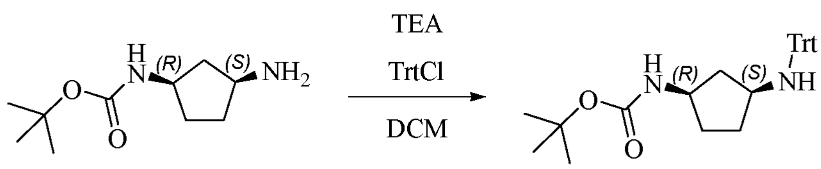


*Tert*-butyl ((1*R*,3*S*)-3-aminocyclopentyl)carbamate (550 mg, 2.75 mmol, 1.0 eq.), triphenylchloromethane (1.15 g, 4.13 mmol, 1.5 eq.) and triethylamine (556.5 mg, 5.5 mmol, 2.0 eq.) were dissolved in DCM (20 mL), and the reaction solution was reacted at room temperature for 3 h. When there were no materials left, as detected by TLC, the reaction solution was added with water (30 mL) and extracted with ethyl acetate (30 mL × 3). The organic phases were combined, dried over anhydrous sodium sulfate and filtered. The filtrate was concentrated under reduced pressure to give a crude product, which was purified by silica gel column chromatography (EA:PE = 1:20-1:15) to give the product (1.16 g, yield: 96.6%).

Step 2: synthesis of (1*R*,3*S*)-$N^1$-methyl-$N^3$-tritylcyclopentane-1,3-diamine

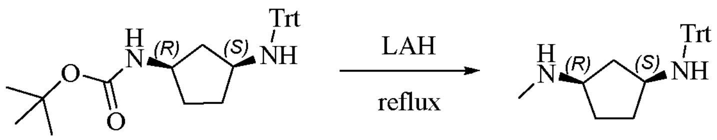


*Tert*-butyl ((1*R*,3*S*)-3-(tritylamino)cyclopentyl)carbamate (1.1 g, 2.48 mmol, 1.0 eq.) was dissolved in anhydrous THF (25 mL), and the reaction solution was added with lithium aluminum hydride (376.5 mg, 9.92 mmol, 4.0 eq.) in batches and heated to reflux for 1 h. When there were no materials left, as detected by TLC, the reaction solution was added with water (376.5 mg) and stirred for 2 min, added with sodium hydroxide solution (mass fraction: 10%, 376.5 mg) and stirred for 2 min, then added with water (1.1 g) and stirred for 5 min, added with ethyl acetate (50 mL), dried over anhydrous magnesium sulfate and filtered, and the filtrate was concentrated under reduced pressure to give the product (884.1 mg, yield: 100%).

Step 3: synthesis of *tert*-butyl methyl ((1*R*,3*S*)-3-(tritylamino)cyclopentyl)carbamate

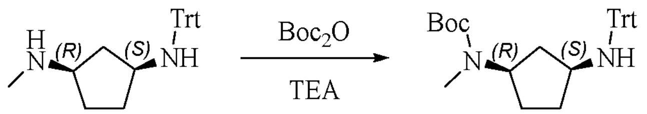


(1*R*,3*S*)-*N*[1]-methyl-*N*[3]-tritylcyclopentane-1,3-diamine (880 mg, 2.47 mmol, 1.0 eq.) was dissolved in DCM (10 mL), and the reaction solution was added with triethylamine (499.9 mg, 4.94 mmol, 2.0 eq.) and di-*tert*-butyl dicarbonate (646 mg, 2.96 mmol, 1.2 eq.), and reacted at room temperature for 15 h. When there were no materials left, as detected by TLC, the reaction solution was added with water (20 mL) and extracted with ethyl acetate (30 mL × 2). The organic phases were combined, washed with aqueous citric acid solution (mass fraction: 5%, 30 mL) and water (30 mL) successively, dried over anhydrous sodium sulfate and filtered, and the filtrate was concentrated under reduced pressure to give the product (1.1 g, yield: 100 %).

Step 4: synthesis of *tert*-butyl ((1*R*,3*S*)-3-aminocyclopentyl)(methyl)carbamate

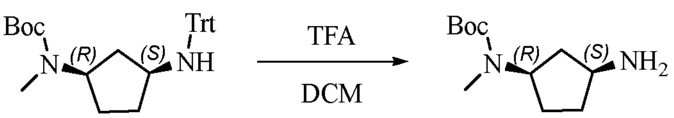


*Tert*-butyl methyl((1*R*,3*S*)-3-(tritylamino)cyclopentyl)carbamate (1.1 g, 2.47 mmol, 1.0 eq.) was dissolved in 1% (v/v) TFA/DCM (50 mL), and the reaction solution was reacted at room temperature for 18 h. When there were new spots generated, as detected by TLC, the reaction solution was added with water (20 mL) and adjusted to pH of about 9 with sodium hydroxide. The liquid separation was performed, the organic phase was retained, and the aqueous phase was extracted with DCM (30 mL). The organic phase was combined, dried over anhydrous sodium sulfate and filtered, and the filtrate was concentrated under reduced pressure to give a crude product, which was purified by silica gel column chromatography (MeOH:DCM = 1:20) to give the product (234 mg, yield: 44.2 %).

Step 5: synthesis of *tert*-butyl ((1*R*,3,*S*)-3-((7-cyano-5-(isopropylamino)-2,6-naphthyridin-3-yl)amino)cyclopentyl)(methyl)carbamate

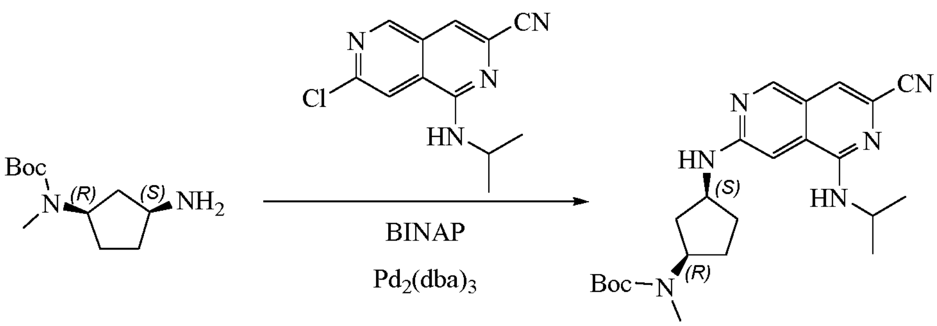


7-chloro-1-(isopropylamino)-2,6-naphthyridine-3-carbonitrile (200 mg, 0.81 mmol, 1.0 eq.), *tert*-butyl ((1*R*,3*S*)-3-aminocyclopentyl)(methyl)carbamate (225 mg, 1.05 mmol, 1.2 eq.), $Pd_2(dba)_3$ (109.9 mg, 0.12 mmol, 0.15 eq.), BINAP (149.4 mg, 0.24 mmol, 0.3 eq.) and cesium carbonate (658.1 mg, 2.02 mmol, 2.5 eq.) were added into 1,4-dioxane (20 mL). The mixture was reacted at 100 °C for 15 h in a sealed tube in nitrogen atmosphere. When there were no materials left, as detected by TLC, the reaction solution was added with water (30 mL) and extracted with ethyl acetate (30 mL × 2). The organic phases were combined, washed with hydrochloric acid solution (0.2 mol/L, 30 mL), dried over anhydrous sodium sulfate and filtered, and the filtrate was concentrated under reduced pressure to give a crude product, which was purified by preparative thin-layer chromatography (EA:PE = 1:2) to give the product (163 mg, yield: 47.4%).

Step 6: synthesis of 1-(isopropylamino)-7-(((1*S*,3*R*)-3-(methylamino)cyclopentyl)amino)-2,6-naphthyridine-3-carbonitrile

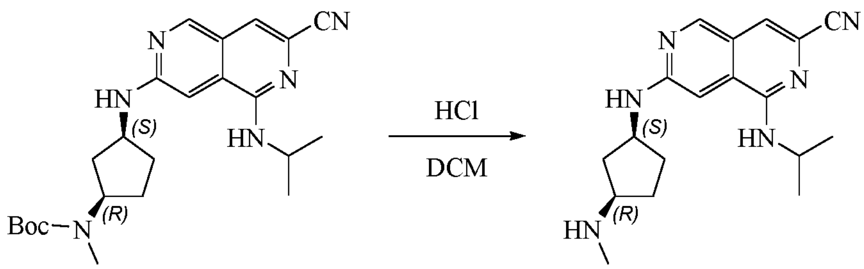


*Tert*-butyl ((1*R*,3*S*)-3-((7-cyano-5-(isopropylamino)-2,6-naphthyridin-3-yl)amino)cyclopentyl)(methyl)carbamate (163 mg, 0.38 mmol, 1.0 eq.) was dissolved in DCM (3 mL). The reaction solution was added dropwise with hydrogen chloride ethanol solution (10 mol/L, 3 mL) and reacted at room temperature for 1 h. When there were no materials left, as detected by TLC, the reaction solution was added with water (20 mL) and back-extracted with DCM (20 mL × 2). The aqueous phase was retained, adjusted to pH of about 8 with sodium bicarbonate and extracted with DCM (30 mL × 2). The organic phases were combined, dried over anhydrous sodium sulfate and filtered, and the filtrate was concentrated under reduced pressure to give a crude product, which was purified by preparative thin-layer chromatography (MeOH:DCM = 1: 10) to give the product (90 mg, yield: 72.9%).
$^1$HNMR (400 MHz, $CD_3OD$) δ(ppm): 8.86 (s, 1H), 7.36 (s, 1H), 6.94 (s, 1H), 4.49-4.43 (m, 1H), 4.29-4.25 (m, 1H), 3.46-3.43 (m, 1H), 2.70-2.63 (m, 1H), 2.61 (s, 3H), 2.14-2.12 (m, 2H), 1.86-1.79 (m, 2H), 1.61-1.53 (m, 1H), 1.35-1.33 (m, 6H).
Molecular formula: $C_{18}H_{24}N_6$ Exact mass: 324.21 LC-MS (Pos, *m/z)* =325.35 $[M+H]^+$.

**Example 33: Synthesis of 7-(((1*S*,3*S*)-3-hydroxycyclopentyl)amino)-1-(isopropylamino)-2,6-naphthyridine-3-carbonitrile (compound 33)**

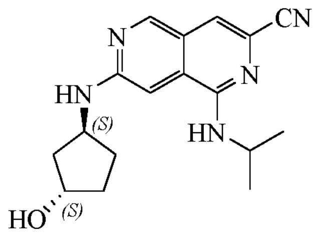

Steps:

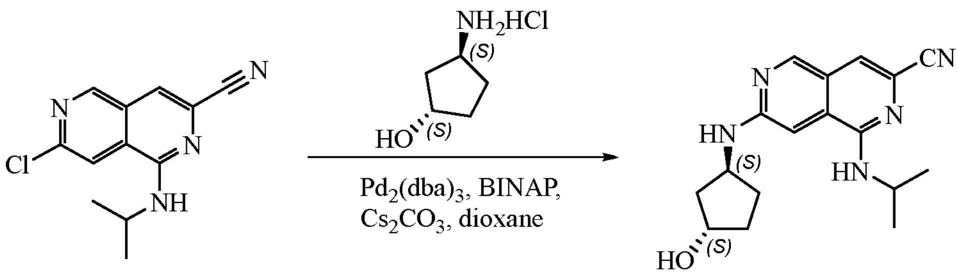


7-chloro-1-(isopropylamino)-2,6-naphthyridine-3-carbonitrile (150 mg, 0.61 mmol, 1.0 eq.), (1*S*,3*S*)-3-aminocyclopentan-1-ol hydrochloride (100.5 mg, 0.73 mmol, 1.2 eq.), $Pd_2(dba)_3$ (82.4 mg, 0.09 mmol, 0.15 eq.), BINAP (112 mg, 0.18 mmol, 0.3 eq.) and cesium carbonate (697.2 mg, 2.14 mmol, 3.5 eq.) were added into 1,4-dioxane (10 mL). The mixture was reacted at 120 °C for 18 h in a sealed tube in nitrogen atmosphere. When there were no materials left, as detected by TLC, the obtained crude material was purified by silica gel column chromatography (MeOH:DCM = 1:100) to give a crude product (150 mg), and the crude product was purified 2 times by preparative thin-layer chromatography (MeOH:DCM = 1:20) to give the product (34 mg, yield: 17.9%).

$^1$HNMR (400 MHz, DMSO) δ(ppm): 8.70 (s, 1H), 7.47 (s, 1H), 7.40-7.37 (d, 1H), 7.09-7.07 (d, 1H), 6.93 (s, 1H), 4.61-4.60 (m, 1H), 4.39-4.25 (m, 3H), 2.27-2.18 (m, 1H), 2.17-1.88 (m, 2H), 1.75-1.67 (m, 1H), 1.54-1.38 (m, 2H), 1.27-1.25 (m, 6H). Molecular formula: $C_{17}H_{21}N_5O$ Exact mass: 311.17 LC-MS (Pos, *m/z)* =312.27 $[M+H]^+$.

**Example 34: Synthesis of 7-(((1*S*,3*S*)-3-aminocyclopentyl)amino)-1-(*tert*-butylamino)-2,6-naphthyridine-3-carbonitrile (compound 34)**

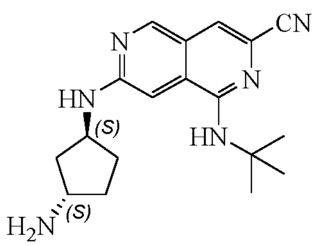

Step 1: synthesis of 1-(*tert*-butylamino)-7-chloro-2,6-naphthyridine-3-carbonitrile

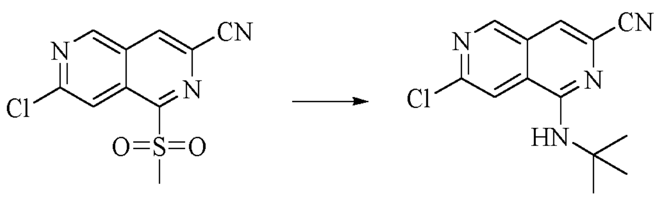

7-chloro-1-(methylsulfonyl)-2,6-naphthyridine-3-carbonitrile (400 mg, 1.49 mmol, 1.0 eq.) and *tert*-butylamine (545 mg, 7.45 mmol, 5.0 eq.) were dissolved in THF (10 mL), and the reaction solution was reacted at 80 °C for 3 h. After the reaction was completed, as detected by TLC, the reaction solution was poured into water (10 mL) and extracted with EA(10 mL × 3). The organic phase was dried and concentrated to give the product (360 mg, yield: 92.7%).

Step 2: synthesis of *tert*-butyl ((1*S*,3*S*)-3-((5-(*tert*-butylamino)-7-cyano-2,6-naphthyridin-3-yl)amino)cyclopentyl)carbamate

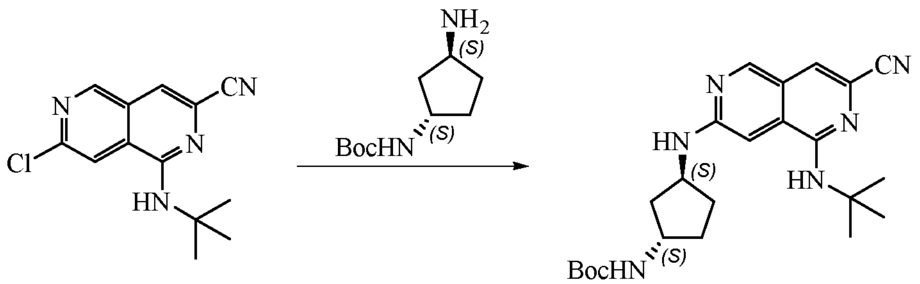

1-(*tert*-butylamino)-7-chloro-2,6-naphthyridine-3-carbonitrile (180 mg, 0.69 mmol, 1.0 eq.), *tert*-butyl ((1*S*,3*S*)-3-aminocyclopentyl)carbamate (166 mg, 0.83 mmol, 1.2 eq.), $Pd_2(dba)_3$ (63 mg, 0.069 mmol, 0.1 eq.), BINAP (86 mg, 0.138 mmol, 0.2 eq.) and cesium carbonate (450 mg, 1.38 mmol, 2.0 eq.) were dispersed in 1,4-dioxane (5 mL), and the reaction solution was reacted at 100 °C for 22 h in nitrogen atmosphere. After the reaction was completed, as detected by TLC, the reaction solution was concentrated under reduced pressure to give a crude product, which was purified by silica gel column chromatography (PE:EA = 2:1) to give the product (70 mg, yield: 23.9%).

Step 3: synthesis of 7-(((1*S*,3*S*)-3-aminocyclopentyl)amino)-1-(*tert*-butylamino)-2,6-naphthyridine-3-carbonitrile

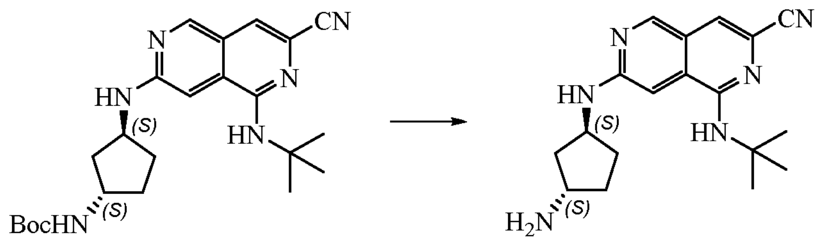

*Tert*-butyl ((1*S*,3*S*)-3-((5-(*tert*-butylamino)-7-cyano-2,6-naphthyridin-3-yl)amino)cyclopentyl)carbamate (70 mg, 0.16 mmol, 1.0 eq.) was dissolved in EA (2 mL), and the reaction solution was added dropwise a solution of hydrogen chloride in 1,4-dioxane (4 mol/L, 1 mL) and reacted at room temperature for 20 h. After the reaction was substantially completed, as detected by LC-MS, the reaction solution was poured into water (5 mL), and the liquid separation was performed. The aqueous phase was retained, adjusted to pH of 9 with sodium carbonate, and extracted with (MeOH:DCM = 1:10, 10 mL × 4). The organic phase was dried and concentrated to give a crude product, which was purified by preparative thin-layer chromatography (MeOH:DCM = 1:5) to give the product (40 mg, yield: 76.9%).
$^1$HNMR (400 MHz, MeOD) δ(ppm): 8.69 (s, 1H), 7.39 (s, 1H), 6.86 (s, 1H), 4.56-4.53 (t, 1H), 3.83-3.80 (t, 1H), 2.37-2.34 (t, 2H), 2.16-2.12 (m, 2H), 1.76-1.73 (m, 2H), 1.58 (s, 9H).
Molecular formula: $C_{18}H_{24}N_6$ Exact mass: 324.21 LC-MS(Pos, *m/z*)=325.36[M+H]$^+$.

**Example 35: Synthesis of 1-(*tert*-butylamino)-7-(((1*S*,3*S*)-3-hydroxycyclopentyl)amino)-2,6-naphthyridine-3-carbonitrile (compound 35)**

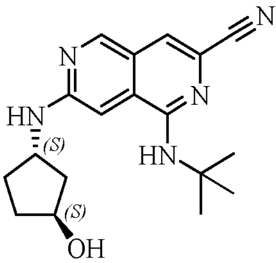

Steps:

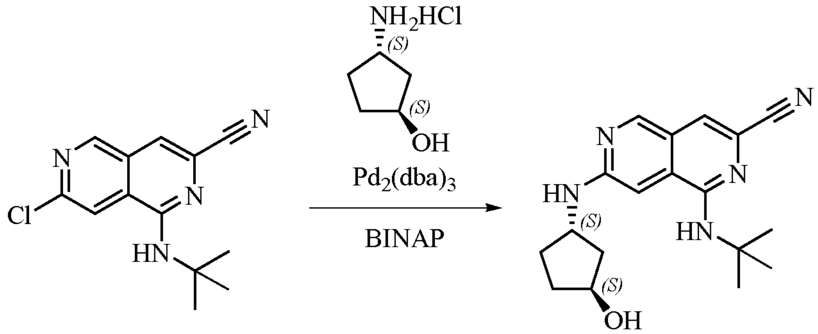


1-(*tert*-butylamino)-7-chloro-2,6-naphthyridine-3-carbonitrile (130 mg, 0.50 mmol, 1.0 eq.), (1*S*,3*S*)-3-aminocyclopentan-1-ol hydrochloride (103.2 mg, 0.75 mmol, 1.5 eq.), $Pd_2(dba)_3$ (68.7 mg, 0.075 mmol, 0.15 eq.), BINAP (93.4 mg, 0.15 mmol, 0.3 eq.) and cesium carbonate (570.2 mg, 1.75 mmol, 3.5 eq.) were added into 1,4-dioxane (15 mL). The mixture was reacted at 120 °C for 17 h in a sealed tube in nitrogen atmosphere. When there were no materials left, as detected by TLC, the reaction solution was added with water (20 mL) and extracted with ethyl acetate (30 mL × 3). The organic phases were combined, washed with hydrochloric acid solution (0.2 mol/L, 50 mL), dried over anhydrous sodium sulfate and filtered to give a crude material, the obtained crude material was purified by silica gel column chromatography (EA:PE = 1:5-EA = 100%) to give a crude product (87 mg), and the crude product was purified by preparative thin-layer chromatography (MeOH:DCM = 1:20) to give the product (52 mg, yield: 31.9%).
$^1$HNMR (400 MHz, MeOD) δ(ppm): 8.65 (s, 1H), 7.37 (s, 1H), 6.83 (s, 1H), 4.46-4.40 (m, 2H), 2.39-2.30 (m, 1H), 2.18-2.08 (m, 2H), 1.82-1.75 (m, 1H), 1.71-1.67 (m, 1H), 1.58 (m, 9H), 1.32-1.31 (m, 1H).
Molecular formula: $C_{18}H_{23}N_5O$ Exact mass: 325.19 LC-MS (Pos, *m*/*z*)=326.34 $[M+H]^+$.

**Example 36: Synthesis of 7-(((1*S*,3*R*)-3-hydroxycyclopentyl)amino)-1-(isopropylamino)-2,6-naphthyridine-3-carbonitrile (compound 36)**

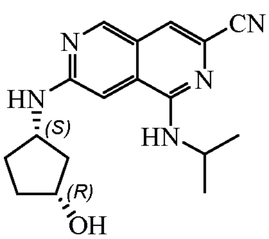

Steps:

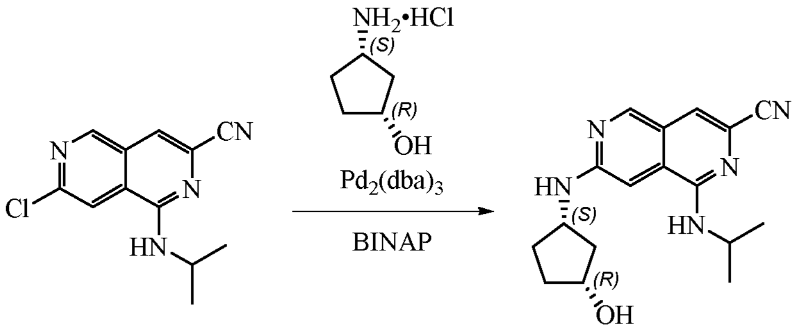


7-chloro-1-(isopropylamino)-2,6-naphthyridine-3-carbonitrile (100 mg, 0.40 mmol, 1.0 eq.), (1*R*,3*S*)-3-aminocyclopentan-1-ol hydrochloride (66 mg, 0.48 mmol, 1.2 eq.), $Pd_2(dba)_3$ (55 mg, 0.06 mmol, 0.15 eq.), BINAP (74.7 mg, 0.12 mmol, 0.3 eq.) and cesium carbonate (456.1 mg, 1.4 mmol, 3.5 eq.) were added into 1,4-dioxane (10 mL). The mixture was reacted at 120 °C for 17 h in a sealed tube in nitrogen atmosphere. When there were no materials left, as detected by TLC, the reaction solution was added with water (20 mL) and extracted with DCM (20 mL × 3). The organic phases were combined, washed with hydrochloric acid solution (0.1 mol/L, 30 mL), dried over anhydrous sodium sulfate and filtered, and the filtrate was concentrated under reduced pressure to give a crude product, which was purified by preparative thin-layer chromatography (MeOH:DCM = 1:20) to give the product (13 mg, yield: 10.4%).
$^1$HNMR (400 MHz, MeOD) δ(ppm): 8.64 (s, 1H), 7.36 (s, 1H), 6.94 (s, 1H), 4.48-4.44 (m, 1H), 4.38-4.36 (m, 1H), 4.29-4.24 (m, 1H), 2.24-2.35 (m, 1H), 2.20-2.21 (m, 1H), 1.94-1.89 (m, 1H), 1.85-1.78 (m, 2H), 1.66-1.60 (m, 1H), 1.35-1.33 (m, 6H). Molecular formula: $C_{17}H_{21}N_5O$ Exact mass: 311.17 LC-MS (Pos, *m/z)* =312.27 $[M+H]^+$.

**Example 37: Synthesis of 7-(((3*S*,5*S*)-5-(hydroxymethyl)pyrrolidin-3-yl)amino)-1-(isopropylamino)-2,6-naphthyridine-3-carbonitrile (compound 37)**

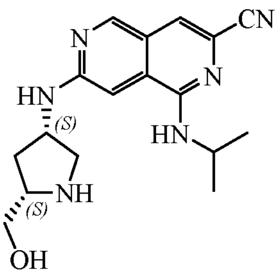

Step 1: synthesis of *tert*-butyl (2*S*,4*S*)-4-((7-cyano-5-(isopropylamino)-2,6-naphthyridin-3-yl)amino)-2-(hydroxymethyl)pyrrolidine-1-carboxylate

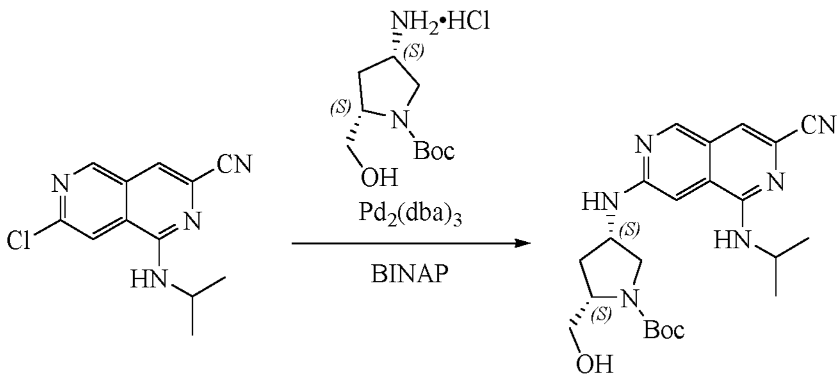


7-chloro-1-(isopropylamino)-2,6-naphthyridine-3-carbonitrile (150 mg, 0.61 mmol, 1.0 eq.), *tert*-butyl (2*S*,4*S*) -4-amino-2-(hydroxymethyl) pyrrolidine-1-carboxylate hydrochloride (308.3 mg, 1.22 mmol, 1.2 eq.), $Pd_2(dba)_3$ (82.4 mg, 0.09 mmol, 0.15 eq.), BINAP (112 mg, 0.18 mmol, 0.3 eq.) and cesium carbonate (697.2 mg, 2.14 mmol, 3.5 eq.) were added into 1,4-dioxane (10 mL). The mixture was reacted at 120 °C for 15 h in a sealed tube in nitrogen atmosphere. When there were no materials left, as detected by TLC, the reaction solution was added with water (20 mL) and extracted with ethyl acetate (20 mL × 3). The organic phases were combined, washed with hydrochloric acid solution (0.1 mol/L, 30 mL), dried over anhydrous sodium sulfate and filtered, and the filtrate was concentrated under reduced pressure to give a crude product, which was purified by preparative thin-layer chromatography (MeOH:DCM = 1:20) to give the product (151 mg, yield: 58%).

Step 2: synthesis of 7-(((3*S*,5*S*)-5-(hydroxymethyl)pyrrolidin-3-yl)amino)-1-(isopropylamino)-2,6-naphthyridine-3-carbonitrile

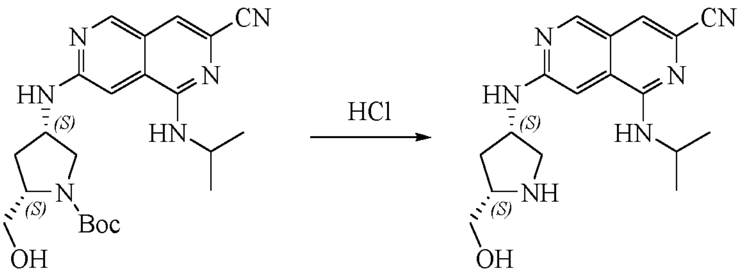


*Tert*-butyl (2*S*,4*S*)-4-((7-cyano-5-(isopropylamino)-2,6-naphthyridin-3-yl)amino)-2-(hydroxymethyl)pyrrolidine-1-carboxylate (150 mg, 0.35 mmol, 1.0 eq.) was dissolved in DCM (3 mL). The reaction solution was added dropwise with hydrogen chloride ethanol solution (10 mol/L, 3 mL) and reacted at room temperature for 2 h. When there were no materials left, as detected by TLC, the reaction solution was added with water (20 mL) and back-extracted with DCM (20 mL × 2). The aqueous phase was retained, adjusted to pH of about 8 with sodium carbonate and extracted with ethyl acetate (20 mL × 3). The organic phases were combined, dried over anhydrous sodium sulfate and filtered, and the filtrate was concentrated under reduced pressure to give a crude product, which was purified by preparative thin-layer chromatography (MeOH:DCM = 1:10) to give the product (33 mg, yield: 28.9%).
$^1$HNMR (400 MHz, $CD_3OD$) δ(ppm): 8.75 (s, 1H), 7.40 (s, 1H), 7.04 (s, 1H), 4.71-4.68 (m, 1H), 4.47-4.44 (m, 1H), 3.96-3.87 (m, 2H), 3.82-3.77 (m, 1H), 3.71-3.66 (m, 1H), 2.68-2.61 (m, 1H), 2.02-1.94 (m, 1H), 1.51-1.45 (m, 1H), 1.35-1.33 (m, 6H).
Molecular formula: $C_{17}H_{22}N_6O$ Exact mass: 326.19 LC-MS (Pos, *m/z)* =327.29[M+H]$^+$.

**Example 38: Synthesis of (*S*)-1-(isopropylamino)-7-(piperidin-3-ylamino)-2,6-naphthyridine-3-carbonitrile (compound 38)**

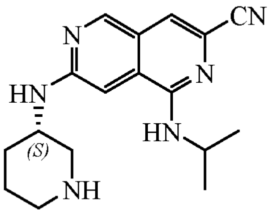

Step 1: synthesis of *tert*-butyl (*S*)-3-((7-cyano-5-(isopropylamino)-2,6-naphthyridin-3-yl)amino)piperidine-1-carboxylate:

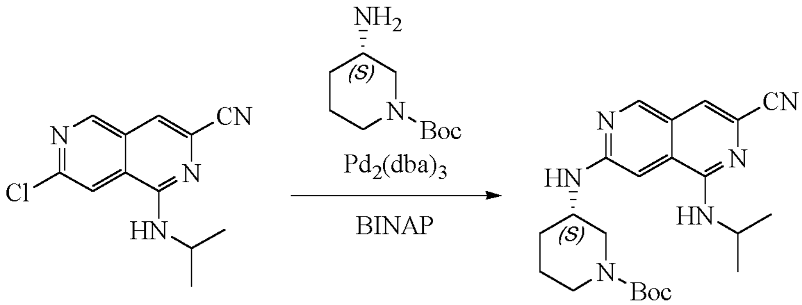


7-chloro-1-(isopropylamino)-2,6-naphthyridine-3-carbonitrile (150 mg, 0.61 mmol, 1.0 eq.), *tert*-butyl (*S*)-3-aminopiperidine-1-carboxylate (146.2 mg, 0.73 mmol, 1.2 eq.), $Pd_2(dba)_3$ (82.4 mg, 0.09 mmol, 0.15 eq.), BINAP (112 mg, 0.18 mmol, 0.3 eq.) and cesium carbonate (498.5 mg, 1.53 mmol, 2.5 eq.) were added into 1,4-dioxane (10 mL). The mixture was reacted at 120 °C for 18 h in a sealed tube in nitrogen atmosphere. When there were no materials left, as detected by TLC, the reaction solution was added with water (20 mL) and extracted with ethyl acetate (20 mL × 3). The organic phases were combined, washed with hydrochloric acid solution (0.2 mol/L, 20 mL), dried over anhydrous sodium sulfate and filtered, and the filtrate was concentrated under reduced pressure to give a crude product, which was purified by silica gel column chromatography (EA:PE = 1:10-1:2) to give the product (128 mg, yield: 51.1%).

Step 2: synthesis of (*S*)-1-(isopropylamino)-7-(piperidin-3-ylamino)-2,6-naphthyridine-3-carbonitrile

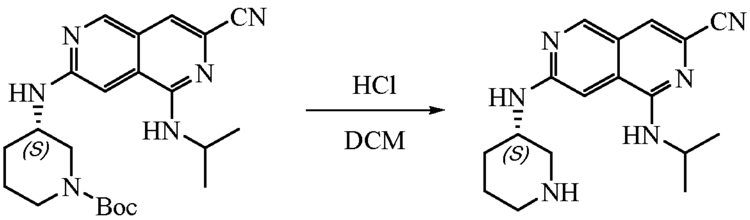


*Tert*-butyl (*S*)-3-((7-cyano-5-(isopropylamino)-2,6-naphthyridin-3-yl)amino)piperidine-1-carboxylate (128 mg, 0.31 mmol, 1.0 eq.) was dissolved in DCM (3 mL). The reaction solution was added dropwise with hydrogen chloride ethanol solution (10 mol/L, 3 mL) and reacted at room temperature for 3 h. When there were no materials left, as detected by TLC, the reaction solution was added with water (20 mL), adjusted to pH of about 8 with sodium carbonate and extracted with DCM (20 mL × 3). The organic phases were combined, dried over anhydrous sodium sulfate and filtered, and the filtrate was concentrated under reduced pressure to give a crude product, which was purified by preparative thin-layer chromatography (MeOH:DCM = 1:20) to give the product (61 mg, yield: 63.4%).

$^1$HNMR (400 MHz, $CD_3OD$) δ(ppm): 8.68 (s, 1H), 7.36 (s, 1H), 6.93 (s, 1H), 4.47-4.42 (m, 1H), 3.97-3.92 (m, 1H), 3.08-3.04 (m, 1H), 2.76-2.70 (m, 1H), 2.65-2.59 (m, 1H), 2.15-2.11 (m, 1H), 1.93-1.88 (m, 1H), 1.76-1.70 (m, 1H), 1.64-1.58 (m, 1H), 1.35-1.33 (m, 7H).

Molecular formula: $C_{17}H_{22}N_6$ Exact mass: 310.19 LC-MS (Pos, *m/z)* =311.17 $[M+H]^+$.

**Example 39: Synthesis of (R)-1-(isopropylamino)-7-(piperidin-3-ylamino)-2,6-naphthyridine-3-carbonitrile (compound 39)**

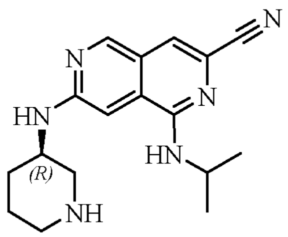

Step 1: synthesis of *tert*-butyl (*R*)-3-((7-cyano-5-(isopropylamino)-2,6-naphthyridin-3-yl)amino)piperidine-1-carboxylate

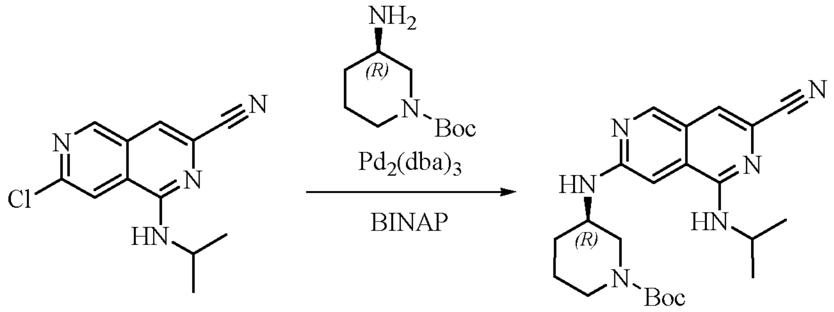


7-chloro-1-(isopropylamino)-2,6-naphthyridine-3-carbonitrile (150 mg, 0.61 mmol, 1.0 eq.), *tert*-butyl (*R*)-3-aminopiperidine-1-carboxylate (184.3 mg, 0.92 mmol, 1.5 eq.), $Pd_2(dba)_3$ (82.4 mg, 0.09 mmol, 0.15 eq.), BINAP (112 mg, 0.18 mmol, 0.3 eq.) and cesium carbonate (498.5 mg, 1.53 mmol, 2.5 eq.) were added into 1,4-dioxane (15 mL). The mixture was reacted at 120 °C for 16 h in a sealed tube in nitrogen atmosphere. When there were no materials left, as detected by TLC, the reaction solution was added with water (20 mL) and extracted with ethyl acetate (20 mL × 3). The organic phases were combined, washed with hydrochloric acid solution (0.2 mol/L, 30 mL), dried over anhydrous sodium sulfate and filtered, and the filtrate was concentrated under reduced pressure to give a crude product, which was purified by silica gel column chromatography (EA:PE = 1:5-1:2) to give the product (183 mg, yield: 73.1%).

Step 2: synthesis of (*R*)-1-(isopropylamino)-7-(piperidin-3-ylamino)-2,6-naphthyridine-3-carbonitrile

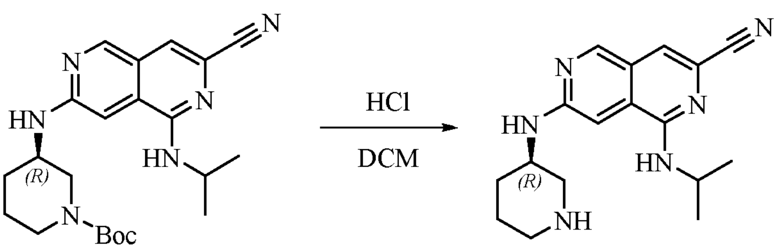


*Tert*-butyl (*R*)-3-((7-cyano-5-(isopropylamino)-2,6-naphthyridin-3-yl)amino)piperidine-1-carboxylate (180 mg, 0.44 mmol, 1.0 eq.) was dissolved in DCM (3 mL). The reaction solution was added dropwise with hydrogen chloride ethanol solution (10 mol/L, 3 mL) and reacted at room temperature for 2 h. When there were no materials left, as detected by TLC, the reaction solution was added with water (30 mL) and back-extracted with ethyl acetate (30 mL × 2). The aqueous phase was retained, adjusted to pH of about 8 with sodium carbonate and extracted with DCM (30 mL × 2). The organic phases were combined, dried over anhydrous sodium sulfate and filtered, and the filtrate was concentrated under reduced pressure to give a crude product, which was purified by preparative thin-layer chromatography (Me-

OH:DCM = 1:10) to give the product (100 mg, yield: 73.2%).
$^1$HNMR (400 MHz, $CD_3OD$) δ(ppm): 8.71 (s, 1H), 7.38 (s, 1H), 6.95 (s, 1H), 4.47-4.44 (m, 1H), 4.14-4.12 (m, 1H), 3.49-3.46 (m, 1H), 3.23-3.20 (m, 1H), 2.93-2.86 (m, 1H), 2.83-2.77 (m, 1H), 2.19-2.14 (m, 1H), 2.04-1.99 (m, 1H), 1.86-1.80 (m, 1H), 1.69-1.67 (m, 1H), 1.35-1.33 (m, 6H).
Molecular formula: $C_{17}H_{22}N_6$ Exact mass: 310.19 LC-MS (Pos, *m/z)* =311.31[M+H]$^+$.

**Example 40: Synthesis of 7-(((1*S*,3*R*)-3-aminocyclohexyl)amino)-1-(isopropylamino)-2,6-naphthyridine-3-carbonitrile (compound 40)**

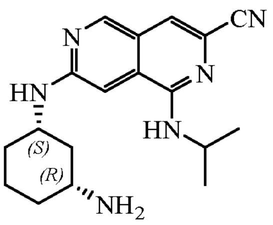

Step 1: synthesis of *tert*-butyl ((1*R*,3*S*)-3-((7-cyano-5-(isopropylamino)-2,6-naphthyridin-3-yl)amino)cyclohexyl)carbamate

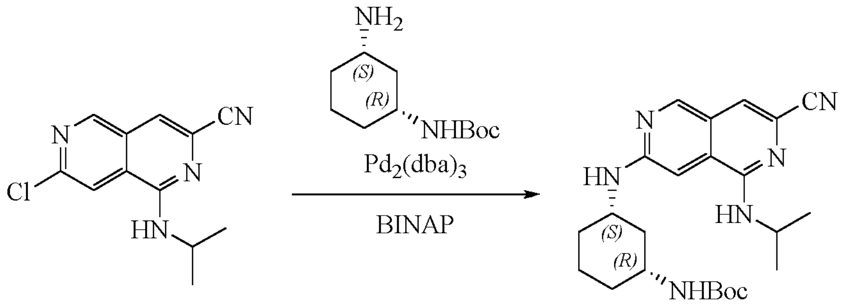


7-chloro-1-(isopropylamino)-2,6-naphthyridine-3-carbonitrile (150 mg, 0.61 mmol, 1.0 eq.), *tert*-butyl ((1*R*,3*S*)-3-aminocyclohexyl)carbamate (169.3 mg, 0.79 mmol, 1.3 eq.), $Pd_2(dba)_3$ (82.4 mg, 0.09 mmol, 0.15 eq.), BINAP (112 mg, 0.18 mmol, 0.3 eq.) and cesium carbonate (498.5 mg, 1.53 mmol, 2.5 eq.) were added into 1,4-dioxane (15 mL). The mixture was reacted at 120 °C for 17 h in a sealed tube in nitrogen atmosphere. When there were no materials left, as detected by TLC, the reaction solution was added with water (20 mL) and extracted with ethyl acetate (20 mL × 3). The organic phases were combined, washed with hydrochloric acid solution (0.1 mol/L, 30 mL), dried over anhydrous sodium sulfate and filtered, and the filtrate was concentrated under reduced pressure to give a crude product, which was purified by preparative thin-layer chromatography (MeOH:DCM = 1:20) to give the product (150 mg, yield: 57.9%).

Step 2: synthesis of 7-(((1*S*,3*R*)-3-aminocyclohexyl)amino)-1-(isopropylamino)-2,6-naphthyridine-3-carbonitrile

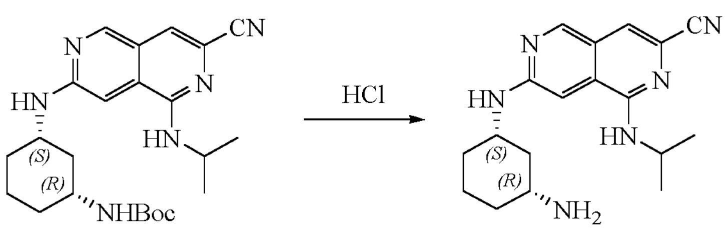

*Tert*-butyl ((1*R*,3*S*)-3-((7-cyano-5-(isopropylamino)-2,6-naphthyridin-3-yl)amino)cyclohexyl)carbamate (150 mg, 0.35 mmol, 1.0 eq.) was dissolved in DCM (3 mL). The reaction solution was added dropwise with hydrogen chloride ethanol solution (10 mol/L, 3 mL) and reacted at room temperature for 0.5 h. When there were no materials left, as detected by TLC, the reaction solution was concentrated under reduced pressure, added with water (20 mL) and back-extracted with DCM (20 mL × 2). The aqueous phase was retained, adjusted to pH of about 8 with sodium carbonate and extracted with ethyl acetate (20 mL × 3). The organic phases were combined, dried over anhydrous sodium sulfate and filtered, and the filtrate was concentrated under reduced pressure to give a crude product, which was purified by preparative thin-layer chromatography (MeOH:DCM = 1:10) to give the product (30 mg, yield: 26.4%).
$^1$HNMR (400 MHz, $CD_3OD$) δ(ppm): 8.68 (s, 1H), 7.37 (s, 1H), 6.93 (s, 1H), 4.49-4.93 (m, 1H), 3.96-3.90 (m, 1H), 3.26-3.23 (m, 1H), 2.47-2.44 (m, 1H), 2.14-2.07 (m, 2H), 2.03-1.98 (m, 1H), 1.61-1.54 (m, 1H), 1.43-1.39 (m, 2H), 1.35-1.33 (m, 6H), 1.31 (m, 1H).
Molecular formula: $C_{18}H_{24}N_6$ Exact mass: 324.21 LC-MS (Pos, *m/z*) =325.45 $[M+H]^+$.

**Example 41: Synthesis of 7-(((1*S*,3*R*)-3-hydroxycyclohexyl)amino)-1-(isopropylamino)-2,6-naphthyridine-3-carbonitrile (compound 41)**

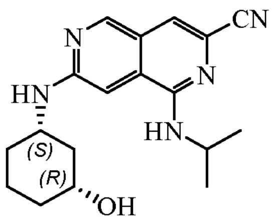

Steps:

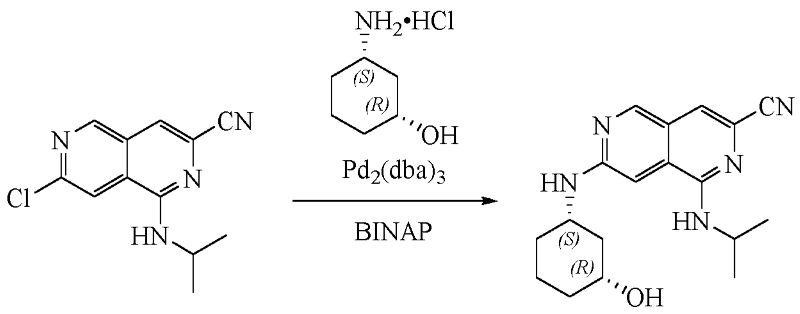


7-chloro-1-(isopropylamino)-2,6-naphthyridine-3-carbonitrile (100 mg, 0.40 mmol, 1.0 eq.), (1*R*,3*S*)-3-aminocyclohexyl-1-ol hydrochloride (121.3 mg, 0.80 mmol, 2.0 eq.), $Pd_2(dba)_3$ (55 mg, 0.06 mmol, 0.15 eq.), BINAP (74.7 mg, 0.12 mmol, 0.3 eq.) and cesium carbonate (456.1 mg, 1.4 mmol, 3.5 eq.) were added into 1,4-dioxane (10 mL). The mixture was reacted at 120 °C for 17 h in a sealed tube in nitrogen atmosphere. When there were no materials left, as detected by TLC, the reaction solution was added with water (20 mL) and extracted with ethyl acetate (30 mL × 3). The organic phases were combined, washed with hydrochloric acid solution (0.1 mol/L, 30 mL), dried over anhydrous sodium sulfate and filtered, and the filtrate was concentrated under reduced pressure to give a crude product, which was purified by preparative thin-layer chromatography (MeOH:DCM = 1:20) to give the product (45 mg, yield: 34.5%).
$^1$HNMR (400 MHz, MeOD) δ(ppm): 8.64 (s, 1H), 7.35 (s, 1H), 6.91 (s, 1H), 4.49-4.43 (m, 1H), 3.85-3.70 (m, 2H), 2.33-2.30 (m, 1H), 2.03-1.96 (m, 2H), 1.90-1.85 (m, 1H), 1.50-1.43 (m, 1H), 1.34-1.33 (m, 6H), 1.31-1.29 (m, 2H), 1.27 (m, 1H).
Molecular formula: $C_1sH_{23}NsO$ Exact mass: 325.19 LC-MS (Pos, *m/z*) =326.27 $[M+H]^+$.

**Example 42: Synthesis of 7-(((3*R*,4*S*)-4-fluoropiperidin-3-yl)amino)-1-(isopropylamino)-2,6-naphthyridine-3-carbonitrile (compound 42)**

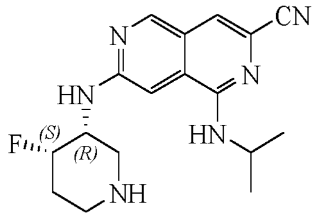

Step 1: synthesis of *tert*-butyl (3*R*,4*S*)-3-((7-cyano-5-(isopropylamino)-2,6-naphthyridin-3-yl)amino)-4-fluoropiperidine-1-carboxylate

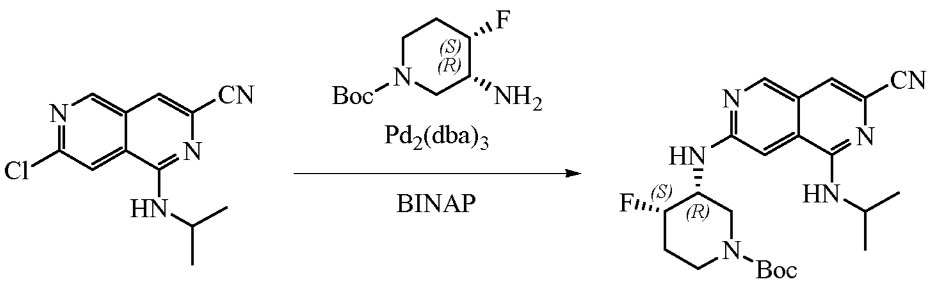


7-chloro-1-(isopropylamino)-2,6-naphthyridine-3-carbonitrile (150 mg, 0.61 mmol, 1.0 eq.), *tert*-butyl (3*R*,4*S*)-3-amino-4-fluoropiperidine-1-carboxylate (174.6 mg, 0.80 mmol, 1.3 eq.), $Pd_2(dba)_3$ (82.4 mg, 0.09 mmol, 0.15 eq.), BINAP (112 mg, 0.18 mmol, 0.3 eq.) and cesium carbonate (498.5 mg, 1.53 mmol, 2.5 eq.) were added into 1,4-dioxane (15 mL). The mixture was reacted at 100 °C for 17 h in a sealed tube in nitrogen atmosphere. When there were no materials left, as detected by TLC, the reaction solution was added with water (30 mL) and extracted with ethyl acetate (30 mL × 2). The organic phases were combined, washed with hydrochloric acid solution (0.5 mol/L, 50 mL), dried over anhydrous sodium sulfate and filtered, and the filtrate was concentrated under reduced pressure to give a crude product, which was purified by silica gel column chromatography (EA:PE = 1:5-1:3) to give the product (100 mg, yield: 38.2%).

Step 2: synthesis of 7-(((3*R*,4*S*)-4-fluoropiperidin-3-yl)amino)-1-(isopropylamino)-2,6-naphthyridine-3-carbonitrile

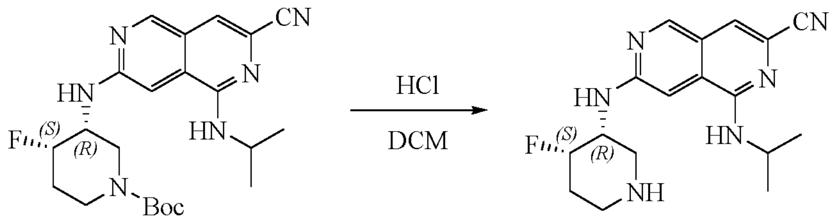


*Tert*-butyl (3*R*,4*S*)-3-((7-cyano-5-(isopropylamino)-2,6-naphthyridin-3-yl)amino)-4-fluoropiperidine-1-carboxylate (100 mg, 0.23 mmol, 1.0 eq.) was dissolved in DCM (5 mL). The reaction solution was added dropwise with hydrogen chloride ethanol solution (10 mol/L, 3 mL) and reacted at room temperature for 1 h. When there were no materials left, as detected by TLC, the reaction solution was added with water (20 mL) and back-extracted with DCM (20 mL × 2). The aqueous phase was retained, adjusted to pH of about 8 with potassium carbonate and extracted with DCM (20 mL × 3). The organic phases were combined, dried over anhydrous sodium sulfate and filtered, and the filtrate was concentrated under reduced pressure to give a crude product, which was purified by preparative thin-layer chromatography (MeOH:DCM = 1: 10) to give the product (62 mg, yield: 82.1%).
$^1$HNMR (400 MHz, $CD_3OD$) δ(ppm): 8.75 (s, 1H), 7.39 (s, 1H), 7.07 (s, 1H), 5.19-5.06 (s, 1H), 4.77-4.66 (m, 1H), 4.47-4.42 (m, 1H), 3.56-3.52 (m, 1H), 3.40-3.37 (m, 1H), 3.26-3.21 (m, 2H), 2.41 (m,1H), 2.31-2.14 (m, 1H), 1.35-1.33 (m, 6H).
Molecular formula: $C_{17}H_{21}FN_6$ Exact mass: 328.18 LC-MS (Pos, *m/z*) =329.31 $[M+H]^+$.

**Example 43: Synthesis of (*S*)-7-((5,5-difluoropiperidin-3-yl)amino)-1-(isopropylamino)-2,6-naphthyridine-3-carbonitrile (compound 43)**

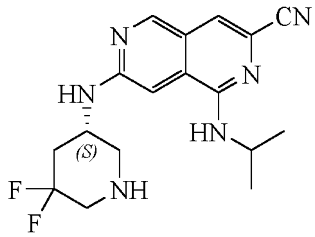

Step 1: synthesis of *tert*-butyl (*S*)-5-((7-cyano-5-(isopropylamino)-2,6-naphthyridin-3-yl)amino)-3,3-difluoropiperidine-1-carboxylate

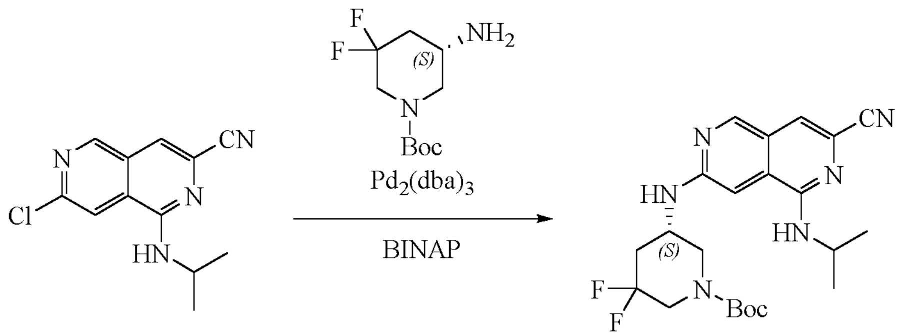


7-chloro-1-(isopropylamino)-2,6-naphthyridine-3-carbonitrile (150 mg, 0.61 mmol, 1.0 eq.), *tert-butyl* (*S*)-5-amino-3,3-difluoropiperidine-1-carboxylate (189 mg, 0.80 mmol, 1.3 eq.), $Pd_2(dba)_3$ (82.4 mg, 0.09 mmol, 0.15 eq.), BINAP (112 mg, 0.18 mmol, 0.3 eq.) and cesium carbonate (498.5 mg, 1.53 mmol, 2.5 eq.) were added into 1,4-dioxane (15 mL). The mixture was reacted at 100 °C for 15 h in a sealed tube in nitrogen atmosphere. When there were no materials left, as detected by TLC, the reaction solution was added with water (30 mL) and extracted with ethyl acetate (30 mL × 2). The organic phases were combined, washed with hydrochloric acid solution (0.5 mol/L, 50 mL), dried over anhydrous sodium sulfate and filtered, and the filtrate was concentrated under reduced pressure to give a crude product, which was purified by silica gel column chromatography (EA:PE = 1:5-1:3) to give the product (164 mg, yield: 60.2%).

Step 2: synthesis of (*S*)-7-((5,5-difluoropiperidin-3-yl)amino)-1-(isopropylamino)-2,6-naphthyridine-3-carbonitrile

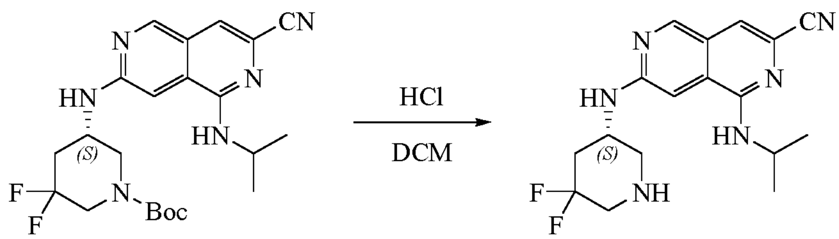


*Tert*-butyl (*S*)-5-((7-cyano-5-(isopropylamino)-2,6-naphthyridin-3-yl)amino)-3,3-difluoropiperidine-1-carboxylate (164 mg, 0.36 mmol, 1.0 eq.) was dissolved in DCM (5 mL). The reaction solution was added dropwise with hydrogen chloride ethanol solution (10 mol/L, 3 mL) and reacted at room temperature for 2 h. When there were no materials left, as detected by TLC, the reaction solution was added with water (20 mL) and back-extracted with DCM (20 mL × 2). The aqueous phase was retained, adjusted to pH of about 8 with potassium carbonate and extracted with DCM (20 mL

× 3). The organic phases were combined, dried over anhydrous sodium sulfate and filtered, and the filtrate was concentrated under reduced pressure to give a crude product, which was purified by preparative thin-layer chromatography (MeOH:DCM = 1:10) to give the product (56.3 mg, yield: 45.1%).

$^1$HNMR (400 MHz, $CD_3OD$) δ(ppm): 8.78 (s, 1H), 7.38 (s, 1H), 6.97 (s, 1H), 4.48-4.42 (m, 1H), 4.24-4.19 (m, 1H), 3.26-3.23 (m, 1H), 3.16-3.11 (m, 1H), 2.98-2.94 (m, 1H), 2.92-2.88 (m, 1H), 2.59-2.50 (m, 2H), 2.10-2.00 (m, 1H), 1.35-1.33 (m, 6H).

Molecular formula: $C_{17}H_{20}F_2N_6$ Exact mass: 346.17 LC-MS (Pos, *m*/*z)* =347.34 $[M+H]^+$.

**Example 44: Synthesis of (*S*)-1-(isopropylamino)-7-(quinuclidin-3-ylamino)-2,6-naphthyridine-3-carbonitrile (compound 44)**

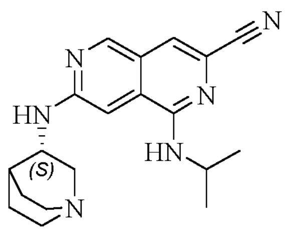

Steps:

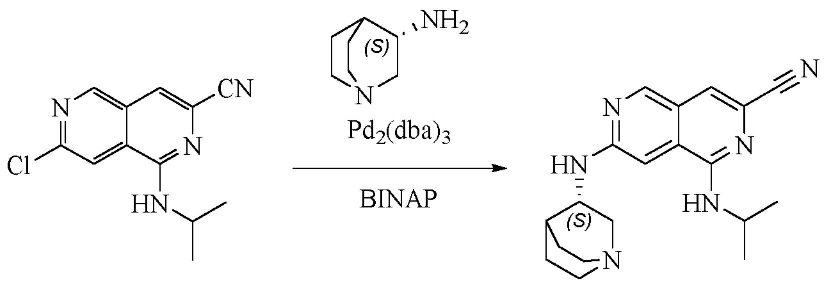


7-chloro-1-(isopropylamino)-2,6-naphthyridine-3-carbonitrile (150 mg, 0.61 mmol, 1.0 eq.), (*S*)-quinuclidin-3-amine (100.9 mg, 0.80 mmol, 1.3 eq.), $Pd_2(dba)_3$ (82.4 mg, 0.09 mmol, 0.15 eq.), BINAP (112 mg, 0.18 mmol, 0.3 eq.) and cesium carbonate (498.5 mg, 1.53 mmol, 2.5 eq.) were added into 1,4-dioxane (15 mL). The mixture was reacted at 100 °C for 22 h in a sealed tube in nitrogen atmosphere. When there were no materials left, as detected by TLC, the reaction solution was added with water (30 mL) and back-extracted with ethyl acetate (30 mL × 3). The aqueous phase was retained, adjusted to pH of about 8 with sodium carbonate and extracted with ethyl acetate (30 mL × 3). The organic phases were combined, dried over anhydrous sodium sulfate and filtered, and the filtrate was concentrated under reduced pressure to give a crude product, which was purified by preparative thin-layer chromatography (MeOH:DCM = 1:10) to give the product (125 mg, yield: 60.9%).

$^1$HNMR (400 MHz, MeOD) δ(ppm): 8.78 (s, 1H), 7.38 (s, 1H), 7.02 (s, 1H), 4.49-4.42 (m, 2H), 3.97-3.91 (m, 1H), 3.47-3.37 (m, 4H), 3.25-3.21 (m, 1H), 2.43-2.41 (m, 1H), 2.35-2.34 (m, 1H), 2.17-2.13 (m, 2H), 1.99-1.95 (m, 1H), 1.35-1.33 (m, 6H).

Molecular formula: $C_{19}H_{24}N_6$ Exact mass: 336.21 LC-MS (Pos, *m*/*z*) =337.39 $[M+H]^+$.

**Example 45: Synthesis of 2-(((1*S*,3*S*)-3-aminocyclopentyl)amino)-8-(isopropylamino)pyrido[3,4-*d*]pyrimidine-6-carbonitrile (compound 45)**

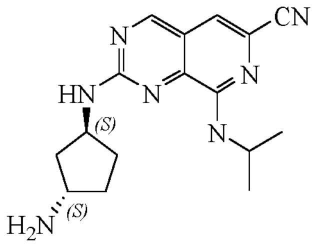

Step 1: synthesis of 8-(isopropylamino)-2-(methylthio)pyrido[3,4-*d*]pyrimidine-6-carbonitrile

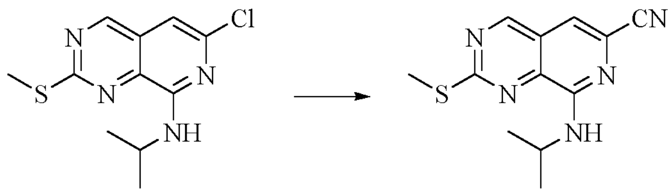

6-chloro-*N*-isopropyl-2-(methylthio)pyrido[3,4-*d*]pyrimidin-8-amine (1.0 g, 3.72 mmol, 1.0 eq.), zinc cyanide (874 mg, 7.44 mmol, 2.0 eq.) and tetrakis(triphenylphosphine)palladium(0) (855 mg, 0.74 mmol, 0.2 eq.) were dissolved in DMAC (10 mL), and the reaction solution was reacted at 120 °C for 1.5 h in nitrogen atmosphere. After the reaction was completed, as detected by TLC, the reaction solution was poured into water (20 mL) and extracted with MTBE (20 mL × 2). The organic phase was washed with water (20 mL), dried and concentrated to give a crude product, which was purified by silica gel column chromatography (PE:EA = 30:1) to give the product (400 mg, yield: 41.1%).

Step 2: synthesis of 8-(isopropylamino)-2-(methylsulfonyl)pyrido[3,4-*d*]pyrimidine-6-carbonitrile

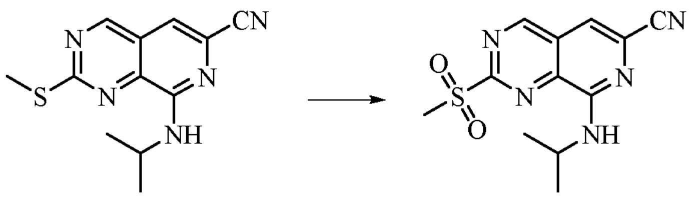

8-(isopropylamino)-2-(methylthio)pyrido[3,4-*d*]pyrimidine-6-carbonitrile (400 mg, 1.54 mmol, 1.0 eq.) was dissolved in DCM (8 mL), and the reaction solution was added with *m*-chloroperoxybenzoic acid (mass fraction: 80%, 664 mg, 3.08 mmol, 2.0 eq.). After the reaction was completed, as detected by TLC, the reaction solution was concentrated under reduced pressure to give a crude product, which was purified by silica gel column chromatography (PE:EA = 2:1) to give the product (360 mg, yield: 80.0%).

Step 3: synthesis of *tert*-butyl ((1*S*,3*S*)-3-((6-cyano-8-(isopropylamino)pyrido[3,4-*d*]pyrimidin-2-yl)amino)cyclopentyl)carbamate

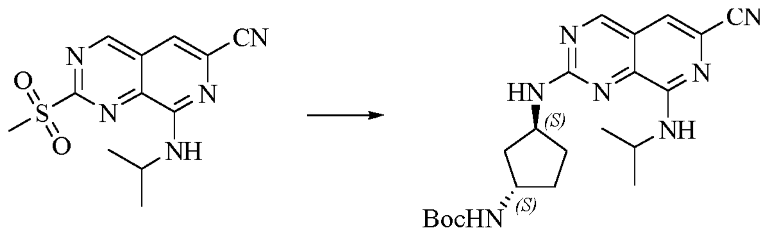

6-chloro-*N*-isopropyl-2-(methylsulfonyl)pyrido[3,4-*d*]pyrimidin-8-amine (360 mg, 1.23 mmol, 1.0 eq.), *Tert*-butyl ((1*S*,3*S*)-3-aminocyclopentyl)carbamate (270 mg, 1.35 mmol, 1.1 eq.) and DIPEA (477 mg, 3.69 mmol, 3.0 eq.) were dissolved in ethanol (5 mL), and the reaction solution was reacted at 85 °C for 2.5 h. After the reaction was completed, as detected by LC-MS, the reaction solution was concentrated to give a crude product, which was purified by silica gel column chromatography (PE:EA= 3:1) to give the product (230 mg, yield: 45.4%).

Step 4: synthesis of 2-(((1*S*,3*S*)-3-aminocyclopentyl)amino)-8-(isopropylamino)pyrido[3,4-*d*]pyrimidine-6-carbonitrile

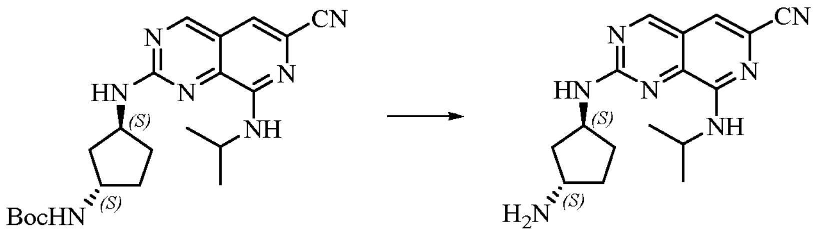

*Tert*-butyl ((1*S*,3*S*)-3-((6-cyano-8-(isopropylamino)pyrido[3,4-*d*]pyrimidin-2-yl)amino)cyclopentyl)carbamate (220 mg, 0.53 mmol, 1.0 eq.) was dissolved in EA (5 mL), and the reaction solution was added dropwise a solution of hydrogen chloride in 1,4-dioxane (4 mol/L, 3 mL) and reacted at room temperature for 4 h. After the reaction was substantially completed, as detected by TLC, the reaction solution was filtered under vacuum. The filter cake was dissolved with (MeOH:DCM = 1:10, 10 mL), washed with saturated aqueous sodium carbonate (10 mL × 2), dried and concentrated to give a crude product, which was slurried with EA (5 mL), and the filter cake was dried to give the product (110 mg, yield: 66.6%).
$^1$HNMR (400 MHz, MeOD) δ(ppm): 8.95 (s, 1H), 7.33 (s, 1H), 4.71-4.68 (t, 1H), 4.37-4.31 (m, 1H), 3.87-3.80 (m, 1H), 2.40-2.33 (m ,2H), 2.20-2.17 (t, 2H), 1.81-1.76 (m, 2H), 1.35-1.34 (d, 6H).
Molecular formula: $C_{16}H_{21}N_7$ Exact mass: 311.19 LC-MS(Pos, *m/z*)=312.14 $[M+H]^+$.

**Example 46: Synthesis of *N*-((1*S*,3*S*)-3-((6-cyano-8-(isopropylamino)pyrido[3,4-*d*]pyrimidin-2-yl)amino)cyclopentyl)acetamide (compound 46)**

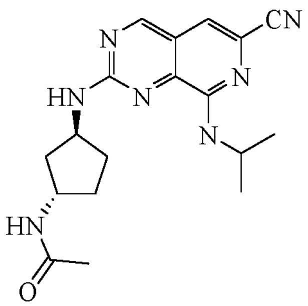

Step 1: synthesis of 2-(((1*S*,3*S*)-3-aminocyclopentyl)amino)-8-(isopropylamino)pyrido[3,4-*d*]pyrimidine-6-carbonitrile

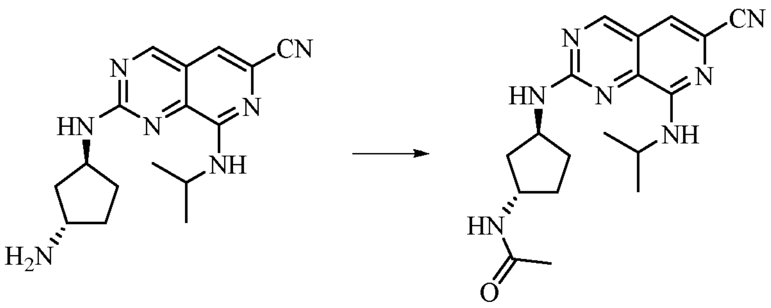

2-(((1*S*,3*S*)-3-aminocyclopentyl)amino)-8-(isopropylamino)pyrido[3,4-*d*]pyrimidine-6-carbonitrile (30 mg, 0.096 mmol, 1.0 eq.), DIPEA (37 mg, 0.29 mmol, 3.0 eq.) and acetic anhydride (15 mg, 0.15 mmol, 1.5 eq.) were dissolved in DCM (2 mL), and the reaction solution was reacted at room temperature for 10 min. After the reaction was completed, as detected by TLC, the reaction solution was concentrated under reduced pressure to give a crude product, which was slurried with MTBE (5 mL) and filtered under vacuum. The filter cake was dried to give the product (24 mg, yield: 70.6%). $^1$HNMR (400 MHz, MeOD) δ(ppm): 8.93 (s, 1H), 7.33 (s, 1H), 4.58-4.54 (t, 1H), 4.36-4.30 (m, 2H), 2.30 (s, 1H), 2.24-2.16 (m , 1H), 2.03-2.01 (d, 2H), 1.96 (s, 3H), 1.70-1.54 (m, 2H), 1.35-1.33 (d, 6H).
Molecular formula: $C_{18}H_{23}N_7O$ Exact mass: 353.20 LC-MS(Pos, *m*/*z*)=354.16[M+H]$^+$.

**Example 47: Synthesis of *N*-((1*R*,3*S*)-3-((7-cyano-5-(isopropylamino)-2,6-naphthyridin-3-yl)amino)cyclopentyl)acetamide (compound 47)**

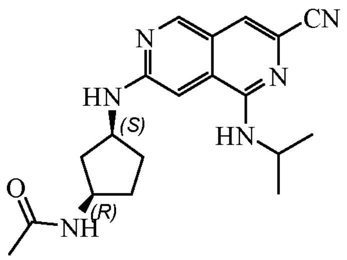

Steps:

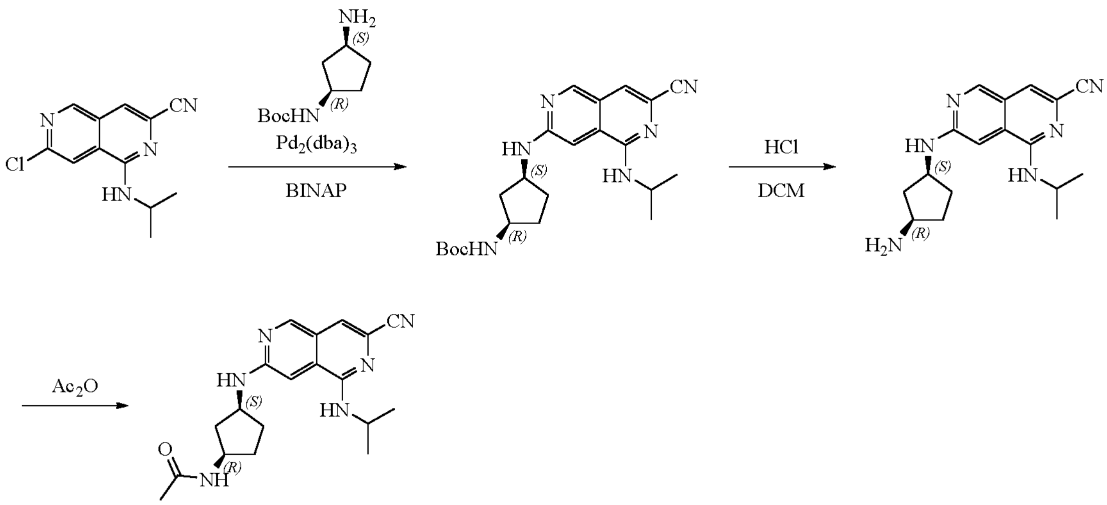

Step 1: synthesis of *tert*-butyl ((1*R*,3*S*)-3-((7-cyano-5-(isopropylamino)-2,6-naphthyridin-3-yl)amino)cyclopentyl)carbamate

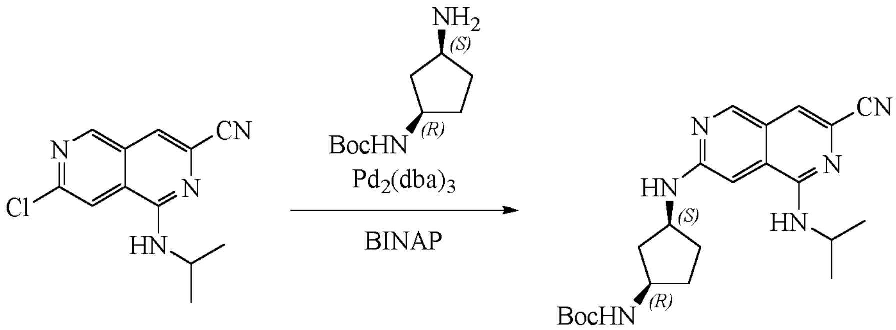


7-chloro-1-(isopropylamino)-2,6-naphthyridine-3-carbonitrile (246.7 mg, 1 mmol, 1.0 eq.), *tert*-butyl ((1*R*,3*S*)-3-aminocyclopentyl)carbamate (240.3 mg, 1.2 mmol, 1.2 eq.), $Pd_2(dba)_3$ (137.4 mg, 0.15 mmol, 0.15 eq.), BINAP (186.8 mg, 0.3 mmol, 0.3 eq.) and cesium carbonate (814.6 mg, 2.5 mmol, 2.5 eq.) were added into 1,4-dioxane (10 mL). The mixture was reacted at 120 °C for 14 h in a sealed tube in nitrogen atmosphere. When there were no materials left, as detected by TLC, the reaction solution was added with water (20 mL) and extracted with ethyl acetate (20 mL × 3). The organic phases were combined, washed with hydrochloric acid solution (0.2 mol/L, 30 mL), dried over anhydrous sodium sulfate and filtered, and the filtrate was concentrated under reduced pressure to give a crude product, which was purified by silica gel column chromatography (EA:PE = 1:10-1:2) to give the product (196 mg, yield: 47.7%).

Step 2: synthesis of 7-(((1*S*,3*R*)-3-aminocyclopentyl)amino)-1-(isopropylamino)-2,6-naphthyridine-3-carbonitrile

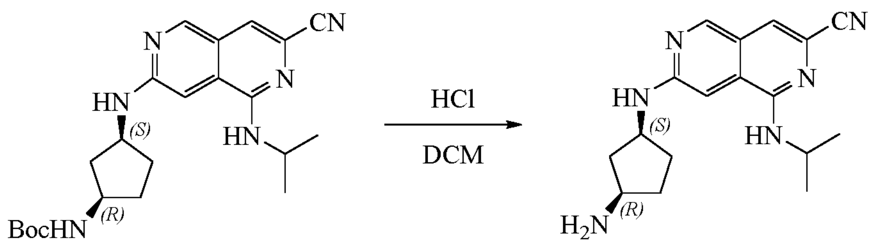


*Tert*-butyl ((1*R*,3*S*)-3-((7-cyano-5-(isopropylamino)-2,6-naphthyridin-3-yl)amino)cyclopentyl)carbamate (196 mg, 0.48 mmol, 1.0 eq.) was dissolved in DCM (5 mL). The reaction solution was added dropwise with hydrogen chloride ethanol solution (10 mol/L, 4 mL) and reacted at room temperature for 2 h. When there were no materials left, as detected by TLC, the reaction solution was added with water (20 mL), adjusted to pH of about 8 with sodium bicarbonate and extracted with DCM (20 mL × 3). The organic phases were combined, dried over anhydrous sodium sulfate and filtered, and the filtrate was concentrated under reduced pressure to give the product (134 mg, yield: 89.9%).

Step 3: synthesis of *N*-((1*R*,3*S*)-3-((7-cyano-5-(isopropylamino)-2,6-naphthyridin-3-yl)amino)cyclopentyl)acetamide

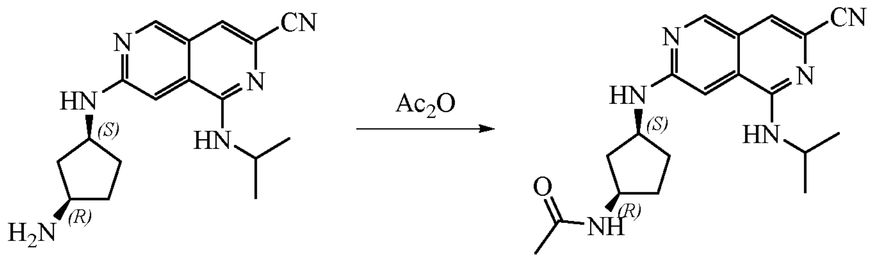


7-(((1*S*,3*R*)-3-aminocyclopentyl)amino)-1-(isopropylamino)-2,6-naphthyridine-3-carbonitrile (134 mg, 0.43 mmol, 1.0 eq.) was dissolved in DCM (4 mL). The reaction solution was added with DIPEA (167.9 mg, 1.30 mmol, 3.0 eq.) and acetic anhydride (65.8 mg, 0.65 mmol, 1.5 eq.) and reacted at room temperature for 1 h. When there were no materials left, as detected by TLC, the reaction solution was added with water (20 mL) and extracted with ethyl acetate (20 mL × 3). The organic phases were combined, dried over anhydrous sodium sulfate and filtered. The filtrate was concentrated under reduced pressure to give a crude product, which was purified by preparative thin-layer chromatography (MeOH:DCM = 1:20) to give the product (45 mg, yield: 29.7%).

$^1$HNMR (400 MHz, $CD_3OD$) δ(ppm): 8.66 (s, 1H), 7.36 (s, 1H), 6.93 (s, 1H), 4.50-4.43 (m, 1H), 4.23-4.16 (m, 2H), 2.65-2.58 (m, 1H), 2.23-2.13 (m, 1H), 2.11-2.04 (m, 1H), 1.95 (s, 3H), 1.76-1.71 (m, 2H), 1.49-1.42 (m, 1H), 1.35-1.33 (m, 6H).

Molecular formula: $C_{19}H_{24}N_6O$ Exact mass: 352.20 LC-MS (Pos, *m/z)* =353.31 $[M+H]^+$.

**Example 48: Synthesis of 7-(((1*S*,3*S*)-3-aminocyclopentyl)amino)-1-(pentan-3-ylamino)-2,6-naphthyridine-3-carbonitrile (compound 48)**

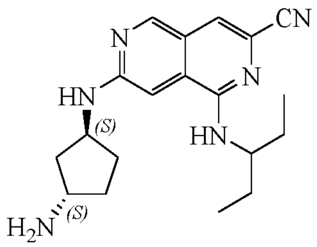

Route:

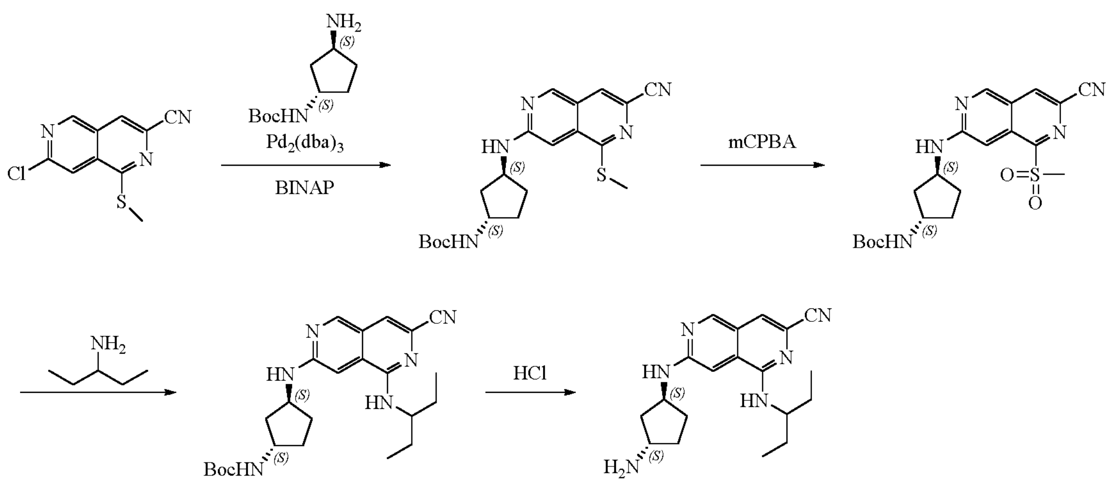

Step 1: synthesis of *tert*-butyl ((1*S*,3*S*)-3-((7-cyano-5-(methylthio)-2,6-naphthyridin-3-yl)amino)cyclopentyl)carbamate:

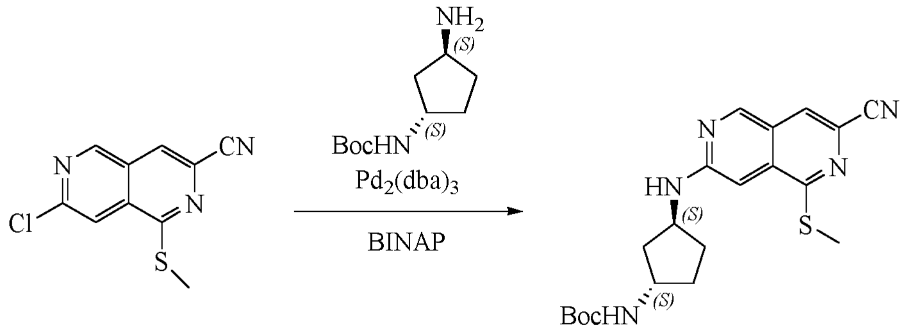


7-chloro-1-(methylthio)-2,6-naphthyridine-3-carbonitrile (500 mg, 2.12 mmol, 1.0 eq.), *tert*-butyl ((1S,3S)-3-aminocyclopentyl)carbamate (508.7 mg, 2.54 mmol, 1.0 eq.), $Pd_2(dba)_3$ (293 mg, 0.32 mmol, 0.15 eq.), BINAP (398.5 mg, 0.64 mmol, 0.3 eq.) and cesium carbonate (1.7 g, 5.30 mmol, 2.5 eq.) were added into 1,4-dioxane (25 mL). The mixture was reacted at 120 °C for 16 h in a sealed tube in nitrogen atmosphere. When there were no materials left, as detected by TLC, the reaction solution was added with water (30 mL) and extracted with ethyl acetate (30 mL × 3). The organic phases were combined, dried over anhydrous sodium sulfate and filtered, and the filtrate was concentrated under reduced pressure to give a crude product, which was purified by silica gel column chromatography (EA:PE = 1:10-1:3) to give the product (385 mg, yield: 45.4%).

Step 2: synthesis of *tert*-butyl ((1*S*,3*S*)-3-((7-cyano-5-(methylsulfonyl)-2,6-naphthyridin-3-yl)amino)cyclopentyl)carbamate

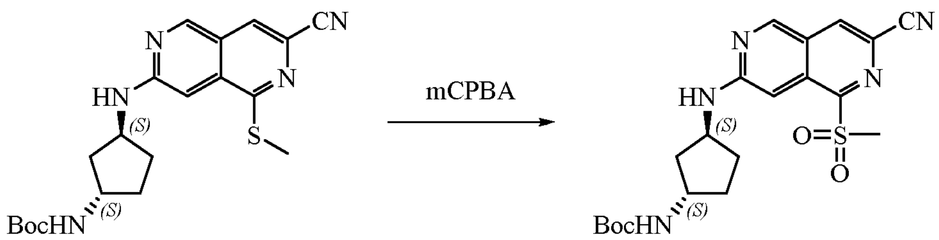


*Tert*-butyl ((1*S*,3*S*)-3-((7-cyano-5-(methylthio)-2,6-naphthyridin-3-yl)amino)cyclopentyl)carbamate (150 mg, 0.37 mmol, 1.0 eq.) was dissolved in DCM (5 mL). The reaction solution was added with mCPBA (mass fraction: 80%, 162 mg, 0.75 mmol, 2.0 eq.) and reacted at room temperature for 0.5 h. When there were no materials left, as detected by TLC, the reaction solution was added with water (20 mL) and extracted with ethyl acetate (20 mL × 3). The organic phases were combined, dried over anhydrous sodium sulfate and filtered, and the filtrate was concentrated under reduced pressure to give a crude product, which was purified by preparative thin-layer chromatography (EA:PE = 1:2) to give the product (100 mg, yield: 61.8%).

Step 3: synthesis of *tert*-butyl ((1*S*,3*S*)-3-((7-cyano-5-(pentan-3-ylamino)-2,6-naphthyridin-3-yl)amino)cyclopentyl)carbamate

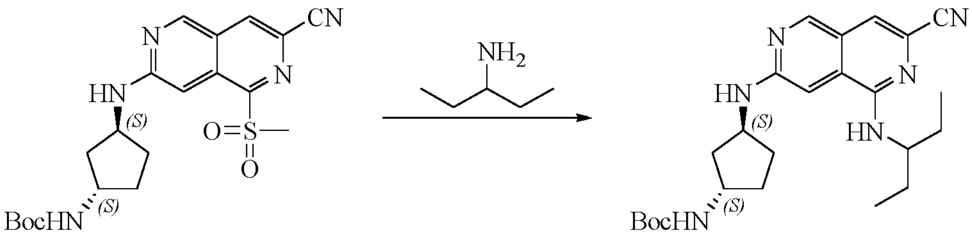

*Tert*-butyl ((1*S*,3*S*)-3-((7-cyano-5-(methylsulfonyl)-2,6-naphthyridin-3-yl)amino)cyclopentyl)carbamate (100 mg, 0.23 mmol, 1.0 eq.) was dissolved in 3-pentylamine (2 mL). The reaction solution was reacted at 120 °C for 21 h. When there were no materials left, as detected by TLC, the reaction solution was concentrated under reduced pressure, added with water (20 mL) and extracted with ethyl acetate (20 mL × 3). The organic phases were combined, dried over anhydrous sodium sulfate and filtered, and the filtrate was concentrated under reduced pressure to give a crude product, which was purified by preparative thin-layer chromatography (EA:PE = 1:2) to give the product (30 mg, yield: 29.7%).

Step 4: synthesis of 7-(((1*S*,3*S*)-3-aminocyclopentyl)amino)-1-(pentan-3-ylamino)-2,6-naphthyridine-3-carbonitrile

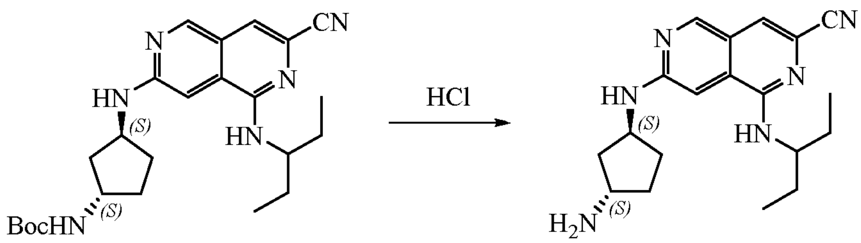


*Tert*-butyl ((1*S*,3*S*)-3-((7-cyano-5-(pentan-3-ylamino)-2,6-naphthyridin-3-yl)amino)cyclopentyl)carbamate (30 mg, 0.06 mmol, 1.0 eq.) was dissolved in DCM (2 mL). The reaction solution was added dropwise with hydrogen chloride ethanol solution (10 mol/L, 1 mL) and reacted at room temperature for 2 h. When there were no materials left, as detected by TCL, the reaction solution was added with water (10 mL), adjusted to pH of about 8 with sodium carbonate and extracted with DCM (10 mL × 3). The organic phases were combined, dried over anhydrous sodium sulfate and filtered, and the filtrate was concentrated under reduced pressure to give a crude product, which was dissolved with methanol (0.5 mL) and water (3 mL), and lyophilized to give the product (20 mg, yield: 98.5%).
$^1$HNMR (400 MHz, $CD_3OD$) δ(ppm): 8.65 (s, 1H), 7.34 (s, 1H), 6.93 (s, 1H), 4.37-4.33 (m, 1H), 4.24 (m, 1H), 3.56-3.53 (m, 1H), 2.40-2.31 (m, 1H), 2.21-2.13 (m, 1H), 1.94-1.90 (m, 2H), 1.78-1.64 (m, 5H), 1.50-1.45 (m, 1H), 1.00-0.96 (m, 6H). Molecular formula: $C_{19}H_{26}N_6$ Exact mass: 338.22 LC-MS (Pos, *m/z)* =339.34[M+H]$^+$.

**Example 49: Synthesis of 7-(((1*S*,3S)-3-aminocyclopentyl)amino)-1-(cyclopentylamino)-2,6-naphthyridine-3-carbonitrile (compound 49)**

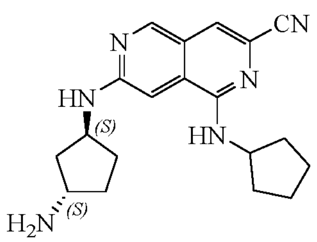

Step 1: synthesis of 7-chloro-1-(methylsulfonyl)-2,6-naphthyridine-3-carbonitrile

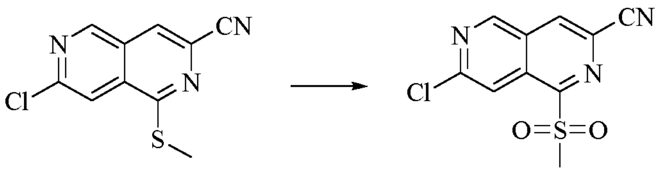

7-chloro-1-(methylthio)-2,6-naphthyridine-3-carbonitrile (1 g, 4.24 mmol, 1.0 eq.) was dissolved in DCM (20 mL), and the reaction solution was added with *m*-chloroperoxybenzoic acid (mass fraction: 80%, 1.83 g, 8.48 mmol, 2.0 eq.) and reacted at room temperature for 0.5 h. After the reaction was completed, as detected by TLC, the reaction solution was poured into water (10 mL) and adjusted to pH of 8 with sodium carbonate. The liquid separation was performed. The aqueous phase was extracted with DCM (20 mL). The organic phases were combined, dried and concentrated to give a crude product, which was slurried with (PE:EA = 10:1, 20 mL) and filtered under vacuum, and the filter cake was dried to give the product (1.1 g, yield: 97.3%).

Step 2: synthesis of 7-chloro-1-(cyclopentylamino)-2,6-naphthyridine-3-carbonitrile

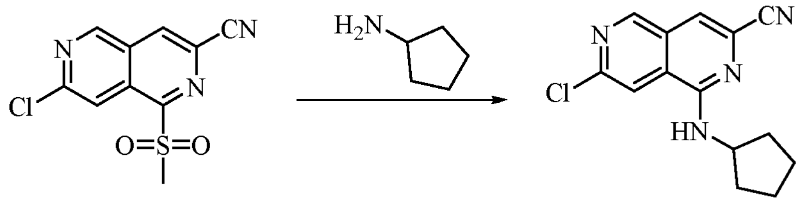

7-chloro-1-(methylsulfonyl)-2,6-naphthyridine-3-carbonitrile (200 mg, 0.75 mmol, 1.0 eq.), cyclopentylamine (128 mg, 1.50 mmol, 2.0 eq.) and DIPEA (290 mg, 2.25 mmol, 3.0 eq.) were dissolved in THF (5 mL), and the reaction solution was reacted at 40 °C for 30 min. After the reaction was completed, as detected by TLC, the reaction solution was poured into water (10 mL) and extracted with EA (10 mL × 2). The organic phase was dried and concentrated to give the product (200 mg, yield: 98%).

Step 3: synthesis of *tert*-butyl ((1*S*,3*S*)-3-((7-cyano-5-(cyclopentylamino)-2,6-naphthyridin-3-yl)amino)cyclopentyl)carbamate

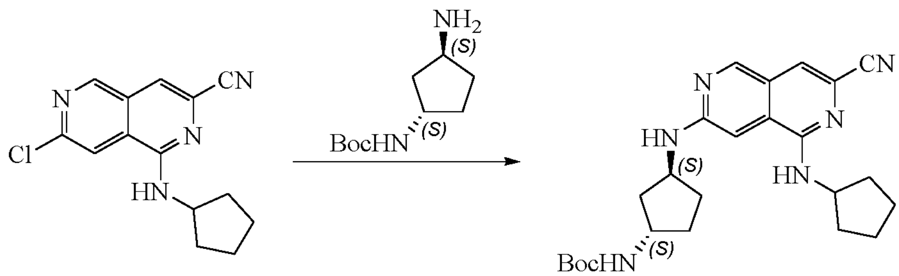

7-chloro-1-(cyclopentylamino)-2,6-naphthyridine-3-carbonitrile (200 mg, 0.73 mmol, 1.0 eq.), *tert*-butyl ((1*S*,3*S*)-3-aminocyclopentyl)carbamate (176 mg, 0.88 mmol, 1.2 eq.), $Pd_2(dba)_3$ (67 mg, 0.073 mmol, 0.1 eq.), BINAP (91 mg, 0.146 mmol, 0.2 eq.) and cesium carbonate (476 mg, 1.46 mmol, 2.0 eq.) were dispersed in 1,4-dioxane (5 mL), and the reaction solution was reacted at 100 °C for 19.5 h in nitrogen atmosphere. When there were no materials left, as detected by TLC, the reaction solution was concentrated under reduced pressure to give a crude product, which was purified by silica gel column chromatography (PE:EA = 2:1) to give the product (160 mg, yield: 50.3%).

Step 4: synthesis of 7-(((1*S*,3*S*)-3-aminocyclopentyl)amino)-1-(cyclopentylamino)-2,6-naphthyridine-3-carbonitrile

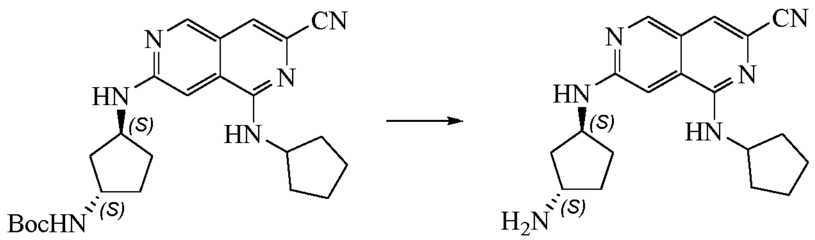

*Tert*-butyl ((1*S*,3*S*)-3-((7-cyano-5-(cyclopentylamino)-2,6-naphthyridin-3-yl)amino)cyclopentyl)carbamate (160 mg, 0.36 mmol, 1.0 eq.) was dissolved in EA (4 mL), and the reaction solution was added dropwise a solution of hydrogen chloride in 1,4-dioxane (4 mol/L, 2 mL) and reacted at room temperature for 3.5 h. After the reaction was substantially completed, as detected by LC-MS, the reaction solution was filtered, and the filter cake was dissolved with water (5 mL) and adjusted to pH of 9 with sodium carbonate. The aqueous phase was extracted with (MeOH:DCM = 1:10, 10 mL × 4). The organic phase was dried and concentrated to give a crude product, which was purified by preparative thin-layer chromatography (MeOH:DCM = 1:5) to give the product (13 mg, yield: 10.7%).

$^1$HNMR (400 MHz, MeOD) δ(ppm): 8.69 (s, 1H), 7.38 (s, 1H), 6.94 (s, 1H), 4.58-4.49 (m, 2H), 3.83-3.79 (t, 1H), 2.38-2.35 (m, 2H), 2.19-2.12 (m, 4H), 1.84-1.82 (t, 2H), 1.78-1.68 (m, 6H).

Molecular formula: $C_{19}H_{24}N_6$ Exact mass: 336.21 LC-MS(Pos, *m*/*z*)=337.38[M+H]$^+$.

**Example 50: Synthesis of 7-(((1*S*,3*S*)-3-aminocyclopentyl)amino)-1-((1-methylcyclopropyl)amino)-2,6-naphthyridine-3-carbonitrile (compound 50)**

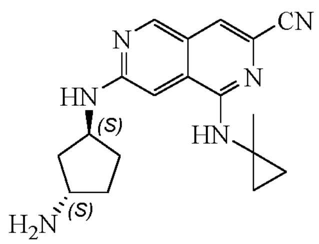

Step 1: synthesis of 7-chloro-1-((1-methylcyclopropyl)amino)-2,6-naphthyridine-3-carbonitrile

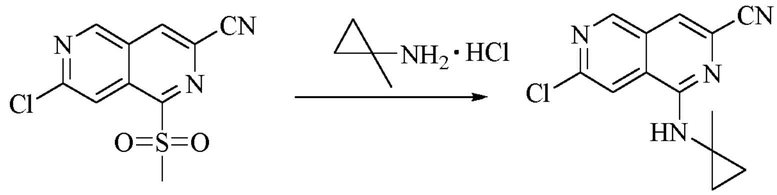

7-chloro-1-(methylsulfonyl)-2,6-naphthyridine-3-carbonitrile (200 mg, 0.75 mmol, 1.0 eq.), 1-methylcyclopropan-1-amine hydrochloride (161 mg, 1.50 mmol, 2.0 eq.) and DIPEA (290 mg, 2.25 mmol, 3.0 eq.) were dissolved in THF (5 mL), and the reaction solution was reacted at 80 °C for 3 h. After the reaction was completed, as detected by TLC, the reaction solution was poured into water (10 mL) and extracted with EA (10 mL × 2). The organic phase was dried and concentrated to give the product (160 mg, yield: 82.5%).

Step 2: synthesis of *tert*-butyl ((1*S*,3*S*)-3-((7-cyano-5-((1-methylcyclopropyl)amino)-naphthyridin-3-yl)amino)cyclopentyl)carbamate

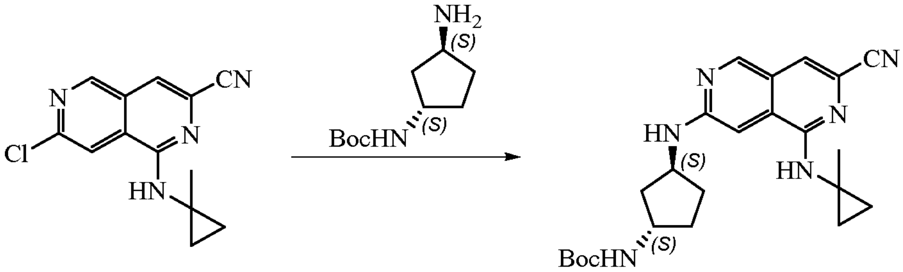

7-chloro-1-((1-methylcyclopropyl)amino)-2,6-naphthyridine-3-carbonitrile (160 mg, 0.62 mmol, 1.0 eq.), *tert*-butyl ((1*S*,3*S*)-3-aminocyclopentyl)carbamate (149 mg, 0.74 mmol, 1.2 eq.), $Pd_2(dba)_3$ (57 mg, 0.062 mmol, 0.1 eq.), BINAP (77 mg, 0.124 mmol, 0.2 eq.) and cesium carbonate (404 mg, 1.24 mmol, 2.0 eq.) were dispersed in 1,4-dioxane (5 mL), and the reaction solution was reacted at 100 °C for 23 h in nitrogen atmosphere. After the reaction was completed, as detected by TLC, the reaction solution was concentrated under reduced pressure to give a crude product, which was purified by silica gel column chromatography (PE:EA = 2:1) to give the product (70 mg, yield: 26.7%).

Step 3: synthesis of 7-(((1*S*,3*S*)-3-aminocyclopentyl)amino)-1-((1-methylcyclopropyl)amino)-2,6-naphthyridine-3-carbonitrile

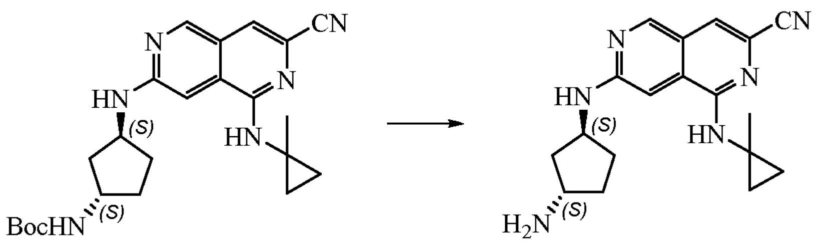

*Tert*-butyl ((1*S*,3*S*)-3-((7-cyano-5-((1-methylcyclopropyl)amino)-naphthyridin-3-yl)amino)cyclopentyl)carbamate (70 mg, 0.16 mmol, 1.0 eq.) was dissolved in EA (2 mL), and the reaction solution was added dropwise a solution of hydrogen chloride in 1,4-dioxane (4 mol/L, 1 mL) and reacted at room temperature for 18 h. After the reaction was completed, as detected by LC-MS, the reaction solution was poured into water (5 mL), and the liquid separation was performed. The aqueous phase was retained, adjusted to pH of 9 with sodium carbonate, and extracted with (MeOH:DCM = 1:10, 10 mL × 4). The organic phase was dried and concentrated to give a crude product, which was purified by preparative thin-layer chromatography (MeOH:DCM = 1:5) to give the product (20 mg, yield: 38.4%).
$^1$HNMR (400 MHz, MeOD) δ(ppm): 8.69 (s, 1H), 7.42 (s, 1H), 6.82 (s, 1H), 4.57-4.54 (t, 1H), 3.80-3.77 (t, 1H), 2.37-2.33 (m, 2H), 2.13-2.10 (t, 2H), 1.76-1.68 (m, 2H), 1.58 (s, 3H), 0.87 (s, 2H), 0.78 (s, 2H).
Molecular formula: $C_{18}H_{22}N_6$ Exact mass: 322.19 LC-MS(Pos, *m*/*z*)=323.24[M+H]$^+$.

**Example 51: Synthesis of 7-(((1*S*,3*S*)-3-aminocyclopentyl)amino)-1-isopropoxy-2,6-naphthyridine-3-carbonitrile (compound 51)**

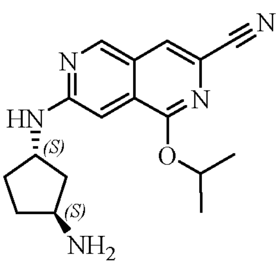

Steps:

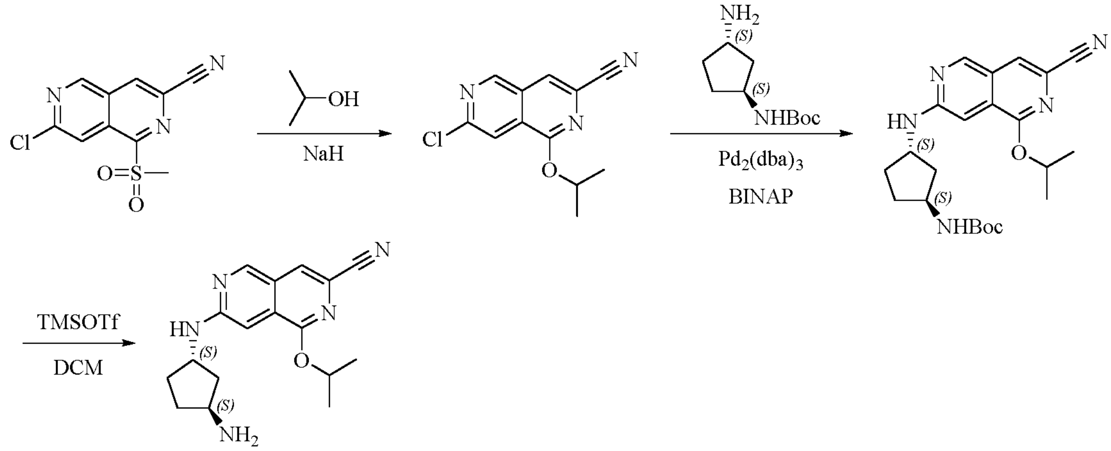


Step 1: synthesis of 7-chloro-1-isopropoxy-2,6-naphthyridine-3-carbonitrile

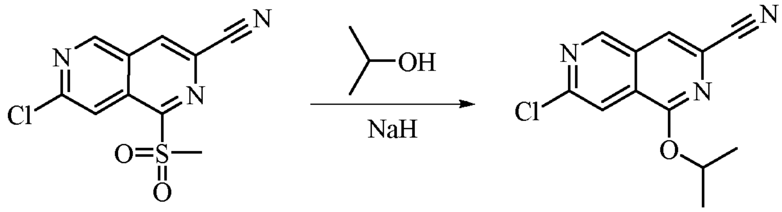


Sodium hydride (mass fraction: 60%, 52.8 mg, 1.32 mmol, 1.1 eq.) was dissolved in isopropanol (10 mL), and the reaction solution was reacted at room temperature for 0.5 h, added with a solution of 7-chloro-1-(methylsulfonyl)-2,6-naphthyridine-3-carbonitrile (300 mg, 1.12 mmol, 1.0 eq.) in 1,4-dioxane (5 mL) and heated to 60 °C for 1 h. When there were no materials left, as detected by TLC, the reaction solution was added with water (20 mL) and extracted with ethyl acetate (20 mL × 3). The organic phases were combined, dried over anhydrous magnesium sulfate and filtered, and the filtrate was concentrated under reduced pressure to give the product (274 mg, yield: 98.7%).

Step 2: synthesis of *tert*-butyl ((1*S*,3*S*)-3-((7-cyano-5-isoropoxy-2,6-naphthyridin-3-yl)amino)cyclopentyl)carbamate

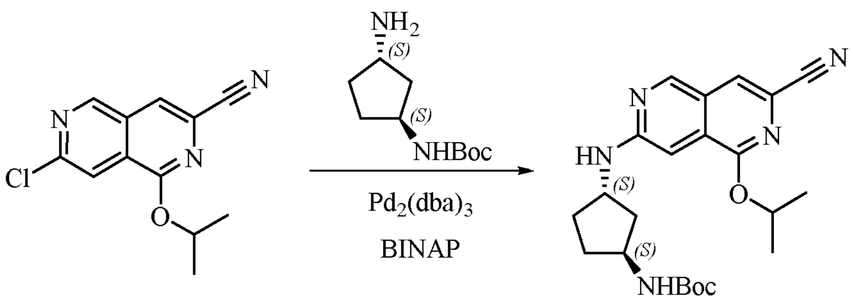


7-chloro-1-isopropoxy-2,6-naphthyridine-3-carbonitrile (270 mg, 1.09 mmol, 1.0 eq.), *tert*-butyl ((1*S*,3*S*)-3-aminocyclopentyl)carbamate (284.4 mg, 1.42 mmol, 1.2 eq.), Pd$_2$(dba)$_3$ (146.5 mg, 0.16 mmol, 0.15 eq.), BINAP (199.3 mg, 0.32 mmol, 0.3 eq.) and cesium carbonate (889.5 mg, 2.73 mmol, 2.5 eq.) were added into 1,4-dioxane (20 mL). The mixture was reacted at 120 °C for 17 h in a sealed tube in nitrogen atmosphere. When there were no materials left,

as detected by TLC, the reaction solution was added with water (30 mL) and extracted with ethyl acetate (30 mL × 3). The organic phases were combined, washed with hydrochloric acid solution (0.2 mol/L, 40 mL), dried over anhydrous sodium sulfate and filtered, and the filtrate was concentrated under reduced pressure to give a crude product, which was purified by preparative thin-layer chromatography (EA:PE = 1:3) to give the product (105 mg, yield: 23.4%).

Step 3: synthesis of 7-(((1*S*,3*S*)-3-aminocyclopentyl)amino)-1-isopropoxy-2,6-naphthyridine-3-carbonitrile

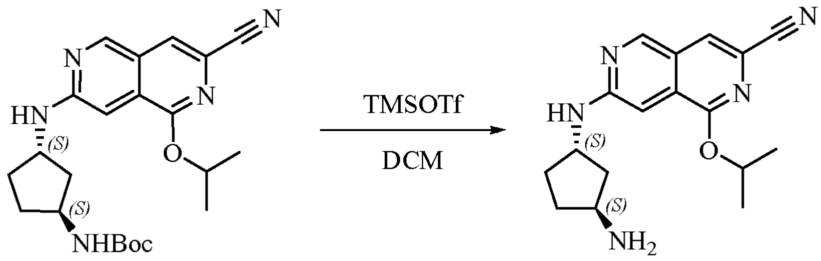


*Tert*-butyl ((1*S*,3*S*)-3-((7-cyano-5-isopropoxy-2,6-naphthyridin-3-yl)amino)cyclopentyl)carbamate (105 mg, 0.25 mmol, 1.0 eq.) was dissolved in DCM (5 mL), and the reaction solution was cooled in an ice water bath, added with 2,6-lutidine (401.8 mg, 3.75 mmol, 15.0 eq.) and trimethylsilyl trifluoromethanesulfonate (555.6 mg, 2.5 mmol, 10.0 eq.), and reacted at 0 °C to 5 °C for 2 h. When there were no materials left, as detected by TLC, the reaction solution was added with water (30 mL) and extracted with ethyl acetate (30 mL × 3). The organic phases were combined, washed with saturated aqueous sodium carbonate (30 mL) and water (30 mL) successively, dried over anhydrous sodium sulfate and filtered, and the filtrate was concentrated under reduced pressure to give a crude product, which was purified by preparative thin-layer chromatography (MeOH:DCM = 1:10) to give the product (58 mg, yield: 64.3 %).
$^1$HNMR (400 MHz, $CD_3OD$) δ(ppm): 8.86 (s, 1H), 7.76 (s, 1H), 6.91 (s, 1H), 5.54-5.48 (m, 1H), 4.40-4.37 (m, 1H), 3.57-3.54 (m, 1H), 2.35-2.83 (m, 1H), 2.20-2.14 (m, 1H), 1.96-1.92 (m, 2H), 1.65-1.61 (m, 1H), 1.59-1.50 (m, 1H), 1.47-1.45 (m, 6H).
Molecular formula: $C_{17}H_{21}N_5O$ Exact mass: 311.17 LC-MS (Pos, *m/z)* =312.27 $[M+H]^+$.

**Example 52: Synthesis of *N*$^7$-((1*S*,3*S*)-3-aminocyclopentyl)-3-(difluoromethyl)-*N*$^1$-isopropyl-2,6-naphthyridine-1,7-diamine (compound 52)**

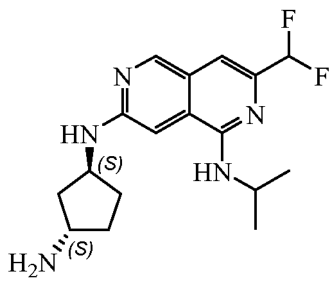

Steps:

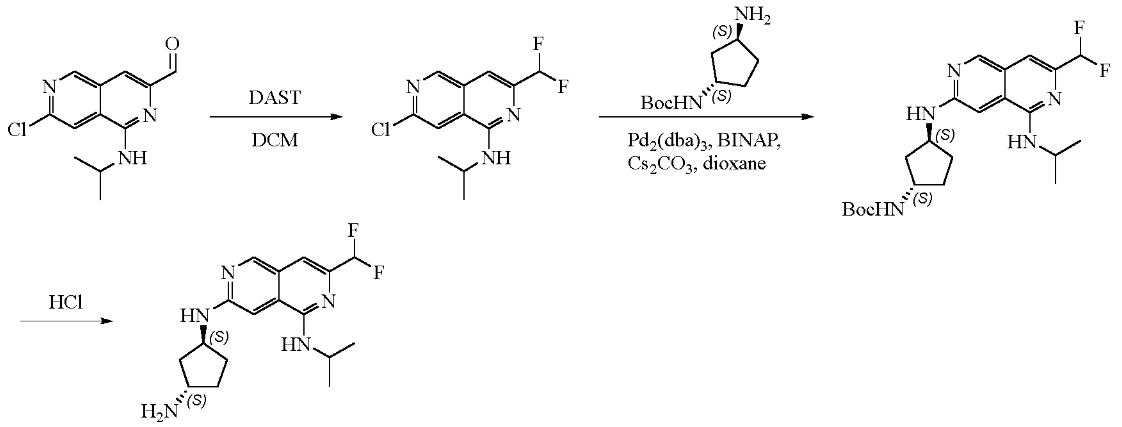


Step 1: synthesis of 7-chloro-3-(difluoromethyl)-*N*-isopropyl-2,6-naphthyridin-1-amine

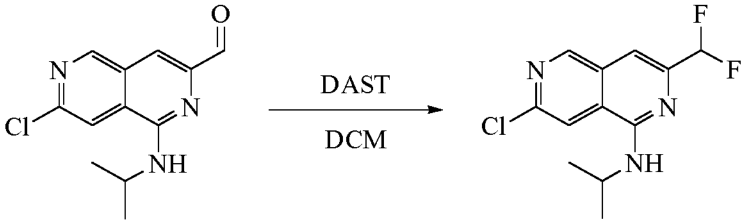


7-chloro-1-(isopropylamino)-2,6-naphthyridine-3-carbaldehyde (1 g, 4.0 mmol, 1.0 eq.) was dissolved in DCM (20 mL), and the reaction solution was cooled to 0 °C to 5 °C in an ice water bath, added dropwise with DAST (2.6 g, 16 mmol, 4.0 eq.), and reacted at 0 °C to 5 °C for 2 h after addition, and incubated at room temperature for 16 h. When there were no materials left, as detected by TLC, the reaction solution was added with water (20 mL) and adjusted to pH of about 8 with sodium carbonate. The liquid separation was performed. The organic phase was retained. The aqueous phase was extracted with DCM (20 mL × 2). The organic phases were combined, washed with water (20 mL) and brine (20 mL) successively, dried over anhydrous sodium sulfate and filtered, and the filtrate was concentrated under reduced pressure to give a crude product, which was purified by silica gel column chromatography (EA:PE = 1:30) to give the product (982 mg, yield: 90.9%).

Step 2: synthesis of *tert*-butyl ((1*S*,3*S*)-3-((7-(difluoromethyl)-5-(isopropylamino)-2,6-naphthyridin-3-yl)amino)cyclopentyl)carbamate

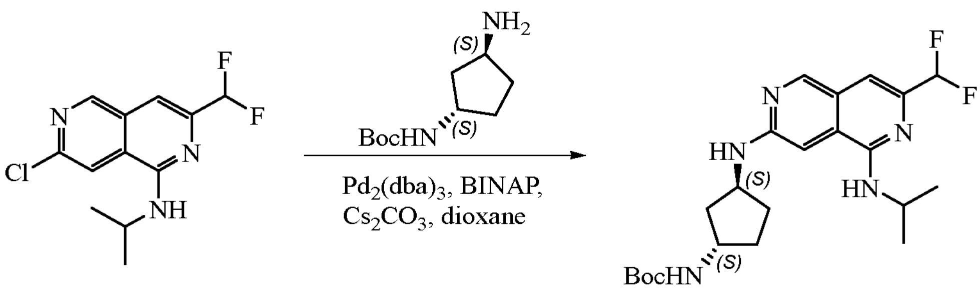


7-chloro-3-(difluoromethyl)-*N*-isopropyl-2,6-naphthyridin-1-amine (166 mg, 0.61 mmol, 1.0 eq.), *tert*-butyl ((1*S*,3*S*)-3-aminocyclopentyl)carbamate (146.2 mg, 0.73 mmol, 1.2 eq.), Pd$_2$(dba)$_3$ (82.4 mg, 0.09 mmol, 0.15 eq.), BINAP (112 mg, 0.18 mmol, 0.3 eq.) and cesium carbonate (498.5 mg, 1.53 mmol, 2.5 eq.) were added into 1,4-dioxane (10 mL). The mixture was reacted at 120 °C for 17 h in a sealed tube in nitrogen atmosphere. When there were no

materials left, as detected by TLC, the reaction solution was added with water (10 mL) and extracted with ethyl acetate (10 mL × 3). The organic phases were combined, washed with hydrochloric acid solution (0.2 mol/L, 30 mL), dried over anhydrous sodium sulfate and filtered, and the filtrate was concentrated under reduced pressure to give a crude product, which was firstly purified by silica gel column chromatography (EA:PE = 1:10-1:2) and then purified by preparative thin-layer chromatography (EA:PE = 1:2) to give the product (93 mg, yield: 35%).

Step 3: synthesis of *N*[7]-((1*S*,3*S*)-3-aminocyclopentyl)-3-(difluoromethyl)-*N*[1]-isopropyl-2,6-naphthyridine-1,7-diamine

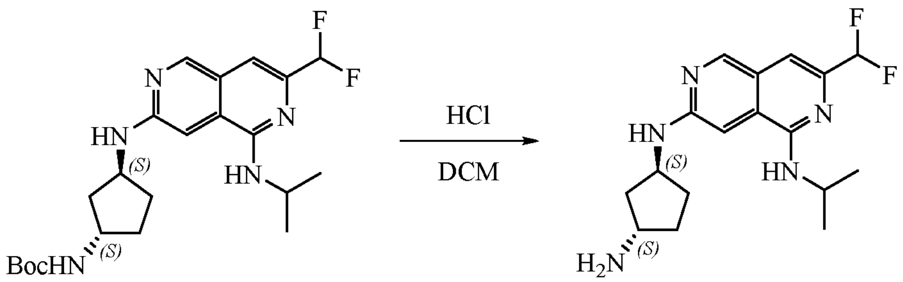


Tert-butyl ((1*S*,3*S*)-3-((7-(difluoromethyl)-5-(isopropylamino)-2,6-naphthyridin-3-yl)amino)cyclopentyl)carbamate (93 mg, 0.21 mmol, 1.0 eq.) was dissolved in DCM (3 mL). The reaction solution was added dropwise with hydrogen chloride ethanol solution (10 mol/L, 3 mL) and reacted at room temperature for 2 h. When there were no materials left, as detected by LC-MS, the reaction solution was added with water (15 mL) and back-extracted with DCM (15 mL × 2). The aqueous phase was retained, adjusted to pH of about 8 with sodium carbonate and extracted with dichloromethane (15 mL × 2). The organic phases were combined, dried over anhydrous magnesium sulfate and filtered, and the filtrate was concentrated under reduced pressure. The residue was dissolved with methanol (1 mL) and water (5 mL), and lyophilized to give the product (45 mg, yield: 63.9%).

$^1$HNMR (400 MHz, MeOD) δ(ppm): 8.66 (s, 1H), 7.07 (s, 1H), 6.90 (s, 1H), 6.65-6.37 (s, 1H), 4.54-4.48 (m, 1H), 4.35-4.31 (m, 1H), 3.59-3.56 (m, 1H), 2.39-2.32 (m, 1H), 2.21-2.16 (m, 1H), 1.96-1.93 (m, 2H), 1.64-1.48 (m, 2H), 1.35-1.33 (m, 6H). Molecular formula: $C_{17}H_{23}F_2N_5$ Exact mass: 335.19 LC-MS (Pos, *m*/*z*) =336.20 $[M+H]^+$.

**Example 53: Synthesis of *N*[7]-((1*S*,3*R*)-3-aminocyclopentyl)-3-(difluoromethyl)-*N*[1]-isopropyl-2,6-naphthyridine-1,7-diamine (compound 53)**

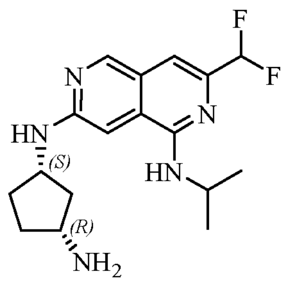

Step 1: synthesis of *tert*-butyl ((1*R*,3*S*)-3-((7-(difluoromethyl)-5-(isopropylamino)-2,6-naphthyridin-3-yl)amino)cyclopentyl)carbamate

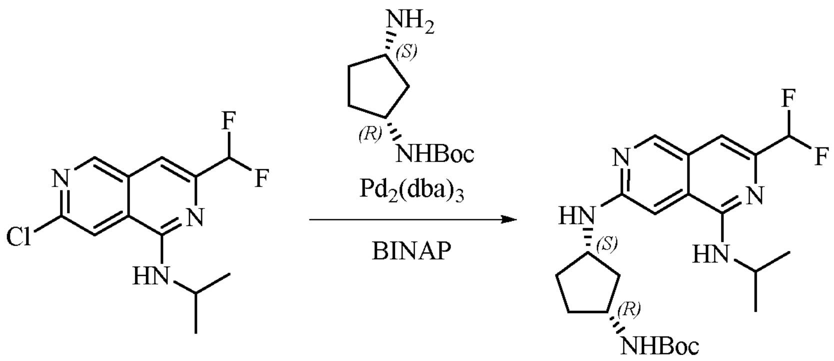


7-chloro-3-(difluoromethyl)-*N*-sopropyl-2,6-naphthyridin-1-amine (166 mg, 0.61 mmol, 1.0 eq.), *tert*-butyl ((1*R*,3*S*)-3-aminocyclopentyl)carbamate (146.2 mg, 0.73 mmol, 1.2 eq.), $Pd_2(dba)_3$ (82.4 mg, 0.09 mmol, 0.15 eq.), BINAP (112 mg, 0.18 mmol, 0.3 eq.) and cesium carbonate (498.5 mg, 1.53 mmol, 2.5 eq.) were added into 1,4-dioxane (15 mL). The mixture was reacted at 120 °C for 20 h in a sealed tube in nitrogen atmosphere. When there were no materials left, as detected by TLC, the reaction solution was added with water (30 mL) and extracted with ethyl acetate (30 mL × 3). The organic phases were combined, washed with hydrochloric acid solution (0.2 mol/L, 50 mL), dried over anhydrous sodium sulfate and filtered, and the filtrate was concentrated under reduced pressure to give a crude product, which was purified by silica gel column chromatography (EA:PE = 1:5-1:2) to give the product (100 mg, yield: 37.6%).

Step 2: synthesis of $N^7$-((1*S*,3*R*)-3-aminocyclopentyl)-3-(difluoromethyl)-$N^1$-isopropyl-2,6-naphthyridine-1,7-diamine

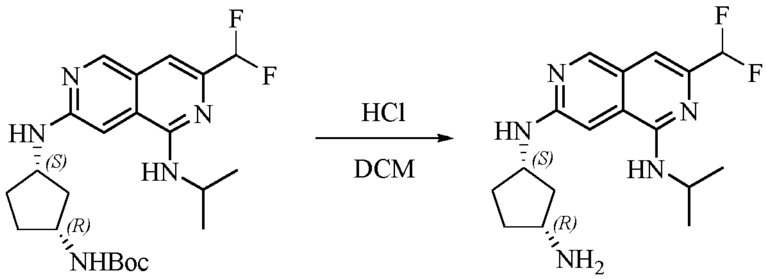


*Tert*-butyl ((1*R*,3*S*)-3-((7-(difluoromethyl)-5-(isopropylamino)-2,6-naphthyridin-3-yl)amino)cyclopentyl)carbamate (100 mg, 0.23 mmol, 1.0 eq.) was dissolved in DCM (2 mL). The reaction solution was added dropwise with hydrogen chloride ethanol solution (10 mol/L, 2 mL) and reacted at room temperature for 1 h. When there were no materials left, as detected by TLC, the reaction solution was concentrated under reduced pressure, added with water (30 mL) and back-extracted with ethyl acetate (30 mL × 2). The aqueous phase was retained, adjusted to pH of about 8 with sodium carbonate and extracted with DCM (30 mL × 3). The organic phases were combined, dried over anhydrous sodium sulfate and filtered, and the filtrate was concentrated under reduced pressure to give a crude product, which was purified by preparative thin-layer chromatography (MeOH:DCM = 1:20) to give the product (45 mg, yield: 58.3%). $^1$HNMR (400 MHz, $CD_3OD$) δ(ppm): 8.68 (s, 1H), 7.08 (s, 1H), 6.94 (s, 1H), 6.65-6.37 (s, 1H), 4.53-4.50 (m, 1H), 4.26-4.22 (m, 1H), 3.62-3.59 (m, 1H), 2.69-2.63 (m, 1H), 2.23-2.14 (m, 2H), 1.83-1.81 (m, 2H), 1.58-1.51 (m, 1H), 1.35-1.33 (m, 6H). Molecular formula: $C_{17}H_{23}F_2N_5$ Exact mass: 335.19 LC-MS (Pos, *m*/*z*) =336.34[M+H]$^+$.

**Example 54: Synthesis of (*S*)-3-(difluoromethyl)-$N^1$-isopropyl-$N^7$-(piperidin-3-yl)-2,6-naphthyridine-1,7-diamine (compound 54)**

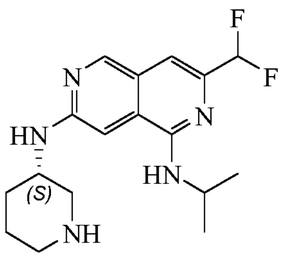

Step 1: synthesis of *tert*-butyl (*S*)-3-((7-(difluoromethyl)-5-(isopropylamino)-2,6-naphthyridin-3-yl)amino)piperidine-1-carboxylate

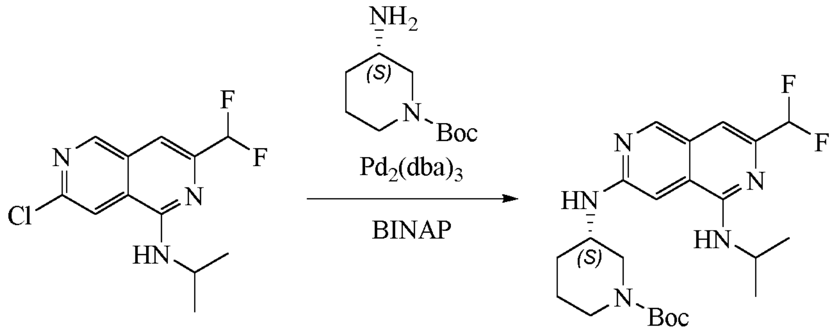


7-chloro-3-(difluoromethyl)-*N*-isopropyl-2,6-naphthyridin-1-amine (150 mg, 0.55 mmol, 1.0 eq.), *tert*-butyl (S)-3-aminopiperidine-1-carboxylate (144.2 mg, 0.42 mmol, 1.3 eq.), $Pd_2(dba)_3$ (73.2 mg, 0.08 mmol, 0.15 eq.), BINAP (99.6 mg, 0.16 mmol, 0.3 eq.) and cesium carbonate (449.6 mg, 1.38 mmol, 2.5 eq.) were added into 1,4-dioxane (15 mL). The mixture was reacted at 100 °C for 18 h in a sealed tube in nitrogen atmosphere. When there were no materials left, as detected by TLC, the reaction solution was added with water (30 mL) and extracted with ethyl acetate (30 mL × 3). The organic phases were combined, washed with hydrochloric acid solution (0.2 mol/L, 50 mL), dried over anhydrous sodium sulfate and filtered, and the filtrate was concentrated under reduced pressure to give a crude product, which was purified by silica gel column chromatography (EA:PE = 1:7-1:5) to give the product (160 mg, yield: 66.8%).

Step 2: synthesis of (*S*)-3-(difluoromethyl)-$N^1$-isopropyl-$N^7$-(piperidin-3-yl)-2,6-naphthyridine-1,7-diamine

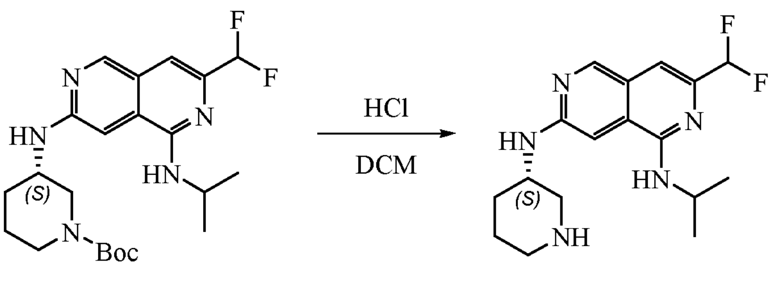


*Tert*-butyl (*S*)-3-((7-(difluoromethyl)-5-(isopropylamino)-2,6-naphthyridin-3-yl)amino)piperidine-1-carboxylate (160 mg, 0.36 mmol, 1.0 eq.) was dissolved in DCM (3 mL). The reaction solution was added dropwise with hydrogen chloride ethanol solution (10 mol/L, 3 mL) and reacted at room temperature for 1 h. When there were no materials left, as detected by TLC, the reaction solution was added with water (20 mL) and back-extracted with DCM (30 mL × 2). The aqueous phase was retained, adjusted to pH of about 8 with potassium carbonate and extracted with DCM (20 mL × 3). The organic phases were combined, dried over anhydrous sodium sulfate and filtered, and the filtrate was concentrated under reduced pressure to give a crude product, which was purified by preparative thin-layer chromatography (MeOH:DCM = 1:10) to give the product (106 mg, yield: 28.9%).

$^1$HNMR (400 MHz, $CD_3OD$) δ(ppm): 8.78 (s, 1H), 7.10 (s, 1H), 7.02 (s, 1H), 6.66-6.38 (s, 1H), 4.52-4.49 (m, 1H), 4.29-4.26 (m, 1H), 3.64-3.60 (m, 1H), 3.37-3.36 (m, 1H), 3.10-2.97 (m, 2H), 2.20-2.11 (m, 2H), 1.95-1.92 (m, 1H), 1.78-1.75 (m, 1H), 1.35-1.33 (m, 6H).
Molecular formula: $C_{17}H_{23}F_2N_5$ Exact mass: 335.19 LC-MS (Pos, *m/z)* =336.40[M+H]$^+$.

**Example 55: Synthesis of (*S*)-8-(isopropylamino)-2-(piperidin-3-ylamino)pyrido[3,4-*d*]pyrimidine-6-carbonitrile (compound 55)**

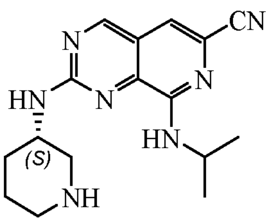

Step 1: synthesis of 8-(isopropylamino)-2-(methylthio)pyrido[3,4-d]pyrimidine-6-carbonitrile

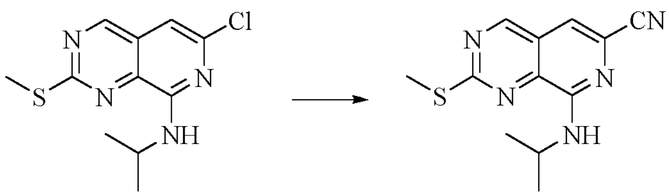

6-chloro-N isopropyl-2-(methylthio)pyrido[3,4-d]pyrimidin-8-amine (1.0 g, 3.72 mmol, 1.0 eq.), zinc cyanide (437 mg, 3.72 mmol, 1.0 eq.) and tetrakis(triphenylphosphine)palladium(0) (647 mg, 0.56 mmol, 0.15 eq.) were dispersed in DMA (10 mL), and the reaction solution was reacted at 120 °C for 17 h in nitrogen atmosphere. After the reaction was completed, as detected by TLC, the reaction solution was poured into water (20 mL) and extracted with MTBE (20 mL × 2). The organic phase was dried and concentrated under reduced pressure to give a crude product, which was purified by silica gel column chromatography (PE:EA = 20:1) to give the product (400 mg, yield: 41.4%).

Step 2: synthesis of 8-(isopropylamino)-2-(methylsulfonyl)pyrido[3,4-*d*]pyrimidine-6-carbonitrile

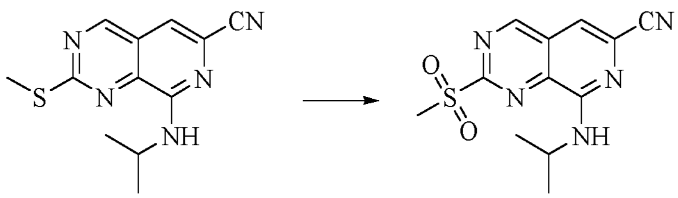

8-(isopropylamino)-2-(methylsulfonyl)pyrido[3,4-*d*]pyrimidine-6-carbonitrile (400 mg, 1.54 mmol, 1.0 eq.) was dissolved in DCM (20 mL), and the reaction solution was added with *m*-chloroperoxybenzoic acid (mass fraction: 80%, 664 mg, 3.08 mmol, 2.0 eq.) at room temperature, and reacted at room temperature for 30 min. After the reaction was completed, as detected by TLC, the reaction solution was added with saturated aqueous sodium carbonate (10 mL), the liquid separation was performed, and the aqueous phase was extracted with DCM (10 mL). The organic phases were combined, dried and concentrated to give a crude product, which was slurried with PE:MTBE = 2:1 (15 mL) and filtered under vacuum, and the filter cake was dried to give the product (360 mg, yield: 80.4%).

Step 3: synthesis of *tert*-butyl (*S*)-3-((6-cyano-8-(isopropylamino)pyrido[3,4-*d*]pyrimidin-2-yl)amino)piperidine-1-carboxylate

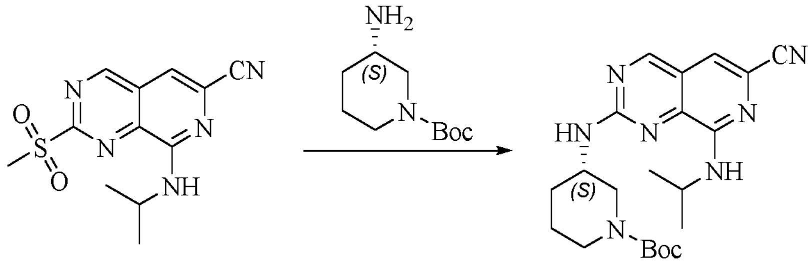

8-(isopropylamino)-2-(methylsulfonyl)pyrido[3,4-*d*]pyrimidine-6-carbonitrile (360 mg, 1.23 mmol, 1.0 eq.) and *tert*-butyl (*S*)-3-aminopiperidine-1-carboxylate (739 mg, 3.69 mmol, 3.0 eq.) were dissolved in ethanol (8 mL), and the reaction solution was reacted at 80 °C for 2 h. After the reaction was completed, as detected by TLC, the reaction solution was concentrated to give a crude product, which was purified by silica gel column chromatography (PE:EA= 3:1) to give the product (175 mg, yield: 34.6%).

Step 4: synthesis of (*S*)-8-(isopropylamino)-2-(piperidin-3-ylamino)pyrido[3,4-*d*]pyrimidine-6-carbonitrile

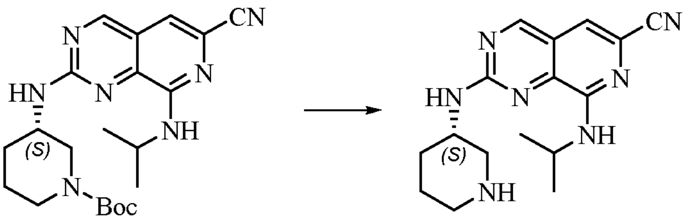

*Tert*-butyl (*S*)-3-((6-cyano-8-(isopropylamino)pyrido[3,4-*d*]pyrimidin-2-yl)amino)piperidine-1-carboxylate (175 mg, 0.42 mmol, 1.0 eq.) was dissolved in EA (2 mL). The reaction solution was added dropwise with a solution of hydrogen chloride in 1,4-dioxane (4 mol/L, 1 mL) and reacted at room temperature for 20 h. After the reaction was substantially completed, as detected by LC-MS, the reaction solution was poured into saturated aqueous potassium carbonate (5 mL) and extracted with EA (10 mL × 3). The organic phase was dried and concentrated to give a crude product, which was purified by preparative thin-layer chromatography (MeOH:DCM = 1:10) to give the product (76 mg, yield: 58.4%).
$^1$HNMR (400 MHz,MeOD) δ(ppm): 8.96 (s, 1H), 7.30 (s, 1H), 4.36 (s, 2H), 3.53-3.50 (t, 1H), 3.27-3.24 (d, 1H), 2.95-2.81 (m, 2H), 2.18-2.14 (m, 1H), 2.06-2.01 (m, 1H), 1.89-1.86 (d, 1H), 1.78-1.68 (m, 1H), 1.36-1.34 (d, 6H).
Molecular formula: $C_{16}H_{21}N_7$ Exact mass: 311.19 LC-MS(Pos, *m*/*z*)=312.33 [M+H]$^+$.

**Example 56: Synthesis of 1-(isopropylamino)-7-(((1*S*,3*S*)-3-((2-methoxyethyl)amino)cyclopentyl)amino)-2,6-naphthyridine-3-carbonitrile (compound 56)**

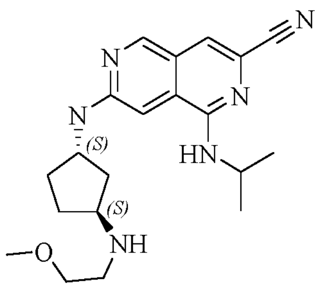

Steps:

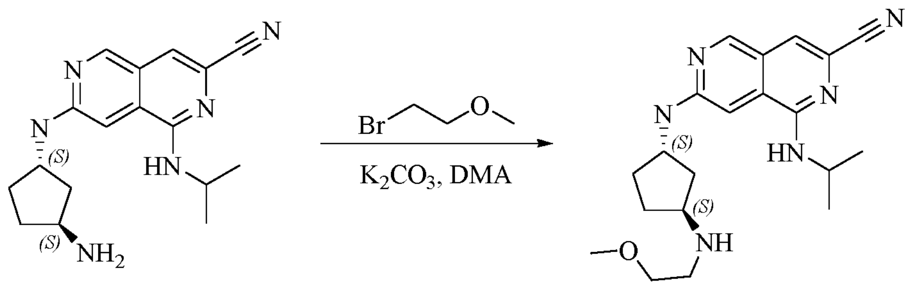


7-(((1*S*,3*S*)-3-aminocyclopentyl)amino)-1-(isopropylamino)-2,6-naphthyridine-3-carbonitrile (220 mg, 0.71 mmol, 1.0 eq.), 1-bromo-2-methoxyethane (98.7 mg,0.71 mmol, 1.0 eq.) and potassium carbonate (98 mg, 0.71 mmol, 1.0 eq.) were dissolved in DMA (3 mL). The reaction solution was heated to 100 °C and reacted for 20 h in nitrogen atmosphere. When there were no materials left, as detected by TLC, the reaction solution was added with water (20 mL) and extracted with ethyl acetate (20 mL × 3). The organic phases were combined, washed with water (20 mL × 2). dried over anhydrous sodium sulfate and filtered. The filtrate was concentrated under reduced pressure to give a crude product, which was purified by preparative thin-layer chromatography (MeOH:DCM = 1:20) to give the product (20 mg, yield: 7.6%).

$^1$HNMR (400 MHz, DMSO) δ(ppm): 8.71(s, 1H), 7.47 (s, 1H), 6.90 (s, 1H), 4.33-4.26 (m, 2H), 3.55 (m, 2H), 3.29 (s, 3H), 3.07 (m, 2H), 2.19 (m, 2H), 2.07-1.96 (m, 2H), 1.60-1.58 (m, 2H), 1.23-1.21 (m, 7H).

Molecular formula: $C_{20}H_{28}N_6O$ Exact mass: 368.23 LC-MS (Pos, *m/z)* =369.38 $[M+H]^+$.

**Example 57: Synthesis of (*S*)-1-(isopropylamino)-7-((1-methylpiperidin-3-yl)amino)-2,6-naphthyridine-3-carbonitrile (compound 57)**

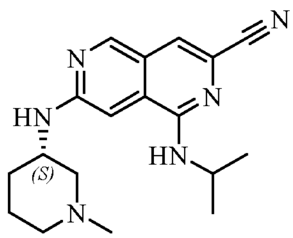

Steps:

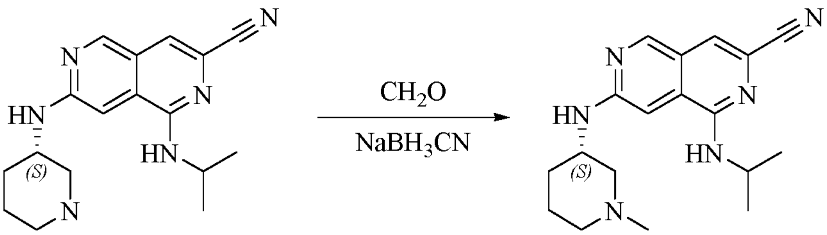


(*S*)-1-(isopropylamino)-7-(piperidin-3-ylamino)-2,6-naphthyridine-3-carbonitrile (108 mg, 0.35 mmol, 1.0 eq.) was dissolved in methanol (3 mL), and the reaction solution was added with acetic acid (21 mg, 0.35 mmol, 1.0 eq.) and aqueous formaldehyde solution (mass function: 37%, 56.8 mg, 0.70 mmol, 2.0 eq.) and reacted at room temperature for 1 h, and then added with sodium cyanoborohydride (66 mg, 1.05 mmol, 3.0 eq.) and reacted at room temperature for 1 h. When there were no materials left, as detected by TLC, the reaction solution was concentrated under reduced pressure, added with water (20 mL) and extracted with ethyl acetate (20 mL × 3). The organic phases were combined, dried over anhydrous sodium sulfate and filtered, and the filtrate was concentrated under reduced pressure to give a crude product, which was purified by preparative thin-layer chromatography (MeOH:DCM = 1:10) to give the product (66 mg, yield: 58.1%).

$^1$HNMR (400 MHz, MeOD) δ(ppm): 8.72 (s, 1H), 7.38 (s, 1H), 7.00 (s, 1H), 4.47-4.44 (m, 1H), 4.27-4.24 (m, 1H), 3.50 (m, 1H), 3.28-3.26 (m, 1H), 2.87 (m, 3H), 2.77 (s, 3H), 2.12-2.07 (m, 2H), 1.93-1.87 (m, 1H), 1.67-1.65 (m, 1H), 1.35-1.33 (m, 6H), 1.31 (m, 1H).

Molecular formula: $C_{18}$ $H_{24}N_6$ Exact mass: 324.21 LC-MS (Pos, *m/z*) =325.31 $[M+H]^+$.

**Example 58: Synthesis of 7-(((1S,3S)-3-aminocyclopentyl)amino)-1-(pentan-3-yl)-2,6-naphthyridine-3-carbonitrile (Compound 58)**

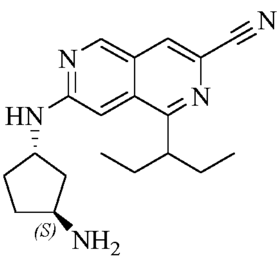

The titled compound was prepared as same as example 38 using corresponding chemicals.

$^1$H-NMR (400 MHz, $CD_3OD$) 6(ppm): 9.00 (s, 1H), 8.08 (s, 1H), 6.99 (s, 1H), 4.54-4.60 (m, 1H), 3.79-3.86 (m, 1H), 3.34-3.37 (m, 1H), 2.32-2.42 (m, 2H), 2.10-2.22 (m, 2H), 1.89-2.00 (m, 2H), 1.72-1.83 (m, 4H), 0.81-0.84 (t, 6H). Molecular formula:$C_{16}H_{25}N_5$ Exact mass: 323.21 LC-MS(Pos, *m/z*)=324.14 $[M+H]^+$

**Example 60: Synthesis of 7-(((1S,3S)-3-aminocyclopentyl)amino)-1-(((4-cyanotetrahydro-2H-pyran-4-yl)methyl)amino)-2,6-naphthyridine-3-carbonitrile(compound 60)**

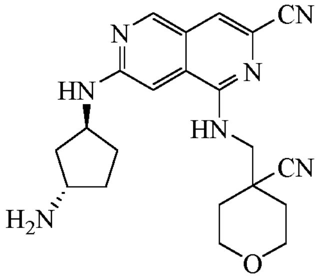

The titled compound was prepared as same as example 34 using corresponding chemicals.

$^1$HNMR (400 MHz, MeOD) δ(ppm): 8.73 (s, 1H), 7.48 (s, 1H), 6.88 (s, 1H), 4.39-4.32 (t, 1H), 4.01-3.99 (d, 2H), 3.96-3.94 (t, 2H), 3.71-3.64 (m, 2H), 3.61-3.58 (t, 1H), 2.41-2.33 (m, 1H), 2.19-2.16 (m, 1H), 1.99-1.95 (m, 2H), 1.91-1.87 (m, 4H), 1.96-1.60 (m, 1H), 1.57-1.52 (m, 1H).

Molecular formula:$C_{21}H_{25}N_7O$ Exact mass: 391.21 LC-MS(Pos, *m*/*z*)=392.15[M+H]$^+$.

**Example 61: Synthesis of 7-(((S)-piperidin-3-yl)amino)-1-((1,1,1-trifluoropropan-2-yl)amino)-2,6-naphthyridine-3-carbonitrile (compound 61)**

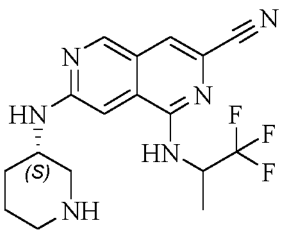

The titled compound was prepared as same as example 38 using corresponding chemicals.

$^1$HNMR (400 MHz, MeOD) δ(ppm): 8.76 (s, 1H), 7.53 (s, 1H), 7.00 (s, 1H), 5.42-5.35 (m, 1H), 3.91 (m, 1H), 3.29 (m, 1H), 3.05-3.01 (m, 2H), 2.72-2.67 (m, 2H), 2.2.62-2.56 (m, 1H), 2.14-2.12 (m, 1H), 2.00 (s, 3H), 1.88-1.86 (m, 1H), 1.71-1.57 (m, 2H), 1.52-1.50 (d, 3H).

Molecular formula: $C_{17}H_{19}F_3N_6$ Exact mass: 364.38 LC-MS (Pos, *m*/*z*) =365.31 [M+H]$^+$.

**Example 63: Synthesis of 2-(dimethylamino)-*N*-((1*R*,4*r*)-4-((6-((*R*)-1-hydroxyethyl)-8-(isopropylamino)pyrido[3,4-*d*]pyrimidin-2-yl)amino)cyclohexyl)acetamide (compound 63)**

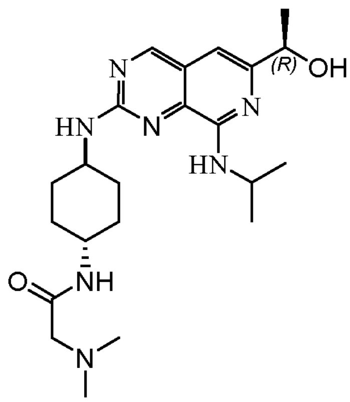

Steps:

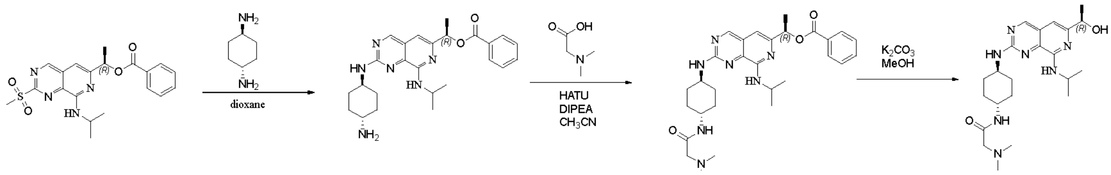

Step 1: synthesis of (*R*)-1-(2-(((1r,4R)-4-aminocyclohexyl)amino)-8-(isopropylamino)pyrido[3,4-*d*]pyrimidin-6-yl)ethyl benzoate

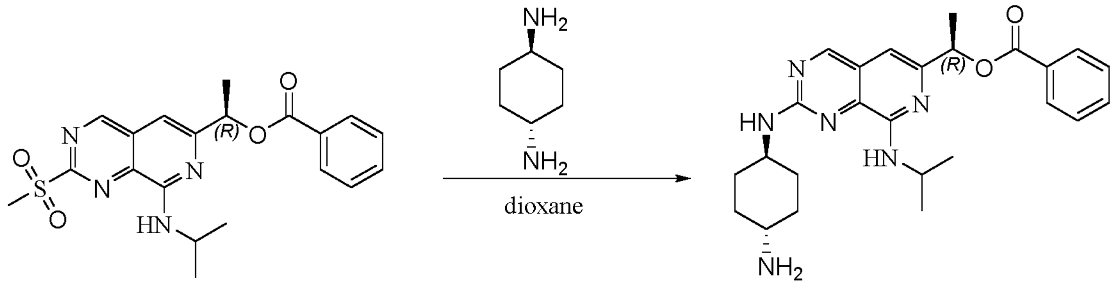


(*R*)-1-(8-(isopropylamino)-2-(methylsulfonyl)pyrido[3,4-*d*]pyrimidin-6-yl)ethyl benzoate (200 mg, 0.48 mmol, 1.0 eq.) was dissolved in dioxane (2 mL), and the reaction solution was added with trans-cyclohexane-1,4-diamine (276 mg, 2.41 mmol, 5.0 eq.) and reacted at 100 °C for overnight. When there were no materials left, as detected by LCMS, the reaction solution was added with water (10 mL) and extracted with ethyl acetate (20 mL × 2). The organic phases were combined, dried over anhydrous sodium sulfate and filtered, and the filtrate was concentrated under reduced pressure to give a crude product, which was purified by amino column chromatography to give the product (199 mg, yield: 91.9%).

Step 2: synthesis of (*R*)-1-(2-(((1*r*,4*R*)-4-(2-(dimethylamino)acetamido)cyclohexyl)amino)-8-(isopropylamino)pyrido[3,4-d]pyrimidin-6-yl)ethyl benzoate

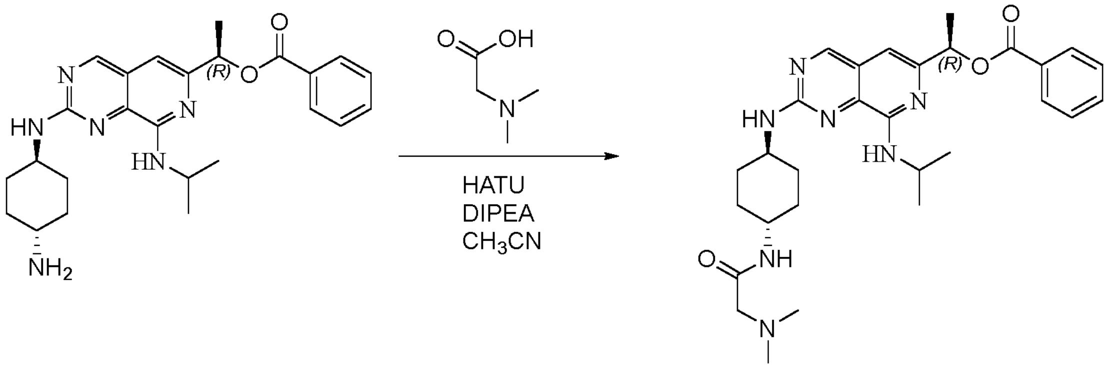


(*R*)-1-(2-(((1*r*,4*R*)-4-aminocyclohexyl)amino)-8-(isopropylamino)pyrido[3,4-*d*]pyrimidin-6-yl)ethyl benzoate (40 mg, 0.089 mmol, 1.0 eq.) and N,N-Dimethylglycine (10 mg, 0.099 mmol, 1.1 eq.) were dissolved in $CH_3CN$ (0.9 mL), and the reaction solution was added with HATU (51 mg, 0.13 mmol, 1.5 eq.) and DIPEA (45 mg, 0.45 mmol, 5.0 eq.). The mixture was stirred at room temperature for overnight. When there were no materials left, as detected by LCMS, the reaction solution was added with water (10 mL) and extracted with ethyl acetate (20 mL × 3). The organic phases were combined, dried over anhydrous sodium sulfate and filtered, and the filtrate was concentrated under reduced pressure to give a crude product, which was used in the next step without further purification.

Step 3: synthesis of 2-(dimethylamino)-*N*-((1*R*,4*r*)-4-((6-((*R*)-1-hydroxyethyl)-8-(isopropylamino)pyrido[3,4-*d*]pyrimidin-2-yl)amino)cyclohexyl)acetamide

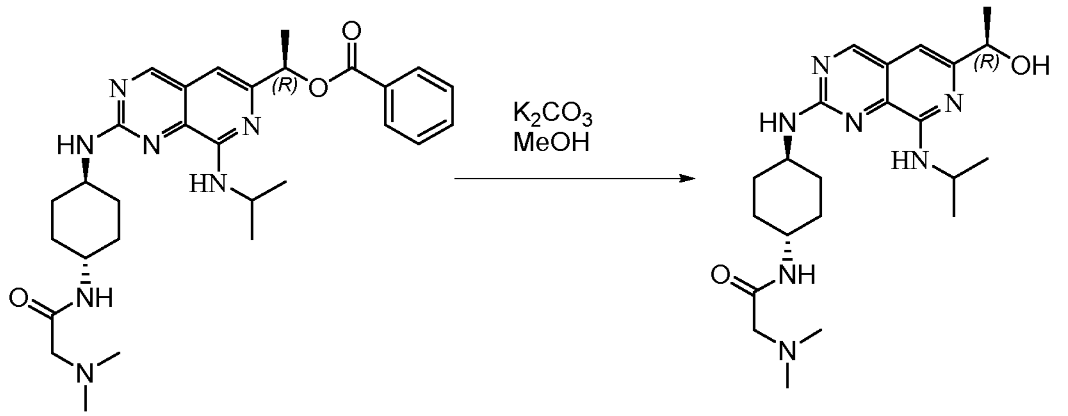


($R$)-1-(2-(((1$r$,4$R$)-4-(2-(dimethylamino)acetamido)cyclohexyl)amino)-8-(isopropylamino)pyrido[3,4-$d$]pyrimidin-6-yl)ethyl benzoate (0.089 mmol, 1.0 eq.) was dissolved in MeOH (0.9 mL), and the reaction solution was added with $K_2CO_3$ (62 mg, 0.45 mmol, 5.0 eq.). The mixture was stirred at room temperature for 3 h. When there were no materials left, as detected by LCMS, the reaction solution was added with water (10 mL) and extracted with ethyl acetate (20 mL $\times$ 3). The organic phases were combined, dried over anhydrous sodium sulfate and filtered, and the filtrate was concentrated under reduced pressure to give a crude product, which was purified by HPLC to give the product (16.9 mg, yield: 44.0%).
$^1$H-NMR (DMSO) $\delta$(ppm): 8.96 (br-s, 1H), 7.51 (d, 1H), 7.30 (br-s, 1H), 6.80 (s, 1H), 6.24 (d, 1H), 5.05 (br-s, 1H), 4.54 (br-s, 1H), 4.17 (br-s, 1H), 3.77 (br-s, 1H), 3.57 (br-s, 1H), 2.80 (br-s, 2H), 2.16 (br-s, 6H), 1.94 (br-s, 2H), 1.75 (m, 2H), 1.50-1.10 (m, 13H).
Molecular formula: $C_{22}H_{35}N_7O_2$ Exact mass: 429.29 LC-MS (Pos, $m/z$) =430.54 $[M+H]^+$.

**Example 64: Synthesis of 2-(dimethylamino)-*N*-((1*R*,4*r*)-4-((6-((*R*)-1-hydroxyethyl)-8-(neopentylamino)pyrido[3,4-*d*]pyrimidin-2-yl)amino)cyclohexyl)acetamide (compound 64)**

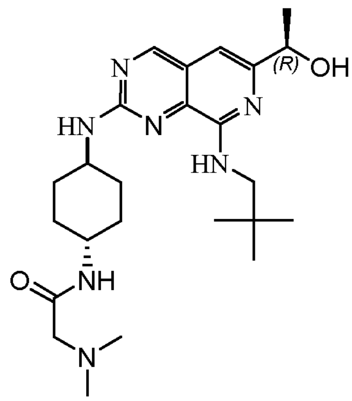

The titled compound was prepared as same as example 63 using corresponding chemicals.

$^1$HNMR (400 MHz, DMSO) $\delta$(ppm): 8.97 (s, 1H), 7.64 (d, 1H), 7.42 (br-s, 1H), 6.81 (s, 1H), 6.57 (t, 1H), 5.06 (d, 1H), 4.54 (t, 1H), 3.73 (s, 1H), 3.59 (s, 1H), 3.42-3.27 (m, 3H), 2.92 (s, 2H), 2.23 (s, 6H), 2.06 (s, 1H), 1.78 (s, 2H), 1.38-1.32 (m, 7H), 0.94 (s, 9H).

Molecular formula: $C_{24}H_{39}N_7O_2$ Exact mass: 457.32 LC-MS (Pos, $m/z$) =458.35 $[M+H]^+$.

**Example 65: Synthesis of *N*-((1*R*,4*r*)-4-((6-((*R*)-1-hydroxyethyl)-8-(isopropylamino)pyrido[3,4-*d*]pyrimidin-2-yl)amino)cyclohexyl)acetamide (compound 65)**

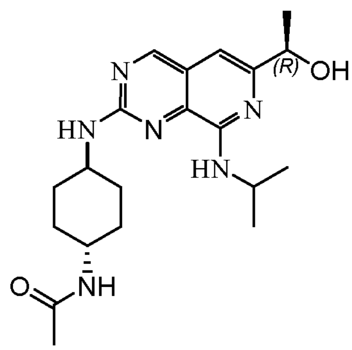

Steps:

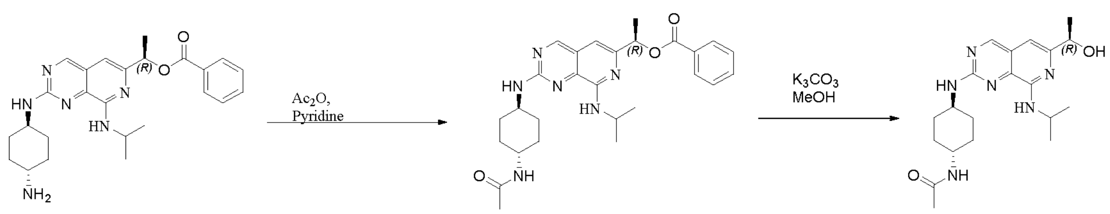


Step 1: synthesis of (*R*)-1-(2-(((1*r*,4*R*)-4-acetamidocyclohexyl)amino)-8-(isopropylamino)pyrido[3,4-d]pyrimidin-6-yl)ethyl benzoate

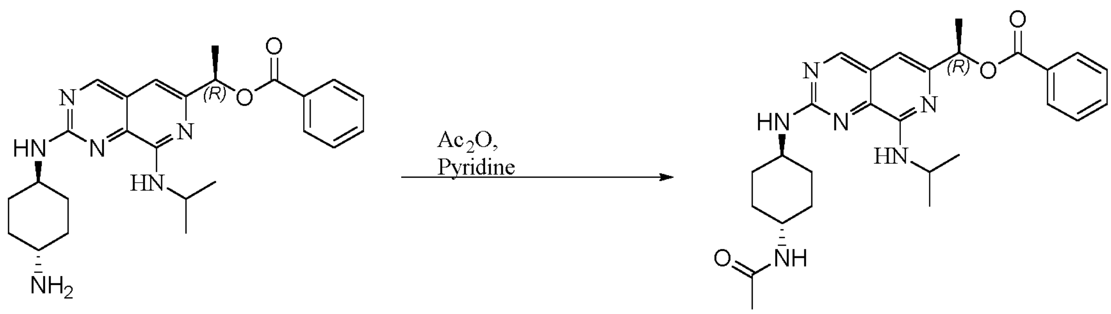


(*R*)-1-(2-(((1*r*,4*R*)-4-aminocyclohexyl)amino)-8-(isopropylamino)pyrido[3,4-*d*]pyrimidin-6-yl)ethyl benzoate (250 mg, 0.56 mmol, 1.0 eq.) was dissolved in pyridine (1.1 mL). The reaction solution was added with acetic anhydride (0.105 mL, 1.11 mmol, 2.0 eq.) and reacted at room temperature for 2 h. When there were no materials left, as detected by LC-MS, the reaction solution was poured into water (10 mL) and extracted with ethyl acetate (30 mL × 2). The organic phases were combined, dried over anhydrous sodium sulfate and filtered. The filtrate was concentrated under reduced pressure to give a crude product, which was purified by silica gel column chromatography to give the product (265 mg, yield: 98.9%,).

Step 2: synthesis of *N*-((1*R*,4*r*)-4-((6-((*R*)-1-hydroxyethyl)-8-(*iso*propylamino)pyrido[3,4-d]pyrimidin-2-yl)amino)cyclohexyl)acetamide

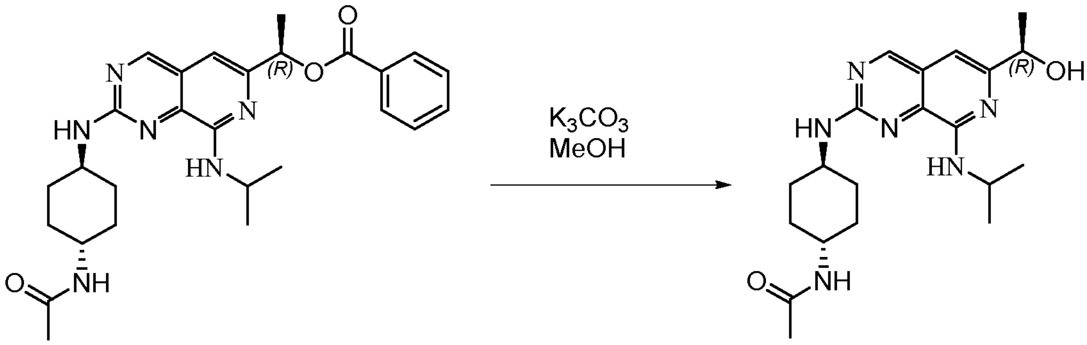


(*R*)-1-(2-(((1*r*,4*R*)-4-acetamidocyclohexyl)amino)-8-(isopropylamino)pyrido[3,4-d]pyrimidin-6-yl)ethyl benzoate (265 mg, 0.54 mmol, 1.0 eq.) was dissolved in MeOH (10 mL), and the reaction solution was added with $K_2CO_3$ (300 mg, 2.17 mmol, 4.0 eq.). The mixture was stirred at room temperature for 6 h. When there were no materials left, as detected by LCMS, the reaction solution was added with water (10 mL) and concentrated under reduced pressure. The crude was extracted with ethyl acetate (40 mL × 2). The organic phases were combined, dried over anhydrous sodium sulfate and filtered, and the filtrate was concentrated under reduced pressure to give a crude product, which was purified by silica gel column chromatography to give the product (178 mg, yield:82.6%).
$^1$H-NMR (DMSO) δ(ppm): 8.97 (s, 1H), 7.75 (d, 1H), 7.30 (br-s, 1H), 6.82 (s, 1H), 6.28 (s, 1H), 5.06 (br-s, 1H), 4.56 (d, 1H), 4.25-4.14 (m, 1H), 3.80 (br-s, 1H), 3.53-3.45 (m, 1H), 1.97 (br-s, 2H), 1.82 (d, 2H), 1.77 (s, 3H), 1.38-1.18 (m, 13H).
Molecular formula: $C_{20}H_{30}N_6O_2$ Exact mass: 386.24 LC-MS (Pos, *m*/*z*) = 387.25 $[M+H]^+$.

**Example 66: Synthesis of N-((1*r*,4*r*)-4-((6-cyano-8-(isopropylamino)pyrido[3,4-d]pyrimidin-2-yl)amino)cyclohexyl)-2-(dimethylamino)acetamide (compound 66)**

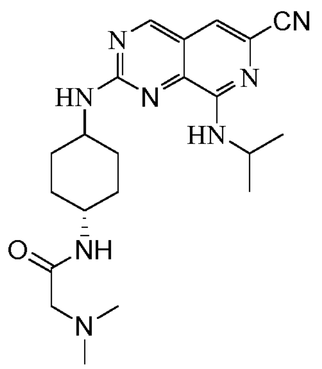

The titled compound was prepared as same as example 63 using corresponding chemicals.

$^1$H-NMR (DMSO) δ(ppm): 8.99 (s, 1H), 8.05 (d, 1H), 7.57 (d, 1H), 7.45 (s, 1H), 6.72 (d, 1H), 4.19 (br-s, 1H), 3.88 (br-s, 1H), 3.59 (br-s, 1H), 2.85 (s, 2H), 2.20 (s, 6H), 2.00-1.73 (m, 4H), 1.47-1.35 (m, 4H), 1.27-1.20 (m, 6H).

Molecular formula: $C_{21}H_{30}N_8O$ Exact mass: 410.25 LC-MS (Pos, *m*/*z*) = 411.25 $[M+H]^+$.

**Example 67: Synthesis of (*R*)-1-(8-(isopropylamino)-2-(((1*r*,4*R*)-4-(4-methylpiperazin-1-yl)cyclohexyl)amino)pyrido[3,4-d]pyrimidin-6-yl)ethan-1-ol (compound 67)**

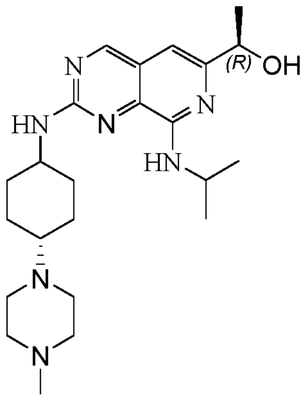

The titled compound was prepared as same as step 1 and step 3 of example 63 using corresponding chemicals.

$^1$H-NMR (DMSO) δ(ppm): 8.97 (s, 1H), 7.28 (br-s, 1H), 6.82 (s, 1H), 6.28 (d, 2H), 5.06 (d, 1H), 4.52-4.60 (m, 1H), 4.16-4.27 (m, 1H), 3.75-3.77 (br-s, 1H), 2.49 (br-s, 4H), 2.30-2.34 (m, 5H), 2.21 (s, 3H), 2.13 (2H, br s), 1.83 (br-s, 2H), 1.27-1.37 (m, 7H), 1.15-1.24 (m, 6H).

Molecular formula: $C_{23}H_{37}N_7O$ Exact mass: 427.31 LC-MS (Pos, *m*/*z*) = 428.3 $[M+H]^+$.

**Example 68: Synthesis of (*R*)-1-(8-(isopropylamino)-2-(((1-(methylsulfonyl)piperidin-4-yl)methyl)amino)pyrido[3,4-d]pyrimidin-6-yl)ethan-1-ol (compound 68)**

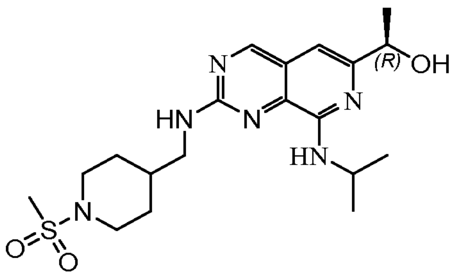

Steps:

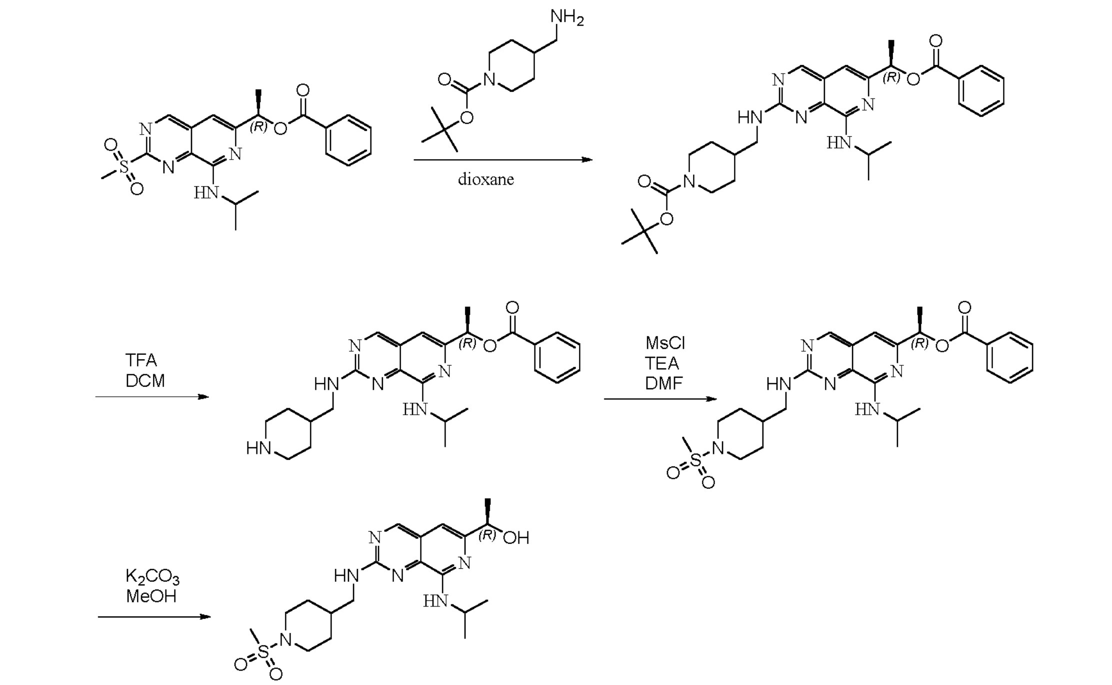


Step 1: synthesis of *tert*-butyl (*R*)-4-(((6-(1-(benzoyloxy)ethyl)-8-(isopropylamino)pyrido[3,4-*d*]pyrimidin-2-yl)amino)methyl)piperidine-1-carboxylate

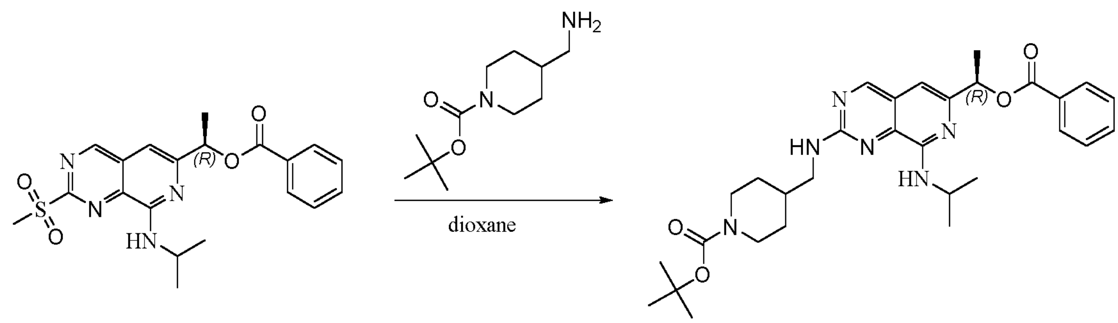


(*R*)-1-(8-(isopropylamino)-2-(methylsulfonyl)pyrido[3,4-*d*]pyrimidin-6-yl)ethyl benzoate (80 mg, 0.193 mmol, 1.0 eq.) was dissolved in dioxane (2 mL), and the reaction solution was added with *tert*-butyl 4-(aminomethyl)piperidine-1-carboxylate (124 mg, 0.579 mmol, 3.0 eq.) and reacted at 100 °C for 6 h. When there were no materials left, as detected by LCMS, the reaction solution was added with water (10 mL) and extracted with ethyl acetate (20 mL × 2). The organic phases were combined, dried over anhydrous sodium sulfate and filtered, and the filtrate was concentrated under reduced pressure to give a crude product, which was purified by silica gel column chromatography to give the product (98 mg, yield: 92.5%).

Step 2: synthesis of (*R*)-1-(8-(isopropylamino)-2-((piperidin-4-ylmethyl)amino)pyrido[3,4-*d*]pyrimidin-6-yl)ethyl benzoate

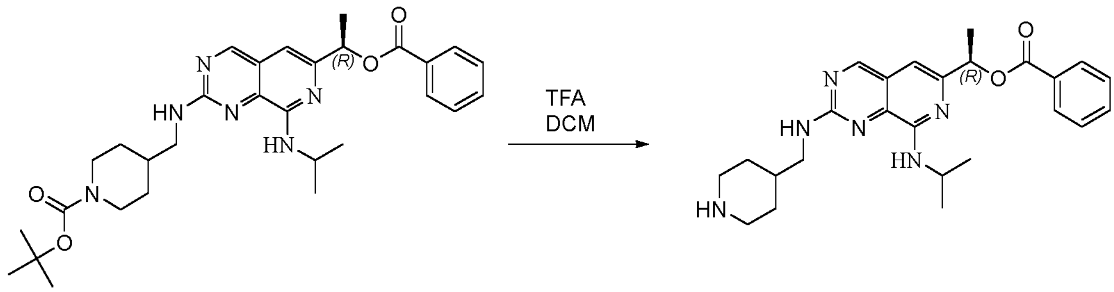


*tert*-butyl (*R*)-4-(((6-(1-(benzoyloxy)ethyl)-8-(isopropylamino)pyrido[3,4-*d*]pyrimidin-2-yl)amino)methyl)piperidine-1-carboxylate (204 mg, 0.372 mmol, 1.0 eq.) was dissolved in DCM (3 mL). The reaction solution was added dropwise with TFA (0.5 mL) and reacted at room temperature for 5 h. When there were no materials left, as detected by LCMS, the reaction solution was concentrated under reduced pressure to give a crude product, which was used in the next step without further purification.

Step 3: synthesis of (*R*)-1-(8-(isopropylamino)-2-(((1-(methylsulfonyl)piperidin-4-yl)methyl)amino)pyrido[3,4-*d*]pyrimidin-6-yl)ethyl benzoate

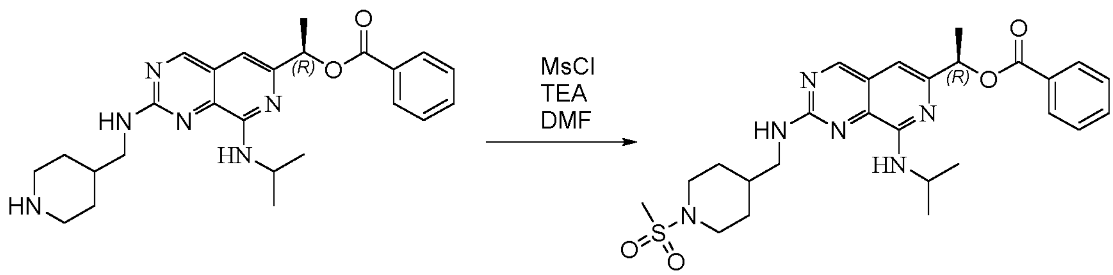


(*R*)-1-(8-(isopropylamino)-2-((piperidin-4-ylmethyl)amino)pyrido[3,4-d]pyrimidin-6-yl)ethyl benzoate TFA salt (67 mg, 0.120 mmol, 1.0 eq.) was dissolved in DMF (1 mL), and the reaction solution was added with MsCl (21 mg, 0.180 mmol, 1.5 eq.) and TEA (0.034 mL, 0.240 mmol, 2.0 eq.) and reacted at room temperature for 2 h. When there were no materials left, as detected by LCMS, the reaction solution was added with water (10 mL) and extracted with ethyl acetate (20 mL × 2). The organic phases were combined, dried over anhydrous sodium sulfate and filtered, and the filtrate was concentrated under reduced pressure to give a crude product, which was used in the next step without further purification.

Step 4: synthesis of (*R*)-1-(8-(isopropylamino)-2-(((1-(methylsulfonyl)piperidin-4-yl)methyl)amino)pyrido[3,4-*d*]pyrimidin-6-yl)ethan-1-ol

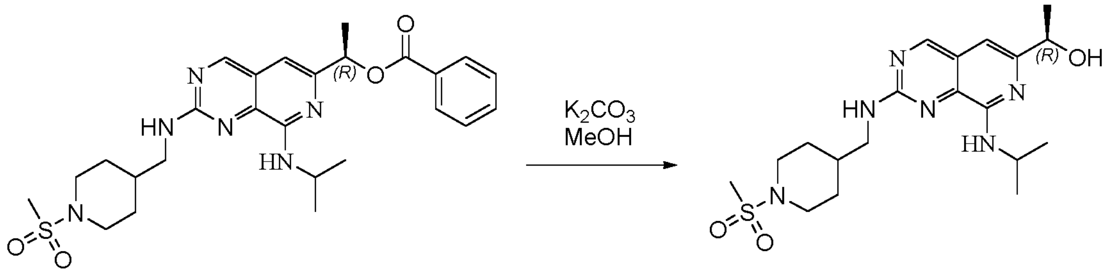


(*R*)-1-(8-(isopropylamino)-2-(((1-(methylsulfonyl)piperidin-4-yl)methyl)amino)pyrido[3,4-*d*]pyrimidin-6-yl)ethyl benzoate (0.120 mmol, 1.0 eq.) was dissolved in MeOH (3 mL), and the reaction solution was added with $K_2CO_3$ (100 mg, 0.723 mmol, 6.0 eq.). The mixture was stirred at room temperature for 6 h. When there were no materials left, as

detected by LCMS, the reaction solution was added with water (10 mL) and extracted with ethyl acetate (20 mL × 3). The organic phases were combined, dried over anhydrous sodium sulfate and filtered, and the filtrate was concentrated under reduced pressure to give a crude product, which was purified by HPLC to give the product (13.9 mg, yield: 27.4%). Molecular formula: $C_{19}H_{30}N_6O_3S$ Exact mass: 422.21 LC-MS (Pos, *m*/*z*) =423.1 $[M+H]^+$.

**Example 69: Synthesis of (*R*)-1-(4-(((6-(1-hydroxyethyl)-8-(isopropylamino)pyrido[3,4-*d*]pyrimidin-2-yl)amino)methyl)piperidin-1-yl)ethan-1-one (compound 69)**

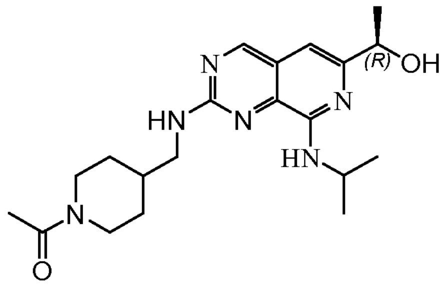

Steps:

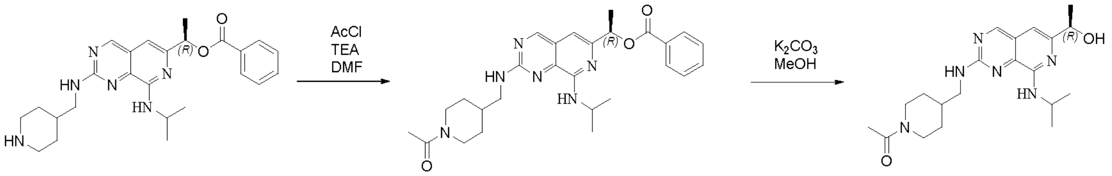


Step 1: synthesis of (*R*)-1-(2-(((1-acetylpiperidin-4-yl)methyl)amino)-8-(isopropylamino)pyrido[3,4-*d*]pyrimidin-6-yl)ethyl benzoate

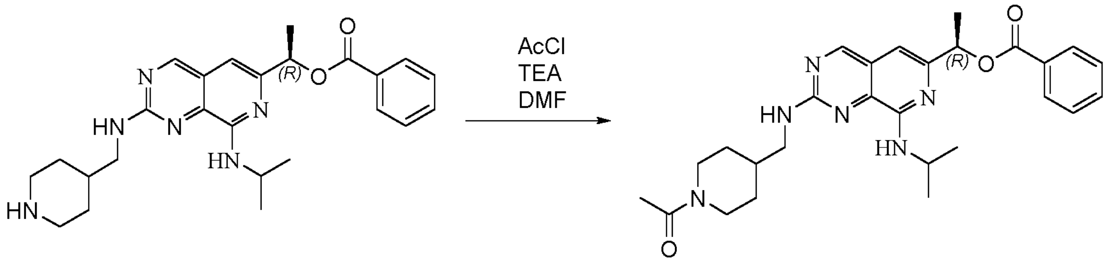


(*R*)-1-(8-(isopropylamino)-2-((piperidin-4-ylmethyl)amino)pyrido[3,4-*d*]pyrimidin-6-yl)ethyl benzoate TFA salt (67 mg, 0.120 mmol, 1.0 eq.) was dissolved in DMF (1 mL), and the reaction solution was added with AcCl (14 mg, 0.180 mmol, 1.5 eq.) and TEA (0.034 mL, 0.240 mmol, 2.0 eq.) and reacted at room temperature for 1 h. When there were no materials left, as detected by LCMS, the reaction solution was added with water (10 mL) and extracted with ethyl acetate (20 mL × 2). The organic phases were combined, dried over anhydrous sodium sulfate and filtered, and the filtrate was concentrated under reduced pressure to give a crude product, which was used in the next step without further purification.

Step 2: synthesis of (*R*)-1-(4-(((6-(1-hydroxyethyl)-8-(isopropylamino)pyrido[3,4-*d*]pyrimidin-2-yl)amino)methyl)piperidin-1-yl)ethan-1-one

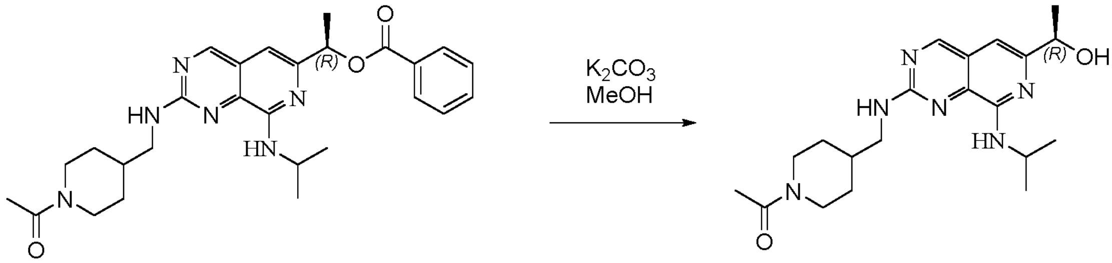


(*R*)-1-(2-(((1-acetylpiperidin-4-yl)methyl)amino)-8-(isopropylamino)pyrido[3,4-*d*]pyrimidin-6-yl)ethyl benzoate (0.120 mmol, 1.0 eq.) was dissolved in MeOH (3 mL), and the reaction solution was added with $K_2CO_3$ (100 mg, 0.723 mmol, 6.0 eq.). The mixture was stirred at room temperature for 6 h. When there were no materials left, as detected by LCMS, the reaction solution was added with water (10 mL) and extracted with ethyl acetate (20 mL × 3). The organic phases were combined, dried over anhydrous sodium sulfate and filtered, and the filtrate was concentrated under reduced pressure to give a crude product, which was purified by HPLC to give the product (35.2 mg, yield: 75.9%).
$^1$H-NMR (DMSO) δ(ppm): 8.96 (s, 1H), 7.54 (br-s, 1H), 6.82 (s, 1H), 6.32 (d, 1H), 5.07 (d, 1H), 4.54-4.58 (m, 1H), 4.34 (d, 1H), 4.18-4.25 (m, 1H), 3.79 (d, 1H), 3.28-3.33 (m, 3H), 2.96 (t, 1H), 1.96 (s, 3H), 1.68-1.90 (m, 3H), 1.34 (d, 3H), 1.04-1.25 (m, 8H). Molecular formula: $C_{20}H_{30}N_6O_2$ Exact mass: 386.24 LC-MS (Pos, *m*/*z*) =387.1 $[M+H]^+$.

**Example 70: Synthesis of (1*R*)-1-(8-(isopropylamino)-2-(((1-(methylsnlfonyl)piperidin-3-yl)methyl)amino)pyrido[3,4-*d*]pyrimidin-6-yl)ethan-1-ol (compound 70)**

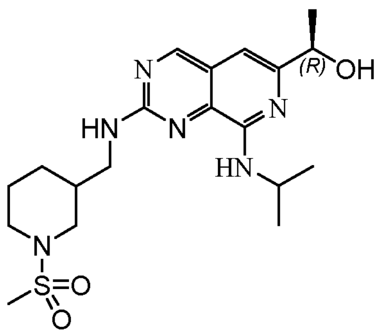

The titled compound was prepared as same as example 68 using corresponding chemicals.

$^1$H-NMR (DMSO) δ(ppm): 8.98 (s, 1H), 7.60 (br-s, 1H), 7.17 (t, 1H), 6.83 (s, 1H), 6.31 (d, 1H), 5.08 (br-s, 1H), 4.55 (br-s, 1H), 4.19-4.24 (m, 1H), 3.59 (d, 1H), 3.43 (d, 1H), 3.31-3.33 (m, 1H), 2.81 (s, 3H), 2.68 (t, 1H), 2.48-2.53 (m, 1H), 1.87-1.93 (m, 1H), 1.72-1.79 (m, 2H), 1.40-1.49 (m, 1H), 1.35 (d, 3H), 1.10-1.25 (m, 7H).

Molecular formula: $C_{19}H_{30}N_6O_3S$ Exact mass: 422.21 LC-MS (Pos, *m*/*z*) =423.1 $[M+H]^+$.

**Example 71: Synthesis of 1-(3-(((6-((*R*)-1-hydroxyethyl)-8-(isopropylamino)pyrido[3,4-*d*]pyrimidin-2-yl)amino)methyl)piperidin-1-yl)ethan-1-one (compound 71)**

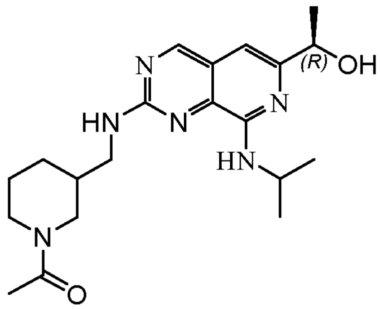

The titled compound was prepared as same as example 69 using corresponding chemicals.

Molecular formula: $C_{20}H_{30}N_6O_2$ Exact mass: 386.24 LC-MS (Pos, *m/z*) =387.1 $[M+H]^+$.

**Example 72: Synthesis of *N*-((1*R*,4*r*)-4-(((6-((*R*)-1-hydroxyethyl)-8-(isopropylamino)pyrido[3,4-*d*]pyrimidin-2-yl)amino)methyl)cyclohexyl)acetamide (compound 72)**

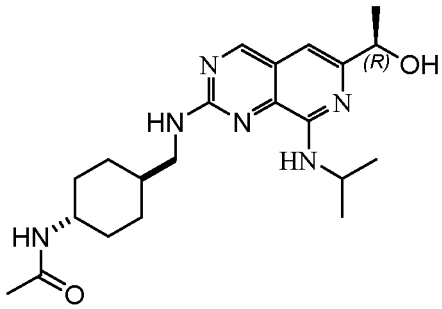

The titled compound was prepared as same as example 69 using corresponding chemicals.

$^1$H-NMR (DMSO) δ(ppm): 8.95 (s, 1H), 7.67 (d, 1H), 7.49 (br-s, 1H), 6.81 (s, 1H), 6.29 (d, 1H), 5.07 (d, 1H), 4.52-4.58 (m, 1H), 4.16-4.25 (m, 1H), 3.40-3.50 (m, 1H), 3.22 (t, 1H), 1.73-1.79 (m, 7H), 1.56-1.59 (m, 1H), 1.37 (d, 3H), 1.24 (d, 6H), 0.95-1.15 (m, 4H).

Molecular formula: $C_{21}H_{32}N_6O_2$ Exact mass: 400.26 LC-MS (Pos, *m/z*) =401.15 $[M+H]^+$.

**Example 73: Synthesis of *N*-((1*R*,4*r*)-4-(((6-((*R*)-1-hydroxyethyl)-8-(isopropylamino)pyrido[3,4-*d*]pyrimidin-2-yl)amino)methyl)cyclohexyl)methanesulfonamide (compound 73)**

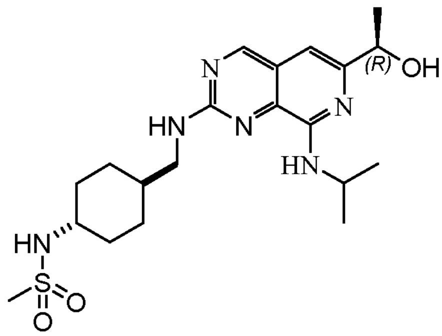

The titled compound was prepared as same as example 68 using corresponding chemicals.

$^1$H-NMR (DMSO) δ(ppm): 8.95 (s, 1H), 7.48 (br-s, 1H), 6.96 (d, 1H), 6.82 (s, 1H), 6.29 (d, 1H), 5.07 (d, 1H), 4.54-4.57 (m, 1H), 4.16-4.25 (m, 1H), 3.23 (t, 2H), 3.00-3.07 (m, 1H), 2.88 (s, 3H), 1.90 (br-d, 2H), 1.79 (br-d, 2H), 1.48-1.60 (m, 1H), 1.34 (d, 3H), 1.24 (d, 6H), 1.00-1.20 (m, 4H).

Molecular formula: $C_{20}H_{32}N_6O_3S$ Exact mass: 436.23 LC-MS (Pos, *m/z)* =437.15 $[M+H]^+$.

**Example 74: Synthesis of (*R*)-1-(8-(isopropylamino)-2-(((1*r*,4*R*)-4-((tetrahydro-2H-pyran-4-yl)amino)cyclohexyl)amino)pyrido[3,4-*d*]pyrimidin-6-yl)ethan-1-ol (compound 74)**

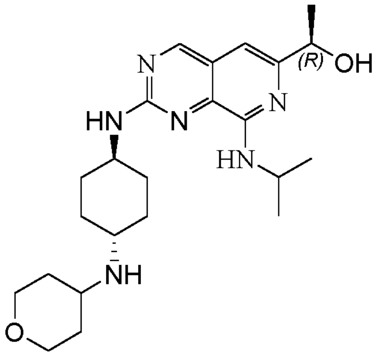

Steps:

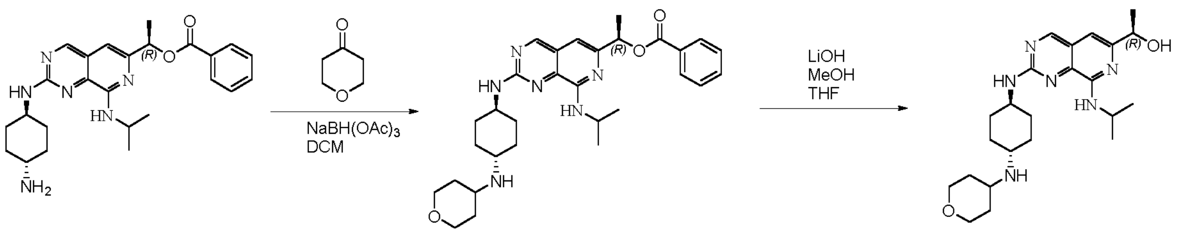


Step 1: synthesis of (*R*)-1-(8-(isopropylamino)-2-(((1*r*,4*R*)-4-((tetrahydro-2H-pyran-4-yl)amino)cyclohexyl)amino)pyrido[3,4-*d*]pyrimidin-6-yl)ethyl benzoate

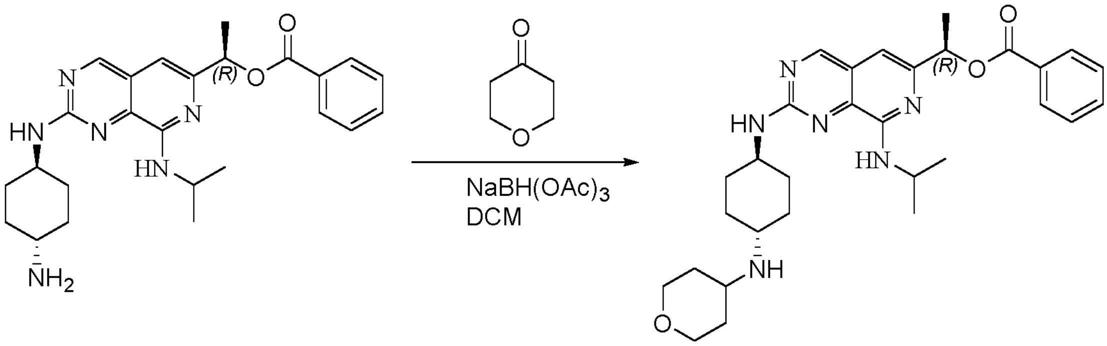


(*R*)-1-(2-(((1*r*,4*R*)-4-aminocyclohexyl)amino)-8-(isopropylamino)pyrido[3,4-*d*]pyrimidin-6-yl)ethyl benzoate (44.9 mg, 0.10 mmol, 1.0 eq.) and tetrahydropyran-4-one (12 mg, 0.120 mmol, 1.2 eq.) were dissolved in DCM (1 mL). After the reaction solution was stirred at room temperature for 30 min, the reaction solution was added with $NaBH(OAc)_3$ (53 mg, 0.250 mmol, 2.5 eq.) and reacted at room temperature for 3 h. When there were no materials left, as detected by LCMS, the reaction solution was added with sat-$NaHCO_3$ aq (5 mL) and extracted with ethyl acetate (20 mL × 2). The organic phases were combined, dried over anhydrous sodium sulfate and filtered, and the filtrate was concentrated under reduced pressure to give a crude product, which was used in the next step without further purification.

Step 2: synthesis of (*R*)-1-(8-(isopropylamino)-2-(((1*r*,4*R*)-4-((tetrahydro-2H-pyran-4-yl)amino)cyclohexyl)amino)pyrido[3,4-*d*]pyrimidin-6-yl)ethan-1-ol

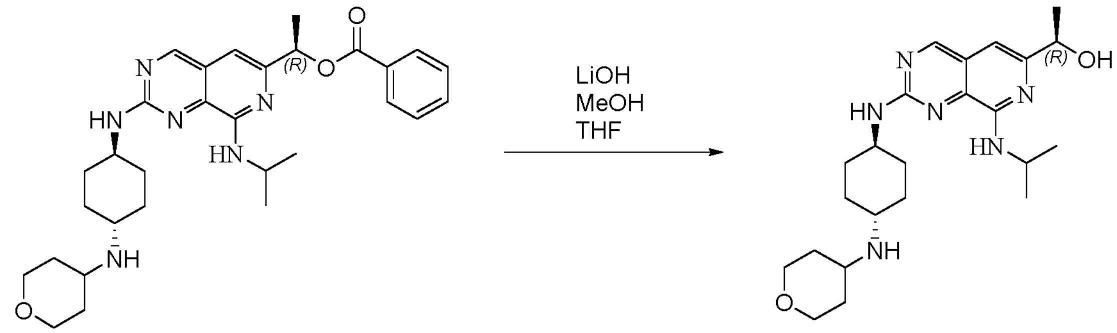


(*R*)-1-(8-(isopropylamino)-2-(((1*r*,4*R*)-4-((tetrahydro-2H-pyran-4-yl)amino)cyclohexyl)amino)pyrido[3,4-*d*]pyrimidin-6-yl)ethyl benzoate (0.10 mmol, 1.0 eq.) was dissolved in MeOH (1 mL) and THF (1 mL), and the reaction solution was added with 4M LiOH aq ( 0.075 mL, 0.30 mmol, 3.0 eq.). The mixture was stirred at room temperature for 3 h. When there were no materials left, as detected by LCMS, the reaction solution was added with water (3 mL) and extracted with ethyl acetate (30 mL × 2). The organic phases were combined, dried over anhydrous sodium sulfate and filtered, and the filtrate was concentrated under reduced pressure to give a crude product, which was purified by HPLC to give the product (17.5 mg, yield: 40.8%).
Molecular formula: $C_{23}H_{36}N_6O_2$ Exact mass: 428.29 LC-MS (Pos, *m*/*z*) =429.4 $[M+H]^+$.

**Example 75: Synthesis of (1*R*,4*r*)-4-((6-((*R*)-1-hydroxyethyl)-8-(isopropylamino)pyrido[3,4-*d*]pyrimidin-2-yl)amino)cyclohexan-1-ol (compound 75)**

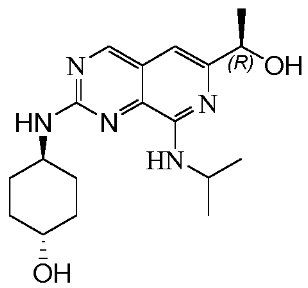

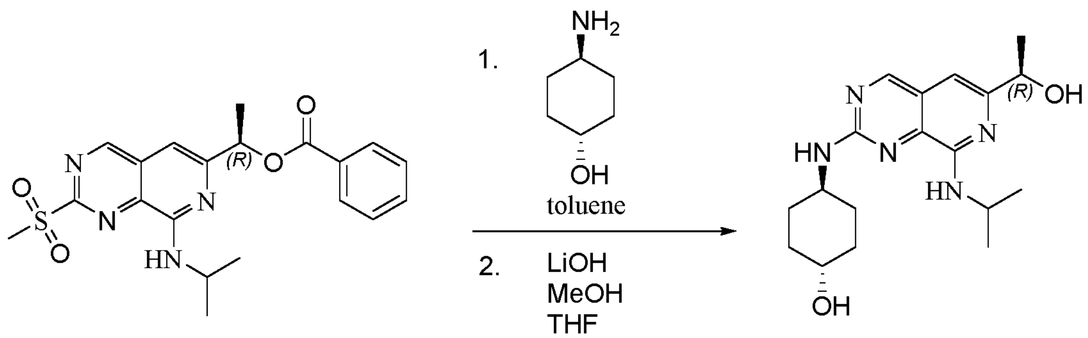


(*R*)-1-(8-(isopropylamino)-2-(methylsulfonyl)pyrido[3,4-*d*]pyrimidin-6-yl)ethyl benzoate (41 mg, 0.10 mmol, 1.0 eq.) was dissolved in toluene (0.5 mL), and the reaction solution was added with 4-aminocyclohexanol (46 mg, 0.40 mmol, 4.0 eq.) and reacted at 120 °C for 48 h. When there were no materials left, as detected by LCMS, the reaction solution was cool to room temperature and added with MeOH (0.5 mL), THF (0.5 mL) and 4M LiOH aq (0.10 mL, 0.40

mmol, 4.0 eq.). The mixture was stirred at room temperature for overnight. When there were no materials left, as detected by LCMS, the reaction solution was added with water (3 mL) and extracted with ethyl acetate (30 mL × 2). The organic phases were combined, dried over anhydrous sodium sulfate and filtered, and the filtrate was concentrated under reduced pressure to give a crude product, which was purified by HPLC to give the product (5.7 mg, yield: 17%). $^1$H-NMR ($CDCl_3$) δ(ppm): 8.79 (s, 1H), 6.56 (s, 1H), 6.22 (d, 1H), 5.10 (d, 1H), 4.74 (q, 1H), 4.30-4.36 (m, 1H), 3.85-3.90 (m, 1H), 3.65-3.77 (m, 1H), 2.16-2.22 (m, 2H), 2.04-2.08 (m, 2H), 1.44-1.60 (m, 5H), 1.30-1.47 (m, 8H).
Molecular formula: $C_{18}H_{27}N_5O_2$ Exact mass: 345.22 LC-MS (Pos, *m*/*z*) =346.3 $[M+H]^+$.

**Example 76: Synthesis of (*R*)-1-(2-(((1*r*,4*R*)-4-aminocyclohexyl)amino)-8-(isopropylamino)pyrido[3,4-*d*]pyrimidin-6-yl)ethan-1-ol (compound 76)**

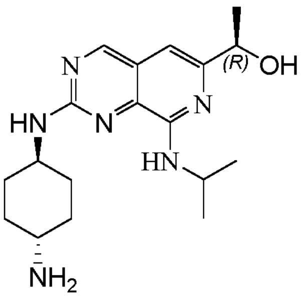

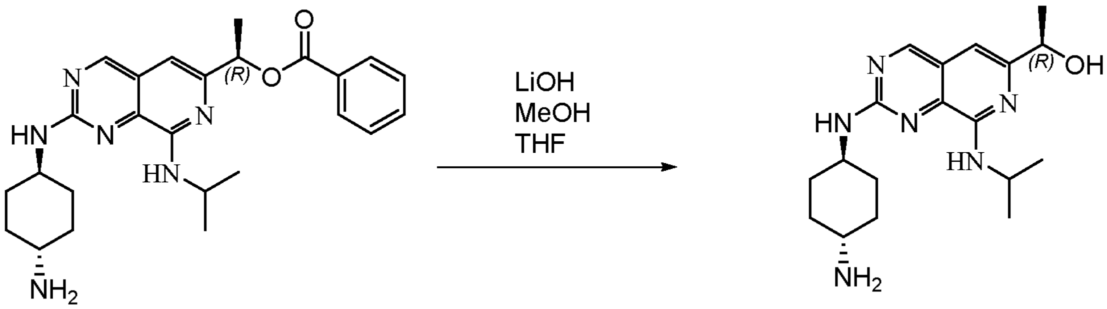


(*R*)-1-(2-(((1*r*,4*R*)-4-aminocyclohexyl)amino)-8-(isopropylamino)pyrido[3,4-*d*]pyrimidin-6-yl)ethyl benzoate (22 mg, 0.05 mmol, 1.0 eq.) was dissolved in MeOH (0.3 mL) and THF (0.3 mL), and the reaction solution was added with 4M LiOH aq ( 0.05 mL, 0.20 mmol, 4.0 eq.). The mixture was stirred at room temperature for 2 h. When there were no materials left, as detected by LCMS, the reaction solution was added with water (3 mL) and extracted with ethyl acetate (30 mL × 2). The organic phases were combined, dried over anhydrous sodium sulfate and filtered, and the filtrate was concentrated under reduced pressure to give a crude product, which was purified by HPLC to give the product (8.3 mg, yield:48%).
Molecular formula: $C_{18}H_{28}N_6O$ Exact mass: 344.23 LC-MS (Pos, *m*/*z*) =345.3 $[M+H]^+$.

**Example 77: Synthesis of (1*S*,3*R*)-3-((6-((*R*)-1-hydroxyethyl)-8-(isopropylamino)pyrido[3,4-*d*]pyrimidin-2-yl)amino)cyclopentane-1-carboxylic acid (compound 77)**

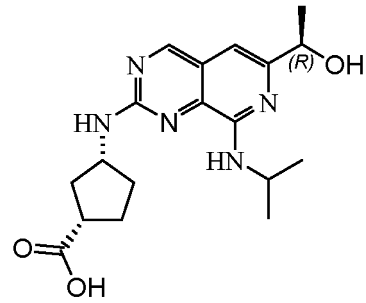

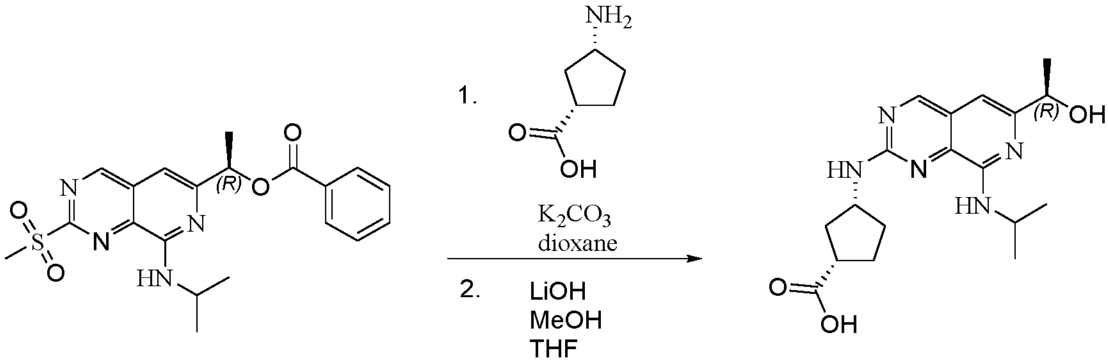


(*R*)-1-(8-(isopropylamino)-2-(methylsulfonyl)pyrido[3,4-*d*]pyrimidin-6-yl)ethyl benzoate (41 mg, 0.10 mmol, 1.0 eq.) was dissolved in dioxane (0.5 mL), and the reaction solution was added with (1*S*,3*R*)-3-aminocyclopentanecarboxylic acid (26 mg, 0.20 mmol, 2.0 eq.) and $K_2CO_3$ (55 mg, 0.40 mmol, 4.0 eq.). The mixture was reacted at 120 °C for 48 h. When there were no materials left, as detected by LCMS, the reaction solution was cool to room temperature and added with MeOH (0.5 mL), THF (0.5 mL) and 4M LiOH aq (0.10 mL, 0.40 mmol, 4.0 eq.). The mixture was stirred at room temperature for overnight. When there were no materials left, as detected by LCMS, the reaction solution was acidified with 4M HCl aq and extracted with a mixuture of ethyl acetate and THF (40 mL × 2). The organic phases were combined, dried over anhydrous sodium sulfate and filtered, and the filtrate was concentrated under reduced pressure to give a crude product, which was purified by HPLC to give the product (9.2 mg, yield: 20%).
Molecular formula: $C_{18}H_{25}N_5O_3$ Exact mass: 359.20 LC-MS (Pos, *m*/*z*) =360.2 $[M+H]^+$.

**Example 78: Synthesis of (1*R*,3*S*)-3-((6-((*R*)-1-hydroxyethyl)-8-(isopropylamino)pyrido[3,4-*d*]pyrimidin-2-yl)amino)cyclopentane-1-carboxylic acid (compound 78)**

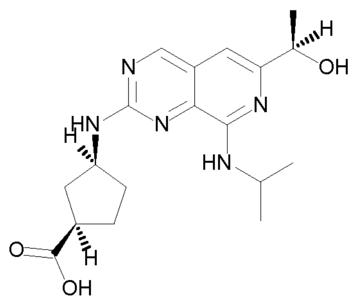

The titled compound was prepared as same as example 77 using corresponding chemicals.
Molecular formula: $C_{18}H_{25}N_5O_3$ Exact mass: 359.20 LC-MS (Pos, *m/z)* =360.19 $[M+H]^+$.

**Example 79: Synthesis of (1*S*,3*R*)-3-((6-((*R*)-1-hydroxyethyl)-8-(isopropylamino)pyrido[3,4-*d*]pyrimidin-2-yl)amino)-*N*-methylcyclopentane-1-carboxamide (compound 79)**

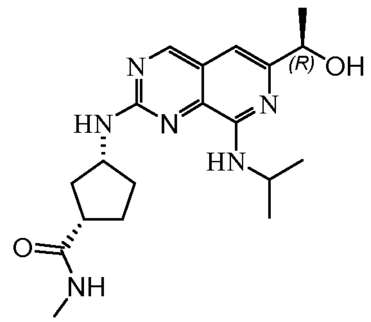

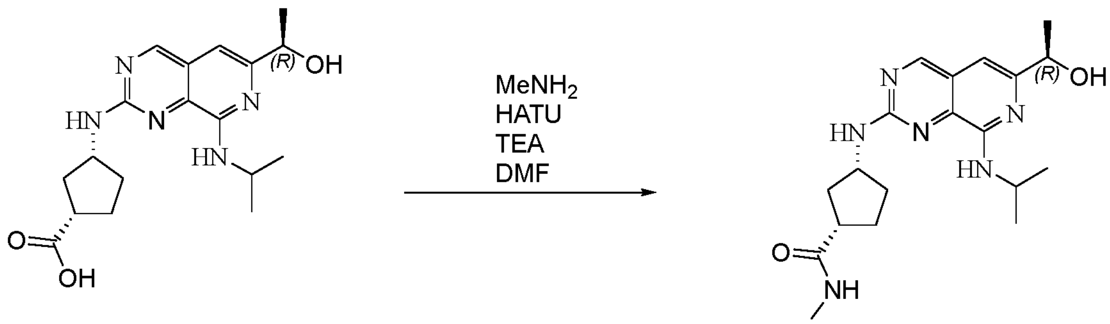


(1*S*,3*R*)-3-((6-((*R*)-1-hydroxyethyl)-8-(isopropylamino)pyrido[3,4-*d*]pyrimidin-2-yl)amino)cyclopentane-1-carboxylic acid (8.5 mg, 0.024 mmol, 1.0 eq.) and methylamine (2.9 mg, 0.028 mmol, 1.2 eq.) were dissolved in DMF (0.12 mL), and the reaction solution was added with HATU (12 mg, 0.031 mmol, 1.3 eq.) and TEA (0.01 mL, 0.071 mmol, 3.0 eq.). The mixture was stirred at room temperature for 1 h. When there were no materials left, as detected by LCMS, the reaction solution was added with water (3 mL) and extracted with ethyl acetate (30 mL × 2). The organic phases were combined, dried over anhydrous sodium sulfate and filtered, and the filtrate was concentrated under reduced pressure to give a crude product, which was purified by HPLC to give the product (7.1 mg, yield:81%).
$^1$H-NMR ($CDCl_3$) δ(ppm): 8.78 (s, 1H), 6.53 (br-s, 2H), 6.30 (s, 1H), 5.57 (s, 1H), 4.74 (d, 1H), 4.51 (d, 1H), 4.25-4.38 (m, 2H), 2.84 (s, 3H), 2.70-2.74 (m, 1H), 2.17-2.23 (m, 1H), 1.91-2.03 (m, 4H), 1.48 (d, 3H), 1.33 (d, 6H).
Molecular formula: $C_{19}H_{28}N_6O_2$ Exact mass: 372.23 LC-MS (Pos, *m*/*z*) =373.3 [M+H]$^+$.

**Example 80: Synthesis of (R)-1-(8-(isopropylamino)-2-(((1*r*,4*R*)-4-(oxetan-3-ylamino)cyclohexyl)amino)pyrido[3,4-*d*]pyrimidin-6-yl)ethan-1-ol (compound 80)**

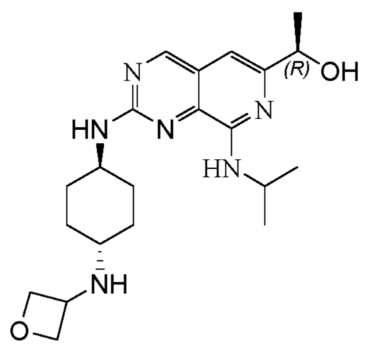

The titled compound was prepared as same as example 74 using corresponding chemicals.

$^1$H-NMR ($CDCl_3$) δ(ppm): 8.78 (s, 1H), 6.55 (s, 1H), 6.20 (d, 1H), 5.11 (d, 1H), 4.81-4.85 (m, 2H), 4.74 (q, 1H), 4.40-4.44 (m, 2H), 4.29-4.37 (m, 1H), 4.05-4.11 (m, 1H), 3.81-3.90 (m, 1H), 2.40-2.50 (m, 1H), 2.18-2.47 (m, 2H), 1.85-1.93 (m, 2H), 1.48 (d, 3H), 1.22-1.33 (m, 10H).

Molecular formula: $C_{21}H_{32}N_6O_2$ Exact mass: 400.26 LC-MS (Pos, *m*/*z*) =401.3 [M+H]$^+$.

**Example 81: Synthesis of (*R*)-*N*-(6-(1-hydroxyethyl)-8-(isopropylamino)pyrido[3,4-*d*]pyrimidin-2-yl)isobutyramide (compound 81)**

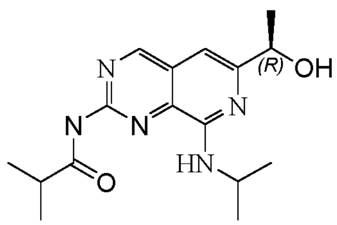

Steps:

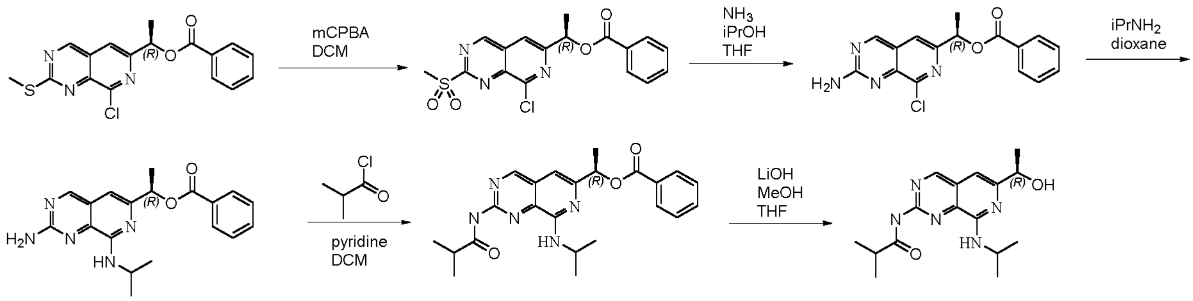


Step 1: synthesis of (*R*)-1-(2-(((1-acetylpiperidin-4-yl)methyl)amino)-8-(isopropylamino)pyrido[3,4-*d*]pyrimidin-6-yl)ethyl benzoate

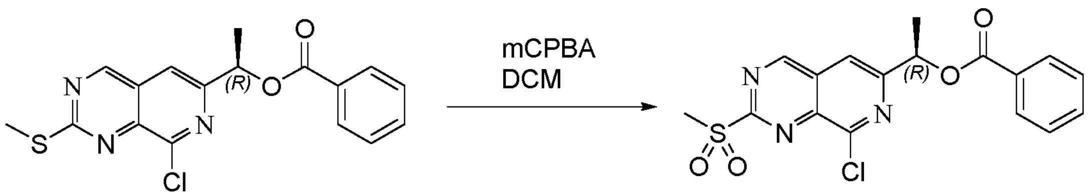


(*R*)-1-(8-chloro-2-(methylthio)pyrido[3,4-*d*]pyrimidin-6-yl)ethyl benzoate (3000 mg, 8.33 mmol, 1.0 eq.) was dissolved in DCM (83 mL), and the reaction solution was added with mCPBA (4317 mg, 17.5 mmol, 2.1 eq.) at 0 °C and reacted at room temperature for overnight. When there were no materials left, as detected by LCMS, the reaction solution was added with sat-NaHCO3 aq (100 mL) and extracted with DCM (150 mL × 2). The organic phases were combined, dried over anhydrous sodium sulfate and filtered, and the filtrate was concentrated under reduced pressure to give a crude product, which was purified by silica gel column chromatography to give the product (2350 mg, yield: 71.9%).

Step 2: synthesis of (*R*)-1-(2-amino-8-chloropyrido[3,4-*d*]pyrimidin-6-yl)ethyl benzoate

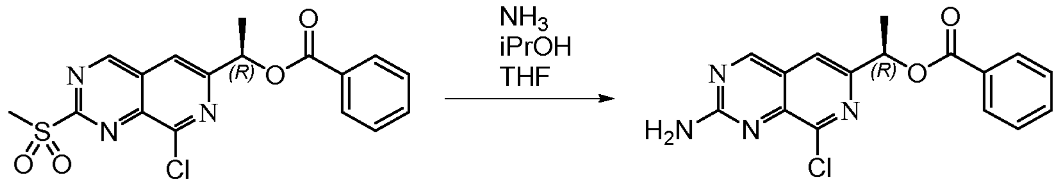


(*R*)-1-(2-(((1-acetylpiperidin-4-yl)methyl)amino)-8-(isopropylamino)pyrido[3,4-*d*]pyrimidin-6-yl)ethyl benzoate (274 mg, 0.70 mmol, 1.0 eq.) was dissolved in THF (83 mL), and the reaction solution was added with 2M $NH_3$ in isopropanol (0.39 mL, 0.77 mmol, 1.1 eq.) and reacted at room temperature for overnight. When there were no materials left, as detected by LCMS, the reaction solution was concentrated under reduced pressure to give a crude product, which was used in the next step without further purification.

Step 3: synthesis of (*R*)-1-(2-amino-8-(isopropylamino)pyrido[3,4-*d*]pyrimidin-6-yl)ethyl benzoate

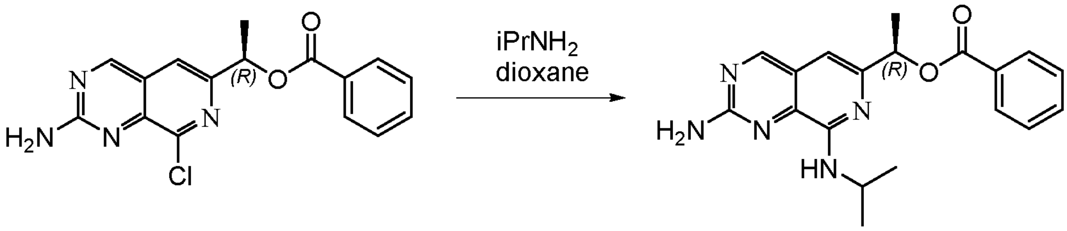


(R*)*-1-(2-amino-8-chloropyrido[3,4-*d*]pyrimidin-6-yl)ethyl benzoate (0.70 mmol, 1.0 eq.) was dissolved in dioxane (5 mL), and the reaction solution was added with propan-2-amine (1.0 mL) and reacted at 120 °C for overnight. When there were no materials left, as detected by LCMS, the reaction solution was concentrated under reduced pressure to give a crude product, which was purified by silica gel column chromatography to give the product (213 mg, yield: 86.6%).

Step 4: synthesis of (*R*)-1-(2-isobutyramido-8-(isopropylamino)pyrido[3,4-*d*]pyrimidin-6-yl)ethyl benzoate

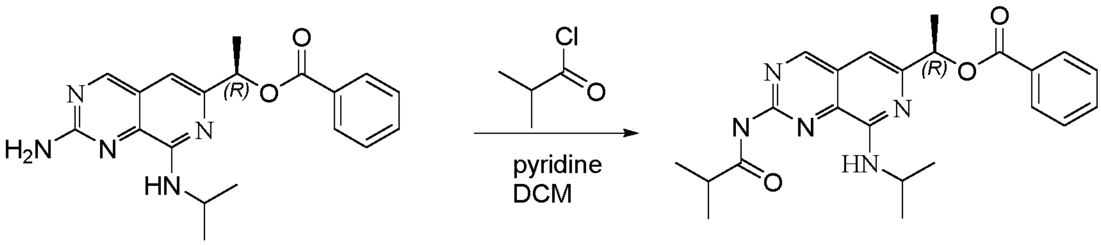


(*R*)-1-(2-amino-8-(isopropylamino)pyrido[3,4-*d*]pyrimidin-6-yl)ethyl benzoate (24.6 mg, 0.070 mmol, 1.0 eq.) was dissolved in DCM (0.7 mL), and the reaction solution was added with 2-methylpropanoyl chloride (0.0089 mL, 00.84 mmol, 1.2 eq.) and pyridine (0.011 mL, 0.14 mmol, 2.0 eq.) at 0 °C and reacted at room temperature. When there were no materials left, as detected by LCMS, the reaction solution was added with sat-NaHCO3 aq (2 mL) and extracted with ethyl acetate (20 mL × 2). The organic phases were combined, dried over anhydrous sodium sulfate and filtered, and the filtrate was concentrated under reduced pressure to give a crude product, which was used in the next step without further purification.

Step 5: synthesis of (*R*)-*N*-(6-(1-hydroxyethyl)-8-(isopropylamino)pyrido[3,4-*d*]pyrimidin-2-yl)isobutyramide

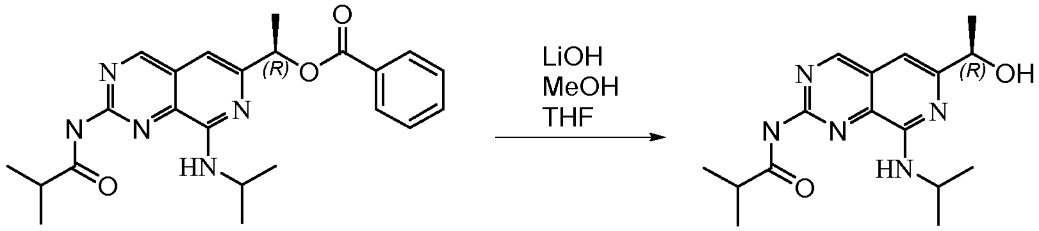


(*R*)-1-(2-isobutyramido-8-(isopropylamino)pyrido[3,4-*d*]pyrimidin-6-yl)ethyl benzoate (0.07 mmol, 1.0 eq.) was dissolved in MeOH (0.7 mL) and THF (0.7 mL), and the reaction solution was added with 4M LiOH aq ( 0.07 mL, 0.28 mmol, 4.0 eq.). The mixture was stirred at room temperature for 2 h. When there were no materials left, as detected by LCMS, the reaction solution was concentrated under reduced pressure to give a crude product, which was purified by silica gel column chromatography and HPLC to give the product (3.9 mg, yield:17%).
Molecular formula: $C_{16}H_{23}N_5O_2$ Exact mass: 317.19 LC-MS (Pos, *m/z*) =318.2 [M+H]$^+$.

**Example 82: Synthesis of (*R*)-1-(2-((((1*r*,4*R*)-4-aminocyclohexyl)methyl)amino)-8-(isopropylamino)pyrido[3,4-*d*]pyrimidin-6-yl)ethan-1-ol (compound 82)**

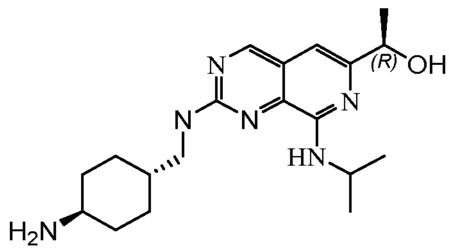

Steps:

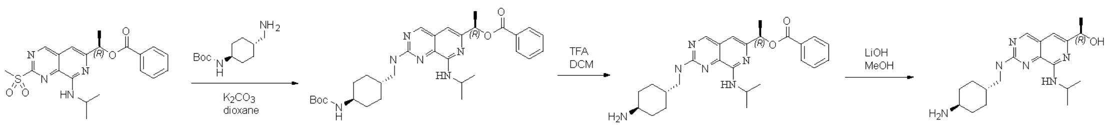


Step 1: synthesis of (*R*)-1-(2-((((1*r*,4*R*)-4-((*tert*-butoxycarbonyl)amino)cyclohexyl)methyl)amino)-8-(isopropylamino)pyrido[3,4-*d*]pyrimidin-6-yl)ethyl benzoate

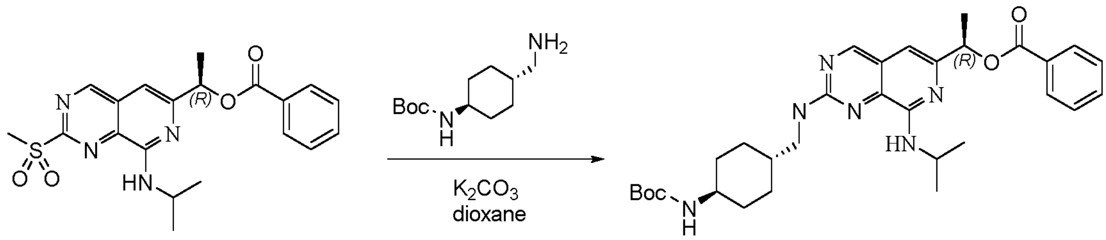


(*R*)-1-(8-(isopropylamino)-2-(methylsulfonyl)pyrido[3,4-*d*]pyrimidin-6-yl)ethyl benzoate (750 mg, 1.81 mmol, 1.0 eq.) was dissolved in dioxane (5 mL), and the reaction solution was added with *tert*- butyl *N*-[4-(aminomethyl)cyclohexyl]carbamate (830 mg, 3.64 mmol, 2.0 eq.) and $K_2CO_3$ (500 mg, 3.64 mmol, 2.0 eq.) and reacted at 100 °C for 3 h. When there were no materials left, as detected by LCMS, the reaction solution was added with water and extracted

with ethyl acetate twice. The organic phases were combined, dried over anhydrous sodium sulfate and filtered, and the filtrate was concentrated under reduced pressure to give a crude product, which was purified by silica gel column chromatography to give the product (694 mg, yield: 68.2%).

Step 2: synthesis of (*R*)-1-(2-((((1*r*,4*R*)-4-aminocyclohexyl)methyl)amino)-8-(isopropylamino)pyrido[3,4-*d*]pyrimidin-6-yl)ethyl benzoate

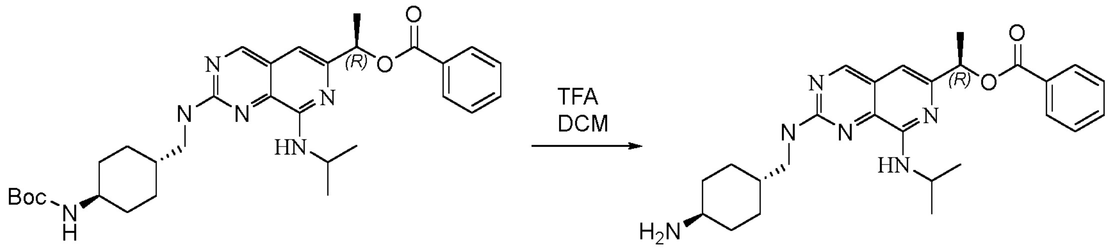


(*R*)-1-(2-((((1*r*,4*R*)-4-((*tert*-butoxycarbonyl)amino)cyclohexyl)methyl)amino)-8-(isopropylamino)pyrido[3,4-*d*]pyrimidin-6-yl)ethyl benzoate (694 mg, 1.23 mmol, 1.0 eq.) was dissolved in DCM (5 mL). The reaction solution was added dropwise with TFA (1 mL) and reacted at room temperature for overnight. When there were no materials left, as detected by LCMS, the reaction solution was concentrated under reduced pressure to give a crude product, which was used in the next step without further purification.

Step 3: synthesis of (*R*)-1-(2-((((1*r*,4*R*)-4-aminocyclohexyl)methyl)amino)-8-(isopropylamino)pyrido[3,4-*d*]pyrimidin-6-yl)ethan-1-ol

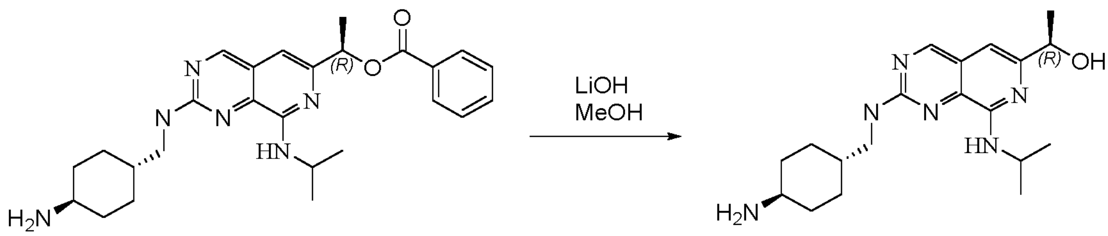


(*R*)-1-(2-((((1*r*,4*R*)-4-aminocyclohexyl)methyl)amino)-8-(isopropylamino)pyrido[3,4-*d*]pyrimidin-6-yl)ethyl benzoate (32 mg, 0.069 mmol, 1.0 eq.) was dissolved in MeOH (2 mL), and the reaction solution was added with 4M LiOH aq (0.2 mL, 0.80 mmol, 12.0 eq.). The mixture was stirred at room temperature for 2 h. When there were no materials left, as detected by LCMS, the reaction solution was concentrated under reduced pressure to give a crude product, which was purified by HPLC to give the product (21 mg, yield:84.7%).
Molecular formula: $C_{19}H_{30}N_6O$ Exact mass: 358.25 LC-MS (Pos, *m*/*z*) =359.3 $[M+H]^+$.

**Example 83: Synthesis of (*R*)-*N*-(7-(1-hydroxyethyl)-5-(piperidin-1-yl)-2,6-naphthyridin-3-yl)isobutyramide (compound 83)**

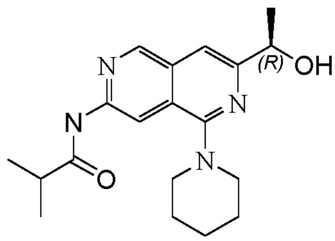

Steps:

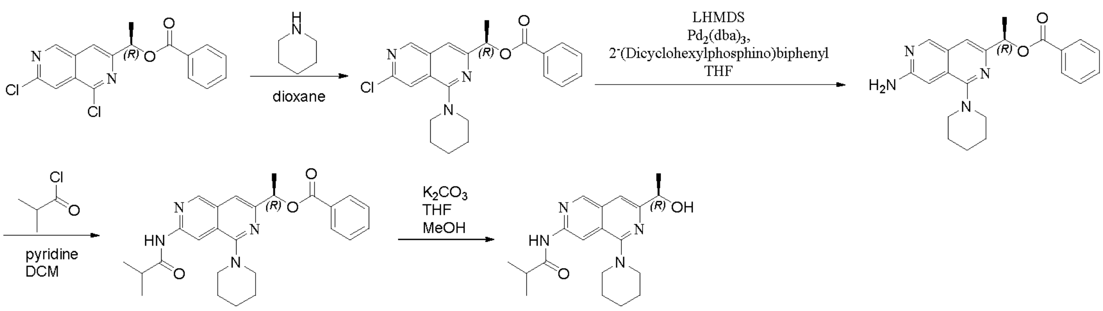


Step 1: synthesis of (*R*)-1-(7-chloro-1-(piperidin-1-yl)-2,6-naphthyridin-3-yl)ethyl benzoate

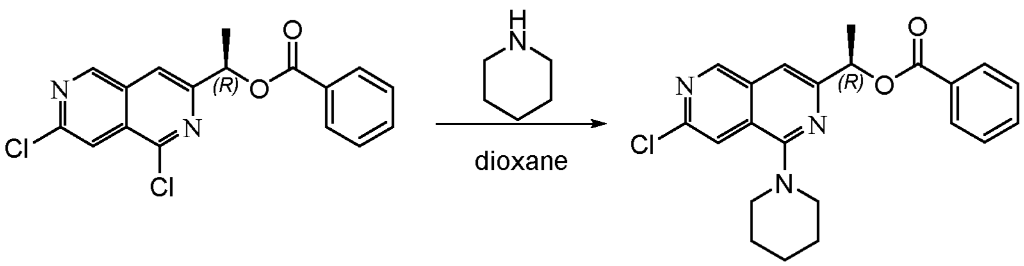


(*R*)-1-(1,7-dichloro-2,6-naphthyridin-3-yl)ethyl benzoate (278 mg, 0.80 mmol, 1.0 eq.) was dissolved in dioxane (5 mL), and the reaction solution was added with piperidine (0.5 mL) and reacted at 100 °C. When there were no materials left, as detected by LCMS, the reaction solution was concentrated under reduced pressure to give a crude product, which was purified by silica gel column chromatography to give the product (308 mg, yield: 97.2%).

Step 2: synthesis of (*R*)-1-(7-amino-1-(piperidin-1-yl)-2,6-naphthyridin-3-yl)ethyl benzoate

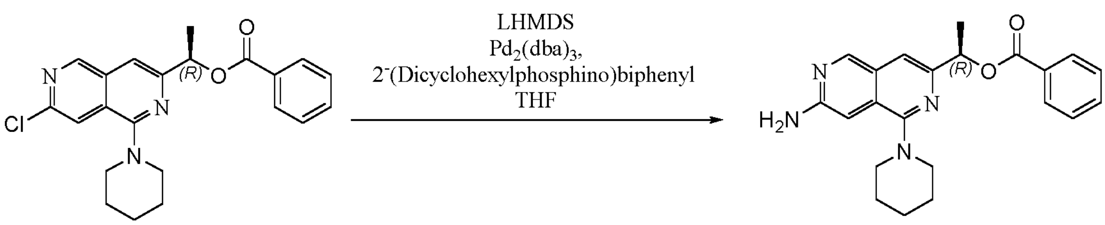


(*R*)-1-(7-chloro-1-(piperidin-1-yl)-2,6-naphthyridin-3-yl)ethyl benzoate (72.2 mg, 0.182 mmol, 1.0 eq.) was dissolved in THF (0.9 mL), and the reaction solution was added with $Pd_2(dba)_3$, (12.8 mg, 0.018 mmol, 0.1 eq.), 2-(Dicyclohexylphosphino)biphenyl (12.8 mg, 0.037 mmol, 0.2 eq.) and 1.3M LHMDS in THF (0.17 mL, 0.22 mmol, 1.2 eq.) and reacted at 65 °C. When there were no materials left, as detected by LCMS, the reaction solution was added with 1M HCl aq (0.2 mL) and stirred at room temperature for 20 min. The mixture was added with water (5 mL) and extracted with ethyl acetate (40 mL × 2). The organic phases were combined, dried over anhydrous sodium sulfate and filtered, and the filtrate was concentrated under reduced pressure to give a crude product, which was purified by silica gel column chromatography to give the product (22 mg, yield: 32.2%).

Step 3: synthesis of (*R*)-1-(7-isobutyramido-1-(piperidin-1-yl)-2,6-naphthyridin-3-yl)ethyl benzoate

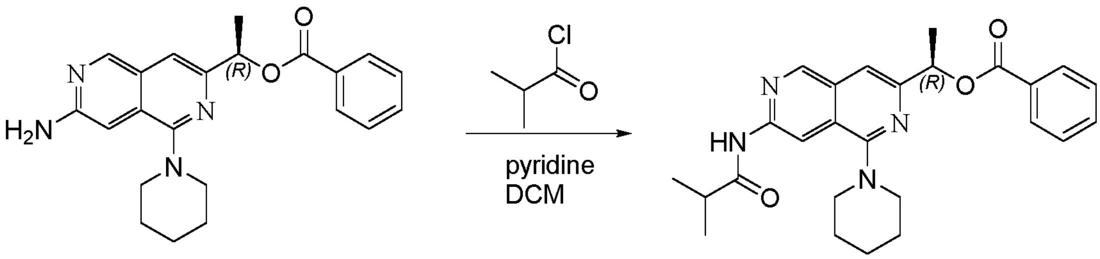


(*R*)-1-(7-amino-1-(piperidin-1-yl)-2,6-naphthyridin-3-yl)ethyl benzoate (22.2 mg, 0.059 mmol, 1.0 eq.) was dissolved in DCM (0.59 mL), and the reaction solution was added with 2-methylpropanoyl chloride (0.0075 mL, 0.071 mmol, 1.2 eq.) and pyridine (0.014 mL, 0.18 mmol, 3.0 eq.) at 0 °C and reacted at room temperature for 1 h. When there were no materials left, as detected by LCMS, the reaction solution was added with sat-NaHCO3 aq (2 mL) and extracted with ethyl acetate (20 mL × 2). The organic phases were combined, dried over anhydrous sodium sulfate and filtered, and the filtrate was concentrated under reduced pressure to give a crude product, which was used in the next step without further purification.

Step 4: synthesis of (*R*)-*N*-(7-(1-hydroxyethyl)-5-(piperidin-1-yl)-2,6-naphthyridin-3-yl)isobutyramide

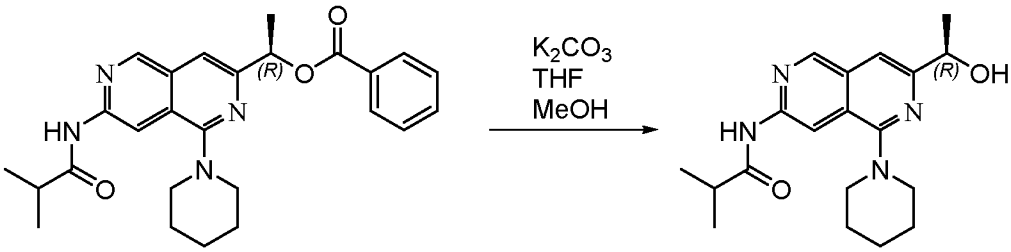


(*R*)-1-(7-isobutyramido-1-(piperidin-1-yl)-2,6-naphthyridin-3-yl)ethyl benzoate (0.059 mmol, 1.0 eq.) was dissolved in MeOH (0.59 mL) and THF (0.59 mL), and the reaction solution was added with $K_2CO_3$ (24.5 mg, 0.18 mmol, 3.0 eq.). The mixture was stirred at room temperature for overnight. When there were no materials left, as detected by LCMS, the reaction solution was passed through filter and concentrated under reduced pressure to give a crude product, which was purified by HPLC to give the product (15.2 mg, yield:74.8%).
$^1$H-NMR ($CDCl_3$) δ(ppm): 8.83 (s, 1H), 8.72 (s, 1H), 8.07 (s, 1H), 7.06 (s, 1H), 4.84-4.88 (m, 1H), 3.48-3.51 (m, 4H), 2.57-2.64 (m, 1H), 1.80-1.86 (m, 4H), 1.70-1.73 (m, 2H), 1.53 (d, 3H), 1.30 (d, 6H).
Molecular formula: $C_{19}H_{26}N_4O_2$ Exact mass: 342.21 LC-MS (Pos, *m*/*z*) =343.3 $[M+H]^+$.

**Example 84: Synthesis of (*R*)-1-(8-(isopropylamino)-2-((piperidin-4-ylmethyl)amino)pyrido[3,4-*d*]pyrimidin-6-yl)ethan-1-ol (compound 84)**

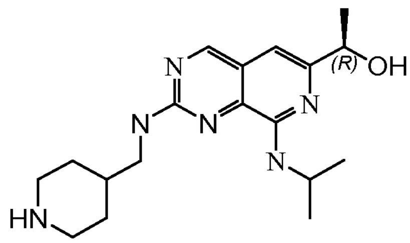

The titled compound was prepared as same as example 82 using corresponding chemicals.
$^1$H-NMR ($CDCl_3$) δ(ppm): 8.76 (s, 1H), 6.54 (s, 1H), 6.22 (d, 1H), 5.45 (br-s, 1H), 4.72 (q, 1H), 4.28-4.37 (m, 1H), 3.37 (br-s, 2H), 3.08-3.11 (m, 2H), 2.58-2.65 (m, 2H), 1.44-1.49 (m, 3H), 1.47 (d, 3H), 1.22-1.36 (m, 8H).

Molecular formula: $C_{18}H_{28}N_6O$ Exact mass: 344.23 LC-MS (Pos, *m*/*z*) =345.3 $[M+H]^+$.

**Example 85: Synthesis of (*R*)-3-((6-(1-hydroxyethyl)-8-(isopropylamino)pyrido[3,4-*d*]pyrimidin-2-yl)amino)propan-1-ol (compound 85)**

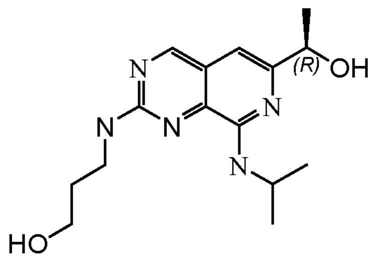

The titled compound was prepared as same as example 75 using corresponding chemicals.

$^1$H-NMR ($CDCl_3$) δ(ppm): 8.80 (s, 1H), 6.57 (s, 1H), 6.19 (br-s, 1H), 5.49 (br-s, 1H), 4.72-4.76 (m, 1H), 4.32-4.38 (m, 1H), 4.17 (br-s, 1H), 3.56-3.75 (m, 4H), 1.84-1.90 (m, 2H), 1.49 (d, 3H), 1.32 (d, 6H).

Molecular formula: $C_{15}H_{23}N_5O_2$ Exact mass: 305.19 LC-MS (Pos, *m*/*z*) =306.3 $[M+H]^+$.

**Example 86: Synthesis of (*R*)-*N*-(7-((*R*)-1-hydroxyethyl)-5-(isopropylamino)-2,6-naphthyridin-3-yl)piperidine-3-carboxamide (compound 86)**

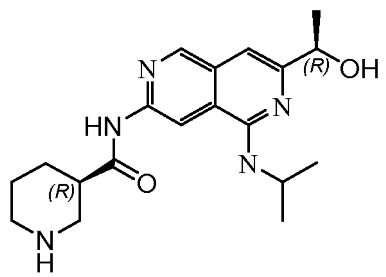

Steps:

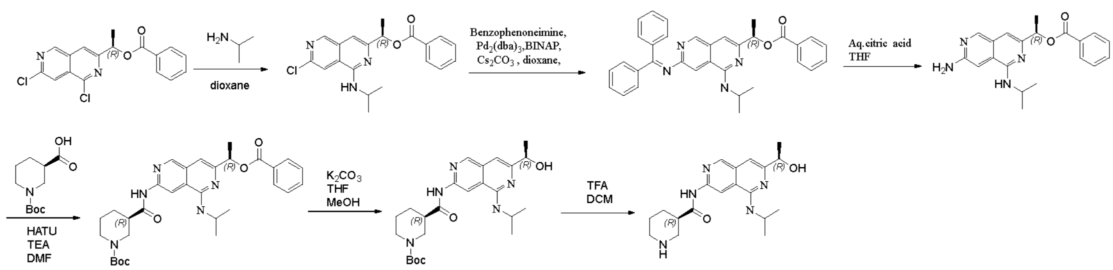


Step 1: synthesis of (*R*)-1-(7-chloro-1-(isopropylamino)-2,6-naphthyridin-3-yl)ethyl benzoate

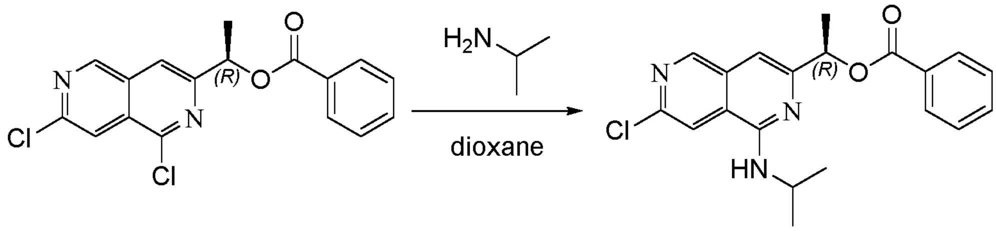


(*R*)-1-(1,7-dichloro-2,6-naphthyridin-3-yl)ethyl benzoate (1000 mg, 2.88 mmol, 1.0 eq.) was dissolved in dioxane (7 mL), and the reaction solution was added with propan-2-amine (1.5 mL) and reacted at 115 °C for overnight. When there were no materials left, as detected by LCMS, the reaction solution was concentrated under reduced pressure to give a crude product, which was purified by silica gel column chromatography to give the product (952 mg, yield: 89.5%).

Step 2: synthesis of (*R*)-1-(7-((diphenylmethylene)amino)-1-(isopropylamino)-2,6-naphthyridin-3-yl)ethyl benzoate

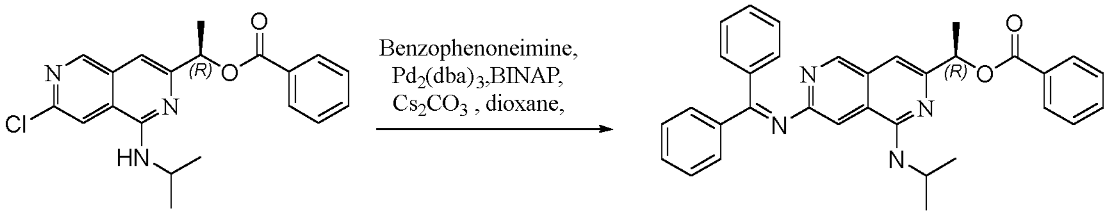


(*R*)-1-(7-chloro-1-(isopropylamino)-2,6-naphthyridin-3-yl)ethyl benzoate (100 mg, 0.27 mmol, 1.0 eq.), diphenylmethanimine (0.054 mL, 0.32 mmol, 1.2 eq.) was dissolved in dioxane (1.4 mL), and the reaction solution was added with $Pd_2(dba)_3$ (25 mg, 0.027 mmol, 0.1 eq.), BINAP (34 mg, 0.054 mmol, 0.2 eq.) and cesium carbonate (264 mg, 0.81 mmol, 3.0 eq.). The mixture was reacted at 120 °C for overnight in a sealed tube in nitrogen atmosphere. When there were no materials left, as detected by LCMS, the reaction solution was added with ethyl acetate (80 mL), stirred for 5 min and filtered, the filter cake was rinsed with ethyl acetate, and the filtrate was concentrated under reduced pressure to give a crude product, which was used in the next step without further purification.

Step 3: synthesis of (R)-1-(7-amino-1-(isopropylamino)-2,6-naphthyridin-3-yl)ethyl benzoate

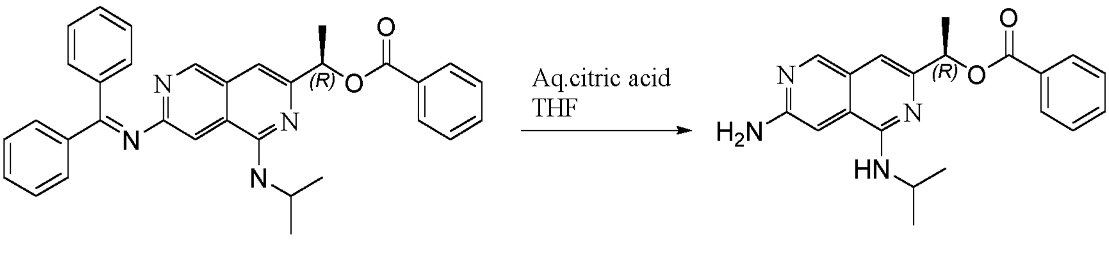


(*R*)-1-(7-((diphenylmethylene)amino)-1-(isopropylamino)-2,6-naphthyridin-3-yl)ethyl benzoate (0.27 mmol, 1.0 eq.) was dissolved in THF (2.7 mL). The reaction solution was added with 2N aqueous citric acid solution (0.68 mL) and reacted at room temperature for overnight. When there were no materials left, as detected by LCMS, the reaction solution was added with sat-NaHCO3 aq (2 mL) and extracted with ethyl acetate (20 mL × 2). The organic phases were combined, dried over anhydrous sodium sulfate and filtered, and the filtrate was concentrated under reduced pressure to give a crude product, which was purified by amino column chromatography to give the product (43.2 mg, yield: 45.6%)

Step 4: synthesis of *tert*-butyl (*R*)-3-((7-((*R*)-1-(benzoyloxy)ethyl)-5-(isopropylamino)-2,6-naphthyridin-3-yl)carbamoyl)piperidine-1-carboxylate

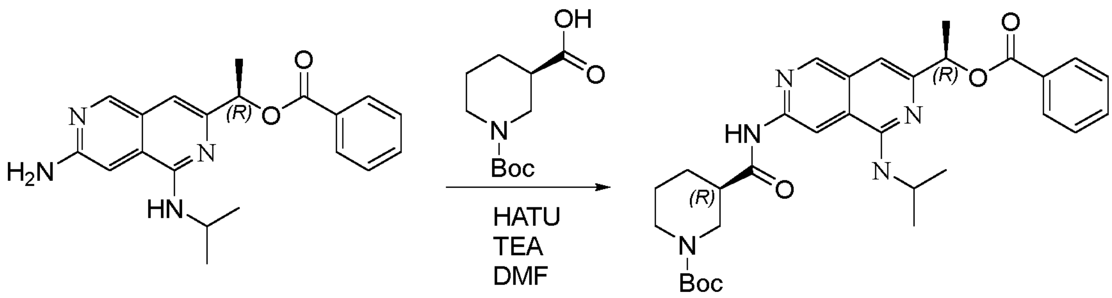


($R$)-1-(7-amino-1-(isopropylamino)-2,6-naphthyridin-3-yl)ethyl benzoate (43.2 mg, 0.123 mmol, 1.0 eq.) and (3$R$)-1-*tert*-butoxycarbonylpiperidine-3-carboxylic acid (34 mg, 0.148 mmol, 1.2 eq.) were dissolved in DCM (0.6 mL), and the reaction solution was added with HATU (56 mg, 0.15 mmol, 1.2 eq.) and TEA (0.026 mL, 0.19 mmol, 1.5 eq.). The mixture was stirred at room temperature for overnight. When there were no materials left, as detected by LCMS, the reaction solution was added with water (5 mL) and extracted with ethyl acetate (20 mL × 2). The organic phases were combined, dried over anhydrous sodium sulfate and filtered, and the filtrate was concentrated under reduced pressure to give a crude product, which was used in the next step without further purification.

Step 5: synthesis of *tert*-butyl ($R$)-3-((7-(($R$)-1-hydroxyethyl)-5-(isopropylamino)-2,6-naphthyridin-3-yl)carbamoyl)piperidine-1-carboxylate

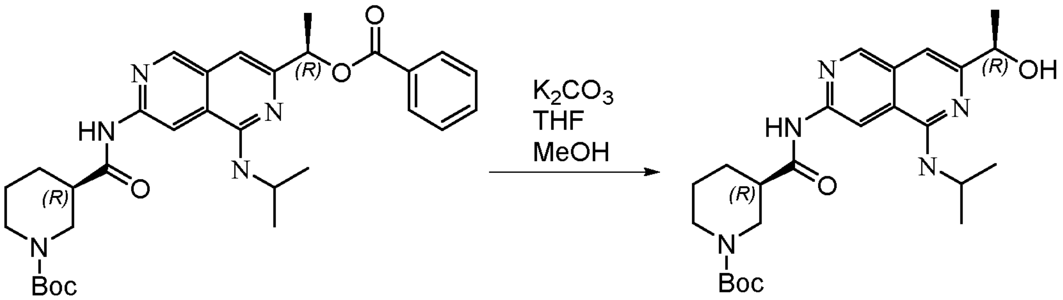


*tert*-butyl ($R$)-3-((7-(($R$)-1-(benzoyloxy)ethyl)-5-(isopropylamino)-2,6-naphthyridin-3-yl)carbamoyl)piperidine-1-carboxylate (0.123 mmol, 1.0 eq.) was dissolved in MeOH (2 mL) and THF (2 mL), and the reaction solution was added with $K_2CO_3$ (100 mg, 0.723 mmol, 5.9 eq.). The mixture was stirred at room temperature for overnight. When there were no materials left, as detected by LCMS, the reaction solution was concentrated under reduced pressure, added with water (5 mL) and extracted with ethyl acetate (20 mL × 2). The organic phases were combined, washed with brine, dried over anhydrous sodium sulfate and filtered, and the filtrate was concentrated under reduced pressure to give a crude product, which was used in the next step without further purification.

Step 6: synthesis of ($R$)-$N$-(7-(($R$)-1-hydroxyethyl)-5-(isopropylamino)-2,6-naphthyridin-3-yl)piperidine-3-carboxamide

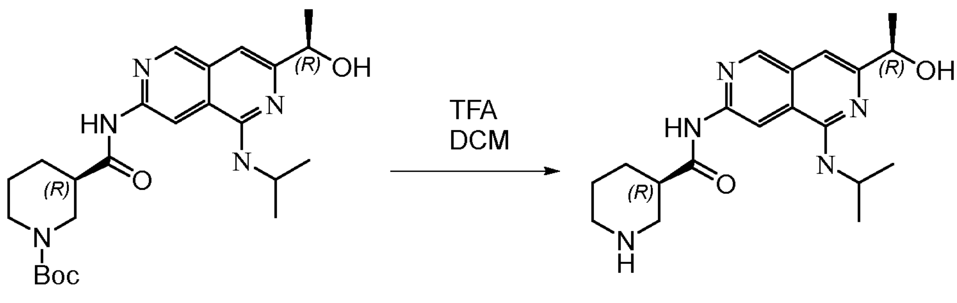

*tert*-butyl (*R*)-3-((7-((*R*)-1-hydroxyethyl)-5-(isopropylamino)-2,6-naphthyridin-3-yl)carbamoyl)piperidine-1-carboxylate (0.123 mmol, 1.0 eq.) was dissolved in DCM (1 mL). The reaction solution was added dropwise with TFA(1 mL) and reacted at room temperature for 2 h. When there were no materials left, as detected by LCMS, the reaction solution was concentrated under reduced pressure to give a crude product, which was purified by HPLC to give the product (10.7 mg, yield:24.3%).
Molecular formula: $C_{19}H_{27}N_5O_2$ Exact mass: 357.22 LC-MS (Pos, *m*/*z*) =358.19 [M+H]+.

**Example 87: Synthesis of (1*r*,4*r*)-4-((6-cyclopropyl-8-(isopropylamino)pyrido[3,4-*d*]pyrimidin-2-yl)amino)cyclohexan-1-ol (compound 87)**

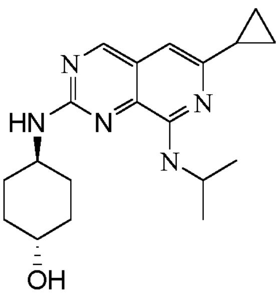

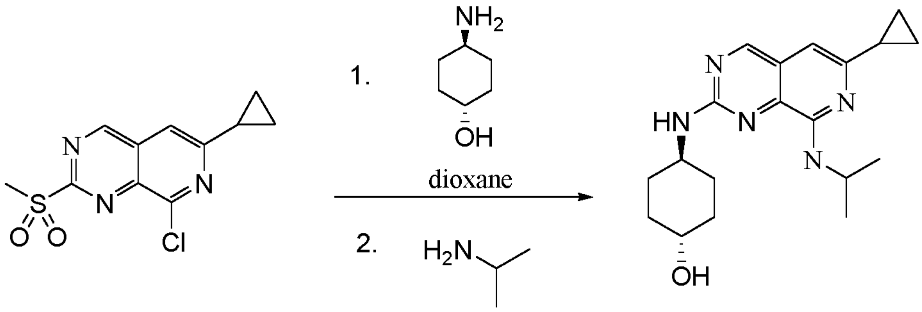


8-chloro-6-cyclopropyl-2-(methylsulfonyl)pyrido[3,4-*d*]pyrimidine (50 mg, 0.177 mmol, 1.0 eq.) was dissolved in dioxane (0.89 mL), and the reaction solution was added with 4-aminocyclohexanol (61 mg, 0.53 mmol, 3.0 eq.) and reacted at room temperature. When there were no materials left, as detected by LCMS, the reaction solution was added with propan-2-amine (0.88 mL) and reacted at 100 °C for overnight. When there were no materials left, as detected by LCMS, the reaction solution was was concentrated under reduced pressure to give a crude product, which was purified by HPLC to give the product (12.3 mg, yield: 20.4%).
Molecular formula: $C_{19}H_{27}N_5O$ Exact mass: 341.22 LC-MS (Pos, *m*/*z*) =342.3 [M+H]+.

**Example 88: Synthesis of 1-(2-(((1*r*,4*r*)-4-hydroxycyclohexyl)amino)-8-(isopropylamino)pyrido[3,4-*d*]pyrimidin-6-yl)ethan-1-one (compound 88)**

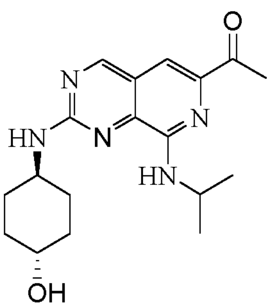

Steps:

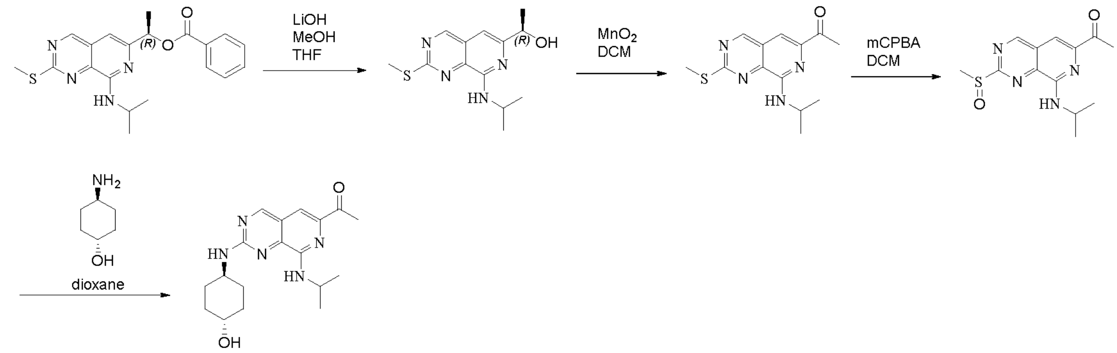


Step 1: synthesis of (R)-1-(8-(isopropylamino)-2-(methylthio)pyrido[3,4-*d*]pyrimidin-6-yl)ethan-1-ol

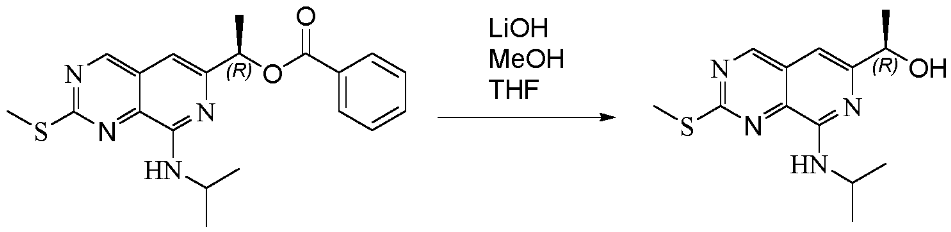


(*R*)-1-(8-(isopropylamino)-2-(methylthio)pyrido[3,4-*d*]pyrimidin-6-yl)ethyl benzoate (1595 mg, 4.17 mmol, 1.0 eq.) was dissolved in MeOH (10 mL) and THF (10 mL), and the reaction solution was added with 4M LiOH aq (1.1 mL, 4.4 mmol, 1.1 eq.). The mixture was stirred at room temperature for 3 h. When there were no materials left, as detected by LCMS, the reaction solution was added with water (20 mL) and extracted with ethyl acetate (100 mL × 2). The organic phases were combined, dried over anhydrous sodium sulfate and filtered, and the filtrate was concentrated under reduced pressure to give a crude product, which was purified by silica gel column chromatography to give the product (1050 mg, yield: 90.5%).

Step 2: synthesis of 1-(8-(isopropylamino)-2-(methylthio)pyrido[3,4-*d*]pyrimidin-6-yl)ethan-1-one

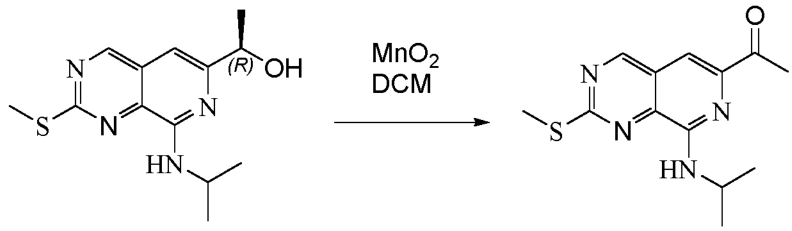


(*R*)-1-(8-(isopropylamino)-2-(methylthio)pyrido[3,4-*d*]pyrimidin-6-yl)ethan-1-ol (1050 mg, 3.77 mmol, 1.0 eq.) was dissolved in DCM (30 mL), and the reaction solution was added with $MnO_2$ (16.3 g, 189 mmol, 50 eq.). The mixture was stirred at room temperature for 5 h. When there were no materials left, as detected by LCMS, the reaction solution was passed through Celite pad and concentrated under reduced pressure to give a crude product, which was purified by silica gel column chromatography to give the product (849 mg, yield: 81.4%).

Step 3: synthesis of 1-(8-(isopropylamino)-2-(methylsulfinyl)pyrido[3,4-*d*]pyrimidin-6-yl)ethan-1-one

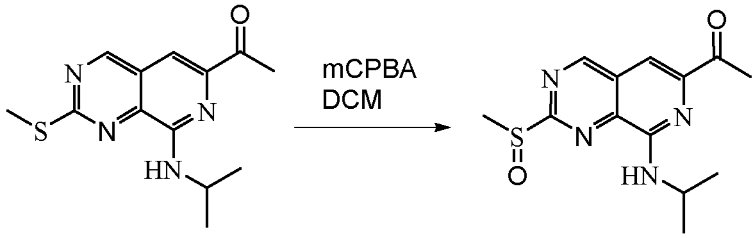


1-(8-(isopropylamino)-2-(methylthio)pyrido[3,4-*d*]pyrimidin-6-yl)ethan-1-one (100 mg, 0.276 mmol, 1.0 eq.) was dissolved in DCM (6 mL), and the reaction solution was added with mCPBA (98 mg, 0.40 mmol, 1.1 eq.) at 0 °C and reacted at 0 °C for 3 h. When there were no materials left, as detected by LCMS, the reaction solution was passed through Celite pad. The solution was added with sat-NaHCO3 aq (10 mL) and extracted with ethyl acetate (40 mL × 2). The organic phases were combined, dried over anhydrous sodium sulfate and filtered, and the filtrate was concentrated under reduced pressure to give a crude product, which was used in the next step without further purification.

Step 4: synthesis of 1-(2-(((1*r*,4*r*)-4-hydroxycyclohexyl)amino)-8-(isopropylamino)pyrido[3,4-*d*]pyrimidin-6-yl)ethan-1-one

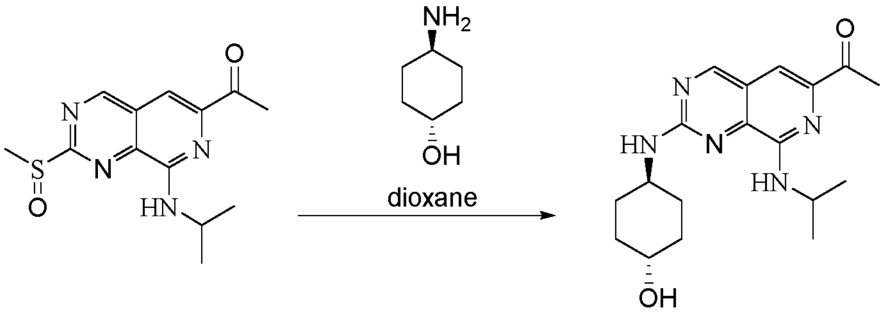


1-(8-(isopropylamino)-2-(methylsulfinyl)pyrido[3,4-*d*]pyrimidin-6-yl)ethan-1-one (53 mg, 0.18 mmol, 1.0 eq.) was dissolved in dioxane (0.9 mL), and the reaction solution was added with 4-aminocyclohexanol (100 mg, 0.90 mmol, 5.0 eq.) and reacted at 120 °C for overnight. When there were no materials left, as detected by LCMS, the reaction solution was concentrated under reduced pressure to give a crude product, which was purified by HPLC to give the product (22.4 mg, yield: 36.1%).
Molecular formula: $C_{18}H_{25}N_5O_2$ Exact mass: 343.20 LC-MS (Pos, *m*/*z*) =344.3 $[M+H]^+$.

**Example 89: Synthesis of (1*r*,4*r*)-4-((8-(isopropylamino)pyrido[3,4-*d*]pyrimidin-2-yl)amino)cyclohexan-1-ol (compound 89)**

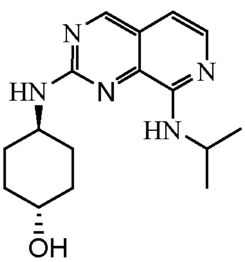

Steps:

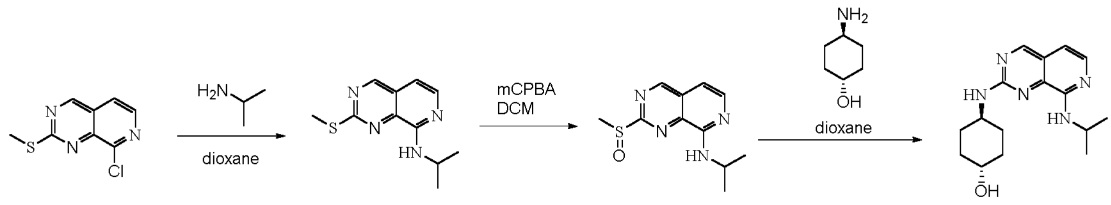


Step 1: synthesis of *N*-isopropyl-2-(methylthio)pyrido[3,4-*d*]pyrimidin-8-amine

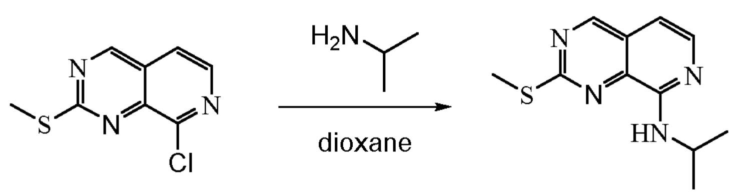


8-chloro-2-(methylthio)pyrido[3,4-d]pyrimidine (100 mg, 0.47 mmol, 1.0 eq.) was dissolved in dioxane (2 mL), and the reaction solution was added with propan-2-amine (1.5 mL) and reacted at 100 °C for overnight. When there were no materials left, as detected by LCMS, the reaction solution was added with water (10 mL) and extracted with ethyl acetate (30 mL × 2). The organic phases were combined, dried over anhydrous sodium sulfate and filtered, and the filtrate was concentrated under reduced pressure to give a crude product, which was purified by silica gel column chromatography to give the product (106 mg, yield: 95.8%).

Step 2: synthesis of *N*-isopropyl-2-(methylsulfinyl)pyrido[3,4-d]pyrimidin-8-amine

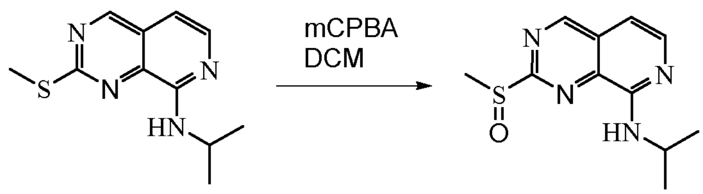


*N*-isopropyl-2-(methylthio)pyrido[3,4-*d*]pyrimidin-8-amine (140 mg, 0.60 mmol, 1.0 eq.) was dissolved in DCM (4 mL), and the reaction solution was added with mCPBA (160 mg, 0.66 mmol, 1.1 eq.) at 0 °C and reacted at 0 °C for 2 h. When there were no materials left, as detected by LCMS, the reaction solution was passed through Celite pad. The solution was added with sat-NaHCO3 aq (10 mL) and extracted with ethyl acetate (40 mL × 2). The organic phases were combined, dried over anhydrous sodium sulfate and filtered, and the filtrate was concentrated under reduced pressure to give a crude product, which was used in the next step without further purification.

Step 3: synthesis of (1*r*,4*r*)-4-((8-(isopropylamino)pyrido[3,4-*d*]pyrimidin-2-yl)amino)cyclohexan-1-ol

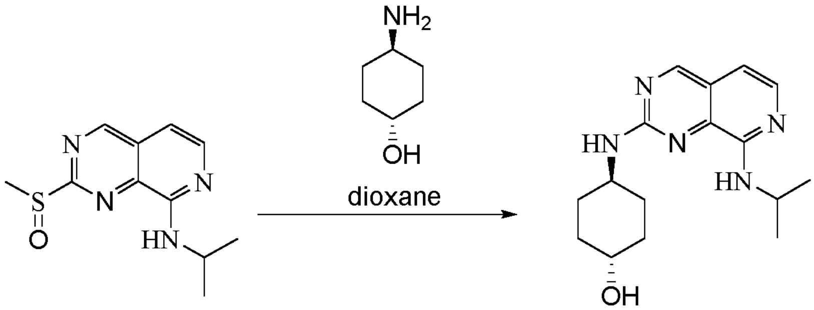


*N*-isopropyl-2-(methylsulfinyl)pyrido[3,4-*d*]pyrimidin-8-amine (75 mg, 0.30 mmol, 1.0 eq.) was dissolved in dioxane (0.5 mL), and the reaction solution was added with 4-aminocyclohexanol (173 mg, 1.5 mmol, 5.0 eq.) and reacted at 120 °C for 2 d. When there were no materials left, as detected by LCMS, the reaction solution was concentrated under reduced pressure to give a crude product, which was purified by HPLC to give the product (38.4 mg, yield: 42.5%). Molecular formula: $C_{16}H_{23}N_5O$ Exact mass: 301.19 LC-MS (Pos, *m*/*z*) =302.2 $[M+H]^+$.

**Example 90: Synthesis of (1*r*,4*r*)-4-((6-ethyl-8-(isopropylamino)pyrido[3,4-*d*]pyrimidin-2-yl)amino)cyclohexan-1-ol (compound 90)**

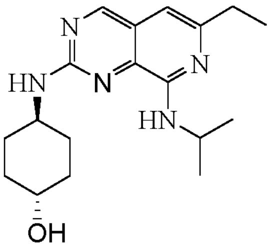

The titled compound was prepared as same as example 89 using corresponding chemicals.

Molecular formula: $C_{18}H_{27}N_5O$ Exact mass: 329.22 LC-MS (Pos, *m*/*z*) =330.3 $[M+H]^+$.

**Example 91: Synthesis of (1*r*,4*r*)-4-((6-(difluoromethyl)-8-(isopropylamino)pyrido[3,4-*d*]pyrimidin-2-yl)amino)cyclohexan-1-ol (compound 91)**

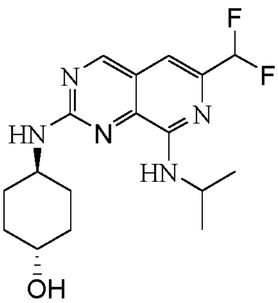

The titled compound was prepared as same as example 89 using corresponding chemicals.

Molecular formula: $C_{17}H_{23}F_2N_5O$ Exact mass: 351.19 LC-MS (Pos, *m*/*z*) =352.3 $[M+H]^+$.

**Example 92: Synthesis of (1*r*,4*r*)-4-((8-(isopropylamino)-6-methylpyrido[3,4-*d*]pyrimidin-2-yl)amino)cyclohexan-1-ol (compound 92)**

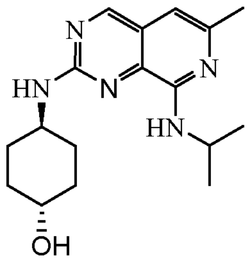

The titled compound was prepared as same as example 89 using corresponding chemicals.

Molecular formula: $C_{17}H_{25}N_5O$ Exact mass: 315.21 LC-MS (Pos, *m/z)* =316.3 $[M+H]^+$.

**Example 93: Synthesis of (1*r*,4*r*)-4-((6-ethynyl-8-(isopropylamino)pyrido[3,4-*d*]pyrimidin-2-yl)amino)cyclohexan-1-ol (compound 93)**

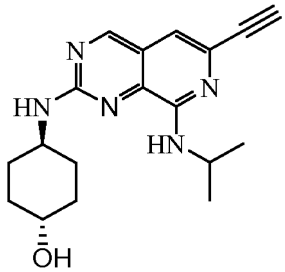

The titled compound was prepared as same as example 89 using corresponding chemicals.

Molecular formula: $C_{18}H_{23}N_5O$ Exact mass: 325.19 LC-MS (Pos, *m/z*) =326.3 $[M+H]^+$.

**Example 94: Synthesis of 2-(((1*r*,4*r*)-4-hydroxycyclohexyl)amino)-8-(isopropylamino)pyrido[3,4-*d*]pyrimidine-6-carbonitrile (compound 94)**

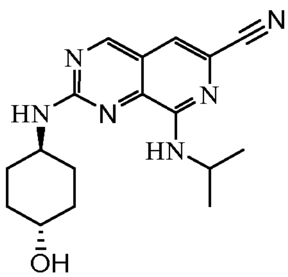

The titled compound was prepared as same as example 89 using corresponding chemicals.

Molecular formula: $C_{17}H_{22}N_6O$ Exact mass: 326.19 LC-MS (Pos, *m/z*) =327.2 $[M+H]^+$.

**Example 95: Synthesis of (1*r*,4*r*)-4-((8-(isopropylamino)-6-(oxetan-3-yl)pyrido[3,4-*d*]pyrimidin-2-yl)amino)cyclohexan-1-ol (compound 95)**

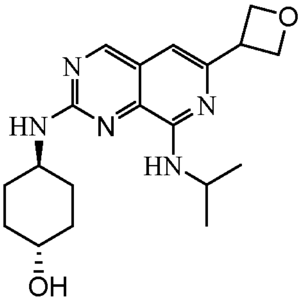

The titled compound was prepared as same as example 89 using corresponding chemicals.

Molecular formula: $C_{19}H_{27}N_5O_2$ Exact mass: 357.22 LC-MS (Pos, *m*/*z*) =358.3 $[M+H]^+$.

**Example 96: Synthesis of (1*r*,4*r*)-4-((6-isopropyl-8-(isopropylamino)pyrido[3,4-*d*]pyrimidin-2-yl)amino)cyclohexan-1-ol (compound 96)**

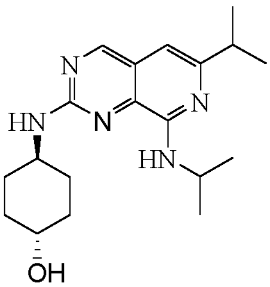

The titled compound was prepared as same as example 89 using corresponding chemicals.

Molecular formula: $C_{19}H_{29}N_5O$ Exact mass: 343.24 LC-MS (Pos, *m*/*z*) =344.3 $[M+H]^+$.

**Example 97: Synthesis of (1*r*,4*r*)-4-((6-(hydroxymethyl)-8-(isopropylamino)pyrido[3,4-*d*]pyrimidin-2-yl)amino)cyclohexan-1-ol (compound 97)**

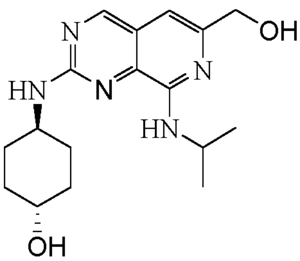

The titled compound was prepared as same as example 75 using corresponding chemicals.

Molecular formula: $C_{17}H_{25}N_5O_2$ Exact mass: 331.20 LC-MS (Pos, *m*/*z*) =332.3 $[M+H]^+$.

**Example 98: Synthesis of (1*S*,3*R*)-3-acetamido-*N*-(7-((*R*)-1-hydroxyethyl)-5-(isopropylamino)-2,6-naphthyridin-3-yl)cyclohexane-1-carboxamide (compound 98)**

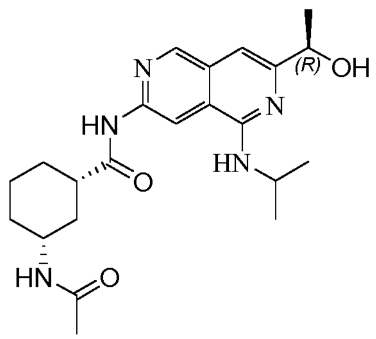

The titled compound was prepared as same as example 1 using corresponding chemicals.

$^1$H-NMR ($CDCl_3$) δ(ppm): 8.75 (s, 1H), 8.45 (s, 1H), 8.28 (s, 1H), 6.81 (s, 1H), 5.44 (d, 1H), 5.30 (d, 1H), 4.77-4.82 (m, 1H), 4.42-4.49 (m, 1H), 3.81-3.90 (m, 1H), 2.45-2.55 (m, 1H), 2.28-2.33 (m, 1H), 1.90-2.01 (m, 6H), 1.35-1.53 (m, 6H), 1.32 (d, 6H).

Molecular formula: $C_{22}H_{31}N_5O_3$ Exact mass: 413.24 LC-MS (Pos, *m/z*) =414.3 $[M+H]^+$.

**Example 99: Synthesis of (1*S*,3*R*)-3-acetamido-*N*-(5-(isopropylamino)-7-vinyl-2,6-naphthyridin-3-yl)cyclohexane-1-carboxamide (compound 99)**

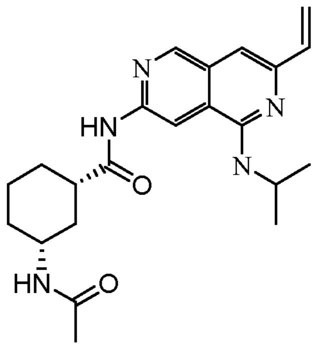

The titled compound was prepared as same as example 1 using corresponding chemicals.

Molecular formula: $C_{22}H_{29}N_5O_2$ Exact mass: 395.23 LC-MS (Pos, *m/z*) = 396.3 $[M+H]^+$.

**Example 100: Synthesis of (1*S*,3*R*)-3-acetamido-*N*-(5-(isopropylamino)-7-vinyl-2,6-naphthyridin-3-yl)cyclohexane-1-carboxamide (compound 100)**

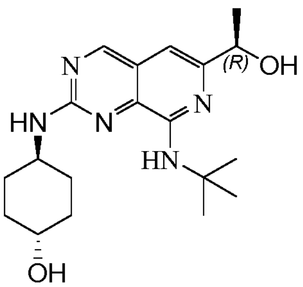

The titled compound was prepared as same as example 75 using corresponding chemicals.

Molecular formula: $C_{19}H_{29}N_5O_2$ Exact mass: 359.23 LC-MS (Pos, *m/z*) = 360.29 $[M+H]^+$.

**Example 101: Synthesis of (*R*)-1-(1-(isopropylamino)-7-(((1*r*,4*R*)-4-(oxetan-3-ylamino)cyclohexyl)amino)-2,6-naphthyridin-3-yl)ethan-1-ol (compound 101)**

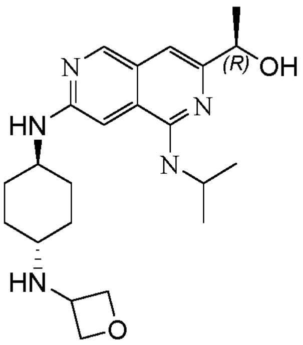

Steps:

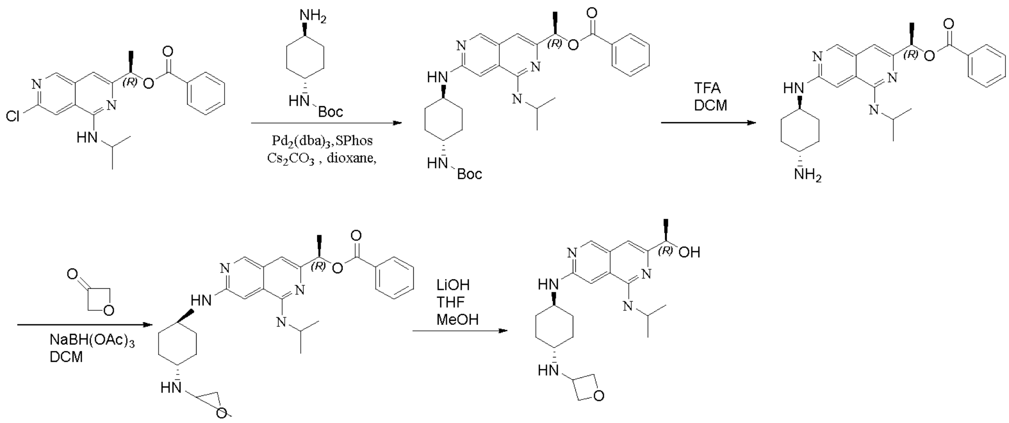


Step 1: synthesis of (*R*)-1-(7-(((1*r*,4*R*)-4-((*tert*-butoxycarbonyl)amino)cyclohexyl)amino)-1-(isopropylamino)-2,6-naphthyridin-3-yl)ethyl benzoate

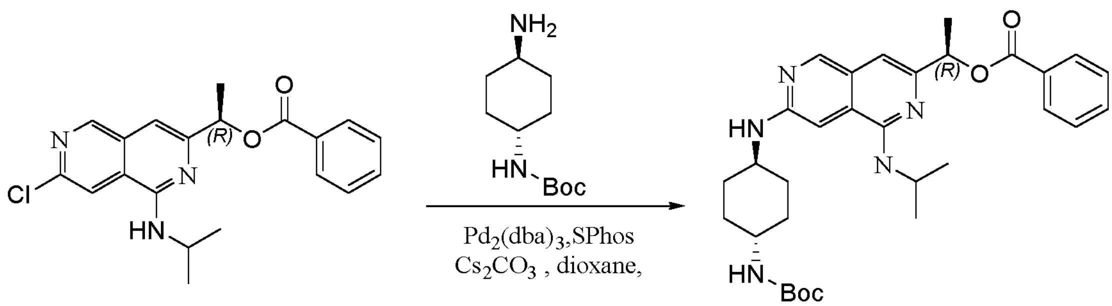


(*R*)-1-(7-chloro-1-(isopropylamino)-2,6-naphthyridin-3-yl)ethyl benzoate (110 mg, 0.30 mmol, 1.0 eq.), *tert*-butyl N-(4-aminocyclohexyl)carbamate (130 mg, 0.60 mmol, 2.0 eq.) was dissolved in dioxane (1.5 mL), and the reaction solution was added with $Pd_2(dba)_3$ (55 mg, 0.06 mmol, 0.2 eq.), SPhos (49 mg, 0.124 mmol, 0.4 eq.) and cesium carbonate (290 mg, 0.9 mmol, 3.0 eq.). The mixture was reacted at 120 °C for 2 h in a sealed tube in nitrogen atmosphere. When there were no materials left, as detected by LCMS, The solution was added with water (15 mL) and extracted with ethyl acetate (30 mL × 3). The organic phases were combined, dried over anhydrous sodium sulfate and filtered,

and the filtrate was concentrated under reduced pressure to give a crude product, which was used in the next step without further purification.

Step 2: synthesis of (*R*)-1-(7-(((1*r*,4*R*)-4-aminocyclohexyl)amino)-1-(isopropylamino)-2,6-naphthyridin-3-yl)ethyl benzoate

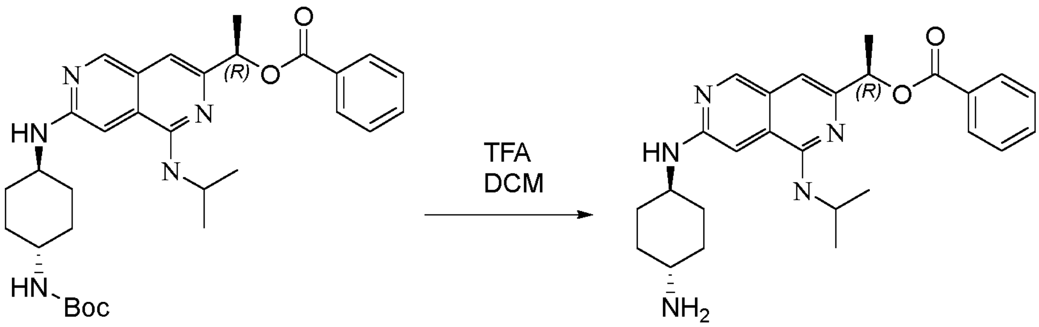


(*R*)-1-(7-(((1*r*,4*R*)-4-((*tert*-butoxycarbonyl)amino)cyclohexyl)amino)-1-(isopropylamino)-2,6-naphthyridin-3-yl)ethyl benzoate (0.3 mmol, 1.0 eq.) was dissolved in DCM (1 mL). The reaction solution was added dropwise with TFA (1 mL) and reacted at room temperature for 2 h. When there were no materials left, as detected by LCMS, the reaction solution was concentrated under reduced pressure to give a crude product, which was purified by cation exchange resin to give the product (52.5 mg).

Step 3: synthesis of (*R*)-1-(1-(isopropylamino)-7-(((1*r*,4*R*)-4-(oxetan-3-ylamino)cyclohexyl)amino)-2,6-naphthyridin-3-yl)ethyl benzoate

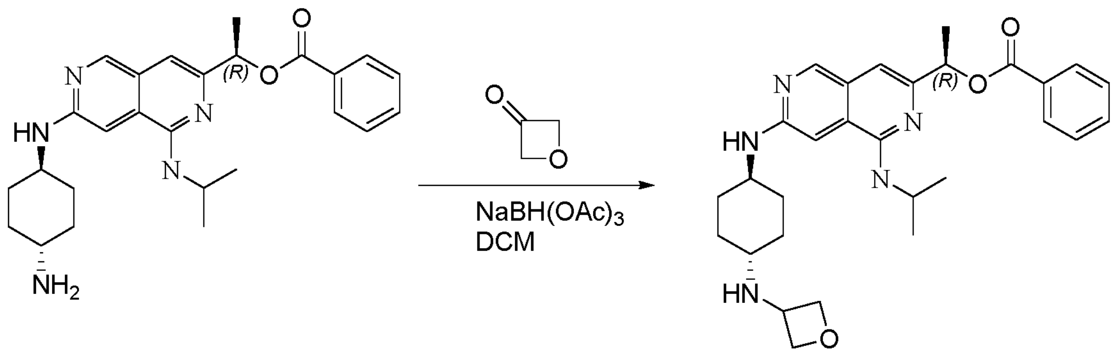


(*R*)-1-(7-(((1*r*,4*R*)-4-aminocyclohexyl)amino)-1-(isopropylamino)-2,6-naphthyridin-3-yl)ethyl benzoate (52.5 mg, 0.12 mmol, 1.0 eq.) and oxetan-3-one (9 mg, 0.13 mmol, 1.1 eq.) were dissolved in DCM (1.2 mL). After the reaction solution was stirred at room temperature for 30 min, the reaction solution was added with $NaBH(OAc)_3$ (62 mg, 0.29 mmol, 2.5 eq.) and reacted at room temperature for 3 h. When there were no materials left, as detected by LCMS, the reaction solution was added with sat-$NaHCO_3$ aq (5 mL) and extracted with ethyl acetate (20 mL × 2). The organic phases were combined, dried over anhydrous sodium sulfate and filtered, and the filtrate was concentrated under reduced pressure to give a crude product, which was used in the next step without further purification.

Step 4: synthesis of (*R*)-1-(1-(isopropylamino)-7-(((1*r*,4*R*)-4-(oxetan-3-ylamino)cyclohexyl)amino)-2,6-naphthyridin-3-yl)ethan-1-ol

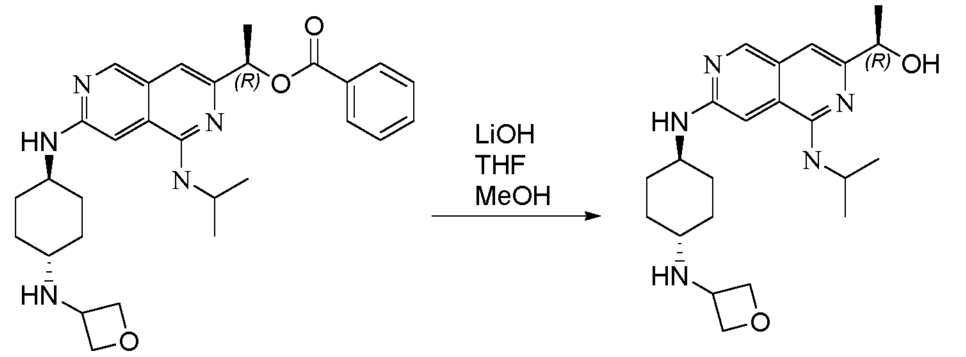


(*R*)-1-(1-(isopropylamino)-7-(((1*r*,4*R*)-4-(oxetan-3-ylamino)cyclohexyl)amino)-2,6-naphthyridin-3-yl)ethyl benzoate (0.12 mmol, 1.0 eq.) was dissolved in MeOH (1.2 mL) and THF (1.2 mL), and the reaction solution was added with 4M LiOH aq (0.088 mL, 0.35 mmol, 3.0 eq.). The mixture was stirred at room temperature for 2 h. When there were no materials left, as detected by LCMS, the reaction solution was concentrated under reduced pressure to give a crude product, which was purified by HPLC to give the product (11.4 mg, yield:28%).
Molecular formula: $C_{22}H_{33}N_5O_2$ Exact mass: 399.26 LC-MS (Pos, *m*/*z*) =400.3 $[M+H]^+$.

**Example 102: Synthesis of (1*R*,4*r*)-4-((8-(*tert*-butylamino)-5-chloro-6-((*R*)-1-hydroxyethyl)pyrido[3,4-*d*]pyrimidin-2-yl)amino)cyclohexan-1-ol (compound 102)**

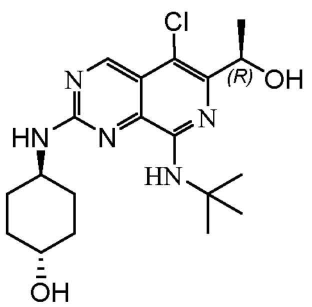

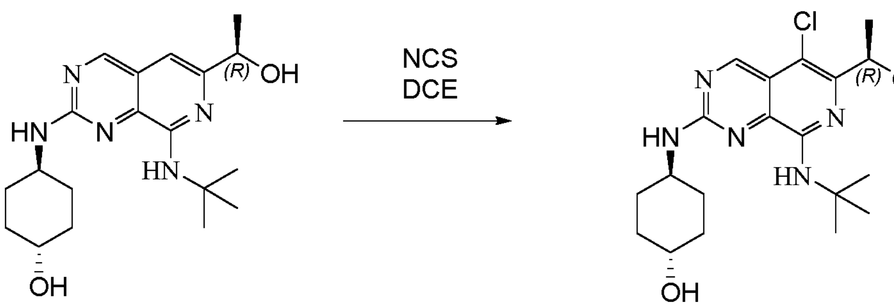


(1*R*,4*r*)-4-((8-(*tert*-butylamino)-6-((*R*)-1-hydroxyethyl)pyrido[3,4-*d*]pyrimidin-2-yl)amino)cyclohexan-1-ol (17.9 mg, 0.05 mmol, 1.0 eq.) was dissolved in DCE (0.5 mL), and the reaction solution was added with *N*-chlorosuccinimide (8 mg, 0.06 mmol, 1.2 eq.) and reacted at 65 °C for 2 h. When there were no materials left, as detected by LCMS, the reaction solution was added with propan-2-amine (0.88 mL) and reacted at 100 °C for overnight. When there were no materials left, as detected by LCMS, the reaction solution was was concentrated under reduced pressure to give a crude product, which was purified by HPLC to give the product (7 mg, yield: 35.9%).
Molecular formula: $C_{19}H_{28}ClN_5O_2$ Exact mass: 393.19 LC-MS (Pos, *m*/*z*) =394.2 $[M+H]^+$.

**Example 103: Synthesis of 1-(isopropylamino)-7-(((3*S*,6*S*)-6-methylpiperidin-3-yl)amino)-2,6-naphthyridine-3-carbonitrile (Compound 103)**

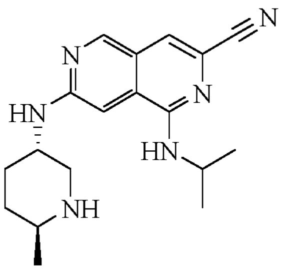

Step 1: synthesis of benzyl (2*S*,5*S*)-((7-cyano-5-(isopropylamino)-2,6-naphthyridin-3-yl)amino)-2-methylpiperidine-1-carboxylate:

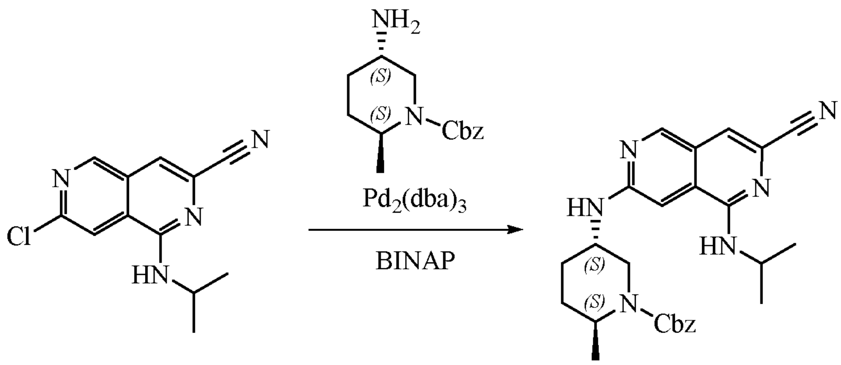


7-Chloro-1-(isopropylamino)-2,6-naphthyridine-3-carbonitrile (150 mg, 0.61 mmol, 1.0 eq.), benzyl (2*S*,5*S*)-5-amino-2-methylpiperidine-1-carboxylate (181.3 mg, 0.73 mmol, 1.3 eq.), $Pd_2(dba)_3$ (82.4 mg, 0.09 mmol, 0.15 eq.), BINAP (112 mg, 0.18 mmol, 0.3 eq.) and cesium carbonate (498.5 mg, 1.53 mmol, 2.5 eq.) were added to 1,4-dioxane (15 mL). The mixture was reacted at 100 °C for 23 h in a sealed tube in nitrogen atmosphere. When there were no starting materials left as detected by TLC, water (20 mL) was added, and the mixture was extracted with ethyl acetate (20 mL × 3). The organic phases were combined, dried over anhydrous sodium sulfate and filtered, and the filtrate was concentrated under reduced pressure to give a crude product, which was purified by silica gel column chromatography (EA:PE = 1:10-1:2) to give the product (195 mg, yield: 69.7%).

Step 2: synthesis of 1-(isopropylamino)-7-(((3*S*,6*S*)-6-methylpiperidin-3-yl)amino)-26-nahthridine-3-carbonitrile:

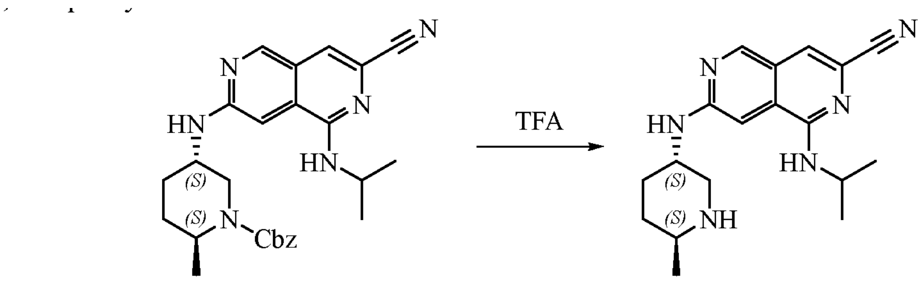


Benzyl (2*S*,5*S*)-5-((7-cyano-5-(isopropylamino)-2,6-naphthyridin-3-yl)amino)-2-methylpiperidine-1-carboxylate (195 mg, 0.42 mmol, 1.0 eq.) was dissolved in TFA (5 mL), and the reaction solution was heated at reflux for 1 h.

When there were no starting materials left as detected by TLC, the reaction solution was concentrated under reduced pressure. Water (20 mL) was added, and the pH was adjusted to about 8 with sodium carbonate. The mixture was extracted with ethyl acetate (20 mL × 3). The organic phases were combined, dried over anhydrous sodium sulfate and filtered, and the filtrate was concentrated under reduced pressure to give a crude product, which was purified by preparative thin-layer chromatography (MeOH:DCM = 1:10) to give the product (100 mg, yield: 73.4%).

$^1$HNMR (400 MHz, $CD_3OD$) δ(ppm): 8.72 (s, 1H), 7.37 (s, 1H), 7.02 (s, 1H), 4.48-4.41 (m, 1H), 4.35-4.30 (m, 1H), 3.72-3.68 (m, 1H), 3.29-3.26 (m, 1H), 2.85-2.79 (m, 2H), 2.23-2.10 (m, 1H), 2.14-2.11 (m, 1H), 1.76-1.72 (m, 2H), 1.71 (d, 3H), 1.40-1.39 (m, 6H).

Molecular formula: $C_{18}H_{24}N_6$ Molecular weight: 324.43 LC-MS (Pos, *m/z*) = 325.25[M+H]$^+$.

**Example 104: Synthesis of 1-(((S)-2-hydroxy-1-phenylethyl)amino)-7-(((S)-piperidin-3-yl)amino)-2,6-naphthyridine-3-carbonitrile (compound 104)**

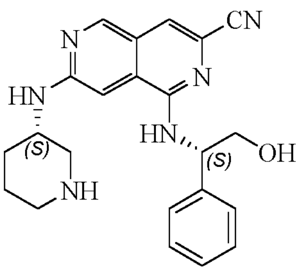

The titled compound was prepared as same as example 147 using corresponding chemicals.

$^1$HNMR (400 MHz, MeOD) δ(ppm): 8.69 (s, 1H), 7.49-7.47 (d, 2H), 7.39 (s, 1H), 7.38-7.32 (m, 2H), 7.27-7.23 (m, 1H), 7.04 (s, 1H), 5.51-5.46 (m, 1H), 3.99-3.97 (m, 3H), 3.34 (s, 1H), 3.06-3.02 (m, 1H), 2.74-2.68 (m, 1H), 2.63-2.57 (m, 1H), 2.16-2.13 (t,1H), 1.92-1.87 (m, 1H), 1.74-1.70 (m, 1H), 1.67-1.59 (m, 1H).

Molecular formula:$C_{22}H_{24}N_6O$ Exact mass: 388.20 LC-MS(Pos, *m/z*)=389.14[M+H]$^+$.

**Example 106: Synthesis of (S)-8-(isopropylamino)-2-((6-oxopiperidin-3-yl)amino)pyrido[3,4-d]pyrimidine-6-carbonitrile (compound 106)**

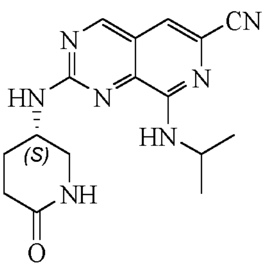

The titled compound was prepared as same as example 45 using corresponding chemicals.

$^1$HNMR (400 MHz, MeOD) δ(ppm): 8.96 (s, 1H), 7.31 (s, 1H), 4.55 (s, 1H), 4.38-4.32 (m, 1H), 3.69-3.65 (m, 1H), 3.30-3.27 (t ,1H), 2.56-2.53 (t, 2H), 2.21-2.15 (m, 1H), 2.08-2.02 (m, 1H), 1.33-1.34 (d, 6H).

Molecular formula:$C_{16}H_{19}N_7O$ Exact mass: 325.17 LC-MS(Pos, *m/z*)=326.10[M+H]$^+$.

**Example 107: Synthesis of 8-(isopropylamino)-2-(((3S,6S)-6-methylpiperidin-3-yl)amino)pyrido[3,4-d]pyrimidine-6-carbonitrile (compound 107)**

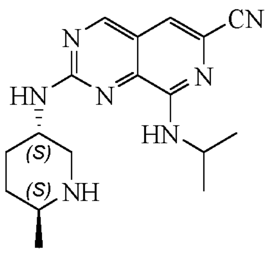

The titled compound was prepared as same as example 55 using corresponding chemicals.

$^1$HNMR (400 MHz, MeOD) δ(ppm): 8.90 (s, 1H), 7.30 (s, 1H), 4.38 (s, 1H), 4.11-4.03 (m, 1H), 3.37 (s, 1H), 2.70-2.66 (d, 1H), 2.49-2.43 (t, 1H), 2.12-2.09 (t, 1H), 1.86-1.82 (m, 1H), 1.60-1.51 (m, 1H), 1.35-1.34 (d, 7H), 1.17-1.15 (d, 3H).

Molecular formula:$C_{17}H_{23}N_7$ Exact mass: 325.20 LC-MS(Pos, *m*/*z*)=326.14[M+H]$^+$.

**Example 108: Synthesis of (S)-1-isobutyl-7-(piperidin-3-ylamino)-2,6-naphthyridine-3-carbonitrile (compound 108)**

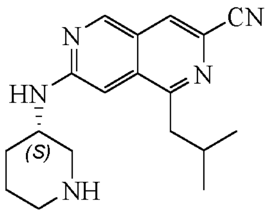

The titled compound was prepared as same as example 38 using corresponding chemicals.

$^1$HNMR (400 MHz, MeOD) δ(ppm): 9.01-9.03 (d, 1H), 8.12 (s, 1H), 7.04 (s, 1H), 4.27-4.33 (m, 1H), 3.54-3.58 (q, 1H), 3.26-3.30 (m, 1H), 2.88-3.02 (m, 4H), 2.23-2.31 (m, 1H), 2.16-2.21 (s, 1H), 2.04-2.12 (s, 1H), 1.82-1.93 (s, 1H), 1.68-1.78 (s, 1H), 1.00-1.01 (d, 6H).

Molecular formula: $C_{18}H_{23}N_5$ Exact mass: 309.20 LC-MS(Pos, *m*/*z*)=310.13 [M+H]$^+$.

**Example 111: Synthesis of (5)-6-(difluoromethyl)-8-(isopropylamino)-2-(piperidin-3-ylamino)pyrido[3,4-*d*]pyrimidine-5-carbonitrile (compound 111)**

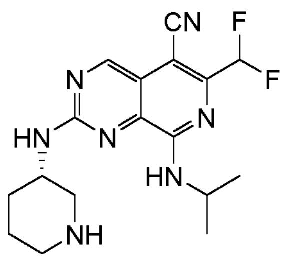

Steps:

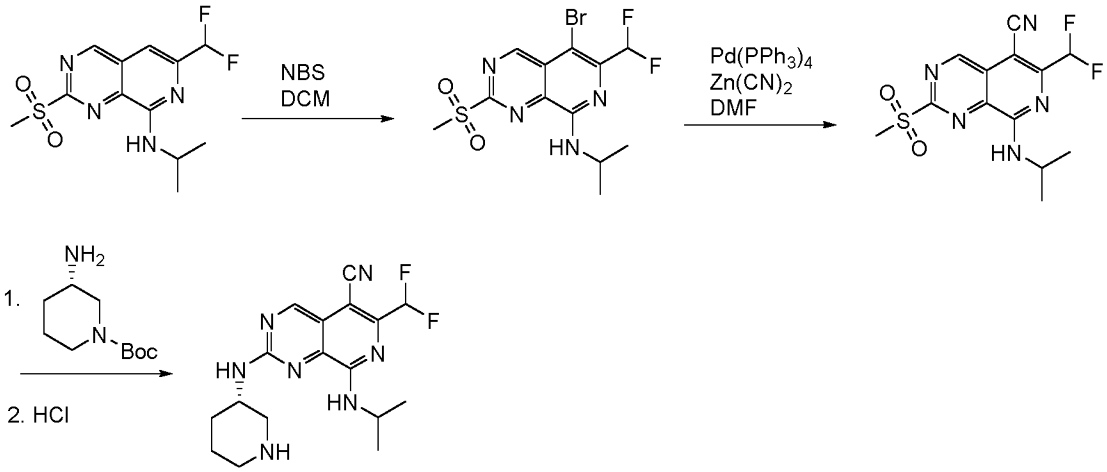


Step 1: synthesis of 5-bromo-6-(difluoromethyl)-*N*-isopropyl-2-(methylsulfonyl)pyrido[3,4-*d*]pyrimidin-8-amine

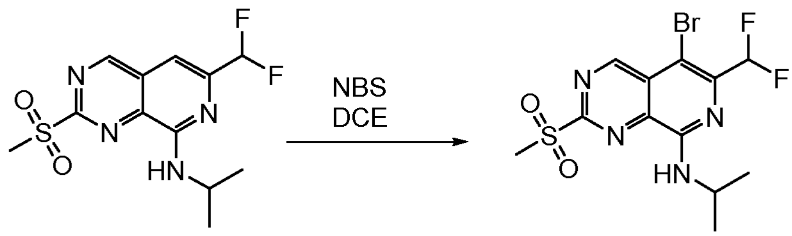


6-(difluoromethyl)-*N*-isopropyl-2-(methylsulfonyl)pyrido[3,4-*d*]pyrimidin-8-amine (660 mg, 2.1 mmol, 1.0 eq.) and NBS (446 mg, 2.5 mmol, 1.2 eq.) were added into DCE (10 mL). The mixture was reacted at 65 °C for overnight. When there were no materials left, as detected by LCMS, the reaction solution was added with sat-$NaHCO_3$ aq (10 mL) and extracted with DCM (10 mL × 2). The organic phases were combined, dried over anhydrous sodium sulfate and filtered, and the filtrate was concentrated under reduced pressure to give a crude product, which was purified by silica gel column chromatography to give the product (740 mg, yield: 89.7%). Exact mass: 393.99, LC-MS (Pos, *m*/*z*)= 395.0 $[M+H]^+$.

Step 2: synthesis of 6-(difluoromethyl)-8-(isopropylamino)-2-(methylsulfonyl)pyrido[3,4-*d*]pyrimidine-5-carbonitrile

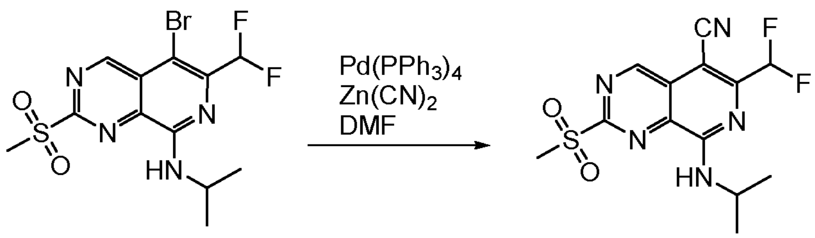


5-bromo-6-(difluoromethyl)-*N*-isopropyl-2-(methylsulfonyl)pyrido[3,4-*d*]pyrimidin-8-amine (582 mg, 1.47 mmol, 1.0 eq.), $Pd(PPh_3)_4$ (340 mg, 0.29 mmol, 0.2 eq.) and zinc cyanide (346 mg, 2.95 mmol, 2.0 eq.) were added into DMF (15 mL). The mixture was reacted at 60 °C for overnight in a sealed tube in nitrogen atmosphere. When there were no materials left, as detected by LCMS, the reaction solution was added with sat-$NaHCO_3$ aq and extracted with ethyl acetate (30 mL × 3). The organic phases were combined, washed with brine, dried over anhydrous sodium sulfate and filtered, and the filtrate was concentrated under reduced pressure to give a crude product, which was purified by silica gel column chromatography to give the product (453 mg, yield: 90.1%). Exact mass: 341.08, LC-MS (Pos, *m*/*z*): 342.1 $[M+H]^+$.

Step 3: synthesis of (*S*)-6-(difluoromethyl)-8-(isopropylamino)-2-(piperidin-3-ylamino)pyrido[3,4-*d*]pyrimidine-5-carbonitrile

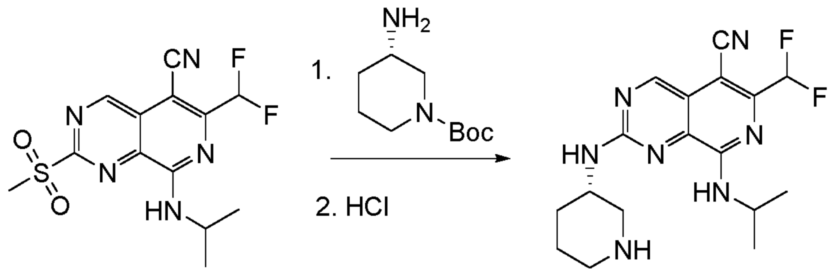


The titled compound was prepared as same as example 55 using corresponding chemicals.

Molecular formula: $C_{17}H_{21}F_2N_7$ Exact mass: 361.18 LC-MS (Pos, *m*/*z*): 362.1 $[M+H]^+$.

**Example 112: Synthesis of (5)-8-(isopropylamino)-2-(pyrrolidin-3-ylamino)pyrido[3,4-*d*]pyrimidine-6-carbonitrile (compound 112)**

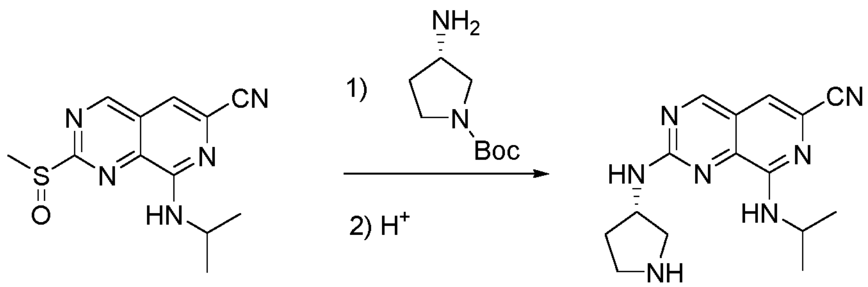


The titled compound was prepared as same as example 55 using corresponding chemicals.

Molecular formula: $C_{15}H_{19}N_7$ Exact mass: 297.17 LC-MS (Pos, *m*/*z*): 298.1 $[M+H]^+$.

**Example 113: Synthesis of (*R*)-8-(isopropylamino)-2-(pyrrolidin-3-ylamino)pyrido[3,4-*d*]pyrimidine-6-carbonitrile (compound 113)**

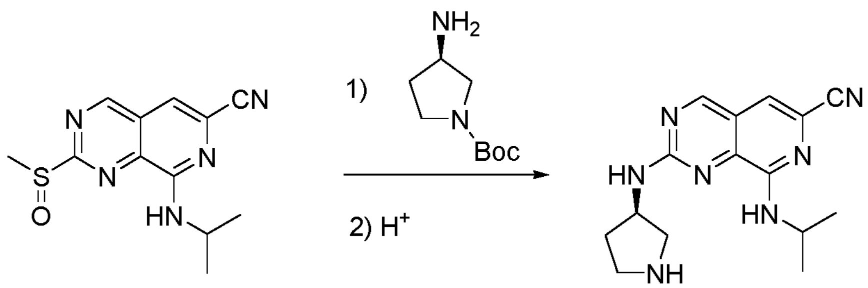


The titled compound was prepared as same as example 55 using corresponding chemicals.

Molecular formula: $C_{15}H_{19}N_7$ Exact mass: 297.17 LC-MS (Pos, *m*/*z*): 298.1 $[M+H]^+$.

**Example 114: Synthesis of 8-(isopropylamino)-2-(piperidin-4-ylamino)pyrido[3,4-*d*]pyrimidine-6-carbonitrile (compound 114)**

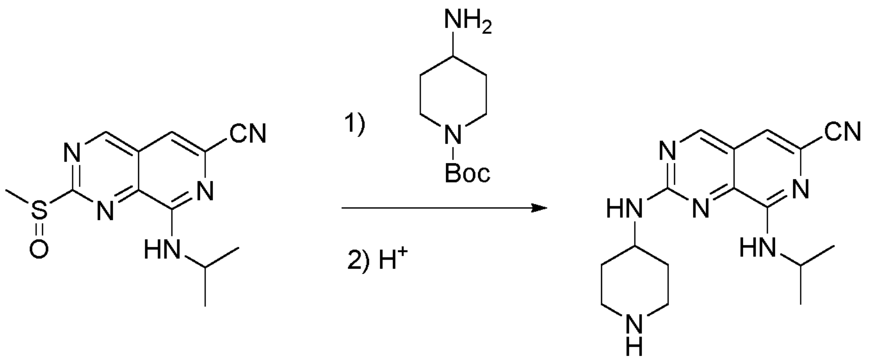


The titled compound was prepared as same as example 55 using corresponding chemicals.

Molecular formula: $C_{16}H_{21}N_7$ Exact mass: 311.19 LC-MS (Pos, *m*/*z*): 312.1 $[M+H]^+$.

**Example 115: Synthesis of 8-(isopropylamino)-2-(piperidin-4-ylamino)pyrido[3,4-*d*]pyrimidine-6-carbonitrile (compound 115)**

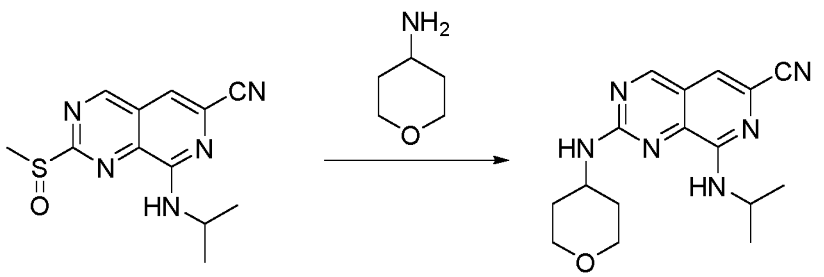

The titled compound was prepared as same as example 89 using corresponding chemicals.

Molecular formula: $C_{16}H_{20}N_6O$ Exact mass: 312.17 LC-MS (Pos, *m*/*z*): 313.1 $[M+H]^+$.

**Example 116: Synthesis of (*S*)-8-(isopropylamino)-2-((tetrahydro-2*H*-pyran-3-yl)amino)pyrido[3,4-*d*]pyrimidine-6-carbonitrile (compound 116)**

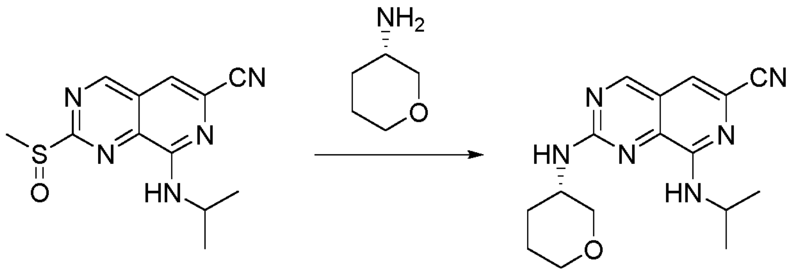

The titled compound was prepared as same as example 89 using corresponding chemicals.

Molecular formula: $C_{16}H_{20}N_6O$ Exact mass: 312.17 LC-MS (Pos, *m*/*z*): 313.2 $[M+H]^+$.

**Example 117: Synthesis of (*R*)-8-(isopropylamino)-2-((tetrahydro-2*H*-pyran-3-yl)amino)pyrido[3,4-*d*]pyrimidine-6-carbonitrile (compound 117)**

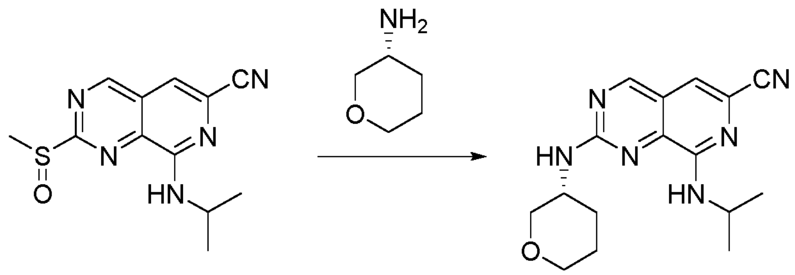

The titled compound was prepared as same as example 89 using corresponding chemicals.

Molecular formula: $C_{16}H_{20}N_6O$ Exact mass: 312.17 LC-MS (Pos, *m/z):* 313.0 $[M+H]^+$.

**Example 118: Synthesis of (*S*)-8-(isopropylamino)-2-((tetrahydrofuran-3-yl)amino)pyrido[3,4-*d*]pyrimidine-6-carbonitrile (compound 118)**

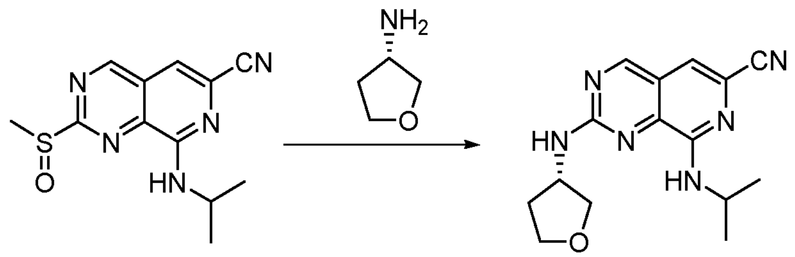

The titled compound was prepared as same as example 89 using corresponding chemicals.

Molecular formula: $C_{15}H_{18}N_6O$ Exact mass: 298.15 LC-MS (Pos, *m/z):* 299.0 $[M+H]^+$.

**Example 119: Synthesis of (*R*)-8-(isopropylamino)-2-((tetrahydrofuran-3-yl)amino)pyrido[3,4-*d*]pyrimidine-6-carbonitrile (compound 119)**

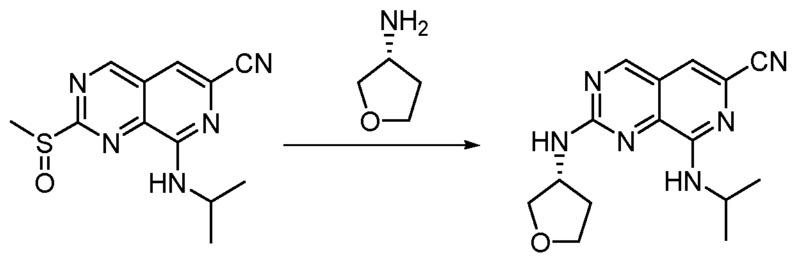

The titled compound was prepared as same as example 89 using corresponding chemicals.

Molecular formula: $C_{15}H_{18}N_6O$ Exact mass: 298.15 LC-MS (Pos, *m/z*): 299.2 $[M+H]^+$.

**Example 120: Synthesis of 2-((1,1-dioxidotetrahydro-2*H*-thiopyran-4-yl)amino)-8-(isopropylamino)pyrido[3,4-*d*]pyrimidine-6-carbonitrile (compound 120)**

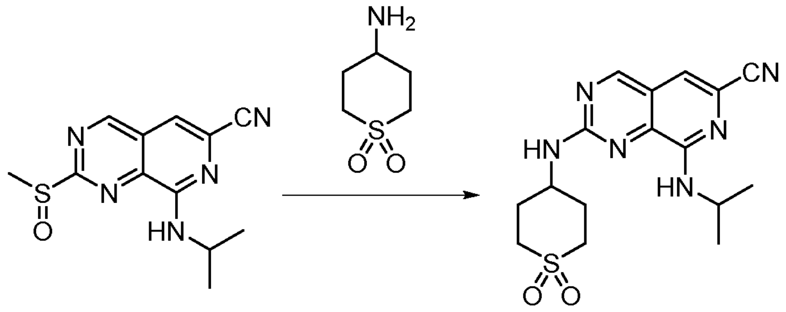

The titled compound was prepared as same as example 89 using corresponding chemicals.
Molecular formula: $C_{16}H_{20}N_6O_2S$ Exact mass: 360.14 LC-MS (Pos, *m*/*z*): 361.1 $[M+H]^+$.

**Example 121: Synthesis of 2-((1,1-dioxidotetrahydro-2*H*-thiopyran-3-yl)amino)-8-(isopropylamino)pyrido[3,4-*d*]pyrimidine-6-carbonitrile (compound 121)**

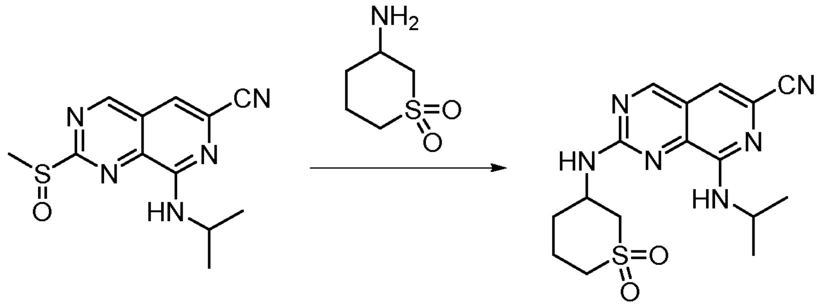

The titled compound was prepared as same as example 89 using corresponding chemicals.
Molecular formula: $C_{16}H_{20}N_6O_2S$ Exact mass: 360.14 LC-MS (Pos, *m*/*z*): 361.0 $[M+H]^+$.

**Example 122: Synthesis of 2-(((1*S*,3*S*)-3-hydroxycyclopentyl)amino)-8-(isopropylamino)pyrido[3,4-*d*]pyrimidine-6-carbonitrile (compound 122)**

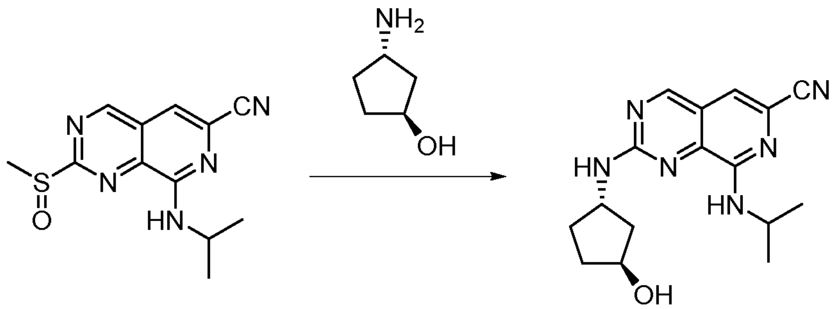

The titled compound was prepared as same as example 89 using corresponding chemicals.
Molecular formula: $C_{16}H_{20}N_6O$ Exact mass: 312.17 LC-MS (Pos, *m*/*z*): 313.1 $[M+H]^+$.

**Example 123: Synthesis of (*S*)-8-(isopropylamino)-2-((2-oxopyrrolidin-3-yl)amino)pyrido[3,4-*d*]pyrimidine-6-carbonitrile (compound 123)**

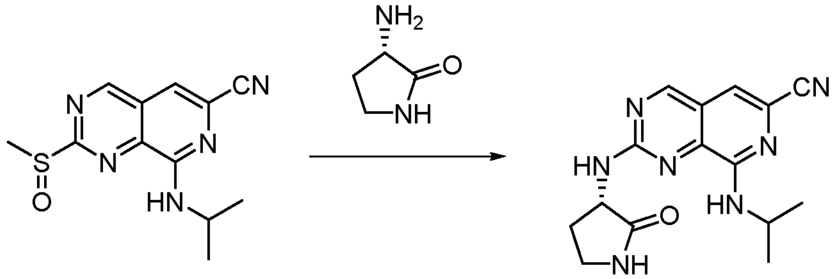

The titled compound was prepared as same as example 89 using corresponding chemicals.

Molecular formula: $C_{15}H_{17}N_7O$ Exact mass: 311.15 LC-MS (Pos, *m/z*): 312.0 $[M+H]^+$.

**Example 124: Synthesis of (*R*)-8-(isopropylamino)-2-((2-oxopyrrolidin-3-yl)amino)pyrido[3,4-*d*]pyrimidine-6-carbonitrile (compound 124)**

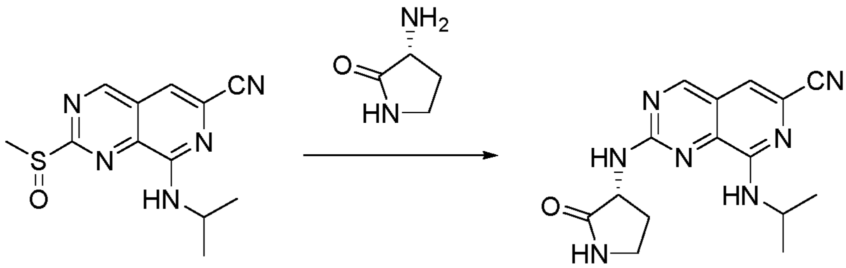

The titled compound was prepared as same as example 89 using corresponding chemicals.

Molecular formula: $C_{15}H_{17}N_7O$ Exact mass: 311.15 LC-MS (Pos, *m/z*): 312.0 $[M+H]^+$.

**Example 125: Synthesis of 2-(((1*s*,4*s*)-4-hydroxycyclohexyl)amino)-8-(isopropylamino)pyrido[3,4-*d*]pyrimidine-6-carbonitrile (compound 125)**

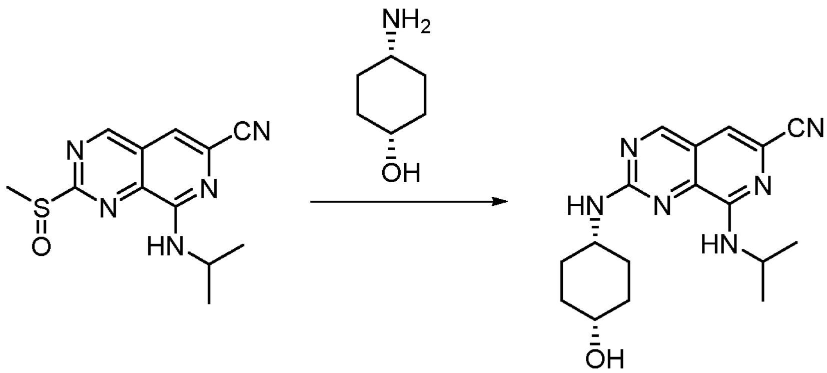

The titled compound was prepared as same as example 89 using corresponding chemicals.

Molecular formula: $C_{17}H_{22}N_6O$ Exact mass: 326.19 LC-MS (Pos, *m/z*): 327.1 $[M+H]^+$.

**Example 126: Synthesis of 2-(((1*s*,3*s*)-3-aminocyclobutyl)amino)-8-(isopropylamino)pyrido[3,4-*d*]pyrimidine-6-carbonitrile (compound 126)**

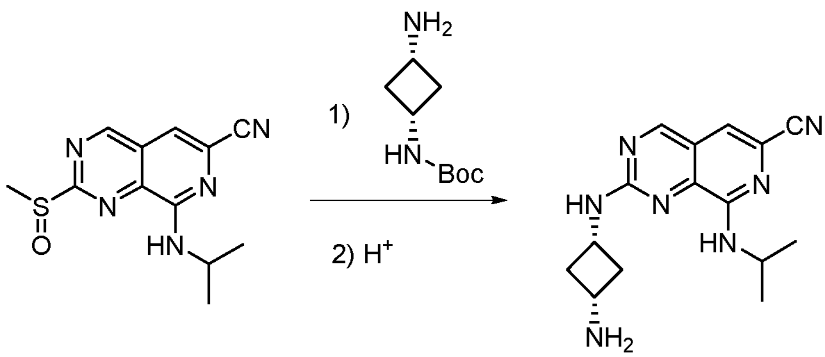


The titled compound was prepared as same as example 55 using corresponding chemicals.

Molecular formula: $C_{15}H_{19}N_7$ Exact mass: 297.17 LC-MS (Pos, *m*/*z*): 298.0 $[M+H]^+$.

**Example 127: Synthesis of 2-(((1*r*,3*r*)-3-aminocyclobutyl)amino)-8-(isopropylamino)pyrido[3,4-*d*]pyrimidine-6-carbonitrile (compound 127)**

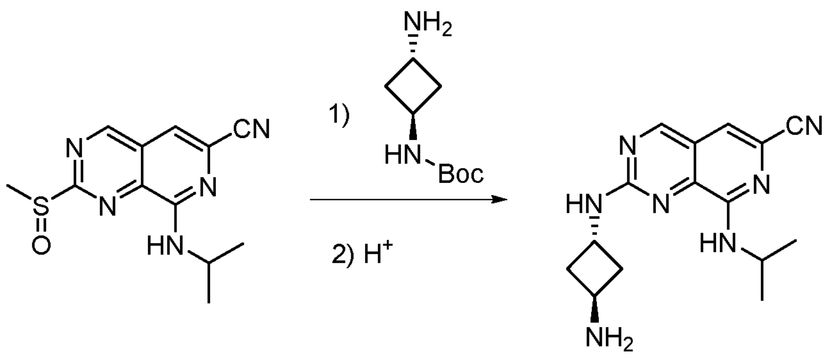


The titled compound was prepared as same as example 55 using corresponding chemicals.

Molecular formula: $C_{15}H_{19}N_7$ Exact mass: 297.17 LC-MS (Pos, *m*/*z*): 298.2 $[M+H]^+$.

**Example 128: Synthesis of 2-((3-aminobicyclo[1.1.1]pentan-1-yl)amino)-8-(isopropylamino)pyrido[3,4-*d*]pyrimidine-6-carbonitrile (compound 128)**

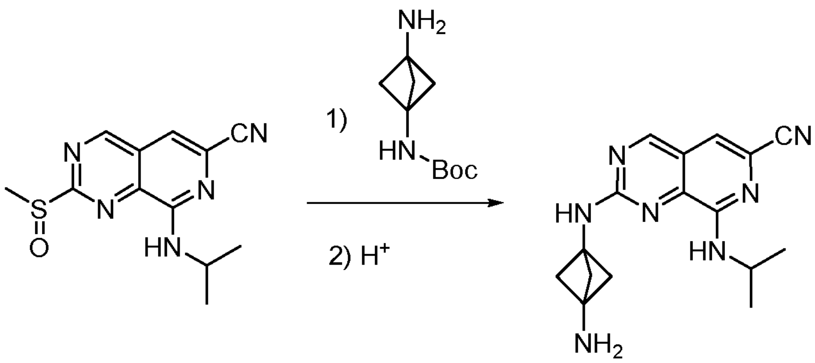


The titled compound was prepared as same as example 55 using corresponding chemicals..

Molecular formula: $C_{16}H_{19}N_7$ Exact mass: 309.17 LC-MS (Pos, *m*/*z*): 310.0 $[M+H]^+$.

**Example 129: Synthesis of 2-(azetidin-3-ylamino)-8-(isopropylamino)pyrido[3,4-*d*]pyrimidine-6-carbonitrile (compound 129)**

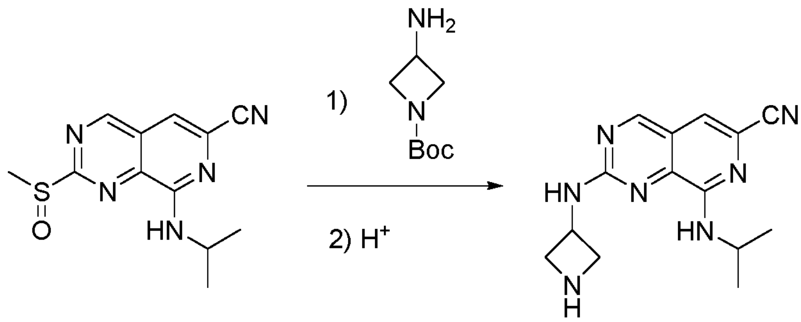


The titled compound was prepared as same as example 55 using corresponding chemicals.
Molecular formula: $C_{14}H_{17}N_7$ Exact mass: 283.15 LC-MS (Pos, *m/z*): 284.0 $[M+H]^+$.

**Example 130: Synthesis of 2-((2-azaspiro[3.3]heptan-6-yl)amino)-8-(isopropylamino)pyrido[3,4-*d*]pyrimidine-6-carbonitrile (compound 130)**

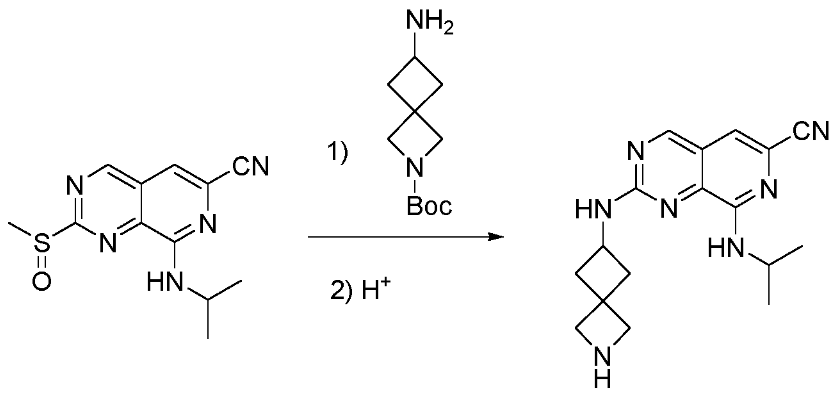


The titled compound was prepared as same as example 55 using corresponding chemicals.
Molecular formula: $C_{17}H_{21}N_7$ Exact mass: 323.19 LC-MS (Pos, *m/z*): 324.2 $[M+H]^+$.

**Example 131: Synthesis of (*S*)-2-(azepan-4-ylamino)-8-(isopropylamino)pyrido[3,4-*d*]pyrimidine-6-carbonitrile (compound 131)**

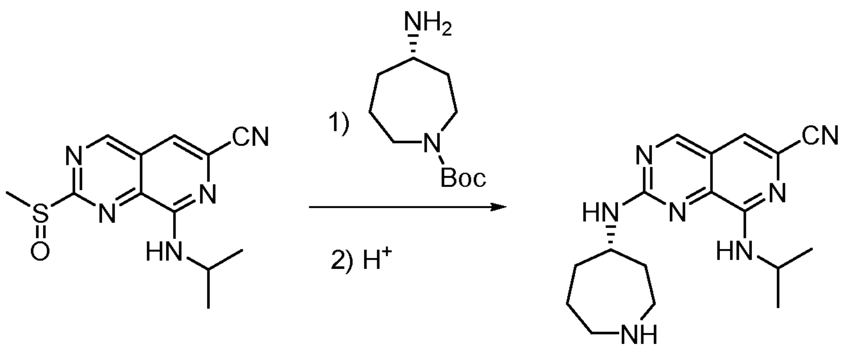

The titled compound was prepared as same as example 55 using corresponding chemicals.

Molecular formula: $C_{17}H_{23}N_7$ Exact mass: 325.20 LC-MS (Pos, *m/z*): 326.2 $[M+H]^+$.

**Example 132: Synthesis of (*R*)-2-(azepan-4-ylamino)-8-(isopropylamino)pyrido[3,4-*d*]pyrimidine-6-carbonitrile (compound 132)**

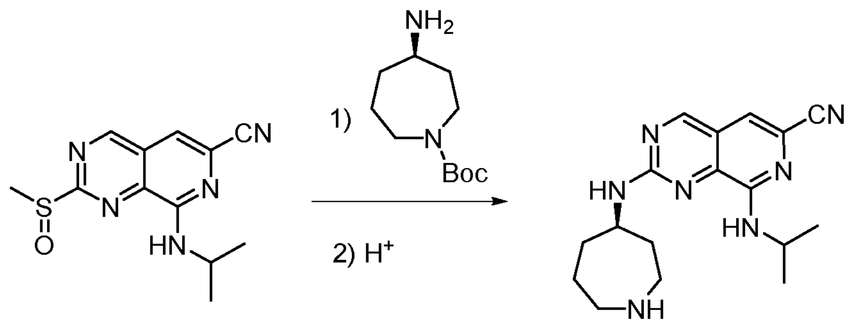


The titled compound was prepared as same as example 55 using corresponding chemicals.

Molecular formula: $C_{17}H_{23}N_7$ Exact mass: 325.20 LC-MS (Pos, *m/z*): 326.2 $[M+H]^+$.

**Example 133: Synthesis of 2-(((1*s*,3*s*)-3-hydroxycyclobutyl)amino)-8-(isopropylamino)pyrido[3,4-*d*]pyrimidine-6-carbonitrile (compound 133)**

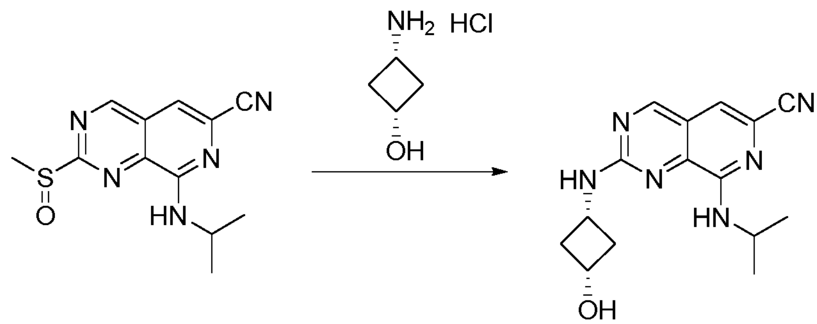

The titled compound was prepared as same as example 89 using corresponding chemicals.

Molecular formula: $C_{15}H_{18}N_6O$ Exact mass: 298.15 LC-MS (Pos, *m/z*): 299.0 $[M+H]^+$.

**Example 134: Synthesis of 2-(((1*r*,3*r*)-3-hydroxycyclobutyl)amino)-8-(isopropylamino)pyrido[3,4-*d*]pyrimidine-6-carbonitrile (compound 134)**

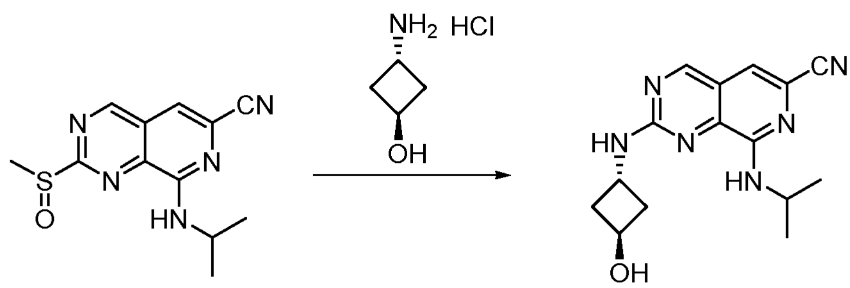

The titled compound was prepared as same as example 89 using corresponding chemicals..

Molecular formula: $C_{15}H_{18}N_6O$ Exact mass: 298.15 LC-MS (Pos, *m/z*): 299.1 $[M+H]^+$.

**Example136: Synthesis of 2-(((1*r*,4*r*)-4-aminocyclohexyl)amino)-8-(isopropylamino)pyrido[3,4-*d*]pyrimidine-6-carbonitrile (compound136)**

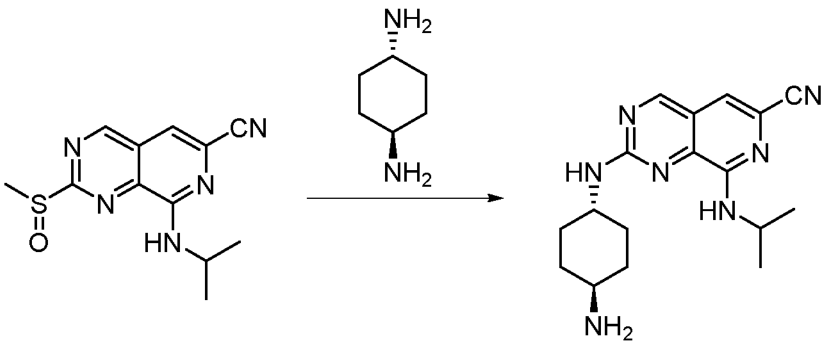

The titled compound was prepared as same as example 89 using corresponding chemicals.

Molecular formula: $C_{17}H_{23}N_7$ Exact mass: 325.20 LC-MS (Pos, *m/z*): 326.2 $[M+H]^+$.

**Example 137: Synthesis of 2-(((1*s*,4*s*)-4-aminocyclohexyl)amino)-8-(isopropylamino)pyrido[3,4-*d*]pyrimidine-6-carbonitrile (compound 137)**

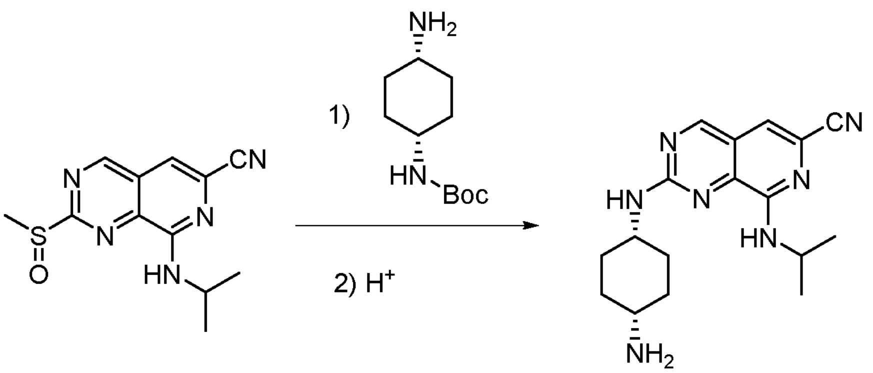


The titled compound was prepared as same as example 55 using corresponding chemicals.

Molecular formula: $C_{17}H_{23}N_7$ Exact mass: 325.20 LC-MS (Pos, *m/z*): 326.1 $[M+H]^+$.

**Example 138: Synthesis of 2-(((1*r*,4*r*)-4-(dimethylamino)cyclohexyl)amino)-8-(isopropylamino)pyrido[3,4-*d*]pyrimidine-6-carbonitrile (compound138)**

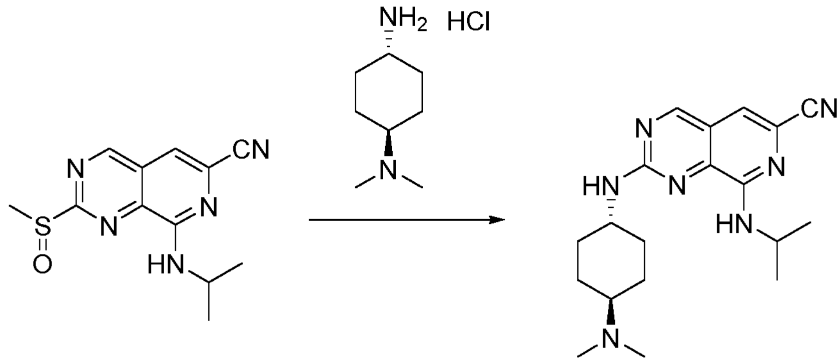


The titled compound was prepared as same as example 89 using corresponding chemicals.

Molecular formula: $C_{19}H_{27}N_7$ Exact mass: 353.23 LC-MS (Pos, *m*/*z*): 354.3 $[M+H]^+$.

**Example 139: Synthesis of 2-(((1*R*,5*S*,7*r*)-3-oxa-9-azabicyclo[3.3.1]nonan-7-yl)amino)-8-(isopropylamino)pyrido[3,4-*d*]pyrimidine-6-carbonitrile (compound 139)**

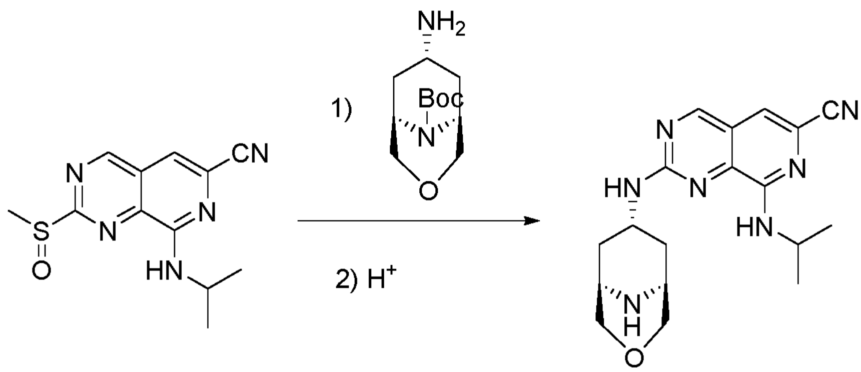


The titled compound was prepared as same as example 55 using corresponding chemicals.

Molecular formula: $C_{18}H_{23}N_7O$ Exact mass: 353.20 LC-MS (Pos, *m*/*z*): 354.2 $[M+H]^+$.

**Example 141: Synthesis of 8-(isopropylamino)-2-(((1*r*,4*r*)-4-morpholinocyclohexyl)amino)pyrido[3,4-*d*]pyrimidine-6-carbonitrile (compound 141)**

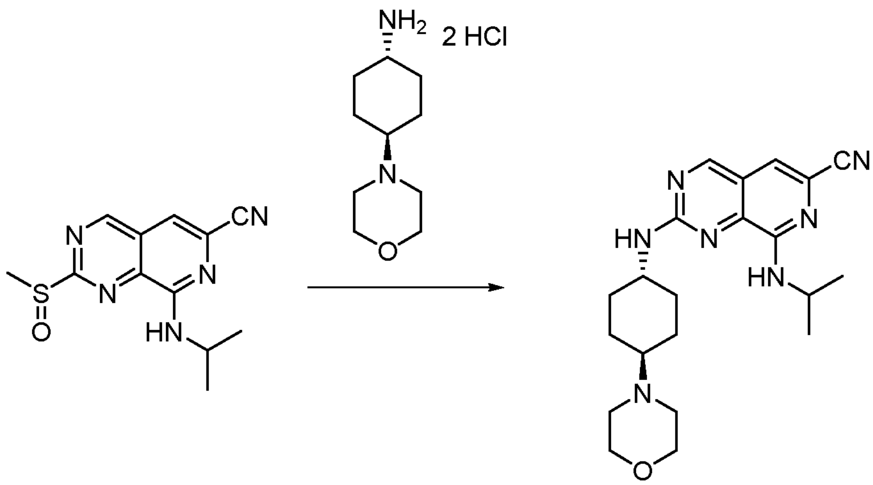

The titled compound was prepared as same as example 89 using corresponding chemicals.

Molecular formula: $C_{21}H_{29}N_7O$ Exact mass: 395.24 LC-MS (Pos, *m*/*z*): 396.1 $[M+H]^+$.

**Example 142: Synthesis of (S)-7-(azepan-3-ylamino)-1-(isopropylamino)-2,6-naphthyridine-3-carbonitrile (Compound 142)**

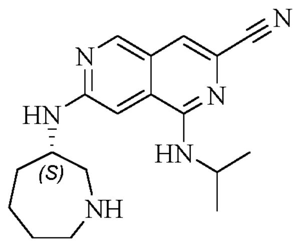

The titled compound was prepared as same as example 38 using corresponding chemicals.

$^1$HNMR (400 MHz, DMSO-$d_6$) δ(ppm): 8.70 (s, 1H), 7.47 (s, 1H), 7.30-7.28 (d, 1H), 6.98 (s, 1H), 6.81-6.78 (d, 1H), 4.36-4.31 (m, 1H), 3.96 (m, 1H), 3.01-2.97 (m, 1H), 2.84-2.75 (m, 2H), 2.72-2.67 (m, 1H), 1.85-1.83 (m, 1H), 1.70-1.60 (m, 4H), 1.55-1.50 (m, 1H), 1.26-1.24 (m, 7H).

Molecular formula: $C_{18}H_{24}N_6$ Exact mass: 324.21 LC-MS (Pos, *m*/*z*) =325.20 $[M+H]^+$.

**Example 143: Synthesis of 7-((2-hydroxycyclohexyl)amino)-1-(isopropylamino)-2,6-naphthyridine-3-carbonitrile (Compound 143)**

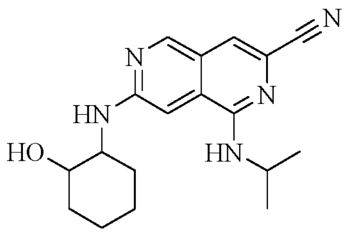

The titled compound was prepared as same as example 44 using corresponding chemicals.

$^1$HNMR (400 MHz, MeOD) δ(ppm): 8.63 (s, 1H), 7.34 (s, 1H), 6.97 (s, 1H), 4.47-4.44 (m, 1H), 3.66-3.61 (m, 1H), 3.53-3.49 (m, 1H), 2.14-2.07 (m, 2H), 1.84-1.75 (m, 2H), 1.49-1.40 (m, 3H), 1.34-1.33 (m, 7H).

Molecular formula: $C_{18}H_{23}N_5O$ Exact mass: 325.19 LC-MS (Pos, *m*/*z)* =326.18$[M+H]^+$.

**Example 144: Synthesis of (*S*)-1-(cyclobutylamino)-7-(piperidin-3-ylamino)-2,6-naphthyridine-3-carbonitrile (Compound 144)**

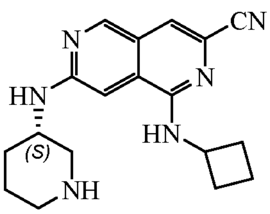

Step 1: synthesis of *tert*-butyl (*S*)-3-((7-cyano-5-(methylthio)-2,6-naphthyridin-3-yl)amino)piperidine-1-carboxylate

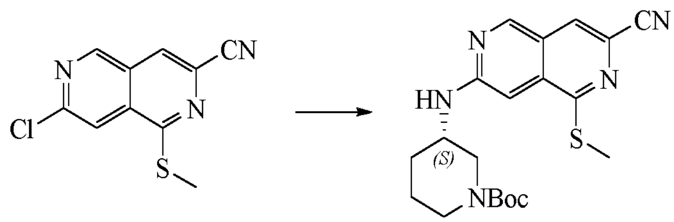

7-chloro-1-(methylthio)-2,6-naphthyridine-3-carbonitrile (500 mg, 2.12 mmol, 1.0 eq.), *tert*-butyl (*S*)-3-aminopiperidine-1-carboxylate (509 mg, 2.54 mmol, 1.2 eq.), $Pd_2(dba)_3$ (192 mg, 0.21 mmol, 0.1 eq.), BINAP (131 mg, 0.21 mmol, 0.1 eq.) and cesium carbonate (1.38 g, 4.24 mmol, 2.0 eq.) were dispersed in 1,4-dioxane (10 mL), and the reaction solution was reacted at 100 °C for 16 h in nitrogen atmosphere. After the reaction was completed, as detected by TLC, the reaction solution was concentrated under reduced pressure to give a crude product, which was purified by silica gel column chromatography (PE:EA = 2: 1) to give the product (847 mg, yield: 100%).

Step 2: synthesis of *tert*-butyl (*S*)-3-((7-cyano-5-(methylsulfonyl)-2,6-naphthyridin-3-yl)amino)piperidine-1-carboxylate

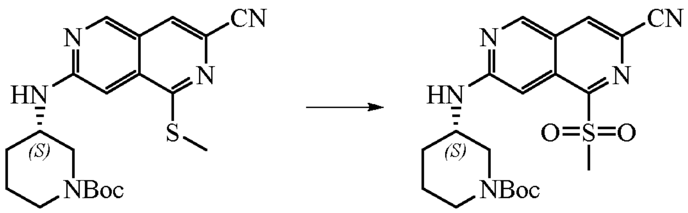

*Tert*-butyl (*S*)-3-((7-cyano-5-(methylthio)-2,6-naphthyridin-3-yl)amino)piperidine-1-carboxylate (847 mg, 2.12 mmol, 1.0 eq.) was dissolved in DCM (15 mL),and meta-chloroperoxybenzoic acid (mass fraction: 85%; 861 mg, 4.24 mmol, 2.0 eq.) was added. After the reaction was completed, as detected by TLC, the reaction solution was poured into saturated aqueous sodium bicarbonate solution (20 mL). The aqueous phase was extracted with DCM (10 mL × 2), and the organic phase was dried and concentrated to give a crude product, which was purified by silica gel column chromatography (PE:EA = 3:1) to give the product (375 mg, yield: 41.0%).

Step 3: synthesis of *tert*-butyl (*S*)-3-((7-cyano-5-(cyclobutylamino)-2,6-naphthyridin-3-yl)amino)piperidine-1-carboxylate

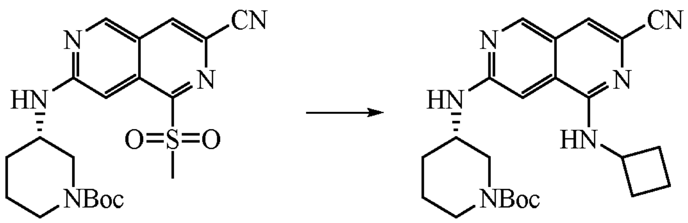

*Tert*-butyl (*S*)-3-((7-cyano-5-(methylsulfonyl)-2,6-naphthyridin-3-yl)amino)piperidine-1-carboxylate (120 mg, 0.27 mmol, 1.0 eq.) and cyclobutylamine (96 mg, 1.35 mmol, 5.0 eq.) were dissolved in THF (3 mL), and the reaction solution was reacted at 60 °C for 24 h. After the reaction was completed, as detected by TLC, the reaction solution was concentrated under reduced pressure to give a crude product, which was purified by silica gel column chromatography (PE:EA= 3:1) to give the product (100 mg, yield: 87.7%).

Step 4: synthesis of (*S*)-1-(cyclobutylamino)-7-(piperidin-3-ylamino)-2,6-naphthyridine-3-carbonitrile

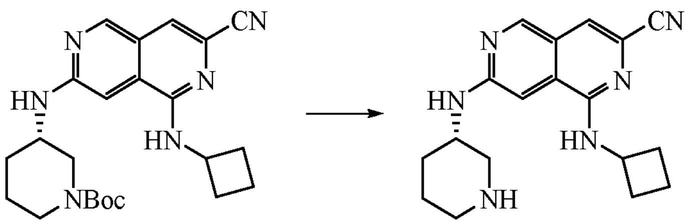

*Tert*-butyl (*S*)-3-((7-cyano-5-(cyclobutylamino)-2,6-naphthyridin-3-yl)amino) piperidine-1-carboxylate (100 mg, 0.20 mmol, 1.0 eq.) was dissolved in EA (2 mL), and a solution of hydrogen chloride in 1,4-dioxane (4 mol/L, 2 mL) was added dropwise. The reaction solution was reacted at room temperature for 1 h. After the reaction was completed, as detected by LC-MS, the reaction solution was poured into saturated aqueous sodium bicarbonate solution (5 mL), and the resulting mixture was extracted with DCM (5 mL × 3). The organic phase was dried and concentrated to give a crude product, which was purified by silica gel preparative thin-layer chromatography (MeOH:DCM = 1:10) to give the product (45 mg, yield: 60.8%).

$^1$HNMR (400 MHz, $CDCl_3$) δ(ppm): 8.68 (s, 1H), 7.32 (s, 1H), 6.38 (s, 1H), 5.41-5.32 (t, 1H), 5.27-5.26 (d, 1H), 4.76-4.66 (m, 1H), 3.81-3.79 (t, 1H), 3.32-3.29 (d, 1H), 2.99-2.96 (d, 1H), 2.79-2.74 (m, 1H), 2.66-2.61 (m, 1H), 2.58-2.51 (m, 2H), 2.04-1.93 (m, 4H), 1.88-1.83 (m, 3H), 1.66-1.59 (m, 2H).

Molecular formula: $C_{18}H_{22}N_6$ Exact mass: 322.19 LC-MS (Pos, *m*/*z*) = 323.11[M+H]$^+$.

**Example 145: Synthesis of 7-((2-aminocyclohexyl)amino)-1-(isopropylamino)-2,6-naphthyridine-3-carbonitrile (Compound 145)**

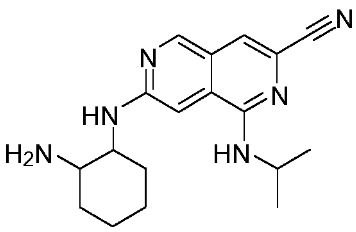

The titled compound was prepared as same as example 38 using corresponding chemicals.

$^1$HNMR (400 MHz, DMSO-*d*$_6$) δ(ppm): 8.70 (s, 1H), 7.47 (s, 1H), 7.30-7.28 (d, 1H), 6.98 (s, 1H), 6.81-6.78 (d, 1H), 4.36-4.31 (m, 1H), 3.96 (m, 1H), 3.01-2.97 (m, 1H), 2.82-2.76 (m, 2H), 2.72-2.67 (m, 1H), 1.87-1.82 (m, 1H), 1.70-1.60 (m, 4H), 1.54-1.50 (m, 1H), 1.25-1.24 (m, 7H).

Molecular formula: $C_{18}H_{24}N_6$ Exact mass: 324.21 LC-MS (Pos, *m*/*z*) =325.23 [M+H]$^+$.

**Example 146: Synthesis of 7-((2-aminocyclopentyl)amino)-1-(isopropylamino)-2,6-naphthyridine-3-carbonitrile (Compound 146)**

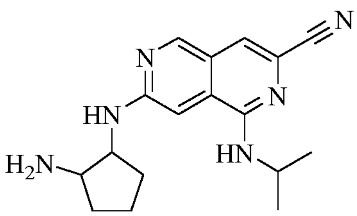

The titled compound was prepared as same as example 38 using corresponding chemicals.

$^1$HNMR (400 MHz, MeOD) δ(ppm): 8.66 (s, 1H), 7.36-7.35 (s, 1H), 6.98-6.93 (s, 1H), 4.48-4.45 (m, 1H), 4.07-4.05 (m, 0.2H), 3.59-3.53 (m, 0.8H), 3.16-3.13 (m, 0.2H), 2.72-2.66 (m, 0.8H), 2.09-2.03 (m, 1H), 1.83-1.66 (m, 2H), 1.51-1.45 (m, 2H), 1.35-1.33 (m, 7H).

Molecular formula: $C_{17}H_{22}N_6$ Exact mass: 310.19 LC-MS (Pos, *m/z*) =311.2 [M+H]$^+$.

**Example 147: Synthesis of 1-(((*R*)-1-phenytethyt)ammo)-7-(((*S*)-piperidin-3-yl)amino)-2,6-naphthyridine-3-carbonitrile (Compound 147)**

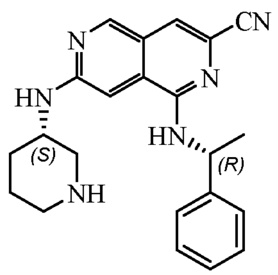

Step 1: synthesis of (*R*)-7-chloro-1-((1-phenylethyl)amino)-2,6-naphthyridine-3-carbonitrile

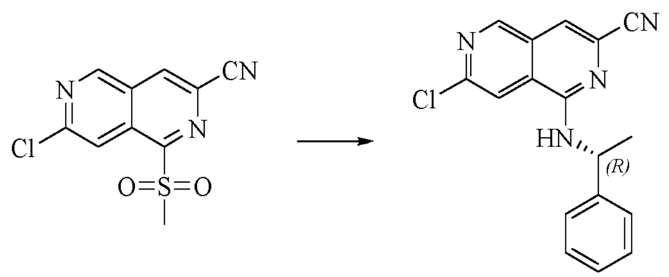

7-chloro-1-(methylsulfonyl)-2,6-naphthyridine-3-carbonitrile (200 mg, 0.75 mmol, 1.0 eq.) and (*R*)-1-phenylethane-1-amine (273 mg, 2.25 mmol, 3.0 eq.) were dissolved in THF (4 mL), and the reaction solution was reacted at 60 °C for 4 h. After the reaction was completed, as detected by TLC, the reaction solution was concentrated under reduced pressure to give a crude product, which was purified by silica gel column chromatography (PE:EA= 5:1) to give the product (200 mg, yield: 86.6%).

Step 2: synthesis of *tert*-butyl (*S*)-3-((7-cyano-5-(((*R*)-1-phenylethyl)amino)-2,6-naphthyridin-3-yl)amino)piperidine-1-carboxylate

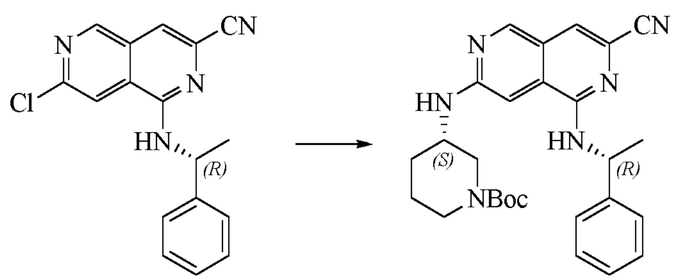

(*R*)-7-chloro-1-((1-phenylethyl)amino)-2,6-naphthyridine-3-carbonitrile (200 mg, 0.65 mmol, 1.0 eq.), *tert*-butyl (*S*)-3-aminopiperidine-1-carboxylate (156 mg, 0.78 mmol, 1.2 eq.), $Pd_2(dba)_3$ (64 mg, 0.07 mmol, 0.1 eq.), BINAP (44 mg, 0.07 mmol, 0.1 eq.) and cesium carbonate (424 mg, 1.30 mmol, 2.0 eq.) were dispersed in 1,4-dioxane (5 mL), and the reaction solution was reacted at 100 °C for 12 h in nitrogen atmosphere. After the reaction was completed, as detected by TLC, the reaction solution was concentrated under reduced pressure to give a crude product, which was purified by silica gel column chromatography (PE:EA = 2:1) to give the product (220 mg, yield: 71.4%).

Step 3: synthesis of 1-(((*R*)-1-phenylethyl)amino)-7-(((*R*)-piperidin-3-yl)amino)-2,6-naphthyridine-3-carbonitrile

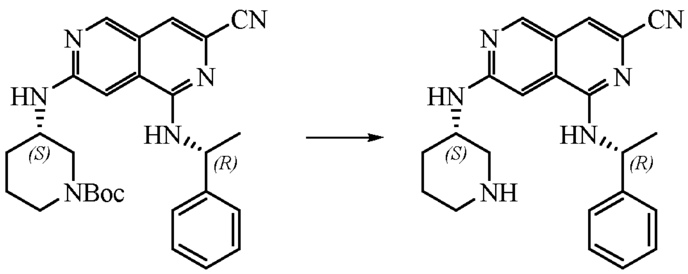

*Tert*-butyl (*S*)-3-((7-cyano-5-(((*R*)-1-phenylethyl)amino)-2,6-naphthyridin-3-yl) amino)piperidine-1-carboxylate (220 mg, 0.46 mmol, 1.0 eq.) was dissolved in EA (2 mL), and a solution of hydrogen chloride in 1,4-dioxane (4 mol/L, 2 mL) was added dropwise. The reaction solution was reacted at room temperature for 1.5 h. After the reaction was completed, as detected by LC-MS, the reaction solution was concentrated under reduced pressure to give a crude product, which was dissolved in water (5 mL). The aqueous phase was adjusted to pH = 9 with saturated aqueous $NaHCO_3$ solution and then extracted with DCM (10 mL × 3). The organic phase was dried and concentrated to give a crude product, which was purified by silica gel preparative thin-layer chromatography (MeOH:DCM = 1:10) to give the product (120 mg, yield: 70.2%).

$^1$HNMR (400 MHz, $CDCl_3$) δ(ppm): 8.67 (s, 1H), 7.49-7.47 (d, 2H), 7.40-7.36 (t, 2H), 7.31-7.29 (d, 2H), 6.41 (s, 1H), 5.57-5.51 (m, 2H), 5.28-5.26 (d, 1H), 3.83-3.81 (t, 1H), 2.29-3.26 (d,1H), 2.96-2.92 (t, 1H), 2.78-2.74 (t, 1H), 2.66-2.61 (m, 1H), 2.00 (s, 3H), 1.84-1.80 (m, 1H), 1.69-1.68 (d, 3H), 1.62-1.60 (d, 1H).

Molecular formula: $C_{22}H_{24}N_6$ Exact mass: 372.21 LC-MS (Pos, *m*/*z*) = 373.18[M+H]$^+$.

**Example 148: Synthesis of (*S*)-1-((1-methylcyclopropyl)amino)-7-(piperidin-3-ylamino)-2,6-naphthyridine-3-carbonitrile (Compound 148)**

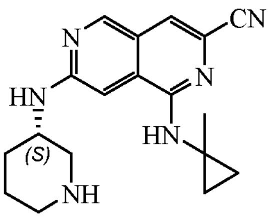

Step 1: synthesis of 7-chloro-1-((1-methylcyclopropyl)amino)-2,6-naphthyridine-3-carbonitrile

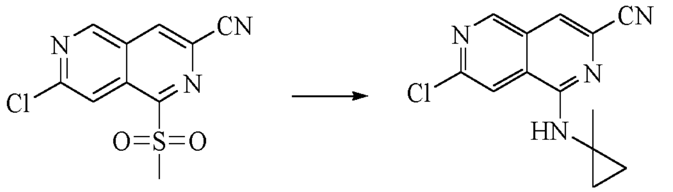

7-Chloro-1-(methylsulfonyl)-2,6-naphthyridine-3-carbonitrile (200 mg, 0.75 mmol, 1.0 eq.), 1-methylcyclopropan-1-amine hydrochloride (242 mg, 2.25 mmol, 3.0 eq.) and TEA (380 mg, 3.75 mmol, 5.0 eq.) were dissolved in THF (5 mL), and the reaction solution was reacted at 60 °C for 4.5 h. After the reaction was completed as detected by TLC, the reaction solution was concentrated under reduced pressure, and the residue was purified by silica gel column chromatography (PE:EA = 5:1) to give the product (150 mg, yield: 77.3%).

Step 2: synthesis of *tert*-butyl (*S*)-3-((7-cyano-5-((1-methylcyclopropyl)amino)-2,6-naphthyridin-3-yl)amino)piperidine-1-carboxylate

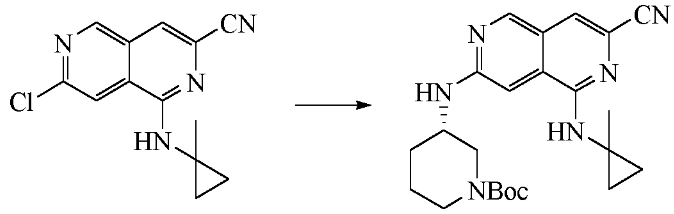

7-Chloro-1-((1-methylcyclopropyl)amino)-2,6-naphthyridine-3-carbonitrile (150 mg, 0.58 mmol, 1.0 eq.), *tert*-butyl (*S*)-3-aminopiperidine-1-carboxylate (140 mg, 0.70 mmol, 1.2 eq.), $Pd_2(dba)_3$ (55 mg, 0.06 mmol, 0.1 eq.), BINAP (37 mg, 0.06 mmol, 0.1 eq.) and cesium carbonate (378 mg, 1.16 mmol, 2.0 eq.) were dispersed in 1,4-dioxane (5 mL), and the reaction solution was reacted at 100 °C for 12 h in nitrogen atmosphere. After the reaction was completed as detected by TLC, the reaction solution was concentrated under reduced pressure, and the crude product was purified by silica gel column chromatography (PE:EA = 2: 1) to give the product (150 mg, yield: 61.2%).

Step 3: synthesis of (*S*)-1-((1-methylcyclopropyl)amino)-7-(piperidin-3-ylamino)-2,6-naphthyridine-3-carbonitrile

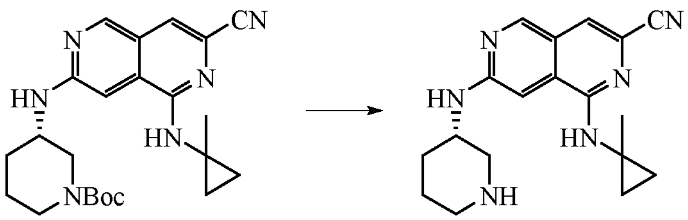

*tert*-Butyl (*S*)-3-((7-cyano-5-((1-methylcyclopropyl)amino)-2,6-naphthyridin-3-yl)amino)piperidine-1-carboxylate (150 mg, 0.35 mmol, 1.0 eq.) was dissolved in EA (2 mL), and a solution of hydrogen chloride in 1,4-dioxane (4 mol/L, 2 mL) was added dropwise. The reaction solution was reacted at room temperature for 1.5 h. After the reaction was completed as detected by LC-MS, the reaction solution was concentrated under reduced pressure to give a crude product, which was dissolved in water (5 mL). The aqueous phase was adjusted to pH = 9 with saturated aqueous $NaHCO_3$ solution and then extracted with DCM (10 mL × 3). The organic phase was dried and concentrated to give a crude product, which was purified by silica gel preparative thin-layer chromatography (MeOH:DCM = 1:10) to give the product (75 mg, yield: 66.3%).

$^1$HNMR (400 MHz, $CDCl_3$) δ(ppm): 8.68 (s, 1H), 7.35 (s, 1H), 6.30 (s, 1H), 5.65 (s, 1H), 5.26-5.25 (d, 1H), 3.77-3.75 (t, 1H), 3.30-3.28 (d, 1H), 2.98-2.94 (t, 1H), 2.78-2.73 (t, 1H), 2.65-2.60 (m, 1H), 2.02-1.93 (t,3H), 1.85-1.81 (m, 1H), 1.62-1.60 (d, 2H), 1.52 (s, 3H), 0.87-0.85 (t, 2H), 0.81-0.78 (t, 3H).

Molecular formula: $C_{18}H_{22}N_6$ Exact mass: 322.19 LC-MS (Pos, *m*/*z*) = 323.20[M+H]$^+$.

**Example 149: Synthesis of (*S*)-1-(*tert*-butylamino)-7-(piperidin-3-ylamino)-2,6-naphthyridine-3-carbonitrile (Compound 149)**

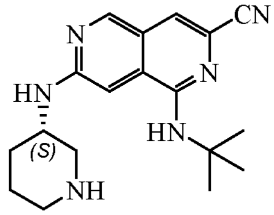

Step 1: synthesis of 1-(*tert*-butylamino)-7-chloro-2,6-naphthyridine-3-carbonitrile

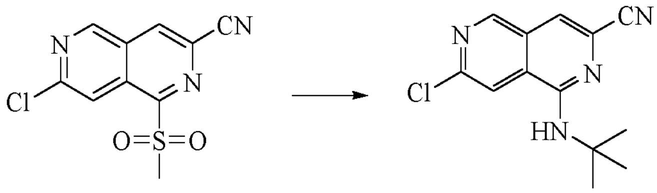

7-Chloro-1-(methylsulfonyl)-2,6-naphthyridine-3-carbonitrile (200 mg, 0.75 mmol, 1.0 eq.) and *tert*-butylamine (274 mg, 3.75 mmol, 5.0 eq.) were dissolved in THF (4 mL), and the reaction solution was reacted at 55 °C for 22 h. After the reaction was completed as detected by TLC, the reaction solution was concentrated under reduced pressure, and the crude product was purified by silica gel column chromatography (PE:EA = 5:1) to give the product (175 mg, yield: 89.7%).

Step 2: synthesis of *tert*-butyl (*S*)-3-((5-(*tert*-butylamino)-7-cyano-2,6-naphthyridin-3-yl)amino)piperidine-1-carboxylate

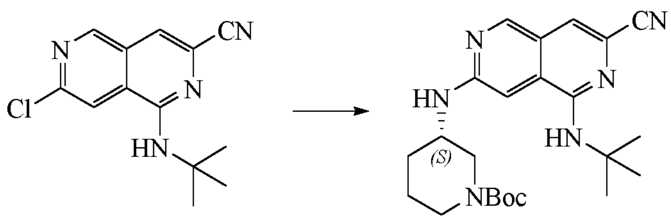

1-(*tert*-Butylatmino)-7-chloro-2,6-naphthyridine-3-carbonitrile (175 mg, 0.67 mmol, 1.0 eq.), *tert*-butyl (*S*)-3-aminopiperidine-1-carboxylate (160 mg, 0.80 mmol, 1.2 eq.), $Pd_2(dba)_3$ (64 mg, 0.07 mmol, 0.1 eq.), BINAP (44 mg, 0.07 mmol, 0.1 eq.) and cesium carbonate (437 mg, 1.34 mmol, 2.0 eq.) were dispersed in 1,4-dioxane (5 mL), and the reaction solution was reacted at 100 °C for 16 h in nitrogen atmosphere. After the reaction was completed as detected by TLC, the reaction solution was concentrated under reduced pressure, and the crude product was purified by silica gel column chromatography (PE:EA = 3:1) to give the product (220 mg, yield: 77.4%).

Step 3: synthesis of (*S*)-1-(*tert*-butylamino)-7-(piperidin-3-ylamino)-2,6-naphthyridine-3-carbonitrile

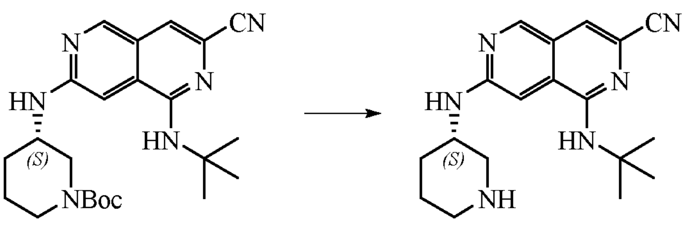

*tert*-Butyl (*S*)-3-((5-(*tert*-butylamino)-7-cyano-2,6-naphthyridin-3-yl)amino)piperidine-1-carboxylate (220 mg, 0.52 mmol, 1.0 eq.) was dissolved in EA (2 mL), and a solution of hydrogen chloride in 1,4-dioxane (4 mol/L, 2 mL) was added dropwise. The reaction solution was reacted at room temperature for 1 h. After the reaction was completed as detected by LC-MS, the reaction solution was concentrated under reduced pressure to give a crude product, which was dissolved in water (5 mL). The aqueous phase was adjusted to pH = 9 with saturated aqueous potassium carbonate solution and then extracted with DCM (10 mL × 3). The organic phase was dried and concentrated to give a crude product, which was purified by silica gel preparative thin-layer chromatography (MeOH:DCM = 1:10) to give the product (125 mg, yield: 74.4%).

$^1$HNMR (400 MHz, $CDCl_3$) δ(ppm): 8.66 (s, 1H), 7.29 (s, 1H), 6.31 (s, 1H), 5.28-5.26 (d, 1H), 5.05 (s, 1H), 3.88-3.86 (t, 1H), 3.27-3.24 (m, 1H), 2.96-2.92 (m, 1H), 2.80-2.75 (m, 1H), 2.70-2.65 (m, 1H), 1.99 (s, 2H), 1.83-1.78 (m, 1H), 1.64-1.60 (m, 2H), 1.57 (s, 9H).

Molecular formula: $C_{18}H_{24}N_6$ Exact mass: 324.21 LC-MS (Pos, *m/z*) = 325.12[M+H]$^+$.

**Example 150: Synthesis of 7-(((S)-piperidin-3-yl)amino)-1-(((R)-tetrahydrofuran-3-yl)amino)-2,6-naphthyridine-3-carbonitrile (Compound 150)**

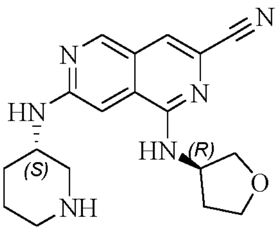

The titled compound was prepared as same as example 49 using corresponding chemicals.

$^1$HNMR (400 MHz, MeOD) δ(ppm): 8.70 (s, 1H), 7.43 (s, 1H), 6.95 (s, 1H), 4.75-4.74 (m, 1H), 4.12-4.03 (m, 2H), 3.91-3.79 (m, 3H), 3.27-3.24 (m, 1H), 3.01-2.98 (m, 1H), 2.68-2.62 (m, 1H), 2.56-2.50 (m, 1H), 2.43-2.39 (m, 1H), 2.14-2.07 (m, 2H), 1.87-1.82 (m, 1H), 1.70-1.54 (m, 2H).

Molecular formula: $C_{18}H_{22}N_6O$ Exact mass: 338.19 LC-MS (Pos, *m/z*) =339.19 [M+H]$^+$.

**Example 151: Synthesis of 7-(((S)-piperidin-3-yl)amino)-1-(((S)-tetrahydrofuran-3-yl)amino)-2,6-naphthyridine-3-carbonitrile (Compound 151)**

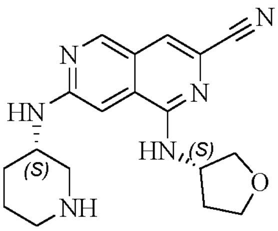

The titled compound was prepared as same as example 49 using corresponding chemicals.

$^1$HNMR (400 MHz, MeOD) δ(ppm): 8.69 (s, 1H), 7.43 (s, 1H), 6.94 (s, 1H), 4.76-4.73 (m, 1H), 4.13-4.04 (m, 2H), 3.90-3.79 (m, 3H), 3.26-3.24 (m, 1H), 3.00-2.97 (m, 1H), 2.64-2.60 (m, 1H), 2.53-2.48 (m, 1H), 2.43-2.39 (m, 1H), 2.13-2.10 (m, 2H), 1.85-1.82 (m, 1H), 1.68-1.54 (m, 2H).

Molecular formula: $C_{18}H_{22}N_6O$ Exact mass: 338.19 LC-MS (Pos, *m/z*) =339.19 [M+H]$^+$.

**Example 152: Synthesis of (S)-1-(tert-butoxy)-7-(piperidin-3-ylamino)-2,6-naphthyridine-3-carbonitrile (Compound 152)**

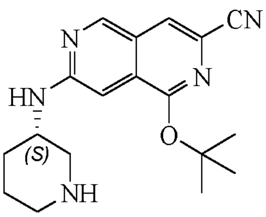

The titled compound was prepared as same as example 153 using corresponding chemicals.

$^1$HNMR (400 MHz, MeOD) δ(ppm): 8.80 (s, 1H), 7.74 (s, 1H), 6.94 (s, 1H), 3.99-3.94 (m, 1H), 3.30-3.26 (m, 1H), 3.00-2.96 (m, 1H), 2.67-2.60 (m, 1H), 2.54-2.49 (m, 1H), 2.12-2.08 (m, 1H), 1.86-1.81 (m, 1H), 1.73 (s, 9H), 1.70-1.67 (m, 1H), 1.66-1.64 (t, 1H).

Molecular formula:$C_{18}H_{23}N_5O$ Exact mass: 325.19 LC-MS(Pos, *m*/*z*)=326.14[M+H]$^+$.

**Example 153: Synthesis of (*S*)-1-(isopropoxy)-7-(piperidin-3-ylamino)-2,6-naphthyridine-3-carbonitrile (Compound 153)**

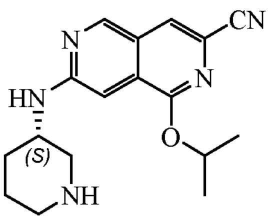

Step 1: synthesis of *tert*-butyl (*S*)-3-((5-(isopropoxy)-7-cyano-2,6-naphthyridin-3-yl)amino)piperidine-1-carboxylate

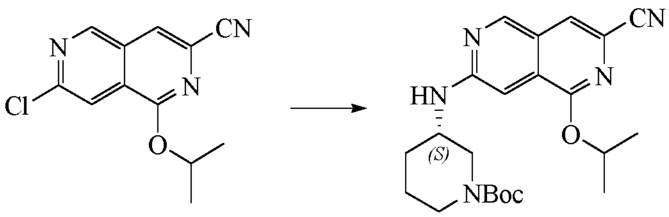

1-(Isopropoxy)-7-chloro-2,6-naphthyridine-3-carbonitrile (120 mg, 0.48 mmol, 1.0 eq.), *tert*-butyl (*S*)-3-aminopiperidine-1-carboxylate (116 mg, 0.58 mmol, 1.2 eq.), $Pd_2(dba)_3$ (46 mg, 0.05 mmol, 0.1 eq.), BINAP (31 mg, 0.05 mmol, 0.1 eq.) and cesium carbonate (313 mg, 0.96 mmol, 2.0 eq.) were dispersed in 1,4-dioxane (5 mL), and the reaction solution was reacted at 100 °C for 14 h in nitrogen atmosphere. After the reaction was completed as detected by TLC, the reaction solution was concentrated under reduced pressure, and the crude product was purified by silica gel column chromatography (PE:EA = 6:1) to give the product (150 mg, yield: 76.1%).

Step 2: synthesis of (*S*)-1-(isopropoxy)-7-(piperidin-3-ylamino)-2,6-naphthyridine-3-carbonitrile

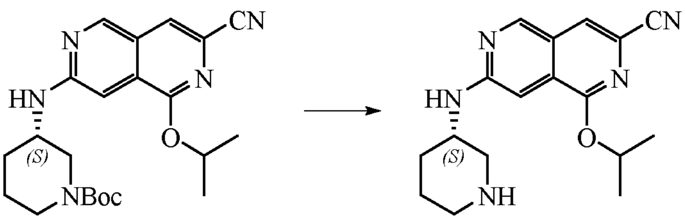

*tert*-Butyl (*S*)-3-((5-(isopropoxy)-7-cyano-2,6-naphthyridin-3-yl)amino)piperidine-1-carboxylate (150 mg, 0.36 mmol, 1.0 eq.) was dissolved in DCM (5 mL) and 2,6-dimethylpyridine (193 mg, 1.80 mmol, 5.0 eq.), and TMSOTf (240 mg, 1.08 mmol, 3.0 eq.) was added dropwise. The reaction solution was reacted at room temperature for 10 min. After the reaction was completed as detected by TLC, the reaction solution was poured into water (10 mL), and the resulting mixture was extracted with DCM (10 mL × 2). The organic phase was dried and concentrated to give a crude product, which was purified by silica gel preparative thin-layer chromatography (MeOH:DCM = 1: 10) to give the product (80 mg, yield: 71.4%).

$^1$HNMR (400 MHz, MeOD) δ(ppm): 8.81 (s, 1H), 7.75 (s, 1H), 6.95 (s, 1H), 5.53-5.47 (m, 1H), 3.96-3.91 (s,

1H), 3.29-3.25 (m, 1H), 2.99-2.95 (m, 1H), 2.65-2.59 (m, 1H), 2.53-2.47 (m, 1H), 2.12-2.09 (t, 1H), 1.85-1.80 (m, 1H), 1.70-1.61 (m, 2H), 1.58-1.52 (m, 1H), 1.47-1.45 (d, 6H).

Molecular formula: $C_{17}H_{21}N_5O$ Exact mass: 311.17 LC-MS (Pos, *m/z*) = 312.07[M+H]$^+$.

**Example 154: Synthesis of 7-(((*S*)-piperidin-3-yl)amino)-1-(((*R*)-tetrahydrofuran-3-yl)oxy)-2,6-naphthyridine-3-carbonitrile (Compound 154)**

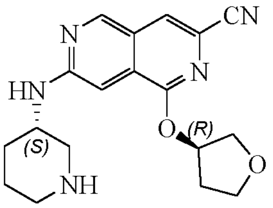

Step 1: synthesis of (*R*)-7-chloro-1-((tetrahydrofuran-3-yl)oxy)-2,6-naphthyridine-3-carbonitrile

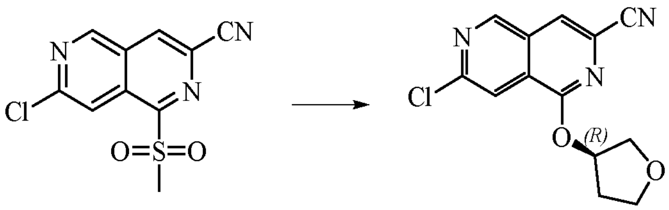

(*R*)-tetrahydrofuran-3-ol (78 mg, 0.89 mmol, 1.2 eq.) and sodium hydride (mass fraction: 60%; 38 mg, 0.96 mmol, 1.3 eq.) were dispersed in THF (4 mL), and the reaction solution was reacted at room temperature for 10 min. 7-chloro-1-(methylsulfonyl)-2,6-naphthyridine-3-carbonitrile (200 mg, 0.74 mmol, 1.0 eq.) was added, and the resulting reaction solution was reacted for 10 min. After the reaction was completed, as detected by TLC, the reaction solution was poured into water (5 mL), and EA (10 mL × 2) was added for extraction. The organic phase was dried and concentrated to give a crude product, which was purified by silica gel column chromatography (PE:EA= 3:1) to give the product (125 mg, yield: 61.3%).

Step 2: synthesis of *tert*-butyl (*S*)-3-((7-cyano-5-(((*R*)-tetrahydrofuran-3-yl)oxy)-2,6-naphthyridin-3-yl)amino)piperidine-1-carboxylate

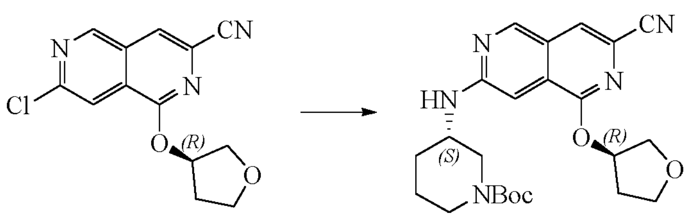

(*R*)-7-chloro-1-((tetrahydrofuran-3-yl)oxy)-2,6-naphthyridine-3-carbonitrile (120 mg, 0.44 mmol, 1.0 eq.), *tert*-butyl (*S*)-3-aminopiperidine-1-carboxylate (106 mg, 0.53 mmol, 1.2 eq.), $Pd_2(dba)_3$ (37 mg, 0.04 mmol, 0.1 eq.), BINAP (25 mg, 0.04 mmol, 0.1 eq.) and cesium carbonate (287 mg, 0.88 mmol, 2.0 eq.) were dispersed in 1,4-dioxane (5 mL), and the reaction solution was reacted at 100 °C for 14 h in nitrogen atmosphere. After the reaction was completed, as detected by TLC, the reaction solution was concentrated under reduced pressure to give a crude product, which was purified by silica gel column chromatography (PE:EA = 3:1) to give the product (150 mg, yield: 77.7%).

Step 3: synthesis of 7-(((*S*)-pipeiidin-3-yl)amino)-1-(((*R*)-tetrahydrofuran-3-yl)oxy)-2,6-naphthyridine-3-carbonitrile

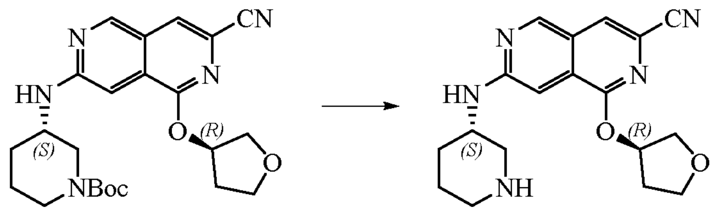

*Tert*-butyl (*S*)-3-((7-cyano-5-(((*R*)-tetrahydrofuran-3-yl)oxy)-2,6-naphthyridin-3-yl) amino)piperidine-1-carboxylate (150 mg, 0.34 mmol, 1.0 eq.) was dissolved in DCM (5 mL) and 2,6-dimethylpyridine (182 mg, 1.70 mmol, 5 eq.), and TMSOTf (227 mg, 1.02 mmol, 3 eq.) was added dropwise. The reaction solution was reacted at room temperature for 10 min. After the reaction was completed, as detected by TLC, the reaction solution was poured into water (5 mL), and the resulting mixture was extracted with DCM (10 mL × 2). The organic phase was dried and concentrated to give a crude product, which was purified by silica gel preparative thin-layer chromatography (MeOH:DCM = 1:10) to give the product (95 mg, yield: 82.6%).

$^1$HNMR (400 MHz, MeOD) δ(ppm): 8.84 (s, 1H), 7.81 (s, 1H), 6.94 (s, 1H), 5.77-5.75 (t, 1H), 4.12-4.02 (m, 3H), 3.97-3.92 (m, 2H), 3.28-3.24 (m, 1H), 2.99-2.95 (m, 1H), 2.64-2.58 (m, 1H), 2.52-2.37 (m, 2H), 2.29-2.23 (m, 1H), 2.12-2.08 (m, 1H), 1.85-1.80 (m, 1H), 1.51-1.47 (m, 2H).

Molecular formula: $C_{18}H_{21}N_5O_2$ Exact mass: 339.17 LC-MS (Pos, *m*/*z*) = 340.10[M+H]$^+$.

**Example 155: Synthesis of 7-(((*S*)-piperidin-3-yl)amino)-1-(((*S*)-tetrahydrofuran-3-yl)oxy)-2,6-naphthyridine-3-carbonitrile (Compound 155)**

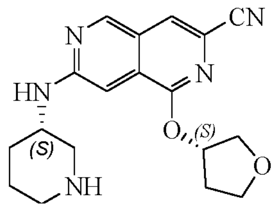

Step 1: synthesis of (*S*)-7-chloro-1-((tetrahydrofuran-3-yl)oxy)-2,6-naphthyridine-3-carbonitrile

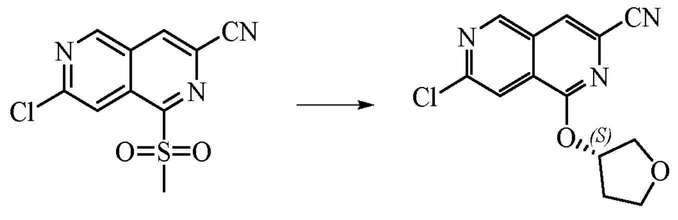

(*R*)-tetrahydrofuran-3-ol (78 mg, 0.89 mmol, 1.2 eq.) and sodium hydride (mass fraction: 60%; 38 mg, 0.96 mmol, 1.3 eq.) were dispersed in THF (4 mL), and the reaction solution was reacted at room temperature for 10 min. 7-chloro-1-(methylsulfonyl)-2,6-naphthyridine-3-carbonitrile (200 mg, 0.74 mmol, 1.0 eq.) was added, and the resulting reaction solution was reacted for 30 min. After the reaction was completed, as detected by TLC, the reaction solution was poured into water (5 mL), and EA (10 mL × 2) was added for extraction. The organic phase was dried and concentrated to give a crude product, which was purified by silica gel column chromatography (PE:EA= 5:1) to give the product (120 mg, yield: 58.8%).

Step 2: synthesis of *tert*-butyl (*S*)-3-((7-cyano-5-(((*S*)-tetrahydrofuran-3-yl)oxy)-2,6-naphthyridin-3-yl)amino)piperidine-1-carboxylate

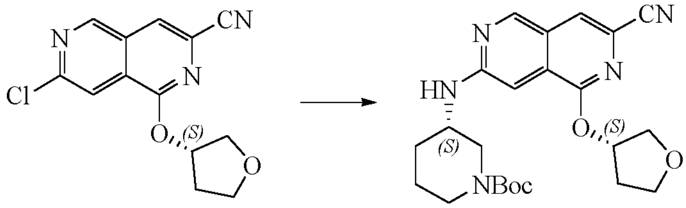

(*S*)-7-chloro-1-((tetrahydrofuran-3-yl)oxy)-2,6-naphthyridine-3-carbonitrile (120 mg, 0.44 mmol, 1.0 eq.), *tert*-butyl (*S*)-3-aminopiperidine-1-carboxylate (106 mg, 0.53 mmol, 1.2 eq.), $Pd_2(dba)_3$ (37 mg, 0.04 mmol, 0.1 eq.), BINAP (25 mg, 0.04 mmol, 0.1 eq.) and cesium carbonate (287 mg, 0.88 mmol, 2.0 eq.) were dispersed in 1,4-dioxane (5 mL), and the reaction solution was reacted at 100 °C for 14 h in nitrogen atmosphere. After the reaction was completed, as detected by TLC, the reaction solution was concentrated under reduced pressure to give a crude product, which was purified by silica gel column chromatography (PE:EA = 3:1) to give the product (150 mg, yield: 77.7%).

Step 3: synthesis of 7-(((*S*)-piperidin-3-yl)amino)-1-(((*S*)-tetrahydrofuran-3-yl)oxy)-2,6-naphthyridine-3-carbonitrile

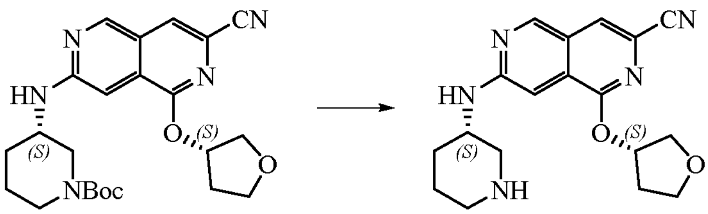

*Tert*-butyl (*S*)-3-((7-cyano-5-(((*S*)-tetrahydrofuran-3-yl)oxy)-2,6-naphthyridin-3-yl) amino)piperidine-1-carboxylate (150 mg, 0.34 mmol, 1.0 eq.) was dissolved in DCM (3 mL) and 2,6-dimethylpyridine (547 mg, 5.10 mmol, 15.0 eq.), and TMSOTf (756 mg, 3.40 mmol, 10.0 eq.) was added dropwise. The reaction solution was reacted at room temperature for 10 min. After the reaction was completed, as detected by TLC, the reaction solution was poured into water (5 mL), and the resulting mixture was extracted with DCM (10 mL × 2). The organic phase was dried and concentrated to give a crude product, which was slurried with DCM (2 mL) to give the product (85 mg, yield: 73.9%).

$^1$HNMR (400 MHz, MeOD) δ(ppm): 8.84 (s, 1H), 7.81 (s, 1H), 6.94 (s, 1H), 5.77-5.75 (t, 1H), 4.12-4.02 (m, 3H), 3.96-3.91 (m, 2H), 3.27-3.23 (m,1H), 2.97-2.94 (m, 1H), 2.63-2.56 (m, 1H), 2.50-2.41 (m, 2H), 2.29-2.24 (m, 1H), 2.11-2.08 (t, 1H), 1.83-1.79 (m, 1H), 1.67-1.63 (m, 1H), 1.57-1.47 (m, 2H).

Molecular formula: $C_{18}H_{21}N_5O_2$ Exact mass: 339.17 LC-MS (Pos, *m/z*) = 340.08[M+H]$^+$.

**Example 156: synthesis of 7-(((1S,2S)-2-hydroxycyclohexyl)amino)-1-(isopropylamino)-2,6-naphthyridine-3-carbonitrile (Compound 156)**

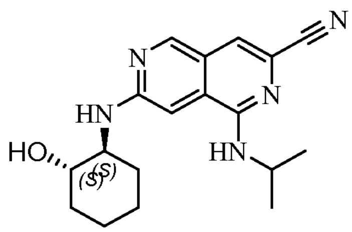

The titled compound was prepared as same as example 44 using corresponding chemicals.

$^1$HNMR (400 MHz, MeOD) δ(ppm): 8.63 (s, 1H), 7.35-7.34 (s, 1H), 6.97 (s, 1H), 4.47-4.42 (m, 1H), 3.67-3.61 (m, 1H), 3.53-3.47 (m, 1H), 2.13-2.06 (m, 2H), 1.84-1.75 (m, 2H), 1.49-1.40 (m, 3H), 1.35-1.33 (m, 7H).
Molecular formula: $C_{18}H_{23}N_5O$ Exact mass: 325.19 LC-MS (Pos, *m*/*z*) =326.21[M+H]$^+$.

**Example 157: synthesis of 7-(((1S,2S)-2-aminocyclopentyl)amino)-1-(isopropylamino)-2,6-naphthyridine-3-carbonitrile (Compound 157)**

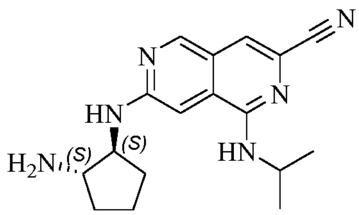

The titled compound was prepared as same as example 38 using corresponding chemicals.
$^1$HNMR (400 MHz, MeOD) δ(ppm): 8.67 (s, 1H), 7.35 (s, 1H), 6.98 (s, 1H), 4.47-4.44 (m, 1H), 4.14-4.12 (m, 1H), 3.56-3.53 (m, 1H), 2.14-2.05 (m, 2H), 1.91-1.88 (m, 1H), 1.77-1.62 (m, 3H), 1.34-1.33 (m, 6H).
Molecular formula: $C_{17}H_{22}N_6$ Exact mass: 310.19 LC-MS (Pos, *m*/*z*) =311.2 [M+H]$^+$.

**Example 158: synthesis of 7-(((1S,2R)-2-aminocyclopentyl)amino)-1-(isopropylamino)-2,6-naphthyridine-3-carbonitrile (Compound 158)**

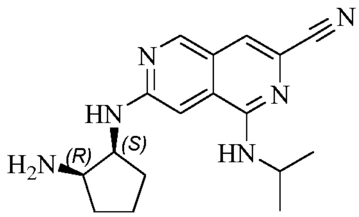

The titled compound was prepared as same as example 38 using corresponding chemicals.
$^1$HNMR (400 MHz, MeOD) δ(ppm): 8.66 (s, 1H), 7.34 (s, 1H), 6.92 (s, 1H), 4.47-4.44 (m, 1H), 3.87-3.85 (m, 1H), 3.21-3.16 (m, 1H), 2.35-2.28 (m, 1H), 2.12-2.03 (m, 1H), 1.91-1.80 (m, 2H), 1.62-1.50 (m, 2H), 1.35-1.32 (m, 6H).
Molecular formula: $C_{17}H_{22}N_6$ Exact mass: 310.19 LC-MS (Pos, *m*/*z*) =311.2 [M+H]$^+$.

**Example 159: synthesis of 7-(((1*R*,2*R*)-2-hydroxycyclohexyl)amino)-1-(isopropylamino)-2,6-naphthyridine-3-carbonitrile (Compound 159)**

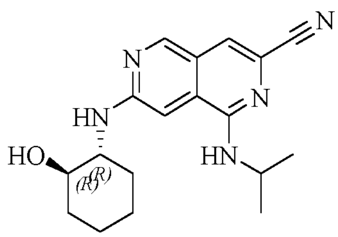

Step 1: synthesis of 7-(((1*R*,2*R*)-2-hydroxycyclohexyl)amino)-1-(isopropylamino)-2,6-naphthyridine-3-carbonitrile

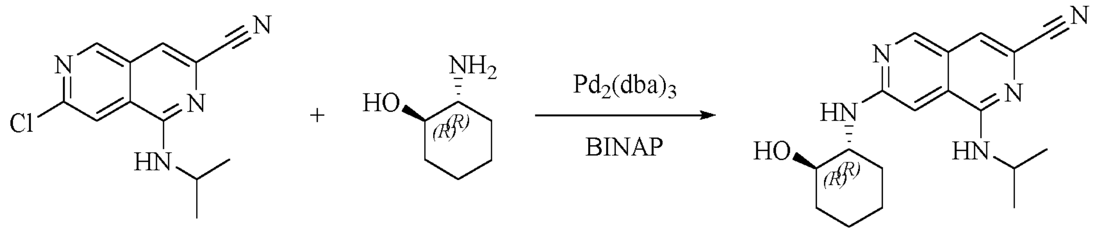


7-chloro-1-(isopropylamino)-2,6-naphthyridine-3-carbonitrile (100 mg, 0.41 mmol, 1.0 eq.), (1*R*,2*R*)-2-aminocyclohexyl-1-ol (61 mg, 0.53 mmol, 1.3 eq.), $Pd_2(dba)_3$ (54.9 mg, 0.06 mmol, 0.15 eq.), BINAP (74.7 mg, 0.12 mmol, 0.3 eq.) and cesium carbonate (332.3 mg, 1.02 mmol, 2.5 eq.) were added into 1,4-dioxane (10 mL). The reaction solution was heated to 120 °C and reacted for 18 h in a sealed tube in nitrogen atmosphere. When there were no materials left, as detected by TLC, the reaction solution was cooled to room temperature, water (30 mL) was added, and then ethyl acetate (30 mL × 3) was added for extraction. The organic phases were combined, dried over anhydrous sodium sulfate and filtered, and the filtrate was concentrated under reduced pressure to give a crude product, which was purified by preparative thin-layer chromatography (MeOH:DCM = 1:20) to give the product (65 mg, yield: 48.7%).

$^1$HNMR (400 MHz, MeOD) δ(ppm): 8.63 (s, 1H), 7.34 (s, 1H), 6.96 (s, 1H), 4.48-4.44 (m, 1H), 3.67-3.61 (m, 1H), 3.52-3.47 (m, 1H), 2.13-2.07 (m, 2H), 1.83-1.77 (m, 2H), 1.48-1.40 (m, 10H).

Molecular formula: $C_{18}H_{23}N_5O$ Exact mass: 325.19 LC-MS (Pos, *m*/*z*) = 326.21[M+H]$^+$.

**Example 160: synthesis of 7-(((1*S*,2*R*)-2-hydroxycyclohexyl)amino)-1-(isopropylamino)-2,6-naphthyridine-3-carbonitrile (Compound 160)**

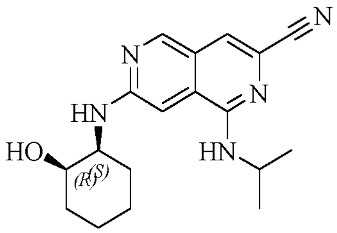

Step 1: synthesis of 7-(((1*S*,2*R*)-2-hydroxycyclohexyl)amino)-1-(isopropylamino)-2,6-naphthyridine-3-carbonitrile

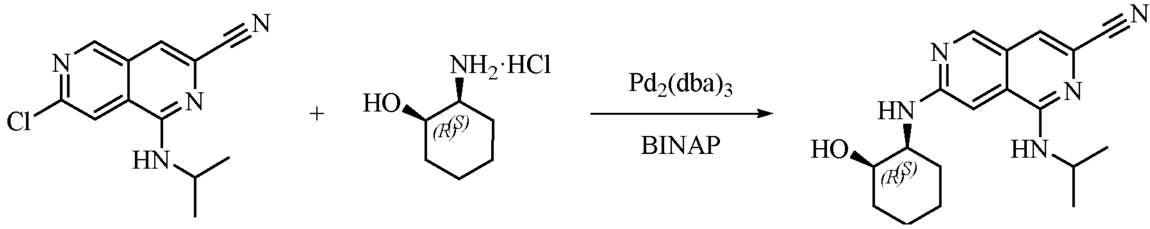


7-chloro-1-(isopropylamino)-2,6-naphthyridine-3-carbonitrile (100 mg, 0.41 mmol, 1.0 eq.), (1*R*,2*S*)-2-aminocyclohexyl-1-ol hydrochloride (80.4 mg, 0.53 mmol, 1.3 eq.), $Pd_2(dba)_3$ (54.9 mg, 0.06 mmol, 0.15 eq.), BINAP (74.7 mg, 0.12 mmol, 0.3 eq.) and cesium carbonate (469.2 mg, 1.44 mmol, 3.5 eq.) were added into 1,4-dioxane (10 mL). The reaction solution was heated to 120 °C and reacted for 18 h in a sealed tube in nitrogen atmosphere. When there were no materials left, as detected by TLC, the reaction solution was cooled to room temperature, water (30 mL) was added, and then ethyl acetate (30 mL × 3) was added for extraction. The organic phases were combined, dried over anhydrous sodium sulfate and filtered, and the filtrate was concentrated under reduced pressure to give a crude product, which was purified by preparative thin-layer chromatography (MeOH:DCM = 1:20) to give the product (70 mg, yield: 52.5%). $^1$HNMR (400 MHz, MeOD) δ(ppm): 8.64 (s, 1H), 7.35 (s, 1H), 6.99 (s, 1H), 4.49-4.42 (m, 1H), 4.06-4.04 (m, 1H), 3.90-3.88 (m, 1H), 1.90-1.87 (m, 1H), 1.77-1.68 (m, 4H), 1.51-1.48 (m, 2H), 1.35-1.33 (m, 7H).

Molecular formula: $C_{18}H_{23}N_5O$ Exact mass: 325.19 LC-MS (Pos, *m*/*z*) = 326.21[M+H]$^+$.

**Example 161: Synthesis of 7-(((1*R*,2*S*)-2-hydroxycyclohexyl)amino)-1-(isopropylamino)-2,6-naphthyridine-3-carbonitrile (Compound 161)**

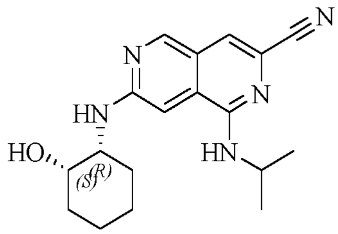

Step 1: synthesis of 7-(((1*R*,2*S*)-2-hydroxycyclohexyl)amino)-1-(isopropylamino)-2,6-naphthyridine-3-carbonitrile

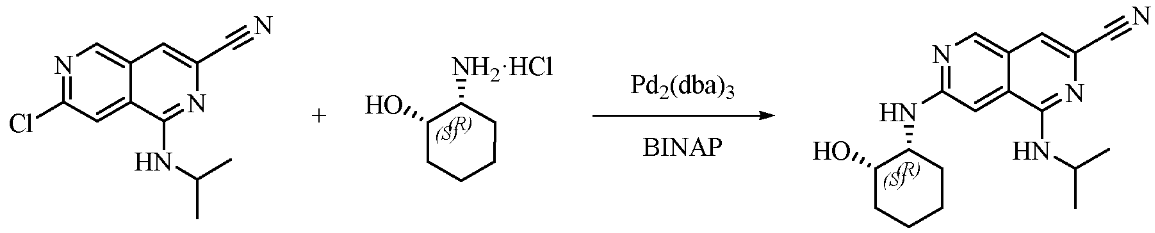


7-chloro-1-(isopropylamino)-2,6-naphthyridine-3-carbonitrile (100 mg, 0.41 mmol, 1.0 eq.), (1*S*,2*R*)-2-aminocyclohexyl-1-ol hydrochloride (80.4 mg, 0.53 mmol, 1.3 eq.), $Pd_2(dba)_3$ (54.9 mg, 0.06 mmol, 0.15 eq.), BINAP (74.7 mg, 0.12 mmol, 0.3 eq.) and cesium carbonate (469.2 mg, 1.44 mmol, 3.5 eq.) were added into 1,4-dioxane (10 mL). The reaction solution was heated to 120 °C and reacted for 18 h in a sealed tube in nitrogen atmosphere. When there were no materials left, as detected by TLC, the reaction solution was cooled to room temperature, water (30 mL) was added, and then ethyl acetate (30 mL × 3) was added for extraction. The organic phases were combined, dried over anhydrous sodium sulfate and filtered, and the filtrate was concentrated under reduced pressure to give a crude product, which was purified by preparative thin-layer chromatography (MeOH:DCM = 1:20) to give the product (62 mg, yield: 46.5%). $^1$HNMR (400 MHz, MeOD) δ(ppm): 8.64 (s, 1H), 7.35 (s, 1H), 6.99 (s, 1H), 4.49-4.44 (m, 1H), 4.05 (m, 1H), 3.89 (m, 1H), 1.90 (m, 1H), 1.77-1.70 (m, 4H), 1.50 (m, 3H), 1.35-1.33 (m, 6H).

Molecular formula: $C_{18}H_{23}N_5O$ Exact mass: 325.19 LC-MS (Pos, *m*/*z*) = 326.21[M+H]$^+$.

**Example 162: Synthesis of 7-(((1*R*,2*S*)-2-aminocyclopentyl)amino)-1-(isopropylamino)-2,6-naphthyridine-3-carbonitrile (Compound 162)**

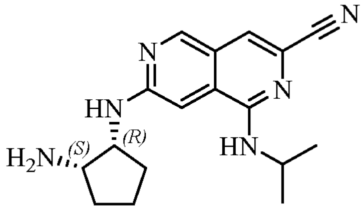

Step 1: synthesis of *tert*-butyl ((1*S*,2*R*)-2-((7-cyano-5-(isopropylamino)-2,6-naphthyridin-3-yl)amino)cyclopentyl)carbamate

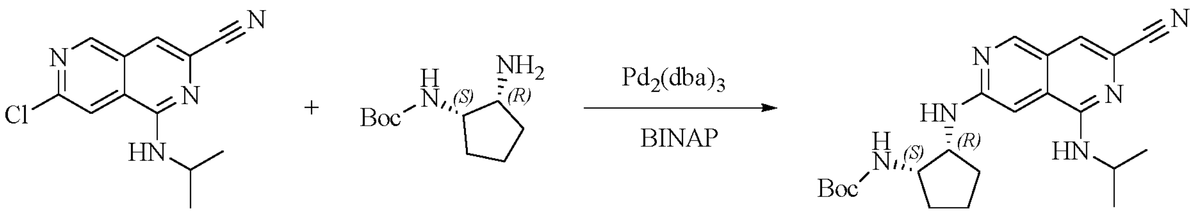


7-chloro-1-(isopropylamino)-2,6-naphthyridine-3-carbonitrile (100 mg, 0.41 mmol, 1.0 eq.), *tert*-butyl ((1*S*,2*R*)-2-aminocyclopentyl)carbamate (98.1 mg, 0.49 mmol, 1.2 eq.), $Pd_2(dba)_3$ (54.9 mg, 0.06 mmol, 0.15 eq.), BINAP (74.7 mg, 0.12 mmol, 0.3 eq.) and cesium carbonate (332.3 mg, 1.02 mmol, 2.5 eq.) were added into 1,4-dioxane (10 mL). The reaction solution was heated to 100 °C and reacted for 18 h in a sealed tube in nitrogen atmosphere. When there were no materials left, as detected by TLC, the reaction solution was cooled to room temperature, water (30 mL) was added, and then ethyl acetate (30 mL × 3) was added for extraction. The organic phases were combined, dried over anhydrous sodium sulfate and filtered, and the filtrate was concentrated under reduced pressure to give a crude product, which was purified by silica gel column chromatography (MeOH:DCM = 1:150-1:100) to give the product (106 mg, yield: 59.4%).

Step 2: synthesis of 7-(((1*R*,2*S*)-2-aminocyclopentyl)amino)-1-(isopropylamino)-2,6-naphthyridine-3-carbonitrile

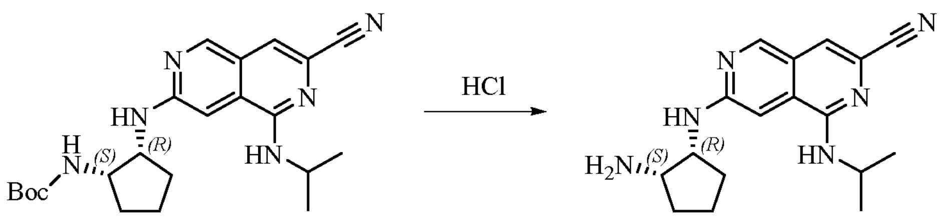


*Tert*-butyl ((1*S*,2*R*)-2-((7-cyano-5-(isopropylamino)-2,6-naphthyridin-3-yl)amino) cyclopentyl)carbamate (106 mg, 0.26 mmol, 1.0 eq.) was dissolved in DCM (3 mL), and hydrogen chloride ethanol solution (10 mol/L, 3 mL) was added dropwise. The reaction solution was reacted at room temperature for 1 h. When there were no materials left, as detected by TLC, the reaction solution was concentrated under reduced pressure, water (30 mL) was added, and DCM (30 mL × 2) was added for back-extraction. The aqueous phase was adjusted to pH of about 8 with aqueous potassium carbonate solution and extracted with DCM (30 mL × 3). The organic phases were combined, dried over anhydrous sodium sulfate and filtered, and the filtrate was concentrated under reduced pressure to give a crude product, which was purified by preparative thin-layer chromatography (MeOH:DCM = 1:10) to give the product (35 mg, yield: 43.4%). $^1$HNMR (400 MHz, MeOD) δ(ppm): 8.67 (s, 1H), 7.36 (s, 1H), 6.98 (s, 1H), 4.47-4.44 (m, 1H), 4.14-4.12 (m, 1H), 3.56-3.52 (m, 1H), 2.14-2.03 (m, 2H), 1.91-1.88 (m, 1H), 1.77-1.59 (m, 3H), 1.35-1.33 (m, 6H).

Molecular formula: $C_{17}H_{22}N_6$ Exact mass: 310.19 LC-MS (Pos, *m/z*) = 311.2[M+H]$^+$.

**Example 163: Synthesis of ((2-((7-(difluoromethyl)-5-(isopropylamino)-2,6-naphthyridin-3-yl)amino)pyridin-4-yl)methyl)(methyl)sulfinylimine (Compound 163)**

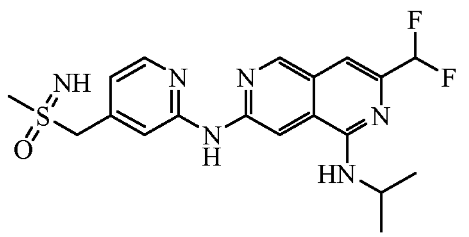

Step 1: synthesis of 3-(difluoromethyl)-*N1*-isopropyl-*N7*-(4-((methylthio)methyl) pyridin-2-yl)-2,6-naphthyridine-1,7-diamine

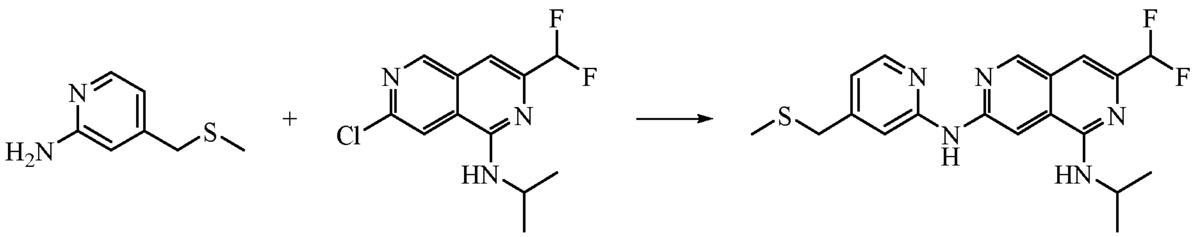

7-chloro-3-(difluoromethyl)-*N*-isopropyl-2,6-naphthyridin-1-amine (800 mg, 2.94 mmol, 1.0 eq.), 4-((methylthio)methyl)pyridin-2-amine (453 mg, 2.94 mmol, 1.0 eq.), $Pd(PPh_3)_4$ (335 mg, 0.29 mmol, 0.1 eq.), Xantphos (168 mg, 0.29 mmol, 0.1 eq.) and cesium carbonate (1.92 g, 5.88 mmol, 2.0 eq.) were dispersed in 1,4-dioxane (20 mL), and the reaction solution was reacted at 100 °C for 16 h in nitrogen atmosphere. After the reaction was completed, as detected by TLC, the reaction solution was concentrated under reduced pressure to give a crude product, which was purified by silica gel column chromatography (MeOH:DCM = 1:50) to give the product (800 mg, yield: 70.1%).

Step 2: synthesis of N-(((2-((7-(difluoromethyl)-5-(isopropylamino)-2,6-naphthyridin-3-yl)amino)pyridin-4-yl)methyl)(methyl)-$\lambda^4$-sulfaneylidene)-2,2,2-trifluoroacetamide

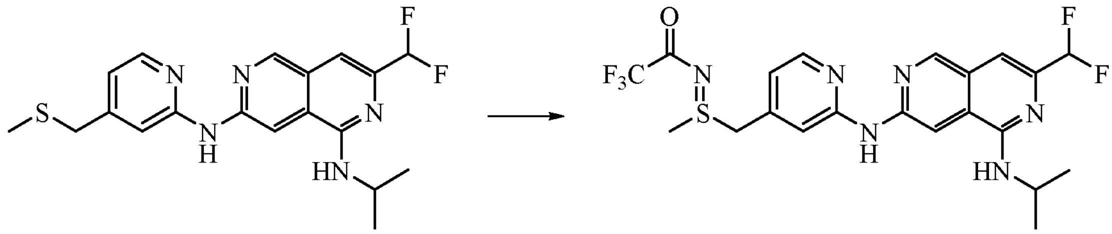

Sodium *tert*-butoxide (197 mg, 2.05 mmol, 1.0 eq.) was dispersed in THF (2 mL), and the reaction solution was cooled to 5 °C, followed by addition of a solution of trifluoroacetamide (348 mg, 3.08 mmol, 1.5 eq.) in THF (2 mL). The reaction solution was reacted for 5 min, followed by addition of a solution of dibromohydantoin (352 mg, 1.23 mmol, 0.6 eq.) in THF (2 mL). The reaction solution was then reacted for 5 min, followed by addition of a solution of 3-(difluoromethyl)-*N1*-isopropyl-*N7*-(4-((methylthio)methyl)pyridin-2-yl)-2,6-naphthyridine-1,7-diamine (800 mg, 2.05 mmol, 1.0eq.) in THF (20 mL). The resulting reaction solution was reacted for 20 min. After the reaction was completed, as detected by TLC, the reaction solution was poured into aqueous solution (20 mL), and the aqueous phase was extracted with EA (20 mL × 2). The organic phase was dried and concentrated to give a crude product, which was purified by silica gel column chromatography (MeOH:DCM = 1:80) to give the product (650 mg, yield: 63.1%).

Step 3: synthesis of ((2-((7-(difluoromethyl)-5-(isopropylamino)-2,6-naphthyridin-3-yl)amino)pyridin-4-yl)methyl)(methyl)sulfinylimine

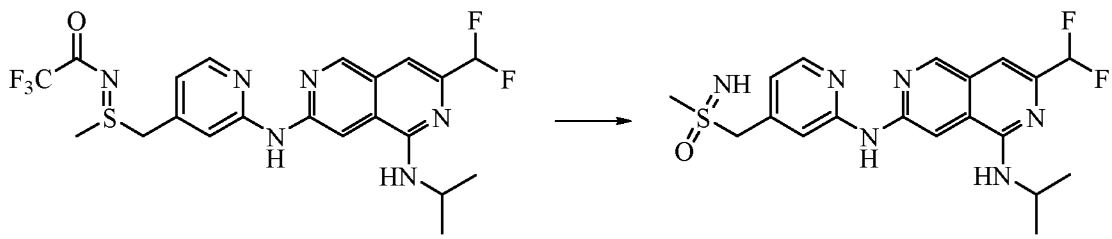

*N*-(((2-((7-(difluoromethyl)-5-(isopropylamino)-2,6-naphthyridin-3-yl)amino)pyridin-4-yl)methyl)(methyl)-$\lambda^4$-sulfaneylidene)-2,2,2-trifluoroacetamide (500 mg, 1.00 mmol, 1.0 eq.) was dispersed in methanol (10 mL) and water (5 mL), Oxone (557 mg, 0.90 mmol, 0.9 eq.) was added, and aqueous NaOH solution (1 mol/L, 1 mL) was added dropwise. The reaction solution was reacted for 30 min. After the reaction was completed, as detected by TLC, the reaction solution was concentrated under reduced pressure, and the aqueous phase was extracted with EA (10 mL × 3). The organic phase was dried and concentrated to give a crude product, which was purified by silica gel column chromatography (MeOH:DCM = 1:50) to give the product (150 mg, yield: 47.6%).

$^1$HNMR (400 MHz, DMSO-$d_6$) δ(ppm): 9.88 (s, 1H), 8.98 (s, 1H), 8.43 (s, 2H), 8.30-8.29 (d, 1H), 7.37 (s, 1H), 7.23-7.20 (t, 2H), 6.98-6.97 (t, 1H), 6.91-6.64 (t, 1H), 4.40 (s, 3H), 3.80 (s, 1H), 2.90 (s, 3H), 1.29-1.27 (s, 6H).

Molecular formula: $C_{19}H_{22}F_2N_6OS$ Exact mass: 420.15 LC-MS (Pos, *m*/*z*) = 421.03[M+H]$^+$.

**Example 164: Synthesis of 7-(((1s,3s)-3-aminocyclobutyl)amino)-1-(isopropylamino)-2,6-naphthyridine-3-carbonitrile (Compound 164)**

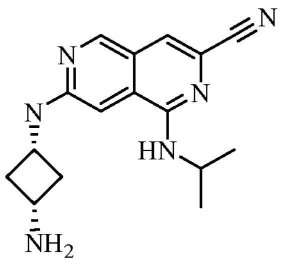

The titled compound was prepared as same as example 38 using corresponding chemicals.

$^1$HNMR (400 MHz, MeOD) δ(ppm): 8.65 (s, 1H), 7.36 (s, 1H), 6.87 (s, 1H), 4.47-4.43 (m, 1H), 3.96-3.92 (m, 1H), 3.26-3.22 (m, 1H), 2.93-2.87 (m, 2H), 1.80-1.73 (m, 2H), 1.35-1.33 (m, 6H).

Molecular formula: $C_{16}H_{20}N_6$ Exact mass: 296.17 LC-MS (Pos, *m*/*z*) =297.14 [M+H]$^+$.

**Example 165: Synthesis of 7-(((1R,2R)-2-aminocyc|opentyl)amino)-1-(isopropylamino)-2,6-naphthyridine-3-carbonitrile (Compound 165)**

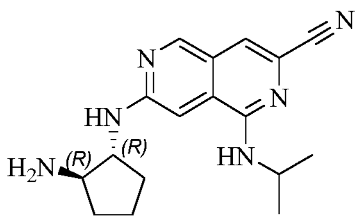

The titled compound was prepared as same as example 38 using corresponding chemicals.

$^1$HNMR (400 MHz, MeOD) δ(ppm): 8.67 (s, 1H), 7.36 (s, 1H), 6.93 (s, 1H), 4.48-4.45 (m, 1H), 3.88-3.86 (m, 1H), 3.22-3.17 (m, 1H), 2.35-2.30 (m, 1H), 2.11-2.08 (m, 1H), 1.1.89-1.82 (m, 2H), 1.61-1.53 (m, 2H), 1.35-1.33 (m, 6H).

Molecular formula: $C_{17}H_{22}N_6$ Exact mass: 310.19 LC-MS (Pos, *m*/*z*) =311.2 [M+H]$^+$.

**Example 166: Synthesis of 7-(((1r,3r)-3-hydroxycyclobutyl)amino)-1-(isopropylamino)-2,6-naphthyridine-3-carbonitrile (Compound 166)**

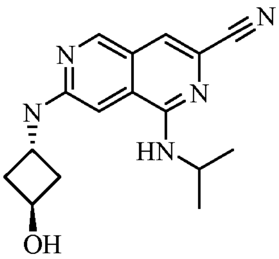

The titled compound was prepared as same as example 44 using corresponding chemicals.

$^1$HNMR (400 MHz, MeOD) δ(ppm): 8.66 (s, 1H), 7.36 (s, 1H), 6.79 (s, 1H), 4.53-4.43 (m, 2H), 4.34-4.30 (m, 1H), 2.48-2.42 (m, 2H), 2.36-2.30 (m, 2H), 1.35-1.33 (m, 6H). Molecular formula: $C_{16}H_{19}N_5O$ Exact mass: 297.16 LC-MS (Pos, *m*/*z*) =298.13 [M+H]$^+$.

**Example 167: Synthesis of N-((1s,3s)-3-((7-cyano-5-(isopropylamino)-2,6-naphthyridin-3-yl)amino)cyclobutyl)acetamide (Compound 167)**

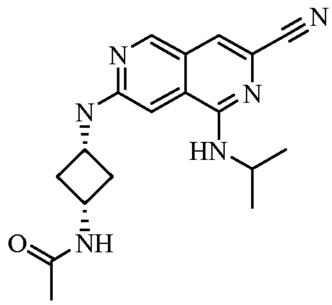

The titled compound was prepared as same as example 47 using corresponding chemicals.

$^1$HNMR (400 MHz, MeOD) δ(ppm): 8.66 (s, 1H), 7.36 (s, 1H), 6.89 (s, 1H), 4.49-4.43 (m, 1H), 4.13-3.99 (m, 2H), 2.97-2.91 (m, 2H), 1.95 (s, 3H), 1.93-1.90 (m, 2H), 1.35-1.33 (m, 6H).

Molecular formula: $C_{18}H_{22}N_6$ O Exact mass: 338.19 LC-MS (Pos, *m*/*z*) =339.17 [M+H]$^+$.

**Example 168: Synthesis of (S)-1-(cyclopentylamino)-7-(piperidin-3-ylamino)-2,6-naphthyridine-3-carbonitrile (Compound 168)**

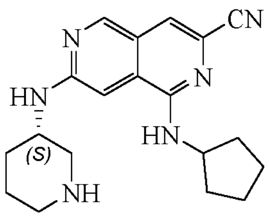

The titled compound was prepared as same as example 148 using corresponding chemicals.

$^1$HNMR (400 MHz, MeOD) δ(ppm): 8.64 (s, 1H), 7.34 (s, 1H), 6.91 (s, 1H), 4.51-4.47 (t, 1H), 3.84-3.81 (m, 1H), 3.26-3.22 (m, 1H), 2.99-2.96 (t, 1H), 2.66-2.60 (m, 1H), 2.53-2.48 (m, 1H), 2.19-2.09 (m, 3H), 1.85-1.80 (m, 3H), 1.69-1.52 (m, 3H).

Molecular formula: $C_{19}H_{24}N_6$ Exact mass: 336.21 LC-MS(Pos, *m*/*z*)=337.11[M+H]$^+$.

**Example 169: Synthesis of (S)-7-(piperidin-3-ylamino)-1-(pyrrolidin-1-yl)-2,6-naphthyridine-3-carbonitrile (Compound 169)**

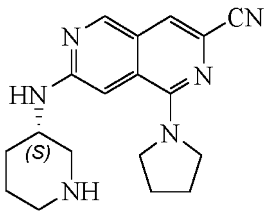

The titled compound was prepared as same as example 148 using corresponding chemicals.

$^1$HNMR (400 MHz, MeOD) δ(ppm): 8.65 (s, 1H), 7.36 (s, 1H), 7.11 (s, 1H), 3.90-3.87 (t, 1H), 3.85-3.80 (m, 4H), 3.28-3.24 (m, 1H), 2.99-2.94 (m, 1H), 2.64-2.61 (m, 1H), 2.50-2.45 (m, 1H), 2.13-2.10 (m, 1H), 2.05-2.01 (m, 2H), 1.84-1.78 (m, 1H), 1.69-1.60 (m, 1H), 1.56-1.47 (m, 1H).

Molecular formula: $C_{18}H_{22}N_6$ Exact mass: 322.19 LC-MS (Pos, *m/z*)=323.11[M+H]$^+$.

**Example 170: Synthesis of 1-(isopropylamino)-7-((4-((S-methylsulfonimidoyl)methyl)pyridin-2-yl)amino)-2,6-naphthyridine-3-carbonitrile (Compound 170)**

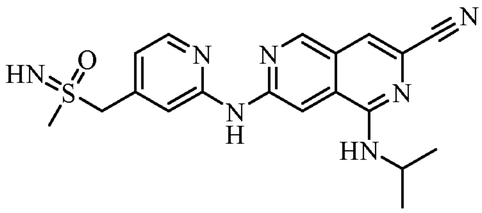

The titled compound was prepared as same as example 163 using corresponding chemicals.

$^1$HNMR (400 MHz, DMSO-$d_6$) δ(ppm): 10.09 (s, 1H), 8.95 (s, 1H), 8.47 (s, 1H), 8.32-8.31 (d, 1H), 7.63 (s, 1H), 7.47-7.45 (d, 1H), 7.37 (s, 1H), 7.02-7.01 (d, 1H), 4.41-4.34 (m, 3H), 3.8 (s, 1H), 2.90 (s, 3H), 1.28-1.27 (m, 6H).

Molecular formula: $C_{19}H_{21}N_7OS$ Exact mass: 395.15 LC-MS (Pos, *m/z*) =396.17[M+H]$^+$.

**Example 171: Synthesis of (S)-1-(diethylamino)-7-(piperidin-3-ylamino)-2,6-naphthyridine-3-carbonitrile (Compound 171)**

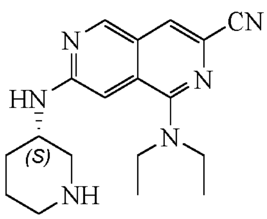

The titled compound was prepared as same as example 148 using corresponding chemicals.

$^1$HNMR (400 MHz, MeOD) δ(ppm): 8.79 (s, 1H), 7.63 (s, 1H), 6.83 (s, 1H), 3.96-3.90 (m, 1H), 3.53-3.48 (m, 4H), 3.29-3.28 (d,1H), 3.03-2.98 (m, 1H), 2.70-2.64 (m, 1H), 2.60-2.55 (m, 1H), 2.15-2.11 (m, 1H), 1.88-1.83 (m, 1H), 1.72-1.65 (m, 2H), 1.28-1.25 (t, 6H).

Molecular formula: $C_{18}H_{24}N_6$ Exact mass: 324.21 LC-MS(Pos, *m/z*)=325.12[M+H]$^+$.

**Example 172: Synthesis of 7-(((1s,3s)-3-aminocyclobutyl)amino)-1-(tert-butylamino)-2,6-naphthyridine-3-carbonitrile (Compound 172)**

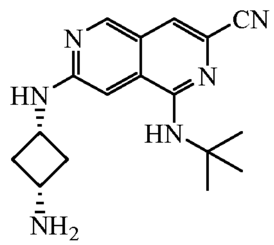

The titled compound was prepared as same as example 179 using corresponding chemicals.

$^1$HNMR (400 MHz, MeOD) 6(ppm): 8.65 (s, 1H), 7.37-7.36 (d, 1H), 6.81 (s, 1H), 4.08-4.03 (m, 1H), 3.30-3.29 (d, 1H), 2.92-2.86 (m , 2H), 1.87-1.80 (m, 2H), 1.58 (s, 9H). Molecular formula: $C_{17}H_{22}N_6$ Exact mass: 310.19 LC-MS(Pos, *m*/*z*)=311.07[M+H]$^+$.

**Example 173: Synthesis of 7-(((1S,3S)-3-aminocyclopentyl)amino)-1-(((R)-tetrahydrofuran-3-yl)amino)-2,6-naphthyridine-3-carbonitrile (Compound 173)**

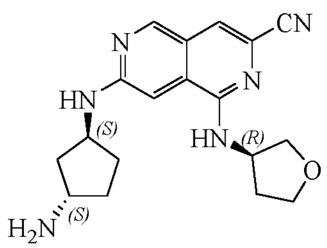

The titled compound was prepared as same as example 179 using corresponding chemicals.

$^1$HNMR (400 MHz, MeOD) δ(ppm): 8.68 (s, 1H), 6.41 (s, 1H), 6.96 (s, 1H), 4.75-4.72 (m, 1H), 4.43-4.40 (t, 1H), 4.13-4.08 (m, 2H), 3.91-3.86 (m, 1H), 3.83-3.79 (m, 1H), 3.69-3.66 (t, 2H), 2.43-2.34 (m, 2H), 2.27-2.23 (m, 1H), 2.16-2.13 (m, 1H), 2.05-2.01 (m, 2H), 1.70-1.58 (m, 1H).

Molecular formula: $C_{18}H_{22}N_6O$ Exact mass: 338.19 LC-MS(Pos, *m*/*z*)=339.07[M+H]$^+$.

**Example 174: Synthesis of 7-(((1s,3S)-3-aminocyclobutyl)amino)-1-(((R)-tetrahydrofuran-3-yl)amino)-2,6-naphthyridine-3-carbonitrile (Compound 174)**

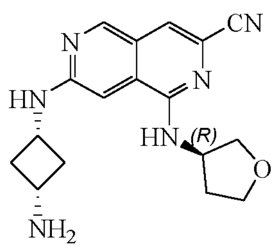

The titled compound was prepared as same as example 179 using corresponding chemicals.

$^1$HNMR (400 MHz, MeOD) δ(ppm): 8.71 (s, 1H), 7.45 (s, 1H), 6.94 (s, 1H), 4.77-4.74 (m, 1H), 4.65 (s, 1H), 4.13-4.08 (m, 2H), 3.92-3.86 (m, 1H), 3.83-3.80 (t, 1H), 3.50-3.45 (m, 1H), 2.99-2.92 (m, 2H), 2.44-2.39 (m, 1H), 2.15-2.11 (m, 1H), 2.06-1.98 (m, 2H).

Molecular formula:$C_{17}H_{20}N_6O$ Exact mass: 324.17 LC-MS (Pos, *m*/*z*)=325.08[M+H]$^+$.

**Example 175: synthesis of (*S*)-1-(cyclopropylamino)-7-(piperidin-3-ylamino)-2,6-naphthyridine-3-carbonitrile(Compound 175)**

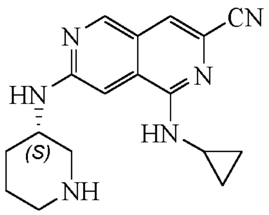

Step 1: synthesis of 7-chloro-1-(cyclopropylamino)-2,6-naphthyridine-3-carbonitrile

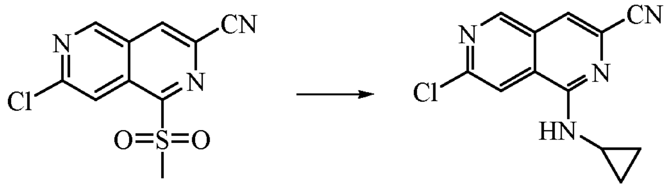

7-Chloro-1-(methylsulfonyl)-2,6-naphthyridine-3-carbonitrile (500 mg, 1.87 mmol, 1.0 eq.) and cyclopropylamine (320 mg, 5.61 mmol, 3.0 eq.) were dissolved in THF (10 mL), and the reaction solution was reacted at room temperature for 30 min. After the reaction was completed as monitored by TLC, the reaction solution was poured into water (10 mL) and extracted with EA (10 mL × 2). The organic phase was dried and concentrated to give the product (458 mg, yield: 100%).

Step 2: synthesis of *tert*-butyl (*S*)-3-((7-cyano-5-(cyclopropylamino)-2,6-naphthyridin-3-yl)amino)piperidine-1-carboxylate

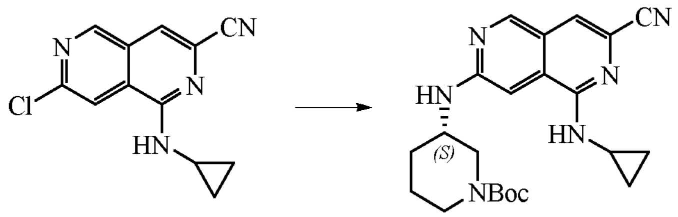

7-Chloro-1-(cyclopropylamino)-2,6-naphthyridine-3-carbonitrile (458 mg, 1.87 mmol, 1.0 eq.), *tert*-butyl (*S*)-3-aminopiperidine-1-carboxylate (449 mg, 2.24 mmol, 1.2 eq.), $Pd_2(dba)_3$ (174 mg, 0.19 mmol, 0.1 eq.), BINAP (118 mg, 0.19 mmol, 0.1 eq.) and cesium carbonate (1.22 g, 3.74 mmol, 2.0 eq.) were dispersed in 1,4-dioxane (10 mL), and the reaction solution was reacted at 100 °C for 16 h in nitrogen atmosphere. After the reaction was completed as monitored by TLC, the reaction solution was concentrated under reduced pressure to give a crude product, which was purified by silica gel column chromatography (MeOH:DCM = 1:100) to give the product (400 mg, yield: 52.4%).

Step 3: synthesis of (*S*)-1-(cyclopropylamino)-7-(piperidin-3-ylamino)-2,6-naphthyridine-3-carbonitrile

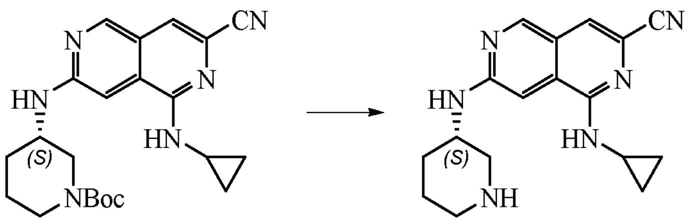

*tert*-Butyl (*S*)-3-((7-cyano-5-(cyclopropylamino)-2,6-naphthyridin-3-yl)amino)piperidine-1-carboxylate (400 mg, 0.98 mmol, 1.0 eq.) was dissolved in EA (10 mL), and a solution of hydrogen chloride in 1,4-dioxane (4 mol/L, 5 mL) was added dropwise. The reaction solution was reacted at room temperature for 60 min. After the reaction was completed as monitored by TLC, the reaction solution was poured into water (10 mL), and the liquid separation was performed. The aqueous phase was adjusted to pH of 10 with saturated aqueous potassium carbonate solution, and extracted with DCM (10 mL × 4). The organic phase was dried and concentrated, and the crude product was purified by silica gel preparative thin-layer chromatography (MeOH:DCM = 1:10) to give the product (240 mg, yield: 79.5%).

$^1$HNMR (400 MHz, MeOD) δ(ppm): 8.71 (s, 1H), 7.46 (s, 1H), 6.85 (s, 1H), 3.92-3.89 (m, 1H), 3.08-3.04 (m, 1H), 2.95-2.89 (m, 1H), 2.76-2.69 (m, 1H), 2.64-2.58 (m, 1H), 2.15-2.11 (m, 1H), 1.92-1.87 (m, 1H), 1.73-1.70 (m, 1H), 1.63-1.60 (t, 1H), 1.32-1.31 (d, 1H), 0.92-0.87 (m, 2H), 0.67-0.63 (m, 2H).

Molecular formula: $C_{17}H_{20}N_6$ Exact mass: 308.17 LC-MS (Pos, *m*/*z*) = 309.11[M+H]$^+$.

**Example 176: Synthesis of 7-(((1s,3S)-3-hydroxycyclobutyl)amino)-1-(((R)-tetrahydrofuran-3-yl)amino)-2,6-naphthyridine-3-carbonitrile(Compound 176)**

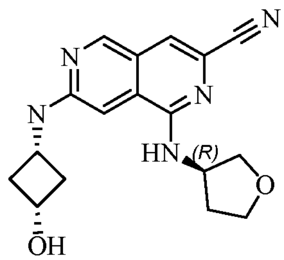

The titled compound was prepared as same as example 179 using corresponding chemicals.

$^1$HNMR (400 MHz, MeOD) δ(ppm): 8.68 (s, 1H), 7.43 (s, 1H), 6.89 (s, 1H), 4.76 (m, 1H), 4.13-4.02 (m, 3H), 3.92-3.79 (m, 3H), 2.93-2.90 (m, 2H), 2.44-2.39 (m, 1H), 2.15-2.07 (m, 1H), 1.91-1.88 (m, 2H).

Molecular formula: $C_{17}H_{19}N_5O_2$ Exact mass: 325.15 LC-MS (Pos, *m*/*z*) =326.17 [M+H]$^+$.

**Example 177: Synthesis of (*S*)-*N*-(5-(*tert*-butylamino)-7-cyano-2,6-naphthyridin-3-yl)piperidine-3-carboxamide (Compound 177)**

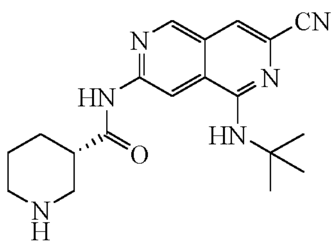

The titled compound was prepared as same as example 178 using corresponding chemicals.

$^1$H-NMR ($CDCl_3$) δ(ppm): 11.65 (brs, 1H), 8.82 (s, 1H), 8.56 (s, 1H), 7.35 (s, 1H), 5.43 (s, 1H), 3.48 (s, 1H), 3.32-3.36 (m, 1H), 3.14-3.18 (m, 1H), 2.95-3.00 (m, 1H), 2.76 (t, 1H, J = 10.4 Hz), 2.60-2.63 (m, 1H), 2.07-2.11 (m, 1H), 1.75-1.91 (m, 3H), 1.55 (s, 9H).

Molecular formula: $C_{19}H_{24}N_6O$ Exact mass: 352.20 LC-MS (Pos, *m/z*) =353.1 $[M+H]^+$.

**Example 178: Synthesis of (*R*)-*N*-(5-(*tert*-butylamino)-7-cyano-2,6-naphthyridin-3-yl)piperidine-3-carboxamide(Compound 178)**

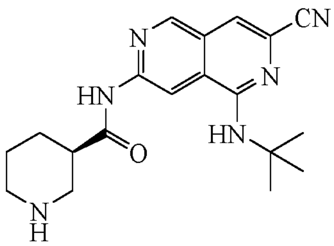

Steps:

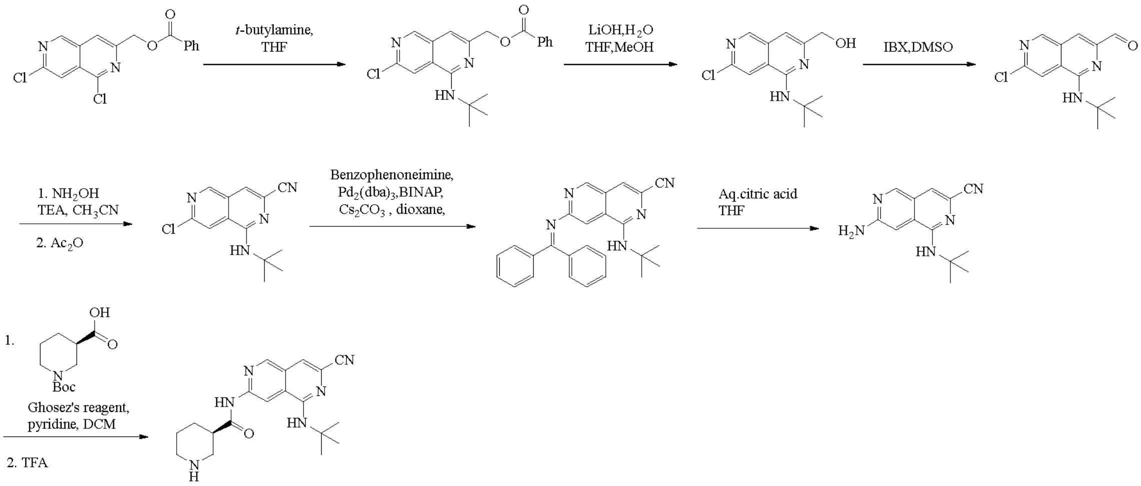


Step 1: synthesis of (1-(tert-butylamino)-7-chloro-2,6-naphthyridin-3-yl)methyl benzoate

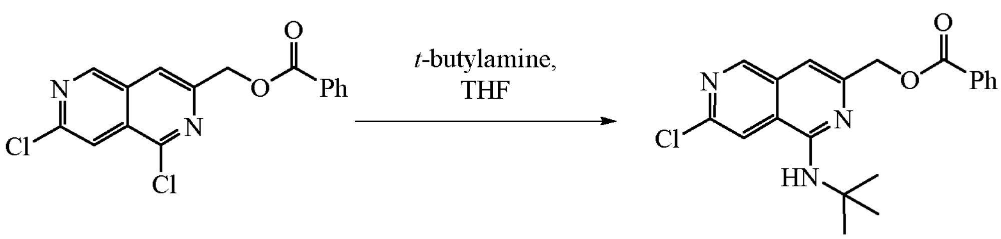


(1,7-dichloro-2,6-naphthyridin-3-yl)methyl benzoate (3.0 g, 9.04 mmol) and tBuNH2 (18.9 ml, 180 mmol, 20 eq.) were dissolved in dry THF (20 mL) and the reaction mixture was heated to 100°C for overnight. LCMS showed SM was major compound along with desired product. The reaction was continued for another 60 h. The reaction mixture was cooled to rt, diluted with water and extracted with EA (100 mLx3). Combined EA layers were further washed with brine, dried over anhydrous sodium sulfate, filtered and concentrated under reduced pressure to get the crude. The crude was purified by silica gel column chromatography to obtain the desired product (1.2 g, yield: 36%) and the SM was recovered. Exact mass: 369.12, LC-MS (Pos, m/z) = 370.0 [M+H]+.

Step 2: synthesis of (1-(tert-butylamino)-7-chloro-2,6-naphthyridin-3-yl)methanol

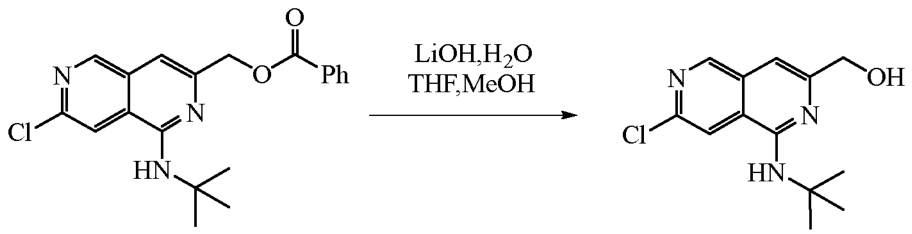


To a stirred solution of (1-(tert-butylamino)-7-chloro-2,6-naphthyridin-3-yl)methyl benzoate (2.8 g, 7.58 mmol) in a mixture of solvents THF (15mL), MeOH (7.0 mL) and water (7.0 mL) was added LiOH.H2O (0.933 g, 22.7 mmol, 3.0 eq.) and stirred at rt for overnight. After TLC showed SM was consumed and polar spot was formed, the solvent was removed under reduced pressure to get the crude product. The crude was dissolved in EA and washed with water and brine, dried over anhydrous sodium sulfate and concentrated under reduced pressure to get the crude. The crude was purified by silica gel column chromatography using 10-15% EA in hexane as eluent to get the pure compound (1.95 g, yield: 97%). Exact mass: 265.10, LC-MS (Pos, m/z) = 266.0 [M+H]+.

Step 3: synthesis of 1-(tert-butylamino)-7-chloro-2,6-naphthyridine-3-carbaldehyde

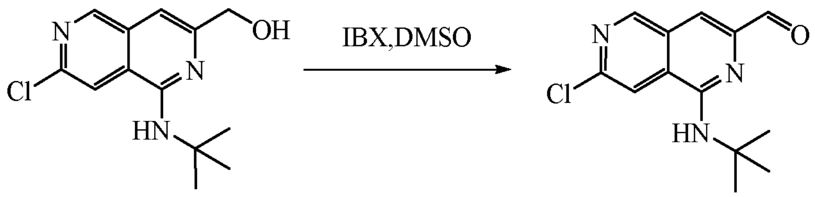


To a stirred solution of (1-(tert-butylamino)-7-chloro-2,6-naphthyridin-3-yl)methanol (1.6 g, 6.038 mmol) in DMSO (40 mL) was added IBX (3.38 g, 12.08 mmol, 2.0 eq.) at 0°C and stirred at rt for 30 min. After TLC showed SM was consumed, the reaction mixture was diluted with cold water (100 mL) and extracted with EA(150 mLx2). The combined organic layers were washed with brine, dried over anhydrous sodium sulfate and concentrated under reduced pressure to get the crude which was used in the next step without further purification. Exact mass: 263.08, LC-MS (Pos, m/z) = 264.0 [M+H]+.

Step 4: synthesis of 1-(*tert*-butylamino)-7-chloro-2,6-naphthyridine-3-carbonitrile

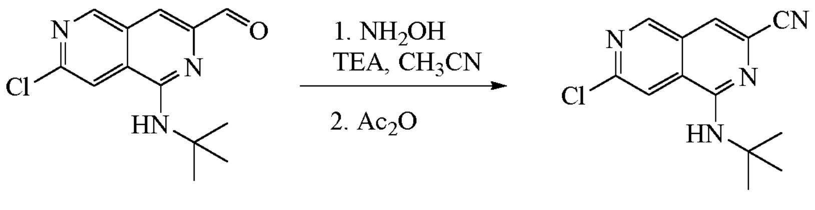


To a stirred solution of 1-(tert-butylamino)-7-chloro-2,6-naphthyridine-3-carbaldehyde (4.1 g, crude, 15.59 mmol) in acetonitrile (60.0 mL) was added TEA (6.31 mL, 62.36 mmol, 4.0 eq.) followed by NH2OH.HCl (2.38 g, 34.30 mmol, 2.2 eq.) and the reaction mixture was heated to reflux for 2h. After TLC showed SM was consumed, the reaction mixture was cooled to rt and concentrated under reduced pressure. The crude was diluted with water and extracted with EA (150 mLx2). The combined organic layers were dried over anhydrous sodium sulfate, filtered and concentrated under reduced pressure to get the crude oxime intermediate (2.5 g), which was further treated with Ac2O (29.72 mL, 311.78

mmol, 20 eq.) and reaction mixture was heated at 120°C for 18 h. TLC showed desired nonpolar spot formed along with little intermediate. Further added Ac2O (10 mL) and continued for another overnight. The reaction mixture was cooled to rt, diluted with 20% EA in hexane (150 mL) and stayed for overnight at rt. The desired product (1.3 g) was isolated by filtration. The mother liquid was concentrated under reduced pressure to get the crude. The crude mixture was purified by silica gel column chromatography using 10-20% EA in hexane as eluent to give another desired product (400 mg). The combined compound (1.7 g, yield: 45%) was used in the next step.

Step 5: synthesis of 1-(tert-butylamino)-7-((diphenylmethylene)amino)-2,6-naphthyridine-3-carbonitrile

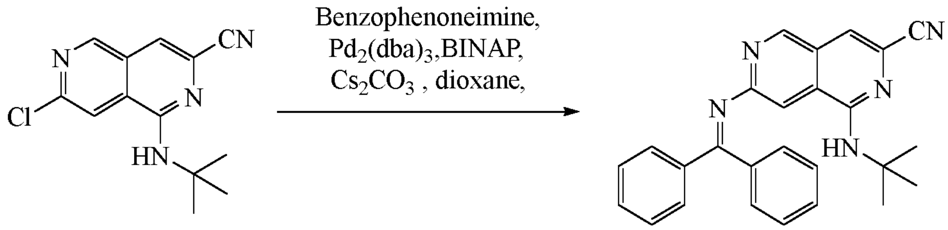


1-(*tert*-butylamino)-7-chloro-2,6-naphthyridine-3-carbonitrile (0.9 g, 3.462 mmol), benzophenone imine (0.87 mL, 5.19 mmol, 1.5 eq.), $Pd_2(dba)_3$ (317 mg, 0.346 mmol, 0.1 eq.), BINAP (431 mg, 0.692 mmol, 0.2 eq.) and $Cs_2CO_3$ (3.4 g, 10.39 mmol, 3.0 eq.) were dissolved in 1,4-dioxane (30 mL) and the reaction mixture was heated to 120°C for overnight. After LCMS showed SM was consumed, the reaction mixture was cooled to rt, filtered through celite pad, washed with EA. The organic layer was washed with brine, dried over anhydrous sodium sulfate, filtered and concentrated under reduced pressure to get the crude. The crude was purified by Combiflash chromatography to obtain the desired compound (1.15 g, yield: 82%). Exact mass: 405.20, LC-MS (Pos, *m*/*z)* = 406.0 $[M+H]^+$.

Step 6: synthesis of 7-amino-1-(*tert*-butylamino)-2,6-naphthyridine-3-carbonitrile

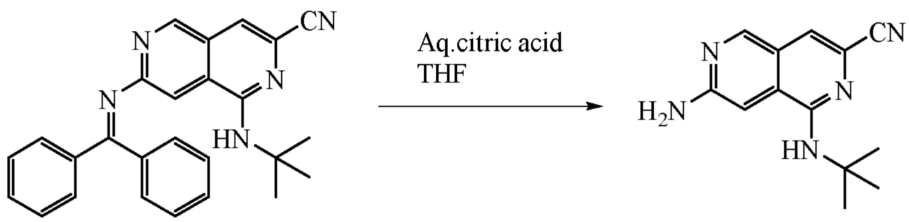


To a stirred solution of 1-(*tert*-butylamino)-7-((diphenylmethylene)amino)-2,6-naphthyridine-3-carbonitrile (1.15 g, 2.83 mmol) in THF (18.0 mL) was added aq. 10% citric acid solution (10 mL) and stirred at rt for 3 h. After TLC showed SM was consumed, THF was removed under reduced pressure. The reaction mixture was extracted with EA. The organic layer was washed with water and brine, dried over anhydrous sodium sulfate and concentrated under reduced pressure to get the crude compound. The crude was purified by Combiflash chromatography to get the pure compound as grey solid (575 mg, yield: 84%). Exact mass: 241.13, LC-MS (Pos, *m*/*z)* = 242.2 $[M+H]^+$.

Step 7: synthesis of (R)-N-(5-(*tert*-butylamino)-7-cyano-2,6-naphthyridin-3-yl)piperidine-3-carboxamide

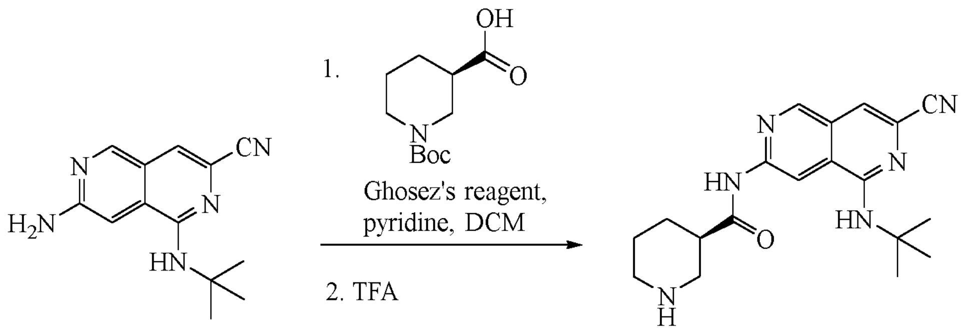


7-amino-1-(*tert*-butylamino)-2,6-naphthyridine-3-carbonitrile (24.1 mg, 0.10 mmol) was dissolved in DCM (0.5 mL). The reaction solution was cooled to 0 °C, added with 1-chloro-*N*,*N*,2-trimethylprop-1-en-1-amine (mass fraction: 95%, 20.0 mg, 0.15 mmol, 1.5 eq.) and reacted at 0 °C for 1 h. (*R*)-1-(*tert*-butoxycarbonyl)piperidine-3-carboxylic acid (27.5 mg, 0.12 mmol, 1.2 eq.) and pyridine (24.2 uL, 0.30 mmol, 3.0 eq.) were added to the reaction mixture and the solution was stirred at rt for overnight. When there were no materials left, as detected by LCMS, the reaction solution was added with TFA (1.0 mL) and reacted at rt for 1 h. When there were no materials left, the reaction solution was concentrated under reduced pressure. The crude was purified by HPLC to give the product (17.2 mg, yield: 49%).

$^1$H-NMR ($CDCl_3$) δ(ppm): 11.60 (brs, 1H), 8.81 (s, 1H), 8.55 (s, 1H), 7.34 (s, 1H), 5.46 (s, 1H), 3.32-3.36 (m, 1H), 3.14-3.17 (m, 1H), 2.96-3.00 (m, 1H), 2.77 (t, 1H, J = 11.2 Hz), 2.62-2.64 (m, 1H), 2.06-2.10 (m, 1H), 1.78-1.90 (m, 4H), 1.55 (s, 9H).

Molecular formula: $C_{19}H_{24}N_6O$ Exact mass: 352.20 LC-MS (Pos, *m/z)* = 353.2 $[M+H]^+$.

**Example 179: Synthesis of 7-(((*trans*)-3-aminocyclobutyl)amino)-1-(((*R*)-tetrahydrofuran-3-yl)amino)-2,6-naphthyridine-3-carbonitrile (Compound 179)**

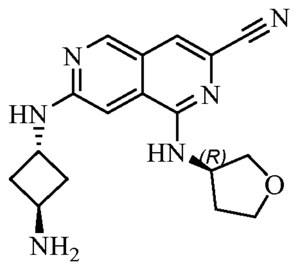

Step 1: synthesis of *tert*-butyl ((*trans*)-3-((7-cyano-5-(((*R*)-tetrahydrofuran-3-yl)amino)-2,6-naphthyridin-3-yl)amino)cyclobutyl)carbamate

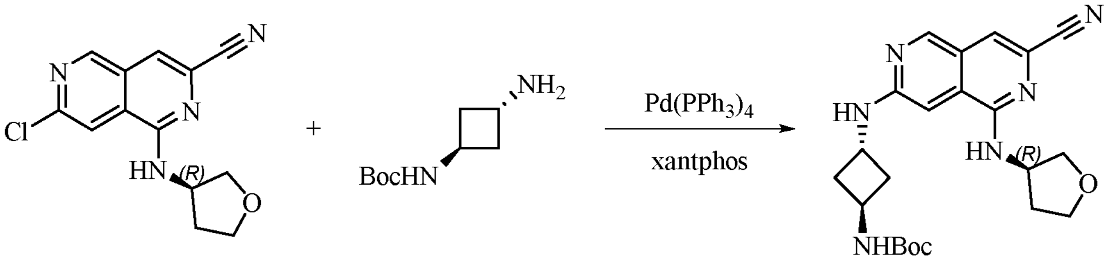


(*R*)-7-chloro-1-((tetrahydrofuran-3-yl)amino)-2,6-naphthyridine-3-carbonitrile (300 mg, 1.09 mmol, 1.0 eq.), *tert*-butyl (*trans*)-3-aminocyclobutyl)carbamate (244 mg, 1.31 mmol, 1.2 eq.), tetrakis(triphenylphosphine)palladium (127.2 mg, 0.11 mmol, 0.1 eq.), Xantphos (127.3 mg, 0.22 mmol, 0.2 eq.) and cesium carbonate (889.5 mg, 2.73 mmol, 2.5 eq.) were added to 1,4-dioxane (20 mL), and the reaction solution was heated to 100 °C and reacted for 12 h in a

sealed tube in nitrogen atmosphere. The reaction solution was then concentrated under reduced pressure to give a crude product, which was purified by silica gel column chromatography (MeOH:DCM = 1:100-1:50) to give the product (200 mg, yield: 68.7%).

Step 2: synthesis of 7-(((*trans*)-3-aminocyclobutyl)amino)-1-(((R)-tetrahydrofuran-3-yl)amino)-2,6-naphthyridine-3-carbonitrile

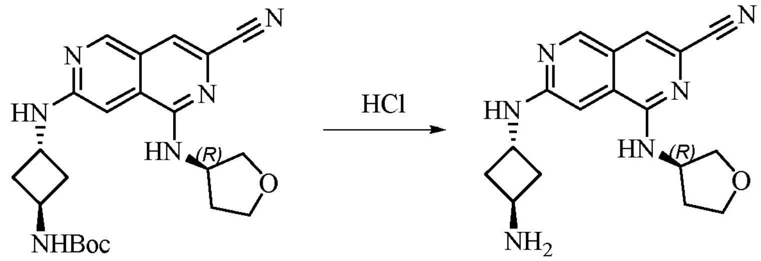


*Tert*-butyl ((*trans*)-3-((7-cyano-5-(((*R*)-tetrahydrofuran-3-yl)amino)-2,6-naphthyridin-3-yl)amino)cyclobutyl)carbamate (200 mg, 0.47 mmol, 1.0 eq.) was dissolved in DCM (3 mL), and hydrogen chloride ethanol solution (10 mol/L, 3 mL) was added dropwise. The reaction solution was reacted at room temperature for 1 h. When there were no materials left, as detected by TLC, the reaction solution was concentrated under reduced pressure, water (30 mL) was added, and DCM (30 mL × 2) was added for back-extraction. The aqueous phase was adjusted to pH of about 8 with aqueous potassium carbonate solution and extracted with DCM (30 mL × 5). The organic phases were combined, dried over anhydrous sodium sulfate and filtered, and the filtrate was concentrated under reduced pressure to give a crude product, which was purified by preparative thin-layer chromatography (MeOH:DCM = 1:10) to give the product (62 mg, yield: 40.7%).

$^1$HNMR (400 MHz, MeOD) δ(ppm): 8.69 (s, 1H), 7.43 (s, 1H), 6.80 (s, 1H), 4.77-4.74 (m, 1H), 4.31-4.28 (m, 1H), 4.13-4.03 (m, 2H), 3.92-3.86 (m, 1H), 3.82-3.79 (m, 1H), 3.71-3.68 (m, 1H), 2.55-2.30 (m, 5H), 2.13-2.11 (m, 1H).

Molecular formula: $C_{17}H_{20}N_6O$ Exact mass: 324.17 LC-MS (Pos, *m/z)* = 325.11[M+H]$^+$.

**Example 180: Synthesis of (S)-1-(cyclohex-1-en-1-yl)-7-(piperidin-3-ylamino)-2,6-naphthyridine-3-carbonitrile (Compound 180)**

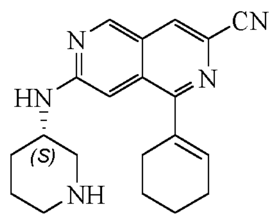

The titled compound was prepared as same as example 38 using corresponding chemicals.

$^1$HNMR (400 MHz, MeOD) 6(ppm): 8.97 (d, 1H), 8.08 (s, 1H), 7.03 (s, 1H), 6.06-6.08 (m, 1H), 4.03-4.09 (m, 1H), 3.35-3.36 (m, 1H), 3.03-3.08 (m, 1H), 2.68-2.75 (m, 1H), 2.59-2.64 (m, 1H), 2.46-2.49 (m, 2H), 2.34-2.36 (m, 2H), 2.10-2.14 (m, 1H), 1.87-1.93 (m, 3H), 1.79-1.85 (m, 2H), 1.65-1.75 (m, 1H) ,1.53-1.62 (m, 1H).

Molecular formula:$C_{20}H_{23}N_5$ Exact mass: 333.20 LC-MS (Pos, *m/z*)=334.07[M+H]$^+$.

**Example 181: Synthesis of (S)-1-(cyclohex-1-en-1-yl)-7-(piperidin-3-ylamino)-2,6-naphthyridine-3-carbonitrile (Compound 181)**

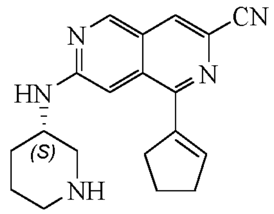

The titled compound was prepared as same as example 38 using corresponding chemicals.

$^1$HNMR (400 MHz, MeOD) δ(ppm): 8.97 (s, 1H), 8.05 (s, 1H), 7.20 (s, 1H), 6.47-6.48 (m, 1H), 4.12-4.17 (m, 1H), 3.41-3.45 (m, 1H), 3.12-3.17 (m, 1H), 2.92-2.97 (m, 2H), 2.79-2.85 (m, 1H), 2.70-2.76 (m, 3H), 2.06-2.17 (m, 3H), 1.94-2.00 (m, 1H), 1.72-1.83 (m, 1H), 1.59-1.68 (m, 1H).

Molecular formula:$C_{19}H_{21}N_5$ Exact mass: 319.18 LC-MS(Pos, *m/z*)=320.08[M+H]$^+$.

**Example 182: Synthesis of (S)-1-(oxetan-3-ylamino)-7-(piperidin-3-ylamino)-2,6-naphthyridine-3-carbonitrile (Compound 182)**

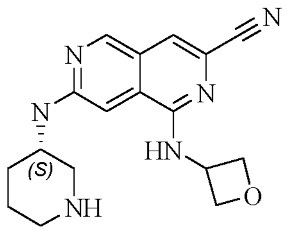

The titled compound was prepared as same as example 154 using corresponding chemicals.

$^1$HNMR (400 MHz, MeOD) δ(ppm): 8.71 (s, 1H), 7.46 (s, 1H), 6.96 (s, 1H), 5.21-5.17 (m, 1H), 5.08-5.04 (m, 2H), 4.77-4.74 (m, 2H), 3.89-3.84 (m, 1H), 3.28-3.27(m, 1H), 3.03-2.99 (m, 1H), 2.71-2.64 (m, 1H), 2.59-2.54 (m, 1H), 2.15-2.12 (m, 1H), 1.89-1.84 (m, 1H), 1.72-1.57 (m, 2H).

Molecular formula: $C_{17}H_{20}N_6O$ Exact mass: 324.17 LC-MS (Pos, *m/z)* =325.22 [M+H]$^+$.

**Example 183: Synthesis of 7-(((1r,3r)-3-aminocyclobutyl)amino)-1-(isopropylamino)-2,6-naphthyridine-3-carbonitrile(Compound 183)**

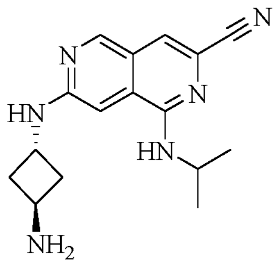

The titled compound was prepared as same as example 38 using corresponding chemicals.

$^1$HNMR (400 MHz, MeOD) δ(ppm): 8.66 (s, 1H), 7.36 (s, 1H), 6.78 (s, 1H), 4.47-4.44 (m, 1H), 4.33-4.30 (m, 1H), 3.73-3.69 (m, 1H), 2.35-2.32 (m, 4H), 1.34-1.33(m, 6H).

Molecular formula: $C_{16}H_{20}N_6$ Exact mass: 296.17 LC-MS (Pos, *m/z)* =297.14 [M+H]$^+$.

**Example 184: Synthesis of 7-(((*trans*)-3-aminocyclobutyl)amino)-1-(ethylamino)-2,6-naphthyridine-3-carbonitrile (Compound 184)**

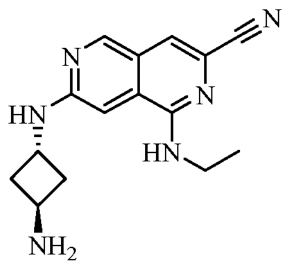

Step 1: synthesis of 7-chloro-1-(ethylamino)-2,6-naphthyridine-3-carbonitrile:

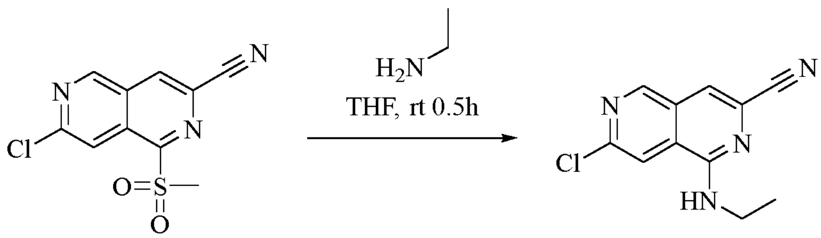


7-Chloro-1-(methylsulfonyl)-2,6-naphthyridine-3-carbonitrile (500 mg, 1.87 mmol, 1.0 eq) and a 65% (by mass) aqueous ethylamine solution (5 mL) was added to anhydrous tetrahydrofuran (5 mL), and the reaction solution was reacted at room temperature for 0.5 h. When there were no starting materials left as monitored by TLC, water (30 mL) was added, and the mixture was extracted with ethyl acetate (30 mL × 3). The organic phases were combined, dried over anhydrous sodium sulfate and filtered, and the filtrate was concentrated under reduced pressure to give the product (420 mg, yield: 96.5%).

Step 2: synthesis of *tert*-butyl ((*trans*)-3-((7-cyano-5-(ethylamino)-2,6-naphthyridin-3-yl)amino)cyclobutyl)carbamate:

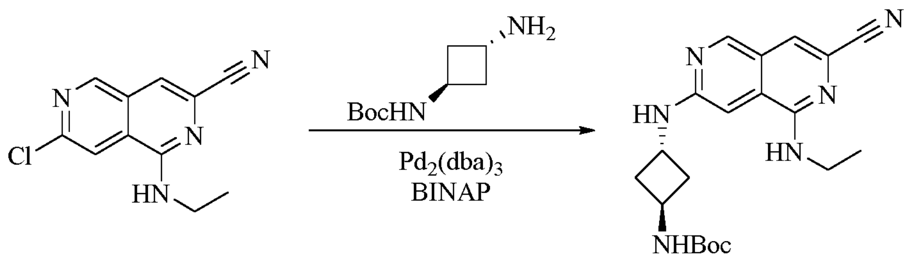


7-Chloro-1-(ethylamino)-2,6-naphthyridine-3-carbonitrile (420 mg, 1.81 mmol, 1.0 eq), *tert*-butyl ((*trans*)-3-aminocyclobutyl)carbamate (404.2 mg, 2.17 mmol, 1.2 eq), $Pd_2(dba)_3$ (247.2 mg, 0.27 mmol, 0.15 eq), BINAP (336.2 mg, 0.54 mmol, 0.3 eq) and cesium carbonate (1.47 g, 4.53 mmol, 2.5 eq) were added to 1,4-dioxane (30 mL). The reaction solution was heated to 100 °C and reacted for 14 h in a sealed tube in nitrogen atmosphere. When there were no starting materials left as monitored by TLC, water (30 mL) was added, and then ethyl acetate (30 mL × 3) was added for extraction. The organic phases were combined, dried over anhydrous sodium sulfate and filtered, and the filtrate was concentrated under reduced pressure to give a crude product, which was purified by silica gel column chromatography (MeOH:DCM = 1:200-1:100) to give the product (242 mg, yield: 35%).

Step 3: synthesis of 7-(((*trans*)-3-aminocyclobutyl)amino)-1-(ethylamino)-2,6-naphthyridine-3-carbonitrile:

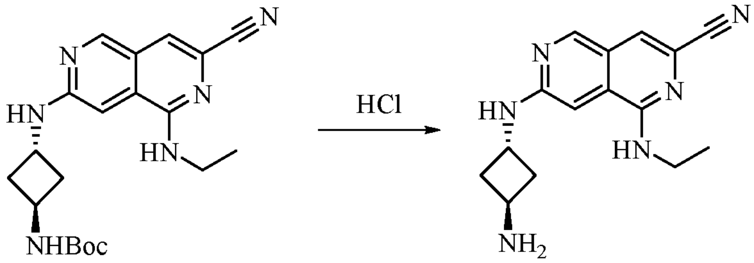


*tert*-Butyl ((*trans*)-3-((7-cyano-5-(ethylamino)-2,6-naphthyridin-3-yl)amino)cyclobutyl)carbamate (240 mg, 0.63 mmol, 1.0 eq) was dissolved in DCM (4 mL), and hydrogen chloride ethanol solution (10 mol/L, 4 mL) was added dropwise. The reaction solution was reacted at room temperature for 2 h. When there were no materials left as monitored by TLC, the reaction solution was concentrated at room temperature under reduced pressure, water (30 mL) was added, and DCM (30 mL × 2) was added for back-extraction. The aqueous phase was adjusted to pH of about 8 with potassium carbonate and extracted with DCM (30 mL × 4). The organic phases were combined, dried over anhydrous sodium sulfate and filtered, and the filtrate was concentrated under reduced pressure to give a crude product, which was purified by preparative thin-layer chromatography (MeOH:DCM = 1:10) to give the product (38 mg, yield: 21.4%).

$^1$HNMR (400 MHz, MeOD) δ(ppm): 8.66 (s, 1H), 7.37 (s, 1H), 6.70 (s, 1H), 4.29-4.26 (m, 1H), 3.72-3.69 (m, 1H), 3.62-3.57 (m, 2H), 2.35-2.31 (m, 4H), 1.34-1.31(m, 3H).

Molecular formula: $C_{15}H_{18}N_6$ Exact mass: 282.16 LC-MS (Pos, *m/z)* =283.14 $[M+H]^+$.

**Example 185: Synthesis of (S)-1-(cyclopentyloxy)-7-(piperidin-3-ylamino)-2,6-naphthyridine-3-carbonitrile (Compound 185)**

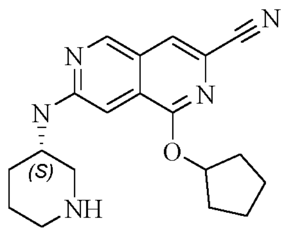

The titled compound was prepared as same as example 205 using corresponding chemicals.

$^1$HNMR (400 MHz, MeOD) δ(ppm): 8.82 (s, 1H), 7.76 (s, 1H), 6.96 (s, 1H), 5.64-5.61 (m, 1H), 4.03-3.98 (m, 1H), 3.06-3.00 (m, 1H), 2.72-2.66 (m, 1H), 2.61-2.55 (m, 1H), 2.12-2.07 (m, 3H), 1.94-1.85 (m, 6H), 1.76-1.71 (m, 3H), 1.61-1.55 (m, 1H). Molecular formula: $C_{19}H_{23}N_5O$ Exact mass: 337.19 LC-MS (Pos, *m/z)* =338.22 $[M+H]^+$.

**Example 186: Synthesis of (*S*)-1-(ethylamino)-7-(piperidin-3-ylamino)-2,6-naphthyridine-3-carbonitrile (Compound 186)**

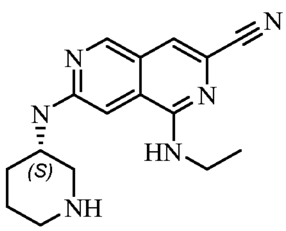

Step 1: synthesis of *tert*-butyl (*S*)-3-((7-cyano-5-(ethylamino)-2,6-naphthyridin-3-yl)amino)piperidine-1-carboxylate:

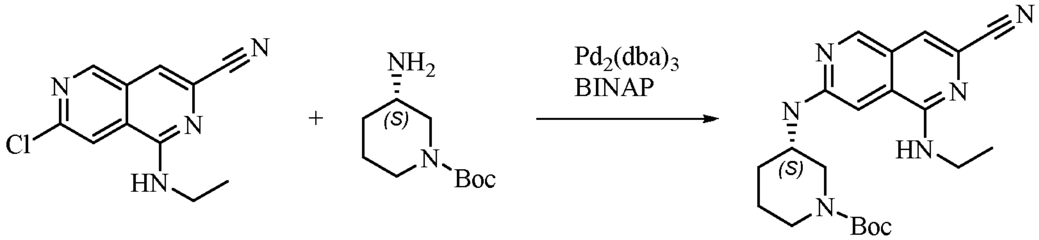


7-Chloro-1-(ethylamino)-2,6-naphthyridine-3-carbonitrile (240 mg, 1.03 mmol, 1.0 eq), tert-butyl (*S*)-3-aminopiperidine-1-carboxylate (248.3 mg, 1.24 mmol, 1.2 eq), $Pd_2(dba)_3$ (137.4 mg, 0.15 mmol, 0.15 eq), BINAP (186.8 mg, 0.3 mmol, 0.3 eq) and cesium carbonate (837.4 mg, 2.57 mmol, 2.5 eq) were added to 1,4-dioxane (20 mL). The mixture was reacted at 100 °C for 14 h in a sealed tube in nitrogen atmosphere. When there were no starting materials left as monitored by TLC, water (30 mL) was added, and the mixture was extracted with ethyl acetate (30 mL × 3). The organic phases were combined, dried over anhydrous sodium sulfate and filtered, and the filtrate was concentrated under reduced pressure to give a crude product, which was purified by silica gel column chromatography (EA:PE = 1:6-1:2) to give the product (300 mg, yield: 73.4%).

Step 2: synthesis of (*S*)-1-(ethylamino)-7-(piperidin-3-ylamino)-2,6-naphthyridine-3-carbonitrile:

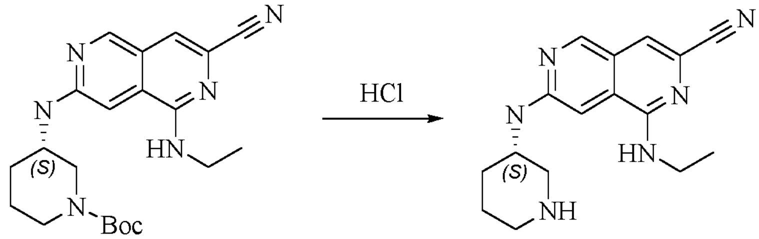


*tert*-Butyl (*S*)-3-((7-cyano-5-(ethylamino)-2,6-naphthyridin-3-yl)amino)piperidine-1-carboxylate (300 mg, 0.76 mmol, 1.0 eq.) was dissolved in DCM (4 mL), and hydrogen chloride ethanol solution (10 mol/L, 4 mL) was added dropwise. The reaction solution was reacted at room temperature for 2 h. When there were no materials left as monitored by TLC, the reaction solution was concentrated at room temperature under reduced pressure, water (30 mL) was added, and DCM (30 mL × 2) was added for back-extraction. The aqueous phase was adjusted to pH of about 8 with potassium carbonate and extracted with DCM (30 mL × 3). The organic phases were combined, dried over anhydrous sodium sulfate and filtered, and the filtrate was concentrated under reduced pressure to give a crude product, which was purified by preparative thin-layer chromatography (MeOH:DCM = 1:10) to give the product (139 mg, yield: 61.7%). $^1$HNMR (400 MHz, MeOD) δ(ppm): 8.66 (s, 1H), 7.37 (s, 1H), 6.86 (s, 1H), 3.85-3.83 (m, 1H), 3.59-3.57 (m, 2H), 3.29-3.26 (m, 1H), 3.02-2.99 (m, 1H), 2.69-2.63 (m, 1H), 2.57-2.52 (m, 1H), 2.12-2.11 (m, 1H), 1.87-1.83 (m, 1H), 1.69-1.55 (m, 2H), 1.34-1.31 (m, 3H).

Molecular formula: $C_{16}H_{20}N_6$ Exact mass: 296.17 LC-MS (Pos, *m/z)* = 297.14[M+H]$^+$.

**Example 187: Synthesis of (S)-1-cyclopentyl-7-(piperidin-3-ylamino)-2,6-naphthyridine-3-carbonitrile (Compound 187)**

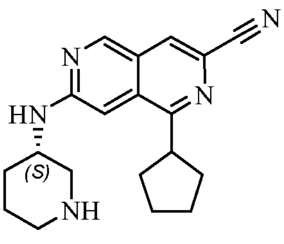

The titled compound was prepared as same as example 38 using corresponding chemicals.

$^1$H-NMR (400 MHz, $CD_3OD$) δ(ppm): 9.01 (s, 1H), 8.08 (s, 1H), 7.12 (s, 1H), 4.32-4.39 (m, 1H), 3.82-3.89 (m, 1H), 3.61-3.66 (m, 1H), 3.35-3.38 (m, 1H), 3.03-3.09 (m, 1H), 2.96-3.02 (m, 1H), 2.18-2.22 (m, 1H), 2.13-2.17 (m, 2H), 2.08-2.12 (m, 1H), 2.00-2.06 (m, 2H), 1.92-1.98 (m, 1H), 1.81-1.92 (m, 4H), 1.72-1.80 (m, 1H).

Molecular formula:$C_{19}H_{23}N_5$ Exact mass: 321.20 LC-MS(Pos, *m*/*z*)=322.13 [M+H]$^+$

**Example 188: Synthesis of (S)-1-(pentan-3-yl)-7-(piperidin-3-ylamino)-2,6-naphthyridine-3-carbonitrile (Compound 188)**

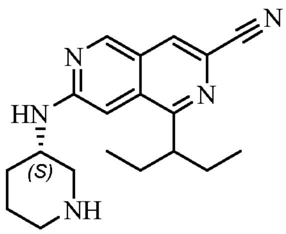

The titled compound was prepared as same as example 38 using corresponding chemicals.

$^1$H-NMR (400 MHz, $CD_3OD$) δ(ppm): 9.04 (s, 1H), 8.11 (s, 1H), 7.14 (s, 1H), 4.31-4.38 (m, 1H), 3.58-3.62 (m, 1H), 3.34-3.40 (m, 1H), 3.00-3.07 (m, 1H), 2.93-2.99 (m, 1H), 2.18-2.22 (m, 1H), 2.09-2.15 (m, 1H), 1.87-2.00 (m, 3H), 1.71-1.86 (m, 4H), 0.80-0.84 (m, 6H).

Molecular formula:$C_{19}H_{25}N_5$ Exact mass: 323.21 LC-MS(Pos, *m*/*z*)=324.15 [M+H]$^+$

**Example 189: Synthesis of (S)-1-ethyl-7-(piperidin-3-ylamino)-2,6-naphthyridine-3-carbonitrile (Compound 189)**

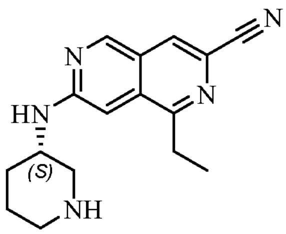

The titled compound was prepared as same as example 38 using corresponding chemicals.

$^1$H-NMR (400 MHz, $CD_3OD$) δ(ppm): 9.03 (s, 1H), 8.12 (s, 1H), 7.04 (s, 1H), 4.30-4.37 (m, 1H), 3.59-3.63 (m, 1H), 3.34-3.37 (m, 1H), 3.15-3.20 (m, 2H), 3.00-3.07 (m, 1H), 2.93-2.98 (m, 1H), 2.18-2.23 (m, 1H), 2.08-2.16 (m, 1H), 1.85-1.96 (m, 1H), 1.71-1.80 (m, 1H), 1.39-1.42 (t, 3H).

Molecular formula:$C_{16}H_{19}N_5$ Exact mass: 281.16 LC-MS(Pos, *m*/*z*)=282.16 [M+H]$^+$

**Example 190: Synthesis of (S)-7-(piperidin-3-ylamino)-2,6-naphthyridine-3-carbonitrile (Compound 190)**

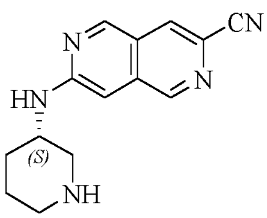

The titled compound was prepared as same as example 38 using corresponding chemicals.

$^1$HNMR (400 MHz, $CD_3OD$) δ(ppm): 9.00-8.99 (d, 1H), 8.22 (s, 1H), 6.84 (s, 1H), 3.97-3.91 (m, 1H), 3.28 (s, 1H), 3.05-2.99 (m, 1H), 2.71-2.64 (m ,1H), 2.60-2.54 (m, 1H), 2.15-2.11 (m, 1H), 1.89-1.83 (m, 1H), 1.73-1.70 (m, 1H), 1.69-1.67 (m, 1H).

Molecular formula:$C_{14}H_{15}N_5$ Exact mass:253.13 LC-MS(Pos, *m*/*z*)=254.07[M+H]$^+$.

**Example 191: Synthesis of 7-(((1r,3r)-3-aminocyclobutyl)amino)-1-ethoxy-2,6-naphthyridine-3-carbonitrile(Compound 191)**

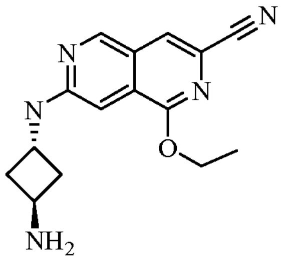

The titled compound was prepared as same as example 51 using corresponding chemicals.

$^1$HNMR (400 MHz, MeOD) δ(ppm): 8.83 (s, 1H), 7.79 (s, 1H), 6.83 (s, 1H), 4.59-4.54 (m, 2H), 4.43-4.40 (m, 1H), 3.79-3.73 (m, 1H), 2.41-2.38 (m, 4H), 1.53-1.48 (m, 3H).

Molecular formula: $C_{15}H_{17}N_5O$ Exact mass: 283.14 LC-MS (Pos, *m*/*z)* =284.21 [M+H]$^+$.

**Example 192: Synthesis of (S)-1-(methylthio)-7-(piperidin-3-ylamino)-2,6-naphthyridine-3-carbonitrile(Compound 192)**

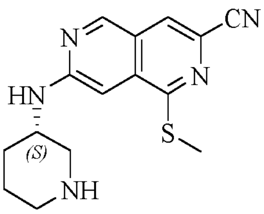

The titled compound was prepared as same as example 38 using corresponding chemicals.

$^1$HNMR (400 MHz, $CD_3OD$) δ(ppm): 8.89 (s, 1H), 7.89 (s, 1H), 6.86 (s, 1H), 4.04-3.99 (m, 1H), 3.30 (s, 1H), 3.06-3.01 (m, 1H), 2.72-2.71 (d ,1H), 2.67 (s, 3H), 2.61-2.56 (m, 1H), 2.15-2.11 (m, 1H), 1.90-1.85 (m, 1H), 1.74-1.70 (m, 1H), 1.69-1.64 (m, 1H). Molecular formula: $C_{15}H_{17}N_5S$ Exact mass: 299.12 LC-MS(Pos, *m*/*z*)=300.05[M+H]$^+$.

**Example 193: Synthesis of (S)-1-cyclohexyl-7-(piperidin-3-ylamino)-2,6-naphthyridine-3-carbonitrile (Compound 193)**

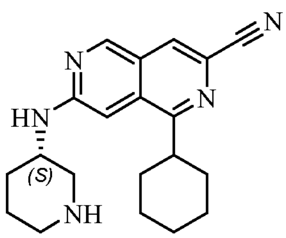

The titled compound was prepared as same as example 38 using corresponding chemicals.

$^1$H-NMR (400 MHz, $CD_3OD$) δ(ppm): 8.98 (s, 1H), 8.05 (s, 1H), 7.02 (s, 1H), 4.10-4.19 (m, 1H), 3.34-3.50 (m, 2H), 3.12-3.17 (m, 1H), 2.70-2.85 (m, 2H), 2.14-2.18 (m, 1H), 1.93-2.00 (m, 4H), 1.82-1.86 (m, 1H), 1.73-1.81 (m, 2H), 1.53-1.72 (m, 4H), 1.34-1.45 (m, 2H).

Molecular formula:$C_{20}H_{25}N_5$ Exact mass: 335.21 LC-MS(Pos, *m*/*z*)=336.13 $[M+H]^+$

**Example 194: Synthesis of (S)-1-cyclopropyl-7-(piperidin-3-ylamino)-2,6-naphthyridine-3-carbonitrile (Compound 194)**

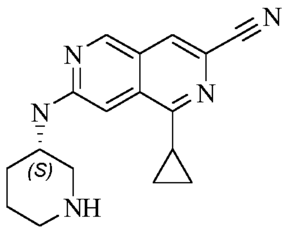

The titled compound was prepared as same as example 38 using corresponding chemicals.

$^1$HNMR (400 MHz, MeOD) δ(ppm): 8.92 (s, 1H), 7.95 (s, 1H), 7.13 (s, 1H), 3.98-3.93 (m, 1H), 3.28 (m, 1H), 3.00-2.97 (m, 1H), 2.66-2.60 (m, 2H), 2.55-2.49 (m, 1H), 2.15-2.12 (m, 1H), 1.86-1.81 (m, 1H), 1.71-1.55 (m, 2H), 1.21-1.15 (m, 4H). Molecular formula: $C_{17}H_{19}N_5$ Exact mass: 293.16 LC-MS (Pos, *m*/*z)* =294.16 $[M+H]^+$.

**Example 195: Synthesis of 7-(((1s,3s)-3-aminocyclobutyl)amino)-1-isopropoxy-2,6-naphthyridine-3-carbonitrile (Compound 195)**

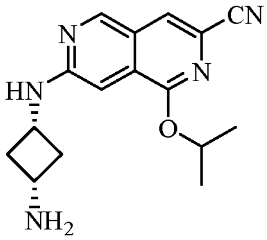

The titled compound was prepared as same as example 51 using corresponding chemicals.

$^1$HNMR (400 MHz, MeOD) δ(ppm): 8.81-8.80 (d, 1H), 7.75 (s, 1H), 6.83 (s, 1H), 5.53-5.47 (m, 1H), 3.98-3.90 (s, 1H), 3.27-3.19 (m, 1H), 2.87-2.81 (m, 1H), 1.78-1.71 (m, 2H), 1.47-1.46 (d, 6H).

Molecular formula:$C_{16}H_{19}N_5O$ Exact mass: 297.16 LC-MS(Pos, *m*/*z*)=298.09$[M+H]^+$.

**Example 196: Synthesis of (S)-1-methyl-7-(piperidin-3-ylamino)-2,6-naphthyridine-3-carbonitrile (Compound 196)**

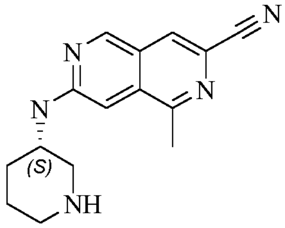

The titled compound was prepared as same as example 38 using corresponding chemicals.

$^1$HNMR (400 MHz, MeOD) δ(ppm): 8.93 (s, 1H), 8.06 (s, 1H), 6.83 (s, 1H), 3.96-3.93 (m, 1H), 3.29-3.26 (m, 1H), 2.99-2.98 (m, 1H), 2.76 (s, 3H), 2.65-2.59 (m, 1H), 2.53-2.48 (m, 1H), 2.13-2.10 (m, 1H), 1.86-1.80 (m, 1H), 1.70-1.52 (m, 2H). Molecular formula: $C_{15}H_{17}N_5$ Exact mass: 267.15 LC-MS (Pos, *m/z)* =268.14 [M+H]$^+$.

**Example 197: Synthesis of (S)-1-cyclopropoxy-7-(piperidin-3-ylamino)-2,6-naphthyridine-3-carbonitrile (Compound 197)**

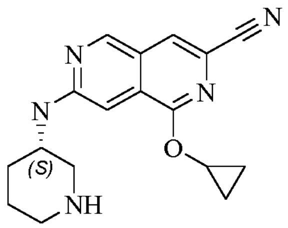

The titled compound was prepared as same as example 205 using corresponding chemicals.
$^1$HNMR (400 MHz, MeOD) δ(ppm): 8.82 (s, 1H), 7.80 (s, 1H), 6.83 (s, 1H), 4.53-4.48 (m, 1H), 3.92-3.88 (m, 1H), 3.27-3.23 (m, 1H), 2.98-2.95 (m, 1H), 2.64-2.58 (m, 1H), 2.52-2.46 (m, 1H), 2.11-2.08 (m, 1H), 1.84-1.79 (m, 1H), 1.66-1.51 (m, 2H), 0.92-0.85 (m, 4H).
Molecular formula: $C_{17}H_{19}N_5O$ Exact mass: 309.16 LC-MS (Pos, *m/z)* =310.14 [M+H]$^+$.

**Example 198: Synthesis of (S)-1-ethoxy-7-(piperidin-3-ylamino)-2,6-naphthyridine-3-carbonitrile (Compound 198)**

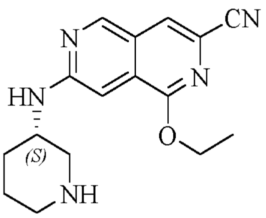

The titled compound was prepared as same as example 205 using corresponding chemicals.
$^1$HNMR (400 MHz, MeOD) δ(ppm): 8.81 (s, 1H), 7.76 (s, 1H), 6.95 (s, 1H), 4.57-4.52 (m, 2H), 3.94-3.89 (m, 1H), 3.28-3.24 (m, 1H), 2.98-2.95 (m, 1H), 2.65-2.58 (m ,1H), 2.53-2.47 (m, 1H), 2.12-2.09 (t, 1H), 1.85-1.79 (m, 1H), 1.69-1.61 (m, 1H), 1.60-1.55 (m, 1H), 1.52-1.48 (t, 3H).
Molecular formula:$C_{16}H_{19}N_5O$ Exact mass: 297.16 LC-MS(Pos, *m/z*)=298.05[M+H]$^+$.

**Example 199: Synthesis of (S)-1-cyclobutoxy-7-(piperidin-3-ylamino)-2,6-naphthyridine-3-carbonitrile (Compound 199)**

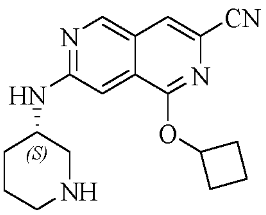

The titled compound was prepared as same as example 205 using corresponding chemicals.
$^1$HNMR (400 MHz, MeOD) δ(ppm): 8.81 (s, 1H), 7.75 (s, 1H), 6.95 (s, 1H), 5.38-5.33 (m, 1H), 3.96-3.92 (m,

1H), 3.31-3.27 (m, 1H), 3.01-2.98 (m, 1H), 2.67-2.50 (m ,4H), 2.30-2.20 (m, 2H), 2.13-2.09 (t, 1H), 1.96-1.89 (m, 1H), 1.86-1.77 (m, 2H), 1.71-1.62 (m, 1H), 1.60-1.53 (m, 1H).

Molecular formula:$C_{18}H_{21}N_5O$ Exact mass: 323.17 LC-MS(Pos, *m/z*)=324.08[M+H]$^+$.

**Example 200: Synthesis of (*trans*)-*N*$^1$-(7-(difluoromethyl)-5-ethoxy-2,6-naphthyridin-3-yl)cyclobutane-1,3-diamine(Compound 200)**

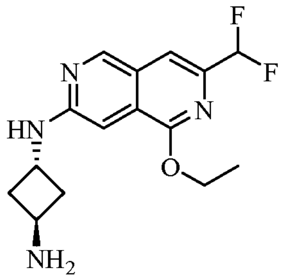

Step 1: synthesis of 7-chloro-3-(difluoromethyl)-1-(methylthio)-2,6-naphthyridine

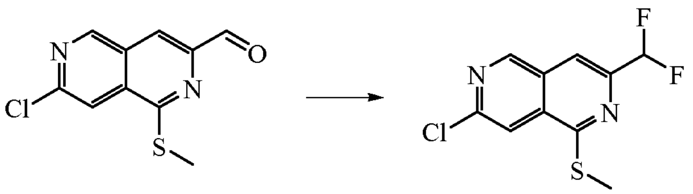

7-Chloro-1-(methylthio)-2,6-naphthyridine-3-carbaldehyde (6.0 g, 25.13 mmol, 1.0 eq.) was dissolved in DCM (60 mL), and DAST (4.46 mg, 27.64 mmol, 1.1 eq.) was added. The reaction solution was reacted at room temperature for 1 h. After the reaction was completed as monitored by TLC, the reaction solution was slowly added dropwise to water (100 mL). The pH was adjusted to about 10 with potassium carbonate, and DCM (50 mL × 3) was added for extraction. The organic phase was dried and concentrated to give a crude product, which was purified by silica gel column chromatography (PE:EA = 10: 1) to give the product (5.0 g, yield: 76.3%).

Step 2: synthesis of 7-chloro-3-(difluoromethyl)-1-(methylsulfonyl)-2,6-naphthyridine

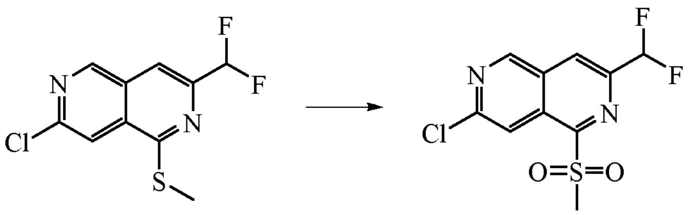

7-Chloro-3-(difluoromethyl)-1-(methylthio)-2,6-naphthyridine (5.0 g, 19.18 mmol, 1.0 eq.) was dissolved in DCM (100 mL), and 85% meta-chloroperoxybenzoic acid (7.79 g, 38.36 mmol, 2.0 eq.) was added. The reaction solution was reacted for 30 min. After the reaction was completed as monitored by TLC, the reaction solution was poured into water (100 mL). The pH was adjusted to about 10 with potassium carbonate, and DCM (50 mL × 2) was added for extraction. The organic phase was dried and concentrated, and the crude product was slurried with a mixed solvent of PE and EA (50 mL, 10:1). The mixture was filtered under vacuum, and the filter cake was dried under vacuum to give the product (5.0 g, yield: 89.1%).

Step 3: synthesis of 7-chloro-3-(difluoromethyl)-1-ethoxy-2,6-naphthyridine

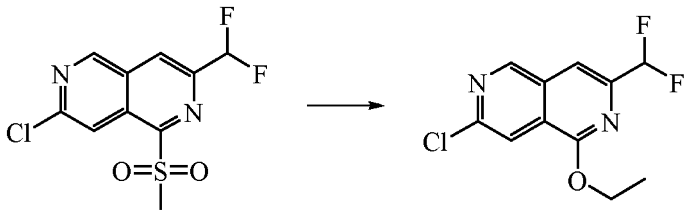

7-Chloro-3-(difluoromethyl)-1-(methylsulfonyl)-2,6-naphthyridine (1.0 g, 3.42 mmol, 1.0 eq.) and potassium carbonate (2.36 g, 17.10 mmol, 5.0 eq.) were dispersed in ethanol (20 mL), and the reaction solution was reacted at room temperature for 17 h. After the reaction was completed as monitored by TLC, the reaction solution was concentrated under reduced pressure. The crude product was slurried with DCM (50 mL), and the mixture was filtered. The filtrate was concentrated under reduced pressure to give the product (800 mg, yield: 90.5%).

Step 4: synthesis of *tert*-butyl ((*trans*)-3-((7-(difluoromethyl)-5-ethoxy-2,6-naphthyridin-3-yl)amino)cyclobutyl)carbamate

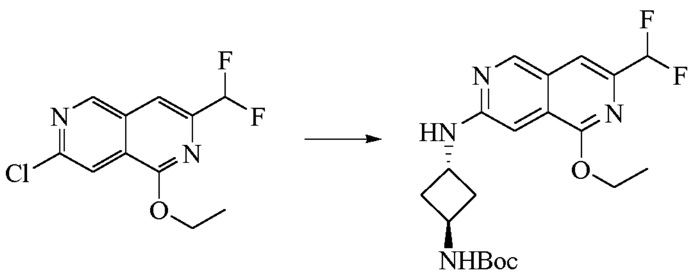

7-Chloro-3-(difluoromethyl)-1-ethoxy-2,6-naphthyridine (800 mg, 3.09 mmol, 1.0 eq.), *tert*-butyl ((*trans*)-3-aminocyclobutyl)carbamate (691 mg, 3.71 mmol, 1.2 eq.), $Pd_2(dba)_3$ (284 mg, 0.31 mmol, 0.1 eq.), BINAP (193 mg, 0.31 mmol, 0.1 eq.) and cesium carbonate (2.01 g, 6.18 mmol, 2.0 eq.) were dispersed in 1,4-dioxane (20 mL), and the reaction solution was reacted at 100 °C for 14 h in nitrogen atmosphere. After the reaction was completed as monitored by TLC, the reaction solution was cooled to room temperature and filtered through celite, and the filtrate was concentrated under reduced pressure to give a crude product (1.26 g, yield: 100%).

Step 5: synthesis of (*trans*)-$N^1$-(7-(difluoromethyl)-5-ethoxy-2,6-naphthyridin-3-yl)cyclobutane-1,3-diamine

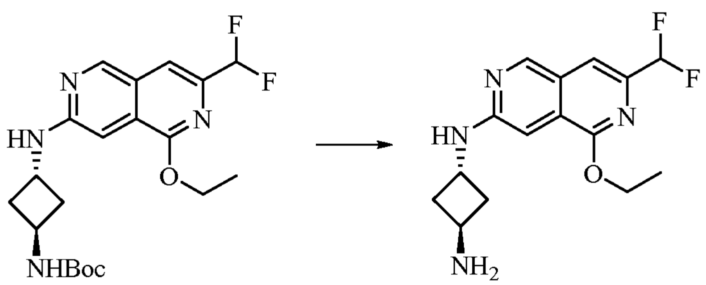

*tert*-Butyl ((*trans*)-3-((7-(difluoromethyl)-5-ethoxy-2,6-naphthyridin-3 - yl)amino)cyclobutyl)carbamate (1.26 g, 3.09 mmol, 1.0 eq.) and 2,6-dimethylpyridine (1.66 g, 15.45 mmol, 5.0 eq.) were dissolved in DCM (20 mL), and TMSOTF (2.06 g, 9.27 mmol, 3.0 eq.) was added dropwise. The reaction solution was reacted at room temperature for 10 min. After the reaction was completed as monitored by TLC, the reaction solution was poured into water (20 mL). The pH was adjusted to about 10 with potassium carbonate, and DCM (20 mL × 2) was added for extraction. The organic phase

was dried and concentrated, and the residue was purified first by silica gel column chromatography (MeOH:DCM = 1:20) and then by preparative thin-layer chromatography (MeOH:DCM = 1:10) to give the product (70 mg, yield: 7.4%).

$^1$HNMR (400 MHz, MeOD) δ(ppm): 8.83 (s, 1H), 7.48 (s, 1H), 6.84 (s, 1H), 6.76 (s, 0.25H), 6.72 (s, 0.5H), 6.48 (s, 0.25H), 4.61-4.56 (m, 2H), 4.37-4.31 (m, 1H), 3.78-3.71 (m, 1H), 2.38-2.35 (t, 4H), 1.52-1.48 (t, 3H).

Molecular formula: $C_{15}H_{18}F_2N_4O$ Exact mass: 308.14 LC-MS (Pos, *m/z)* = 309.04[M+H]$^+$.

**Example 201: Synthesis of 3-(difluoromethyl)-*N*$^7$-((*S*)-piperidin-3-yl)-*N*$^1$-((*R*)-tetrahydrofuran-3-yl)-2,6-naphthyridine-1,7-diamine(Compound 201)**

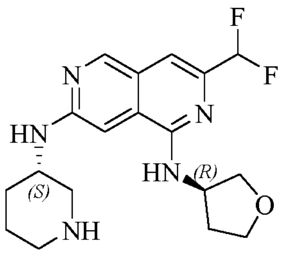

Step 1: synthesis of (*R*)-7-chloro-3-(difluoromethyl)-*N*-(tetrahydrofuran-3-yl)-2,6-naphthyridin-1-amine

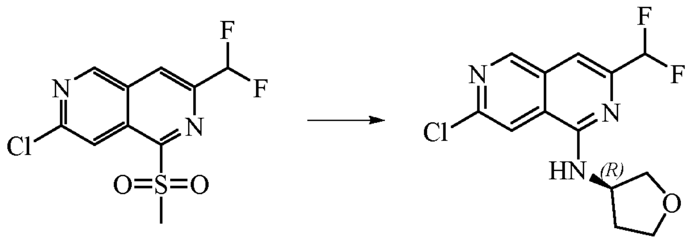

7-Chloro-3-(difluoromethyl)-1-(methylsulfonyl)-2,6-naphthyridine (0.5 g, 1.71 mmol, 1.0 eq.), (*R*)-tetrahydrofuran-3-amine hydrochloride (316 mg, 2.56 mmol, 1.5 eq.) and triethylamine (519 mg, 5.13 mmol, 3.0 eq.) were dissolved in THF (10 mL), and the reaction solution was reacted at room temperature for 24 h. After the reaction was completed as monitored by TLC, the reaction solution was poured into water (10 mL) and extracted with EA (10 mL × 2). The organic phase was dried and concentrated to give the product (420 mg, yield: 82.0%).

Step 2: synthesis of *tert*-butyl (*S*)-3-((7-(difluoromethyl)-5-(((*R*)-tetrahydrofuran-3-yl)amino)-2,6-naphthyridin-3-yl)amino)piperidine-1-carboxylate

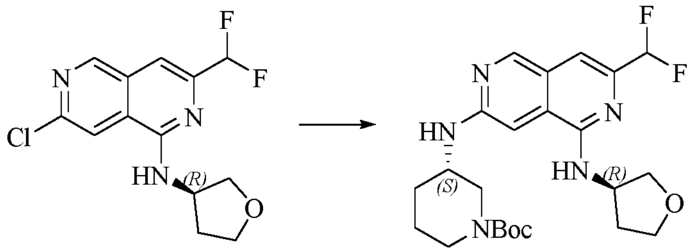

(*R*)-7-chloro-3-(difluoromethyl)-*N*-(tetrahydrofuran-3-yl)-2,6-naphthyridine-1-amine (420 mg, 1.40 mmol, 1.0 eq.), *tert*-butyl (5)-3-aminopiperidine-1-carboxylate (336 mg, 1.68 mmol, 1.2 eq.), $Pd_2(dba)_3$ (128 mg, 0.14 mmol, 0.1 eq.), BINAP (87 mg, 0.14 mmol, 0.1 eq.) and cesium carbonate (912 mg, 2.80 mmol, 2.0 eq.) were dispersed in 1,4-dioxane (20 mL), and the reaction solution was reacted at 100 °C for 14 h in nitrogen atmosphere. After the reaction was completed as monitored by TLC, the reaction solution was cooled to room temperature and filtered through celite, and the filtrate was concentrated under reduced pressure to give a crude product (649 mg, yield: 100%).

Step 3: synthesis of 3-(difluoromethyl)-*N*[7]-((*S*)-piperidin-3-yl)-*N*[1]-((*R*)-tetrahydrofuran-3-yl)-2,6-naphthyridine-1,7-diamine

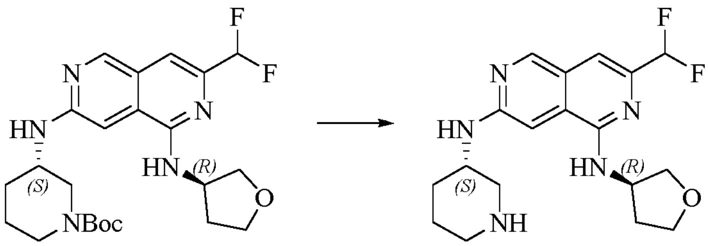

*tert*-Butyl (*S*)-3 -((7-(difluoromethyl)-5-(((*R*)-tetrahydrofuran-3-yl)amino)-2,6-naphthyridin-3-yl)amino)piperidine-1-carboxylate (649 mg, 1.40 mmol, 1.0 eq.) was dissolved in EA (10 mL), and a solution of hydrogen chloride in 1,4-dioxane (4 mol/L, 10 mL) was added dropwise. The reaction solution was reacted at room temperature for 1 h. After the reaction was completed as monitored by LC-MS, the reaction solution was poured into water (10 mL), and the liquid separation was performed. The aqueous phase was retained, adjusted to pH of about 10 with $K_2CO_3$, and extracted with DCM (10 mL × 2). The organic phase was dried and concentrated, and the crude product was purified by preparative thin-layer chromatography (MeOH:DCM = 1: 10) to give the product (260 mg, yield: 51.2%).

$^1$HNMR (400 MHz, MeOD) δ(ppm): 8.69 (s, 1H), 7.13 (s, 1H), 6.95 (s, 1H), 6.67 (s, 0.25H), 6.53 (s, 0.5H), 6.39 (s, 0.25H), 4.81-4.75 (m, 1H), 4.15-4.11 (m, 1H), 4.08-4.03 (m, 1H), 3.91-3.86 (m, 1H), 3.82-3.77 (m, 2H), 3.28-3.24 (t, 1H), 3.00-2.96 (m, 1H), 2.68-2.61 (m, 1H), 2.54-2.49 (m, 1H), 2.43-2.38 (m, 1H), 2.16-2.10 (m, 2H), 1.85-1.81 (m, 1H), 1.68-1.65 (m, 1H), 1.56-1.50 (m, 1H).

Molecular formula: $C_{18}H_{23}F_2N_5O$ Exact mass: 363.19 LC-MS (Pos, *m/z)* = 364.09[M+H]$^+$.

**Example 202: Synthesis of 7-(difluoromethyl)-N-((S)-piperidin-3-yl)-5-(((R)-tetrahydrofuran-3-yl)oxy)-2,6-naphthyridin-3-amine (Compound 202)**

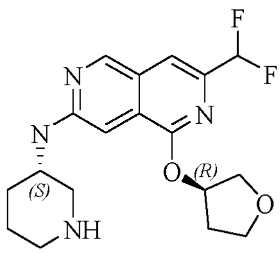

Step 1: synthesis of (*R*)-7-chloro-3-(difluoromethyl)-1-((tetrahydrofuran-3-yl)oxy)-2,6-naphthyridine:

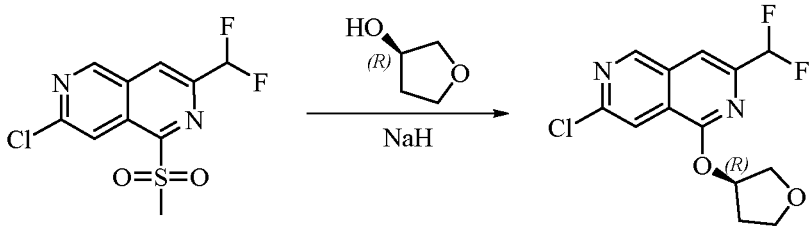

(*R*)-Tetrahydrofuran-3-ol (301.3 mg, 3.42 mmol, 2.0 eq) was added to anhydrous tetrahydrofuran (10 mL), and 60% (by mass) sodium hydride (75.2 mg, 1.88 mmol, 1.1 eq) was added. The reaction solution was reacted at room temperature for 0.5 h. 7-Chloro-3-(difluoromethyl)-1-(methylsulfonyl)-2,6-naphthyridine (500 mg, 1.71 mmol, 1.0 eq) was added. The reaction solution was heated to 50 °C and reacted for 22 h. When there were no starting materials left as monitored by TLC, water (30 mL) was added, and ethyl acetate (30 mL × 3) was added for extraction. The organic phases were combined, dried over anhydrous sodium sulfate and filtered, and the filtrate was concentrated under reduced pressure. The crude product was purified by silica gel column chromatography (EA:PE = 1:15) to give the product (411 mg, yield: 79.9%).

Step 2: synthesis of *tert*-butyl (*S*)-3-((7-(difluoromethyl)-5-(((*R*)-tetrahydrofuran-3-yl)oxy)-2,6-naphthyridin-3-yl)amino)piperidine-1-carboxylate:

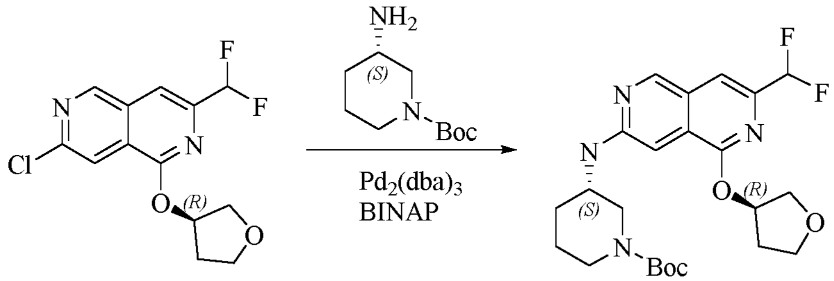


(*R*)-7-Chloro-3-(difluoromethyl)-1-((tetrahydrofuran-3-yl)oxy)-2,6-naphthyridine (210 mg, 0.70 mmol, 1.0 eq.), tert-butyl (*S*)-3-aminopiperidine-1-carboxylate (168.2 mg, 0.84 mmol, 1.2 eq.), $Pd_2(dba)_3$ (64.1 mg, 0.07 mmol, 0.10 eq.), BINAP (87.2 mg, 0.14 mmol, 0.20 eq.) and cesium carbonate (570.2 mg, 1.75 mmol, 2.5 eq.) were added to 1,4-dioxane (15 mL). The mixture was reacted at 100 °C for 14 h in a sealed tube in nitrogen atmosphere. When there were no starting materials left as monitored by TLC, water (30 mL) was added, and the mixture was extracted with ethyl acetate (30 mL × 3). The organic phases were combined, dried over anhydrous sodium sulfate and filtered, and the filtrate was concentrated under reduced pressure to give a crude product, which was purified by silica gel column chromatography (EA:PE = 1:7) to give the product (230 mg, yield: 70.7%).

Step 3: synthesis of 7-(difluoromethyl)-*N*-((*S*)-piperidin-3-yl)-5-(((*R*)-tetrahydrofuran-3-yl)oxy)-2,6-naphthyridin-3-amine:

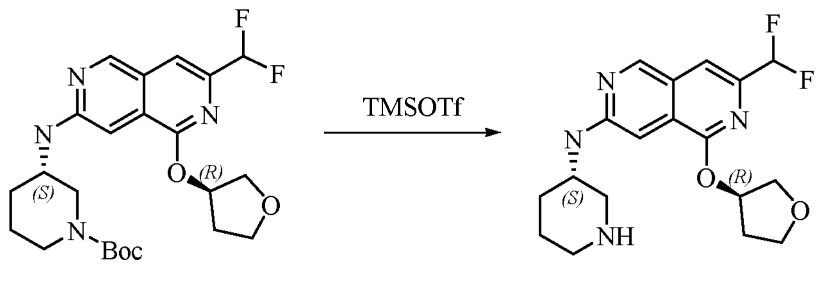


*tert*-Butyl (*S*)-3-((7-(difluoromethyl)-5-(((*R*)-tetrahydrofuran-3-yl)oxy)-2,6-naphthyridin-3-yl)amino)piperidine-1-carboxylate (230 mg, 0.49 mmol, 1.0 eq) was dissolved in DCM (5 mL), and 2,6-dimethylpyridine (315 mg, 2.94 mmol, 6.0 eq) and trimethylsilyl trifluoromethanesulfonate (326.7 mg, 1.47 mmol, 3.0 eq) were added. The reaction solution was reacted at room temperature for 1 h. When there were no starting materials left as monitored by TLC, water (30 mL) was added. The pH was adjusted to about 8 with potassium carbonate, and DCM (30 mL × 3) was added for extraction. The organic phases were combined, dried over anhydrous sodium sulfate and filtered, and the filtrate was concentrated under reduced pressure. The crude product was purified by preparative thin-layer chromatography (MeOH:DCM = 1:10) to give the product (140 mg, yield: 78.4%).

$^1$HNMR (400 MHz, MeOD) δ(ppm): 8.84 (s, 1H), 7.50 (s, 1H), 6.97 (s, 1H), 6.76 (s, 0.25H), 6.26 (s, 0.5H), 6.48 (s, 0.25H), 5.79 (m, 1H), 4.13-4.10 (m, 1H), 4.06-4.00 (m, 2H), 3.97-3.89 (m, 2H), 3.29-3.26 (m, 1H), 2.99-2.96 (m, 1H), 2.66-2.60 (m, 1H), 2.53-2.48 (m, 1H), 2.43-2.36 (m, 1H), 2.29-2.24 (m, 1H), 2.12-2.09 (m, 1H), 1.85-1.80 (m, 1H),

1.67-1.61 (m, 1H), 1.58-1.53 (m, 1H).

Molecular formula: $C_{18}H_{22}F2N_4O_2$ Exact mass: 364.17 LC-MS (Pos, *m/z)* = 365.18[M+H]+.

**Example 203: Synthesis of *N*7-((1s,3s)-3-aminocyclobutyl)-*N*1-(tert-butyl)-3-(difluoromethyl)-2,6-naphthyridine-1,7-diamine (Compound 203)**

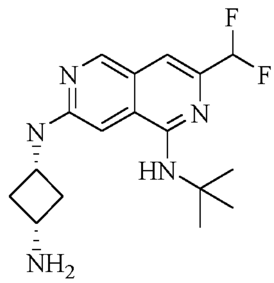

The titled compound was prepared as same as example 201 using corresponding chemicals.

$^1$HNMR (400 MHz, MeOD) δ(ppm): 8.64 (s, 1H), 7.07 (s, 1H), 6.79 (s, 1H), 6.65 (s, 0.25H), 6.51 (s, 0.5H), 6.37 (s, 0.25H), 3.98-3.94 (m, 1H), 3.24-3.20 (m, 1H), 2.91-2.84 (m, 2H), 1.74-1.71 (m, 2H), 1.56 (s, 9H).

Molecular formula: $C_{17}H_{23}F_2N_5$ Exact mass: 335.19 LC-MS (Pos, *m/z)* =336.22[M+H]+.

**Example 204: Synthesis of (*S*)-1-(1-methylcyclopropoxy)-7-(piperidin-3-amino)-2,6-naphthyridine-3-carbonitrile (Compound 204)**

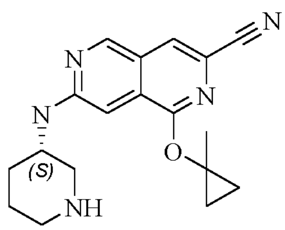

Step 1: synthesis of 7-chloro-1-(1-methylcyclopropoxy)-2,6-naphthyridine-3-carbonitrile

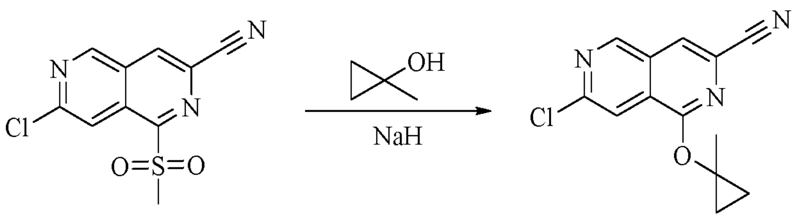


1-Methoxypropan-1-ol (323 mg, 4.48 mmol, 2.0 eq) was dissolved in anhydrous THF (10 mL), and 60% (by mass) sodium hydride (98.4 mg, 2.46 mmol, 1.1 eq) was added. The reaction solution was reacted at room temperature for 0.5 h. 7-Chloro-1-(methylsulfonyl)-2,6-naphthyridine-3-carbonitrile (600 mg, 2.24 mmol, 1.0 eq) was added. The reaction solution was heated to 60 °C and reacted for 22 h. When a new dot was present as monitored by TLC, the reaction solution was cooled to room temperature. Water (30 mL) was added, and ethyl acetate (30 mL × 3) was added for extraction. The organic phases were combined, dried over anhydrous sodium sulfate and filtered, and the filtrate was concentrated under reduced pressure. The crude product was purified by silica gel column chromatography (EA:PE = 1:40-1:20) to give the product (154 mg, yield: 26.5%).

Step 2: synthesis of *tert*-butyl (*S*)-3-(7-cyano-5-(1-methylcyclopropoxy)-2,6-naphthyridin-3-yl)aminopiperidine-1-carboxylate

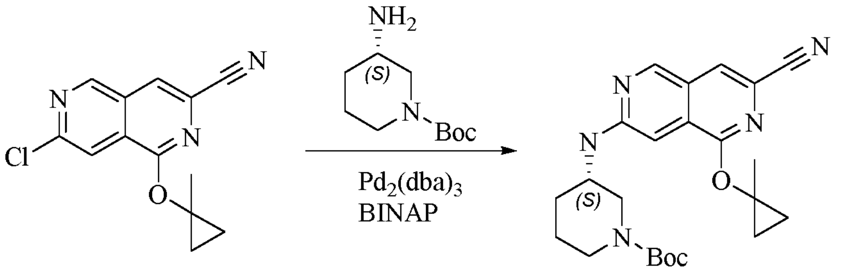


7-Chloro-1-(1-methylcyclopropoxy)-2,6-naphthyridine-3-carbonitrile (154 mg, 0.59 mmol, 1.0 eq), tert-butyl (*S*)-3-aminopiperidine-1-carboxylate (142.2 mg, 0.71 mmol, 1.2 eq), $Pd_2(dba)_3$ (54.9 mg, 0.06 mmol, 0.1 eq), BINAP (74.7 mg, 0.12 mmol, 0.2 eq) and cesium carbonate (478.9 mg, 1.47 mmol, 2.5 eq.) were added to 1,4-dioxane (15 mL). The mixture was reacted at 100 °C for 14 h in a sealed tube in nitrogen atmosphere. When there were no starting materials left as monitored by TLC, water (30 mL) was added, and the mixture was extracted with ethyl acetate (30 mL × 3). The organic phases were combined, dried over anhydrous sodium sulfate and filtered, and the filtrate was concentrated under reduced pressure to give a crude product, which was purified by silica gel column chromatography (EA:PE = 1:6) to give the product (107 mg, yield: 42.8%).

Step 3: synthesis of (*S*)-1-(1-methylcyclopropoxy)-7-(piperidin-3-amino)-2,6-naphthyridine-3-carbonitrile

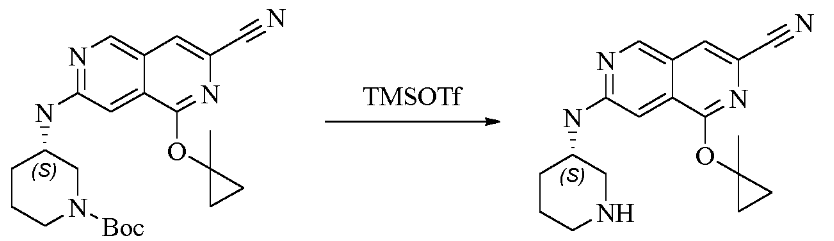


*tert*-Butyl (*S*)-3-(7-cyano-5-(1-methylcyclopropoxy)-2,6-naphthyridin-3-yl)aminopiperidine-1-carboxylate (107 mg, 0.25 mmol, 1.0 eq) was dissolved in DCM (5 mL), and 2,6-dimethylpyridine (160.7 mg, 1.5 mmol, 6.0 eq) and trimethylsilyl trifluoromethanesulfonate (166.7 mg, 0.75 mmol, 3.0 eq) were added. The reaction solution was reacted at room temperature for 1 h. When there were no starting materials left as monitored by TLC, water (30 mL) was added. The pH was adjusted to about 8 with potassium carbonate, and DCM (30 mL × 2) was added for extraction. The organic phases were combined, dried over anhydrous sodium sulfate and filtered, and the filtrate was concentrated under reduced pressure. The crude product was purified by thin-layer chromatography (MeOH:DCM = 1:10) to give the product (18 mg, yield: 22.3 %).

$^1$HNMR(400 MHz, $CD_3OD$) δ(ppm): 8.83 (s, 1H), 7.80 (s, 1H), 6.87 (s, 1H), 3.93-3.88 (m, 1H), 3.28-3.24 (m, 1H), 3.00-2.95 (m, 1H), 2.65-2.59 (m, 1H), 2.52-2.47 (m, 1H), 2.12-2.08 (m, 1H), 1.85-1.83 (m, 1H), 1.75 (s, 3H), 1.67-1.54 (m, 2H), 1.06-1.02 (m, 2H), 0.87-0.84 (m, 2H).

Molecular formula: $C_{18}H_{21}N_5O$ Exact mass: 323.17 LC-MS (Pos, *m/z)* = 324.21[M+H]$^+$.

**Example 205: Synthesis of 1-(isopropylamino)-7-((3-methylpiperidin-3-yl)amino)-2,6-naphthyridine-3-carbonitrile (Compound 205)**

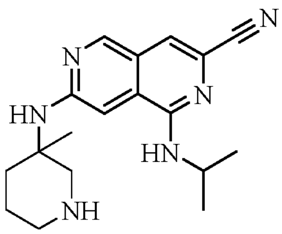

The titled compound was prepared as same as example 38 using corresponding chemicals.

$^1$HNMR (400 MHz, $CD_3OD$) δ(ppm): 8.68 (s, 1H), 7.35 (s, 1H), 6.91 (s, 1H), 4.55-4.42 (m, 1H), 3.58-3.55 (m, 1H), 2.98-2.95 (m, 1H), 2.73-2.67 (m, 2H), 2.39-2.36 (m, 1H), 1.76-1.70 (m, 1H), 1.63-1.58 (m, 2H), 1.47 (s, 3H), 1.34-1.32 (m, 6H).

Molecular formula: $C_{18}H_{24}N_6$ Exact mass: 324.21 LC-MS (Pos, *m/z)* =325.25 $[M+H]^+$.

**Example 206: Synthesis of $N^7$-((1r,3r)-3-aminocyclobutyl)-3-(difluoromethyl)-$N^1$-isopropyl-2,6-naphthyridine-1,7-diamine (Compound 206)**

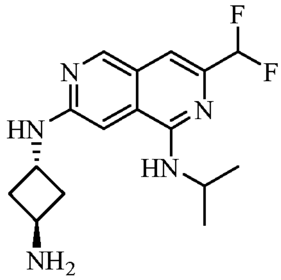

The titled compound was prepared as same as example 184 using corresponding chemicals.

$^1$HNMR (400 MHz, MeOD) δ(ppm): 8.67 (s, 1H), 7.07 (s, 1H), 6.77 (s, 1H), 6.65 (s, 0.25H), 6.51 (s, 0.5H), 6.37 (s, 0.25H), 4.53-4.49 (m, 1H), 4.33-4.30 (m, 1H), 3.74-3.70 (m, 1H), 2.35-2.32 (m, 4H), 1.35-1.33 (m, 6H).

Molecular formula: $C_{16}H_{21}F_2N_5$ Exact mass: 321.18 LC-MS (Pos, *m/z)* =322.21 $[M+H]^+$.

**Example 207: Synthesis of 7-(((1r,3r)-3-aminocyclobutyl)amino)-1-isopropoxy-2,6-naphthyridine-3-carbonitrile (Compound 207)**

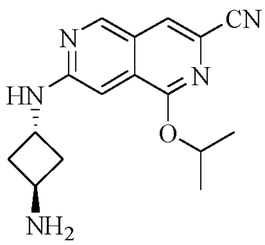

The titled compound was prepared as same as example 51 using corresponding chemicals.

$^1$HNMR (400 MHz, MeOD) δ (ppm): 8.82 (s, 1H), 7.77 (s, 1H), 6.80 (s, 1H), 5.54-5.48 (m, 1H), 4.36-4.34 (m, 1H), 3.71-3.67 (m, 1H), 2.34-2.30 (m, 4H), 1.47-1.46 (m, 6H). Molecular formula: $C_{16}H_{19}N_5O$ Exact mass: 297.16 LC-MS (Pos, *m/z)* =298.30 $[M+H]^+$.

**Example 208: Synthesis of 7-(((1r,3r)-3-amino-3-methylcyclobutyl)amino)-1-(ethylamino)-2,6-naphthyridine-3-carbonitrile(Compound 208)**

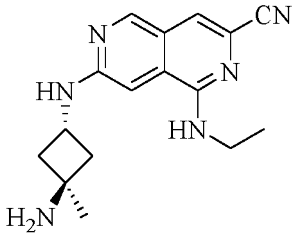

The titled compound was prepared as same as example 210 using corresponding chemicals.

$^1$HNMR (400 MHz, MeOD) δ(ppm): 8.67 (s, 1H), 7.38 (s, 1H), 6.71 (s, 1H), 4.35-4.32 (m, 1H), 3.63-3.58 (m, 2H), 2.60-2.54 (m, 2H), 2.09-2.04 (m, 2H) 1.41 (s, 3H), 1.33-1.32 (m, 6H).

Molecular formula: $C_{16}H_{20}N_6$ Exact mass: 296.17 LC-MS (Pos, *m/z)* =297.17 $[M+H]^+$.

**Example 209: Synthesis of 7-((*trans*)-3-amino-3-methylcyclobutyl)amino-1-(isopropylamino)-2,6-naphthyridine-3-carbonitrile (Compound 209)**

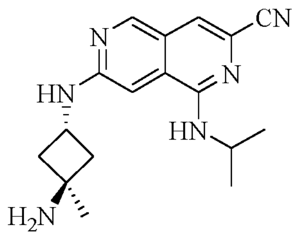

Step 1: synthesis of *tert*-butyl ((*trans*)-3-(7-cyano-5-(isopropylamino)-2,6-naphthyl-3-yl)amino)-1-methylcyclobutyl)carbamate

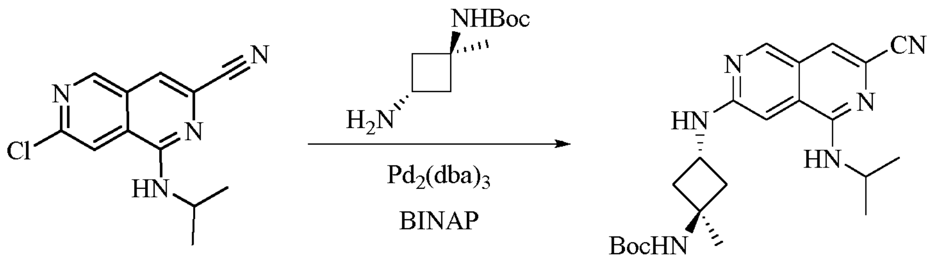


7-Chloro-1-(isopropylamino)-2,6-naphthyridine-3-carbonitrile (246.7 mg, 1 mmol, 1.0 eq), *tert*-butyl ((*trans*)-3-amino-1-methylcyclobutyl)carbamate (240.3 mg, 1.2 mmol, 1.2 eq), $Pd_2(dba)_3$ (91.6 mg, 0.1 mmol, 0.1 eq), BINAP (124.5 mg, 0.2 mmol, 0.2 eq) and cesium carbonate (814.5 mg, 2.5 mmol, 2.5 eq) were added to 1,4-dioxane (20 mL). The reaction solution was heated to 100 °C and reacted for 17 h in a sealed tube in nitrogen atmosphere. When there were no starting materials left as monitored by TLC, water (30 mL) was added, and then ethyl acetate (30 mL × 3) was added for extraction. The organic phases were combined, dried over anhydrous sodium sulfate and filtered, and the filtrate was concentrated under reduced pressure to give a crude product, which was purified by silica gel column chromatography (EA:PE = 1:10-1:2) to give the product (100 mg, yield: 24.4%).

Step 2: synthesis of 7-((*trans*)-3-amino-3-methylcyclobutyl)amino-1-(isopropylamino)-2,6-naphthyridine-3-carbonitrile

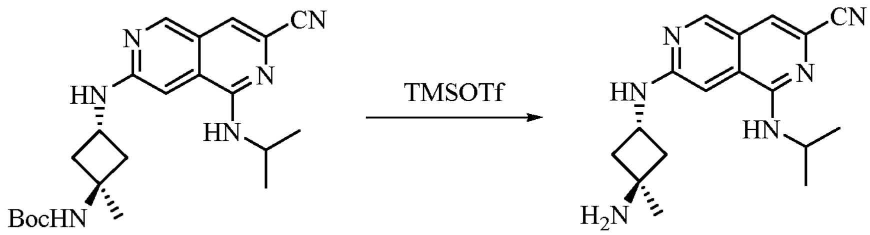


*tert*-Butyl ((*trans*)-3-(7-cyano-5-(isopropylamino)-2,6-naphthyl-3-yl)amino)-1-methylcyclobutyl)carbamate (100 mg, 0.24 mmol, 1.0 eq) was dissolved in DCM (6 mL), 2,6-dimethylpyridine (154.3 mg, 1.44 mmol, 6.0 eq) was added, and a solution of trimethylsilyl trifluoromethanesulfonate (160 mg, 0.72 mmol, 3.0 eq) in DCM (1 mL) was added dropwise. The reaction solution was reacted at room temperature for 1 h. When there were no starting materials left as monitored by TLC, water (30 mL) was added. The pH was adjusted to about 8 with potassium carbonate, and DCM (30 mL × 3) was added for extraction. The organic phases were combined, dried over anhydrous sodium sulfate and filtered, and the filtrate was concentrated under reduced pressure. The crude product was purified first by silica gel column chromatography (MeOH:DCM = 1:100-1:10) and then by thin-layer chromatography (MeOH:DCM = 1:10) to give the product (30 mg, yield: 40.3 %).

$^1$HNMR (400 MHz, MeOD) δ(ppm): 8.66 (s, 1H), 7.37 (s, 1H), 6.78 (s, 1H), 4.48-4.34 (m, 2H), 2.56-2.51 (m, 2H), 2.07-2.02 (m, 2H), 1.40 (s, 3H), 1.35-1.33 (m, 6H). Molecular formula: $C_{17}H_{22}N_6$ Exact mass: 310.19 LC-MS (Pos, *m/z)* = 311.25[M+H]$^+$.

**Example 210: synthesis of 7-((trans)-3-amino-3-methylcyclobutyl)amino-1-(cyclopropylamine)-2,6-naphthyridine-3-carbonitrile(Compound 210)**

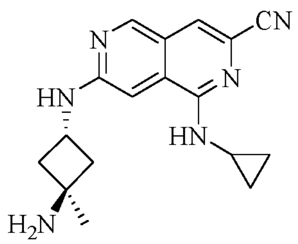

Step 1: synthesis of 7-chloro-1-(cyclopropylamine)-2,6-naphthyridine-3-carbonitrile

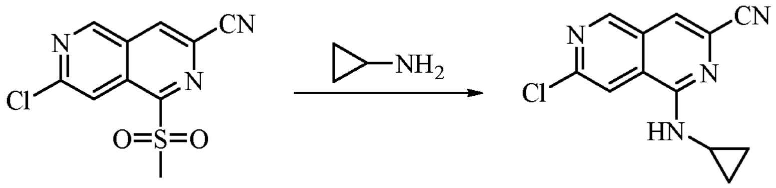

7-Chloro-1-(methylsulfonyl)-2,6-naphthyridine-3-carbonitrile (803.1 mg, 3.0 mmol, 1.0 eq), cyclopropylamine (856.6 mg, 15.0 mmol, 5.0 eq) and DIPEA (1.9 g, 15.0 mmol, 5.0 eq) were dissolved in 1,4-dioxane (10 mL), and the reaction solution was heated to 100 °C and reacted for 2 h. When there were no starting materials left as monitored by TLC, the reaction solution was cooled to room temperature. Water (50 mL) was added, and ethyl acetate (100 mL × 3) was added for extraction. The organic phases were combined, dried over anhydrous sodium sulfate and filtered, and the filtrate was concentrated under reduced pressure. The crude product was purified by silica gel column chromatography (EA:PE = 1:5) to give the product (700 mg, yield: 95.4%).

Step 2: synthesis of *tert*-Butyl ((*trans*)-3-(7-cyano-5-(cyclopropylamine)-2,6-naphthyridin-3-yl)amino)-1-methylcyclobutyl)carbamate

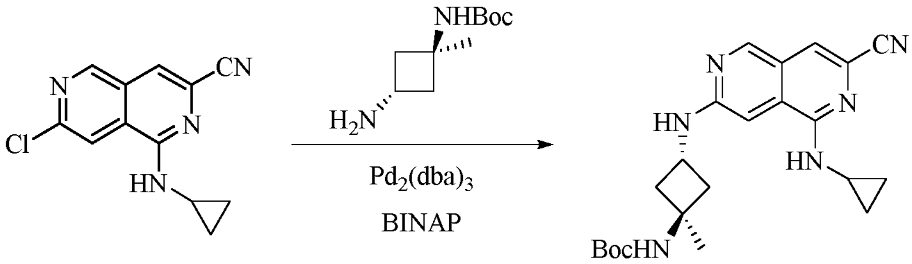


7-Chloro-1-(cyclopropylamine)-2,6-naphthyridine-3-carbonitrile (416 mg, 1.7 mmol, 1.0 eq), *tert*-butyl ((*trans*)-3-amino-1-methylcyclobutyl)carbamate (408.6 mg, 2.04 mmol, 1.2 eq), $Pd_2(dba)_3$ (155.7 mg, 0.17 mmol, 0.1 eq), BINAP (211.7 mg, 0.34 mmol, 0.2 eq) and cesium carbonate (1.38 g, 4.25 mmol, 2.5 eq) were dissolved in 1,4-dioxane (15 mL). The reaction solution was heated to 100 °C and reacted for 16 h in a sealed tube in nitrogen atmosphere. When there were no starting materials left as monitored by TLC, water (30 mL) was added, and then ethyl acetate (30 mL × 3) was added for extraction. The organic phases were combined, dried over anhydrous sodium sulfate and filtered, and the filtrate was concentrated under reduced pressure to give a crude product, which was purified by silica gel column chromatography (EA:PE = 1:10-1:2) to give the product (243 mg, yield: 35%).

Step 3: synthesis of 7-((*trans)*-3-amino-3-methylcyclobutyl)amino-1-(cyclopropylamine)-2,6-naphthyridine-3-carbonitrile

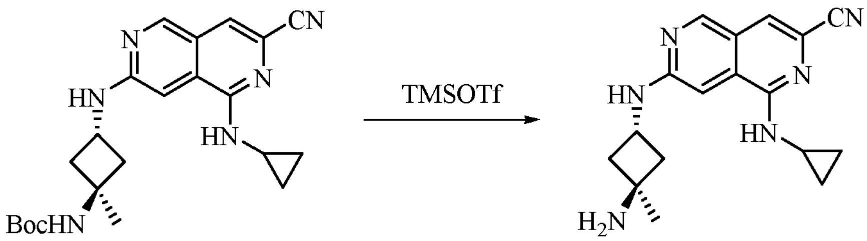


*tert*-Butyl ((*trans*)-3-(7-cyano-5-(cyclopropylamine)-2,6-naphthyridin-3-yl)amino)-1-methylcyclobutyl)carbamate (243 mg, 0.59 mmol, 1.0 eq) was dissolved in DCM (10 mL), 2,6-dimethylpyridine (379.3 mg, 3.54 mmol, 6.0 eq) was added, and a solution of trimethylsilyl trifluoromethanesulfonate (393.4 mg, 1.77 mmol, 3.0 eq) in DCM (2 mL) was added dropwise. The reaction solution was reacted at room temperature for 1 h. When there were no starting materials left as monitored by TLC, water (30 mL) was added. The pH was adjusted to about 8 with potassium carbonate, and DCM (30 mL × 3) was added for extraction. The organic phases were combined, dried over anhydrous sodium sulfate and filtered, and the filtrate was concentrated under reduced pressure. The crude product was purified first by silica gel column chromatography (MeOH:DCM = 1:100-1:10) and then by thin-layer chromatography (MeOH:DCM = 1:10) to give the product (61 mg, yield: 33.5%).

1HNMR (400 MHz, MeOD) δ(ppm): 8.68 (s, 1H), 7.45 (s, 1H), 6.69 (s, 1H), 4.32-4.29 (m, 1H), 2.92-2.90 (m, 1H), 2.55-2.50 (m, 2H), 2.05-2.00 (m, 2H) 1.39 (s, 3H), 0.91-0.88 (m, 2H).

Molecular formula: $C_{17}H_{20}N_6$ Exact mass: 308.17 LC-MS (Pos, *m/z)* = 309.31[M+H]$^+$.

**Example 211: synthesis of 7-((*trans*)-3-amino-3-methylcyclobutyl)amino-1-((1-methylcyclopropyl)amino)-2,6-naphthyridine-3-carbonitrile(Compound 211)**

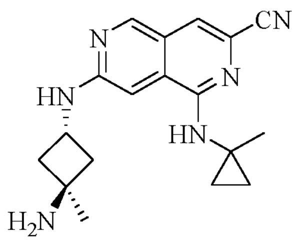

Step 1: synthesis of 7-chloro-1-((1-methylcyclopropyl)amino)-2,6-naphthyridine-3-carbonitrile

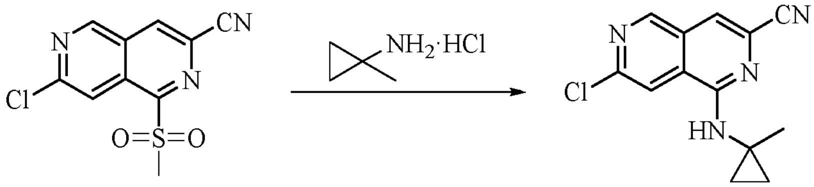

1-Methylcyclopropylamine hydrochloride (806.8 mg, 7.5 mmol, 2.5 eq) was dissolved in 1,4-dioxane (10 mL), and DIPEA (1.9 g, 15.0 mmol, 5.0 eq) was added. The mixture was stirred for 10 min, and 7-chloro-1-(methylsulfonyl)-2,6-naphthyridine-3-carbonitrile (803.1 mg, 3.0 mmol, 1.0 eq) was added. The reaction solution was heated to 80 °C and reacted for 18 h. When there were no starting materials left as monitored by TLC, the reaction solution was cooled to room temperature. Water (50 mL) was added, and ethyl acetate (50 mL × 3) was added for extraction. The organic phases were combined, dried over anhydrous sodium sulfate and filtered, and the filtrate was concentrated under reduced pressure. The crude product was purified by silica gel column chromatography (EA:PE = 1:5) to give the product (446 mg, yield: 57.5%).

Step 2: synthesis of *tert*-butyl ((*trans*)-3-((7-cyano-5-(1-methylcyclopropyl)amino)-2,6-naphthyridin-3-yl)amino-1-methylcyclobutyl)carbamate

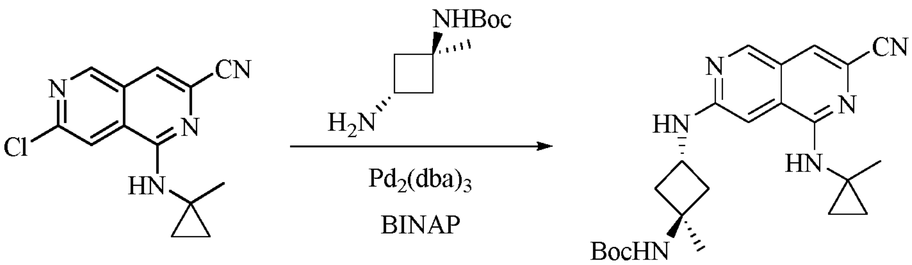


7-Chloro-1-((1-methylcyclopropyl)amino)-2,6-naphthyridine-3-carbonitrile (440 mg, 1.7 mmol, 1.0 eq), *tert*-butyl ((*trans*)-3-amino-1-methylcyclobutyl)carbamate (408.6 mg, 2.04 mmol, 1.2 eq), $Pd_2(dba)_3$ (155.7 mg, 0.17 mmol, 0.1 eq), BINAP (211.7 mg, 0.34 mmol, 0.2 eq) and cesium carbonate (1.38 g, 4.25 mmol, 2.5 eq) were dissolved in 1,4-dioxane (15 mL). The reaction solution was heated to 100 °C and reacted for 16 h in a sealed tube in nitrogen atmosphere. When there were no starting materials left as monitored by TLC, water (30 mL) was added, and then ethyl acetate (30 mL × 3) was added for extraction. The organic phases were combined, dried over anhydrous sodium sulfate and filtered, and the filtrate was concentrated under reduced pressure to give a crude product, which was purified by silica gel column chromatography (EA:PE = 1:10-1:2) to give the product (220 mg, yield: 30.6%).

Step 3: synthesis of 7-((*trans*)-3-amino-3-methylcyclobutyl)amino-1-((1-methylcyclopropyl)amino)-2,6-naphthyridine-3-carbonitrile

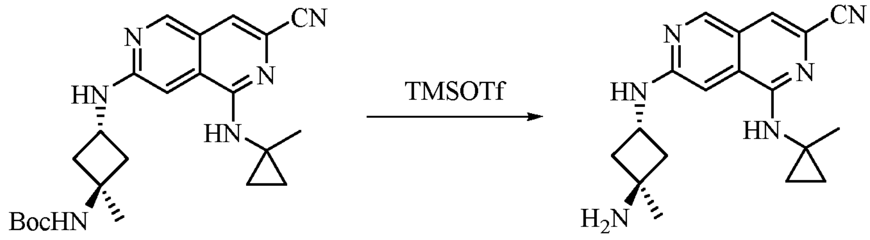


*tert*-Butyl ((*trans*)-3-((7-cyano-5-(1-methylcyclopropyl)amino)-2,6-naphthyridin-3-yl)amino-1-methylcyclobutyl)carbamate (220 mg, 0.52 mmol, 1.0 eq) was dissolved in DCM (10 mL), 2,6-dimethylpyridine (334.3 mg, 3.12 mmol, 6.0 eq) was added, and a solution of trimethylsilyl trifluoromethanesulfonate (346.7 mg,1.56 mmol, 3.0 eq) in DCM (2 mL) was added dropwise. The reaction solution was reacted at room temperature for 1 h. When there were no starting materials left as monitored by TLC, water (30 mL) was added. The pH was adjusted to about 8 with potassium carbonate, and DCM (30 mL × 3) was added for extraction. The organic phases were combined, dried over anhydrous sodium sulfate and filtered, and the filtrate was concentrated under reduced pressure. The crude product was purified first by silica gel column chromatography (MeOH:DCM = 1:100-1:10) and then by thin-layer chromatography (MeOH:DCM = 1:10) to give the product (57 mg, yield: 34 %).

$^1$HNMR (400 MHz, MeOD) δ(ppm): 8.69 (s, 1H), 7.43 (s, 1H), 6.72 (s, 1H), 4.47-4.43 (m, 1H), 2.75-2.69 (m, 2H), 2.20-2.14 (m, 2H), 1.53-1.51 (s, 6H), 0.87 (m, 2H), 0.78 (m, 2H).

Molecular formula: $C_{18}H_{22}N_6$ Exact mass: 322.19 LC-MS (Pos, *m/z)* = 323.30[M+H]$^+$.

**Example 301: Synthesis of ((*S*)-*N*$^8$-(*tert*-butyl)-6-(difluoromethyl)-*N*$^2$-(piperidin-3-yl)pyrido[3,4-d]pyrimidine-2,8-diamine (compound C1)**

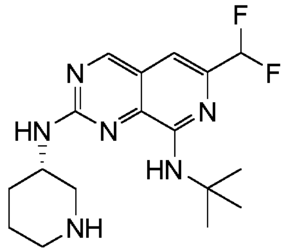

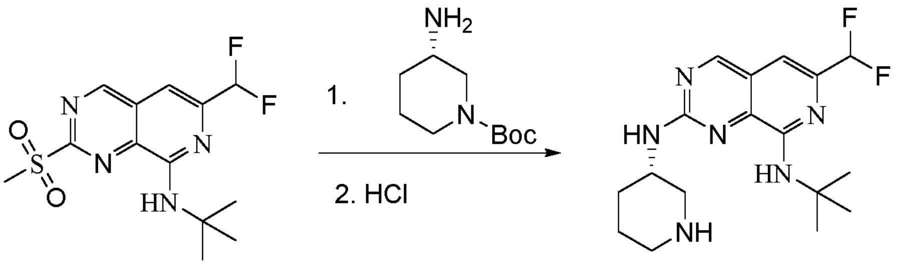


*N*-(*tert*-butyl)-6-(difluoromethyl)-2-(methylsulfonyl)pyrido[3,4-*d*]pyrimidin-8-amine (94 mg, 0.3 mmol, 1.0 eq.) and *tert*-butyl (3*S*)-3-aminopiperidine-1-carboxylate (72 mg, 0.36 mmol, 1.2 eq.) were dissolved in 1,4-dioxane (1.5 mL). The mixture was reacted at 100°C for 2 h. When there were no materials left, as detected by LCMS, the reaction solution was concentrated under reduced pressure. HCl (4N in 1,4-dioxane, 0.5 mL, 2.0 mmol) was added to the crude. The mixture was reacted at room temperature for overnight. When there were no materials left, as detected by LCMS, the reaction solution was concentrated under reduced pressure to give a crude product, which was purified by HPLC to give the product (37.7 mg, yield: 36%).

Molecular formula: $C_{17}H_{24}F_2N_6$, Exact mass: 350.20, LC-MS (Pos, *m*/*z):* 351.2 [M+H]$^+$.

**Example 302: Synthesis of $N^8$-(*tert*-butyl)-6-(difluoromethyl)-$N^2$-(piperidin-4-yl)pyrido[3,4-*d*]pyrimidine-2,8-diamine (compound C2)**

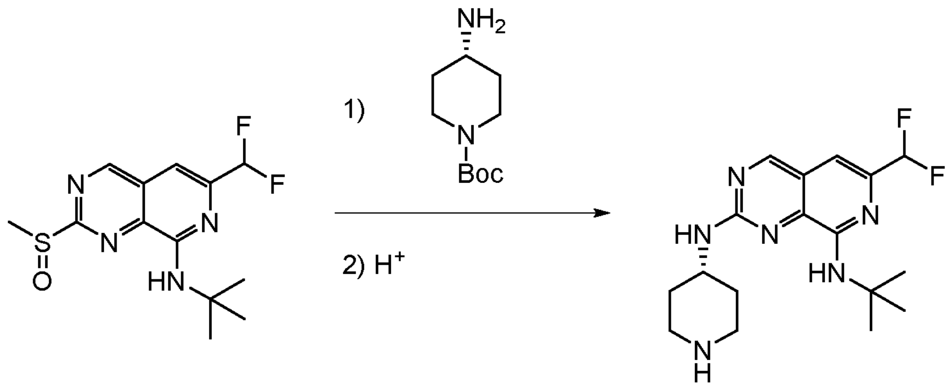


The titled compound was prepared as same as example 301 using corresponding chemicals.
Molecular formula: $C_{17}H_{24}F_2N_6$, Exact mass: 350.20, LC-MS (Pos, *m/z*): 351.1 $[M+H]^+$.

**Example 303: Synthesis of 2-(4-((6-cyano-8-(isopropylamino)pyrido[3,4-*d*]pyrimidin-2-yl)amino)piperidin-1-yl)-*N,N*-dimethylacetamide (compound C3)**

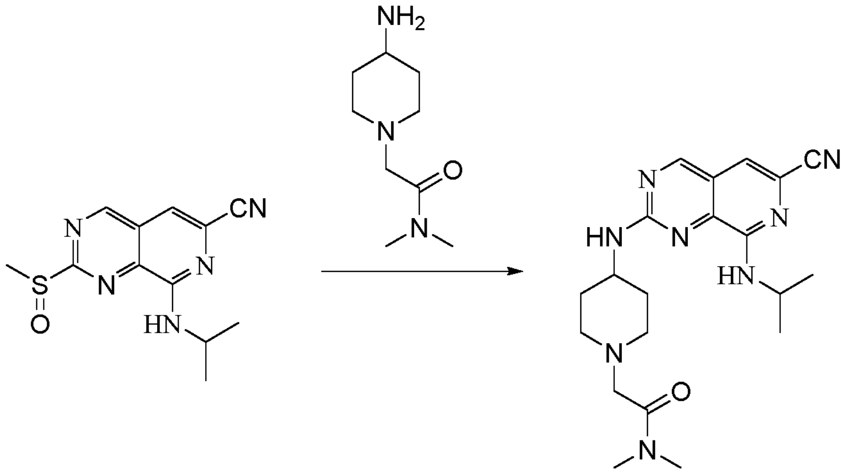

8-(isopropylamino)-2-(methylsulfinyl)pyrido[3,4-*d*]pyrimidine-6-carbonitrile (40 mg, 0.145 mmol, 1.0 eq.) and 2-(4-aminopiperidin-1-yl)-*N*,*N*-dimethylacetamide (30 mg, 0.16 mmol, 1.1 eq.) were dissolved in 1,4-dioxane (10 mL). The mixture was reacted at 100°C for 2 h. When there were no materials left, as detected by LCMS, the reaction solution was concentrated under reduced pressure to give a crude product, which was purified by HPLC to give the product (13 mg, yield: 23%). Molecular formula: $C_{20}H_{28}N_8O$, Exact mass: 396.24, LC-MS (Pos, *m/z*): 397.1 $[M+H]^+$.

**Example 304: Synthesis of $N^2$-((1*R*,5*S*,6*s*)-3-azabicyclo[3.1.0]hexan-6-yl)-$N^8$-(*tert*-butyl)-6-(difluoromethyl)pyrido[3,4-*d*]pyrimidine-2,8-diamine (compound C4)**

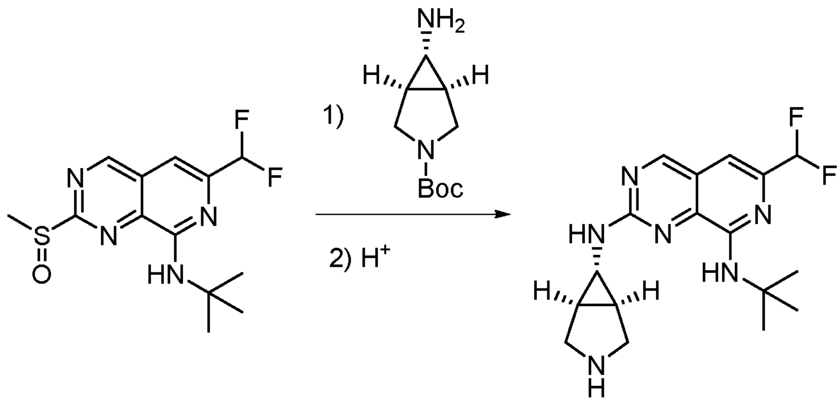


The titled compound was prepared as same as example 301 using corresponding chemicals.

Molecular formula: $C_{17}H_{22}F_2N_6$, Exact mass: 348.19, LC-MS (Pos, *m/z):* 349.2 $[M+H]^+$.

**Example 305: Synthesis of (*R*)-2-(azepan-3-ylamino)-8-(isopropylamino)pyrido[3,4-*d*]pyrimidine-6-carbonitrile (compound C5)**

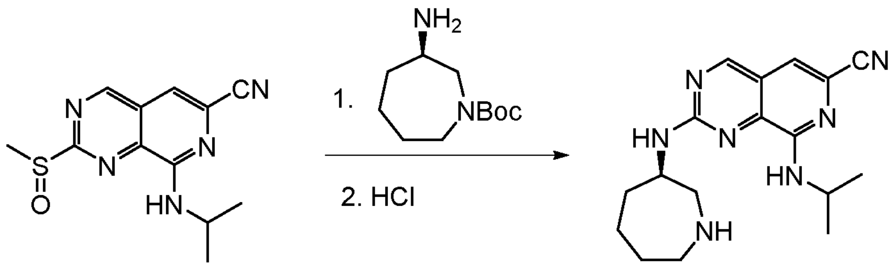


8-(isopropylamino)-2-(methylsulfinyl)pyrido[3,4-*d*]pyrimidine-6-carbonitrile (27.5 mg, 0.1 mmol, 1.0 eq.) and *tert*-butyl (*R*)-3-aminoazepane-1-carboxylate (32 mg, 0.15 mmol, 1.5 eq.) were dissolved in 1,4-dioxane (0.5 mL). The mixture was reacted at 100°C for 2 h. When there were no materials left, as detected by LCMS, the reaction solution was concentrated under reduced pressure. HCl (4N in 1,4-dioxane, 0.5 mL, 2.0 mmol) was added to the crude. The mixture was reacted at room temperature for overnight. When there were no materials left, as detected by LCMS, the reaction solution was concentrated under reduced pressure to give a crude product, which was purified by HPLC to give the product (16.9 mg, yield: 52%).

Molecular formula: $C_{17}H_{23}N_7$, Exact mass: 325.20, LC-MS (Pos, *m/z):* 326.3 $[M+H]^+$.

**Example 306: Synthesis of (S)-2-(azepan-3-ylamino)-8-(isopropylamino)pyrido[3,4-*d*]pyrimidine-6-carbonitrile (compound C6**)

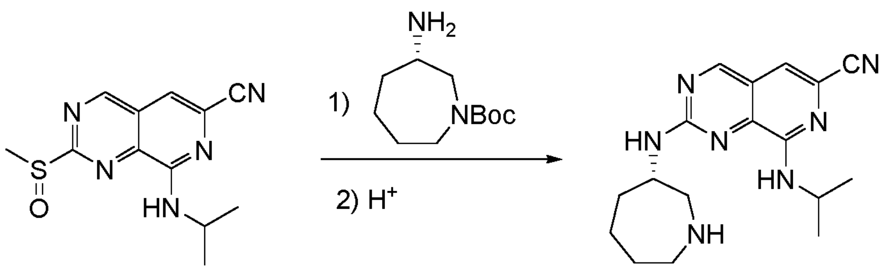


The titled compound was prepared as same as example 305 using corresponding chemicals.

Molecular formula: $C_{17}H_{23}N_7$, Exact mass: 325.20, LC-MS (Pos, *m/z):* 326.2 $[M+H]^+$.

**Example 307: Synthesis of (*S*)-8-(tert-butylamino)-2-(piperidin-3-ylamino)pyrido[3,4-*d*]pyrimidine-6-carbonitrile (compound C7)**

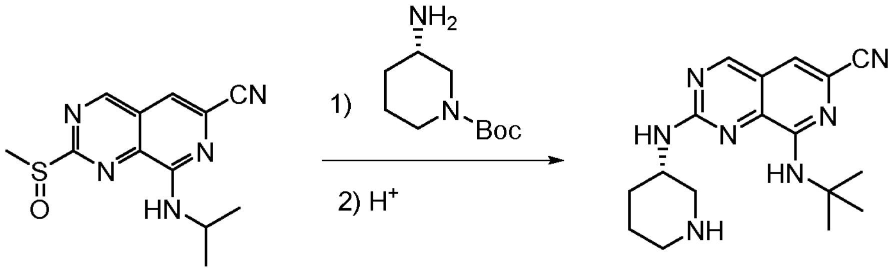


The titled compound was prepared as same as example 305 using corresponding chemicals.

Molecular formula: $C_{17}H_{23}N_7$, Exact mass: 325.20, LC-MS (Pos, *m*/*z*): 326.2 $[M+H]^+$.

**Example 308: Synthesis of (1*r*,3*r*)-3-((8-(*tert*-butylamino)-6-(difluoromethyl)pyrido[3,4-*d*]pyrimidin-2-yl)amino)cyclobutan-1-ol (compound C8)**

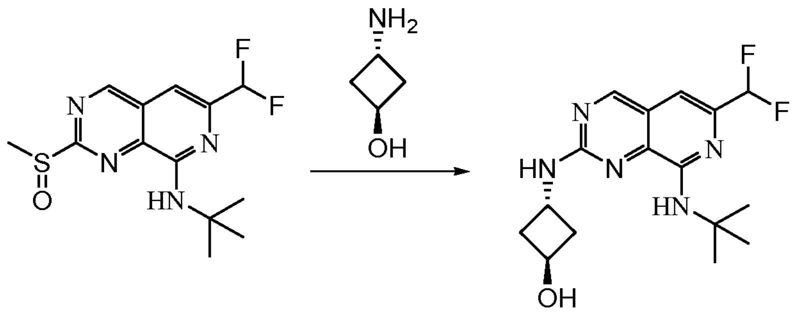

The titled compound was prepared as same as example 303 using corresponding chemicals.

Molecular formula: $C_{16}H_{21}F_2N_5O$, Exact mass: 337.17, LC-MS (Pos, *m*/*z*): 338.0 $[M+H]^+$.

**Example 309: Synthesis of 8-(*tert*-butylamino)-2-(((1*r*,3*r*)-3-hydroxycyclobutyl)amino)pyrido[3,4-*d*]pyrimidine-6-carbonitrile (compound C9)**

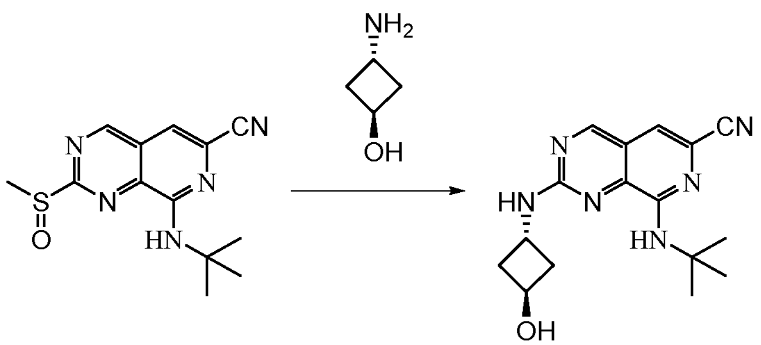

The titled compound was prepared as same as example 303 using corresponding chemicals.

Molecular formula: $C_{16}H_{20}N_6O$, Exact mass: 312.17, LC-MS (Pos, *m*/*z*): 313.0 $[M+H]^+$.

**Example 310: Synthesis of 2-(((1*r*,3*r*)-3-hydroxy-1-methylcyclobutyl)amino)-8-(isopropylamino)pyrido[3,4-*d*]pyrimidine-6-carbonitrile (compound C10)**

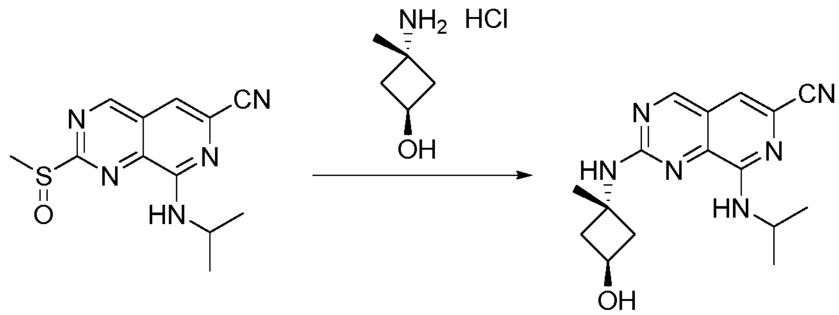

The titled compound was prepared as same as example 303 using corresponding chemicals.

Molecular formula: $C_{16}H_{20}N_6O$, Exact mass: 312.17, LC-MS (Pos, *m/z):* 313.0 $[M+H]^+$.

**Example 311: Synthesis of 2-(((1*r*,3*r*)-3-hydroxy-3-methylcyclobutyl)amino)-8-(isopropylamino)pyrido[3,4-*d*]pyrimidine-6-carbonitrile (compound C11)**

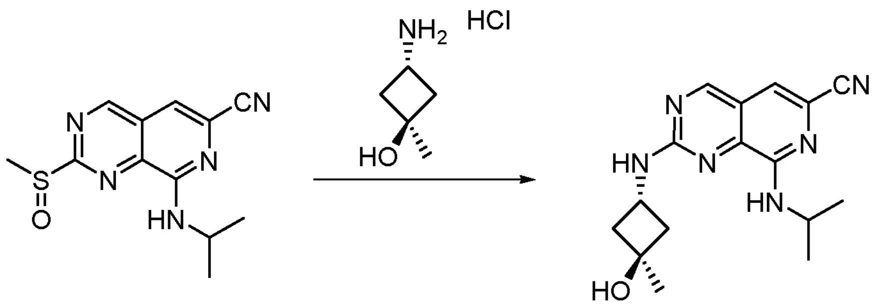

The titled compound was prepared as same as example 303 using corresponding chemicals.

Molecular formula: $C_{16}H_{20}N_6O$, Exact mass: 312.17, LC-MS (Pos, *m/z):* 313.0 $[M+H]^+$.

**Example 312: Synthesis of 8-(isopropylamino)-2-(((1*r*,3*r*)-3-methoxycyclobutyl)amino)pyrido[3,4-*d*]pyrimidine-6-carbonitrile (compound C12)**

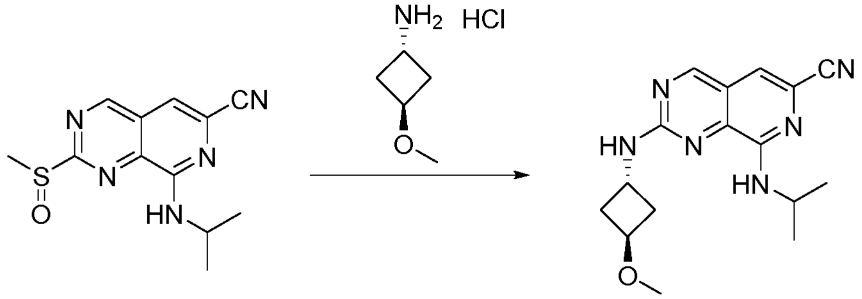

The titled compound was prepared as same as example 303 using corresponding chemicals.

Molecular formula: $C_{16}H_{20}N_6O$, Exact mass: 312.17, LC-MS (Pos, *m/z):* 313.0 $[M+H]^+$.

**Example 313: Synthesis of 2-(((1*r*,3*R*)-3-aminocyclobutyl)amino)-8-(((*R*)-tetrahydrofuran-3-yl)amino)pyrido[3,4-*d*]pyrimidine-6-carbonitrile (compound C13)**

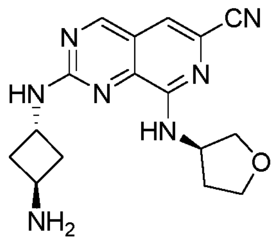

Steps:

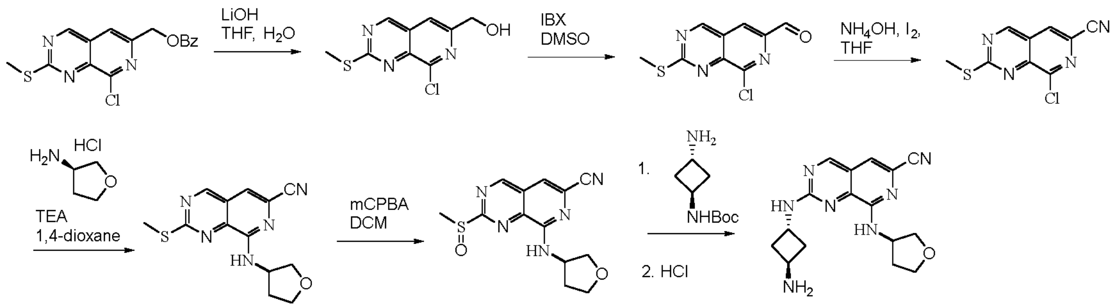


Step 1: synthesis of (8-chloro-2-(methylthio)pyrido[3,4-*d*]pyrimidin-6-yl)methanol

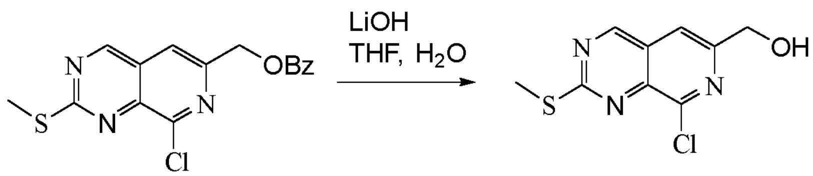


To a stirred solution of (8-chloro-2-(methylthio)pyrido[3,4-*d*]pyrimidin-6-yl)methyl benzoate (4.0 g, 11.59 mmol) in THF (50 mL) was added aq LiOH solution (1.4 g, 34.78 mmol, in 15 mL of $H_2O$) and stirred the resulting reaction mixture at room temperature for 4 h. Monitored by TLC which shows reaction completed. Neutralized the reaction mixture by aq. solution of citric acid and extracted with ethyl acetate (2x50 mL). Combined organic part was washed with saturated aq $NaHCO_3$ solution, dried over $Na_2SO_4$ and evaporated the solvent to get crude (2.0 g) compound which was pure enough to forwarded to next step.

Step 2: synthesis of 8-chloro-2-(methylthio)pyrido[3,4-*d*]pyrimidine-6-carbaldehyde

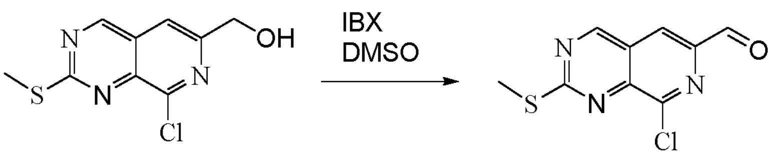


To a stirred solution of (8-chloro-2-(methylthio)pyrido[3,4-*d*]pyrimidin-6-yl)methanol (1.0 g, 4.19 mmol) in DMSO

(15.0 ml) was added IBX (2.3 g, 8.30 mmol) at 0°C. Stirred the resulting reaction mixture at room temperature for 0.5 h. Monitored by TLC which shows reaction completed. the reaction mixture was quenched with cold water (25 ml) and extracted with ethyl acetate (3x25 mL). Combined organic part was further washed with cold water (3x50 mL) and then with brine, dried over $Na_2SO_4$ and concentrated under redsuced pressure to get crude (1.0 g) compound which was pure enough and forwarded to next step.

Step 3: synthesis of 8-chloro-2-(methylthio)pyrido[3,4-*d*]pyrimidine-6-carbonitrile

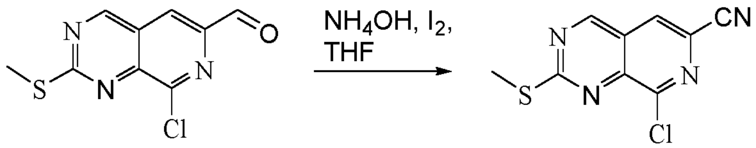


To a stirred solution of 8-chloro-2-(methylthio)pyrido[3,4-*d*]pyrimidine-6-carbaldehyde (2.6 g, 10.88 mmol) in THF (35.0 mL) were added $NH_4OH$ (20 mL) and $I_2$ (3.02 g, 11.97 mmol) and stirred the reaction mixture at room temperature for 4h. Reaction was monitored by TLC which shows SM was consumed. The reaction mixture was quenched with aq. sodium thiosulphate (20 ml) and extracted with ethyl acetate (3x50 mL). Combined organic part was washed with brine, dried over $Na_2SO_4$ and concentrated under reduced pressure to get crude compound. Crude was purified by silics gel column chromatography to get the compound (850 mg, yield: 33%).

Step 4: synthesis of (*R*)-2-(methylthio)-8-((tetrahydrofuran-3-yl)amino)pyrido[3,4-*d*]pyrimidine-6-carbonitrile

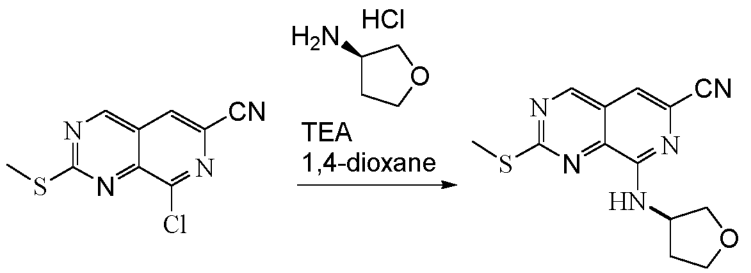


To a stirred solution of 8-chloro-2-(methylthio)pyrido[3,4-*d*]pyrimidine-6-carbonitrile (94.7 mg, 0.4 mmol) in 1,4-dioxane (2 mL) was added (3*R*)-tetrahydrofuran-3-amine hydrochloride (59.3 mg, 0.48 mmol) and TEA (0.139 mL, 1.0 mmol) and stirred the reaction mixture at 100°C for overnight. Reaction was monitored by LCMS which shows SM was consumed. The reaction mixture was quenched with water and extracted with ethyl acetate (2x20 mL). Combined organic part was washed with brine, dried over $Na_2SO_4$ and concentrated under reduced pressure to get crude compound which was used in the next step without further purification. Exact mass: 287.08, LC-MS (Pos, *m/z):* 288.7 $[M+H]^+$.

Step 5: synthesis of 2-(methylsulfinyl)-8-(((*R*)-tetrahydrofuran-3-yl)amino)pyrido[3,4-*d*]pyrimidine-6-carbonitrile

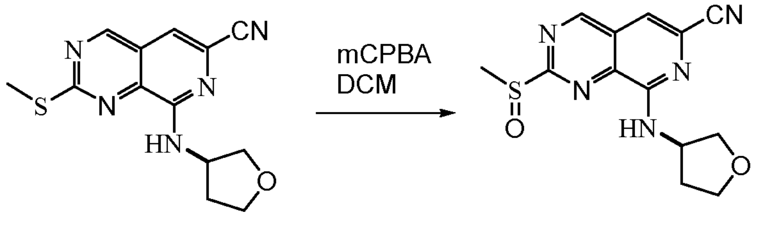


To a stirred solution of (*R*)-2-(methylthio)-8-((tetrahydrofuran-3-yl)amino)pyrido[3,4-*d*]pyrimidine-6-carbonitrile (0.4 mmol) in DCM (2.0 mL) was added mCPBA (127 mg, 0.48 mmol, 65%) and stirred the reaction mixture at 0°C for

4h. Reaction was monitored by LCMS which shows SM was consumed. The reaction mixture was quenched with saturated aq $NaHCO_3$ solution and extracted with ethyl acetate (2x20 mL). Combined organic part was washed with brine, dried over $Na_2SO_4$ and concentrated under reduced pressure to get crude compound which was purified by short silica gel. The crude was used in the next step without further purification. Exact mass: 303.08, LC-MS (Pos, *m/z):* 304.8 $[M+H]^+$.

Step 6: synthesis of 2-(((1*r*,3*R*)-3-aminocyclobutyl)amino)-8-(((*R*)-tetrahydrofuran-3-yl)amino)pyrido[3,4-*d*]pyrimidine-6-carbonitrile

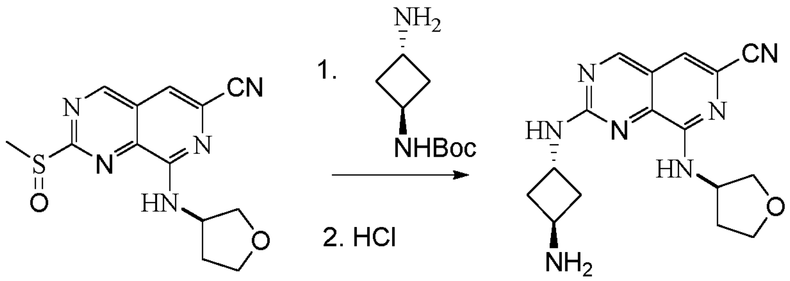


2-(methylsulfinyl)-8-(((*R*)-tetrahydrofuran-3-yl)amino)pyrido[3,4-*d*]pyrimidine-6-carbonitrile (30.3 mg, 0.1 mmol, 1.0 eq.) and *tert*-butyl *N*-(3-aminocyclobutyl)carbamate (20.5 mg, 0.11 mmol, 1.1 eq.) were dissolved in 1,4-dioxane (0.5 mL). The mixture was reacted at 100°C for 1 h. When there were no materials left, as detected by LCMS, the reaction solution was concentrated under reduced pressure. HCl (4N in 1,4-dioxane, 0.5 mL, 2.0 mmol) was added to the crude. The mixture was reacted at room temperature for overnight. When there were no materials left, as detected by LCMS, the reaction solution was concentrated under reduced pressure to give a crude product, which was purified by HPLC to give the product (17.5 mg, yield: 54%).
Molecular formula: $C_{16}H_{19}N_7O$, Exact mass: 325.17, LC-MS (Pos, *m/z):* 326.2 $[M+H]^+$.

**Example 314: Synthesis of 2-(((1*r*,3*r*)-3-aminocyclobutyl)amino)-8-(propylamino)pyrido[3,4-*d*]pyrimidine-6-carbonitrile (compound C14)**

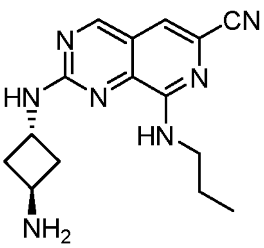

Steps:

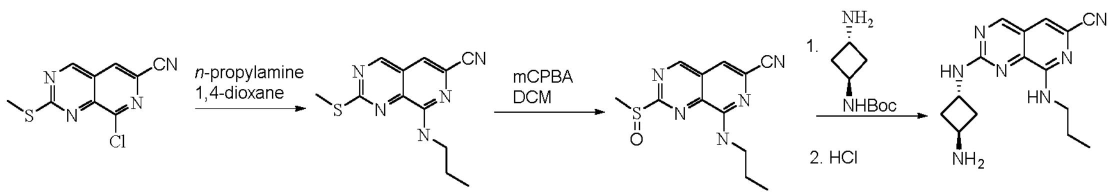

Step 1: synthesis of 2-(methylthio)-8-(propylamino)pyrido[3,4-*d*]pyrimidine-6-carbonitrile

The titled compound was prepared as same as the step 4 of example 313 using corresponding chemicals.

Step 2: synthesis of 2-(methylsulfinyl)-8-(propylamino)pyrido[3,4-*d*]pyrimidine-6-carbonitrile

The titled compound was prepared as same as the step 5 of example 313 using corresponding chemicals.
Exact mass: 275.08, LC-MS (Pos, *m/z):* 276.0 $[M+H]^+$.

Step 3: synthesis of 2-(((1*r*,3*r*)-3-aminocyclobutyl)amino)-8-(propylamino)pyrido[3,4-*d*]pyrimidine-6-carbonitrile

The titled compound was prepared as same as the step 6 of example 313 using corresponding chemicals.
Molecular formula: $C_{15}H_{19}N_7$, Exact mass: 297.17, LC-MS (Pos, *m/z):* 298.2 $[M+H]^+$.

**Example 315: Synthesis of 2-(((1*r*,3*r*)-3-aminocyclobutyl)amino)-8-(piperidin-1-yl)pyrido[3,4-*d*]pyrimidine-6-carbonitrile (compound C15)**

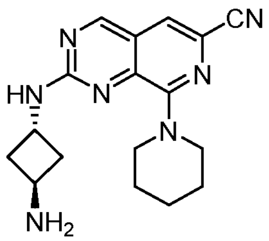

Steps:

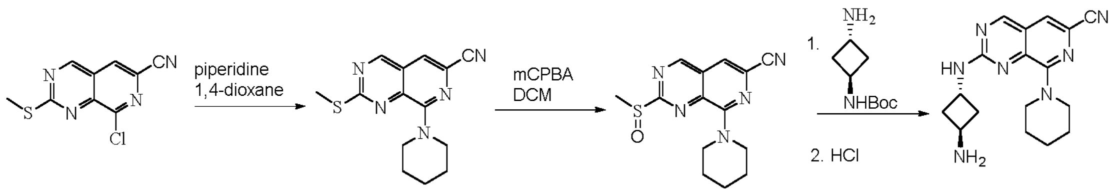


Step 1: synthesis of 2-(methylthio)-8-(piperidin-1-yl)pyrido[3,4-*d*]pyrimidine-6-carbonitrile

The titled compound was prepared as same as the step 4 of example 313 using corresponding chemicals.

Step 2: synthesis of 2-(methylsulfinyl)-8-(piperidin-1-yl)pyrido[3,4-*d*]pyrimidine-6-carbonitrile

The titled compound was prepared as same as the step 5 of example 313 using corresponding chemicals.
Exact mass: 301.10, LC-MS (Pos, *m/z):* 302.05 $[M+H]^+$.

Step 3: synthesis of 2-(((1*r*,3*r*)-3-aminocyclobutyl)amino)-8-(piperidin-1-yl)pyrido[3,4-*d*]pyrimidine-6-carbonitrile

The titled compound was prepared as same as the step 6 of example 313 using corresponding chemicals.
Molecular formula: $C_{17}H_{21}N_7$, Exact mass: 323.19, LC-MS (Pos, *m/z):* 324.2 $[M+H]^+$.

**Example 316: Synthesis of 2-(((1*r*,3*r*)-3-(aminomethyl)cyclobutyl)amino)-8-(isopropylamino)pyrido[3,4-*d*]pyrimidine-6-carbonitrile (compound C16)**

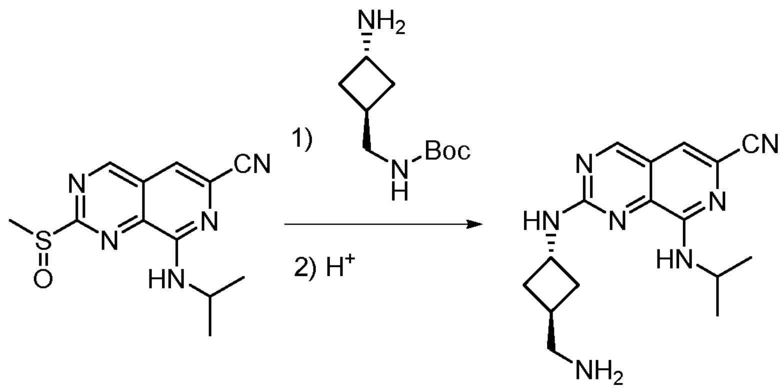


The titled compound was prepared as same as example 305 using corresponding chemicals.

Molecular formula: $C_{16}H_{21}N_7$, Exact mass: 311.19, LC-MS (Pos, *m*/*z*): 312.2 $[M+H]^+$.

**Example 317: Synthesis of 2-(((1*s*,3*s*)-3-(aminomethyl)cyclobutyl)amino)-8-(isopropylamino)pyrido[3,4-*d*]pyrimidine-6-carbonitrile (compound C17)**

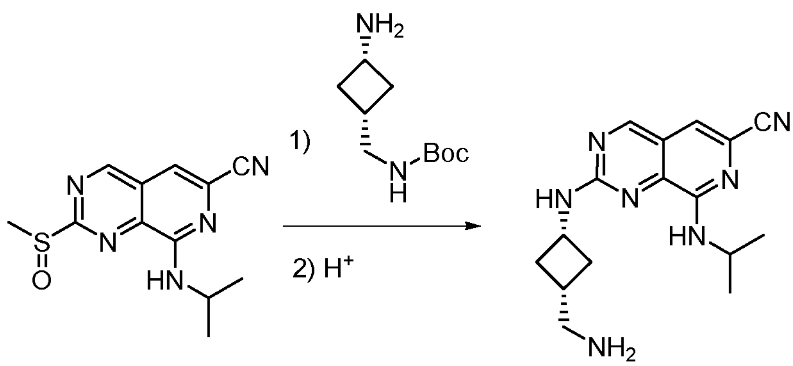


The titled compound was prepared as same as example 305 using corresponding chemicals.

Molecular formula: $C_{16}H_{21}N_7$, Exact mass: 311.19, LC-MS (Pos, *m*/*z*): 312.2 $[M+H]^+$.

**Example 318: Synthesis of 2-(((1*r*,3*r*)-3-aminocyclobutyl)amino)-8-(*tert*-butylamino)pyrido[3,4-*d*]pyrimidine-6-carbonitrile (compound C18)**

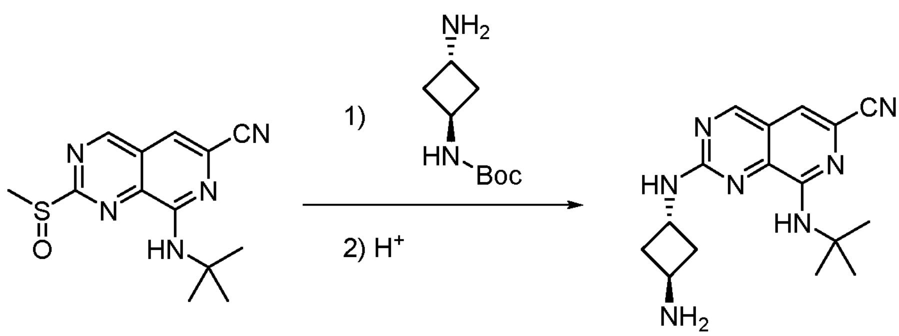


The titled compound was prepared as same as example 305 using corresponding chemicals.

Molecular formula: $C_{16}H_{21}N_7$, Exact mass: 311.19, LC-MS (Pos, *m*/*z*): 312.2 $[M+H]^+$.

**Example 319: Synthesis of 2-(((1*r*,3*r*)-3-aminocyclobutyl)amino)-8-(*tert*-butytamino)pyrido[3,4-*d*]pyrimidine-6-carboxamide (compound C19)**

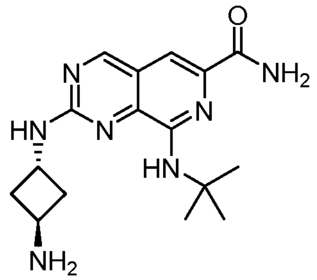

Steps:

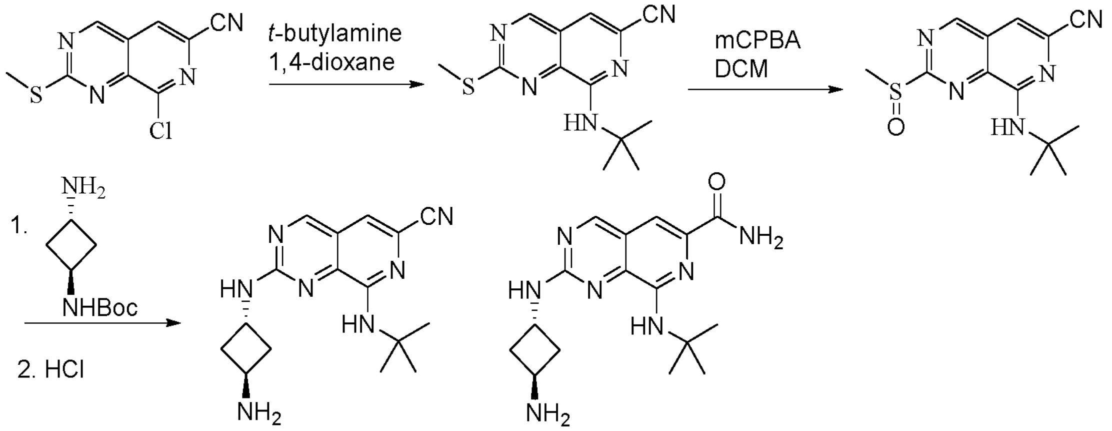


Step 1: synthesis of 8-(*tert*-butylamino)-2-(methylthio)pyrido[3,4-*d*]pyrimidine-6-carbonitrile

The titled compound was prepared as same as the step 4 of example 313 using corresponding chemicals.

Exact mass: 273.10, LC-MS (Pos, *m/z):* 274.1 $[M+H]^+$.

Step 2: synthesis of 8-(*tert*-butylamino)-2-(methylsulfinyl)pyrido[3,4-*d*]pyrimidine-6-carbonitrile

The titled compound was prepared as same as the step 5 of example 313 using corresponding chemicals.

Exact mass: 289.10, LC-MS (Pos, *m/z):* 290.1 $[M+H]^+$.

Step 3: synthesis of 2-(((1*r*,3*r*)-3-aminocyclobutyl)amino)-8-(*tert*-butylamino)pyrido[3,4-*d*]pyrimidine-6-carbonitrile (compound C18) and 2-(((1*r*,3*r*)-3-aminocyclobutyl)amino)-8-(*tert*-butylamino)pyrido[3,4-*d*]pyrimidine-6-carboxamide (compound C19)

The titled compound was prepared as same as the step 6 of example 313 using corresponding chemicals.

Molecular formula: $C_{16}H_{21}N_7$, Exact mass: 311.19, LC-MS (Pos, *m/z):* 312.2 $[M+H]^+$ (compound C18).

Molecular formula: $C_{16}H_{23}N_7O$, Exact mass: 329.20, LC-MS (Pos, *m/z):* 329.95 $[M+H]^+$ (compound C19).

**Example 320: Synthesis of 8-(isopropylamino)-2-(((1*r*,3*r*)-3-(methylamino)cyclobutyl)amino)pyrido[3,4-*d*]pyrimidine-6-carbonitrile (compound C20)**

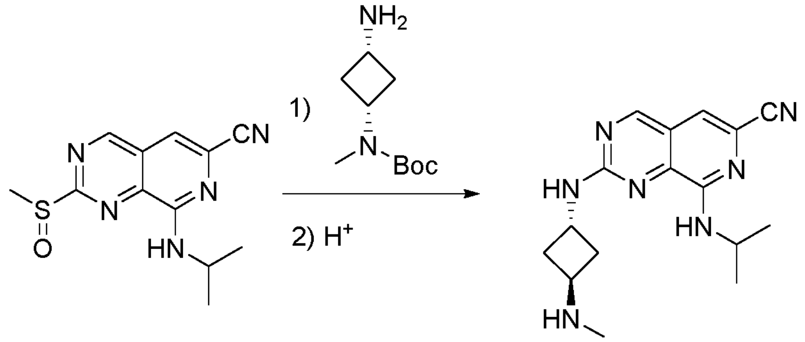


The titled compound was prepared as same as example 305 using corresponding chemicals.

Molecular formula: $C_{16}H_{21}N_7$, Exact mass: 311.19, LC-MS (Pos, *m/z):* 312.0 $[M+H]^+$.

**Example 321: Synthesis of 8-(isopropylamino)-2-(((1*r*,3*r*)-3-morpholinocyclobutyl)amino)pyrido[3,4-*d*]pyrimidine-6-carbonitrile (compound C21)**

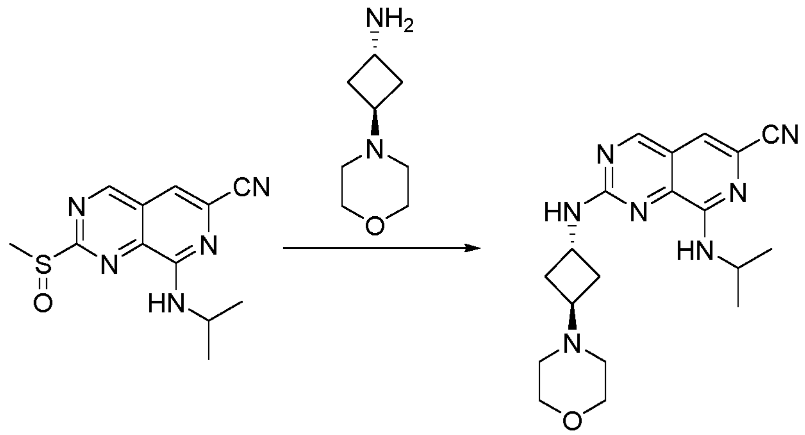

The titled compound was prepared as same as example 303 using corresponding chemicals.

Molecular formula: $C_{19}H_{25}N_7O$, Exact mass: 367.21, LC-MS (Pos, *m/z):* 368.0 $[M+H]^+$.

**Example 322: Synthesis of 8-(isopropylamino)-2-(((1*r*,3*r*)-3-(4-methylpiperazin-1-yl)cyclobutyl)amino)pyrido[3,4-*d*]pyrimidine-6-carbonitrile (compound C22)**

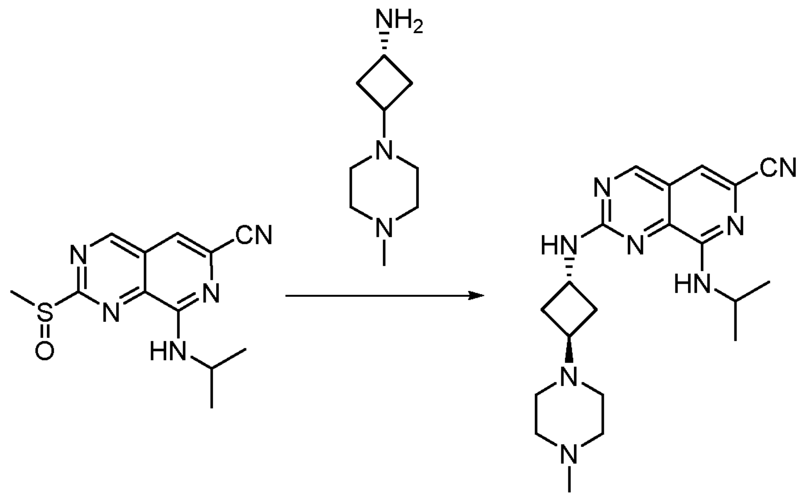

The titled compound was prepared as same as example 303 using corresponding chemicals.

Molecular formula: $C_{20}H_{28}N_8$, Exact mass: 380.24, LC-MS (Pos, *m/z*): 381.0 $[M+H]^+$.

**Example 323: Synthesis of 8-(isopropylamino)-2-(((1*s*,3*s*)-3-(4-methylpiperazin-1-yl)cyclobutyl)amino)pyrido[3,4-*d*]pyrimidine-6-carbonitrile (compound C23)**

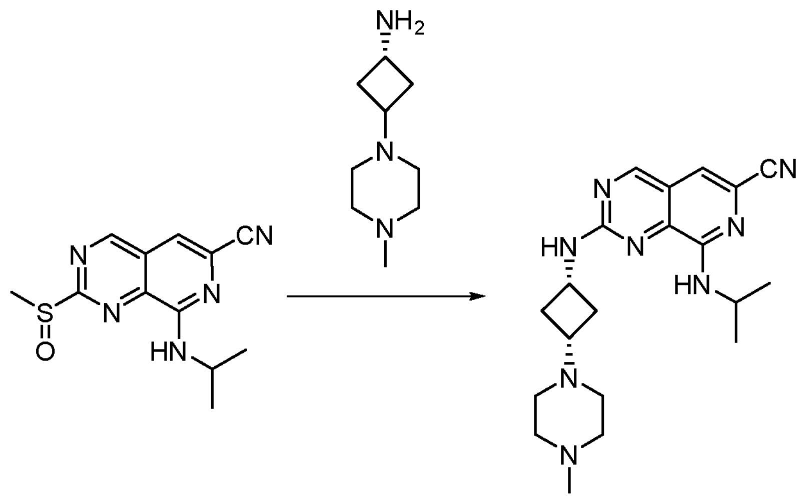

The titled compound was prepared as same as example 303 using corresponding chemicals.

Molecular formula: $C_{20}H_{28}N_8$, Exact mass: 380.24, LC-MS (Pos, *m/z*): 381.1 $[M+H]^+$.

**Example 324: Synthesis of 2-(((1*r*,3*r*)-3-(dimethylamino)cyclobutyl)amino)-8-(isopropylamino)pyrido[3,4-*d*]pyrimidine-6-carbonitrile (compound C24)**

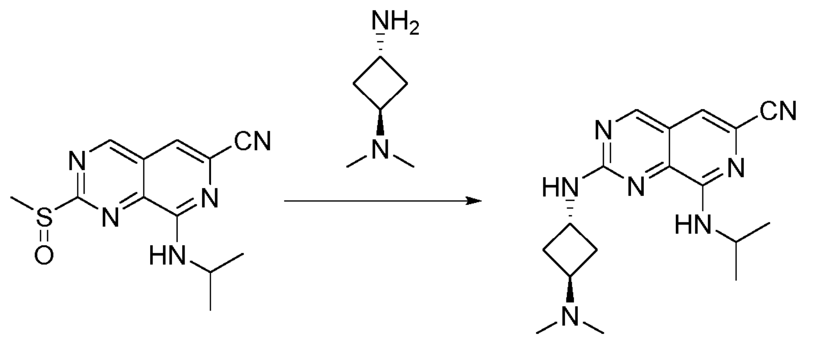

The titled compound was prepared as same as example 303 using corresponding chemicals.
Molecular formula: $C_{17}H_{23}N_7$, Exact mass: 325.20, LC-MS (Pos, *m/z):* 326.0 [M+H]+.

**Example 325: Synthesis of 2-(((1*r*,3*r*)-3-aminocyclobutyl)amino)-8-(isobutylamino)pyrido[3,4-*d*]pyrimidine-6-carbonitrile (compound C25)**

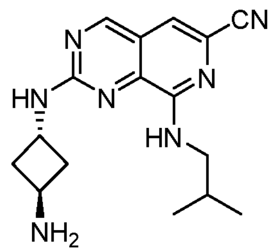

Steps:

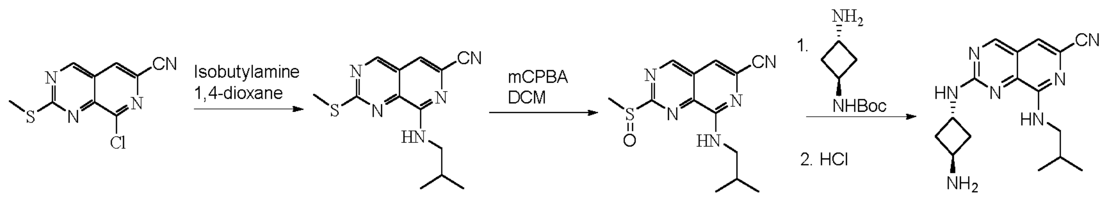


Step 1: synthesis of 8-(isobutylamino)-2-(methylthio)pyrido[3,4-*d*]pyrimidine-6-carbonitrile

The titled compound was prepared as same as the step 4 of example 313 using corresponding chemicals.
Exact mass: 273.10, LC-MS (Pos, *m/z):* 274.1 [M+H]+.

Step 2: synthesis of 8-(isobutylamino)-2-(methylsulfinyl)pyrido[3,4-*d*]pyrimidine-6-carbonitrile

The titled compound was prepared as same as the step 5 of example 313 using corresponding chemicals.
Exact mass: 289.10, LC-MS (Pos, *m/z):* 290.1 [M+H]+.

Step 3: synthesis of 2-(((1*r*,3*r*)-3-aminocyclobutyl)amino)-8-(isobutylamino)pyrido[3,4-*d*]pyrimidine-6-carbonitrile

The titled compound was prepared as same as the step 6 of example 313 using corresponding chemicals.
Molecular formula: $C_{16}H_{21}N_7$, Exact mass: 311.19, LC-MS (Pos, *m/z):* 312.07 [M+H]+.

**Example 326: Synthesis of 2-(((1*r*,3*r*)-3-amino-3-methylcyclobutyl)amino)-8-(isopropylamino)pyrido[3,4-*d*]pyrimidine-6-carbonitrile (compound C26)**

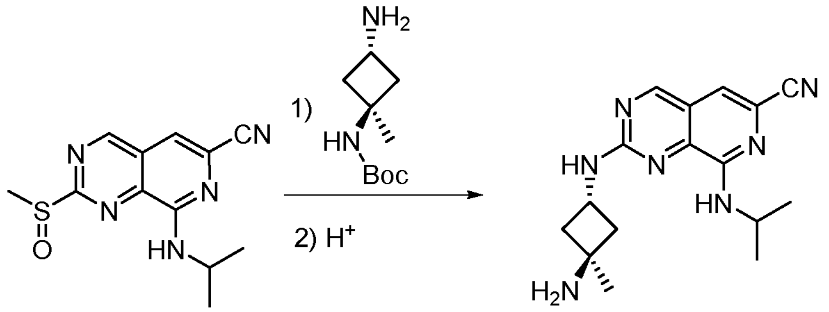


The titled compound was prepared as same as example 305 using corresponding chemicals.

Molecular formula: $C_{16}H_{21}N_7$, Exact mass: 311.19, LC-MS (Pos, *m*/*z*): 312.07 $[M+H]^+$.

**Example 327: Synthesis of 2-(((1*r*,3*r*)-3-amino-1-methylcyclobutyl)amino)-8-(isopropylamino)pyrido[3,4-*d*]pyrimidine-6-carbonitrile (compound C27)**

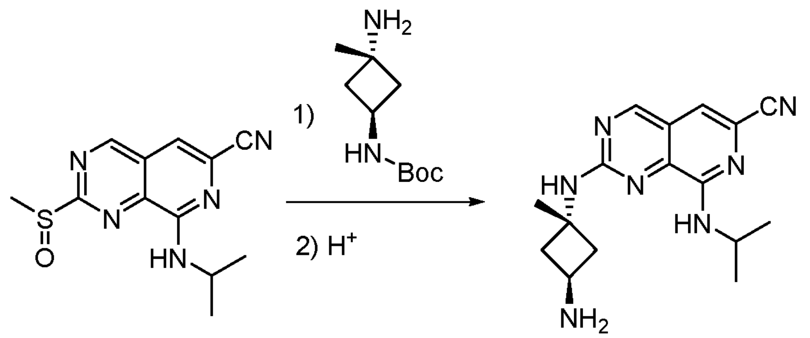


The titled compound was prepared as same as example 305 using corresponding chemicals.

Molecular formula: $C_{16}H_{21}N_7$, Exact mass: 311.19, LC-MS (Pos, *m*/*z*): 312.07 $[M+H]^+$.

**Example 328: Synthesis of 2-(((1*r*,3*r*)-3-aminocyclobutyl)amino)-8-(ethylamino)pyrido[3,4-*d*]pyrimidine-6-carbonitrile (compound C28)**

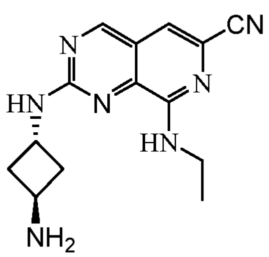

Steps:

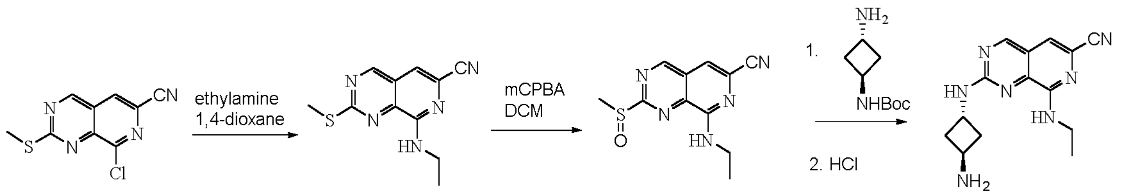


Step 1: synthesis of 8-(ethylamino)-2-(methylthio)pyrido[3,4-*d*]pyrimidine-6-carbonitrile

The titled compound was prepared as same as the step 4 of example 313 using corresponding chemicals.

Exact mass: 245.07, LC-MS (Pos, *m*/*z*): 245.95 $[M+H]^+$.

Step 2: synthesis of 8-(ethylamino)-2-(methylsulfinyl)pyrido[3,4-*d*]pyrimidine-6-carbonitrile

The titled compound was prepared as same as the step 5 of example 313 using corresponding chemicals.

Exact mass: 261.07, LC-MS (Pos, *m*/*z*): 261.95 $[M+H]^+$.

Step 3: synthesis of 2-(((1*r*,3*r*)-3-aminocyclobutyl)amino)-8-(ethylamino)pyrido[3,4-d]pyrimidine-6-carbonitrile

The titled compound was prepared as same as the step 6 of example 313 using corresponding chemicals.

Molecular formula: $C_{14}H_{17}N_7$, Exact mass: 283.15, LC-MS (Pos, *m*/*z*): 284.2 $[M+H]^+$.

**Example 329: Synthesis of 2-(((1s,3s)-3-(hydroxymethyl)cyclobutyl)amino)-8-(isopropylamino)pyrido[3,4-*d*]pyrimidine-6-carbonitrile (compound C29)**

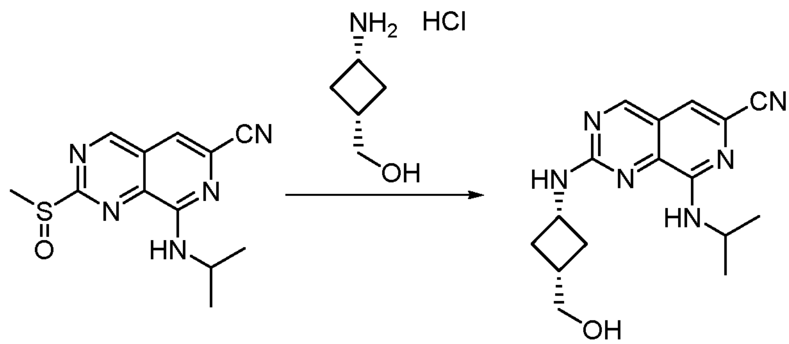

The titled compound was prepared as same as example 303 using corresponding chemicals.

Molecular formula: $C_{16}H_{20}N_6O$, Exact mass: 312.17, LC-MS (Pos, *m*/*z*): 313.2 $[M+H]^+$.

**Example 330: Synthesis of 2-(((1*r*,3*r*)-3-aminocyclobutyl)amino)-8-((tetrahydro-2H-pyran-4-yl)amino)pyrido[3,4-*d*]pyrimidine-6-carbonitrile (compound C30)**

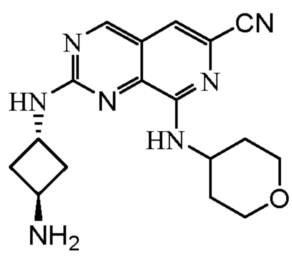

Steps:

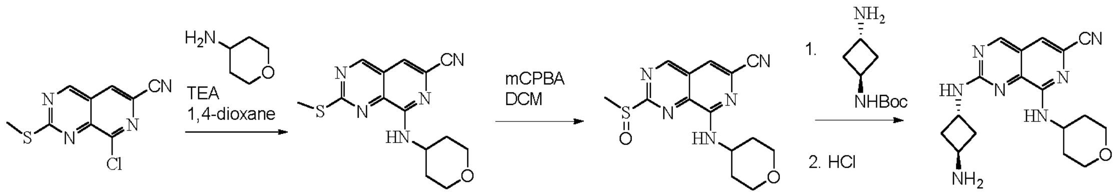


Step 1: synthesis of 2-(methylthio)-8-((tetrahydro-2*H*-pyran-4-yl)amino)pyrido[3,4-*d*]pyrimidine-6-carbonitrile

The titled compound was prepared as same as the step 4 of example 313 using corresponding chemicals.

Exact mass: 301.10, LC-MS (Pos, *m*/*z*): 302.8 $[M+H]^+$.

Step 2: synthesis of 2-(methylsulfinyl)-8-((tetrahydro-2*H*-pyran-4-yl)amino)pyrido[3,4-*d*]pyrimidine-6-carbonitrile

The titled compound was prepared as same as the step 5 of example 313 using corresponding chemicals.

Exact mass: 317.09, LC-MS (Pos, *m*/*z*): 318.1 $[M+H]^+$.

Step 3: synthesis of 2-(((1*r*,3*r*)-3-aminocyclobutyl)amino)-8-((tetrahydro-2*H*-pyran-4-yl)amino)pyrido[3,4-*d*]pyrimidine-6-carbonitrile

The titled compound was prepared as same as the step 6 of example 313 using corresponding chemicals.

Molecular formula: $C_{17}H_{21}N_7O$, Exact mass: 339.18, LC-MS (Pos, *m*/*z*): 339.95 $[M+H]^+$.

**Example 331: Synthesis of 2-(((1*r*,3*r*)-3-(hydroxymethyl)cyclobutyl)amino)-8-(isopropylamino)pyrido[3,4-*d*]pyrimidine-6-carbonitrile (compound C31)**

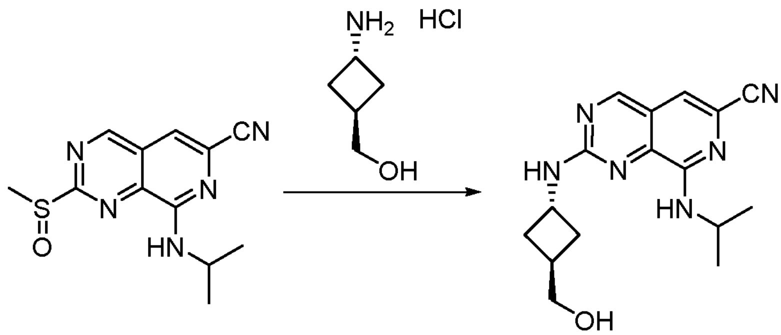


The titled compound was prepared as same as example 303 using corresponding chemicals.

Molecular formula: $C_{16}H_{20}N_6O$, Exact mass: 312.17, LC-MS (Pos, *m*/*z*): 312.95 $[M+H]^+$.

**Example 332: Synthesis of 2-(((1*r*,3*r*)-3-(4-acetylpiperazin-1-yl)cyclobutyl)amino)-8-(isopropylamino)pyrido[3,4-*d*]pyrimidine-6-carbonitrile (compound C32)**

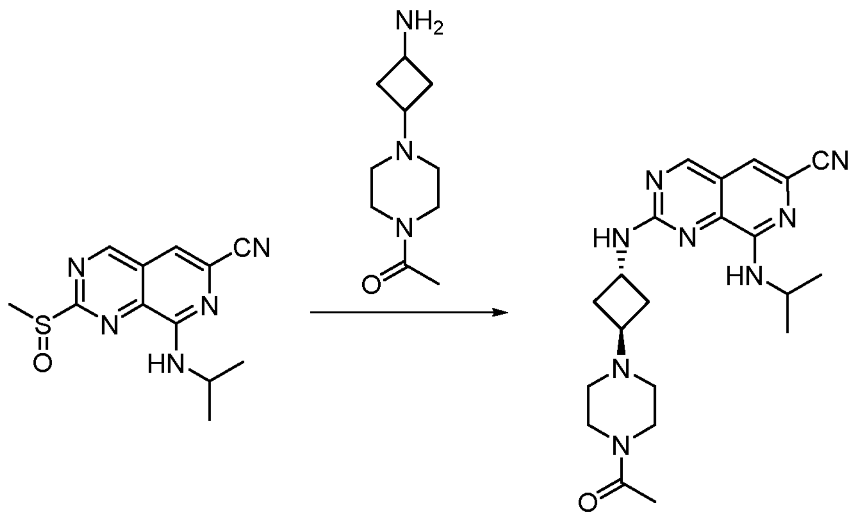

The titled compound was prepared as same as example 303 using corresponding chemicals.

Molecular formula: $C_{21}H_{28}N_8O$, Exact mass: 408.24, LC-MS (Pos, *m/z):* 409.0 $[M+H]^+$.

**Example 333: Synthesis of 2-(((1*s*,3*s*)-3-(4-acetylpiperazin-1-yl)cyclobutyl)amino)-8-(isopropylamino)pyrido[3,4-*d*]pyrimidine-6-carbonitrile (compound C33)**

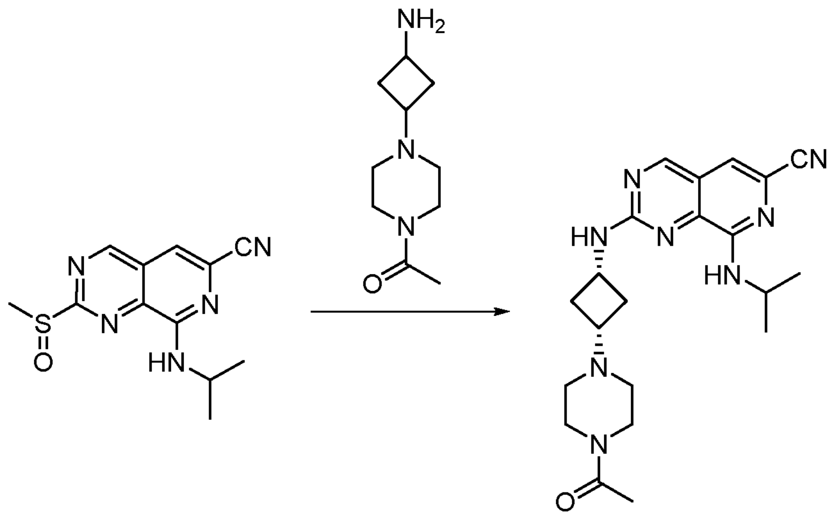

The titled compound was prepared as same as example 303 using corresponding chemicals.

Molecular formula: $C_{21}H_{28}N_8O$, Exact mass: 408.24, LC-MS (Pos, *m/z*): 409.05 $[M+H]^+$.

**Example 334: Synthesis of 2-(((1*r*,3*r*)-3-amino-3-methylcyclobutyl)amino)-8-(*tert*-butylamino)pyrido[3,4-*d*]pyrimidine-6-carbonitrile (compound C34)**

Steps:

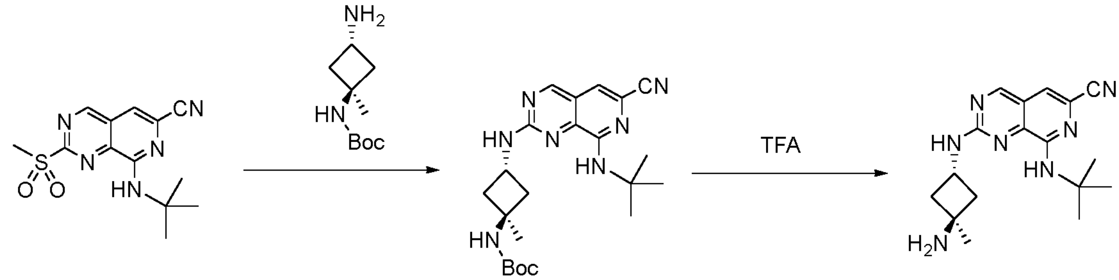


Step 1: synthesis of tert-butyl ((1r,3r)-3-((8-(tert-butylamino)-6-cyanopyrido[3,4-d]pyrimidin-2-yl)amino)-1-methylcyclobutyl)carbamate

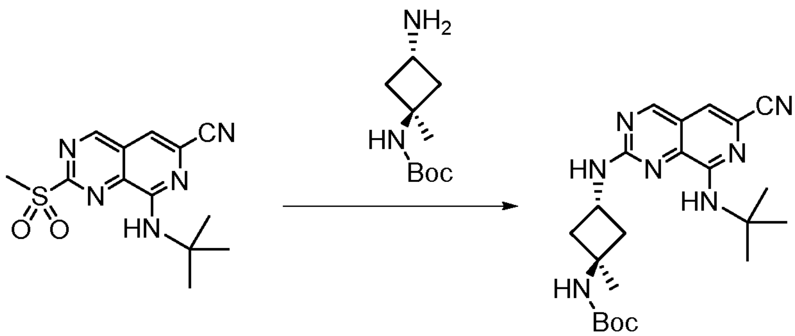

8-(tert-butylamino)-2-(methylsulfonyl)pyrido[3,4-*d*]pyrimidine-6-carbonitrile (30 mg, 0.10 mmol, 1.0 eq.) and *tert-butyl* ((1*r*,3*r*)-3-amino-1-methylcyclobutyl)carbamate (22 mg, 0.11 mmol, 1.1 eq.) were dissolved in dioxane (0.5 mL), and the reaction solution was reacted at 100 °C for 1 h. After the reaction was completed, as detected by LC-MS, the reaction solution was quenched with water and extracted with EA. The organic layer was concentrated to give a crude product, which was used in the next step without further purification.

Step 2: synthesis of 2-(((1*r*,3*r*)-3-amino-3-methylcyclobutyl)amino)-8-(*tert*-butylamino)pyrido[3,4-*d*]pyrimidine-6-carbonitrile

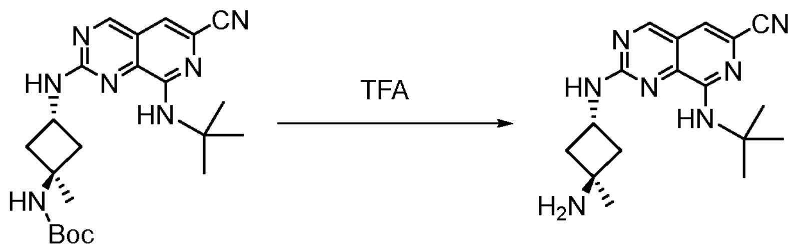


The mixture of tert-butyl ((1r,3r)-3-((8-(tert-butylamino)-6-cyanopyrido[3,4-d]pyrimidin-2-yl)amino)-1-methylcyclobutyl)carbamate (0.10 mmol) and TFA (0.2 mL) was stirred at room temperature for 2 h. After the reaction was completed, as detected by LC-MS, the reaction solution was concentrated under reduced pressure to give a crude product, which was purified by HPLC to give the product (12.6 mg, yield: 38.7%).

$^1$HNMR (400 MHz, $CDCl_3$) δ(ppm): 8.77 (s, 1H), 7.11 (s, 1H), 6.57 (br-s, 1H), 5.68 (br-s 1H), 4.50 (br-s, 1H), 2.47-2.40 (m, 2H), 2.02-1.97 (m, 2H), 1.52 (s, 9H), 1.37 (s, 3H).

Molecular formula: $C_{17}H_{23}N_7$, Exact mass: 325.20, LC-MS (Pos, *m/z):* 326.2 $[M+H]^+$.

**Example 335: Synthesis of 2-(((1*r*,3*r*)-3-aminocyclobutyl)amino)-8-((1-methylcyclopropyl)amino)pyrido[3,4-*d*]pyrimidine-6-carbonitrile (compound C35)**

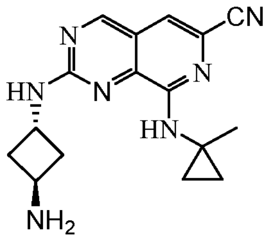

Steps:

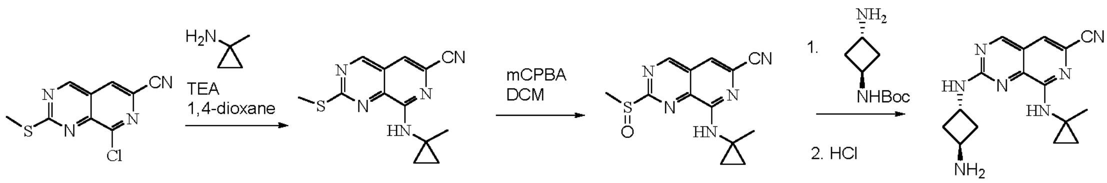


Step 1: synthesis of 8-((1-methylcyclopropyl)amino)-2-(methylthio)pyrido[3,4-*d*]pyrimidine-6-carbonitrile

The titled compound was prepared as same as the step 4 of example 313 using corresponding chemicals.

Exact mass: 271.09, LC-MS (Pos, *m*/*z*): 272.1 $[M+H]^+$.

Step 2: synthesis of 8-((1-methylcyclopropyl)amino)-2-(methylsulfinyl)pyrido[3,4-*d*]pyrimidine-6-carbonitrile

The titled compound was prepared as same as the step 5 of example 313 using corresponding chemicals.

Exact mass: 287.08, LC-MS (Pos, *m*/*z*): 288.1 $[M+H]^+$.

Step 3: synthesis of 2-(((1*r*,3*r*)-3-aminocyclobutyl)amino)-8-((1-methylcyclopropyl)amino)pyrido[3,4-*d*]pyrimidine-6-carbonitrile The titled compound was prepared as same as the step 6 of example 313 using corresponding chemicals.

Molecular formula: $C_{16}H_{19}N_7$, Exact mass: 309.17, LC-MS (Pos, *m*/*z*): 310.2 $[M+H]^+$.

**Example 336: Synthesis of 2-(((1*r*,3*r*)-3-aminocyclobutyl)amino)-8-(bicyclo[1.1.1]pentan-1-ylamino)pyrido[3,4-*d*]pyrimidine-6-carbonitrile (compound C36)**

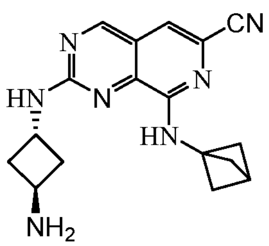

Steps:

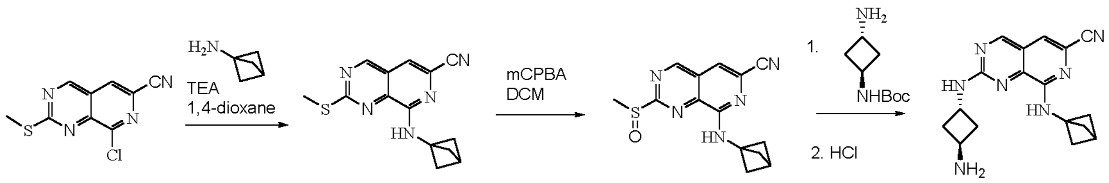


Step 1: synthesis of 8-(bicyclo[1.1.1]pentan-1-ylamino)-2-(methylthio)pyrido[3,4-*d*]pyrimidine-6-carbonitrile

The titled compound was prepared as same as the step 4 of example 313 using corresponding chemicals.

Exact mass: 283.09, LC-MS (Pos, *m/z*): 284.1 $[M+H]^+$.

Step 2: synthesis of 8-(bicyclo[1.1.1]pentan-1-ylamino)-2-(methylsulfinyl)pyrido[3,4-*d*]pyrimidine-6-carbonitrile

The titled compound was prepared as same as the step 5 of example 313 using corresponding chemicals.

Exact mass: 299.08, LC-MS (Pos, *m/z*): 300.1 $[M+H]^+$.

Step 3: synthesis of 2-(((1*r*,3*r*)-3-aminocyclobutyl)amino)-8-(bicyclo[1.1.1]pentan-1-ylamino)pyrido[3,4-*d*]pyrimidine-6-carbonitrile

The titled compound was prepared as same as the step 6 of example 313 using corresponding chemicals.

Molecular formula: $C_{17}H_{19}N_7$, Exact mass: 321.17, LC-MS (Pos, *m/z*): 322.2 $[M+H]^+$.

**Example 337: Synthesis of 2-(((4*r*,6*s*)-1-azaspiro[3.3]heptan-6-yl)amino)-8-(isopropylamino)pyrido[3,4-*d*]pyrimidine-6-carbonitrile (compound C37)**

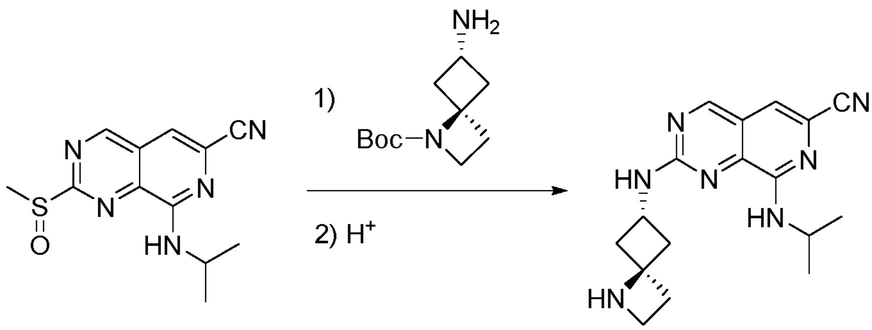


The titled compound was prepared as same as example 305 using corresponding chemicals.

Molecular formula: $C_{17}H_{21}N_7$, Exact mass: 323.19, LC-MS (Pos, *m/z*): 324.2 $[M+H]^+$.

**Example 338: Synthesis of 2-(((1*r*,3*r*)-3-amino-3-methylcyclobutyl)amino)-8-(ethylamino)pyrido[3,4-*d*]pyrimidine-6-carbonitrile (compound C38)**

Steps:

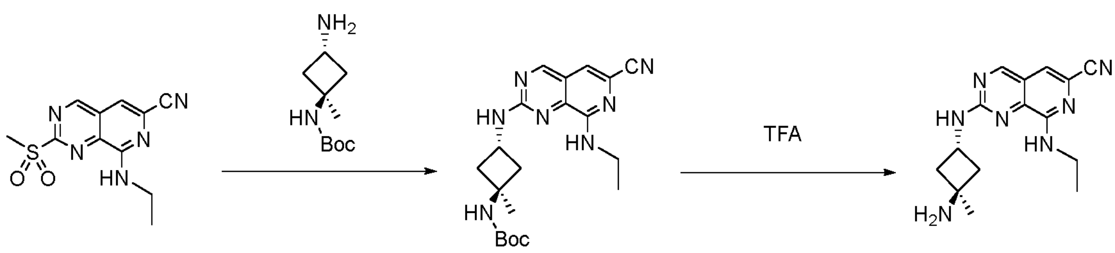

Step 1: synthesis of *tert*-butyl ((1*r*,3*r*)-3-((6-cyano-8-(ethylamino)pyrido[3,4-*d*]pyrimidin-2-yl)amino)-1-methylcyclobutyl)carbamate

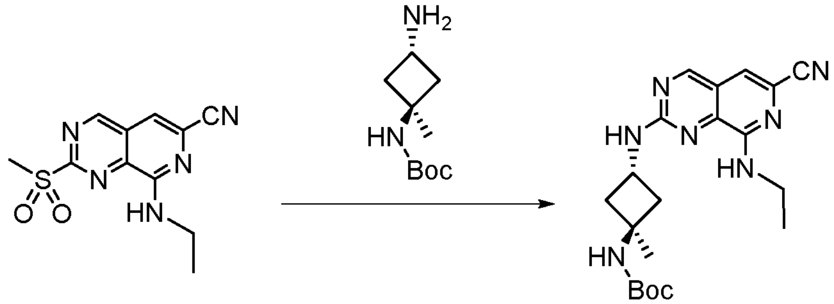

8-(ethylamino)-2-(methylsulfonyl)pyrido[3,4-*d*]pyrimidine-6-carbonitrile (27.7 mg, 0.10 mmol, 1.0 eq.) and *tert*-butyl ((1*r*,3*r*)-3-amino-1-methylcyclobutyl)carbamate (24 mg, 0.12 mmol, 1.2 eq.) were dissolved in dioxane (0.5 mL), and the reaction solution was reacted at 100 °C for 1 h. After the reaction was completed, as detected by LC-MS, the reaction solution was quenched with water and extracted with EA. The organic layer was concentrated to give a crude product, which was used in the next step without further purification.

Step 2: synthesis of 2-(((1*r*,3*r*)-3-amino-3-methylcyclobutyl)amino)-8-(ethylamino)pyrido[3,4-*d*]pyrimidine-6-carbonitrile

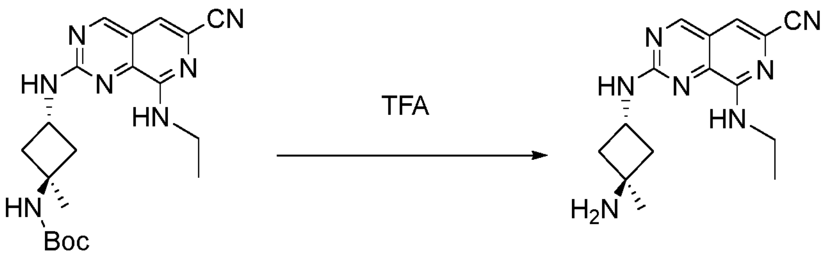


The mixture of *tert*-butyl ((1*r*,3*r*)-3-((6-cyano-8-(ethylamino)pyrido[3,4-*d*]pyrimidin-2-yl)amino)-1-methylcyclobutyl)carbamate (0.10 mmol) and TFA (0.2 mL) was stirred at room temperature for 2 h. After the reaction was completed, as detected by LC-MS, the reaction solution was concentrated under reduced pressure to give a crude product, which was purified by HPLC to give the product (25.8 mg, yield: 86.8%).
$^1$HNMR (400 MHz, $CDCl_3$) δ(ppm): 8.77 (s, 1H), 7.12 (s, 1H), 6.53 (br-s, 1H), 5.75 (br-s 1H), 4.68-4.56 (m, 1H), 3.62-3.55 (m, 2H), 2.48-2.43 (m, 2H), 1.99-1.94 (m, 2H), 1.37 (s, 3H), 1.29 (t, 3H).
Molecular formula: $C_{15}H_{19}N_7$, Exact mass: 297.17, LC-MS (Pos, *m/z*): 298.1 $[M+H]^+$.

**Example 339: Synthesis of 2-(((1*r*,3*R*)-3-aminocyclobutyl)amino)-8-(((*R*)-tetrahydro-2*H*-pyran-3-yl)amino)pyrido[3,4-*d*]pyrimidine-6-carbonitrile (compound C39)**

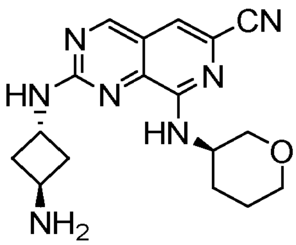

Steps:

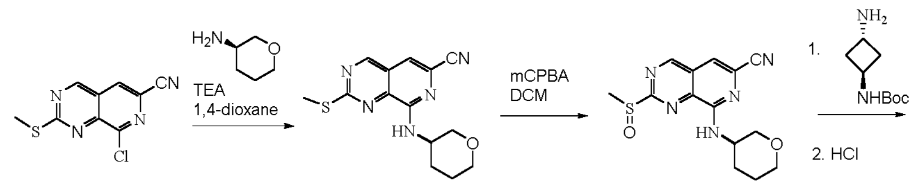


Step 1: synthesis of (*R*)-2-(methylthio)-8-((tetrahydro-2*H*-pyran-3-yl)amino)pyrido[3,4-d]pyrimidine-6-carbonitrile

The titled compound was prepared as same as the step 4 of example 313 using corresponding chemicals.
Exact mass: 301.10, LC-MS (Pos, *m*/*z*): 302.1 $[M+H]^+$.

Step 2: synthesis of 2-(methylsulfinyl)-8-(((*R*)-tetrahydro-2*H*-pyran-3-yl)amino)pyrido[3,4-*d*]pyrimidine-6-carbonitrile

The titled compound was prepared as same as the step 5 of example 313 using corresponding chemicals.
Exact mass: 317.09, LC-MS (Pos, *m*/*z*): 318.1 $[M+H]^+$.

Step 3: synthesis of 2-(((1*r*,3*R*)-3-aminocyclobutyl)amino)-8-(((*R*)-tetrahydro-2H-pyran-3-yl)amino)pyrido[3,4-*d*]pyrimidine-6-carbonitrile

The titled compound was prepared as same as the step 6 of example 313 using corresponding chemicals.
Molecular formula: $C_{17}H_{21}N_7O$, Exact mass: 339.18, LC-MS (Pos, *m*/*z*): 340.2 $[M+H]^+$.

**Example 340: Synthesis of 2-(((1*r*,4*R*)-4-(dimethylamino)cyclohexyl)amino)-8-(((*R*)-tetrahydrofuran-3-yl)amino)pyrido[3,4-*d*]pyrimidine-6-carbonitrile (compound C40)**

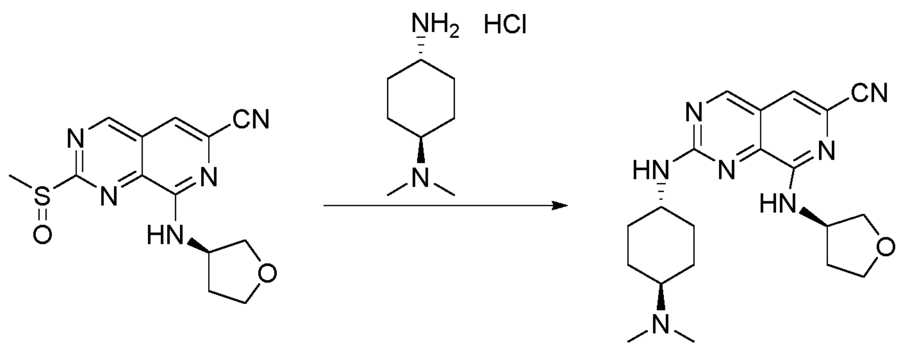

The titled compound was prepared as same as example 303 using corresponding chemicals.
Molecular formula: $C_{20}H_{27}N_7O$, Exact mass: 381.23, LC-MS (Pos, *m*/*z*): 382.3 $[M+H]^+$.

**Example 341: Synthesis of 2-(((1*r*,3*R*)-3-aminocyclobutyl)amino)-8-(((*R*)-1-methoxypropan-2-yl)amino)pyrido[3,4-*d*]pyrimidine-6-carbonitrile (compound C41)**

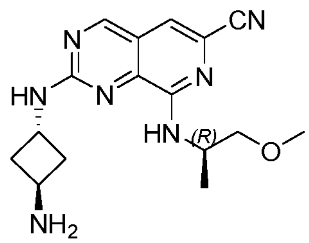

Steps:

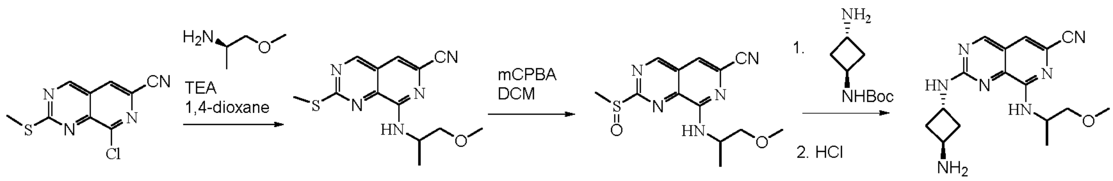


Step 1: synthesis of (*R*)-8-((1-methoxypropan-2-yl)amino)-2-(methylthio)pyrido[3,4-*d*]pyrimidine-6-carbonitrile

The titled compound was prepared as same as the step 4 of example 313 using corresponding chemicals.
Exact mass: 289.10, LC-MS (Pos, *m*/*z*): 290.1 $[M+H]^+$.

Step 2: synthesis of 8-(((*R*)-1-methoxypropan-2-yl)amino)-2-(methylsulfinyl)pyrido[3,4-*d*]pyrimidine-6-carbonitrile

The titled compound was prepared as same as the step 5 of example 313 using corresponding chemicals.
Exact mass: 305.09, LC-MS (Pos, *m*/*z*): 306.1 $[M+H]^+$.

Step 3: synthesis of 2-(((1*r*,3*R*)-3-aminocyclobutyl)amino)-8-(((*R*)-1-methoxypropan-2-yl)amino)pyrido[3,4-*d*]pyrimidine-6-carbonitrile

The titled compound was prepared as same as the step 6 of example 313 using corresponding chemicals.
Molecular formula: $C_{16}H_{21}N_7O$, Exact mass: 327.18, LC-MS (Pos, *m*/*z*): 328.2 $[M+H]^+$.

**Example 342: Synthesis of 2-(((1*r*,3*S*)-3-aminocyclobutyl)amino)-8-(((*S*)-1-methoxypropan-2-yl)amino)pyrido[3,4-*d*]pyrimidine-6-carbonitrile (compound C42)**

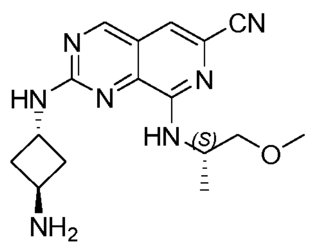

Steps:

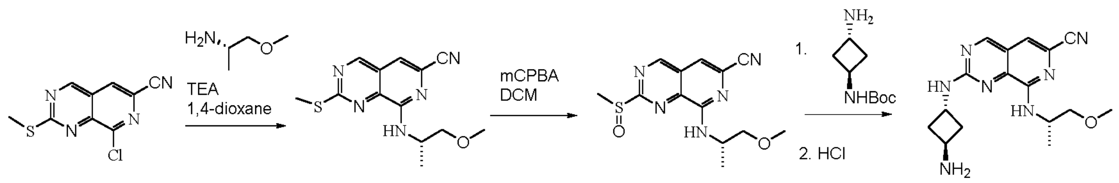


Step 1: synthesis of (*S*)-8-((1-methoxypropan-2-yl)amino)-2-(methylthio)pyrido[3,4-*d*]pyrimidine-6-carbonitrile

The titled compound was prepared as same as the step 4 of example 313 using corresponding chemicals.

Exact mass: 289.10, LC-MS (Pos, *m*/*z*): 290.1 $[M+H]^+$.

Step 2: synthesis of 8-(((*S*)-1-methoxypropan-2-yl)amino)-2-(methylsulfinyl)pyrido[3,4-*d*]pyrimidine-6-carbonitrile

The titled compound was prepared as same as the step 5 of example 313 using corresponding chemicals.

Exact mass: 305.09, LC-MS (Pos, *m*/*z*): 306.1 $[M+H]^+$.

Step 3: synthesis of 2-(((1*r*,3*S*)-3-aminocyclobutyl)amino)-8-(((*S*)-1-methoxypropan-2-yl)amino)pyrido[3,4-d]pyrimidine-6-carbonitrile

The titled compound was prepared as same as the step 6 of example 313 using corresponding chemicals.

Molecular formula: $C_{16}H_{21}N_7O$, Exact mass: 327.18, LC-MS (Pos, *m*/*z*): 328.2 $[M+H]^+$.

**Example 343: Synthesis of 8-(ethylamino)-2-(((1*r*,3*r*)-3-(methylamino)cyclobutyl)amino)pyrido[3,4-*d*]pyrimidine-6-carbonitrile (compound C43)**

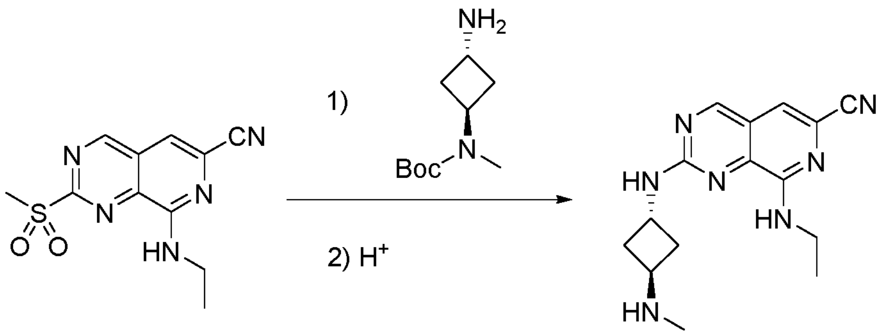


The titled compound was prepared as same as example 305 using corresponding chemicals.

Molecular formula: $C_{15}H_{19}N_7$, Exact mass: 297.17, LC-MS (Pos, *m*/*z*): 298.2 $[M+H]^+$.

**Example 344: Synthesis of (R)-8-(isopropylamino)-2-(piperidin-3-ylamino)pyrido[3,4-*d*]pyrimidine-6-carbonitrile (compound C44)**

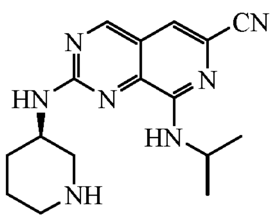

The titled compound was prepared as same as example 305 using corresponding chemicals.

Molecular formula: $C_{16}H_{21}N_7$, Exact mass: 311.19, LC-MS (Pos, *m*/*z*): 312.09$[M+H]^+$.

**Example 345: Synthesis of (5)-8-(isopropylamino)-2-(quinuclidin-3-ylamino)pyrido[3,4-*d*]pyrimidine-6-carbonitrile (compound 45)**

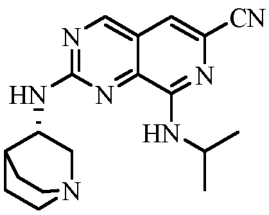

The titled compound was prepared as same as example 303 using corresponding chemicals.

Molecular formula: $C_{18}H_{23}N_7$, Exact mass: 337.20, LC-MS (Pos, *m/z*): 338.14 $[M+H]^+$.

**Example 346: Synthesis of (*S*)-*N*$^8$-isopropyl-N$^2$-(piperidin-3-yl)-6-(trifluoromethyt)pyrido[3,4-*d*]pyrimidine-2,8-diamine (compound C46)**

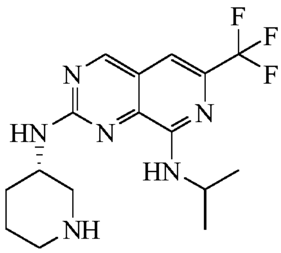

Steps:

Step 1: synthesis of 4-bromo-2-chloro-6-(trifluoromethyl)pyridin-3-amine

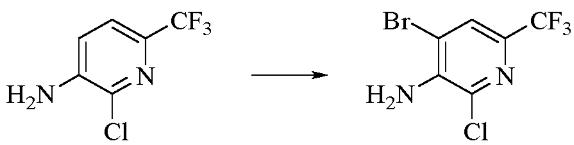

2-chloro-6-(trifluoromethyl)pyridin-3-amine (15 g, 76.31 mmol, 1.0 eq.) and *N*-bromosuccinimide (14.94 g, 83.94 mmol, 1.1 eq.) were dissolved in DMF (150 mL), and the reaction solution was reacted at 40 °C for 4h. After the reaction was completed as detected by TLC, the reaction solution was poured into water (300 mL), and the aqueous phase was extracted with MTBE (300 mL × 2) and washed with water (300 mL × 2). The organic phase was dried and concentrated, and the crude product was purified by silica gel column chromatography (PE:EA = 10: 1) to give the product (20.5 g, yield: 97.5%).

Step 2: synthesis of phenyl 3-amino-2-chloro-6-(trifluoromethyl)isonicotinate

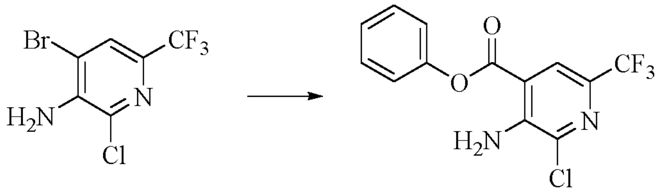

4-bromo-2-chloro-6-(trifluoromethyl)pyridin-3-amine (18 g, 65.35 mmol, 1.0 eq.), phenyl formate (8.78 g, 71.88 mmol, 2.1 eq.), palladium acetate (1.47 g, 6.54 mmol, 0.1 eq.), xanthphos (3.78 g, 6.54 mmol, 0.1 eq.) and TEA (9.92 g, 98.02 mmol, 1.5 eq.) were dissolved in toluene (400 mL), and the reaction solution was reacted at 80 °C for 21 h under nitrogen atmosphere. After the reaction was completed as detected by TLC, the reaction solution was concentrated under reduced pressure, and the crude product was purified by silica gel column chromatography (PE:EA = 20:1) to give the product (7.7 g, yield: 37.2%).

Step 3: synthesis of (3-amino-2-chloro-6-(trifluoromethyl)pyridin-4-yl)methanol

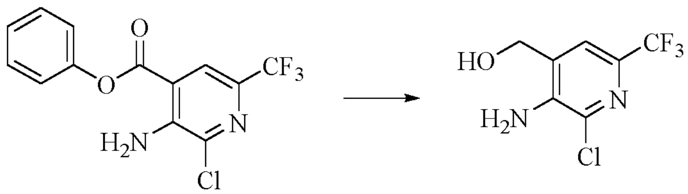

Phenyl 3-amino-2-chloro-6-(trifluoromethyl)isonicotinate (7.7 g, 24.31 mmol, 1.0 eq.) was dissolved in THF (150 mL), and the reaction solution was cooled to 5 °C, added with lithium aluminum hydride (922 mg, 24.31 mmol, 1.0 eq.) in portions and reacted for 10 min. After the reaction was completed as detected by TLC, the reaction solution was added with water (0.92 mL), 15% aqueous NaOH solution (0.92 mL), water (2.8 mL) and an appropriate amount of anhydrous sodium sulfate in sequence, stirred for 10 min, and filtered, and the filtrate was concentrated under reduced pressure to give the product (5.0 g, yield: 63%).

Step 4: synthesis of *N*-((2-chloro-4-(hydroxymethyl)-6-(trifluoromethyl)pyridin-3-yl)aminomethylthio)benzamide

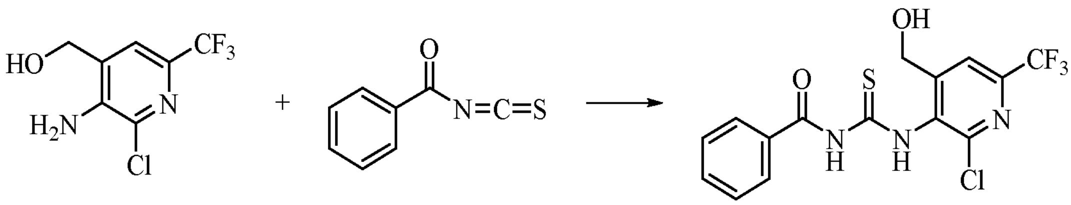

(3-amino-2-chloro-6-(trifluoromethyl)pyridin-4-yl)methanol (5.0 g, 22.06 mmol, 1.0 eq.) and benzoyl isothiocyanate (3.96 g, 24.27 mmol, 1.1 eq.) were dissolved in THF (100 mL), and the reaction solution was reacted at 50 °C for 17 h. After the reaction was completed as detected by TLC, the reaction solution was concentrated under reduced pressure, and the crude product was purified by silica gel column chromatography (PE:EA = 4:1) to give the product (8.2 g, yield: 95.3%).

Step 5: synthesis of methyl *N*-benzoyl-*N*-(2-chloro-4-(hydroxymethyl)-6-(trifluoromethyl)pyridin-3-yl)thiocarbamate

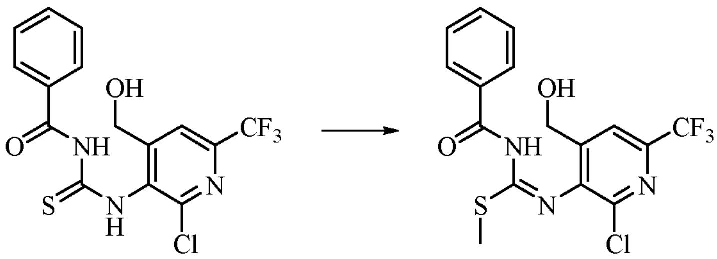

N ((2-chloro-4-(hydroxymethyl)-6-(trifluoromethyl)pyridin-3-yl)aminomethylthio)benzamide (8.2 g, 21.04 mmol, 1.0 eq.), iodomethane (3.28 g, 23.14 mmol, 1.1 eq.) and potassium carbonate (3.2 g, 23.14 mmol, 1.1 eq.) were dissolved in DMF (50 mL), and the reaction solution was reacted at room temperature for 0.5 h. After the reaction was completed as monitored by LC-MS, the reaction solution was poured into water(100 mL), and the aqueous phase was extracted with EA (100 mL × 3) and washed with water (150 mL × 2). The organic phase was dried and concentrated to give the product (8.5 g, yield: 100%).

Step 6: synthesis of methyl *N*-benzoyl-*N*-(2-chloro-4-formyl-6-(trifluoromethyl)pyridin-3-yl)thiocarbamate

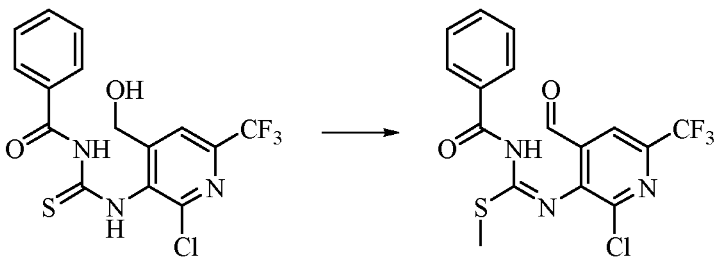

Methyl *N*-benzoyl-*N*'-(2-chloro-4-(hydroxymethyl)-6-(trifluoromethyl)pyridin-3-yl)thiocarbamate (8.5 g, 21.04 mmol, 1.0 eq.) and IBX (5.89 g, 21.04 mmol, 1.0 eq.) were dissolved in DMSO (50 mL), and the reaction solution was reacted for 20 min. After the reaction was completed as monitored by TLC, the reaction solution was poured into saturated aqueous potassium carbonate (100 mL), and extracted with MTBE (100 mL × 3). The organic phase was dried and concentrated to give the product (8.45 g, yield: 100%).

Step 7: synthesis of 8-chloro-2-(methylthio)-6-(trifluoromethyl)pyrido[3,4-*d*]pyrimidine

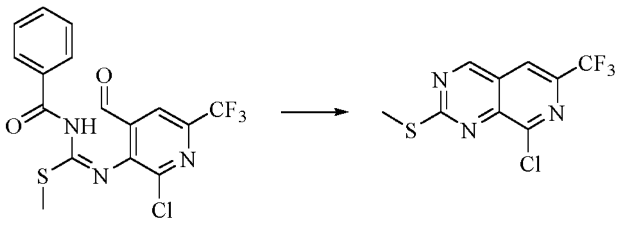

Methyl *N*-benzoyl-*N'*-(2-chloro-4-formyl-6-(trifluoromethyl)pyridin-3-yl)thiocarbamate (8.45 g, 21.04 mmol, 1.0 eq.) and potassium carbonate (3.2 g, 21.04 mmol, 1.0 eq.) were dissolved in acetonitrile (90 mL), and the reaction solution was reacted at 90 °C for 20 min. After the reaction was completed as detected by TLC, the reaction solution was concentrated under reduced pressure, and the crude product was purified by silica gel column chromatography (PE:EA = 10:1) to give the product (1.4 g, yield: 23.8 %).

Step 8: synthesis of *N*-isopropyl-2-(methylthio)-6-(trifluoromethyl)pyrido[3,4-*d*]pyrimidin-8-amine

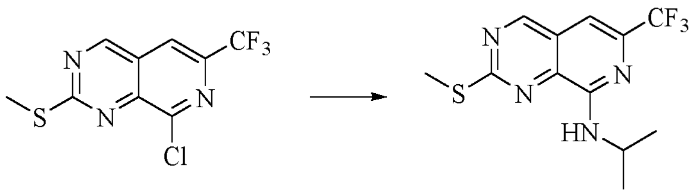

8-chloro-2-(methylthio)-6-(trifluoromethyl)pyrido[3,4-*d*]pyrimidine (1.4 g, 5.00 mmol, 1.0 eq.) and isopropylamine (1.48 g, 25.00 mmol, 5.0 eq.) were dissolved in EA (28 mL), and the reaction solution was reacted at 80 °C for 20 min. After the reaction was completed as detected by TLC, the reaction solution was concentrated under reduced pressure, and the crude product was purified by silica gel column chromatography (PE:EA = 20:1) to give the product (1.35 g, yield: 89.4%).

Step 9: synthesis of *N*-isopropyl-2-(methylsulfonyl)-6-(trifluoromethyl)pyrido[3,4-*d*]pyrimidin-8-amine

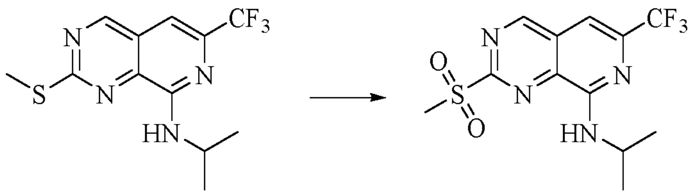

N isopropyl-2-(methylthio)-6-(trifluoromethyl)pyrido[3,4-*d*]pyrimidin-8-amine (1.3 g, 4.30 mmol, 1.0 eq.) was dissolved in DCM (26 mL), and *m*-chloroperoxybenzoic acid (1.83 g, 9.03 mmol, 2.1 eq.) was added at a mass fraction of 85%. After the reaction was completed as monitored by TLC, the reaction solution was poured into saturated aqueous potassium carbonate (50 mL), the liquid separation was performed, and the aqueous phase was extracted with DCM (20 mL). The organic phase was dried and concentrated to give the product (1.4 g, yield: 97.9%).

Step 10: synthesis of *tert*-butyl (*S*)-3-((8-(isopropylamino)-6-(trifluoromethyl)pyrido[3,4-*d*]pyrimidin-2-yl)amino)piperidine-1-carboxylate

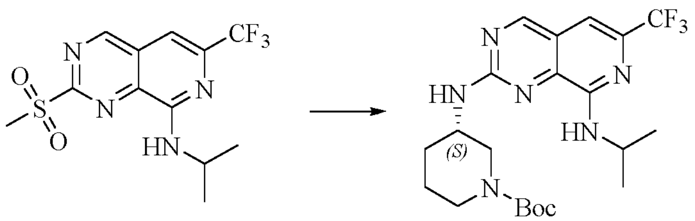

*N*-isopropyl-2-(methylsulfonyl)-6-(trifluoromethyl)pyrido[3,4-*d*]pyrimidin-8-amine (200 mg, 0.60 mmol, 1.0 eq.) and *tert*-butyl (*S*)-3-aminopiperidine-1-carboxylate (360 mg, 1.80 mmol, 3.0 eq.) were dissolved in 1,4-dioxane (4 mL), and the reaction solution was reacted at 100 °C for 23 h. After the reaction was completed as monitored by TLC, the reaction solution was concentrated, and the crude product was purified by silica gel column chromatography (PE:EA = 5:1) to give the product (260 mg, yield: 95.6%).

Step 11: synthesis of (*S*)-$N^8$-isopropyl-$N^2$-(piperidin-3-yl)-6-(trifluoromethyl)pyrido[3,4-*d*]pyrimidine-2,8-diamine

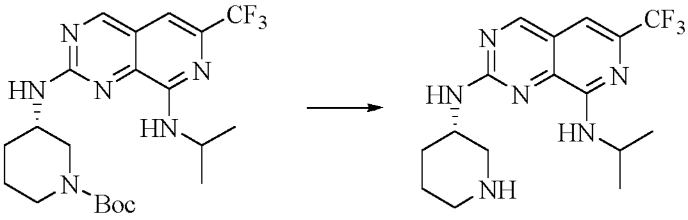

*Tert*-butyl (*S*)-3-((8-(isopropylamino)-6-(trifluoromethyl)pyrido[3,4-*d*]pyrimidin-2-yl)amino)piperidine-1-carboxylate (260 mg, 0.57 mmol, 1.0 eq.) was dissolved in EA (5 mL), and the reaction solution was added dropwise with a solution of hydrogen chloride in 1,4-dioxane (4 mol/L, 5 mL) and reacted at room temperature for 1 h. After the reaction was substantially completed as detected by LC-MS, the reaction solution was poured into saturated aqueous potassium carbonate (10 mL) and extracted with DCM (10 mL × 2). The organic phase was dried and concentrated, and the crude product was purified by silica gel column chromatography (MeOH:DCM = 1:15) to give the product (180 mg, yield: 89.1%).

$^1$HNMR (400 MHz, $CDCl_3$) δ(ppm): 8.87 (s, 1H), 7.07 (s, 1H), 6.36-6.34 (d, 1H), 5.80-5.79 (d, 1H), 4.44-4.39 (m, 1H), 4.16-4.12 (m, 1H), 3.27-3.24 (d, 1H), 2.93-2.91 (t, 1H), 2.84-2.72 (m, 2H), 1.99 (s, 1H), 1.89 (s, 1H), 1.84-1.80 (m, 1H), 1.69-1.64 (m, 2H), 1.35-1.34 (d, 6H).

Molecular formula: $C_{16}H_{21}F_3N_6$ Exact mass: 354.18 LC-MS(Pos, *m*/*z*)=355.17[M+H]$^+$

**Example 347: Synthesis of 2-(((1r,3r)-3-aminocyclobutyl)amino)-8-ethoxypyrido[3,4-d]pyrimidine-6-carbonitrile (compound C47)**

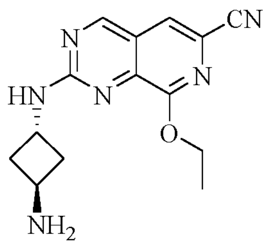

The titled compound was prepared as same as example 55 using corresponding chemicals.

$^1$HNMR (400 MHz, $CDCl_3$) δ(ppm): 9.02-8.93 (t, 1H), 7.56 (s, 1H), 6.07-5.91 (m, 1H), 4.65 (s, 3H), 3.76 (s, 1H), 2.29 (s, 4H), 1.54-1.52 (d, 3H).

Molecular formula: $C_{14}H_{16}N_6O$ Exact mass: 284.14 LC-MS(Pos, *m*/*z*)=285.02[M+H]$^+$.

**Example 348: Synthesis of 8-(isopropylamino)-2-((3-methylpiperidin-3-yl)amino)pyrido[3,4-d]pyrimidine-6-carbonitrile(compound C48)**

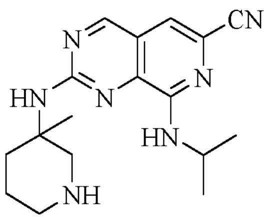

The titled compound was prepared as same as example 55 using corresponding chemicals.

$^1$HNMR (400 MHz, MeOD) δ(ppm): 8.93 (s, 1H), 7.31 (s, 1H), 4.33-4.27 (m, 1H), 3.57-3.54 (d, 1H), 2.96-2.93

(t, 1H), 2.71-2.62 (m ,2H), 2.48-2.46 (d, 1H), 1.74-1.68 (m, 1H), 1.63-1.53 (m, 2H), 1.50 (s, 3H),1.34-1.32 (d, 6H).

Molecular formula:$C_{17}H_{23}N_7$ Exact mass:325.20 LC-MS(Pos, *m/z*)=326.14[M+H]$^+$.

**Example 349: Synthesis of (S)-8-isopropoxy-2-(piperidin-3-ylamino)pyrido[3,4-d]pyrimidine-6-carbonitrile(compound C49)**

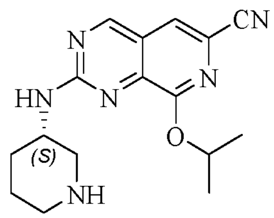

The titled compound was prepared as same as example 55 using corresponding chemicals.

$^1$HNMR(400 MHz, $CD_3OD$) δ(ppm): 9.15-9.04 (s, 1H), 7.75 (s, 1H), 5.54-5.48 (m, 1H), 4.17 (m, 1H), 3.28 (m, 1H), 2.97-2.94 (m, 1H), 2.64-2.54 (m, 2H), 2.12-2.09 (m, 1H), 1.84-1.80 (m, 1H), 1.67-1.61 (m, 2H), 1.47-1.45 (m, 6H).

Molecular formula: $C_{16}H_{20}N_6O$ Exact mass:312.17 LC-MS (Pos, *m/z)* =313.25 [M+H]$^+$.

The present invention can be better understood according to the following examples. However, it is easily understood by those skilled in the art that the content described in the examples are only used to illustrate the present invention, and should not and will not limit the present invention described in detail in the claims.

**Experimental Example 1: Assay on CDK Enzymatic Activity of Compounds of the Present Invention**

Test samples: the compounds of the present invention, having structures shown above.

Reagent and consumable:

ADP-Glo Kinase Assay(ADP-Glo kinase kit), DMSO (dimethyl sulfoxide), CDK9/CycT1 (cyclin-dependent kinase 9/cyclin T1), CDK tide(cyclin-dependent kinase substrate peptide fragments), CDK1/CycE1 (cyclin-dependent kinase 1/cyclin E1), CDK2/CycA2 (cyclin-dependent kinase 2/cyclin A2), CDK3/CycE1 (cyclin-dependent kinase 3/cyclin E1), CDK4/CycD3 (cyclin-dependent kinase 4/cyclin D3), CDK5/p25NCK (cyclin-dependent kinase 5/p25), and AZD 4573.

A 384-well white plate, a 96-well plate, a microplate oscillator, a centrifuge, and a microplate reader.

Test method:

1. Preparation of the compounds

The compounds were dissolved in DMSO and diluted 4-fold in the 96-well plate to obtain stock solutions with 10 concentration gradients, and added into a 384-well plate system to obtain a series of 10 concentration gradients of compound solutions with final concentrations of 10 μM starting and 4-fold gradient decreasing. 1 μM AZD4573 was used as compound positive control (PC) and 0.5% DMSO as solvent vehicle control (VC), ten duplicated wells each for PC and VC.

2. Experimental procedures

1 μL of compound solution and 2 μL of 2× kinase solution were transferred to each well, and the plate was centrifuged at 1000 g for 30 s and left at room temperature for 10 min. A mixture of 2× substrate and ATP was prepared in 1× kinase buffer. The reaction was started after adding 2 μL of substrate and ATP into the plate. The plate was centrifuged at 1000 g for 30 s. The assay plate was closed and left at room temperature for 60 min. The reaction system was added with 4 μL of ADP Glo reagent, centrifuged at 1000 rpm for 1 min, and incubated at room temperature for 40 min. The reaction system was added with 8 μL of kinase assay reagent, centrifuged at 1000 rpm for 1 min and incubated at room temperature for 40 min. The fluorescence was read on Biotek.

The inhibition rate was calculated according to the following formula:

$$\%\cdot Inhibition = \left[1 - \frac{Lum_{cmpd} - \overline{Lum}_{positive}}{\overline{Lum}_{vehicle} - \overline{Lum}_{positive}}\right] * 100$$

$Lum_{cmpd}$: compound fluorescence reading; $\overline{Lum}_{positive}$: average value of the fluorescence reading of positive compound control; $\overline{Lum}_{vehicle}$: average value of the fluorescence reading of solvent vehicle control.

$IC_{50}$ values were fitted using GraphPad 7.0 based on inhibition rates of compounds at different concentrations.

The test results are shown in Table 3-a, 3-b and 3-c.

Table 3-a: Inhibitory activities of compounds of the present invention on CDK kinases ($IC_{50}$)

| Compound | CDK9/Cy cT1/nM | CDK 1/Cy cE1/nM | CDK2/Cy cA2/nM | CDK3/Cy cE1/nM | CDK5/p25 NCK/nM |
|---|---|---|---|---|---|
| Compound 2 | 8 | / | 669 | 179 | 783 |
| Compound 7 | 44 | | | | |
| Compound 10 | 46 | | | | |
| Compound 13 | 13 | / | 402 | 41 | 527 |
| Compound 14 | 11 | / | 284 | 91 | 187 |
| Compound 15 | 1 | / | 182 | 273 | 770 |
| Compound 16 | 20 | / | 1456 | 951 | 2502 |
| Compound 17 | 4 | / | 279 | 750 | 664 |
| Compound 18 | 15 | / | 3374 | >10000 | >10000 |
| Compound 19 | 26 | / | 2367 | 1473 | 6079 |
| Compound 20 | 11 | / | 2154 | 1753 | 4317 |
| Compound 22 | 17 | / | 4512 | 1911 | >10000 |
| Compound 23 | 38 | / | 3871 | 5648 | >10000 |
| Compound 24 | 26 | / | 3335 | 862 | 5344 |
| Compound 25 | 12 | / | 521 | 602 | 1210 |
| Compound 27 | 51 | / | 2520 | 1633 | 3964 |
| Compound 28 | 3 | 220 | 50 | 97 | 96 |
| Compound 29 | 4 | / | 514 | 1207 | 461 |
| Compound 30 | 2 | / | 15 | 95 | 31 |
| Compound 31 | 4 | / | 594 | 1297 | 1481 |
| Compound 32 | 7 | / | 1079 | 2046 | 1580 |
| Compound 33 | 1 | / | 3 | 9 | 3 |
| Compound 34 | 2 | / | 128 | 137 | 238 |
| Compound 35 | 1 | / | 14 | 35 | 36 |
| Compound 36 | 1 | / | 4 | 23 | 7 |
| Compound 37 | 5 | / | 520 | 1130 | 941 |
| Compound 38 | 2 | 83 | 110 | 66 | 103 |
| Compound 39 | 2 | / | 84 | 278 | 161 |
| Compound 41 | 6 | / | / | 90 | / |
| Compound 42 | 18 | / | 2023 | 1296 | 3041 |
| Compound 43 | 2 | / | 13 | 75 | 23 |

(continued)

| Compound | CDK9/CycT1/nM | CDK 1/CycE1/nM | CDK2/CycA2/nM | CDK3/CycE1/nM | CDK5/p25 NCK/nM |
|---|---|---|---|---|---|
| Compound 44 | 2 | / | 1171 | 288 | 3635 |
| Compound 45 | 2 | 114 | 47 | 85 | 153 |
| Compound 46 | 3 | / | 33 | 271 | 72 |
| Compound 47 | 17 | / | 494 | 987 | 1047 |
| Compound 48 | 2 | / | 199 | 477 | 266 |
| Compound 49 | 2 | / | 57 | 105 | 93 |
| Compound 50 | 2 | / | 73 | 208 | 119 |
| Compound 51 | 1 | / | 73 | 88 | 142 |
| Compound 52 | 1 | / | 12 | 23 | 16 |
| Compound 53 | 2 | 709 | 161 | 315 | 549 |
| Compound 54 | 1 | / | 15 | 14 | 10 |
| Compound 55 | 1 | 43 | 60 | 24 | 56 |
| Compound 56 | 3 | / | 656 | 1112 | 927 |
| Compound 57 | 30 | / | 427 | 2517 | 1762 |
| Compound 60 | 29 | / | 1809 | 12090 | 8391 |
| Compound 61 | 3 | 173 | 227 | 124 | 484 |
| Compound 103 | 3 | 206 | 167 | 398 | 294 |
| Compound 104 | 10 | / | 888 | 550 | 2202 |
| Compound 106 | 4 | 26 | / | 29 | / |
| Compound 107 | 18 | 947 | 443 | 831 | 435 |
| Compound 111 | 69 | | | | |
| Compound 112 | 17 | | | | |
| Compound 113 | 9 | | | | |
| Compound 114 | 9 | 216 | 504 | 413 | 1171 |
| Compound 115 | 6 | | | | |
| Compound 116 | 2 | 114 | 29 | 92 | 63 |
| Compound 117 | 6 | | | | |
| Compound 118 | 12 | | | | |

(continued)

| Compound | CDK9/CycT1/nM | CDK 1/CycE1/nM | CDK2/CycA2/nM | CDK3/CycE1/nM | CDK5/p25NCK/nM |
|---|---|---|---|---|---|
| Compound 119 | 6 | | | | |
| Compound 120 | 32 | | | | |
| Compound 121 | 14 | | | | |
| Compound 122 | 1 | 3 | 6 | 8 | 17 |
| Compound 124 | 26 | | | | |
| Compound 125 | 9 | | | | |
| Compound 126 | 2 | 95 | 181 | 287 | 238 |
| Compound 127 | 1 | 10 | 14 | 8 | 46 |
| Compound 128 | 2 | 43 | 54 | 34 | 65 |
| Compound 129 | 7 | | | | |
| Compound 130 | 2 | 25 | 74 | 32 | 447 |
| Compound 131 | 6 | 678 | 388 | 629 | 1534 |
| Compound 132 | 12 | | | | |
| Compound 133 | 2 | 29 | 10 | | |
| Compound 134 | 1 | 11 | 5 | | |
| Compound 136 | 2 | 315 | 13 | 27 | 116 |
| Compound 137 | 10 | | | | |
| Compound 138 | 3 | 166 | 49 | | |
| Compound 139 | 6 | | | | |
| Compound 141 | 2 | 197 | 23 | | |

Table 3-b: Inhibitory activities of compounds of the present invention on CDK kinases ($IC_{50}$)

| Compound | CDK9/Cy cT1/nM | CDK1/Cy cE1/nM | CDK2/Cy cA2/nM | CDK3/Cy cE1/nM | CDK5/p25 NCK/nM |
|---|---|---|---|---|---|
| Compound 142 | 2 | 90 | 600 | 66 | 306 |
| Compound 143 | 12 | / | / | / | / |
| Compound 144 | 2 | 90 | 146 | 28 | 195 |
| Compound 146 | 9 | / | / | / | / |
| Compound 147 | 3 | 158 | 531 | 215 | 2232 |
| Compound 148 | 1 | 104 | 171 | 49 | 165 |
| Compound 149 | 1 | 42 | 126 | 60 | 295 |
| Compound 150 | 2 | 608 | 450 | 281 | 1460 |
| Compound 151 | 8 | 411 | 1291 | 832 | 4143 |
| Compound 152 | 2 | 110 | 267 | 124 | 1001 |
| Compound 153 | 2 | 133 | 562 | 67 | 706 |
| Compound 154 | 5 | 1016 | 2319 | 371 | 3842 |
| Compound 155 | 33 | / | / | / | / |
| Compound 156 | 14 | / | / | / | / |
| Compound 158 | 3 | / | / | / | / |
| Compound 159 | 22 | / | / | / | / |
| Compound 160 | 26 | / | / | / | / |
| Compound 161 | 14 | / | / | / | / |
| Compound 163 | 2 | 31 | 7 | / | / |
| Compound 164 | 1 | 138 | 135 | / | / |
| Compound 165 | 47 | / | / | / | / |
| Compound 166 | 2 | 14 | 10 | / | / |
| Compound 167 | 2 | 55 | 19 | / | / |
| Compound 168 | 1 | 63 | 125 | / | / |
| Compound 169 | 5 | 798 | 2207 | / | / |
| Compound 170 | 4 | 144 | 34 | / | / |
| Compound 171 | 4 | 638 | 815 | / | / |
| Compound 172 | 1 | 395 | 287 | / | / |
| Compound 173 | 4 | 952 | 436 | / | / |
| Compound 174 | 2 | 871 | 510 | / | / |
| Compound 175 | 3 | 234 | 489 | 1476 | 435 |
| Compound 176 | 3 | / | / | / | / |
| Compound 177 | 19 | / | / | / | / |
| Compound 178 | 7 | / | / | / | / |
| Compound 179 | 1 | 272 | 144 | / | / |
| Compound 180 | 7 | 204 | 844 | / | / |
| Compound 181 | 7 | 518 | 153 | / | / |
| Compound 182 | 15 | | | | |

(continued)

| Compound | CDK9/Cy cT1/nM | CDK1/Cy cE1/nM | CDK2/Cy cA2/nM | CDK3/Cy cE1/nM | CDK5/p25 NCK/nM |
|---|---|---|---|---|---|
| Compound 183 | 1 | 50 | 28 | | |
| Compound 184 | 1 | 163 | 125 | 990 | 96 |
| Compound 185 | 3 | 134 | 356 | | |
| Compound 186 | 3 | 336 | 352 | 915 | 176 |
| Compound 187 | 8 | 413 | 958 | | |
| Compound 188 | 10 | | | | |
| Compound 191 | 5 | 1948 | 1108 | | |
| Compound 192 | 11 | | | | |
| Compound 193 | 5 | 398 | 679 | | |
| Compound 194 | 15 | | | | |
| Compound 195 | 2 | 14 | 540 | | |
| Compound 197 | 14 | | | | |
| Compound 198 | 12 | | | | |
| Compound 199 | 4 | | | | |
| Compound 200 | 2 | 149 | 244 | 1101 | 263 |
| Compound 201 | 2 | 215 | 83 | 189 | 53 |
| Compound 202 | 4 | 521 | 337 | 398 | 168 |
| Compound 203 | 1 | | | | |
| Compound 204 | 2 | 903 | 1014 | 1412 | 523 |
| Compound 206 | 1 | 25 | 6 | | |
| Compound 207 | 1 | 126 | 29 | | |
| Compound 208 | 7 | | | | |
| Compound 209 | 2 | 170 | 306 | | |
| Compound 210 | 12 | | | | |
| Compound 211 | 3 | 233 | 434 | | |

Table 3-c: Inhibitory activities of compounds of the present invention on CDK kinases ($IC_{50}$)

| Compounds | CDK9/Cy cT1/nM | CDK1/Cy cE1/nM | CDK2/Cy cA2/nM | CDK3/Cy cE1/nM | CDK5/p25 NCK/nM |
|---|---|---|---|---|---|
| Compound C1 | 2 | 7 | 21 | 7 | 17 |
| Compound C2 | 4 | 48 | 48 | 53 | 465 |
| Compound C3 | 27 | | | | |
| Compound C4 | 9 | | | | |
| Compound C5 | 50 | | | | |
| Compound C6 | 4 | 202 | 330 | | |
| Compound C7 | 3 | 74 | 144 | | |
| Compound C8 | 2 | 8 | 5 | | |
| Compound C9 | 1 | 8 | 8 | | |

(continued)

| Compounds | CDK9/Cy cT1/nM | CDK1/Cy cE1/nM | CDK2/Cy cA2/nM | CDK3/Cy cE1/nM | CDK5/p25 NCK/nM |
|---|---|---|---|---|---|
| Compound C10 | 8 | | | | |
| Compound C11 | 1 | | | | |
| Compound C12 | 2 | | | | |
| Compound C13 | 2 | 187 | 85 | | |
| Compound C14 | 2 | 127 | 32 | | |
| Compound C15 | 2 | 114 | 253 | | |
| Compound C16 | 2 | 81 | 22 | | |
| Compound C17 | 2 | | | | |
| Compound C18 | 2 | 23 | 8 | | |
| Compound C19 | 10 | | | | |
| Compound C20 | 1 | 71 | 18 | | |
| Compound C21 | 4 | | | | |
| Compound C22 | 2 | 218 | 24 | | |
| Compound C23 | 6 | 288 | 59 | | |
| Compound C24 | 2 | 142 | 115 | | |
| Compound C25 | 1 | 86 | 34 | | |
| Compound C26 | 1 | 34 | 93 | | |
| Compound C27 | 19 | | | | |
| Compound C28 | 1 | 186 | 34 | | |
| Compound C29 | 3 | 51 | 35 | | |
| Compound C30 | 2 | 99 | 83 | | |
| Compound C31 | 2 | 21 | 5 | | |
| Compound C32 | 4 | | | | |
| Compound C33 | 19 | | | | |
| Compound C34 | 1 | | | | |
| Compound C35 | 1 | 112 | 19 | | |
| Compound C36 | 2 | 25 | 32 | | |
| Compound C37 | 2 | | | | |
| Compound C38 | 1 | | | | |
| Compound C39 | 1 | | | | |
| Compound C40 | 5 | | | | |
| Compound C41 | 1 | 540 | 82 | | |
| Compound C42 | 2 | | | | |
| Compound C43 | 1 | | | | |
| Compound C44 | 20 | | | | |
| Compound C45 | 6 | 467 | 1311 | 340 | 7980 |
| Compound C46 | 2 | 19 | 33 | 8 | 60 |
| Compound C47 | 24 | | | | |

(continued)

| Compounds | CDK9/Cy cT1/nM | CDK1/Cy cE1/nM | CDK2/Cy cA2/nM | CDK3/Cy cE1/nM | CDK5/p25 NCK/nM |
|---|---|---|---|---|---|
| Compound C49 | 20 | | | | |

It can be seen from the results in Table 3-a, 3-b and 3-c that the compounds of the present invention show obvious inhibition effect on CDK9, and compared with CDK1, 2, 3 and 5, the compounds of the present invention have better selectivity for CDK9, which indicates that the compounds of the present invention has better clinical application potential in treating CDK9-mediated diseases, which can reduce the side effects caused by drug off-target.

**Experimental Example 2: Assay on CDK Enzymatic Activity of Compounds of the Present Invention**

1. Preparation for test compounds

Prepare 100x concentrated Test compound stock solutions with DMSO. Then, Dilute the solution 25 times with Assay Buffer(20 mM HEPES, 0.01% Triton X-100, 2 mM DTT, pH7.5) to prepare 4x test compound solutions.

2. Kinase

Serine/Threonine kinase

| Kinase | Description |
|---|---|
| CDK9/CycT1 | Full-length human CDK9 [1-372(end) amino acids of accession number NP_001252.1] was co-expressed as N-terminal GST-fusion protein (70 kDa) with His-CyclinT1 [1-726(end) amino acids of accession number NP_001231.2] using baculovirus expression system GST-CDK9 was purified by using glutathione sepharose chromatography. |

3. Reagents and methods

Off-chip Mobility Shift Assay (MSA)

Test compounds were evaluated at 4-point concentration(0.0016, 0.008, 0.04, 0.2 uM).

1) Mix 5 uL of 4x test compound solution with 5 uL of 4x substrate/ATP/Metal solution and 10 uL of 2x kinase solution in 384-well polypropylene plate. Incubate for 5 hours at room temperature.
2) Add 70 uL of Termination Buffer (QuickScout Screening Assist MSA; Carna Biosciences) to stop the reaction.
3) Separate and detect unphosphorylated/phosphorylated substrate peptides in the reactant using LabChip system (Perkin Elmer).
4) Evaluate kinase reaction from product ratio(P/(P+S)) calculated by peak height of unphosphorylated substrate(S) and peak height of phosphorylated substrate(P).

4. Reaction condition

| Kinase | Platform | Substrate | | ATP ($\mu$M) | | Metal | | Positive control |
|---|---|---|---|---|---|---|---|---|
| | | Name | (nM) | Km | Assay | Name | (mM) | |
| CDK9/CycT1 1) | MSA | CDK9 substrate | 1000 | 9.4 | 10 | Mg | 5 | Staurosporine |
| 1) Reaction time is 5 hours. | | | | | | | | |

The test results are shown in Table 4.

Table 4: Inhibitory activities of compounds of the present invention on CDK kinases

| Compound | CDK9/CycT1/nM |
|---|---|
| Compound 1 | 7 |
| Compound 63 | 14 |
| Compound 64 | 26 |
| Compound 65 | 21 |
| Compound 66 | 5 |
| Compound 67 | 10 |
| Compound 68 | 82 |
| Compound 69 | 81 |
| Compound 70 | 61 |
| Compound 72 | 97 |
| Compound 73 | 61 |
| Compound 74 | 19 |
| Compound 75 | 36 |
| Compound 76 | 10 |
| Compound 77 | 85 |
| Compound 79 | 23 |
| Compound 80 | 12 |
| Compound 82 | 63 |
| Compound 84 | 47 |
| Compound 85 | 64 |
| Compound 86 | 50 |
| Compound 88 | 44 |
| Compound 89 | 24 |
| Compound 91 | 6 |
| Compound 92 | 64 |
| Compound 93 | 7 |
| Compound 94 | 4 |
| Compound 95 | 34 |
| Compound 97 | 17 |
| Compound 98 | 28 |
| Compound 99 | 29 |
| Compound 100 | 5 |
| Compound 101 | 15 |
| Compound 102 | 10 |

It can be seen from the results in Table 4 that the compounds of the present invention show obvious inhibition effect on CDK9, which indicates that the compounds of the present invention has better clinical application potential in treating CDK9-mediated diseases.

**Experimental Example 3: Assay on Cell Activity of Compounds of the Present Invention**

Test samples: the compounds of the present invention, having structures shown above.

Reagent and consumable:

RMPI 1640, Fetal Bovine Serum (FBS), a CCK-8 method cell proliferation assay kit, DMSO (dimethyl sulfoxide), a 96-well cell culture plate, an Iscov'Modified Dulbecco's culture medium (IMDM), a multifunctional microplate reader, MV-4-11 (human myelomonocytic leukemia cells) and NCI-H929 (human myeloma cells).

Test method:

The compounds were dissolved in DMSO and diluted 3-fold in the 96-well plate to obtain stock solutions with 11 concentration gradients. Each cell was inoculated into a 96-well plate, and the diluted compound stock solution was added to the 96-well plate to obtain a series of 11 concentration gradients of compound solutions with final concentrations of 5000 nM starting and 3-fold gradient decreasing, wherein the final DMSO content was 1‰. There was a medium containing 1‰ DMSO in the negative control well. The treated cells were incubated at 37 °C with 5% $CO_2$ and 95% humidity, wherein MV-4-11 were incubated for 24 h and NCI-H929 for 72 h. After incubation was completed, each well was added with 10 $\mu$L of CCK-8 reagent, incubated for 1-3 h at room temperature and placed in the microplate reader, and the absorbance was read at 450 nm.

The inhibition rate was calculated according to the following formula:

$$\%Inhibition = \left[1 - \frac{ODcmpd - \overline{OD}\ vehicle}{\overline{OD}\ positive - \overline{OD}\ vehicle}\right] * 100$$

$OD_{cmpd}$: compound absorbance; $\overline{OD}_{positive}$: average value of the absorbance of positive control without compound; $\overline{OD}_{vehicle}$: average value of the absorbance of solvent vehicle control.

$IC_{50}$ values were fitted using GraphPad 7.0 based on inhibition rates of compounds at different concentrations.

The test results are shown in Table 5-a, 5-b and 5-c. A represents an $IC_{50}$ value<0.4 $\mu$M, B represents an $IC_{50}$ value ≥ 0.4 $\mu$M, and <1.0 $\mu$M, C represents an $IC_{50}$ value≥ 1.0 $\mu$M.

Table 5-a: Inhibitory activities of compounds of the present invention on MV-4-11 cell proliferation

| No. | MV-4-11 |
|---|---|
| Compound 1 | A |
| Compound 2 | A |
| Compound 3 | C |
| Compound 4 | C |
| Compound 5 | C |
| Compound 6 | C |
| Compound 7 | C |
| Compound 8 | C |
| Compound 9 | C |
| Compound 10 | C |
| Compound 11 | C |
| Compound 12 | C |
| Compound 13 | A |
| Compound 14 | A |
| Compound 15 | B |

(continued)

| No. | MV-4-11 |
|---|---|
| Compound 16 | C |
| Compound 17 | B |
| Compound 18 | C |
| Compound 19 | C |
| Compound 20 | C |
| Compound 21 | C |
| Compound 22 | B |
| Compound 23 | C |
| Compound 24 | A |
| Compound 25 | B |
| Compound 26 | A |
| Compound 27 | B |
| Compound 28 | A |
| Compound 29 | A |
| Compound 30 | A |
| Compound 31 | B |
| Compound 32 | B |
| Compound 33 | A |
| Compound 34 | A |
| Compound 35 | A |
| Compound 36 | B |
| Compound 37 | C |
| Compound 38 | A |
| Compound 39 | A |
| Compound 40 | B |
| Compound 41 | C |
| Compound 42 | C |
| Compound 43 | A |
| Compound 44 | A |
| Compound 45 | A |
| Compound 46 | B |
| Compound 47 | B |
| Compound 48 | A |
| Compound 49 | A |
| Compound 50 | A |
| Compound 51 | A |
| Compound 52 | A |
| Compound 53 | B |

(continued)

| No. | MV-4-11 |
|---|---|
| Compound 54 | A |
| Compound 55 | A |
| Compound 56 | B |
| Compound 57 | C |
| Compound 58 | A |
| Compound 60 | A |
| Compound 61 | A |
| Compound 103 | A |
| Compound 104 | A |
| Compound 106 | B |
| Compound 107 | B |
| Compound 108 | B |
| Compound 112 | B |
| Compound 113 | B |
| Compound 114 | B |
| Compound 115 | C |
| Compound 116 | A |
| Compound 117 | C |
| Compound 118 | C |
| Compound 119 | C |
| Compound 121 | B |
| Compound 122 | A |
| Compound 125 | C |
| Compound 126 | A |
| Compound 127 | A |
| Compound 128 | A |
| Compound 129 | C |
| Compound 130 | A |
| Compound 131 | A |
| Compound 132 | B |
| Compound 133 | A |
| Compound 134 | A |
| Compound 136 | A |
| Compound 137 | C |
| Compound 138 | A |
| Compound 139 | C |
| Compound 141 | A |

Table 5-b: Inhibitory activities of compounds of the present invention on MV-4-11 cell proliferation

| No. | MV-4-11 |
|---|---|
| Compound 142 | A |
| Compound 143 | C |
| Compound 144 | A |
| Compound 146 | C |
| Compound 147 | B |
| Compound 148 | A |
| Compound 149 | A |
| Compound 150 | A |
| Compound 151 | A |
| Compound 152 | A |
| Compound 153 | A |
| Compound 154 | A |
| Compound 158 | B |
| Compound 163 | A |
| Compound 164 | A |
| Compound 166 | A |
| Compound 167 | A |
| Compound 168 | A |
| Compound 169 | A |
| Compound 170 | A |
| Compound 171 | A |
| Compound 172 | A |
| Compound 173 | A |
| Compound 174 | A |
| Compound 175 | A |
| Compound 176 | A |
| Compound 177 | C |
| Compound 178 | B |
| Compound 179 | A |
| Compound 180 | A |
| Compound 181 | A |
| Compound 183 | A |
| Compound 184 | A |
| Compound 185 | A |
| Compound 186 | A |
| Compound 187 | A |
| Compound 191 | A |

(continued)

| No. | MV-4-11 |
|---|---|
| Compound 193 | B |
| Compound 194 | C |
| Compound 195 | B |
| Compound 196 | C |
| Compound 197 | C |
| Compound 198 | A |
| Compound 199 | A |
| Compound 200 | A |
| Compound 201 | A |
| Compound 202 | A |
| Compound 203 | A |
| Compound 204 | A |
| Compound 206 | A |
| Compound 207 | A |
| Compound 208 | B |
| Compound 209 | A |
| Compound 210 | B |
| Compound 211 | A |

Table 5-c: Inhibitory activities of compounds of the present invention on MV-4-11 cell proliferation

| No. | MV-4-11 |
|---|---|
| Compound C1 | A |
| Compound C2 | A |
| Compound C4 | B |
| Compound C5 | B |
| Compound C6 | A |
| Compound C7 | A |
| Compound C8 | A |
| Compound C9 | A |
| Compound C10 | C |
| Compound C11 | A |
| Compound C12 | A |
| Compound C13 | A |
| Compound C14 | A |
| Compound C15 | A |
| Compound C16 | A |
| Compound C17 | A |

(continued)

| No. | MV-4-11 |
|---|---|
| Compound C18 | A |
| Compound C19 | B |
| Compound C20 | A |
| Compound C21 | A |
| Compound C22 | A |
| Compound C23 | A |
| Compound C24 | A |
| Compound C25 | A |
| Compound C26 | A |
| Compound C27 | C |
| Compound C28 | A |
| Compound C29 | B |
| Compound C30 | A |
| Compound C31 | A |
| Compound C32 | A |
| Compound C33 | C |
| Compound C34 | A |
| Compound C35 | A |
| Compound C36 | A |
| Compound C37 | A |
| Compound C38 | A |
| Compound C39 | A |
| Compound C40 | A |
| Compound C41 | A |
| Compound C42 | A |
| Compound C43 | A |
| Compound C45 | A |
| Compound C46 | A |

Table 6: Inhibitory activities of compounds of the present invention on NCI-H929 cell proliferation

| No. | NCI-H929/nM |
|---|---|
| Compound 28 | 25 |
| Compound 30 | 14 |
| Compound 33 | 8 |
| Compound 45 | 22 |
| Compound 52 | 9 |
| Compound 55 | 26.10 |

It can be seen from the results in Tables 5-a, 5-b, 5-c and 6 that the compounds of the present invention have good inhibition activities on the proliferation of tumor cells, which indicates that the compounds of the present invention have greater potential in treating tumors.

Also, the compounds of the present invention have good selectivity for CDK9.

## Claims

1. A compound represented by formula (I), its pharmaceutically acceptable salt or isomer:

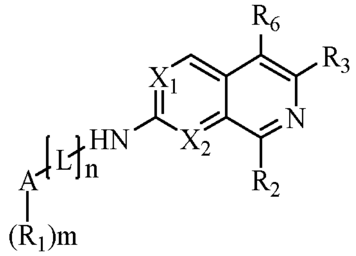

(I)

$X_1$ is selected from N or $CR_4$;
$X_2$ is selected from N or $CR_5$;
L is selected from bond, -C(O)-, $-(CH_2)p-$;
A is selected from bond, 3-12 membered cycloalkyl, 3-12 membered cycloalkenyl, 3-12 membered heterocyclyl, aryl, 5-10 membered heteroaryl, wherein the heteroatoms of the 3-12 membered heterocyclyl are selected from one of O, S, N or any combination thereof, C atom can be optionally oxidized to C(O), S atom can be optionally oxidized to S(O) or $S(O)_2$, the heteroatoms of the 5-10 membered heteroaryl are selected from one of O, S, and N or any combination thereof;
$R_1$ is selected from hydrogen, halogen, hydroxyl, carboxyl, amino, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonylamino, $C_{1-6}$ alkylaminocarbonyl, ($C_{1-6}$ alkyl)$_2$ aminocarbonyl, $C_{1-6}$ alkoxycarbonyl, $C_{1-6}$ alkylcarbonyl, $C_{1-6}$ alkylsulfonyl, aminosulfonyl, $C_{1-6}$ alkylthio, 3-12 membered cycloalkyl, 3-12 membered heterocyclyl, 3-12 membered cycloalkenyl,
wherein the heteroatoms of the 3-12 membered heterocyclyl are selected from one of O, S, N or any combination thereof, C atom can be optionally oxidized to C(O), S atom can be optionally oxidized to S(O) or $S(O)_2$, the heteroatoms of the 5-10 membered heteroaryl are selected from one of O, S, and N or any combination thereof, wherein the amino, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl , halogenated $C_{1-6}$ alkoxy, $C_{2-8}$ alkenyl, $C_{2-8}$ alkynyl, $C_{1-6}$ alkylcarbonylamino, $C_{1-6}$ alkylaminocarbonyl, ($C_{1-6}$ alkyl)$_2$ aminocarbonyl, $C_{1-6}$ alkoxycarbonyl,$C_{1-6}$ alkylcarbonyl, $C_{1-6}$ alkylsulfonyl, aminosulfonyl, $C_{1-6}$ alkylthio, 3-12 membered cycloalkyl, 3-12 membered heterocyclyl, 3-12 membered cycloalkenyl are unsubstituted or optionally substituted by one or more groups selected from hydroxyl, amino, carboxy, cyano, nitro, halogen, $C_{1-6}$ alkyl, $C_{1-6}$ alkoxy, halogenated $C_{1-6}$ alkyl, halogenated $C_{1-6}$ alkoxy, $C_{1-6}$ alkylamino, ($C_{1-6}$ alkyl)$_2$ amino, $C_{1-6}$ alkylsulfonyl, HS(O)(=NH)-, $C_{1-6}$ alkyl-S(O)(=NH)-, ($C_{1-6}$ alkyl)$_2$ aminocarbonyl, $C_{1-6}$ alkyl carbonyl, 3-12 membered cycloalkyl, 3-12 membered heterocyclyl;
$R_2$ is selected from halogen, cyano, hydroxyl, carboxyl, amino, $C_{1-6}$ alkyl, $C_{1-6}$ alkoxy, $C_{1-6}$ alkylthio, $C_{1-6}$ alkylamino, ($C_{1-6}$ alkyl)$_2$ amino, 3-12 membered cycloalkyl, 3-12 membered heterocyclyl, 3-12 membered cycloalkenyl, 3-12 membered heterocyclyloxy, 3-12 membered cycloalkyloxy, 3-12 membered cycloalkyl amino, 3-12 membered heterocyclyl-$CH_2$-amino, wherein the amino, $C_{1-6}$ alkyl, $C_{1-6}$ alkoxy, $C_{1-6}$ alkylthio, $C_{1-6}$ alkylamino, ($C_{1-6}$ alkyl)$_2$ amino, 3-12 membered cycloalkyl, 3-12 membered heterocyclyl, 3-12 membered cycloalkenyl, 3-12 membered heterocyclyloxy, 3-12 membered cycloalkyloxy, 3-12 membered cycloalkyl amino, 3-12 membered heterocyclyl-$CH_2$-amino are unsubstituted or optionally substituted by one or more groups selected from halogen, cyano, hydroxyl, carboxy, amino, $C_{1-6}$ alkyl, $C_{1-6}$ alkoxy, halogenated $C_{1-6}$ alkyl, halogenated $C_{1-6}$ alkoxy, $C_{1-6}$ alkylamino, ($C_{1-6}$ alkyl)$_2$ amino, 3-12 membered cycloalkyl, 3-12 membered heterocyclyl, aryl, 3-12 membered cycloalkenyl and 5-10 membered heteroaryl;
$R_3$ is selected from hydrogen, hydroxy, amino, carboxy, aminocarbonyl, cyano, nitro, halogen, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonyl, $C_{1-6}$ alkylsulfonyl, $C_{1-6}$ alkylthio , 3-12 membered cycloalkyl, 3-12 membered cycloalkenyl, 3-12 membered heterocyclyl, wherein the amino, aminocarbonyl, $C_{1-6}$ alkyl, $C_{1-6}$ alkoxy, $C_{1-6}$

alkylamino, ($C_{1-6}$ alkyl)$_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonyl, $C_{1-6}$ alkylsulfonyl, $C_{1-6}$ alkylthio, 3-12 membered cycloalkyl, 3-12 membered cycloalkenyl, 3-12 membered heterocyclyl are unsubstituted or optionally substituted by one or more groups selected from hydroxyl, amino, carboxyl, cyano, nitro, halogen, $C_{1-6}$ alkyl, $C_{1-6}$ alkoxy, $C_{1-6}$ alkoxy$C_{1-6}$ alkoxy, $C_{1-6}$ alkylamino, ($C_{1-6}$ alkyl)$_2$ amino, $C_{1-6}$ alkylcarbonylamino and $C_{1-6}$ alkylsulfonylamino;
n is 0 or 1;
m is 0, 1, 2 or 3;
p is 1, 2 or 3;
$R_4$, $R_5$ and $R_6$ are each independently selected from hydrogen, halogen, hydroxy, amino, carboxy, cyano, nitro, $C_{1-6}$ alkyl, $C_{2-8}$ alkenyl, $C_{2-8}$ alkynyl, and halogenated $C_{1-6}$ alkyl.

**2.** The compound or the pharmaceutically acceptable salt or the isomer thereof according to claim 1, the following cases are excluded from the compound represented by formula (I) ;

case (i) $X_1$ is N, $X_2$ is N, n is 0, A is piperidinyl, and $R_1$ is selected from $C_{1-6}$ alkylsulfonyl, aminosulfonyl or $C_{1-6}$ alkylaminosulfonyl,
case (ii) $X_1$ is N, $X_2$ is N, n is 0, A is bond and $R_1$ is piperidinyl, wherein the piperidinyl is substituted by $C_{1-6}$ alkylsulfonyl, aminosulfonyl or $C_{1-6}$ alkylaminosulfonyl.

**3.** The compound or the pharmaceutically acceptable salt or the isomer thereof according to claim 1 or 2,

$X_1$ is selected from N or $CR_4$;
$X_2$ is selected from N or $CR_5$;
L is selected from bond, -C(O)-, -($CH_2$)p-;
A is selected from bond, 3-12 membered cycloalkyl, 3-12 membered cycloalkenyl, 3-12 membered heterocyclyl, aryl, 5-10 membered heteroaryl, wherein the heteroatoms of the 3-12 membered heterocyclyl are selected from one of O, S, N or any combination thereof, C atom can be optionally oxidized to C(O), S atom can be optionally oxidized to S(O) or S(O)$_2$, the heteroatoms of the 5-10 membered heteroaryl are selected from one of O, S, and N or any combination thereof;
$R_1$ is selected from hydrogen, halogen, hydroxyl, carboxyl, amino, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonylamino, $C_{1-6}$ alkylaminocarbonyl, ($C_{1-6}$ alkyl)$_2$ aminocarbonyl, $C_{1-6}$ alkoxycarbonyl, $C_{1-6}$ alkylcarbonyl, $C_{1-6}$ alkylsulfonyl, aminosulfonyl, $C_{1-6}$ alkylthio, 3-12 membered cycloalkyl, 3-12 membered heterocyclyl, 3-12 membered cycloalkenyl, wherein the heteroatoms of the 3-12 membered heterocyclyl are selected from one of O, S, N or any combination thereof, C atom can be optionally oxidized to C(O), S atom can be optionally oxidized to S(O) or S(O)$_2$, the heteroatoms of the 5-10 membered heteroaryl are selected from one of O, S, and N or any combination thereof, wherein the amino, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl , halogenated $C_{1-6}$ alkoxy, $C_{2-8}$ alkenyl, $C_{2-8}$ alkynyl, $C_{1-6}$ alkylcarbonylamino, $C_{1-6}$ alkylaminocarbonyl, ($C_{1-6}$ alkyl)$_2$ aminocarbonyl, $C_{1-6}$ alkoxycarbonyl,$C_{1-6}$ alkylcarbonyl, $C_{1-6}$ alkylsulfonyl, aminosulfonyl, $C_{1-6}$ alkylthio, 3-12 membered cycloalkyl, 3-12 membered heterocyclyl, 3-12 membered cycloalkenyl are unsubstituted or optionally substituted by one or more groups selected from hydroxyl, amino, carboxy, cyano, nitro, halogen, $C_{1-6}$ alkyl, $C_{1-6}$ alkoxy, halogenated $C_{1-6}$ alkyl, halogenated $C_{1-6}$ alkoxy, $C_{1-6}$ alkylamino, ($C_{1-6}$ alkyl)$_2$ amino, $C_{1-6}$ alkylsulfonyl, 3-12 membered cycloalkyl, 3-12 membered heterocyclyl;
$R_2$ is selected from halogen, cyano, hydroxyl, carboxyl, amino, $C_{1-6}$ alkyl, $C_{1-6}$ alkoxy, $C_{1-6}$ alkylamino, ($C_{1-6}$ alkyl)$_2$ amino, 3-12 membered cycloalkyl, 3-12 membered heterocyclyl, 3-12 membered cycloalkenyl, wherein the amino, $C_{1-6}$ alkyl, $C_{1-6}$ alkoxy, $C_{1-6}$ alkylamino, ($C_{1-6}$ alkyl)$_2$ amino, 3-12 membered cycloalkyl, 3-12 membered heterocyclyl, 3-12 membered cycloalkenyl are unsubstituted or optionally substituted by one or more groups selected from halogen, cyano, hydroxyl, carboxy, amino, $C_{1-6}$ alkyl, $C_{1- 6}$ alkoxy, halogenated $C_{1-6}$ alkyl, halogenated $C_{1-6}$ alkoxy, $C_{1-6}$ alkylamino, ($C_{1-6}$ alkyl)$_2$ amino, 3-12 membered cycloalkyl, 3-12 membered heterocyclyl, aryl and 5-10 membered heteroaryl;
$R_3$ is selected from hydrogen, hydroxy, amino, carboxy, cyano, nitro, halogen, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonyl, $C_{1-6}$ alkylsulfonyl, $C_{1-6}$ alkylthio , 3-12 membered cycloalkyl, 3-12 membered cycloalkenyl, 3-12 membered heterocyclyl, wherein the amino, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonyl, $C_{1-6}$ alkylsulfonyl, $C_{1-6}$ alkylthio, 3-12 membered cycloalkyl, 3-12 membered cycloalkenyl, 3-12 membered heterocyclyl are unsubstituted or optionally substituted by one or more groups selected from hydroxyl, amino, carboxyl, cyano, nitro, halogen, $C_{1-6}$ alkyl, $C_{1-6}$ alkoxy, $C_{1-6}$ alkoxy$C_{1-6}$ alkoxy, $C_{1-6}$ alkylamino, ($C_{1-6}$ alkyl)$_2$ amino, $C_{1-6}$ alkyl-

carbonylamino and $C_{1-6}$ alkylsulfonylamino;
n is 0 or 1;
m is 0, 1, 2 or 3;
p is 1, 2 or 3;
$R_4$, $R_5$ and $R_6$ are each independently selected from hydrogen, halogen, hydroxy, amino, carboxy, cyano, nitro, $C_{1-6}$ alkyl, $C_{2-8}$ alkenyl, $C_{2-8}$ alkynyl, and halogenated $C_{1-6}$ alkyl.

**4.** The compound or the pharmaceutically acceptable salt or the isomer thereof according to claim 1 or 2,

$X_1$ is N;
$X_2$ is N;
L is selected from bond -C(O)-, or $-(CH_2)p-$;
A is selected from bond, 3-12 membered cycloalkyl, or 3-12 membered heterocyclyl , wherein the heteroatoms of the 3-12 membered heterocyclyl are selected from any one of O, S, N or any combination thereof;
$R_1$ is selected from hydrogen, hydroxyl, $C_{1-6}$ alkyl, $C_{1-6}$ alkoxy, amino, $(C_{1-6}$ alkyl$)_2$ amino, $C_{1-6}$ alkylcarbonyl, 3-12 membered heterocyclyl, halogen, carboxyl, or 3-12 membered cycloalkyl,
wherein the heteroatoms of the 3-12 membered heterocyclyl are selected from any one of O, S, N or any combination thereof,
wherein the $C_{1-6}$ alkyl, $C_{1-6}$ alkoxy, amino, $(C_{1-6}$ alkyl$)_2$ amino, $C_{1-6}$ alkylcarbonyl, 3-12 membered heterocyclyl and 3-12 membered cycloalkyl are optionally substituted by any one or more groups selected from hydroxyl, amino, carboxy, cyano, nitro, halogen, $C_{1-6}$ alkyl, $C_{1-6}$ alkoxy, halogenated $C_{1-6}$ alkyl, halogenated $C_{1-6}$ alkoxy, $C_{1-6}$ alkylamino, $(C_{1-6}$ alkyl$)_2$ aminocarbonyl, $C_{1-6}$ alkyl carbonyl, $(C_{1-6}$ alkyl$)_2$ amino, $C_{1-6}$ alkylsulfonyl, 3-12 membered cycloalkyl, or 3-12 membered heterocyclyl;
$R_2$ is selected from amino, $C_{1-6}$ alkylamino, 3-12 membered heterocyclyl, or 3-12 membered cycloalkyl amino, halogen, cyano, hydroxyl, or $(C_{1-6}$ alkyl$)_2$ amino, wherein the heteroatoms of the 3-12 membered heterocyclyl are selected from any one of O, S, N or any combination thereof,
wherein the amino, $C_{1-6}$ alkylamino, 3-12 membered heterocyclyl, 3-12 membered cycloalkyl amino, and $(C_{1-6}$ alkyl$)_2$ amino are optionally substituted by any one or more groups selected from $C_{1-6}$ alkyl, $C_{1-6}$ alkoxy, 3-12membered cycloalkyl, 3-12 membered heterocyclyl, or 3-12 membered cycloalkenyl;
$R_3$ is selected from cyano, $C_{1-6}$ alkyl, aminocarbonyl, hydrogen, hydroxy, amino, halogen, or $C_{1-6}$ alkoxy, wherein the $C_{1-6}$ alkyl, aminocarbonyl, amino and $C_{1-6}$ alkoxy are optionally substituted by any one or more groups selected from halogen;
$R_6$ is selected from hydrogen, halogen, hydroxy, amino, or $C_{1-6}$ alkyl;
n is 0 or 1;
m is 0, 1, 2 or 3;
p is 1, 2 or 3;
provided that
a case in which .n is 0, A is bond, $R_1$ is piperidinyl, and m is 1, and wherein the piperidinyl is substituted by $C_{1-6}$ alkylsulfonyl is excluded.

**5.** The compound or the pharmaceutically acceptable salt or the isomer thereof according to claim 1 or 2, when L is bond, and n is 0, wherein the general formula (I) has a structure of general formula (II),

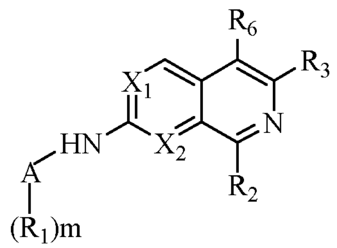

(II)

$X_1$ is selected from N or $CR_4$;
$X_2$ is selected from N or $CR_5$;
A is selected from 3-12 membered cycloalkyl, 3-12 membered cycloalkenyl, 3-12 membered heterocyclyl, aryl, 5-10 membered heteroaryl, wherein the heteroatoms of the 3-12 membered heterocyclyl are selected from one of O, S, N or any combination thereof, C atom can be optionally oxidized to C(O), S atom can be optionally

oxidized to S(O) or $S(O)_2$, the heteroatoms of the 5-10 membered heteroaryl are selected from one of O, S, and N or any combination thereof;

$R_1$ is selected from hydrogen, halogen, hydroxyl, carboxyl, amino, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl , halogenated $C_{1-6}$ alkoxy, $C_{2-8}$ alkenyl, $C_{2-8}$ alkynyl, $C_{1-6}$ alkylcarbonylamino, $C_{1-6}$ alkylaminocarbonyl, ($C_{1-6}$ alkyl)$_2$ aminocarbonyl, $C_{1-6}$ alkoxycarbonyl,$C_{1-6}$ alkylcarbonyl, $C_{1-6}$ alkylsulfonyl, aminosulfonyl, $C_{1-6}$ alkylthio, 3-12 membered cycloalkyl, 3-12 membered heterocyclyl, 3-12 membered cycloalkenyl, wherein the heteroatoms of the 3-12 membered heterocyclyl are selected from one of O, S, N or any combination thereof, C atom can be optionally oxidized to C(O), S atom can be optionally oxidized to S(O) or $S(O)_2$, the heteroatoms of the 5-10 membered heteroaryl are selected from one of O, S, and N or any combination thereof, wherein the amino, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl , halogenated $C_{1-6}$ alkoxy, $C_{2-8}$ alkenyl, $C_{2-8}$ alkynyl, $C_{1-6}$ alkylcarbonylamino, $C_{1-6}$ alkylaminocarbonyl, ($C_{1-6}$ alkyl)$_2$ aminocarbonyl, $C_{1-6}$ alkoxycarbonyl,$C_{1-6}$ alkylcarbonyl, $C_{1-6}$ alkylsulfonyl, aminosulfonyl, $C_{1-6}$ alkylthio, 3-12 membered cycloalkyl, 3-12 membered heterocyclyl, 3-12 membered cycloalkenyl are unsubstituted or optionally substituted by one or more groups selected from hydroxyl, amino, carboxy, cyano, nitro, halogen, $C_{1-6}$ alkyl, $C_{1-6}$ alkoxy, halogenated $C_{1-6}$ alkyl, halogenated $C_{1-6}$ alkoxy, $C_{1-6}$ alkylamino, ($C_{1-6}$ alkyl)$_2$ amino, $C_{1-6}$ alkylsulfonyl, HS(O)(=NH)-, $C_{1-6}$ alkyl-S(O)(=NH)-, 3-12 membered cycloalkyl, 3-12 membered heterocyclyl;

$R_2$ is selected from halogen, cyano, hydroxyl, carboxyl, amino, $C_{1-6}$ alkyl, $C_{1-6}$ alkoxy, $C_{1-6}$ alkylamino, ($C_{1-6}$ alkyl)$_2$ amino, 3-12 membered cycloalkyl, 3-12 membered heterocyclyl, 3-12 membered cycloalkenyl, 3-12 membered heterocyclyloxy, 3-12 membered cycloalkyl amino, 3-12 membered heterocyclyl-$CH_2$-amino, wherein the amino, $C_{1-6}$ alkyl, $C_{1-6}$ alkoxy, $C_{1-6}$ alkylamino, ($C_{1-6}$ alkyl)$_2$ amino, 3-12 membered cycloalkyl, 3-12 membered heterocyclyl, 3-12 membered cycloalkenyl, 3-12 membered heterocyclyloxy, 3-12 membered cycloalkyl amino, 3-12 membered heterocyclyl-$CH_2$-amino are unsubstituted or optionally substituted by one or more groups selected from halogen, cyano, hydroxyl, carboxy, amino, $C_{1-6}$ alkyl, $C_{1-6}$ alkoxy, halogenated $C_{1-6}$ alkyl, halogenated $C_{1-6}$ alkoxy, $C_{1-6}$ alkylamino, ($C_{1-6}$ alkyl)$_2$ amino, 3-12 membered cycloalkyl, 3-12 membered heterocyclyl, aryl and 5-10 membered heteroaryl;

$R_3$ is selected from hydrogen, hydroxy, amino, carboxy, cyano, nitro, halogen, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonyl, $C_{1-6}$ alkylsulfonyl, $C_{1-6}$ alkylthio , 3-12 membered cycloalkyl, 3-12 membered cycloalkenyl, 3-12 membered heterocyclyl, wherein the amino, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonyl, $C_{1-6}$ alkylsulfonyl, $C_{1-6}$ alkylthio , 3-12 membered cycloalkyl, 3-12 membered cycloalkenyl, 3-12 membered heterocyclyl are unsubstituted or optionally substituted by one or more groups selected from hydroxyl, amino, carboxyl, cyano, nitro, halogen, $C_{1-6}$ alkyl, $C_{1-6}$ alkoxy, $C_{1-6}$ alkoxy$C_{1-6}$ alkoxy, $C_{1-6}$ alkylamino, ($C_{1-6}$ alkyl)$_2$ amino, $C_{1-6}$ alkylcarbonylamino and $C_{1-6}$ alkylsulfonylamino;

m is 0, 1, 2 or 3;

$R_4$, $R_5$ and $R_6$ are each independently selected from hydrogen, halogen, hydroxy, amino, carboxy, cyano, nitro, $C_{1-6}$ alkyl, $C_{2-8}$ alkenyl, $C_{2-8}$ alkynyl, and halogenated $C_{1-6}$ alkyl;

preferably, at least one of $X_1$ and $X_2$ is N.

**6.** The compound or the pharmaceutically acceptable salt or the isomer thereof according to claim 5,

$X_1$ is selected from N or $CR_4$;

$X_2$ is selected from N or $CR_5$;

A is selected from 3-12 membered cycloalkyl, 3-12 membered cycloalkenyl, 3-12 membered heterocyclyl, aryl, 5-10 membered heteroaryl, wherein the heteroatoms of the 3-12 membered heterocyclyl are selected from one of O, S, N or any combination thereof, C atom can be optionally oxidized to C(O), S atom can be optionally oxidized to S(O) or $S(O)_2$, the heteroatoms of the 5-10 membered heteroaryl are selected from one of O, S, and N or any combination thereof;

$R_1$ is selected from hydrogen, halogen, hydroxyl, carboxyl, amino, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl , halogenated $C_{1-6}$ alkoxy, $C_{2-8}$ alkenyl, $C_{2-8}$ alkynyl, $C_{1-6}$ alkylcarbonylamino, $C_{1-6}$ alkylaminocarbonyl, ($C_{1-6}$ alkyl)$_2$ aminocarbonyl, $C_{1-6}$ alkoxycarbonyl,$C_{1-6}$ alkylcarbonyl, $C_{1-6}$ alkylsulfonyl, aminosulfonyl, $C_{1-6}$ alkylthio, 3-12 membered cycloalkyl, 3-12 membered heterocyclyl, 3-12 membered cycloalkenyl, wherein the heteroatoms of the 3-12 membered heterocyclyl are selected from one of O, S, N or any combination thereof, C atom can be optionally oxidized to C(O), S atom can be optionally oxidized to S(O) or $S(O)_2$, the heteroatoms of the 5-10 membered heteroaryl are selected from one of O, S, and N or any combination thereof, wherein the amino, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl , halogenated $C_{1-6}$ alkoxy, $C_{2-8}$ alkenyl, $C_{2-8}$ alkynyl, $C_{1-6}$ alkylcarbonylamino, $C_{1-6}$ alkylami-

nocarbonyl, ($C_{1-6}$ alkyl)$_2$ aminocarbonyl, $C_{1-6}$ alkoxycarbonyl,$C_{1-6}$ alkylcarbonyl, $C_{1-6}$ alkylsulfonyl, aminosulfonyl, $C_{1-6}$ alkylthio, 3-12 membered cycloalkyl, 3-12 membered heterocyclyl, 3-12 membered cycloalkenyl are unsubstituted or optionally substituted by one or more groups selected from hydroxyl, amino, carboxy, cyano, nitro, halogen, $C_{1-6}$ alkyl, $C_{1-6}$ alkoxy, halogenated $C_{1-6}$ alkyl, halogenated $C_{1-6}$ alkoxy, $C_{1-6}$ alkylamino, ($C_{1-6}$ alkyl)$_2$ amino, $C_{1-6}$ alkylsulfonyl, 3-12 membered cycloalkyl, 3-12 membered heterocyclyl;

$R_2$ is selected from halogen, cyano, hydroxyl, carboxyl, amino, $C_{1-6}$ alkyl, $C_{1-6}$ alkoxy, $C_{1-6}$ alkylamino, ($C_{1-6}$ alkyl)$_2$ amino, 3-12 membered cycloalkyl, 3-12 membered heterocyclyl, 3-12 membered cycloalkenyl, wherein the amino, $C_{1-6}$ alkyl, $C_{1-6}$ alkoxy, $C_{1-6}$ alkylamino, ($C_{1-6}$ alkyl)$_2$ amino, 3-12 membered cycloalkyl, 3-12 membered heterocyclyl, 3-12 membered cycloalkenyl are unsubstituted or optionally substituted by one or more groups selected from halogen, cyano, hydroxyl, carboxy, amino, $C_{1-6}$ alkyl, $C_{1-6}$ alkoxy, halogenated $C_{1-6}$ alkyl, halogenated $C_{1-6}$ alkoxy, $C_{1-6}$ alkylamino, ($C_{1-6}$ alkyl)$_2$ amino, 3-12 membered cycloalkyl, 3-12 membered heterocyclyl, aryl and 5-10 membered heteroaryl;

$R_3$ is selected from hydrogen, hydroxy, amino, carboxy, cyano, nitro, halogen, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonyl, $C_{1-6}$ alkylsulfonyl, $C_{1-6}$ alkylthio , 3-12 membered cycloalkyl, 3-12 membered cycloalkenyl, 3-12 membered heterocyclyl, wherein the amino, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonyl, $C_{1-6}$ alkylsulfonyl, $C_{1-6}$ alkylthio , 3-12 membered cycloalkyl, 3-12 membered cycloalkenyl, 3-12 membered heterocyclyl are unsubstituted or optionally substituted by one or more groups selected from hydroxyl, amino, carboxyl, cyano, nitro, halogen, $C_{1-6}$ alkyl, $C_{1-6}$ alkoxy, $C_{1-6}$ alkoxy$C_{1-6}$ alkoxy, $C_{1-6}$ alkylamino, ($C_{1-6}$ alkyl)$_2$ amino, $C_{1-6}$ alkylcarbonylamino and $C_{1-6}$ alkylsulfonylamino;

m is 0, 1, 2 or 3;

$R_4$, $R_5$ and $R_6$ are each independently selected from hydrogen, halogen, hydroxy, amino, carboxy, cyano, nitro, $C_{1-6}$ alkyl, $C_{2-8}$ alkenyl, $C_{2-8}$ alkynyl, and halogenated $C_{1-6}$ alkyl;

preferably, at least one of $X_1$ and $X_2$ is N.

**7.** The compound or the pharmaceutically acceptable salt or the isomer thereof according to Claim 5,

$R_1$ is selected from hydrogen, halogen, hydroxyl, carboxyl, amino, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl , halogenated $C_{1-6}$ alkoxy, $C_{2-8}$ alkenyl, $C_{2-8}$ alkynyl, $C_{1-6}$ alkylaminocarbonyl, ($C_{1-6}$ alkyl)$_2$ aminocarbonyl, $C_{1-6}$ alkoxycarbonyl, $C_{1-6}$ alkylcarbonyl, $C_{1-6}$ alkylsulfonyl, $C_{1-6}$ alkylthio, 3-12 membered cycloalkyl, 3-12 membered heterocyclyl, 3-12 membered cycloalkenyl, wherein the amino, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl , halogenated $C_{1-6}$ alkoxy, $C_{2-8}$ alkenyl, $C_{2-8}$ alkynyl, $C_{1-6}$ alkylaminocarbonyl, ($C_{1-6}$ alkyl)$_2$ aminocarbonyl, $C_{1-6}$ alkoxycarbonyl, $C_{1-6}$ alkylcarbonyl, $C_{1-6}$ alkylsulfonyl, $C_{1-6}$ alkylthio, 3-12 membered cycloalkyl, 3-12 membered heterocyclyl, 3-12 membered cycloalkenyl are unsubstituted or optionally substituted by one or more groups selected from hydroxyl, amino, carboxy, cyano, nitro, halogen, $C_{1-6}$ alkyl, $C_{1-6}$ alkoxy, halogenated $C_{1-6}$ alkyl, halogenated $C_{1-6}$ alkoxy, $C_{1-6}$ alkylamino, ($C_{1-6}$ alkyl)$_2$ amino, $C_{1-6}$ alkylsulfonyl, HS(O)(=NH)-, $C_{1-6}$ alkyl-S(O)(=NH)-, 3-12 membered cycloalkyl, 3-12 membered heterocyclyl;

$R_3$ is selected from cyano, and halogenated $C_{1-6}$ alkyl.

**8.** The compound or the pharmaceutically acceptable salt or the isomer thereof according to claim 5,

$X_1$ is N;

$X_2$ is N;

A is selected from bond, 3-12 membered cycloalkyl, or 3-12 membered heterocyclyl , wherein the heteroatoms of the 3-12 membered heterocyclyl are selected from any one of O, S, N or any combination thereof;

$R_1$ is selected from hydrogen, hydroxyl, $C_{1-6}$ alkyl, $C_{1-6}$ alkoxy, amino, ($C_{1-6}$ alkyl)$_2$ amino, $C_{1-6}$ alkylcarbonyl, 3-12 membered heterocyclyl, halogen, carboxyl, or 3-12 membered cycloalkyl,

wherein the heteroatoms of the 3-12 membered heterocyclyl are selected from any one of O, S, N or any combination thereof,

wherein the $C_{1-6}$ alkyl, $C_{1-6}$ alkoxy, amino, ($C_{1-6}$ alkyl)$_2$ amino, $C_{1-6}$ alkylcarbonyl, 3-12 membered heterocyclyl and 3-12 membered cycloalkyl are optionally substituted by any one or more groups selected from hydroxyl, amino, carboxy, cyano, nitro, halogen, $C_{1-6}$ alkyl, $C_{1-6}$ alkoxy, halogenated $C_{1-6}$ alkyl, halogenated $C_{1-6}$ alkoxy, $C_{1-6}$ alkylamino, ($C_{1-6}$ alkyl)$_2$ aminocarbonyl, $C_{1-6}$ alkyl carbonyl, ($C_{1-6}$ alkyl)$_2$ amino, $C_{1-6}$ alkyl sulfonyl, 3-12 membered cycloalkyl, or 3-12 membered heterocyclyl;

$R_2$ is selected from amino, $C_{1-6}$ alkylamino, 3-12 membered heterocyclyl, or 3-12 membered cycloalkyl amino, halogen, cyano, hydroxyl, or ($C_{1-6}$ alkyl)$_2$ amino, wherein the heteroatoms of the 3-12 membered heterocyclyl,

or 3-12 membered cycloalkyl amino, are selected from any one of O, S, N or any combination thereof, wherein the amino, $C_{1-6}$ alkylamino, 3-12 membered heterocyclyl and $(C_{1-6}$ alkyl$)_2$ amino are optionally substituted by any one or more groups selected from $C_{1-6}$ alkyl, $C_{1-6}$ alkoxy, 3-12membered cycloalkyl, 3-12 membered heterocyclyl, or 3-12 membered cycloalkenyl;

$R_3$ is selected from cyano, $C_{1-6}$ alkyl, aminocarbonyl, hydrogen, hydroxy, amino, halogen, or $C_{1-6}$ alkoxy, wherein the $C_{1-6}$ alkyl, aminocarbonyl, amino and $C_{1-6}$ alkoxy are optionally substituted by any one or more groups selected from halogen;

$R_6$ is selected from hydrogen, halogen, hydroxy, amino, or $C_{1-6}$ alkyl;

m is 0, 1, 2 or 3;

p is 1, 2 or 3.

**9.** The compound or the pharmaceutically acceptable salt or the isomer thereof according to claim 1 or 2, when L is -C(O)-, n is 1, wherein the general formula (I) has a structure of general formula (III),

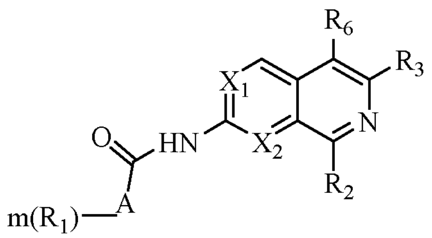

(III)

$X_1$ is selected from N or $CR_4$;

$X_2$ is selected from N or $CR_5$;

A is selected from 3-12 membered cycloalkyl, 3-12 membered cycloalkenyl, 3-12 membered heterocyclyl, aryl, 5-10 membered heteroaryl, wherein the heteroatoms of the 3-12 membered heterocyclyl are selected from one of O, S, N or any combination thereof, C atom can be optionally oxidized to C(O), S atom can be optionally oxidized to S(O) or $S(O)_2$, the heteroatoms of the 5-10 membered heteroaryl are selected from one of O, S, and N or any combination thereof;

$R_1$ is selected from hydrogen, halogen, hydroxyl, carboxyl, amino, $C_{1-6}$ alkyl, $C_{1-6}$ alkoxy, $C_{1-6}$ alkylamino, $(C_{1-6}$ alkyl$)_2$ amino, halogenated $C_{1-6}$ alkyl , halogenated $C_{1-6}$ alkoxy, $C_{2-8}$ alkenyl, $C_{2-8}$ alkynyl, $C_{1-6}$ alkylcarbonylamino, $C_{1-6}$ alkylaminocarbonyl, $(C_{1-6}$ alkyl$)_2$ aminocarbonyl, $C_{1-6}$ alkoxycarbonyl,$C_{1-6}$ alkylcarbonyl, $C_{1-6}$ alkylsulfonyl, aminosulfonyl, $C_{1-6}$ alkylthio, 3-12 membered cycloalkyl, 3-12 membered heterocyclyl, 3-12 membered cycloalkenyl, wherein the heteroatoms of the 3-12 membered heterocyclyl are selected from one of O, S, N or any combination thereof, C atom can be optionally oxidized to C(O), S atom can be optionally oxidized to S(O) or $S(O)_2$, the heteroatoms of the 5-10 membered heteroaryl are selected from one of O, S, and N or any combination thereof, wherein the amino, $C_{1-6}$ alkyl, $C_{1-6}$ alkoxy, $C_{1-6}$ alkylamino, $(C_{1-6}$ alkyl$)_2$ amino, halogenated $C_{1-6}$ alkyl , halogenated $C_{1-6}$ alkoxy, $C_{2-8}$ alkenyl, $C_{2-8}$ alkynyl, $C_{1-6}$ alkylcarbonylamino, $C_{1-6}$ alkylaminocarbonyl, $(C_{1-6}$ alkyl$)_2$ aminocarbonyl, $C_{1-6}$ alkoxycarbonyl,$C_{1-6}$ alkylcarbonyl, $C_{1-6}$ alkylsulfonyl, aminosulfonyl, $C_{1-6}$ alkylthio, 3-12 membered cycloalkyl, 3-12 membered heterocyclyl, 3-12 membered cycloalkenyl are unsubstituted or optionally substituted by one or more groups selected from hydroxyl, amino, carboxy, cyano, nitro, halogen, $C_{1-6}$ alkyl, $C_{1-6}$ alkoxy, halogenated $C_{1-6}$ alkyl, halogenated $C_{1-6}$ alkoxy, $C_{1-6}$ alkylamino, $(C_{1-6}$ alkyl$)_2$ amino, $C_{1-6}$ alkylsulfonyl, HS(O)(=NH)-, $C_{1-6}$ alkyl-S(O)(=NH)-, 3-12 membered cycloalkyl, 3-12 membered heterocyclyl;

$R_2$ is selected from halogen, cyano, hydroxyl, carboxyl, amino, $C_{1-6}$ alkyl, $C_{1-6}$ alkoxy, $C_{1-6}$ alkylamino, $(C_{1-6}$ alkyl$)_2$ amino, 3-12 membered cycloalkyl, 3-12 membered heterocyclyl, 3-12 membered cycloalkenyl, 3-12 membered heterocyclyloxy, 3-12 membered cycloalkyl amino, 3-12 membered heterocyclyl-$CH_2$-amino, wherein the amino, $C_{1-6}$ alkyl, $C_{1-6}$ alkoxy, $C_{1-6}$ alkylamino, $(C_{1-6}$ alkyl$)_2$ amino, 3-12 membered cycloalkyl, 3-12 membered heterocyclyl, 3-12 membered cycloalkenyl, 3-12 membered heterocyclyloxy, 3-12 membered cycloalkyl amino, 3-12 membered heterocyclyl-$CH_2$-amino are unsubstituted or optionally substituted by one or more groups selected from halogen, cyano, hydroxyl, carboxy, amino, $C_{1-6}$ alkyl, $C_{1-6}$ alkoxy, halogenated $C_{1-6}$ alkyl, halogenated $C_{1-6}$ alkoxy, $C_{1-6}$ alkylamino, $(C_{1-6}$ alkyl$)_2$ amino, 3-12 membered cycloalkyl, 3-12 membered heterocyclyl, aryl and 5-10 membered heteroaryl;

$R_3$ is selected from hydrogen, hydroxy, amino, carboxy, cyano, nitro, halogen, $C_{1-6}$ alkyl, $C_{1-6}$ alkoxy, $C_{1-6}$ alkylamino, $(C_{1-6}$ alkyl$)_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonyl, $C_{1-6}$ alkylsulfonyl, $C_{1-6}$ alkylthio , 3-12 membered cycloalkyl, 3-12 membered cycloalkenyl, 3-12 membered heterocyclyl, wherein the amino, $C_{1-6}$ alkyl, $C_{1-6}$ alkoxy, $C_{1-6}$ alkylamino, $(C_{1-6}$ alkyl$)_2$ amino, halo-

genated $C_{1-6}$ alkyl, halogenated $C_{1-6}$ alkoxy, $C_{2-8}$ alkenyl, $C_{2-8}$ alkynyl, $C_{1-6}$ alkylcarbonyl, $C_{1-6}$ alkylsulfonyl, $C_{1-6}$ alkylthio , 3-12 membered cycloalkyl, 3-12 membered cycloalkenyl, 3-12 membered heterocyclyl are unsubstituted or optionally substituted by one or more groups selected from hydroxyl, amino, carboxyl, cyano, nitro, halogen, $C_{1-6}$ alkyl, $C_{1-6}$ alkoxy, $C_{1-6}$ alkoxy$C_{1-6}$ alkoxy, $C_{1-6}$ alkylamino, ($C_{1-6}$ alkyl)$_2$ amino, $C_{1-6}$ alkylcarbonylamino and $C_{1-6}$ alkylsulfonylamino;
m is 0, 1, 2 or 3;
$R_4$, $R_5$ and $R_6$ are each independently selected from hydrogen, halogen, hydroxy, amino, carboxy, cyano, nitro,$C_{1-6}$ alkyl, $C_{2-8}$ alkenyl, $C_{2-8}$ alkynyl, and halogenated $C_{1-6}$ alkyl;
preferably, at least one of $X_1$ and $X_2$ is N.

**10.** The compound or the pharmaceutically acceptable salt or the isomer thereof according to claim 9,

$X_1$ is selected from N or $CR_4$;
$X_2$ is selected from N or $CR_5$;
A is selected from 3-12 membered cycloalkyl, 3-12 membered cycloalkenyl, 3-12 membered heterocyclyl, aryl, 5-10 membered heteroaryl, wherein the heteroatoms of the 3-12 membered heterocyclyl are selected from one of O, S, N or any combination thereof, C atom can be optionally oxidized to C(O), S atom can be optionally oxidized to S(O) or S(O)$_2$, the heteroatoms of the 5-10 membered heteroaryl are selected from one of O, S, and N or any combination thereof;
$R_1$ is selected from hydrogen, halogen, hydroxyl, carboxyl, amino, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl , halogenated $C_{1-6}$ alkoxy, $C_{2-8}$ alkenyl, $C_{2-8}$ alkynyl, $C_{1-6}$ alkylcarbonylamino, $C_{1-6}$ alkylaminocarbonyl, ($C_{1-6}$ alkyl)$_2$ aminocarbonyl, $C_{1-6}$ alkoxycarbonyl,$C_{1-6}$ alkylcarbonyl, $C_{1-6}$ alkylsulfonyl, aminosulfonyl, $C_{1-6}$ alkylthio, 3-12 membered cycloalkyl, 3-12 membered heterocyclyl, 3-12 membered cycloalkenyl, wherein the heteroatoms of the 3-12 membered heterocyclyl are selected from one of O, S, N or any combination thereof, C atom can be optionally oxidized to C(O), S atom can be optionally oxidized to S(O) or S(O)$_2$, the heteroatoms of the 5-10 membered heteroaryl are selected from one of O, S, and N or any combination thereof, wherein the amino, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl , halogenated $C_{1-6}$ alkoxy, $C_{2-8}$ alkenyl, $C_{2-8}$ alkynyl, $C_{1-6}$ alkylcarbonylamino, $C_{1-6}$ alkylaminocarbonyl, ($C_{1-6}$ alkyl)$_2$ aminocarbonyl, $C_{1-6}$ alkoxycarbonyl,$C_{1-6}$ alkylcarbonyl, $C_{1-6}$ alkylsulfonyl, aminosulfonyl, $C_{1-6}$ alkylthio, 3-12 membered cycloalkyl, 3-12 membered heterocyclyl, 3-12 membered cycloalkenyl are unsubstituted or optionally substituted by one or more groups selected from hydroxyl, amino, carboxy, cyano, nitro, halogen, $C_{1-6}$ alkyl, $C_{1-6}$ alkoxy, halogenated $C_{1-6}$ alkyl, halogenated $C_{1-6}$ alkoxy, $C_{1-6}$ alkylamino, ($C_{1-6}$ alkyl)$_2$ amino, $C_{1-6}$ alkylsulfonyl, 3-12 membered cycloalkyl, 3-12 membered heterocyclyl;
$R_2$ is selected from halogen, cyano, hydroxyl, carboxyl, amino, $C_{1-6}$ alkyl, $C_{1-6}$ alkoxy, $C_{1-6}$ alkylamino, ($C_{1-6}$ alkyl)$_2$ amino, 3-12 membered cycloalkyl, 3-12 membered heterocyclyl, 3-12 membered cycloalkenyl, wherein the amino, $C_{1-6}$ alkyl, $C_{1-6}$ alkoxy, $C_{1-6}$ alkylamino, ($C_{1-6}$ alkyl)$_2$ amino, 3-12 membered cycloalkyl, 3-12 membered heterocyclyl, 3-12 membered cycloalkenyl are unsubstituted or optionally substituted by one or more groups selected from halogen, cyano, hydroxyl, carboxy, amino, $C_{1-6}$ alkyl, $C_{1-6}$ alkoxy, halogenated $C_{1-6}$ alkyl, halogenated $C_{1-6}$ alkoxy, $C_{1-6}$ alkylamino, ($C_{1-6}$ alkyl)$_2$ amino, 3-12 membered cycloalkyl, 3-12 membered heterocyclyl, aryl and 5-10 membered heteroaryl;
$R_3$ is selected from hydrogen, hydroxy, amino, carboxy, cyano, nitro, halogen, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonyl, $C_{1-6}$ alkylsulfonyl, $C_{1-6}$ alkylthio , 3-12 membered cycloalkyl, 3-12 membered cycloalkenyl, 3-12 membered heterocyclyl, wherein the amino, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonyl, $C_{1-6}$ alkylsulfonyl, $C_{1-6}$ alkylthio , 3-12 membered cycloalkyl, 3-12 membered cycloalkenyl, 3-12 membered heterocyclyl are unsubstituted or optionally substituted by one or more groups selected from hydroxyl, amino, carboxyl, cyano, nitro, halogen, $C_{1-6}$ alkyl, $C_{1-6}$ alkoxy, $C_{1-6}$ alkoxy$C_{1-6}$ alkoxy, $C_{1-6}$ alkylamino, ($C_{1-6}$ alkyl)$_2$ amino, $C_{1-6}$ alkylcarbonylamino and $C_{1-6}$ alkylsulfonylamino;
m is 0, 1, 2 or 3;
$R_4$, $R_5$ and $R_6$ are each independently selected from hydrogen, halogen, hydroxy, amino, carboxy, cyano, nitro,$C_{1-6}$ alkyl, $C_{2-8}$ alkenyl, $C_{2-8}$ alkynyl, and halogenated $C_{1-6}$ alkyl;
preferably, at least one of $X_1$ and $X_2$ is N.

**11.** The compound or the pharmaceutically acceptable salt or the isomer thereof according to claim 1 or 2, when L is-$(CH_2)$p-, p is 1, and n is 1,wherein the general formula (I) has a structure of general formula (IV),

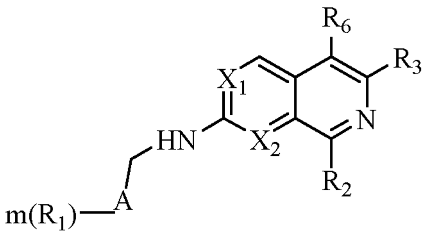

(IV)

$X_1$ is selected from N or $CR_4$;
$X_2$ is selected from N or $CR_5$;
A is selected from 3-12 membered cycloalkyl, 3-12 membered cycloalkenyl, 3-12 membered heterocyclyl, aryl, 5-10 membered heteroaryl, wherein the heteroatoms of the 3-12 membered heterocyclyl are selected from one of O, S, N or any combination thereof, C atom can be optionally oxidized to C(O), S atom can be optionally oxidized to S(O) or $S(O)_2$, the heteroatoms of the 5-10 membered heteroaryl are selected from one of O, S, and N or any combination thereof;
$R_1$ is selected from hydrogen, halogen, hydroxyl, carboxyl, amino, $C_{1-6}$ alkyl, $C_{1-6}$ alkoxy, $C_{1-6}$ alkylamino, $(C_{1-6}$ alkyl$)_2$ amino, halogenated $C_{1-6}$ alkyl , halogenated $C_{1-6}$ alkoxy, $C_{2-8}$ alkenyl, $C_{2-8}$ alkynyl, $C_{1-6}$ alkylcarbonylamino, $C_{1-6}$ alkylaminocarbonyl, $(C_{1-6}$ alkyl$)_2$ aminocarbonyl, $C_{1-6}$ alkoxycarbonyl, $C_{1-6}$ alkylcarbonyl, $C_{1-6}$ alkylsulfonyl, aminosulfonyl, $C_{1-6}$ alkylthio, 3-12 membered cycloalkyl, 3-12 membered heterocyclyl, 3-12 membered cycloalkenyl, wherein the heteroatoms of the 3-12 membered heterocyclyl are selected from one of O, S, N or any combination thereof, C atom can be optionally oxidized to C(O), S atom can be optionally oxidized to S(O) or $S(O)_2$, the heteroatoms of the 5-10 membered heteroaryl are selected from one of O, S, and N or any combination thereof, wherein the amino, $C_{1-6}$ alkyl, $C_{1-6}$ alkoxy, $C_{1-6}$ alkylamino, $(C_{1-6}$ alkyl$)_2$ amino, halogenated $C_{1-6}$ alkyl , halogenated $C_{1-6}$ alkoxy, $C_{2-8}$ alkenyl, $C_{2-8}$ alkynyl, $C_{1-6}$ alkylcarbonylamino, $C_{1-6}$ alkylaminocarbonyl, $(C_{1-6}$ alkyl$)_2$ aminocarbonyl, $C_{1-6}$ alkoxycarbonyl,$C_{1-6}$ alkylcarbonyl, $C_{1-6}$ alkylsulfonyl, aminosulfonyl, $C_{1-6}$ alkylthio, 3-12 membered cycloalkyl, 3-12 membered heterocyclyl, 3-12 membered cycloalkenyl are unsubstituted or optionally substituted by one or more groups selected from hydroxyl, amino, carboxy, cyano, nitro, halogen, $C_{1-6}$ alkyl, $C_{1-6}$ alkoxy, halogenated $C_{1-6}$ alkyl, halogenated $C_{1-6}$ alkoxy, $C_{1-6}$ alkylamino, $(C_{1-6}$ alkyl$)_2$ amino, $C_{1-6}$ alkylsulfonyl, HS(O)(=NH)-, $C_{1-6}$ alkyl -S(O)(=NH)-, 3-12 membered cycloalkyl, 3-12 membered heterocyclyl;
$R_2$ is selected from halogen, cyano, hydroxyl, carboxyl, amino, $C_{1-6}$ alkyl, $C_{1-6}$ alkoxy, $C_{1-6}$ alkylamino, $(C_{1-6}$ alkyl$)_2$ amino, 3-12 membered cycloalkyl, 3-12 membered heterocyclyl, 3-12 membered cycloalkenyl, 3-12 membered heterocyclyloxy, 3-12 membered cycloalkyl amino, 3-12 membered heterocyclyl-$CH_2$-amino, wherein the amino, $C_{1-6}$ alkyl, $C_{1-6}$ alkoxy, $C_{1-6}$ alkylamino, $(C_{1-6}$ alkyl$)_2$ amino, 3-12 membered cycloalkyl, 3-12 membered heterocyclyl, 3-12 membered cycloalkenyl, 3-12 membered heterocyclyloxy, 3-12 membered cycloalkyl amino, 3-12 membered heterocyclyl-$CH_2$-amino are unsubstituted or optionally substituted by one or more groups selected from halogen, cyano, hydroxyl, carboxy, amino, $C_{1-6}$ alkyl, $C_{1-6}$ alkoxy, halogenated $C_{1-6}$ alkyl, halogenated $C_{1-6}$ alkoxy, $C_{1-6}$ alkylamino, $(C_{1-6}$ alkyl$)_2$ amino, 3-12 membered cycloalkyl, 3-12 membered heterocyclyl, aryl and 5-10 membered heteroaryl;
$R_3$ is selected from hydrogen, hydroxy, amino, carboxy, cyano, nitro, halogen, $C_{1-6}$ alkyl, $C_{1-6}$ alkoxy, $C_{1-6}$ alkylamino, $(C_{1-6}$ alkyl$)_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonyl, $C_{1-6}$ alkylsulfonyl, $C_{1-6}$ alkylthio , 3-12 membered cycloalkyl, 3-12 membered cycloalkenyl, 3-12 membered heterocyclyl, wherein the amino, $C_{1-6}$ alkyl, $C_{1-6}$ alkoxy, $C_{1-6}$ alkylamino, $(C_{1-6}$ alkyl$)_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonyl, $C_{1-6}$ alkylsulfonyl, $C_{1-6}$ alkylthio , 3-12 membered cycloalkyl, 3-12 membered cycloalkenyl, 3-12 membered heterocyclyl are unsubstituted or optionally substituted by one or more groups selected from hydroxyl, amino, carboxyl, cyano, nitro, halogen, $C_{1-6}$ alkyl, $C_{1-6}$ alkoxy, $C_{1-6}$ alkoxy$C_{1-6}$ alkoxy, $C_{1-6}$ alkylamino, $(C_{1-6}$ alkyl$)_2$ amino, $C_{1-6}$ alkylcarbonylamino and $C_{1-6}$ alkylsulfonylamino;
m is 0, 1, 2 or 3;
$R_4$, $R_5$ and $R_6$ are each independently selected from hydrogen, halogen, hydroxy, amino, carboxy, cyano, nitro, $C_{1-6}$ alkyl, $C_{2-8}$ alkenyl, $C_{2-8}$ alkynyl, and halogenated $C_{1-6}$ alkyl;
preferably, at least one of $X_1$ and $X_2$ is N.

**12.** The compound or the pharmaceutically acceptable salt or the isomer thereof according to claim 11,

$X_1$ is selected from N or $CR_4$;
$X_2$ is selected from N or $CR_5$;

A is selected from 3-12 membered cycloalkyl, 3-12 membered cycloalkenyl, 3-12 membered heterocyclyl, aryl, 5-10 membered heteroaryl, wherein the heteroatoms of the 3-12 membered heterocyclyl are selected from one of O, S, N or any combination thereof, C atom can be optionally oxidized to C(O), S atom can be optionally oxidized to S(O) or $S(O)_2$, the heteroatoms of the 5-10 membered heteroaryl are selected from one of O, S, and N or any combination thereof;

$R_1$ is selected from hydrogen, halogen, hydroxyl, carboxyl, amino, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl , halogenated $C_{1-6}$ alkoxy, $C_{2-8}$ alkenyl, $C_{2-8}$ alkynyl, $C_{1-6}$ alkylcarbonylamino, $C_{1-6}$ alkylaminocarbonyl, ($C_{1-6}$ alkyl)$_2$ aminocarbonyl, $C_{1-6}$ alkoxycarbonyl, $C_{1-6}$ alkylcarbonyl, $C_{1-6}$ alkylsulfonyl, aminosulfonyl, $C_{1-6}$ alkylthio, 3-12 membered cycloalkyl, 3-12 membered heterocyclyl, 3-12 membered cycloalkenyl, wherein the heteroatoms of the 3-12 membered heterocyclyl are selected from one of O, S, N or any combination thereof, C atom can be optionally oxidized to C(O), S atom can be optionally oxidized to S(O) or $S(O)_2$, the heteroatoms of the 5-10 membered heteroaryl are selected from one of O, S, and N or any combination thereof, wherein the amino, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl , halogenated $C_{1-6}$ alkoxy, $C_{2-8}$ alkenyl, $C_{2-8}$ alkynyl, $C_{1-6}$ alkylcarbonylamino, $C_{1-6}$ alkylaminocarbonyl, ($C_{1-6}$ alkyl)$_2$ aminocarbonyl, $C_{1-6}$ alkoxycarbonyl,$C_{1-6}$ alkylcarbonyl, $C_{1-6}$ alkylsulfonyl, aminosulfonyl, $C_{1-6}$ alkylthio, 3-12 membered cycloalkyl, 3-12 membered heterocyclyl, 3-12 membered cycloalkenyl are unsubstituted or optionally substituted by one or more groups selected from hydroxyl, amino, carboxy, cyano, nitro, halogen, $C_{1-6}$ alkyl, $C_{1-6}$ alkoxy, halogenated $C_{1-6}$ alkyl, halogenated $C_{1-6}$ alkoxy, $C_{1-6}$ alkylamino, ($C_{1-6}$ alkyl)$_2$ amino, $C_{1-6}$ alkylsulfonyl, 3-12 membered cycloalkyl, 3-12 membered heterocyclyl;

$R_2$ is selected from halogen, cyano, hydroxyl, carboxyl, amino, $C_{1-6}$ alkyl, $C_{1-6}$ alkoxy, $C_{1-6}$ alkylamino, ($C_{1-6}$ alkyl)$_2$ amino, 3-12 membered cycloalkyl, 3-12 membered heterocyclyl, 3-12 membered cycloalkenyl, wherein the amino, $C_{1-6}$ alkyl, $C_{1-6}$ alkoxy, $C_{1-6}$ alkylamino, ($C_{1-6}$ alkyl)$_2$ amino, 3-12 membered cycloalkyl, 3-12 membered heterocyclyl, 3-12 membered cycloalkenyl are unsubstituted or optionally substituted by one or more groups selected from halogen, cyano, hydroxyl, carboxy, amino, $C_{1-6}$ alkyl, $C_{1-6}$ alkoxy, halogenated $C_{1-6}$ alkyl, halogenated $C_{1-6}$ alkoxy, $C_{1-6}$ alkylamino, ($C_{1-6}$ alkyl)$_2$ amino, 3-12 membered cycloalkyl, 3-12 membered heterocyclyl, aryl and 5-10 membered heteroaryl;

$R_3$ is selected from hydrogen, hydroxy, amino, carboxy, cyano, nitro, halogen, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonyl, $C_{1-6}$ alkylsulfonyl, $C_{1-6}$ alkylthio , 3-12 membered cycloalkyl, 3-12 membered cycloalkenyl, 3-12 membered heterocyclyl, wherein the amino, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonyl, $C_{1-6}$ alkylsulfonyl, $C_{1-6}$ alkylthio , 3-12 membered cycloalkyl, 3-12 membered cycloalkenyl, 3-12 membered heterocyclyl are unsubstituted or optionally substituted by one or more groups selected from hydroxyl, amino, carboxyl, cyano, nitro, halogen, $C_{1-6}$ alkyl, $C_{1-6}$ alkoxy, $C_{1-6}$ alkoxy$C_{1-6}$ alkoxy, $C_{1-6}$ alkylamino, ($C_{1-6}$ alkyl)$_2$ amino, $C_{1-6}$ alkylcarbonylamino and $C_{1-6}$ alkylsulfonylamino;

m is 0, 1, 2 or 3;

$R_4$, $R_5$ and $R_6$ are each independently selected from hydrogen, halogen, hydroxy, amino, carboxy, cyano, nitro, $C_{1-6}$ alkyl, $C_{2-8}$ alkenyl, $C_{2-8}$ alkynyl, and halogenated $C_{1-6}$ alkyl;

preferably, at least one of $X_1$ and $X_2$ is N.

**13.** The compound or the pharmaceutically acceptable salt or the isomer thereof according to claim 1 or 2, A is selected from the group consisting of 3-8 membered cycloalkyl, 6-11 membered ortho-fused cycloalkyl, 6-11 membered bridged cycloalkyl, 7-12 membered spirocycloalkyl, 3-8 membered monocycloalkenyl, 7-11 membered spiro cycloalkenyl, 7-11 membered ortho-fused cycloalkenyl, 6-11 membered bridged cycloalkenyl, 3-8 membered heterocyclyl, 6-12 membered ortho-fused heterocyclyl, 6-12 membered spiro heterocyclyl, 6-12 membered bridged heterocyclyl, 5-10 membered heteroaryl, wherein the heteroatoms of 3-8 membered heterocyclyl, 6-12 membered ortho-fused heterocyclyl, 6-12 membered spiro heterocyclyl, 6-12 membered bridged heterocyclyl are selected from one of O, S, N or any combination thereof, the C atom can be optionally oxidized to C(O), and the S atom can be optionally oxidized to S(O) or $S(O)_2$, The heteroatoms of the 5-10 membered heteroaryl are selected from one of O, S, and N or any combination thereof.

**14.** The compound or the pharmaceutically acceptable salt or the isomer thereof according to claim 13, A is selected from

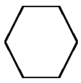, 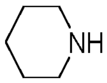, 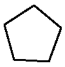, 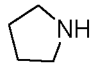, 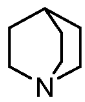, 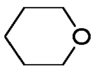, 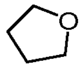, 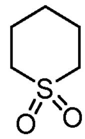,

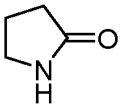, 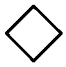, 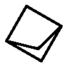, 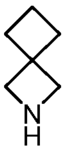, 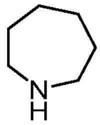, 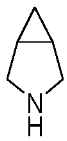, 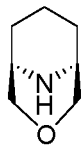, 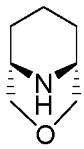, 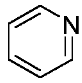, 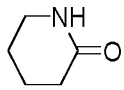,

or

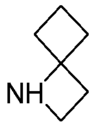

**15.** The compound or the pharmaceutically acceptable salt or the isomer thereof according to claim 1 or 2,

$X_1$ is N;
$X_2$ is $CR_5$;
L is selected from bond or -C(O)-;
A is selected from 3-12 membered heterocyclyl, 3-12 membered cycloalkyl, or 5-10 membered heteroaryl, wherein the heteroatoms of the 3-12 membered heterocyclyl and 5-10 membered heteroaryl are N;
$R_1$ is selected from hydrogen, hydroxyl, amino, $C_{1-6}$ alkyl, or $C_{1-6}$ alkylcarbonylamino, wherein the amino, $C_{1-6}$ alkyl, or $C_{1-6}$ alkylcarbonylamino are unsubstituted or optionally substituted by one or more groups selected from HS(O)(=NH)-, $C_{1-6}$ alkyl-S(O)(=NH)-;
$R_2$ is selected from amino, $C_{1-6}$ alkylamino, ($C_{1-6}$ alkyl)$_2$ amino, $C_{1-6}$ alkoxy, 3-12 membered heterocyclyloxy, 3-12 membered heterocyclyl, or 3-12 membered cycloalkenyl, 3-12 membered cycloalkyl amino;
wherein the heteroatoms of the 3-12 membered heterocyclyl and 3-12 membered heterocyclyloxy are N, O, or any combination thereof,
wherein the amino, $C_{1-6}$ alkylamino, ($C_{1-6}$ alkyl)$_2$ amino, $C_{1-6}$ alkoxy, 3-12 membered heterocyclyloxy, 3-12 membered heterocyclyl, or 3-12 membered cycloalkenyl, 3-12 membered cycloalkyl amino are unsubstituted or optionally substituted by one or more groups selected from 3-12 membered cycloalkyl, 3-12 membered heterocyclyl, aryl, $C_{1-6}$ alkyl;
$R_3$ is selected from cyano, or halogenated $C_{1-6}$ alkyl;
$R_5$ and $R_6$ are each independently hydrogen;
n is 0 or 1;
m is 0 or 1.

**16.** The compound or the pharmaceutically acceptable salt or the isomer thereof according to claim 1 or 2,

$X_1$ is selected from N or $CR_4$;
$X_2$ is selected from N or $CR_5$;
L is selected from bond, -C(O)-, -$(CH_2)$p-;
A is bond;
$R_1$ is selected from hydrogen, halogen, hydroxyl, carboxyl, amino, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl , halogenated $C_{1-6}$ alkoxy, $C_{2-8}$ alkenyl, $C_{2-8}$ alkynyl, $C_{1-6}$ alkylcarbonylamino, $C_{1-6}$ alkylaminocarbonyl, ($C_{1-6}$ alkyl)$_2$ aminocarbonyl, $C_{1-6}$ alkoxycarbonyl, $C_{1-6}$ alkylcarbonyl, $C_{1-6}$ alkylsulfonyl, aminosulfonyl, $C_{1-6}$ alkylthio, 3-12 membered cycloalkyl, 3-12 membered heterocyclyl, 3-12 membered cycloalkenyl, wherein the heteroatoms of the 3-12 membered heterocyclyl are selected from one of O, S, N or any combination thereof, C atom can be optionally oxidized to C(O), S atom can be optionally oxidized to S(O) or S(O)$_2$, the heteroatoms of the 5-10 membered heteroaryl are selected from one of O, S, and N or any combination thereof, wherein the amino, $C_{1-6}$ alkyl, $C_{1-6}$ alkoxy, $C_{1-6}$ alkylamino, ($C_{1-6}$ alkyl)$_2$ amino, hal-

ogenated $C_{1-6}$ alkyl , halogenated $C_{1-6}$ alkoxy, $C_{2-8}$ alkenyl, $C_{2-8}$ alkynyl, $C_{1-6}$ alkylcarbonylamino, $C_{1-6}$ alkylaminocarbonyl, ($C_{1-6}$ alkyl)$_2$ aminocarbonyl, $C_{1-6}$ alkoxycarbonyl,$C_{1-6}$ alkylcarbonyl, $C_{1-6}$ alkylsulfonyl, aminosulfonyl, $C_{1-6}$ alkylthio, 3-12 membered cycloalkyl, 3-12 membered heterocyclyl, 3-12 membered cycloalkenyl are unsubstituted or optionally substituted by one or more groups selected from hydroxyl, amino, carboxy, cyano, nitro, halogen, $C_{1-6}$ alkyl, $C_{1-6}$ alkoxy, halogenated $C_{1-6}$ alkyl, halogenated $C_{1-6}$ alkoxy, $C_{1-6}$ alkylamino, ($C_{1-6}$ alkyl)$_2$ amino, $C_{1-6}$ alkylsulfonyl, 3-12 membered cycloalkyl, 3-12 membered heterocyclyl;

$R_2$ is selected from halogen, cyano, hydroxyl, carboxyl, amino, $C_{1-6}$ alkyl, $C_{1-6}$ alkoxy, $C_{1-6}$ alkylamino, ($C_{1-6}$ alkyl)$_2$ amino, 3-12 membered cycloalkyl, 3-12 membered heterocyclyl, 3-12 membered cycloalkenyl, wherein the amino, $C_{1-6}$ alkyl, $C_{1-6}$ alkoxy, $C_{1-6}$ alkylamino, ($C_{1-6}$ alkyl)$_2$ amino, 3-12 membered cycloalkyl, 3-12 membered heterocyclyl, 3-12 membered cycloalkenyl are unsubstituted or optionally substituted by one or more groups selected from halogen, cyano, hydroxyl, carboxy, amino, $C_{1-6}$ alkyl, $C_{1-6}$ alkoxy, halogenated $C_{1-6}$ alkyl, halogenated $C_{1-6}$ alkoxy, $C_{1-6}$ alkylamino, ($C_{1-6}$ alkyl)$_2$ amino, 3-12 membered cycloalkyl, 3-12 membered heterocyclyl, aryl and 5-10 membered heteroaryl;

$R_3$ is selected from hydrogen, hydroxy, amino, carboxy, cyano, nitro, halogen, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonyl, $C_{1-6}$ alkylsulfonyl, $C_{1-6}$ alkylthio , 3-12 membered cycloalkyl, 3-12 membered cycloalkenyl, 3-12 membered heterocyclyl, wherein the amino, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonyl, $C_{1-6}$ alkylsulfonyl, $C_{1-6}$ alkylthio , 3-12 membered cycloalkyl, 3-12 membered cycloalkenyl, 3-12 membered heterocyclyl are unsubstituted or optionally substituted by one or more groups selected from hydroxyl, amino, carboxyl, cyano, nitro, halogen, $C_{1-6}$ alkyl, $C_{1-6}$ alkoxy, $C_{1-6}$ alkoxy$C_{1-6}$ alkoxy, $C_{1-6}$ alkylamino, ($C_{1-6}$ alkyl)$_2$ amino, $C_{1-6}$ alkylcarbonylamino and $C_{1-6}$ alkylsulfonylamino;

n is 0 or 1;

m is 0, 1, 2 or 3;

p is 1, 2 or 3;

$R_4$, $R_5$ and $R_6$ are each independently selected from hydrogen, halogen, hydroxy, amino, carboxy, cyano, nitro, $C_{1-6}$ alkyl, $C_{2-8}$ alkenyl, $C_{2-8}$ alkynyl, and halogenated $C_{1-6}$ alkyl.

**17.** The compound or the pharmaceutically acceptable salt or the isomer thereof according to claim 1 or 2,

$X_1$ is N;

$X_2$ is N;

L is bond;

A is selected from 3-12 membered cycloalkyl, or 3-12 membered heterocyclyl,

wherein the heteroatoms of the 3-12 membered heterocyclyl are N,

$R_1$ is selected from hydrogen, hydroxyl, $C_{1-6}$ alkyl, $C_{1-6}$ alkoxy, amino, ($C_{1-6}$ alkyl)$_2$ amino, $C_{1-6}$ alkylcarbonyl, or 3-12 membered heterocyclyl,

wherein the heteroatoms of the 3-12 membered heterocyclyl are selected from any one of O, N or any combination thereof,

wherein the $C_{1-6}$ alkyl, $C_{1-6}$ alkoxy, amino, ($C_{1-6}$ alkyl)$_2$ amino, $C_{1-6}$ alkylcarbonyl and 3-12 membered heterocyclyl are optionally substituted by any one or more groups selected from hydroxyl, amino, $C_{1-6}$ alkyl, ($C_{1-6}$ alkyl)$_2$ aminocarbonyl or $C_{1-6}$ alkyl carbonyl; $R_2$ is selected from amino, $C_{1-6}$ alkylamino, 3-12 membered heterocyclyl, or 3-12 membered cycloalkyl amino,

wherein the heteroatoms of the 3-12 membered heterocyclyl are selected from any one of O, N, any combination thereof,

wherein the amino, $C_{1-6}$ alkylamino, 3-12 membered heterocyclyl, or 3-12 membered cycloalkyl amino are optionally substituted by any one or more groups selected from $C_{1-6}$ alkyl, $C_{1-6}$ alkoxy, 3-12membered cycloalkyl, or 3-12 membered heterocyclyl;

$R_3$ is selected from cyano, $C_{1-6}$ alkyl, aminocarbonyl, hydrogen, hydroxy, amino, halogen, or $C_{1-6}$ alkoxy, wherein the $C_{1-6}$ alkyl and aminocarbonyl are optionally substituted by any one or more halogen;

$R_6$ is hydrogen;

n is 0;

m is 0, 1, 2 or 3;

p is 1, 2 or 3.

**18.** The compound or the pharmaceutically acceptable salt or the isomer thereof according to any one of claim 1,

$X_1$ is N;

$X_2$ is $CR_5$;
L is bond;
n is 0;
A is selected from 3-12 membered cycloalkyl, or 3-12 membered heterocyclyl; wherein the heteroatoms of the 3-12 membered heterocyclyl is N; the 3-12 membered cycloalkyl is substituted by amino;
$R_1$ is hydrogen;
$R_2$ is selected from amino, $C_{1-6}$ alkoxy or 3-12 membered heterocyclyloxy, wherein the amino is substituted by 3-12 membered heterocyclyl;
$R_3$ is selected from cyano or halogenated $C_{1-6}$ alkyl;
$R_5$ and $R_6$ are each independently hydrogen; and
m is 1.

**19.** A compound or the pharmaceutically acceptable salt or the isomer thereof, selected from the group consisting of:

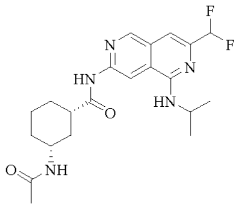 , 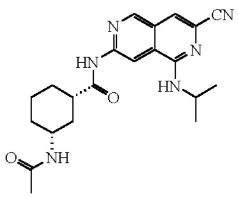 , 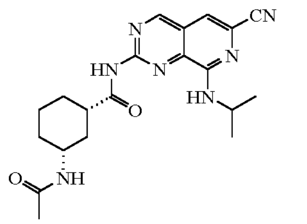 ,

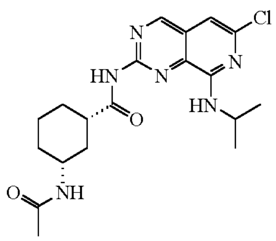 , 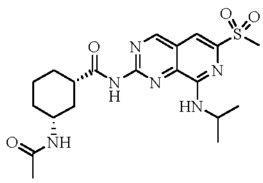 , 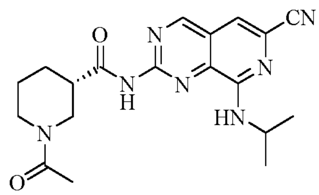 ,

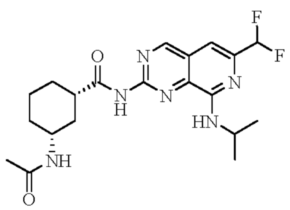 , 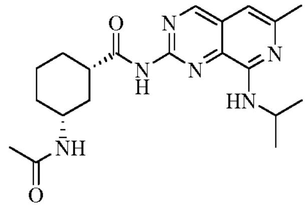 , 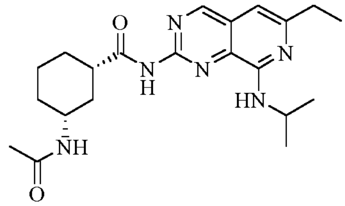 ,

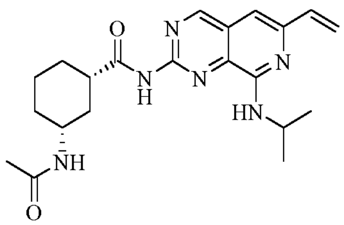 , 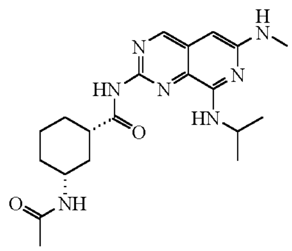 , 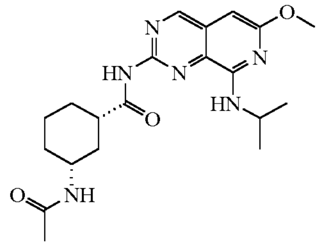 ,

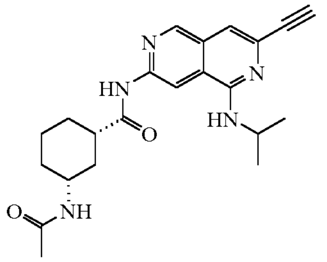
,
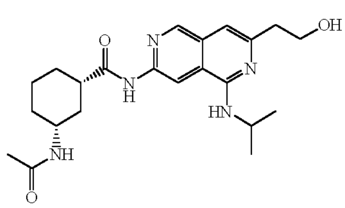
,
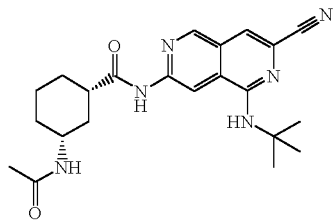
,

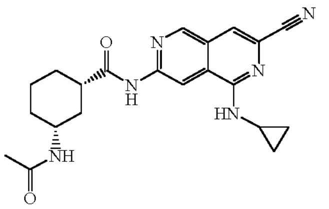
,
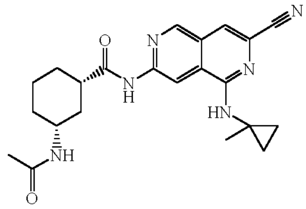
,
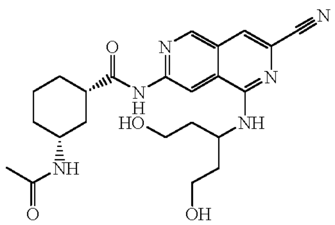
,

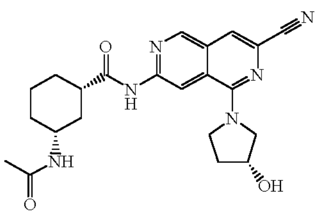
,
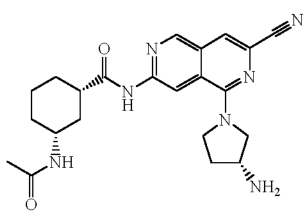
,
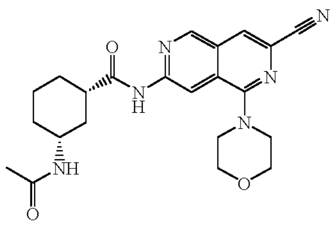
,

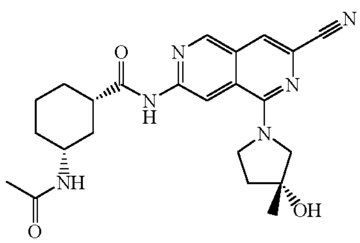
,
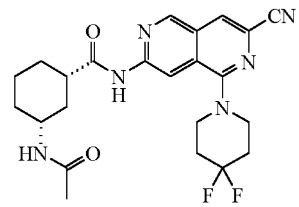
,
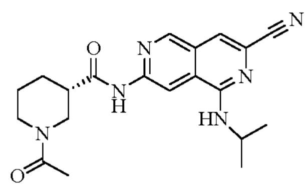
,

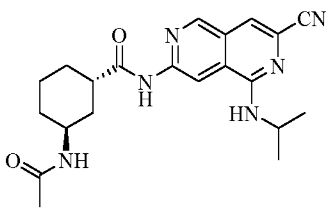
,
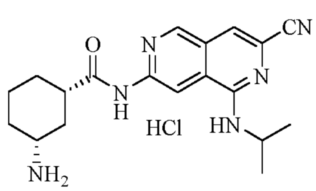
,
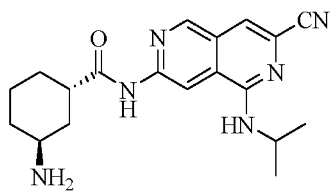
,

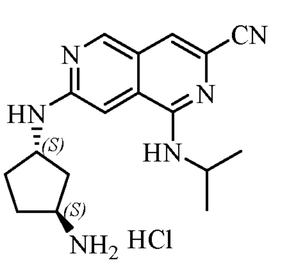
,
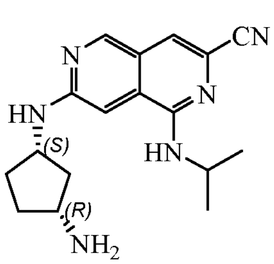
,
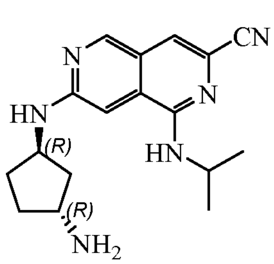
,

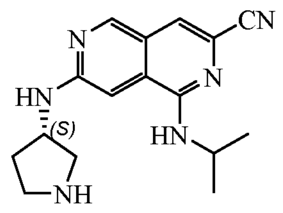,
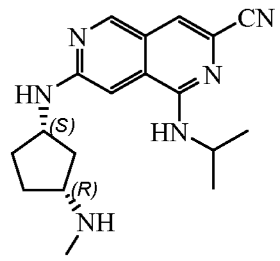,
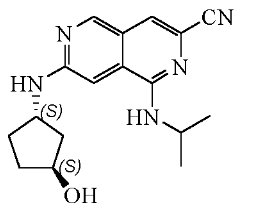,

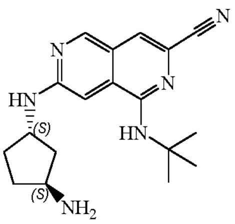,
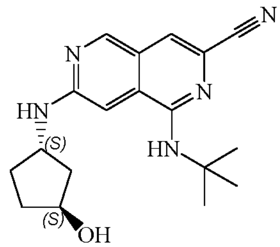,
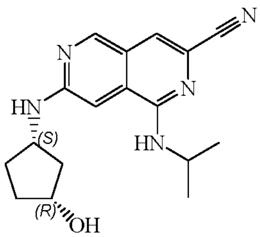,

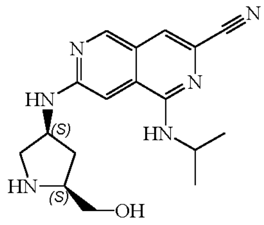,
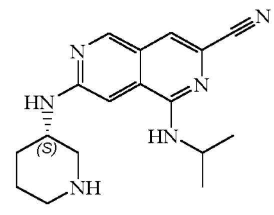,
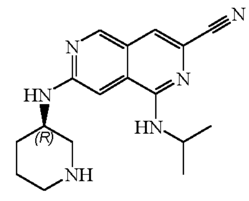,

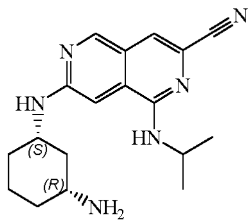,
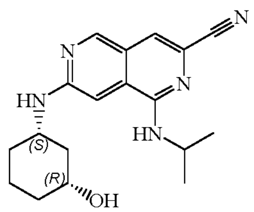,
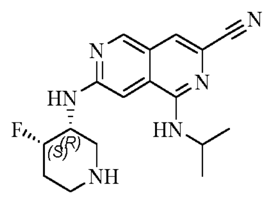,

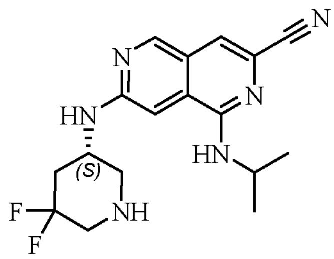,
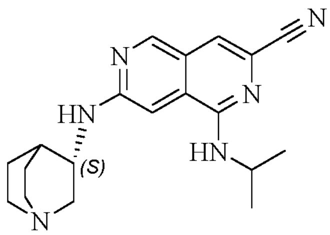,
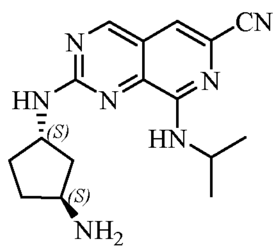,

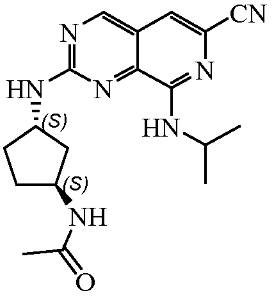,
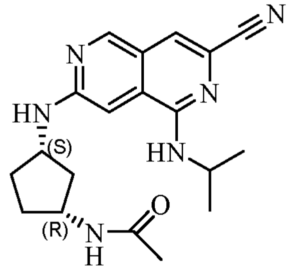,
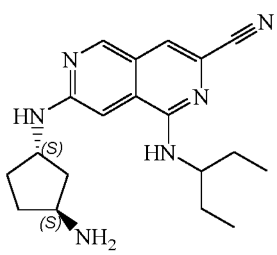,

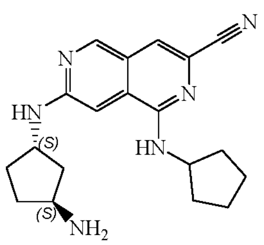 , 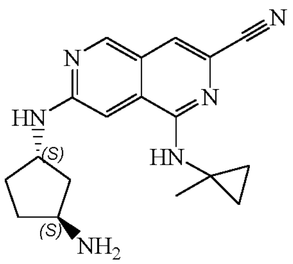 , 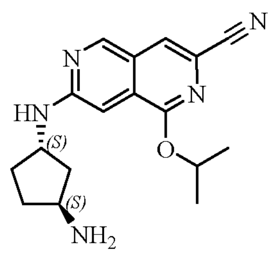 ,

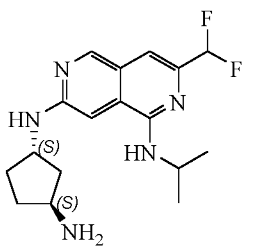 , 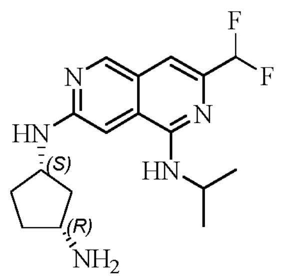 , 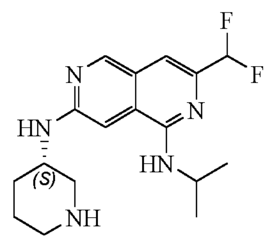 ,

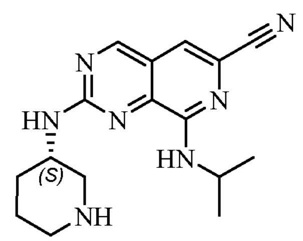 , 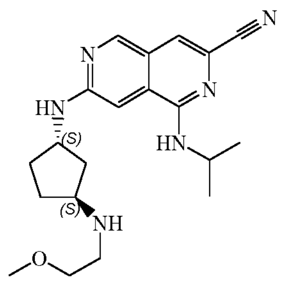 , 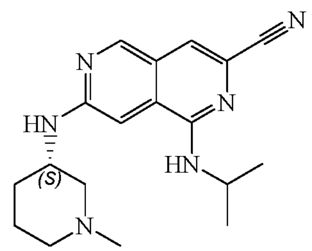 ,

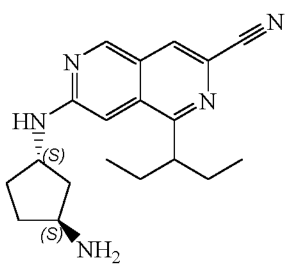 , 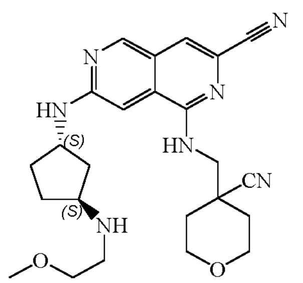 , 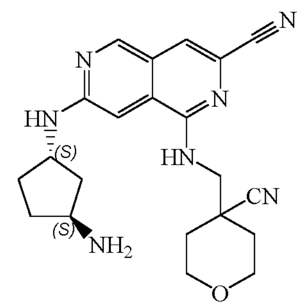 ,

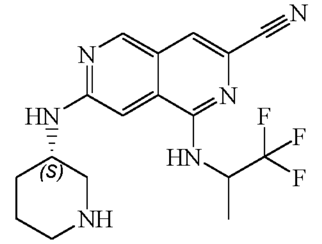 , 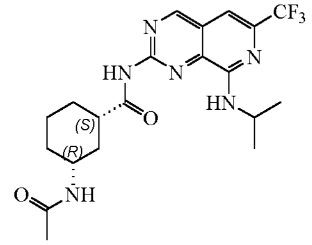 , 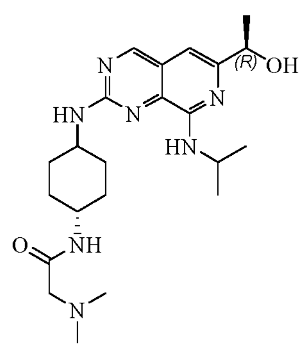 ,

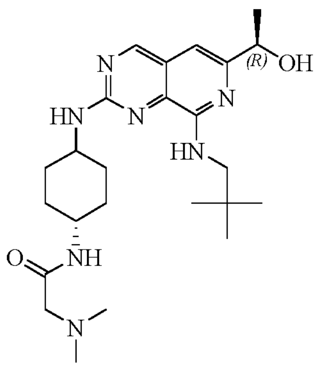 , 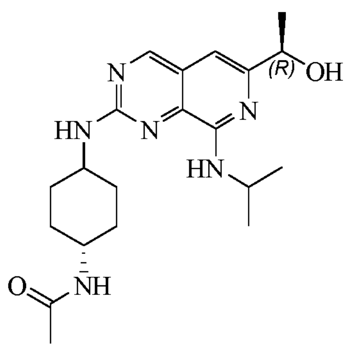 , 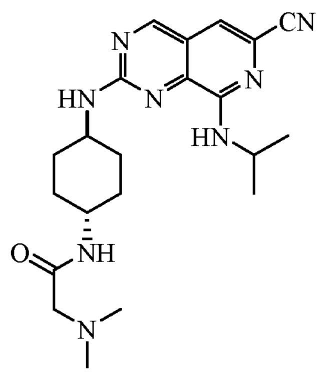 ,

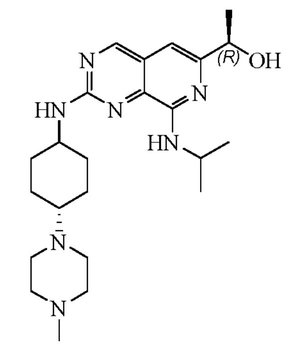 , 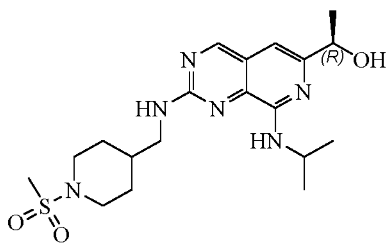 , 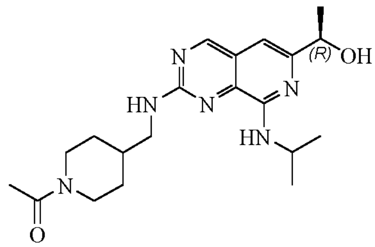 ,

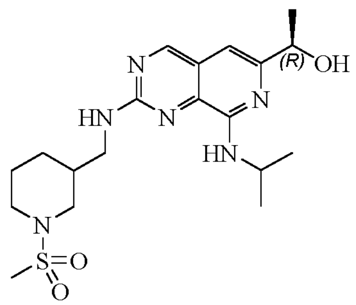 , 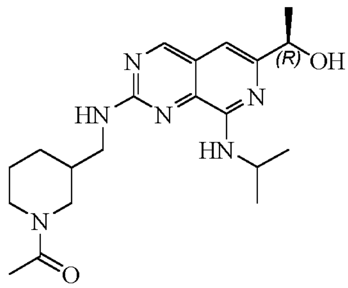 , 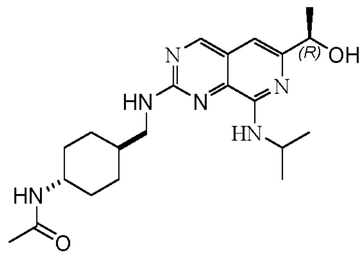 ,

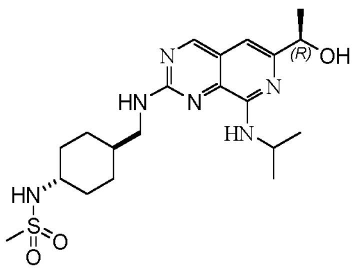 , 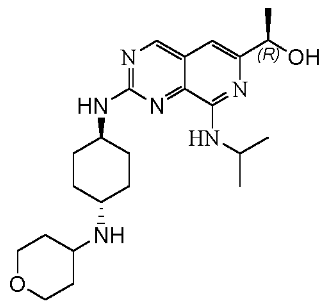 , 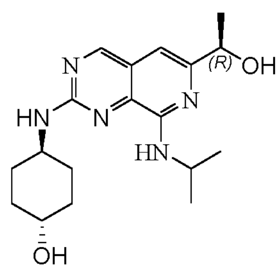 ,

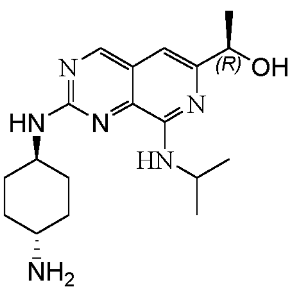 , 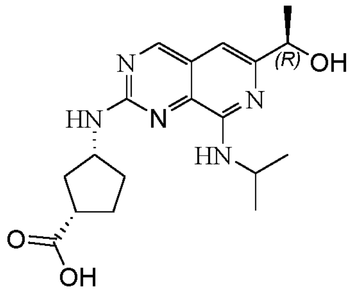 , 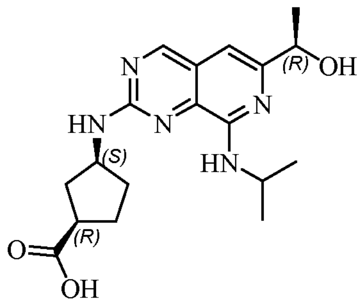 ,

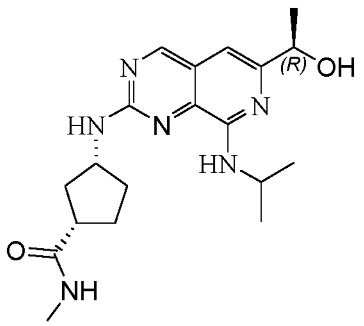 , 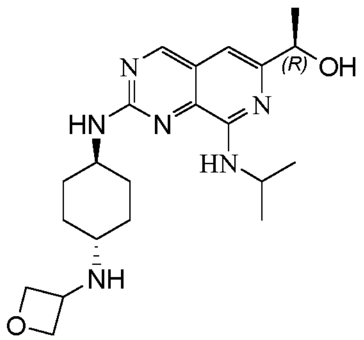 , 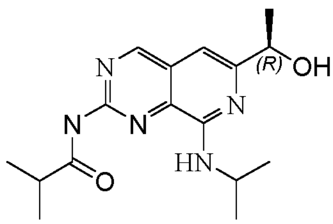 ,

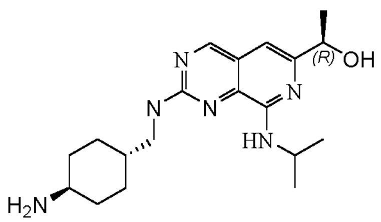 , 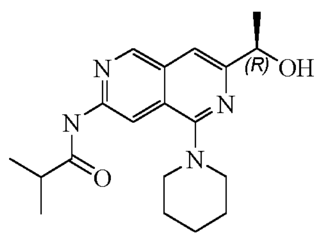 , 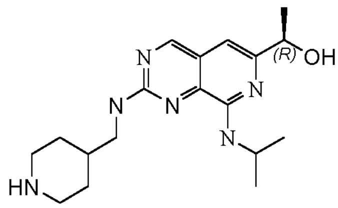 ,

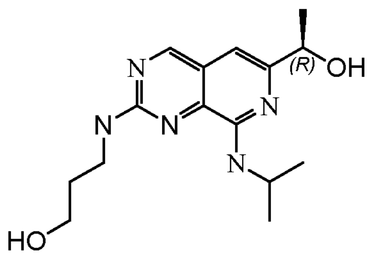 , 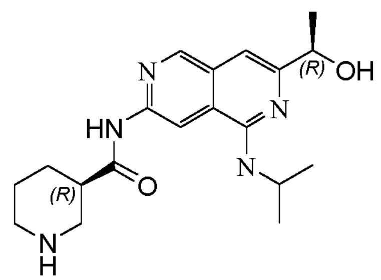 , 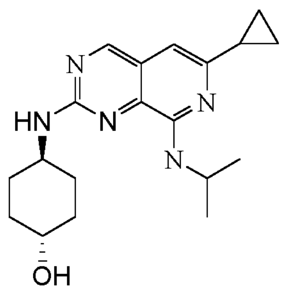 ,

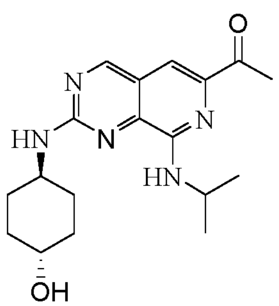 , 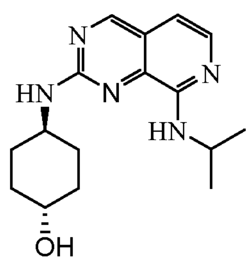 , 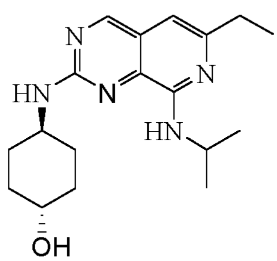 ,

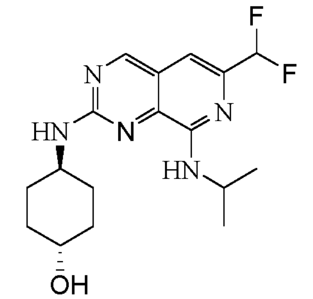
,
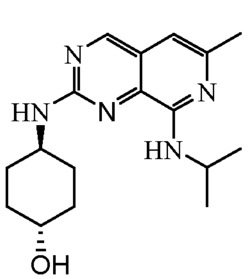
,
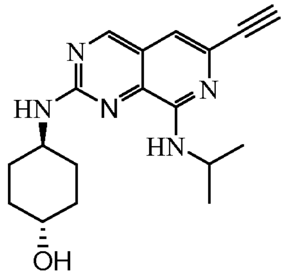
,
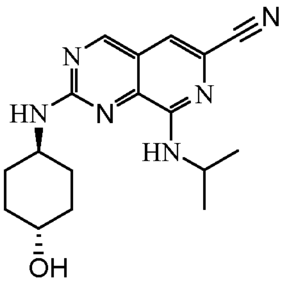
,
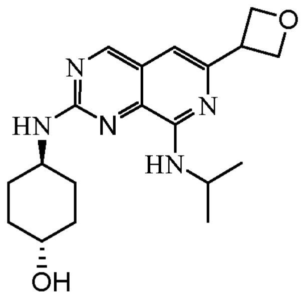
,
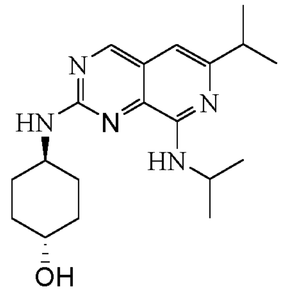
,
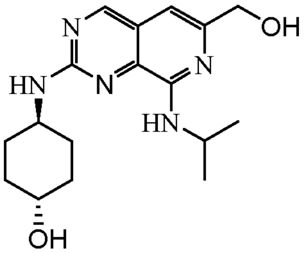
,
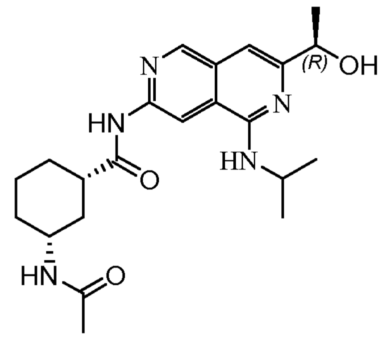
,
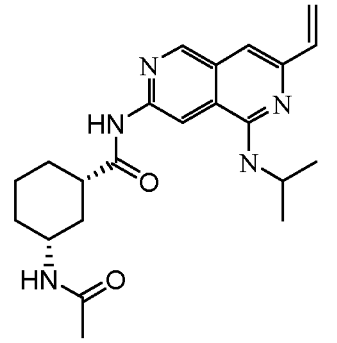
,
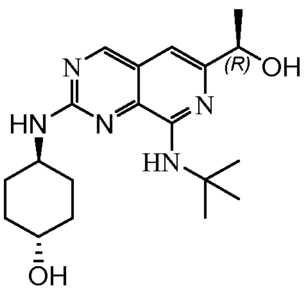
,
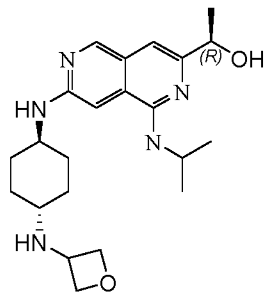
,
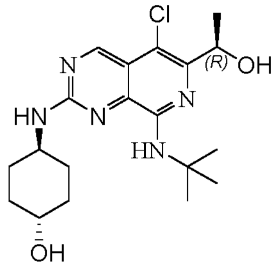
,
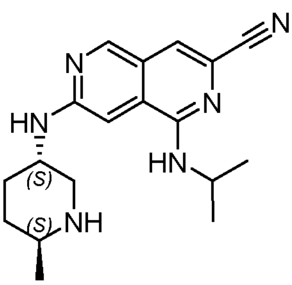
,
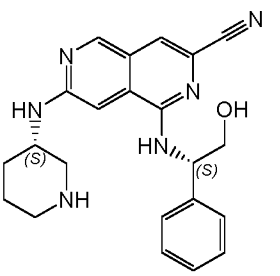
,
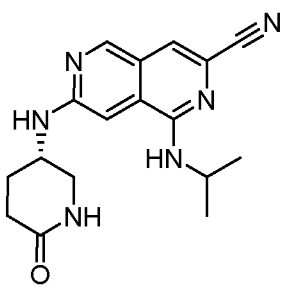
,

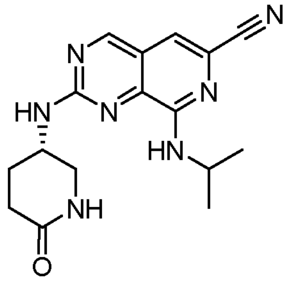 , 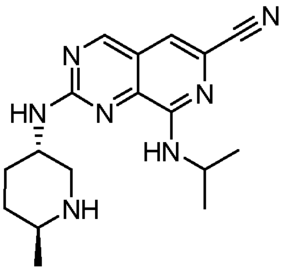 , 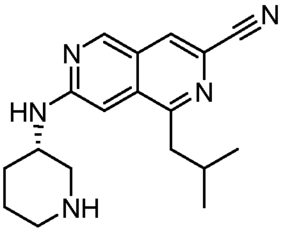 ,

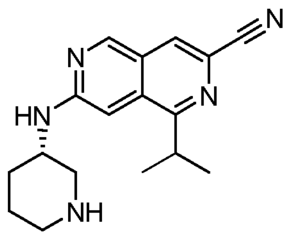 , 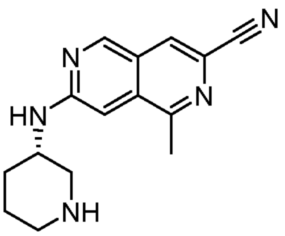 , 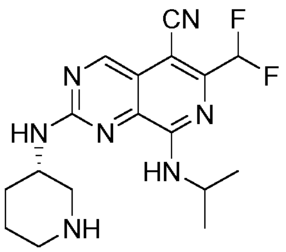 ,

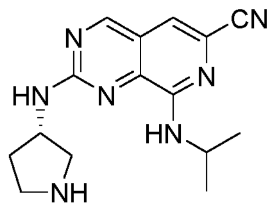 , 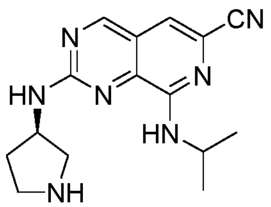 , 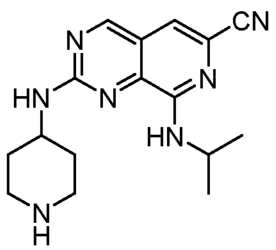 ,

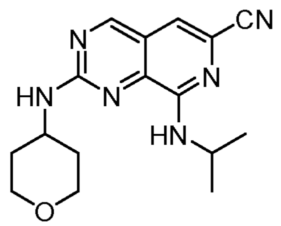 , 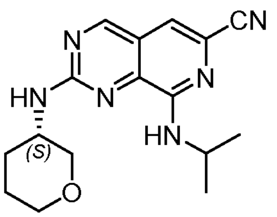 , 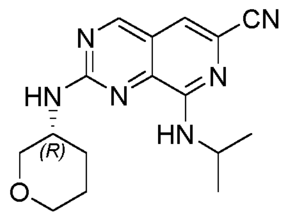 ,

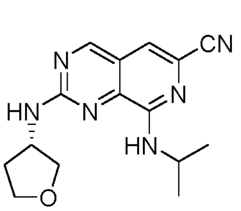 , 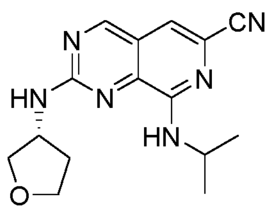 , 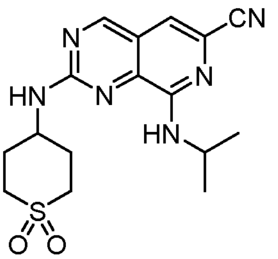 ,

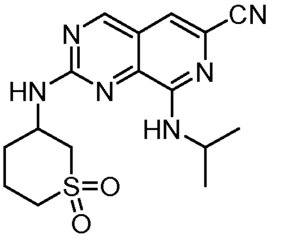 , 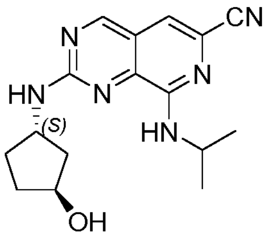 , 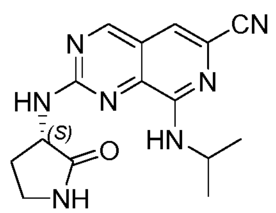 ,

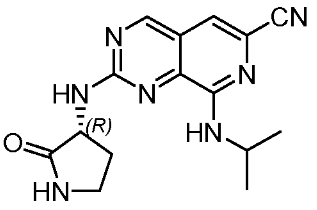,
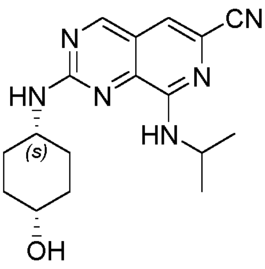,
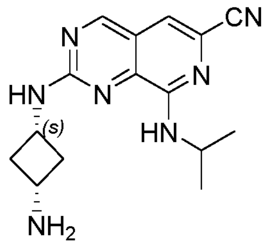,

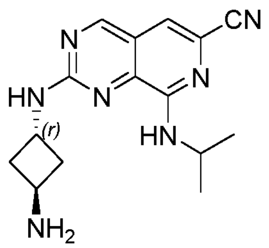,
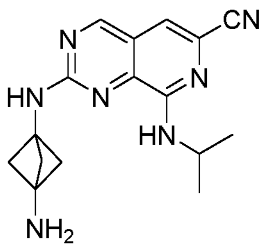,
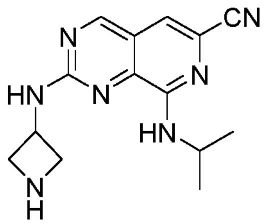,

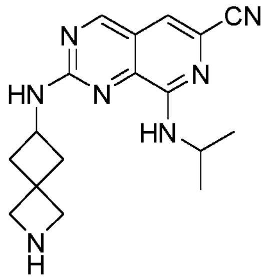,
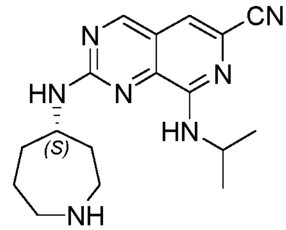,
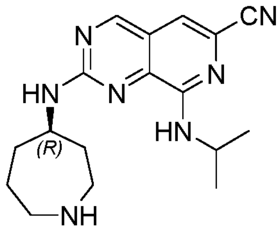,

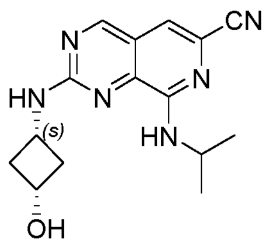,
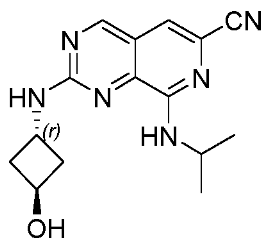,
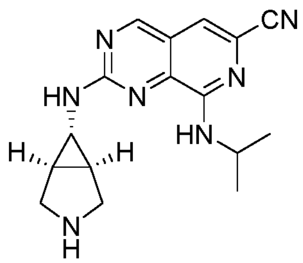,

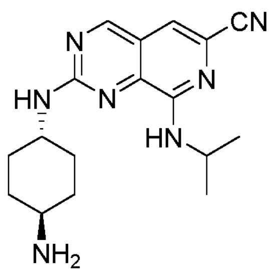,
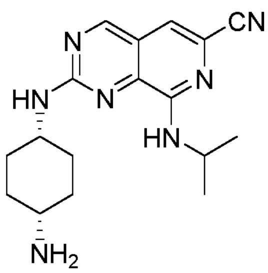,
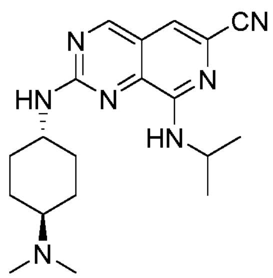,

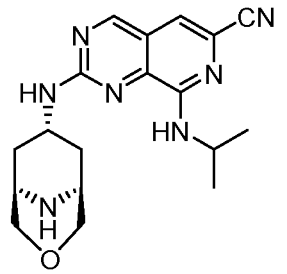,
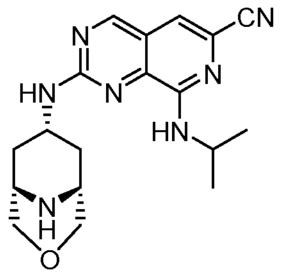,
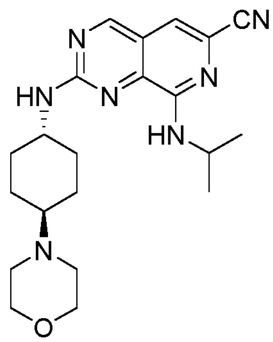,

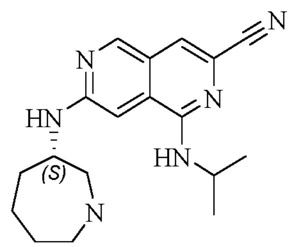,
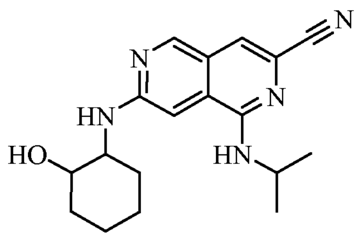,
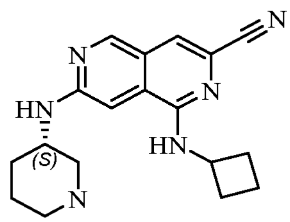,

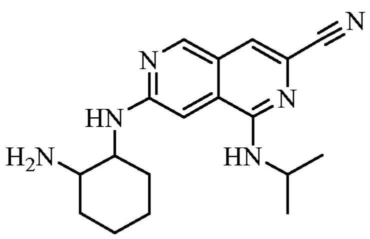,
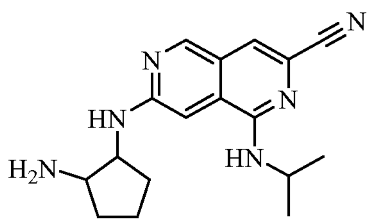,
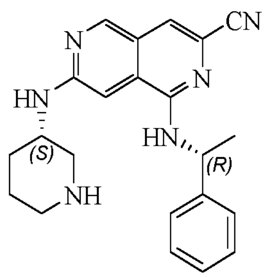,

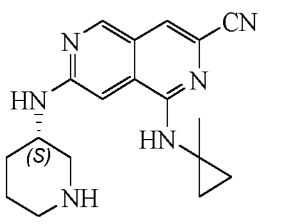,
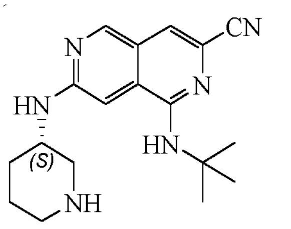,
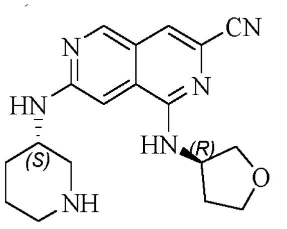,

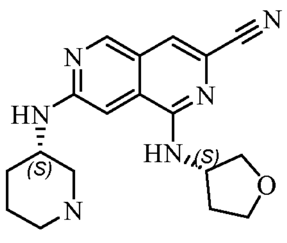,
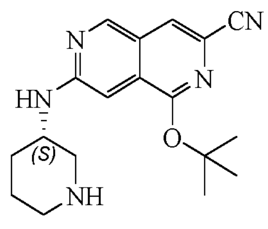,
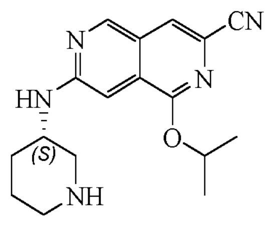,

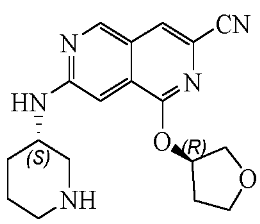 , 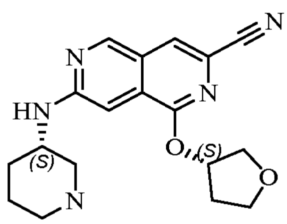 , 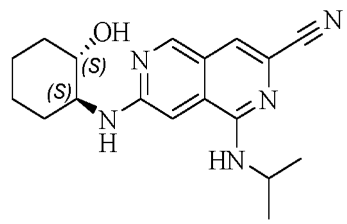 ,

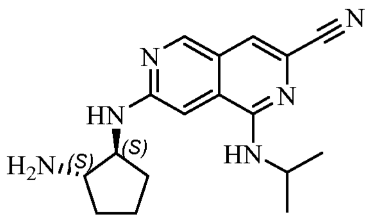 , 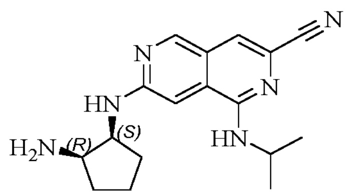 , 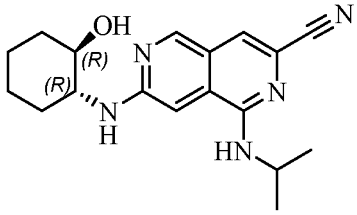 ,

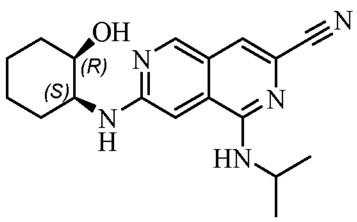 , 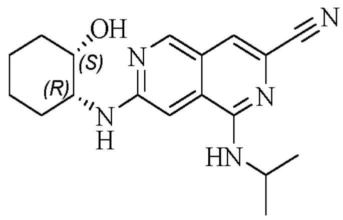 , 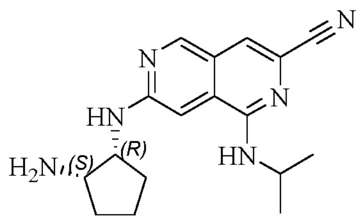 ,

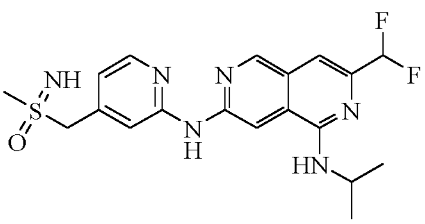 , 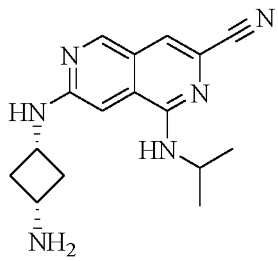 , 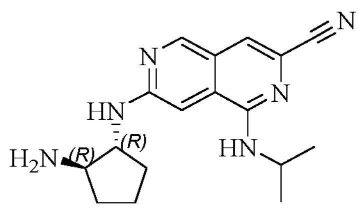 ,

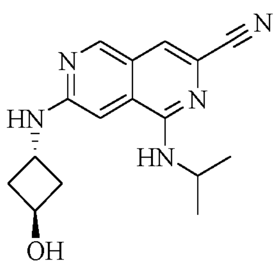 , 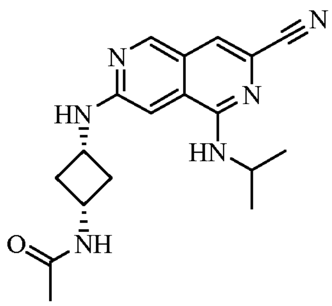 , 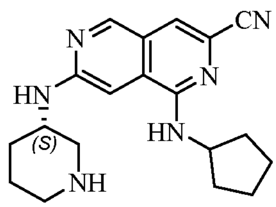 ,

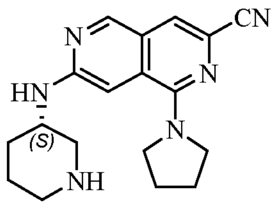 , 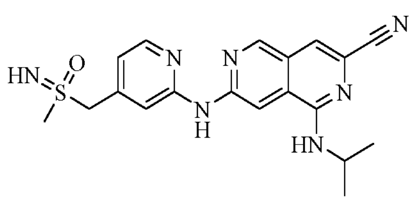 , 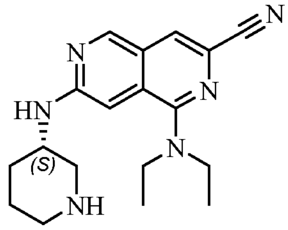 ,

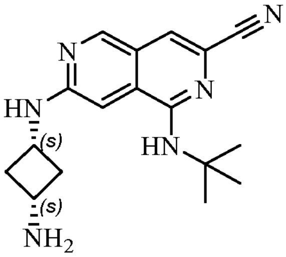
,
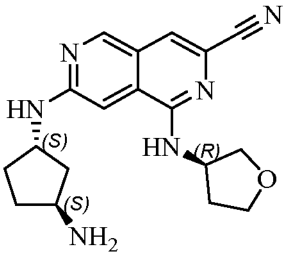
,
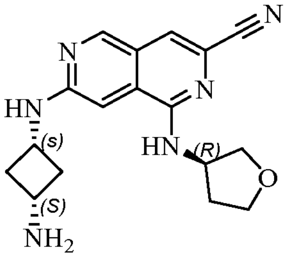
,

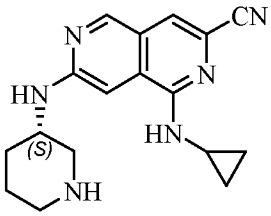
,
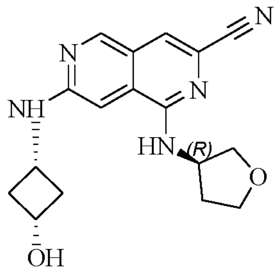
,
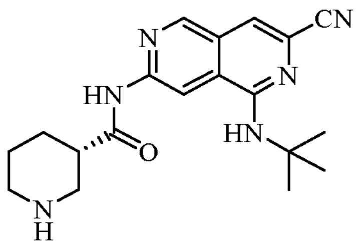
,

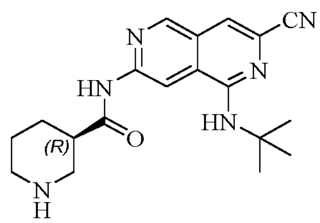
,
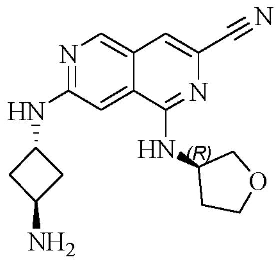
,
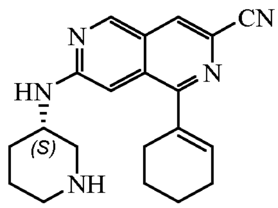
,

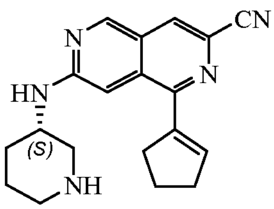
,
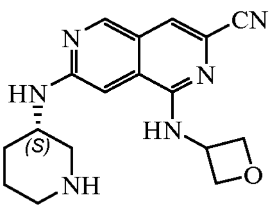
,
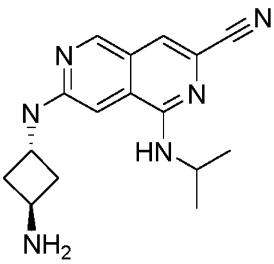
,

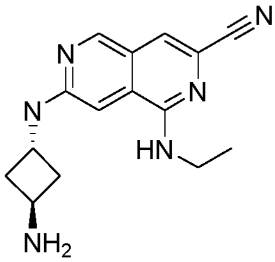
,
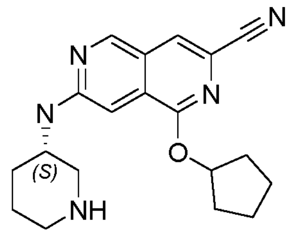
,
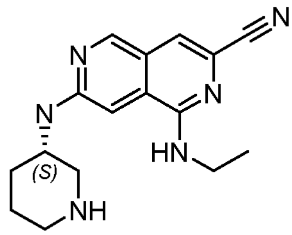
,

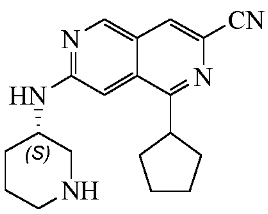
,
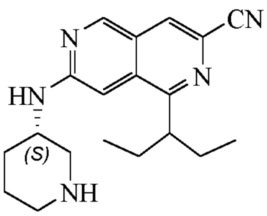
,
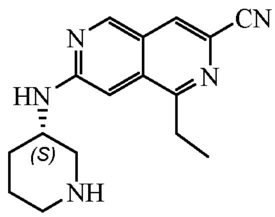
,

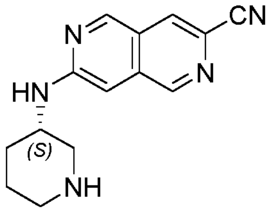
,
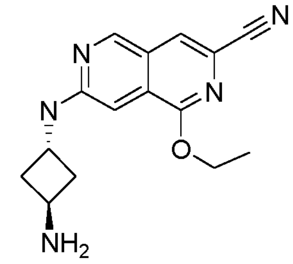
,
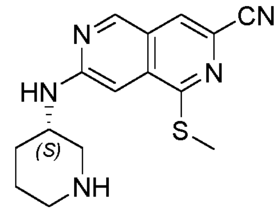
,

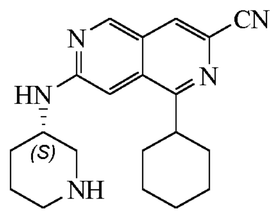
,
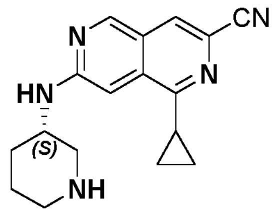
,
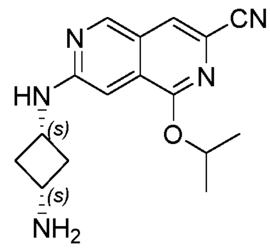
,

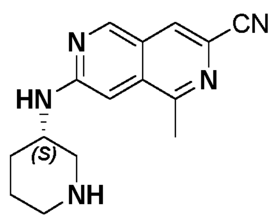
,
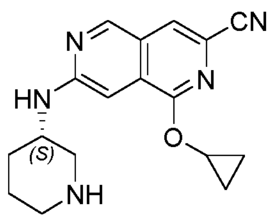
,
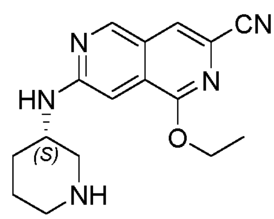
,

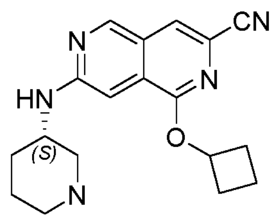
,
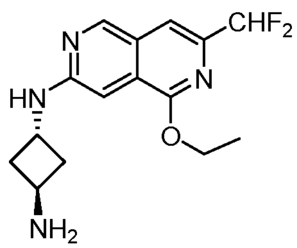
,
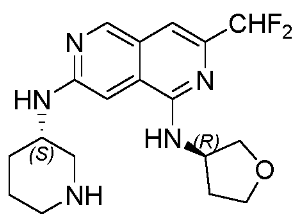
,

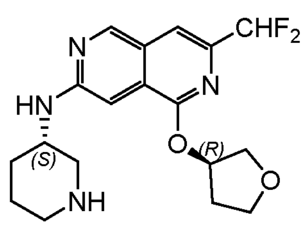
,
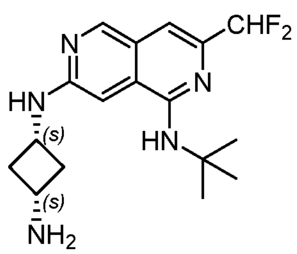
,
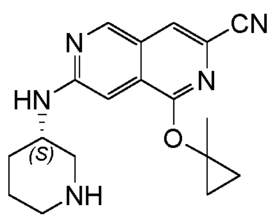
,

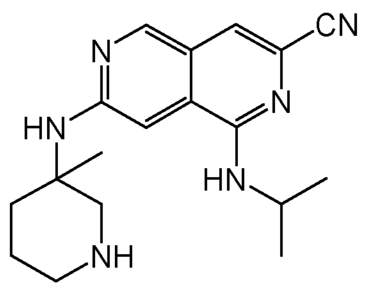 , 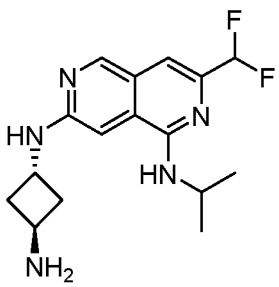 , 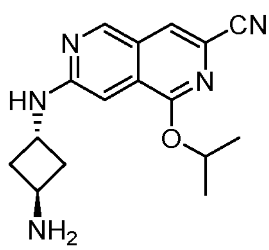 ,

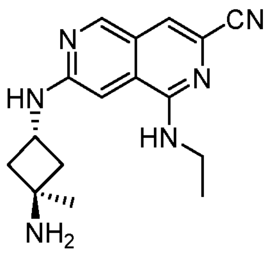 , 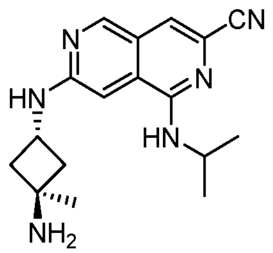 , 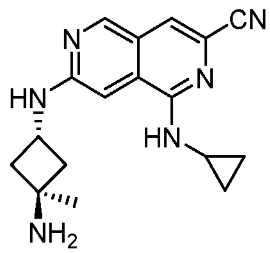 ,

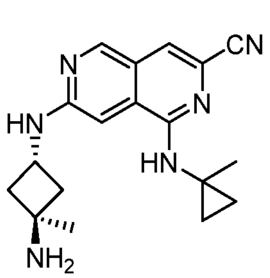 , 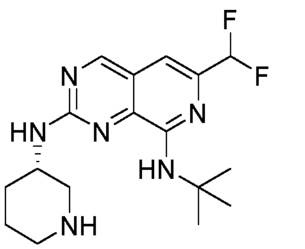 , 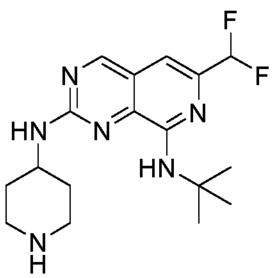 ,

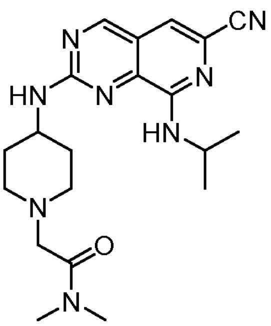 , 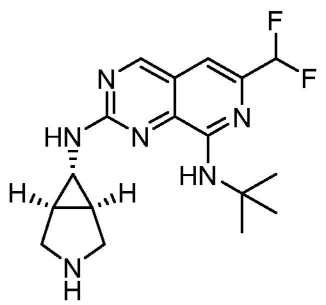 , 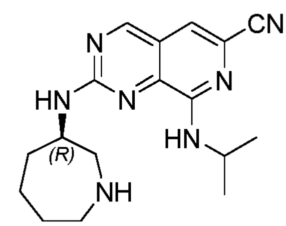 ,

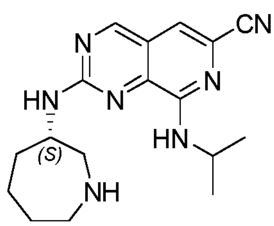 , 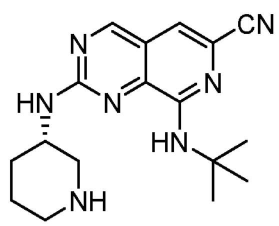 , 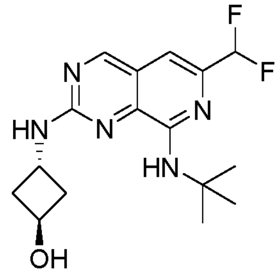 ,

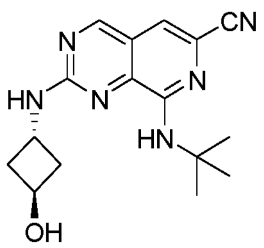,
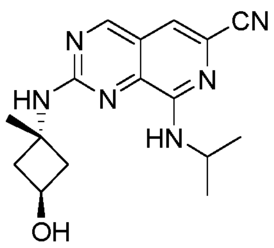,
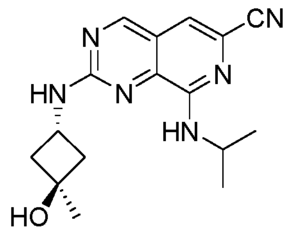,

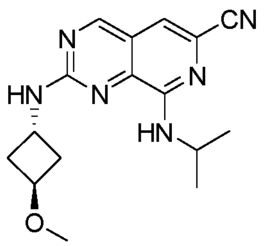,
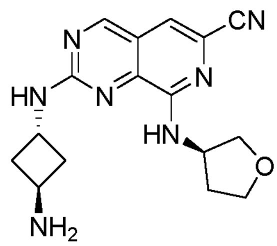,
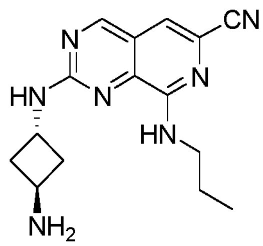,

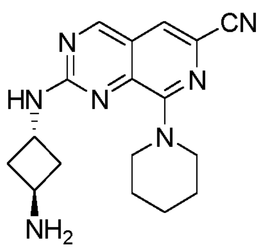,
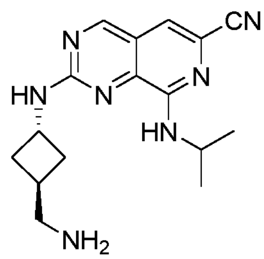,
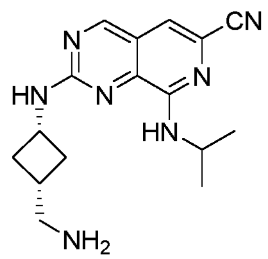,

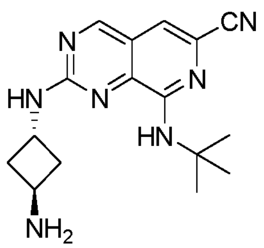,
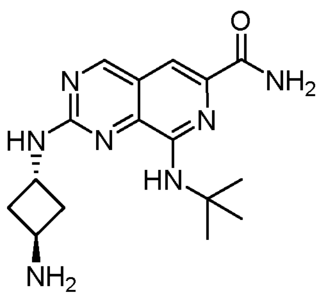,
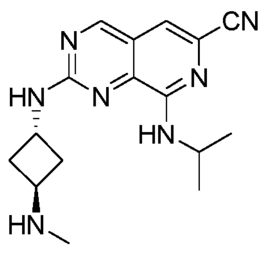,

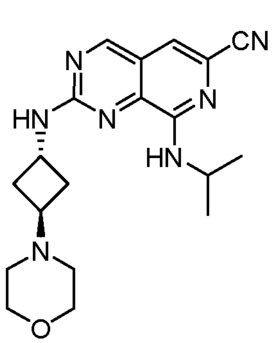,
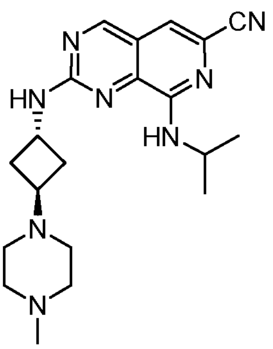,
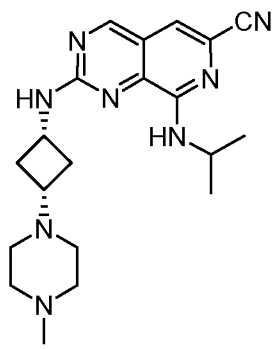,

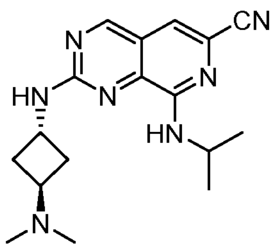 , 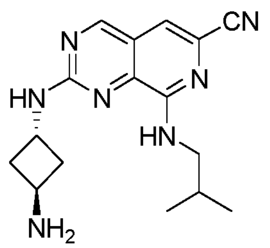 , 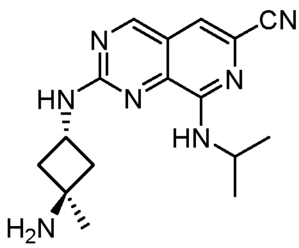 ,

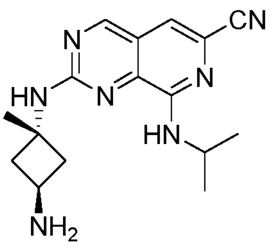 , 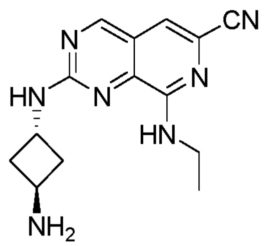 , 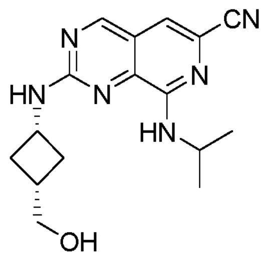 ,

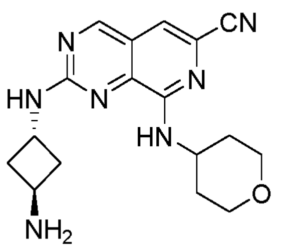 , 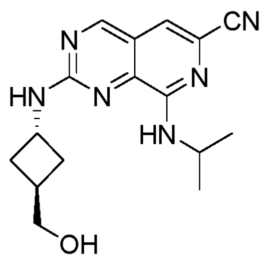 , 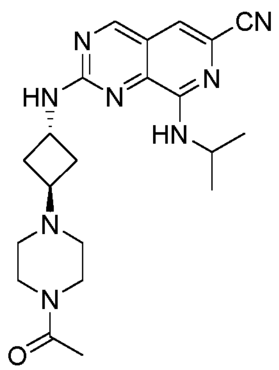 ,

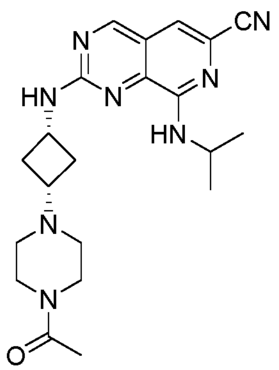 , 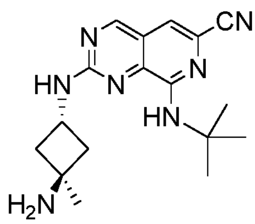 , 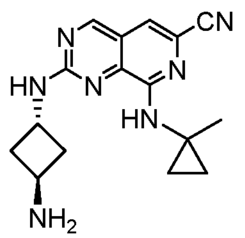 ,

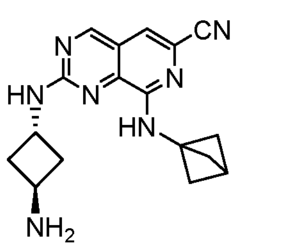 , 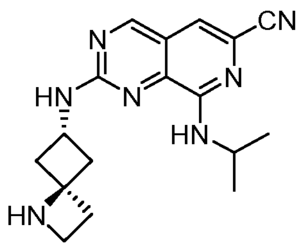 , 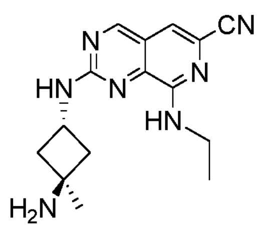 ,

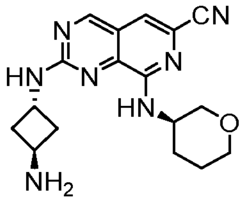, 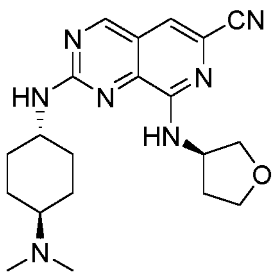, 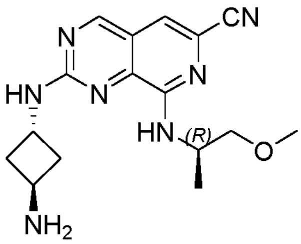,

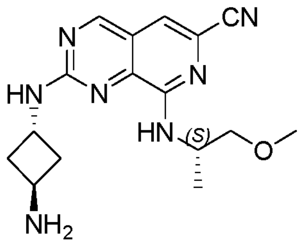, 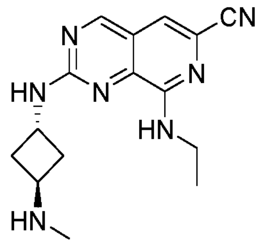, 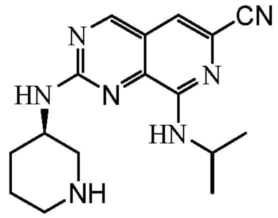,

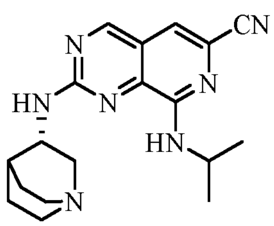, 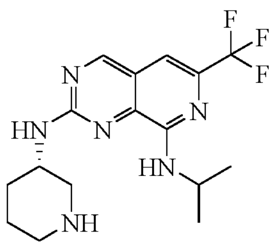, 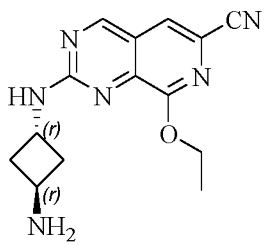,

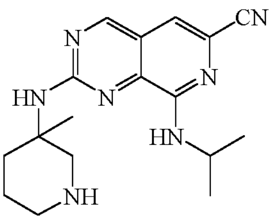, 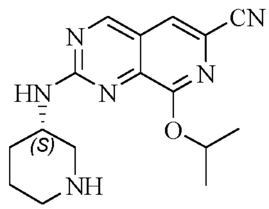

**20.** The compound or the pharmaceutically acceptable salt or the isomer thereof according to claim 1 or 2:

$X_1$ is selected from N or $CR_4$;
$X_2$ is selected from N or $CR_5$;
L is selected from bond, -C(O)-, $-(CH_2)p-$;
A is selected from bond, 3-12 membered cycloalkyl, 3-12 membered cycloalkenyl, 3-12 membered heterocyclyl, aryl, 5-10 membered heteroaryl, wherein the heteroatoms of the 3-12 membered heterocyclyl are selected from one of O, S, N or any combination thereof, C atom can be optionally oxidized to C(O), S atom can be optionally oxidized to S(O) or $S(O)_2$, the heteroatoms of the 5-10 membered heteroaryl are selected from one of O, S, and N or any combination thereof;
$R_1$ is selected from hydrogen, halogen, hydroxyl, carboxyl, amino, $C_{1-6}$ alkyl, $C_{1-6}$ alkoxy, $C_{1-6}$ alkylamino, $(C_{1-6}$ alkyl$)_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonylamino, $C_{1-6}$ alkylaminocarbonyl, $(C_{1-6}$ alkyl$)_2$ aminocarbonyl, $C_{1-6}$ alkoxycarbonyl, $C_{1-6}$ alkylcarbonyl, $C_{1-6}$ alkylsulfonyl, aminosulfonyl, $C_{1-6}$ alkylthio, 3-12 membered cycloalkyl, 3-12 membered heterocyclyl, 3-12 membered cycloalkenyl,
wherein the heteroatoms of the 3-12 membered heterocyclyl are selected from one of O, S, N or any combination thereof, C atom can be optionally oxidized to C(O), S atom can be optionally oxidized to S(O) or $S(O)_2$, the heteroatoms of the 5-10 membered heteroaryl are selected from one of O, S, and N or any combination thereof,

wherein the amino, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl , halogenated $C_{1-6}$ alkoxy, $C_{2-8}$ alkenyl, $C_{2-8}$ alkynyl, $C_{1-6}$ alkylcarbonylamino, $C_{1-6}$ alkylaminocarbonyl, ($C_{1-6}$ alkyl)$_2$ aminocarbonyl, $C_{1-6}$ alkoxycarbonyl,$C_{1-6}$ alkylcarbonyl, $C_{1-6}$ alkylsulfonyl, aminosulfonyl, $C_{1-6}$ alkylthio, 3-12 membered cycloalkyl, 3-12 membered heterocyclyl, 3-12 membered cycloalkenyl are unsubstituted or optionally substituted by one or more groups selected from hydroxyl, amino, carboxy, cyano, nitro, halogen, $C_{1-6}$ alkyl, $C_{1-6}$ alkoxy, halogenated $C_{1-6}$ alkyl, halogenated $C_{1-6}$ alkoxy, $C_{1-6}$ alkylamino, ($C_{1-6}$ alkyl)$_2$ amino, $C_{1-6}$ alkylsulfonyl, HS(O)(=NH)-, $C_{1-6}$ alkyl-S(O)(=NH)-, ($C_{1-6}$ alkyl)$_2$ aminocarbonyl, $C_{1-6}$ alkyl carbonyl, 3-12 membered cycloalkyl, 3-12 membered heterocyclyl;

$R_2$ is selected from halogen, cyano, hydroxyl, carboxyl, amino, $C_{1-6}$ alkyl, $C_{1-6}$ alkoxy, $C_{1-6}$ alkylamino, ($C_{1-6}$ alkyl)$_2$ amino, 3-12 membered cycloalkyl, 3-12 membered heterocyclyl, 3-12 membered cycloalkenyl, 3-12 membered heterocyclyloxy, 3-12 membered cycloalkyl amino, 3-12 membered heterocyclyl-$CH_2$-amino, wherein the amino, $C_{1-6}$ alkyl, $C_{1-6}$ alkoxy, $C_{1-6}$ alkylamino, ($C_{1-6}$ alkyl)$_2$ amino, 3-12 membered cycloalkyl, 3-12 membered heterocyclyl, 3-12 membered cycloalkenyl, 3-12 membered heterocyclyloxy, 3-12 membered cycloalkyl amino, 3-12 membered heterocyclyl-$CH_2$-amino are unsubstituted or optionally substituted by one or more groups selected from halogen, cyano, hydroxyl, carboxy, amino, $C_{1-6}$ alkyl, $C_{1-6}$ alkoxy, halogenated $C_{1-6}$ alkyl, halogenated $C_{1-6}$ alkoxy, $C_{1-6}$ alkylamino, ($C_{1-6}$ alkyl)$_2$ amino, 3-12 membered cycloalkyl, 3-12 membered heterocyclyl, aryl and 5-10 membered heteroaryl;

$R_3$ is selected from hydrogen, hydroxy, amino, carboxy, cyano, nitro, halogen, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonyl, $C_{1-6}$ alkylsulfonyl, $C_{1-6}$ alkylthio , 3-12 membered cycloalkyl, 3-12 membered cycloalkenyl, 3-12 membered heterocyclyl, wherein the amino, $C_{1-6}$ alkyl, $C_{1-6}$ alkoxy, $C_{1-6}$ alkylamino, ($C_{1-6}$ alkyl)$_2$ amino, halogenated $C_{1-6}$ alkyl, halogenated $C_{1-6}$ alkoxy, $C_{2-8}$ alkenyl, $C_{2-8}$ alkynyl, $C_{1-6}$ alkylcarbonyl, $C_{1-6}$ alkylsulfonyl, $C_{1-6}$ alkylthio, 3-12 membered cycloalkyl, 3-12 membered cycloalkenyl, 3-12 membered heterocyclyl are unsubstituted or optionally substituted by one or more groups selected from hydroxyl, amino, carboxyl, cyano, nitro, halogen, $C_{1-6}$ alkyl, $C_{1-6}$ alkoxy, $C_{1-6}$ alkoxy$C_{1-6}$ alkoxy, $C_{1-6}$ alkylamino, ($C_{1-6}$ alkyl)$_2$ amino, $C_{1-6}$ alkylcarbonylamino and $C_{1-6}$ alkylsulfonylamino;

n is 0 or 1;

m is 0, 1, 2 or 3;

p is 1, 2 or 3;

$R_4$, $R_5$ and $R_6$ are each independently selected from hydrogen, halogen, hydroxy, amino, carboxy, cyano, nitro, $C_{1-6}$ alkyl, $C_{2-8}$ alkenyl, $C_{2-8}$ alkynyl, and halogenated $C_{1-6}$ alkyl.

**21.** A pharmaceutical composition comprising a compound or a pharmaceutically acceptable salt or isomer thereof according to any one of claims 1-20, and any one or more second therapeutically active agents.

**22.** A pharmaceutical formulation having cyclin-dependent kinase 9 inhibitory activity, comprising a compound or a pharmaceutically acceptable salt or isomer thereof according to any one of claims 1-20, and any one or more pharmaceutically acceptable carriers.

**23.** Use of a compound or a pharmaceutically acceptable salt or isomer thereof according to any one of claims 1-20 in the manufacture of a medicament for treating or preventing CDK9-mediated related diseases.

**24.** The use according to claim 23, wherein the CDK9-mediated related diseases are cancer, preferably, the cancer is solid tumor or hematological malignancies, more preferably, the cancer is selected from adrenal tumor, melanoma, head and neck cancer, kidney cancer, bladder cancer, prostate cancer, endometrial carcinoma, cervical cancer, gastric carcinoma, colon cancer, pancreatic cancer, rectal cancer, esophageal cancer, liver cancer, lung cancer, sarcoma, breast cancer ovarian cancer, non hodgkin’s lymphoma, acute myeloid leukemia, acute lymphoblastic leukemia or myeloma.

## INTERNATIONAL SEARCH REPORT

International application No.
**PCT/CN2022/139704**

**A. CLASSIFICATION OF SUBJECT MATTER**

C07D 471/04(2006.01)i;C07D 519/00(2006.01)i;A61P 35/00(2006.01)i;A61P 35/02(2006.01)i;A61K 31/519(2006.01)i;A61K 31/4375(2006.01)i;A61K 31/5377(2006.01)i;A61K 31/439(2006.01)i;A61K 31/55(2006.01)i;A61K 31/5386(2006.01)i

According to International Patent Classification (IPC) or to both national classification and IPC

**B. FIELDS SEARCHED**

Minimum documentation searched (classification system followed by classification symbols)

C07D A61P A61K

Documentation searched other than minimum documentation to the extent that such documents are included in the fields searched

Electronic data base consulted during the international search (name of data base and, where practicable, search terms used)

CNABS, CNTXT, ENTXT, DWPI, 中国知网, CNKI, 万方数据库, WANFANG Database, Web of science, 百度学术, BAIDU SCHOLAR, Registry, Caplus: 药捷安康, 帝人制药, 吴永谦, 李琳, 王武伟, 横坂卓也, 宫也奈津美, 川崎昌纪, 环烷基, 杂环基, 吡啶, 喹啉, 细胞周期蛋白依赖性激酶, 细胞周期依赖激酶, CDK, 癌症, 肿瘤, 癌, cycloalkyl?, heterocyclic?, pyrimidin?, quinolin?, cell cycle dependent kinase?, kinase?, cancer?, tumour?, tumor?.

**C. DOCUMENTS CONSIDERED TO BE RELEVANT**

| Category* | Citation of document, with indication, where appropriate, of the relevant passages | Relevant to claim No. |
|---|---|---|
| PX | WO 2022037592 A1 (MEDSHINE DISCOVERY INC.) 24 February 2022 (2022-02-24)<br>description, pp. 6-11, 36, 43-44 and 50-52 | 1-24 |
| X | WO 2021057853 A1 (NOVARTIS AG) 01 April 2021 (2021-04-01)<br>description, pp. 33, 36 and 87 | 1-6, 9-14, 16, 20 |
| A | WO 2010094695 A1 (BOEHRINGER INGELHEIM INTERNATIONAL GMBH) 26 August 2010 (2010-08-26)<br>entire document | 1-24 |
| X | INNOCENTI, P. et al. "Expanding the scope of fused pyrimidines as kinase inhibitor scaffolds:synthesis and modification of pyrido[3,4-d]pyrimidines"<br>*Org. Biomol. Chem.*, Vol. 13, No. 3, 19 November 2014 (2014-11-19), ISSN: 1477-0520,<br>pp. 893-904 | 1-6, 8-14, 16-17, 20, 22 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 February 2023** | **23 February 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| Facsimile No. **(86-10)62019451** | Telephone No. |

Form PCT/ISA/210 (second sheet) (July 2022)

INTERNATIONAL SEARCH REPORT

International application No.
PCT/CN2022/139704

C. DOCUMENTS CONSIDERED TO BE RELEVANT

| Category* | Citation of document, with indication, where appropriate, of the relevant passages | Relevant to claim No. |
|---|---|---|
| X | STN. "compound with RN number No. 2094907-55-6" *STN Registry*, 03 May 2017 (2017-05-03), text, p. 1 | 1-3, 5, 9-14, 16, 20 |
| X | STN. "compound with RN number No. 1823295-18-6" *STN Registry*, 06 December 2015 (2015-12-06), text, pp. 1-2 | 1-3, 5, 9-14, 16, 20 |
| X | STN. "compound with RN number No. 1538830-01-1" *STN Registry*, 07 February 2014 (2014-02-07), text, p. 2 | 1-3, 5, 9-14, 16, 20 |
| X | STN. "compound with RN number No. 1367738-48-4" *STN Registry*, 13 April 2012 (2012-04-13), text, p. 15 | 1-3, 5, 9-14, 16, 20 |
| X | STN. "compound with RN number No. 1506804-51-8" *STN Registry*, 30 December 2013 (2013-12-30), text, p. 3 | 1-3, 5, 9-14, 16, 20 |
| X | STN. "compound with RN number No. 1504100-94-0" *STN Registry*, 26 December 2013 (2013-12-26), text, p. 3 | 1-3, 5, 9-14, 16, 20 |
| X | STN. "compound with RN number No. 1538337-11-9" *STN Registry*, 06 February 2014 (2014-02-06), text, pp. 2-3 | 1-3, 5, 9-14, 16, 20 |
| X | STN. "compound with RN number No. 1425031-89-5" *STN Registry*, 18 March 2013 (2013-03-18), text, p. 4 | 1-3, 5, 9-14, 16, 20 |
| X | STN. "compound with RN number No. 1369293-14-0" *STN Registry*, 26 December 2023 (2023-12-26), text, p. 3 | 1-3, 5, 9-14, 16, 20 |
| X | STN. "compound with RN number No. 1369286-27-0" *STN Registry*, 16 April 2012 (2012-04-16), text, p. 5 | 1-3, 5, 9-14, 16, 20 |
| X | STN. "compound with RN number No. 1369282-72-3" *STN Registry*, 16 April 2012 (2012-04-16), text, pp. 5-6 | 1-3, 5, 9-14, 16, 20 |
| X | STN. "compound with RN number No. 1369280-17-0" *STN Registry*, 16 April 2012 (2012-04-16), text, p. 6 | 1-3, 5, 9-14, 16, 20 |
| X | STN. "compound with RN number No. 1369279-91-3" *STN Registry*, 16 April 2012 (2012-04-16), text, p. 7 | 1-3, 5, 9-14, 16, 20 |
| X | STN. "compound with RN number No. 1369182-48-8" *STN Registry*, 16 April 2012 (2012-04-16), text, p. 7 | 1-3, 5, 9-14, 16, 20 |
| X | STN. "compound with RN number No. 1369182-34-2" *STN Registry*, 16 April 2012 (2012-04-16), text, pp. 7-8 | 1-3, 5, 9-14, 16, 20 |
| X | STN. "compound with RN number No. 1369174-20-8" *STN Registry*, 16 April 2012 (2012-04-16), text, p. 8 | 1-3, 5, 9-14, 16, 20 |

Form PCT/ISA/210 (second sheet) (July 2022)

INTERNATIONAL SEARCH REPORT

International application No.
**PCT/CN2022/139704**

**C. DOCUMENTS CONSIDERED TO BE RELEVANT**

| Category* | Citation of document, with indication, where appropriate, of the relevant passages | Relevant to claim No. |
|---|---|---|
| X | STN. "compound with RN number No. 1368391-51-8" *STN Registry,* 15 April 2012 (2012-04-15), text, pp. 8-9 | 1-3, 5, 9-14, 16, 20 |
| X | STN. "compound with RN number No. 1368254-30-1" *STN Registry,* 15 April 2012 (2012-04-15), text, p. 9 | 1-3, 5, 9-14, 16, 20 |
| X | STN. "compound with RN number No. 1368088-62-3" *STN Registry,* 15 April 2012 (2012-04-15), text, pp. 9-10 | 1-3, 5, 9-14, 16, 20 |
| X | STN. "compound with RN number No. 1368067-46-2" *STN Registry,* 15 April 2012 (2012-04-15), text, p. 10 | 1-3, 5, 9-14, 16, 20 |
| X | STN. "compound with RN number No. 1368062-17-2" *STN Registry,* 15 April 2012 (2012-04-15), text, pp. 10-11 | 1-3, 5, 9-14, 16, 20 |
| X | STN. "compound with RN number No. 1368027-32-0" *STN Registry,* 15 April 2012 (2012-04-15), text, p. 11 | 1-3, 5, 9-14, 16, 20 |
| X | STN. "compound with RN number No. 1367838-10-5" *STN Registry,* 13 April 2012 (2012-04-13), text, pp. 11-12 | 1-3, 5, 9-14, 16, 20 |
| X | STN. "compound with RN number No. 1367827-03-9" *STN Registry,* 13 April 2012 (2012-04-13), text, p. 12 | 1-3, 5, 9-14, 16, 20 |
| X | STN. "compound with RN number No. 1367822-36-3" *STN Registry,* 13 April 2012 (2012-04-13), text, p. 12 | 1-3, 5, 9-14, 16, 20 |
| X | STN. "compound with RN number No. 1367807-87-1" *STN Registry,* 13 April 2012 (2012-04-13), text, p. 13 | 1-3, 5, 9-14, 16, 20 |
| X | STN. "compound with RN number No. 1367778-06-0" *STN Registry,* 13 April 2012 (2012-04-13), text, p. 13 | 1-3, 5, 9-14, 16, 20 |
| X | STN. "compound with RN number No. 1367744-99-7" *STN Registry,* 13 April 2012 (2012-04-13), text, p. 14 | 1-3, 5, 9-14, 16, 20 |
| X | STN. "compound with RN number No. 1367743-10-9" *STN Registry,* 13 April 2012 (2012-04-13), text, pp. 14-15 | 1-3, 5, 9-14, 16, 20 |
| X | STN. "compound with RN number No. 1500319-19-6" *STN Registry,* 22 December 2013 (2013-12-22), text, p. 4 | 1-3, 5, 9-14, 16, 20 |

Form PCT/ISA/210 (second sheet) (July 2022)

**INTERNATIONAL SEARCH REPORT**
**Information on patent family members**

International application No.
**PCT/CN2022/139704**

| Patent document cited in search report | | | Publication date (day/month/year) | Patent family member(s) | | | Publication date (day/month/year) |
|---|---|---|---|---|---|---|---|
| WO | 2022037592 | A1 | 24 February 2022 | None | | | |
| WO | 2021057853 | A1 | 01 April 2021 | EP | 4034535 | A1 | 03 August 2022 |
| | | | | KR | 20220070229 | A | 30 May 2022 |
| | | | | IL | 291499 | A | 01 May 2022 |
| | | | | JP | 2022550353 | A | 01 December 2022 |
| | | | | CA | 3150284 | A1 | 01 April 2021 |
| | | | | AU | 2020355837 | A1 | 24 February 2022 |
| | | | | US | 2022280509 | A1 | 08 September 2022 |
| | | | | CN | 114746414 | A | 12 July 2022 |
| WO | 2010094695 | A1 | 26 August 2010 | EP | 2398797 | A1 | 28 December 2011 |
| | | | | EP | 2398797 | B1 | 06 November 2013 |
| | | | | CA | 2752265 | A1 | 26 August 2010 |
| | | | | JP | 2012517986 | A | 09 August 2012 |
| | | | | JP | 5603883 | B2 | 08 October 2014 |
| | | | | US | 2012046270 | A1 | 23 February 2012 |
| | | | | US | 8569316 | B2 | 29 October 2013 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Structural insights into the functional diversity of the CDK-cyclin family[J. *Open Biology,* 2018, vol. 8 (9), 180112
- CDK9: A Comprehensive Review of Its Biology, and Its Role as a Potential Target for Anti-Cancer Agents[J. *Frontiers in Oncology,* 2021, vol. 11, 678559
- **DANG C V ; REDDY E P ; SHOKAT K M et al.** Drugging the 'undruggable' cancer targets[J. *Nature Reviews Cancer,* 2017
- **WU T ; QIN Z ; TIAN Y et al.** Recent Developments in the Biology and Medicinal Chemistry of CDK9 Inhibitors: An Update[J. *Journal of Medicinal Chemistry,* 2020, vol. 63 (22), 13228-13257